# EUROPEAN PATENT APPLICATION

(11) **EP 2 088 143 A1**
(43) Date of publication of application: **12.08.2009**
(21) Application number: 08290065.5
(22) Date of filing: 24.01.2008
(51) Int. Cl.: C07D 241/08, A61K 31/496, A61P 31/06

(54) **Novel inhibitors of mycobacterium tuberculosis complex cytochrome P450 CYP121**

(71) Applicant: COMMISSARIAT A L'ENERGIE ATOMIQUE, 75015 Paris (FR)
(72) Inventor: Belin, Pascal, 91430 Igny (FR); Lecoq, Alain, 91540 Mennecy (FR); Beaurepaire Ledu, Marie-Hélène, 91120 Palaiseau (FR); Gondry, Muriel, 91190 Gif-sur-Yvette (FR); Pernodet, Jean-Luc, 94230 Cachan (FR)
(74) Representative: Thomas, Dean

(57) **Abstract**

The present invention relates to the identification of the cyclodipeptide cyclo(L-Tyr-L-Tyr) as substrate of CYP121 from *Mycobacterium tuberculosis,* to the crystal structures of CYP121 complexed either with cyclo(L-Tyr-L-Tyr) or with other cyclodipeptides, to the use of these structures in rational drug design methods to generate cyclodipeptide-based molecules that could inhibit CYP121 activity, and to the design of new anti-*Mycobacterium tuberculosis* agents based on the DKP scaffold.

## Description

The invention relates to a novel class of inhibitor to the *Mycobacterium tuberculosis* complex cytochrome P450 CYP121 and methods of rationally designing and testing members of this novel class of inhibitor. Such inhibitors may be useful as antimycobacterial and antitubercular agents.

Tuberculosis (TB) is a disease currently responsible of nearly 2 million deaths annually (World Health Organization fact sheet on TB at http://www.who.int/mediacentre/factsheets/fs104/en/index.html). The major causes of these deaths are the high susceptibility to TB of HIV-infected individuals and the appearance of numerous multidrug-resistant strains of *Mycobacterium tuberculosis,* the human pathogen responsible for TB (1,2).

This situation has been exasperated for more than thirty years by the absence of new classes of therapeutics reaching the market, illustrating the difficulty in finding active compounds against *M*. *tuberculosis* (3).

Today, it is clear that to fight against TB, novel drugs are needed in order to stop the spread of this threat to human health. During the last years, several efforts have been made using novel strategies to find new molecules with antimycobacterial activity (4). As one of several targets, *M*. *tuberculosis* cytochrome P450s have recently received much attention (5).

Cytochrome P450s (P450s) are heme-containing monooxygenases that metabolize structurally different but rather hydrophobic substrates into more hydrophilic products concomitantly to the reductive scission of molecular oxygen (6). P450s are widely distributed in living organisms from bacteria to mammals where they have various functions. In prokaryotes, P450s have been essentially implicated in secondary metabolite synthesis and alternative carbon source utilization and it was surprising when 20 P450 encoding-genes were found in the *M*. *tuberculosis* genome (7).

This number of P450 genes has been postulated to be linked to lipid metabolism, which in *Mycobacterium tuberculosis* involves the synthesis of a set of highly diverse and complex lipophilic molecules which are found especially in the cell wall and which are an essential component of *M*. *tuberculosis* pathogenicity (8). This view is supported by the observation that incubation of mycobacteria with azole molecules that are known inhibitors of P450s activity, results in the alteration of the cell wall glycopeptidolipid content (9).

An azole is a class of five-membered nitrogen heterocyclic ring compounds containing at least one other noncarbon atom, nitrogen, sulfur or oxygen.

Furthermore, these azole molecules have recently been found to exhibit antimycobacterial and antitubercular activities, suggesting that P450s could support essential functions in mycobacteria and highlighting mycobacterial P450s as potential targets for new drugs (9-12). Among the twenty P450s encoded in the *M*. *tuberculosis* genome, CYP121 has appeared as a potential target to explain mycobacteria sensitivity to azoles indicating that inhibition of CYP121 may provide a new way for antimycobacterial and antitubercular drug design (5).

CYP121 is encoded in all genome sequences available for strains of the *Mycobacterium tuberculosis* complex (*Mycobacterium tuberculosis*, *Mycobacterium africanum*, *Mycobacterium bovis* and *Mycobacterium microti*) that are all responsible for tuberculosis in human or animals.

The determinations of X-ray structures of free CYP121 and azole-bound CYP121 has opened the way to the rational design of CYP121 azole-based inhibitors (13,14). However, since the azoles are non-selective ligands of cytochrome P450, the use of azole-derived drugs in the treatment of human/animal disease gives rise to numerous undesired side effects. In particular azoles target the human liver and hence modify a treated human's metabolism. So far several azoles have been shown to modify P450s activities in experimental animals and also to be hepatotoxic (15).

A way to get more selective enzyme inhibitors is based on the design of substrate analogs that are not metabolized as is the natural substrate, but which display efficient enzyme binding. Such an approach was previously used to design specific inhibitors of aromatase, a human P450 implicated in the synthesis of oestrogen and whose inhibition it is hoped could be efficient in the diminution of breast cancer induction (16).

The present invention provides the identification of the CYP121 substrate, cyclodipeptide cyclo(L-Tyr-L-Tyr) (SEQ ID NO:4), the invention also provides a X-ray structure of cyclo(L-Tyr-L-Tyr)-bound CYP121, X-ray structures of CYP121 bound to various other cyclodipeptides and a rational design scheme to generate substrate analogues that inhibit CYP121 activity.

The inventors have investigated the CYP121 substrate and the structural characterization of its interaction with the enzyme. To identify the substrate of CYP121, they took advantage of their previous studies of Rv2275 from *M*. *tuberculosis* (Belin et al., PCT/IB2006/001852) whose gene is adjacent to the CYP121-encoding gene *rv2276* (7). The two genes are transcribed in the same direction, overlap by 4 nucleotides and no transcription and termination signals can be identified between them.

*rv2275* encodes a protein homologous to the cyclodipeptide synthase AlbC of *Streptomyces noursei*, involved in the synthesis of cyclo(L-Phe-L-Leu) (26). The inventors have recently shown that Rv2275 catalyses the synthesis of the cyclodipeptide cyclo(L-Tyr-L-Tyr) (Belin et al., PCT/IB2006/001852).

The identification by the inventors that cyclo(L-Tyr-L-Tyr) (SEQ ID NO:4) is a substrate for CYP121 is surprising as the cyclo(L-Tyr-L-Tyr) (SEQ ID NO:4) is a small molecule that occupies only partially (∼ 300 Å³) the large active site cavity (∼ 1350 Å³) of CYP121, thus splitting it in two cavities filled with water.

The spectral binding constant the inventors have determined (27 µM) is relatively weak when compared to other dissociation constants obtained for various prokaryotic P450 natural substrate systems and whose values are generally in the micromolar range (30). However, the rate of cyclo(L-Tyr-L-Tyr) (SEQ ID NO:4) transformation was found to be 3.8 nmoles/min/nmole of CYP121 under the *in vitro* conditions used here, that is consistent with P450/substrate relationship. Although it is not excluded that CYP121 could bind larger molecules *in vivo*, the inventors data together with the chromosomal organization of the CYP121 gene and *rv2275* strongly suggest that the cyclo(L-Tyr-L-Tyr) (SEQ ID NO:4) synthesized by Rv2275 is the natural substrate of CYP 121.

With this finding therefore and the substantial body of structural data concerning the interactions of CYP121 and its substrate, the inventors have been able to establish a new class of CYP121 inhibitors which therefore also affect *Mycobacterium tuberculosis* complex viability/pathogenicity.

According to a first aspect of the present invention there is provided a compound of the formula (i): 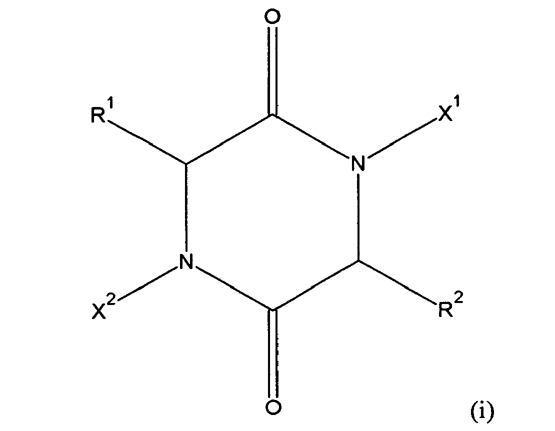 wherein the dione six-membered ring is referred to as the diketopiperazine cycle
wherein one of R¹ and R² is a tyrosyl side chain or a substituted tyrosyl side chain, then the other is any proteinogenic or non-proteinogenic amino acid side chain except for a tyrosyl side chain when said one of R¹ and R² is a tyrosyl side chain; and
wherein X¹ and X² which may be identical or different are any suitable chemical groups and in particular may be a hydrogen atom, a hydroxyl group, amino group, a (C₁-C₆) alkyl group (linear or branched), an aromatic group optionally substituted and suitable combinations of these groups and in particular O-alkyl moiety, O-aromatic moiety, N-alkyl moiety, N-aromatic moiety, functionalized alkyl group, functionalized aromatic group;
for use in a method of inhibiting CYP121 activity in conditions
wherein said CYP121 is active.

In the instant invention for instance:
- aromatic group means a C₆ aromatic ring optionally substituted (functionalized) and aromatic groups containing at least one heteroatom (N, O, S,...).
- functionalized group means that said group is substituted.
- substituted tyrosyl chain means that it is substituted on positions 3, 5 or 6 of the ring by any group as defined for X¹ or X².

The inventors have shown that compounds comprising a diketopiperazine cycle, with a tyrosine or modified tyrosine residue and a second amino acid residue are able to interact with CYP121 and wherein this compound does not comprise two natural tyrosine residues, such compounds can inhibit CYP121 activity by a competitive binding mechanism or other means. Therefore as previous work has shown that CYP121 binds with high affinity to econazole and clotrimazole (12) which are azole molecules with antimycobacterial activity (11) and antitubercular activity (10), the inhibition of this gene product makes this new class of molecules suitable for use in a method for treating conditions in which CYP121 is active such as Tuberculosis (*Mycobacterium tuberculosis* infection) or other conditions associated with a *Mycobacterium* infection.

For the purpose of the invention, the term "diketopiperazine" or "DKP" or "2,5-diketopiperazine" or "cyclic dipeptide" or "cyclodipeptide" or "cyclic diaminoacids" is intended to mean molecules having diketopiperazine (piperazine-2,5-dione or 2,5-dioxopiperazine) ring.

An amino acid is a molecule that contains both amine and carboxyl functional groups. In biochemistry, this term refers to alpha-amino acids with the general formula H₂NCHRCOOH, where R is an organic substituent. In the alpha amino acids, the amino and carboxylate groups are attached to the same carbon, which is called the α-carbon. The various alpha amino acids differ in which side chain (R group) is attached to their alpha carbon. They can vary in size from just a hydrogen atom in glycine, through a methyl group in alanine, to a large heterocyclic group in tryptophan. Twenty standard amino acids are used by cells in protein biosynthesis, and these are specified by the general genetic code.

Aside from the twenty standard amino acids, there are a vast number of "non-standard" amino acids. Two of these can be specified by the genetic code, but are rather rare in proteins. Selenocysteine is incorporated into some proteins at a UGA codon, which is normally a stop codon. Pyrrolysine is used by some methanogenic archaea in enzymes that they use to produce methane. It is coded for with the codon UAG.

Examples of nonstandard amino acids that are not found in proteins (non-proteinogenic amino acids) include lanthionine, 2-aminoisobutyric acid, dehydroalanine and the neurotransmitter gamma-aminobutyric acid. Nonstandard amino acids often occur as intermediates in the metabolic pathways for standard amino acids - for example ornithine and citrulline occur in the urea cycle, part of amino acid catabolism.

Nonstandard amino acids are usually formed through modifications to standard amino acids. For example, homocysteine is formed through the transsulfuration pathway or by the demethylation of methionine via the intermediate metabolite S-adenosyl methionine, while dopamine is synthesized from L-DOPA, and hydroxyproline is made by a posttranslational modification of proline.

In addition nonstandard or substituted natural amino acids can also be generated *in vitro,* which are entirely absent from nature. Such completely artificial amino acids can comprise chemical groups which would normaly be toxic to an organism or be impossible to create with an *in vivo* system.

Herein proteinogenic amino acid side chain means any natural or artificial amino acid which can be used to form a protein or peptide.

Herein non-proteinogenic amino acid side chain means any natural or artificial amino acid which can not be used to form a protein.

In particular the at least one condition is caused by infection by a Mycobacterium.

Preferably said Mycobacterium is *Mycobacterium tuberculosis.*

In particular the compound is not catalysed by CYP121.

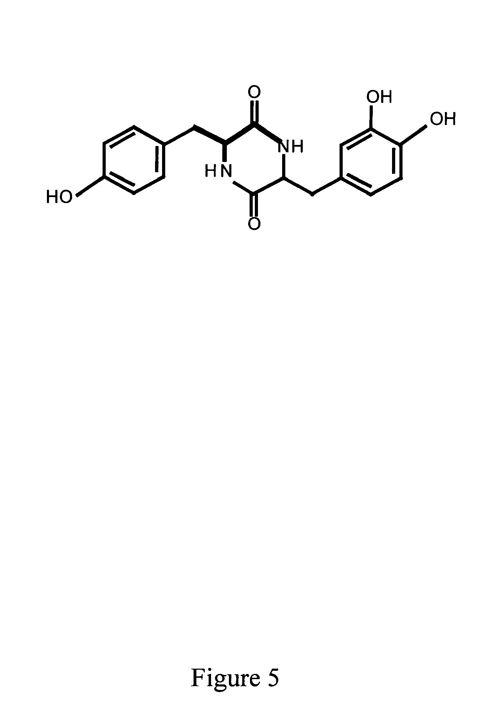

A non-catalysed compound has many benefits such as a longer half-life and the potential to remain within or around the enzymatic active site so increasing its inhibitory effect. An example of a non-catalysed compound is cyclo(L-3 or 5,4-dihydroxyphenylalanine-L-Tyr), called herein cyclo(L-DOPA-L-Tyr) (SEQ ID NO: 8) (Figure 5).

Catalysed compounds are also within the scope of the current invention and such compounds may have benefits such as a reduced toxicity of the products of catalysis versus the substrate to a subject treated with such a substrate compound and the compounds can comprise further chemical moieties which before or following catalysis can lead to the permanent bonding of the compound to CYP121 so permanently deactivating the enzyme. A example of a catalysed compound is cyclo(L-Tyr-L-Trp) (SEQ ID NO: 5), which the inventors show herein to be catalysed by CYP121 and able to inhibit the action of CYP 121 on its natural target cyclo(L-Tyr-L-Tyr) (SEQ ID NO: 4).

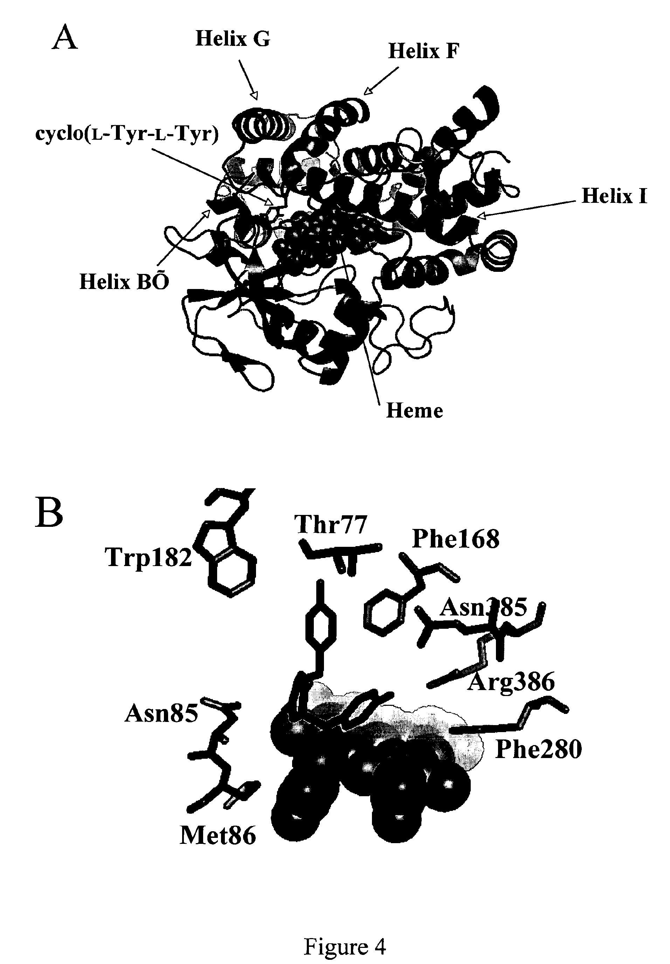

The inventors have using the structural information they have generated of CYP121 in combination with cyclo(L-Tyr-L-Tyr) (SEQ ID NO:4) and other cyclodipeptides determined several key features of molecules which have a high affinity for CYP121. This data is set out in Table I below and Figures 4, 9, 13, 14, 15, 16, 17 and 18.

In particular the compound establishes contacts with one or more portions of CYP121 selected from the group:
the heme group,
at least one of the residues of the 3₁₀ helix-B' helix region which comprises residues P79 P80 E81 V82 V83 N84 N85 M86 G87 N88 I89 A90 D91 A92,
residues Phe 168 and/or Trp182 and /or Phe280,
residues Gln385 and/or Arg386.

The inventors have also determined several molecular changes which occur in CYP 121 following its interaction with a target compound.

In particular the interaction of the compound with CYP121 causes a shift in spin equilibrium of the heme iron of CYP121.

In particular the interaction of the compound with CYP121 causes the spin equilibrium of the heme iron of CYP121 to change from the low spin form to the high spin form.

In particular the compound is selected from the list consisting of: SEQ ID NO: 5, SEQ ID NO: 6, SEQ ID NO: 7, SEQ ID NO: 8.

In particular the compound following catalysis by CYP121 generates a reactive intermediate that can bind to CYP121 irreversibly.

The use of a compound comprising one or more chemical features which following catalysis by CYP121 results in an active intermediate which can then react in turn with CYP121 and deactivate this enzyme, is a particularly attractive potential use of a compound with affinity for CYP121 according to the current invention. This so called 'suicide mechanism' of deactivating CYP121 can be mediated by a number of chemical moieties (32).

In particular the compound comprises at least one dihalomethyl and/or acetylenic moiety.

According to a second aspect of the present invention there is provided a medicament, **characterized in that** it comprises a compound of the formula (i): 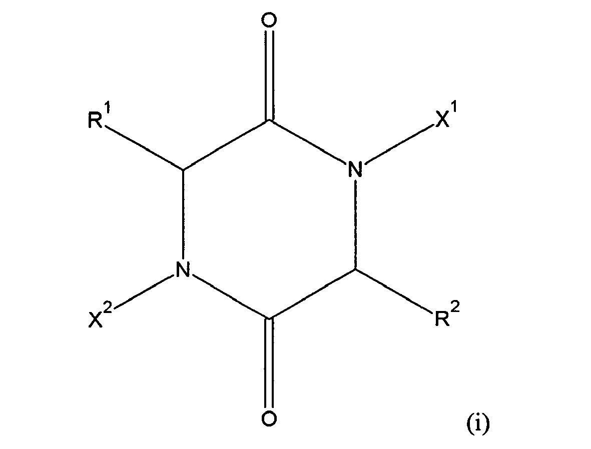 wherein the dione six-membered ring is referred to as the diketopiperazine cycle
wherein one of R¹ and R² is a tyrosyl side chain or a substituted tyrosyl side chain, then the other is any proteinogenic or non-proteinogenic amino acid side chain except for a tyrosyl side chain when said one of R¹ and R² is a tyrosyl side chain; and
wherein X¹ and X² which may be identical or different are any suitable chemical groups and in particular may be a hydrogen atom, a hydroxyl group, amino group, a (C₁-C₆) alkyl group (linear or branched), an aromatic group optionally substituted and suitable combinations of these groups and in particular O-alkyl moiety, O-aromatic moiety, N-alkyl moiety, N-aromatic moiety, functionalized alkyl group, functionalized aromatic group;
for use in a method of treating a *Mycobacterium* infection.

The compound comprised in this medicament can have any of the features previously described as part of the first aspect of the present invention.

According to a third aspect of the present invention there is provided a pharmaceutical composition, **characterized in that** it comprises:
a compound of the formula (i): 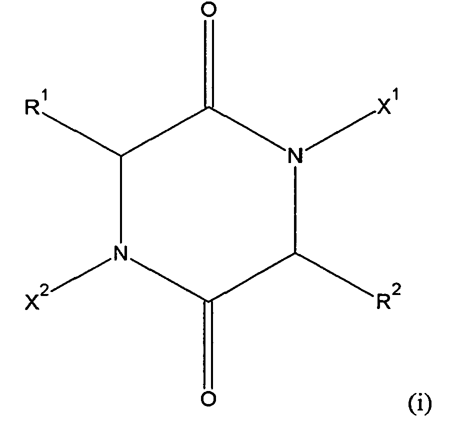
wherein the dione six-membered ring is referred to the diketopiperazine cycle
wherein one of R¹ and R² is a tyrosyl side chain or a substituted tyrosyl side chain, then the other is any proteinogenic or non-proteinogenic amino acid side chain except for a tyrosyl side chain when said one R¹ and R² is a tyrosyl side chain; and
wherein X¹ and X² which may be identical or different are any suitable chemical groups and in particular may be a hydrogen atom, a hydroxyl group, amino group, a (C₁-C₆) alkyl group (linear or branched), an aromatic group optionally substituted and suitable combinations of these groups and in particular O-alkyl moiety, O-aromatic moiety, N-alkyl moiety, N-aromatic moiety, functionalized alkyl group, functionalized aromatic group;
at least one pharmaceutically acceptable vehicle;
for use in a method of treating a *Mycobacterium* infection.

The compound comprised in this pharmaceutical composition can have any of the features previously described as part of the first aspect of the present invention.

According to a fourth aspect of the present invention there is provided X-ray three dimensional structures of CYP121 in combination with a target selected from the list consisting of SEQ ID NO: 4, SEQ ID NO: 5, SEQ ID NO: 6, SEQ ID NO: 7, SEQ ID NO: 8; SEQ ID NO: 9;
generated with the atomic coordinates listed in Table I and Figures 4, 9, 13, 14, 15, 16, 17 and 18.

The inventors have using the structural information they have generated of CYP121 in combination with cyclodipeptides selected from a list consisting of SEQ ID NO: 4, SEQ ID NO: 5, SEQ ID NO: 6, SEQ ID NO: 7, SEQ ID NO: 8; SEQ ID NO: 9 determined several key features of molecules which have affinity for CYP121. This data is set out in Table I below and Figures 4, 9, 13, 14, 15, 16, 17 and 18.

In particular the compound establishes contacts with one or more portions of CYP121 selected from the group:
the heme group,
at least one of the residues of the 3₁₀ helix-B' helix region which comprises residues P79 P80 E81 V82 V83 N84 N85 M86 G87 N88 I89 A90 D91 A92,
residues Gln385 and/or Arg386
residues Phe168 and/or Trp 182 and /or Phe280.

According to a fifth aspect of the present invention there is provided a method to design a compound which inhibits the activity of CYP121 by using the atomic coordinates and X-ray three dimensional structures of CYP121 bound to any of the cyclodipeptide selected from a list consisting of SEQ ID NO: 4, SEQ ID NO: 5, SEQ ID NO: 6, SEQ ID NO: 7, SEQ ID NO: 8; SEQ ID NO: 9 and described in the fourth aspect of the invention and consisting of the steps:
a) generating a 3-dimensional model of said compound in combination with CYP121 by using the interaction network between CYP121 and cyclodipeptides selected from a list consisting of SEQ ID NO: 4, SEQ ID NO: 5, SEQ ID NO: 6, SEQ ID NO: 7, SEQ ID NO: 8; SEQ ID NO: 9 and described in the fourth aspect of the invention:
   the heme group,
   at least one of the residues of the 3₁₀ helix-B' helix region which comprises residues P79 P80 E81 V82 V83 N84 N85 M86 G87 N88 I89 A90 D91 A92, and
   residues Gln385 and/or Arg386
   residues Phe168 and/or Trp182 and or/Phe280
b) designing said compound by modifying chemical groups thereon and testing the interaction of said compound with CYP121 using the model generated in a) by using bioinformatics tools to determine interactions of said compound with one of the features of CYP121 selected from the group:
   i) the heme group,
   ii) at least one of the residues bordering the active site first cavity located between helices F and G and comprising residues S163 L164 I166 A167 F168 I175 A178 K179 N181 W182 D185,
   iii) at least one of the residues bordering the active site second cavity located between the F/G loop and B/B' loop and comprising residues R72 L73 N74 L76 V78 A167 F168 M169 S170 S171 W182;
   iv) at least one of the residues bordering the active site third cavity located just above the heme and comprising residues M62 R72 L73 N74 L76 T77 F168 A233 I236 S237 F280 D282 G283 L284 P285 Q385 R386.

In particular, the said compound could be designed by adding any chemical group to any of the cyclodipeptide selected from a list consisting of SEQ ID NO: 4, SEQ ID NO: 5, SEQ ID NO: 6, SEQ ID NO: 7, SEQ ID NO: 8; SEQ ID NO: 9

In particular, the said compound could be designed by adding any chemical group to the diketopiperazine cycle,

In particular, the said compound could be designed by substituting the constituents of the cycle,

In particular, the said compound could be designed by modifying the size of the cycle.

According to a sixth aspect of the present invention there is provided a method to screen at least one compound for the ability to inhibit CYP121 activity, comprising the steps:
a) synthesising said at lest one compound.
b) incubating CYP121, an exogenous electron transport chain, NADPH, a substrate of CYP121 and said at least one compound together in predetermined reaction conditions,
c) collecting the products of step b),
d) analysing the products of step c),
e) comparing the products of step c) to a negative control experiment in which said at least one compound is omitted from step b),
f) determining the inhibitory effects of said at least one compound upon CYP121 based upon the results of step e).

In particular the substrate is cyclo(L-Tyr-L-Tyr) (SEQ ID NO:4).

In particular the at least one compound is designed according to the design method described above.

In particular the method is used to screen a library of compounds.

In particular the analysis of step d) is performed by reverse phase HPLC.

In particular the method comprises a further step:
e') of comparing the products of step c) to a positive control experiment in which an azole is present in step b).

In particular the azole is selected from the group comprising: clotrimazole, ketoconazole, miconazole, fluconazole, econazole.

The above list of suitable azoles is not considered to be exhaustive by the inventors.

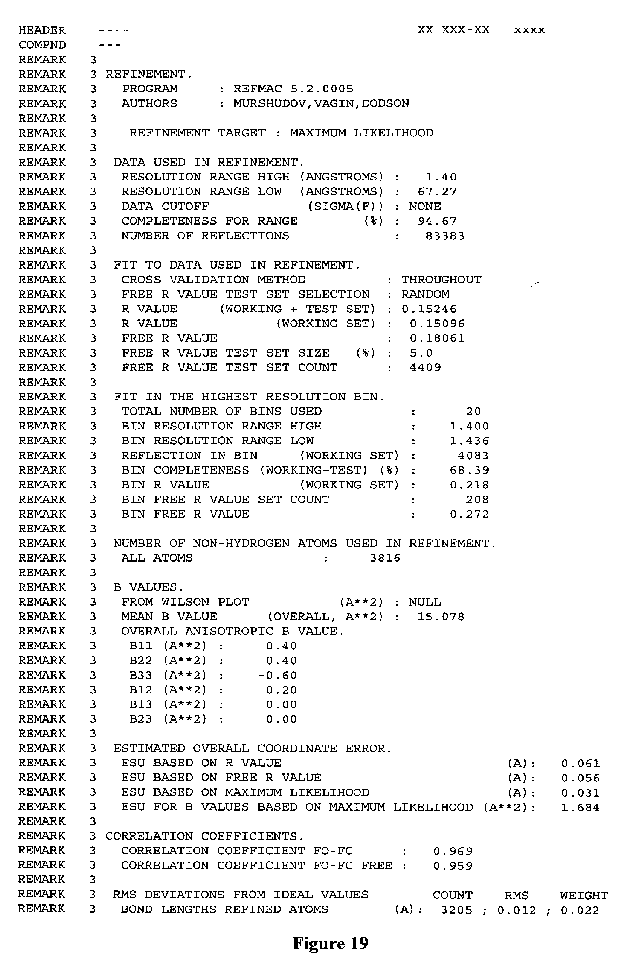

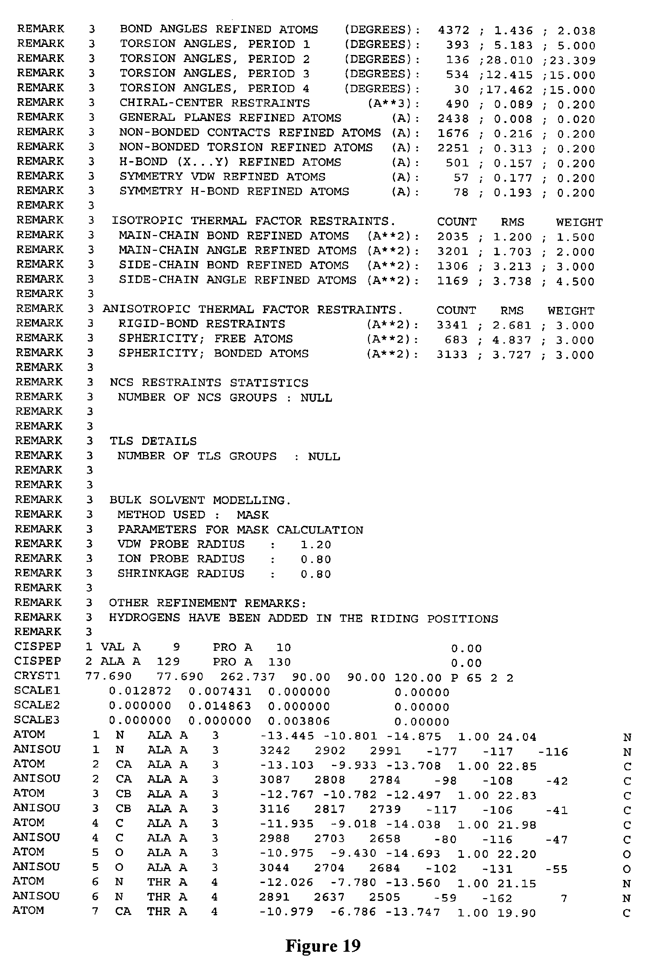

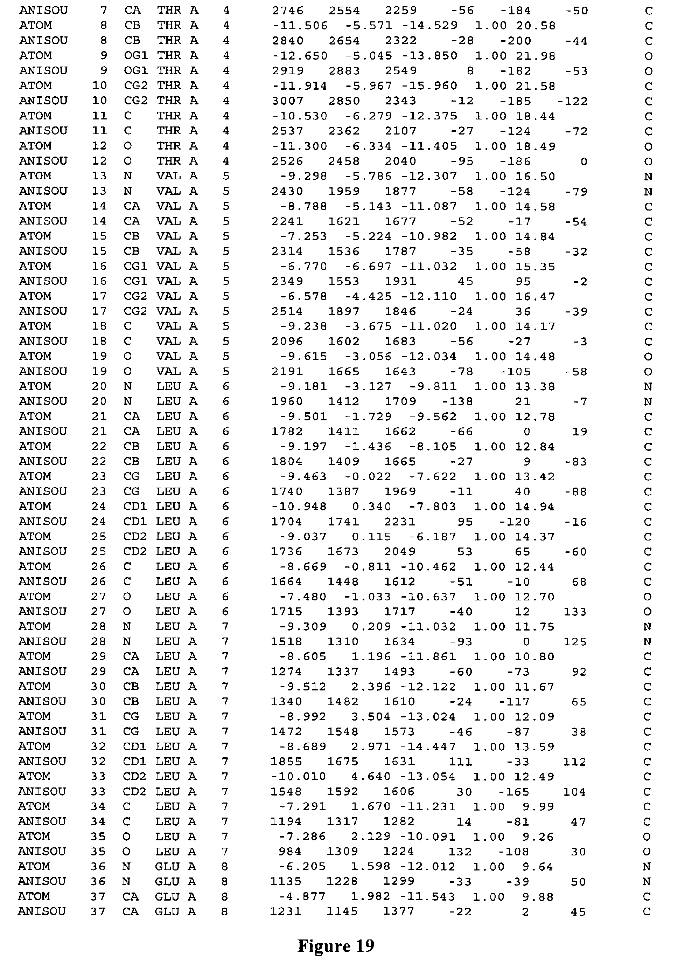

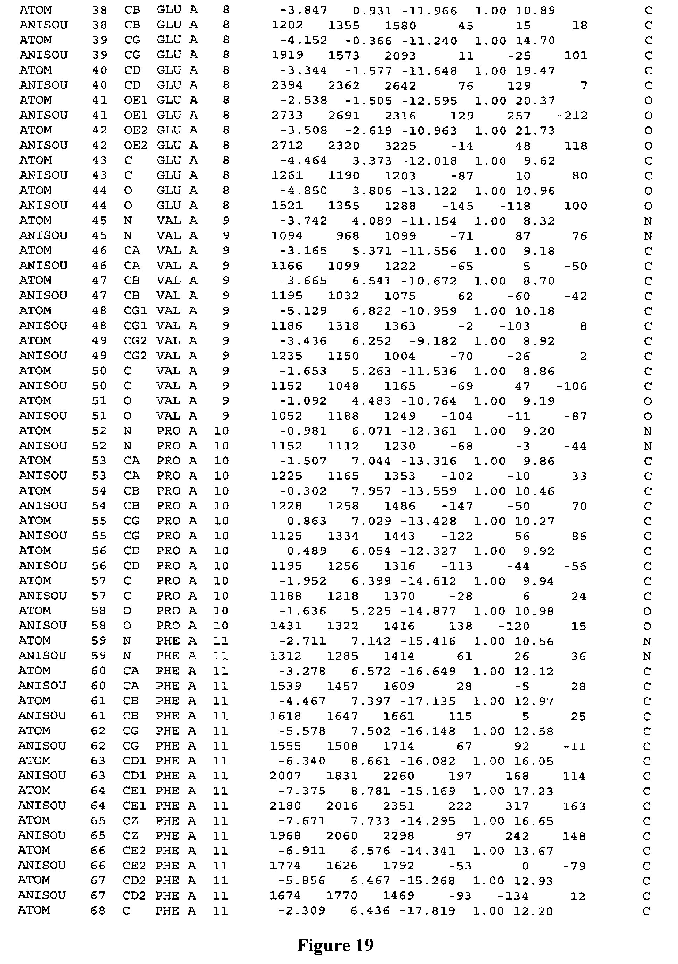

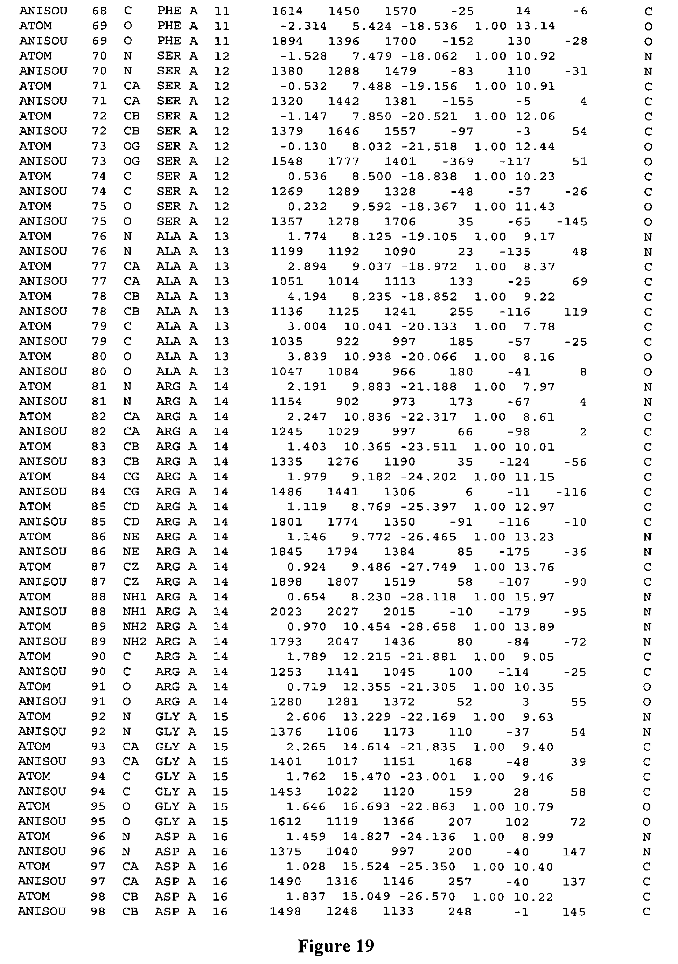

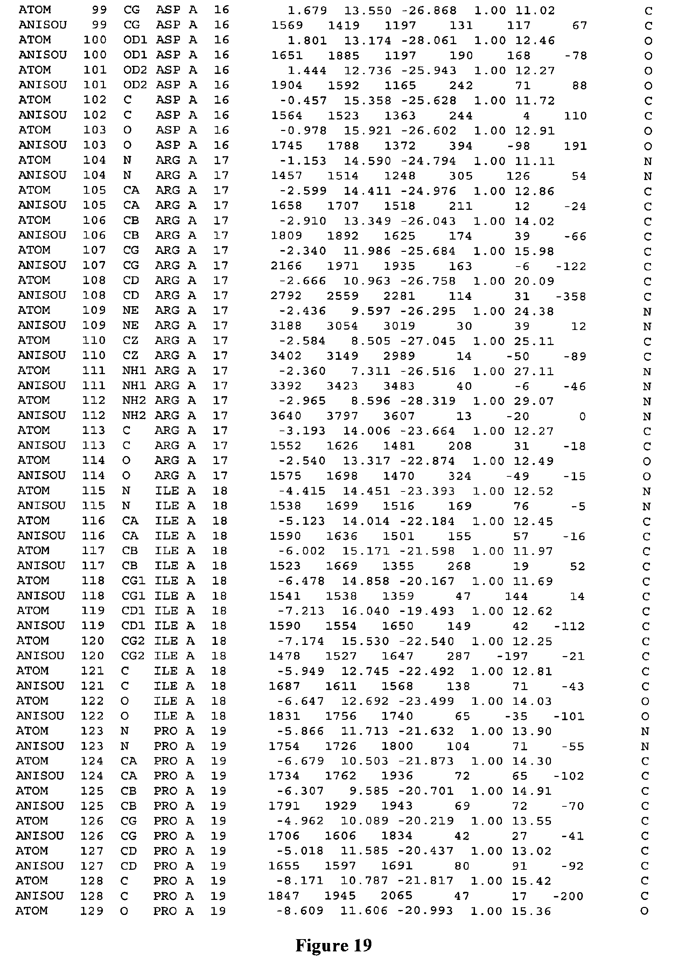

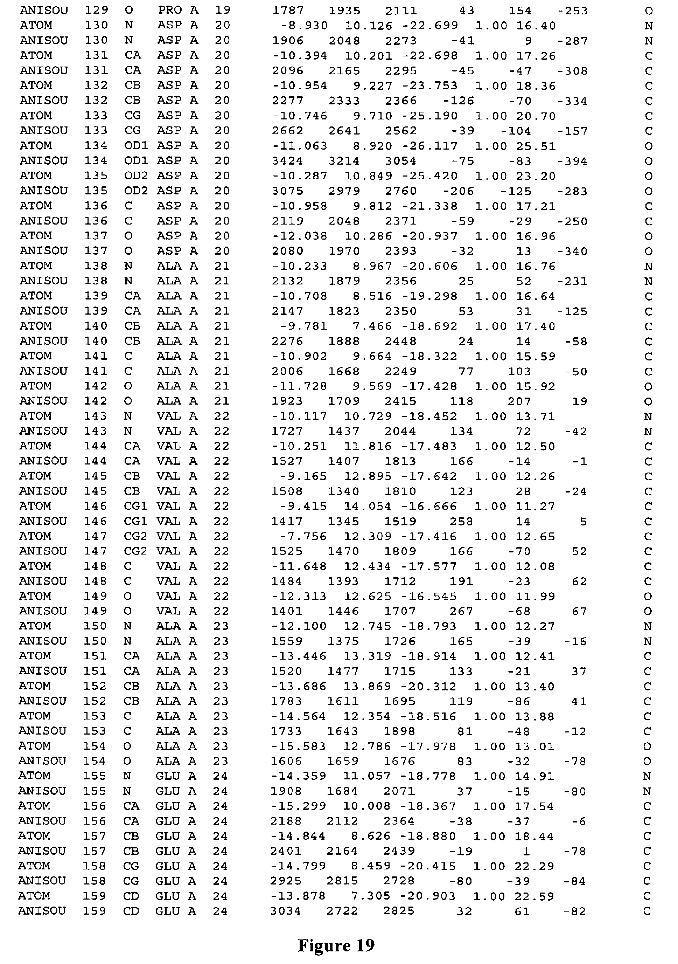

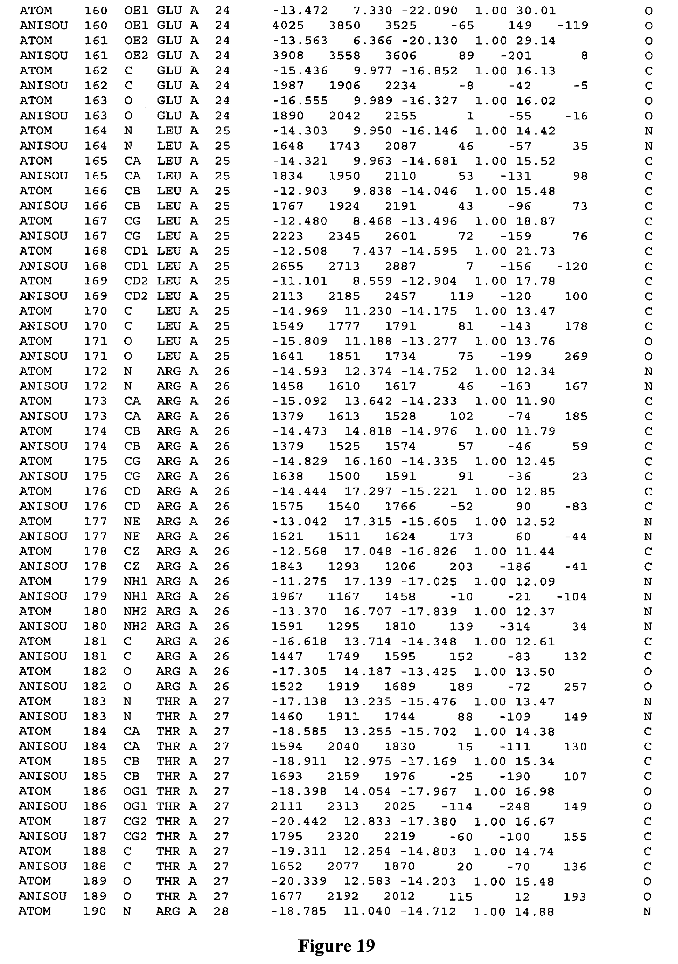

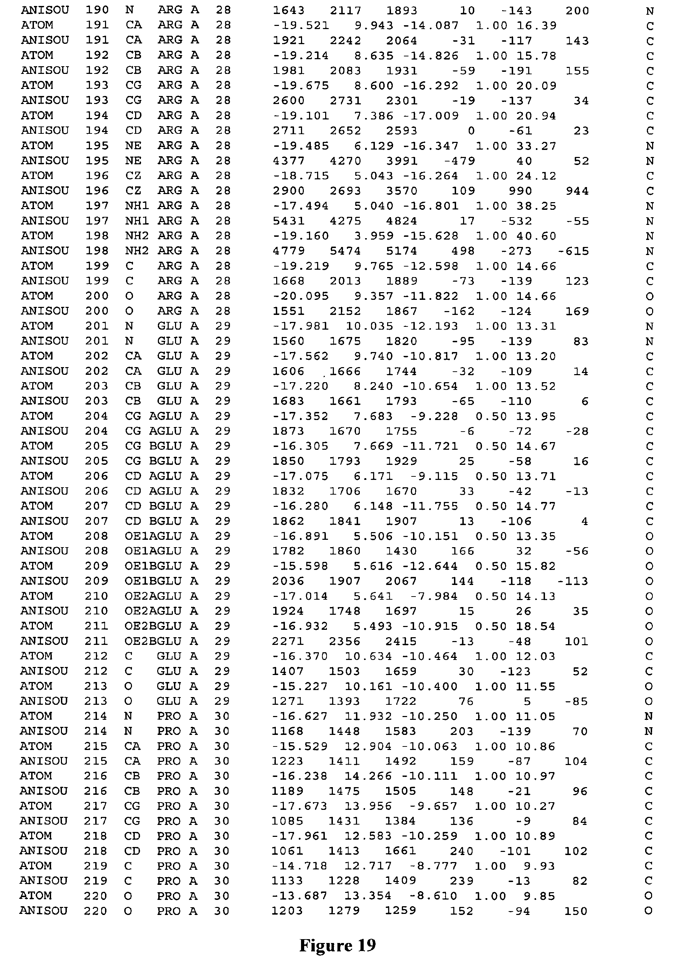

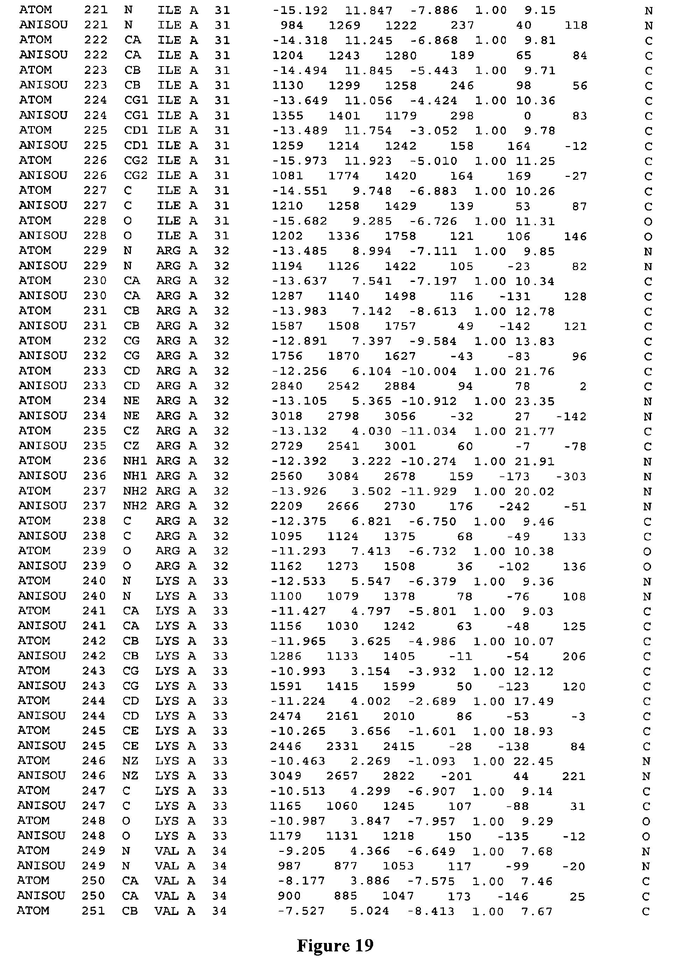

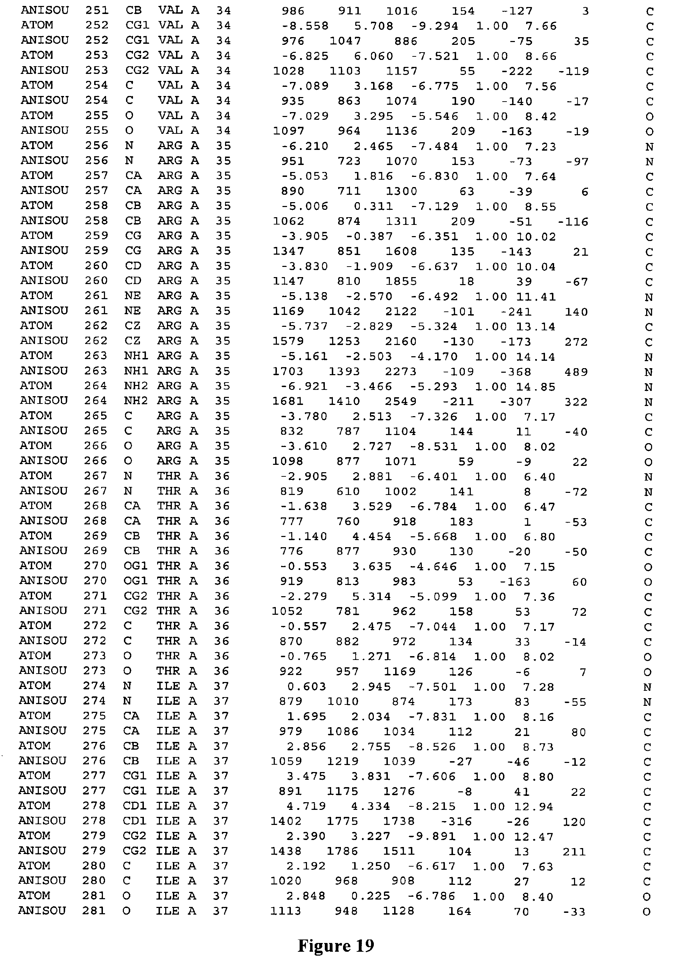

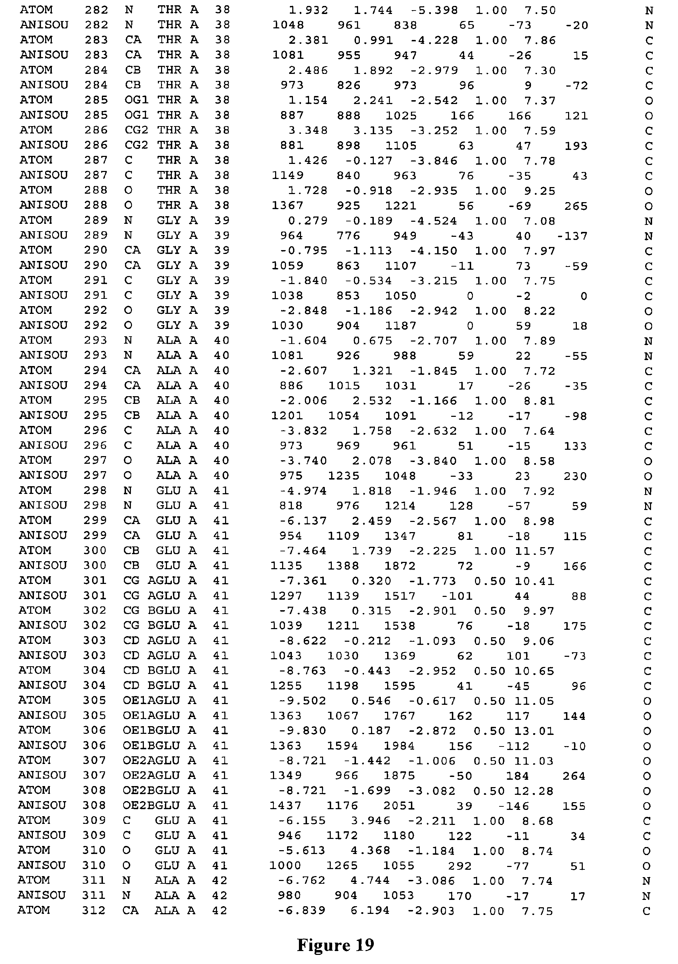

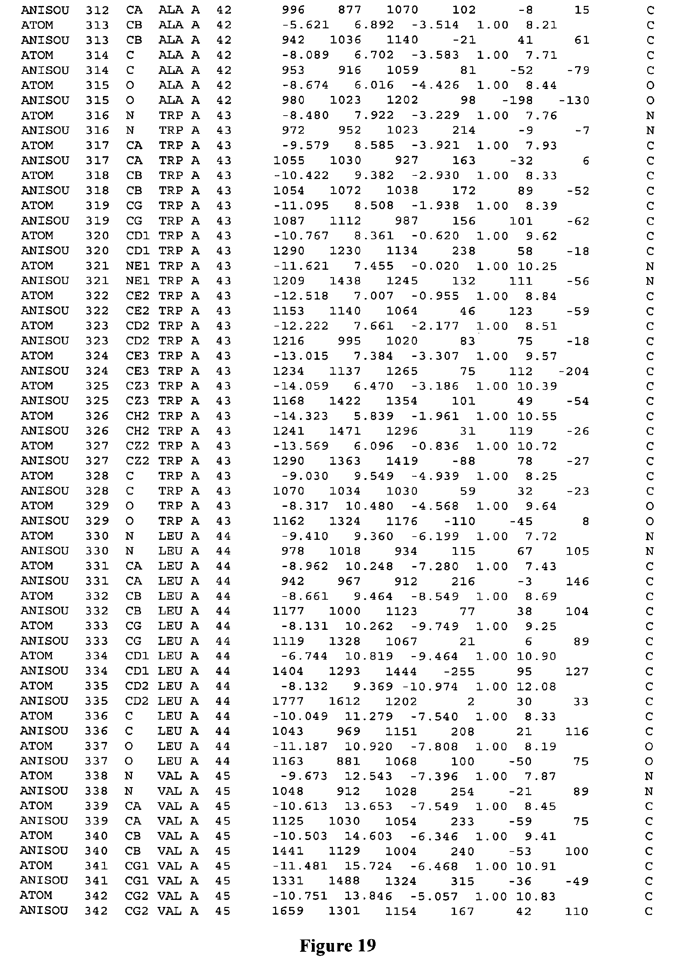

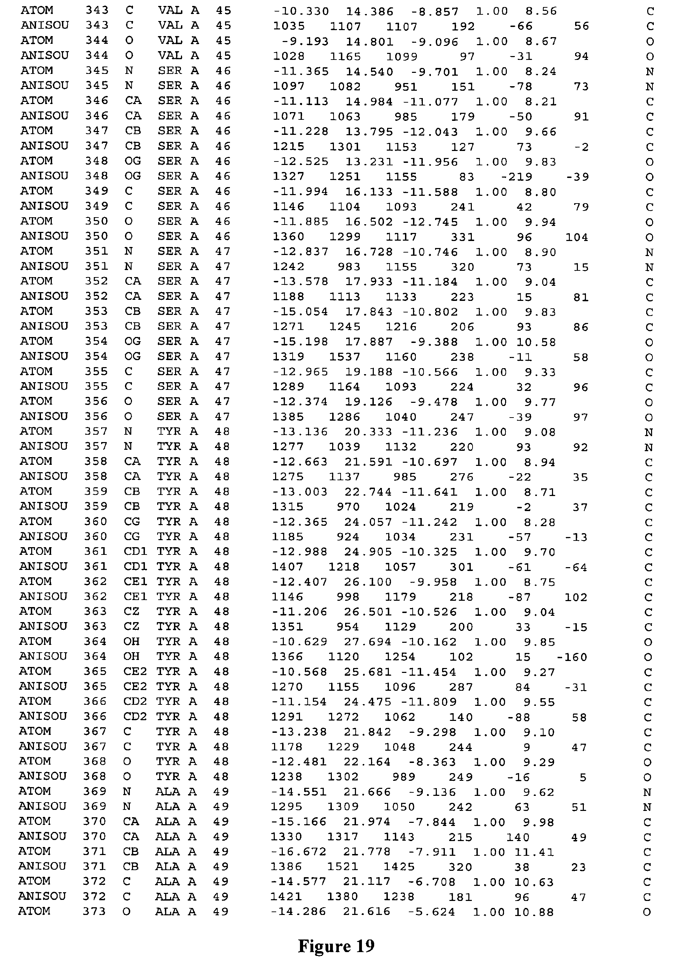

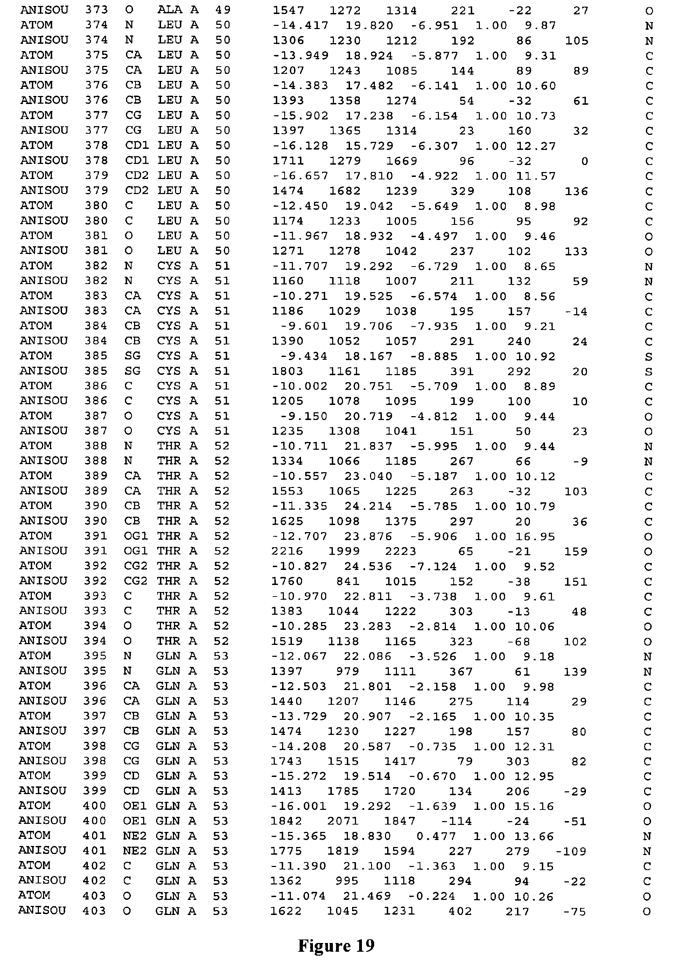

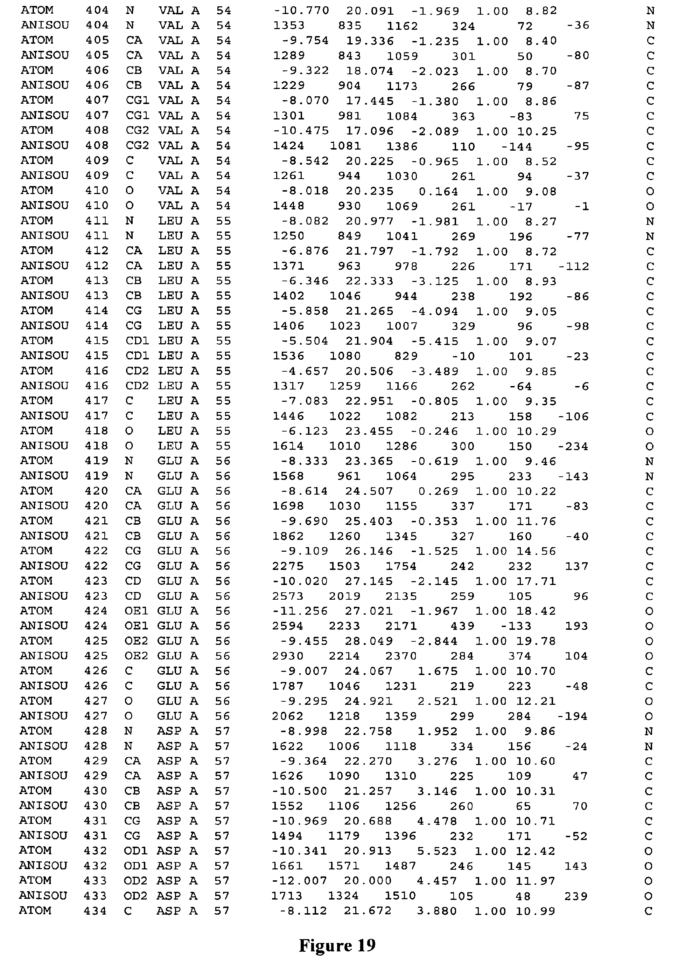

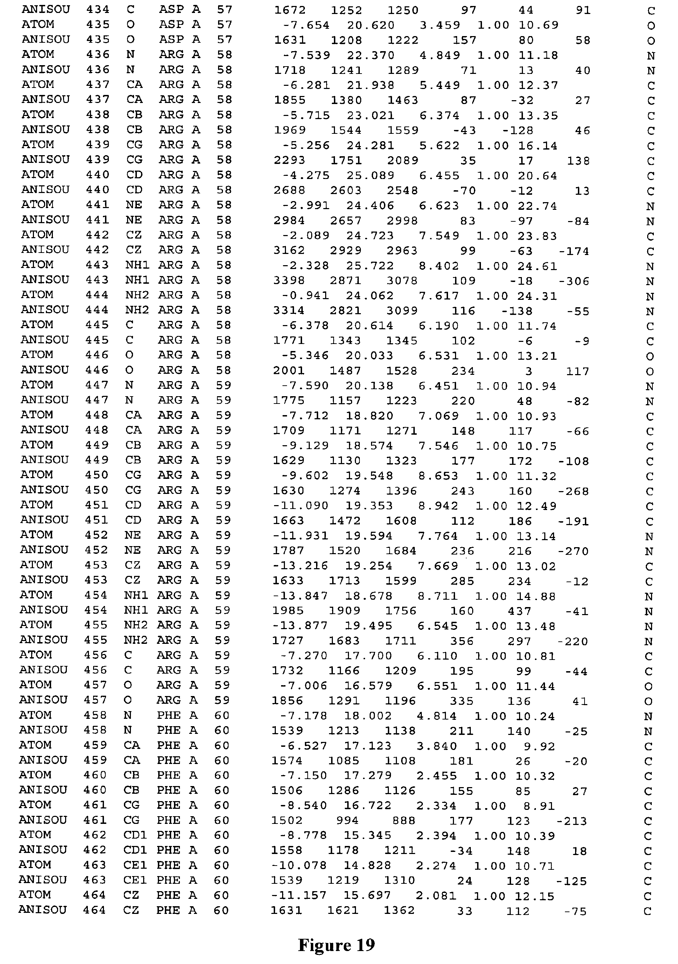

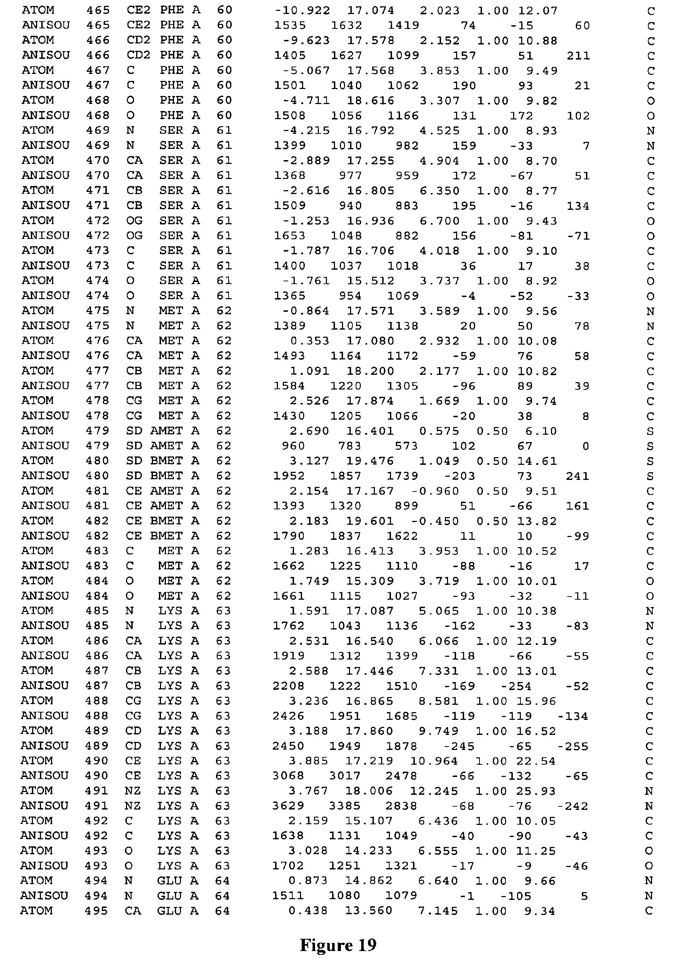

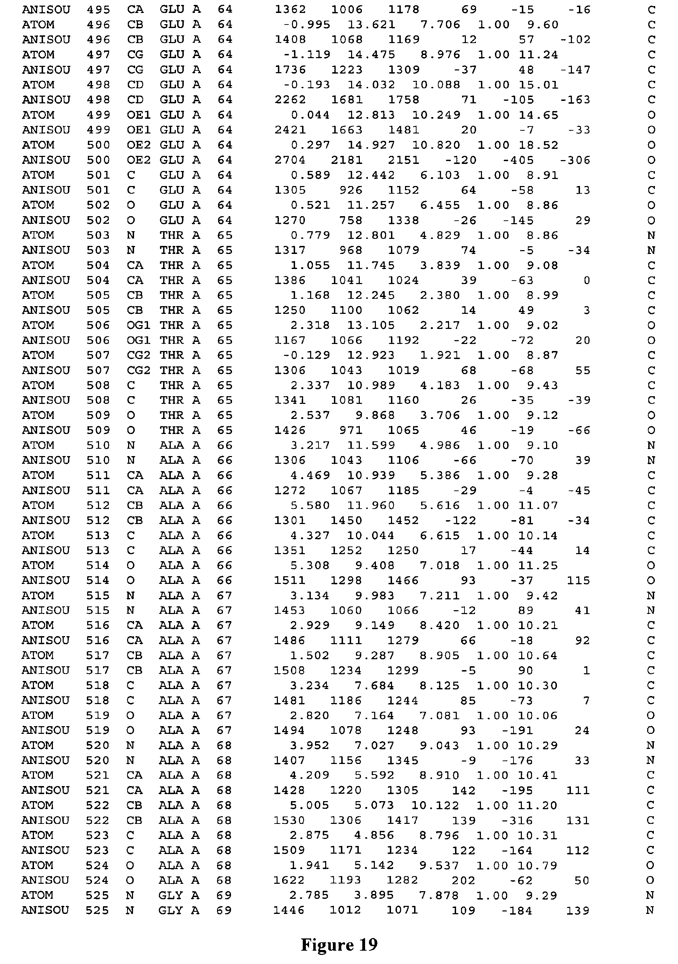

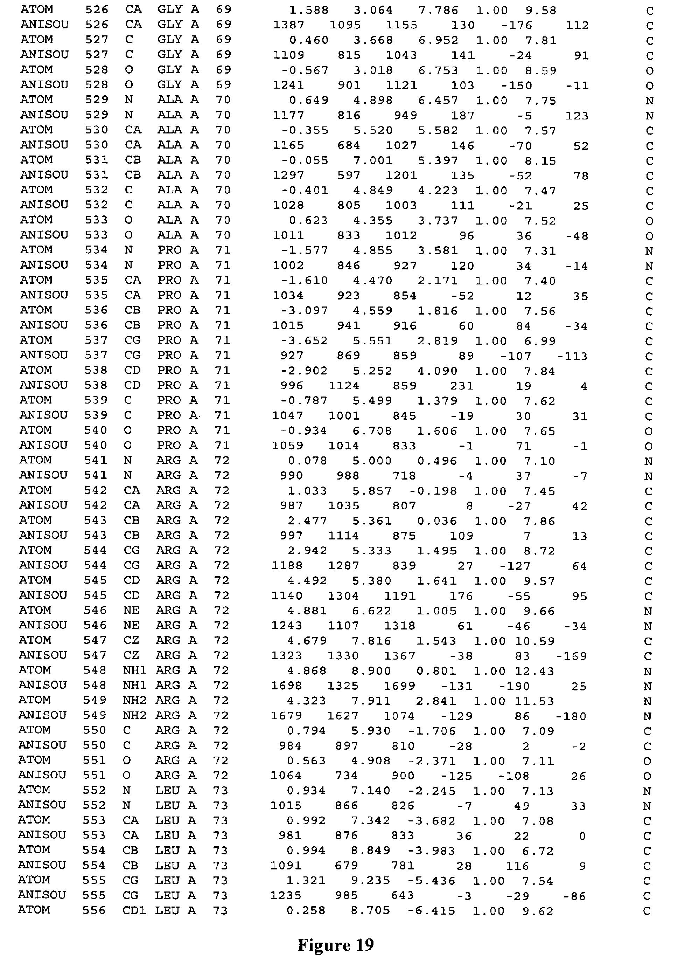

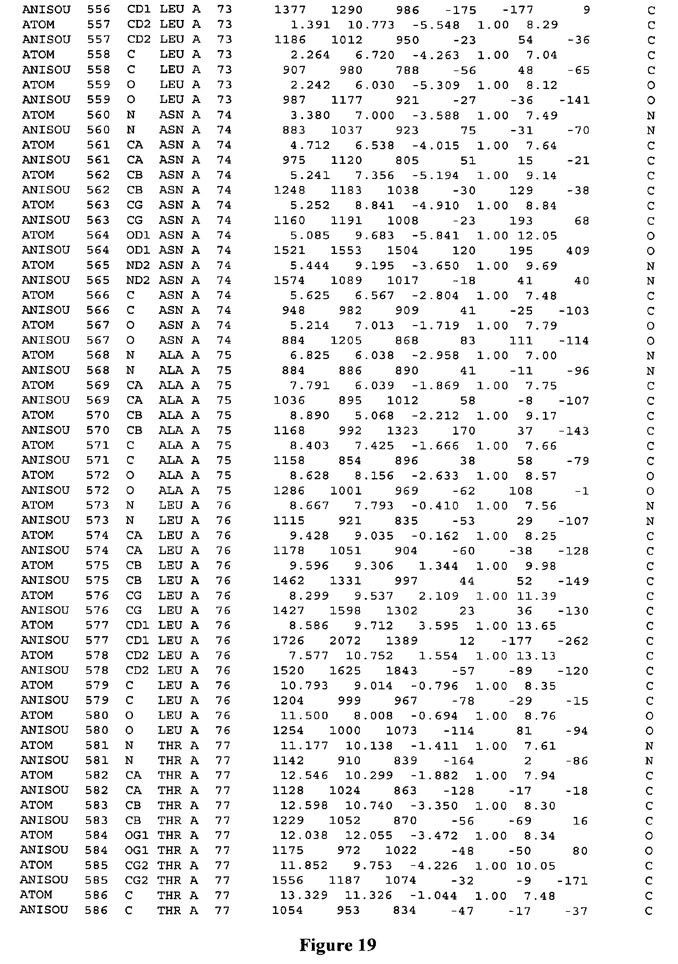

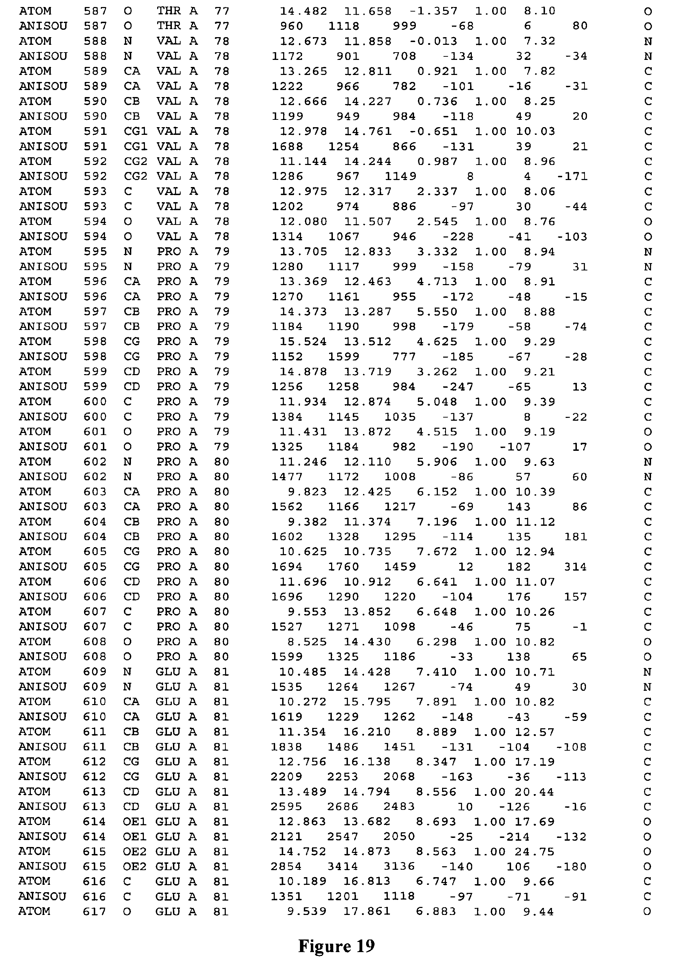

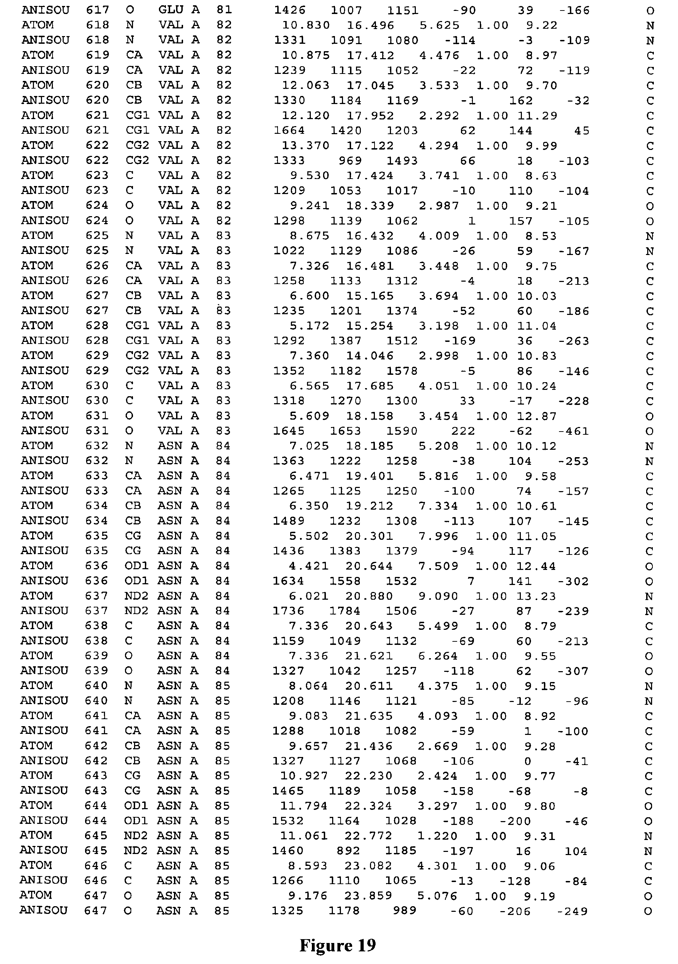

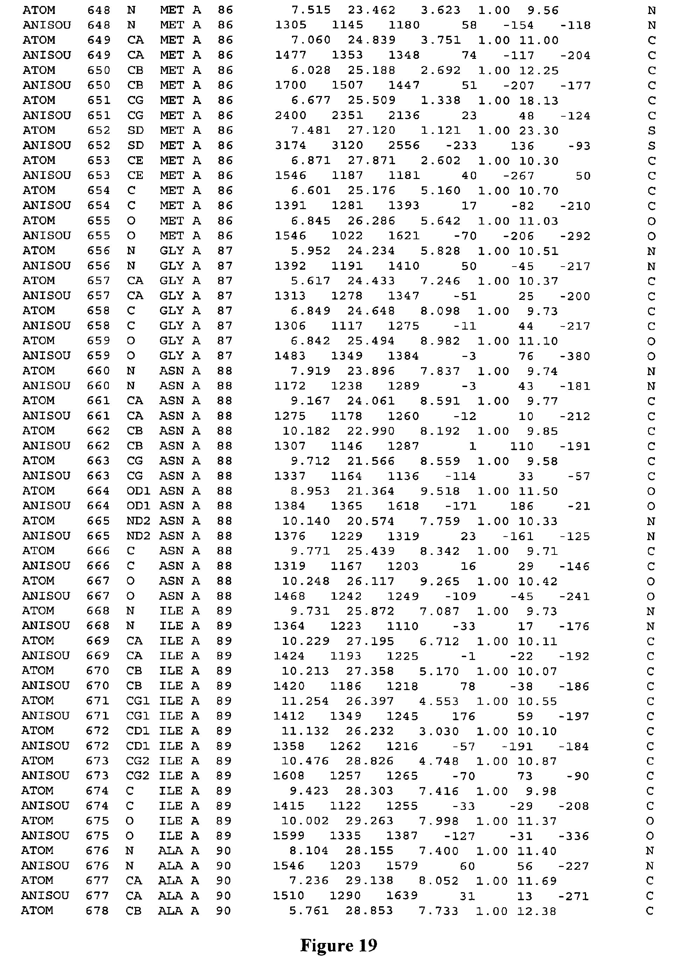

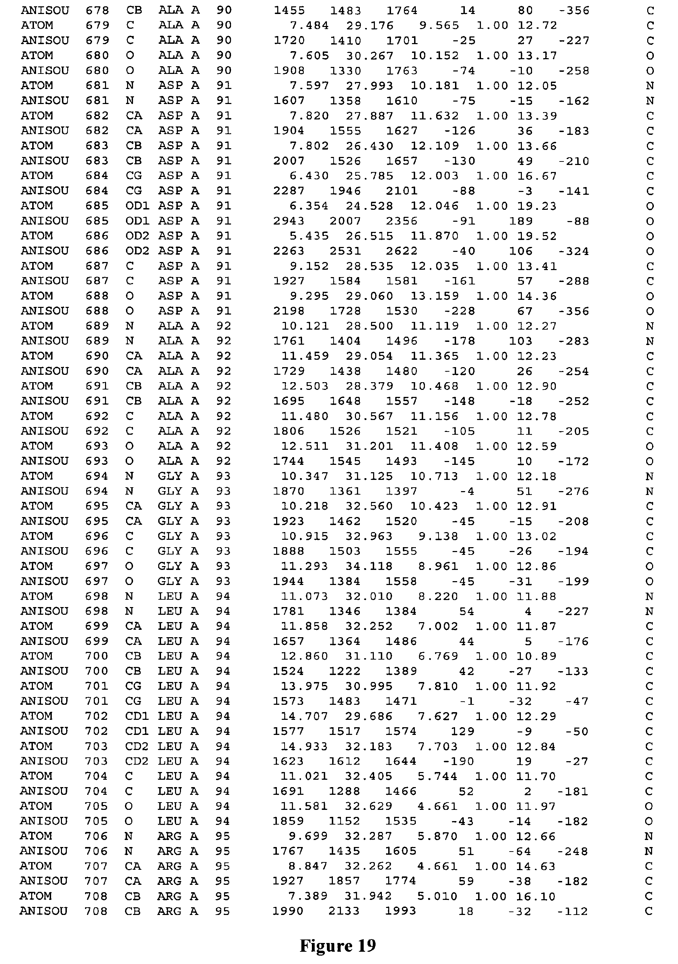

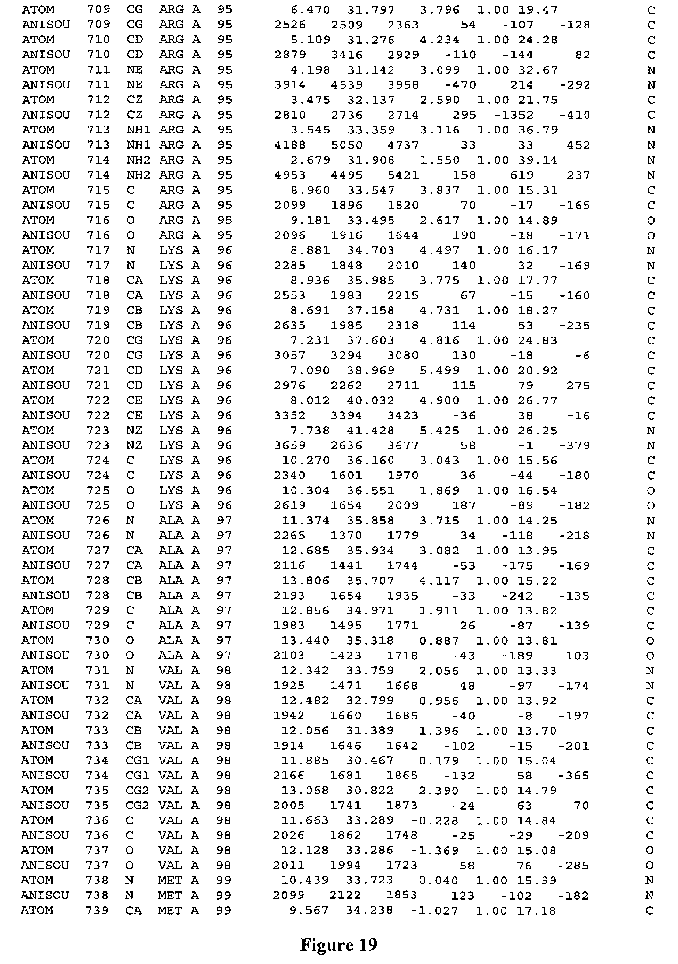

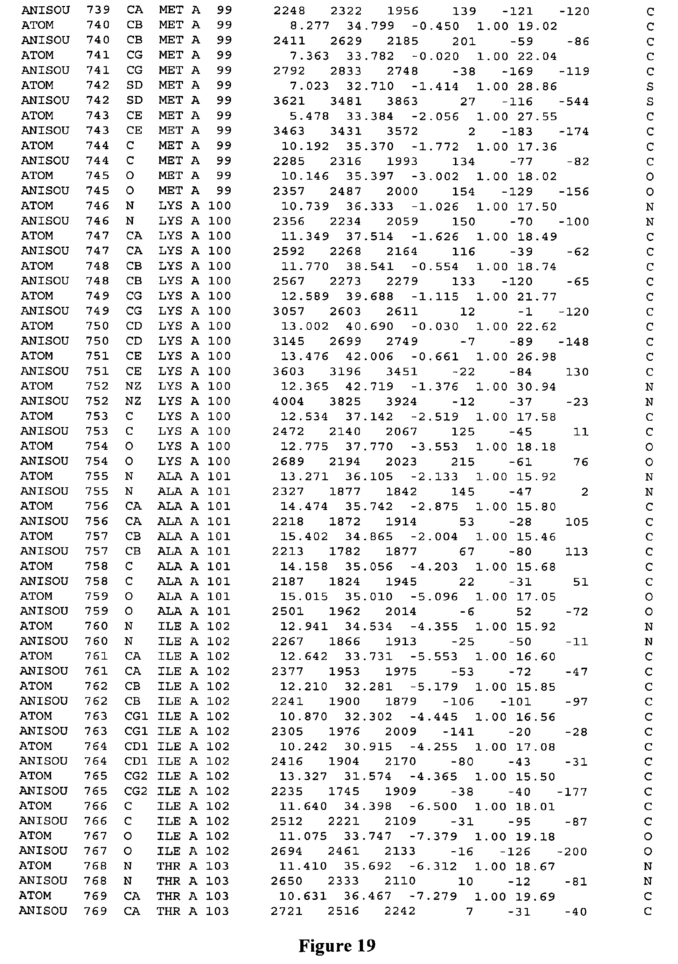

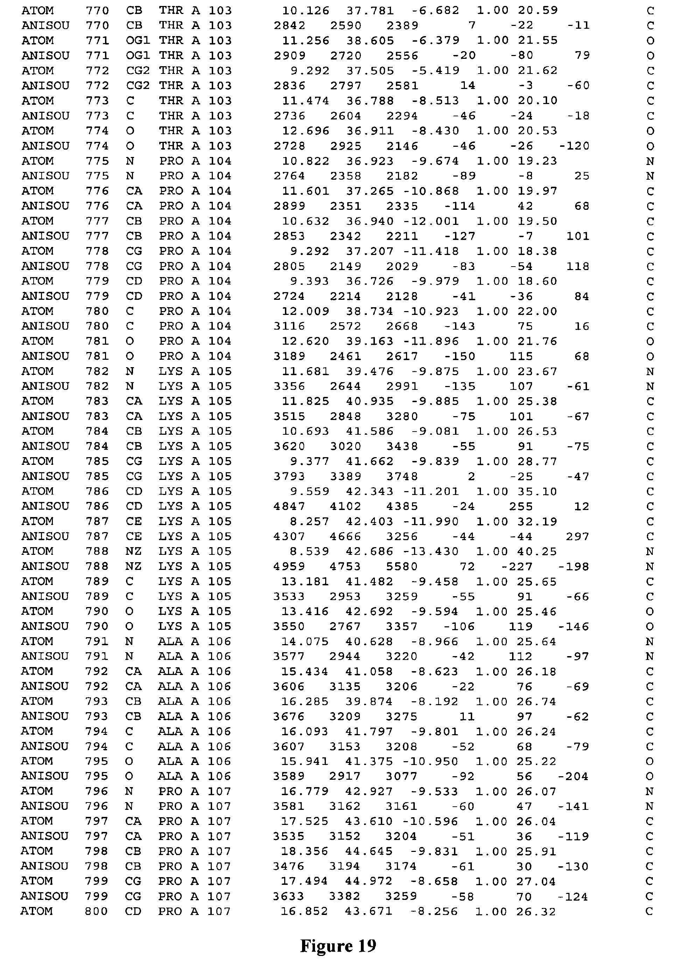

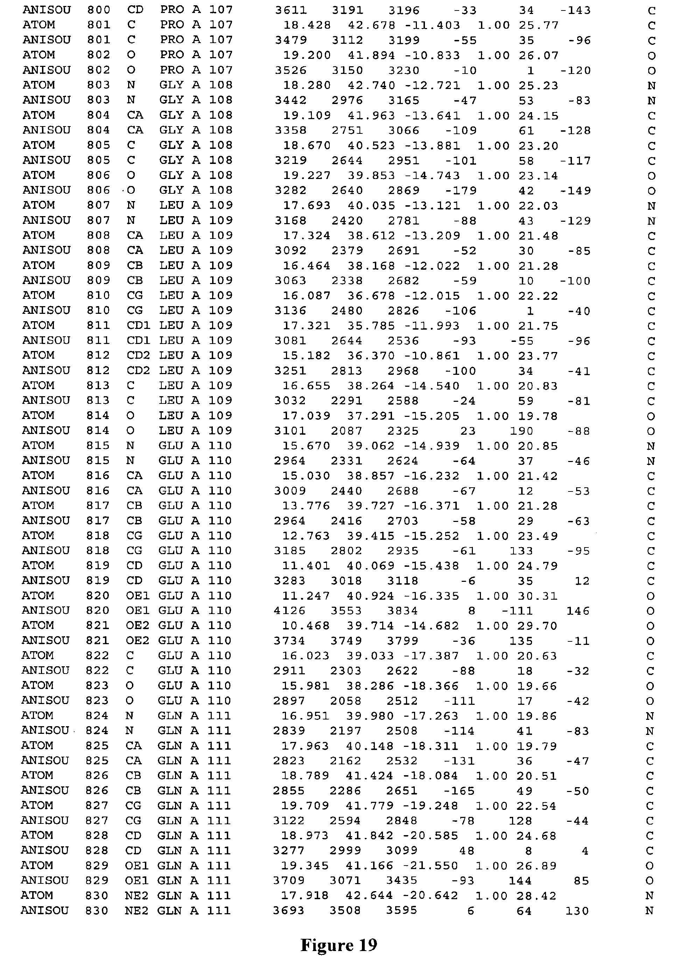

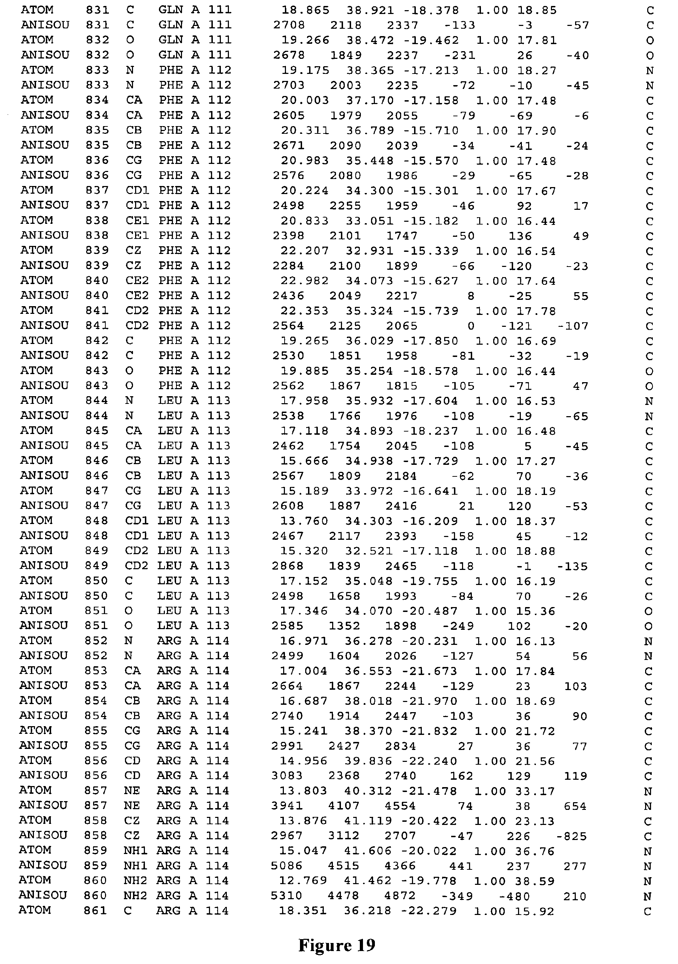

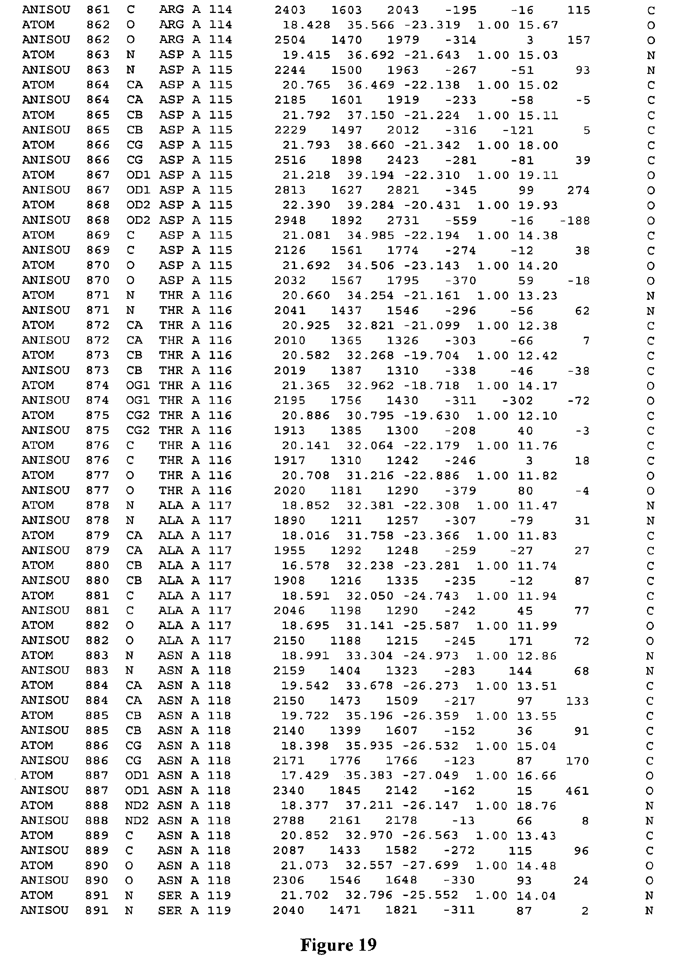

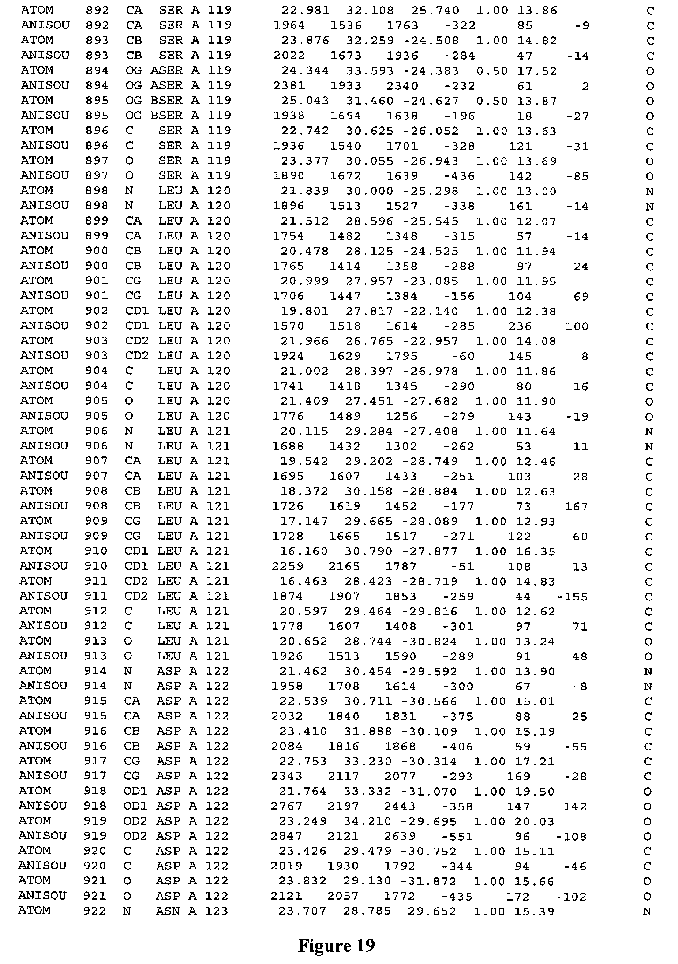

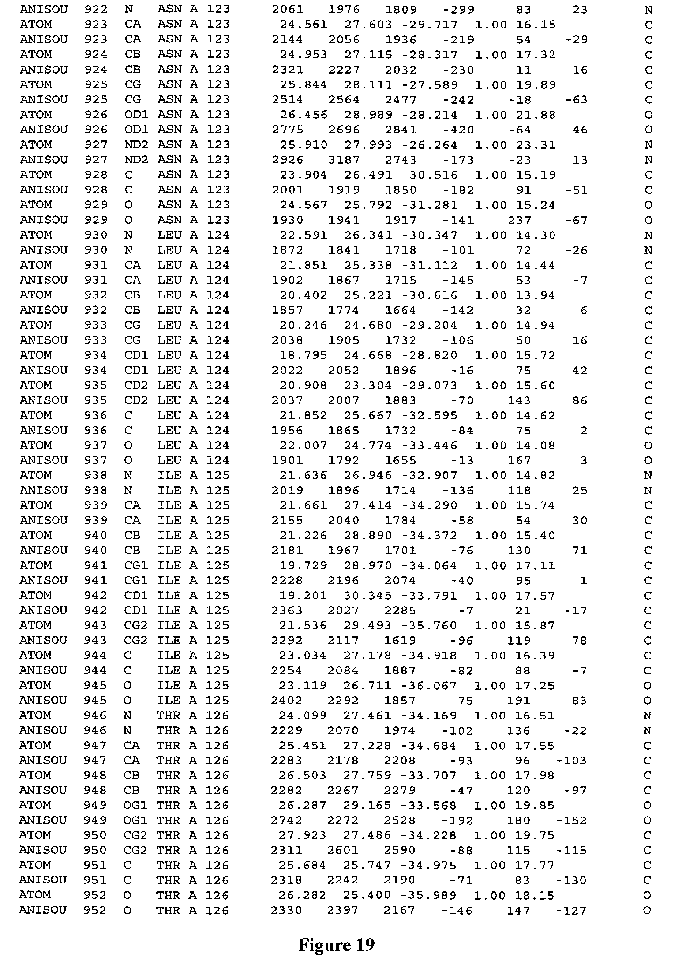

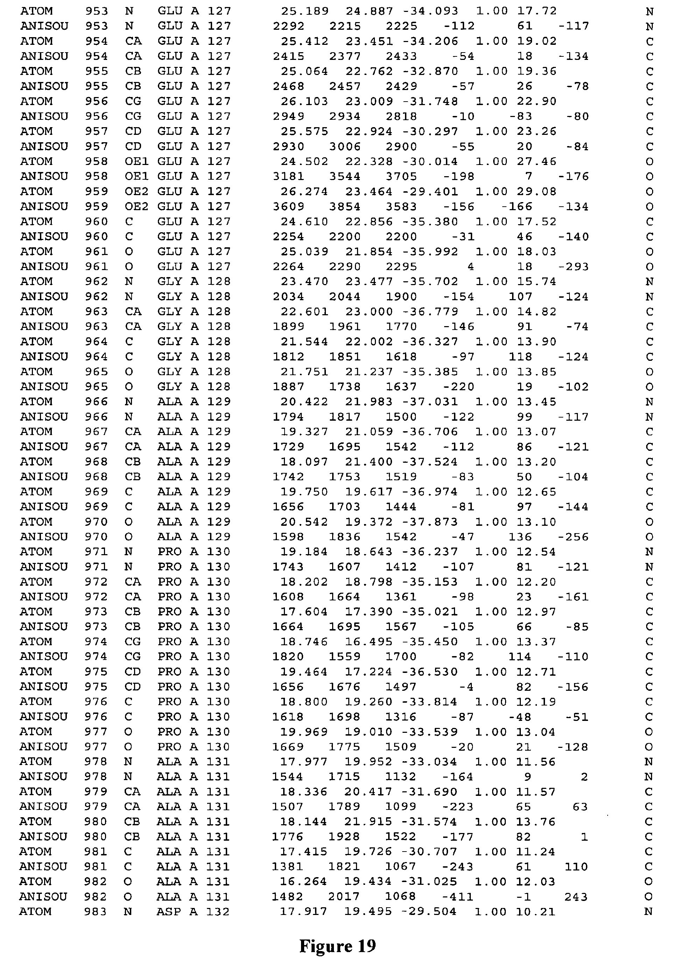

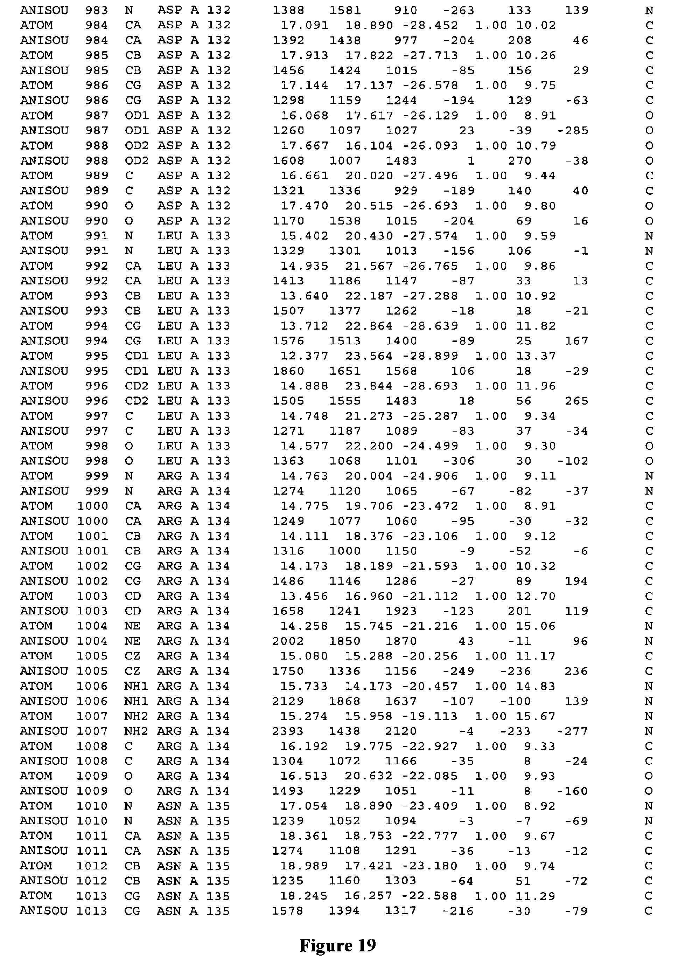

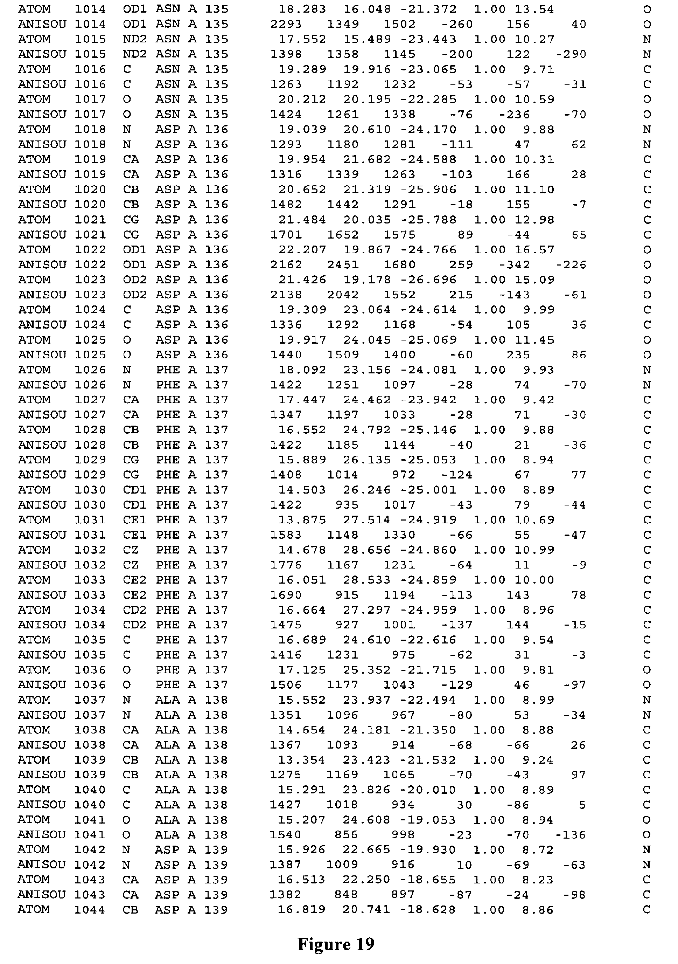

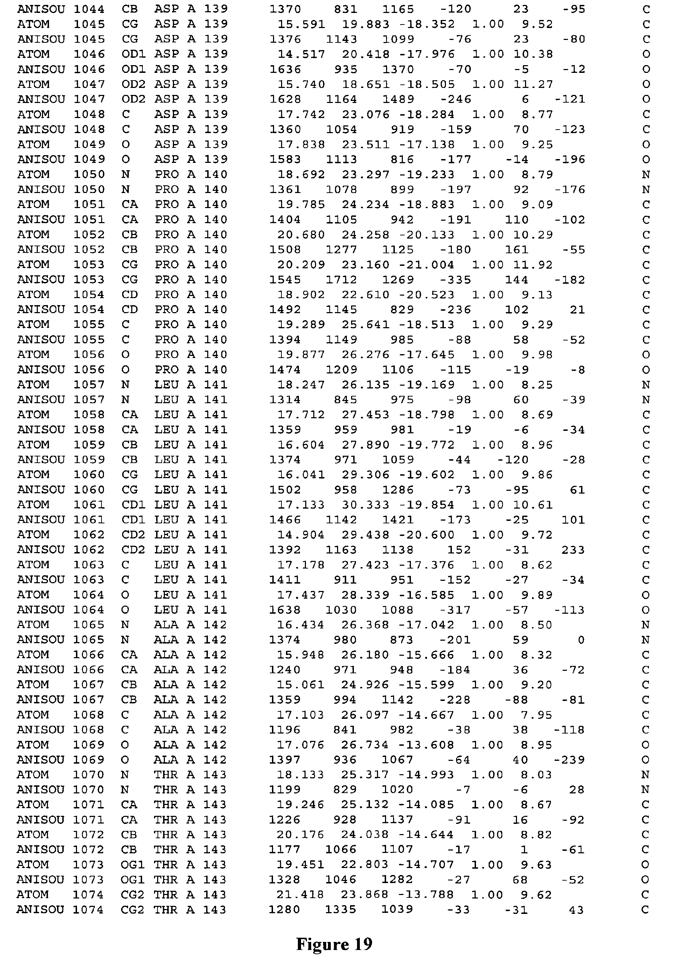

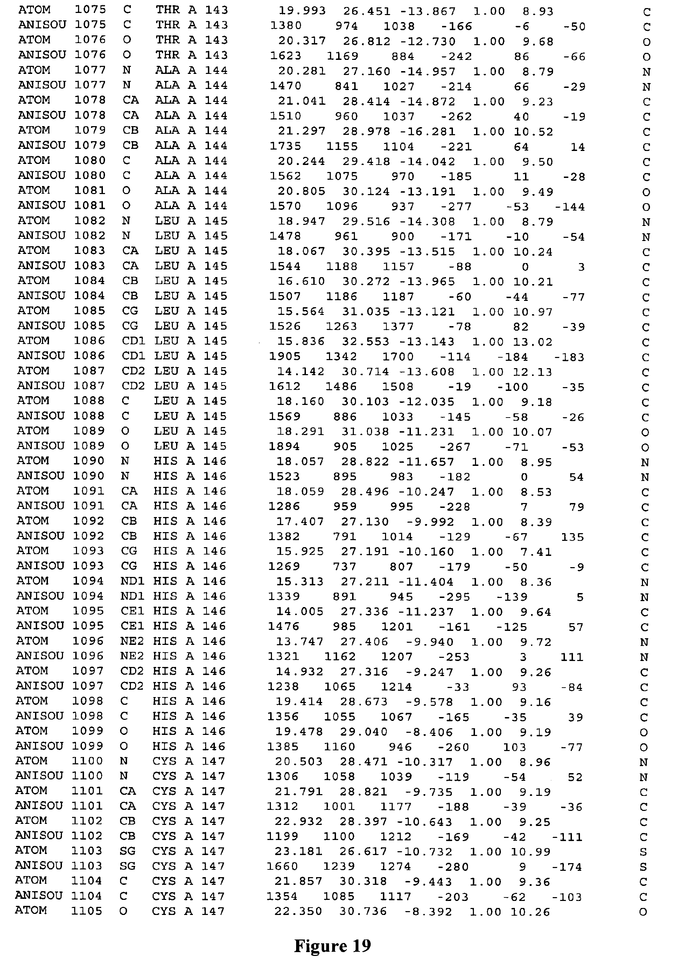

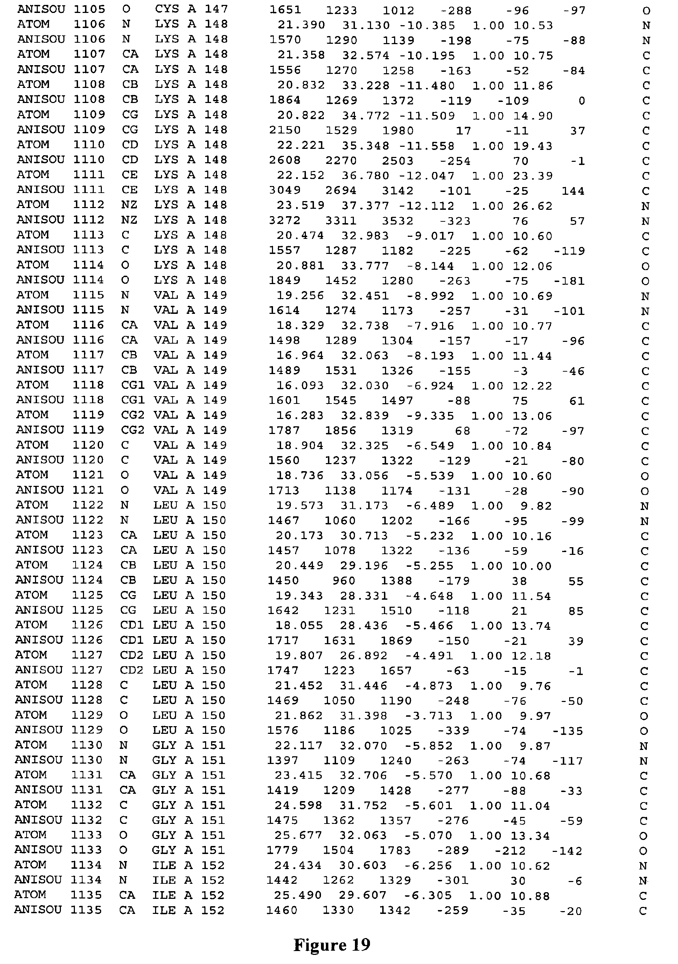

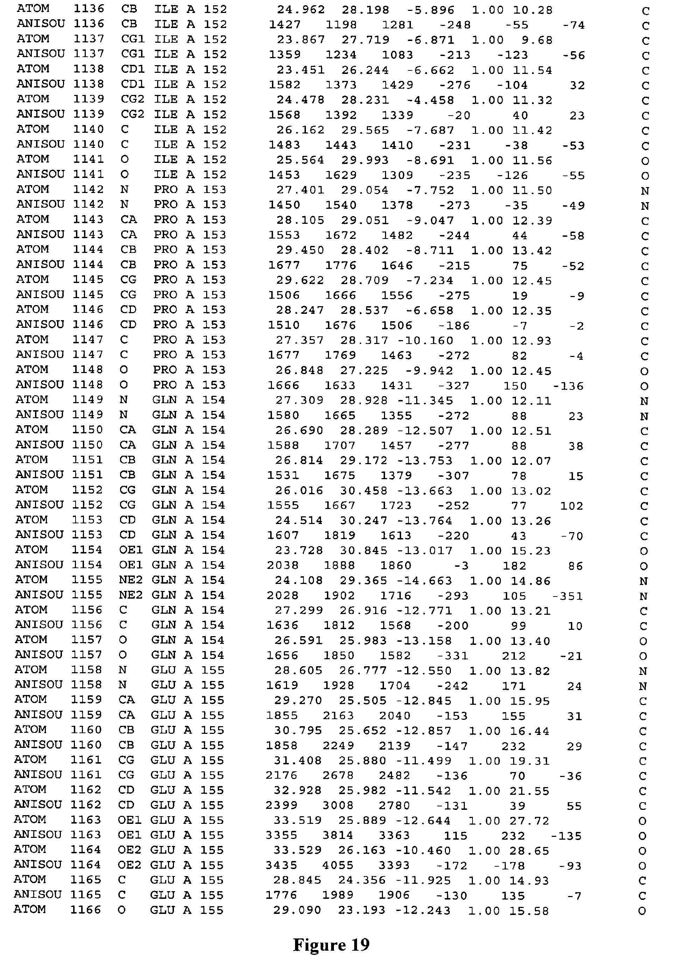

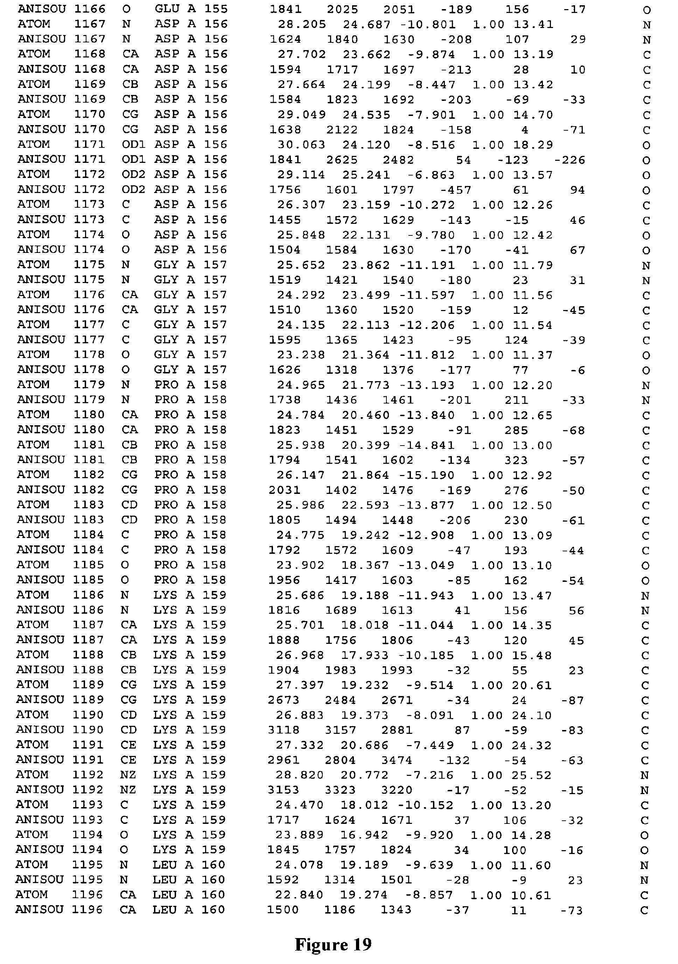

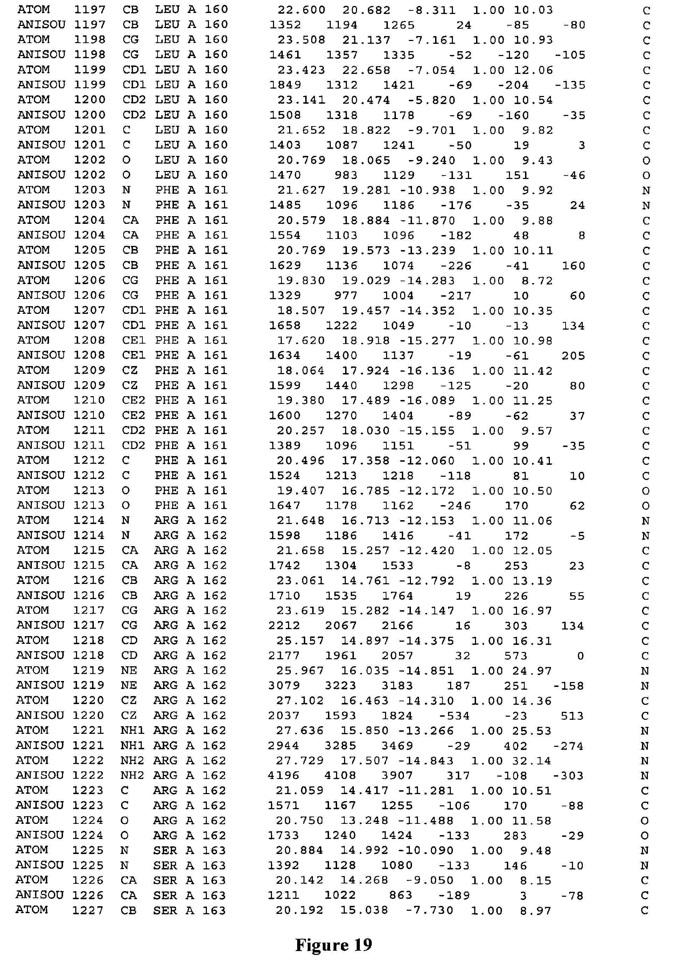

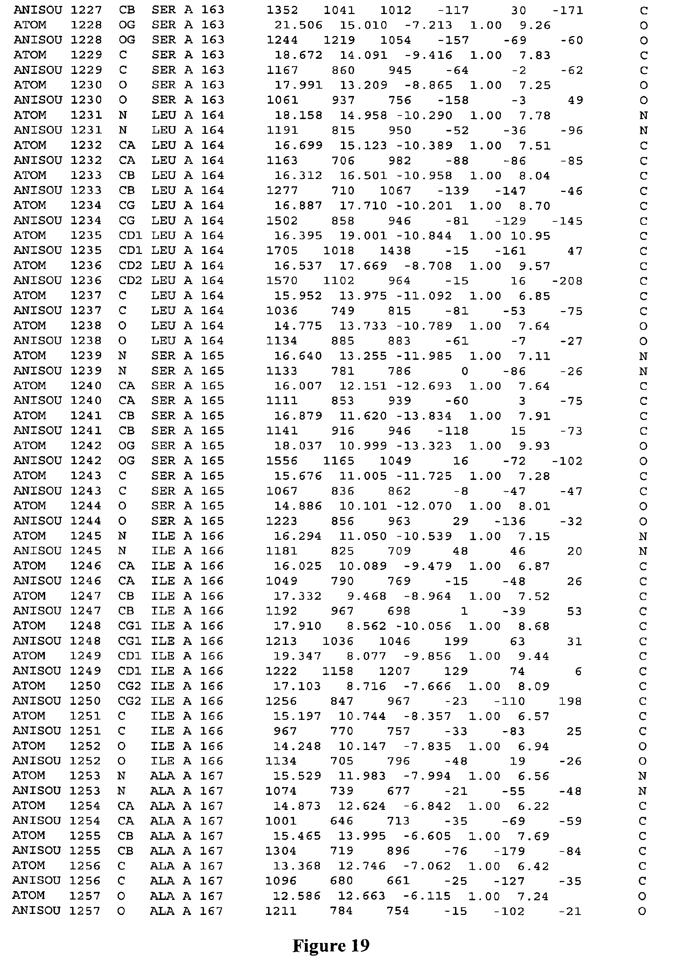

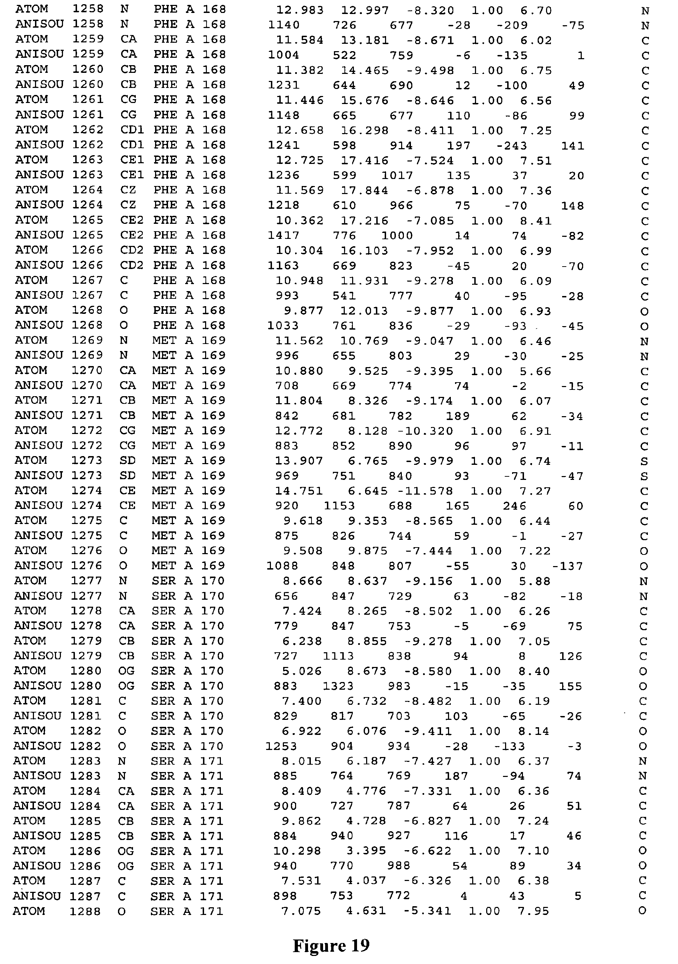

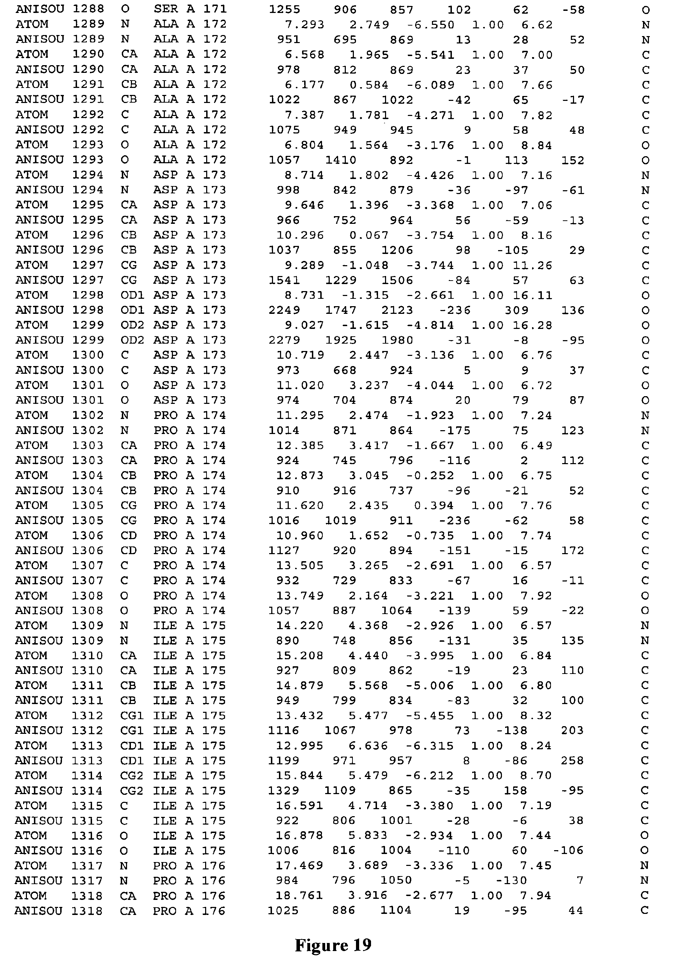

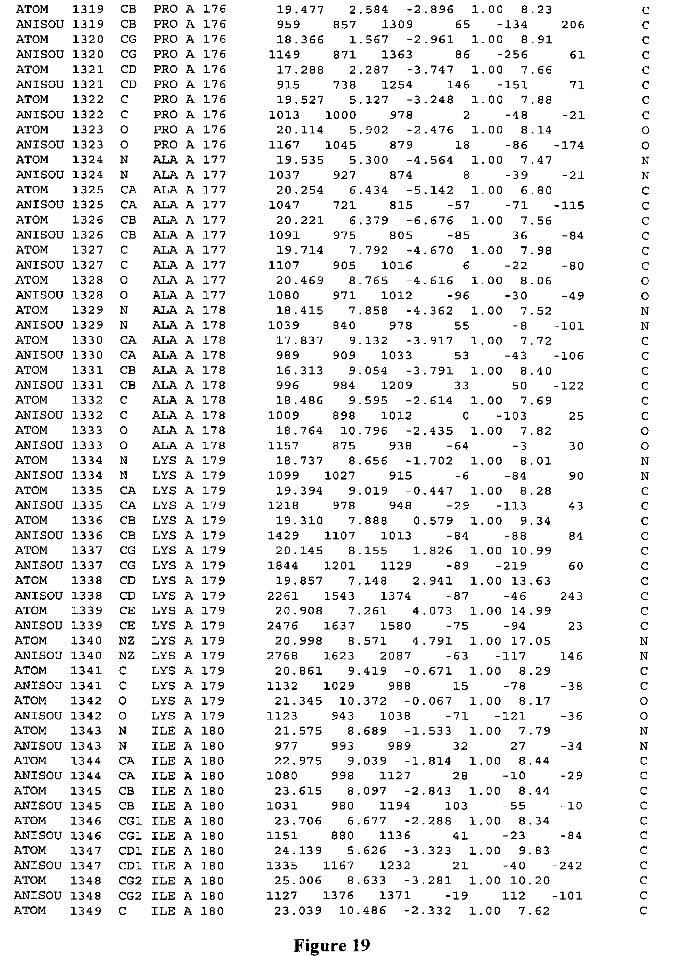

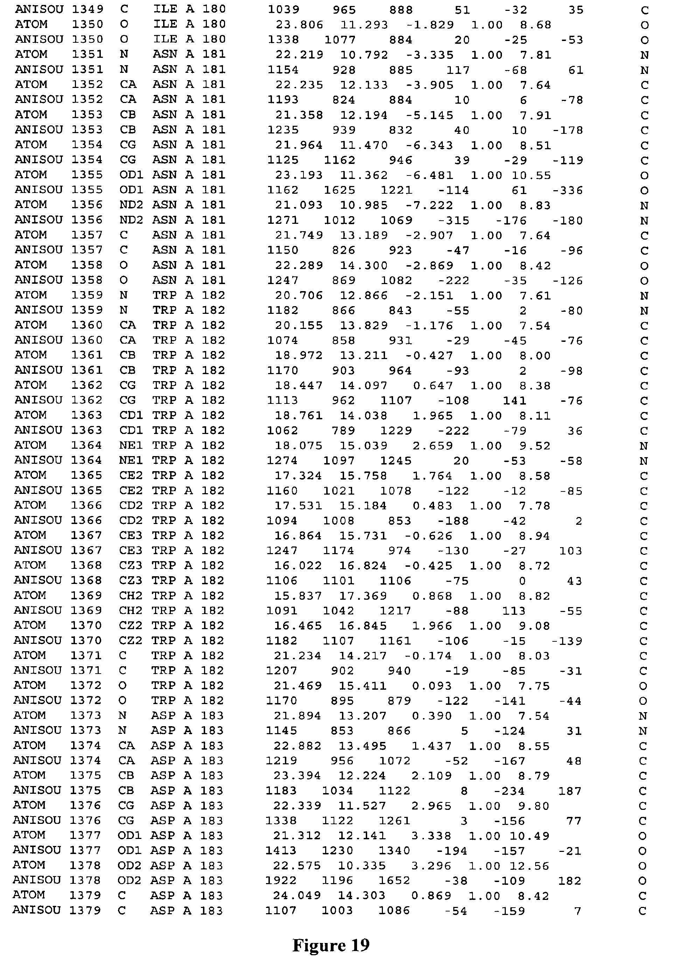

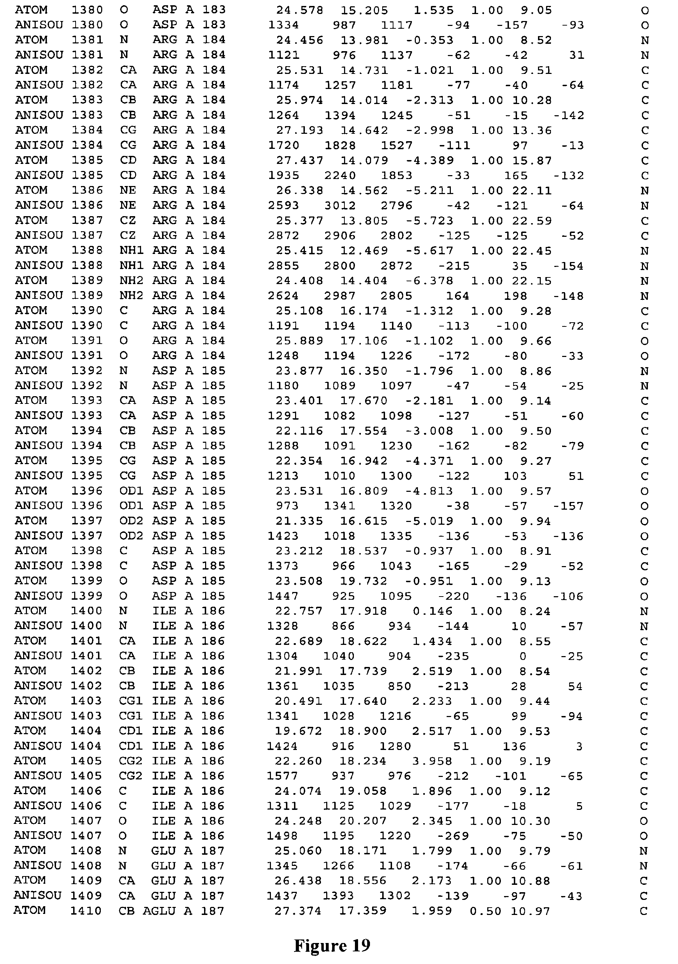

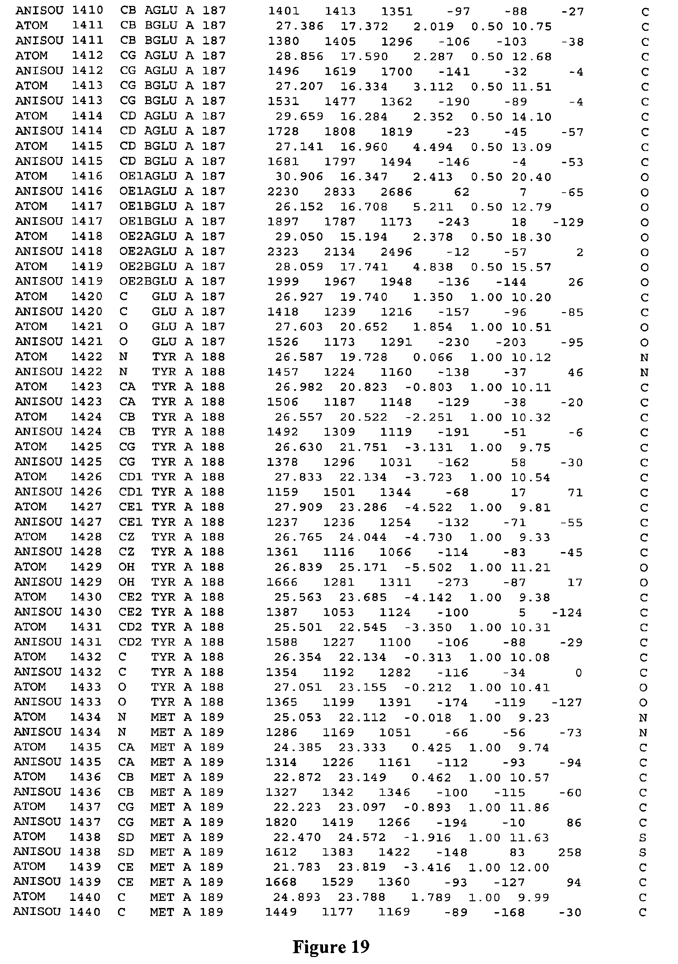

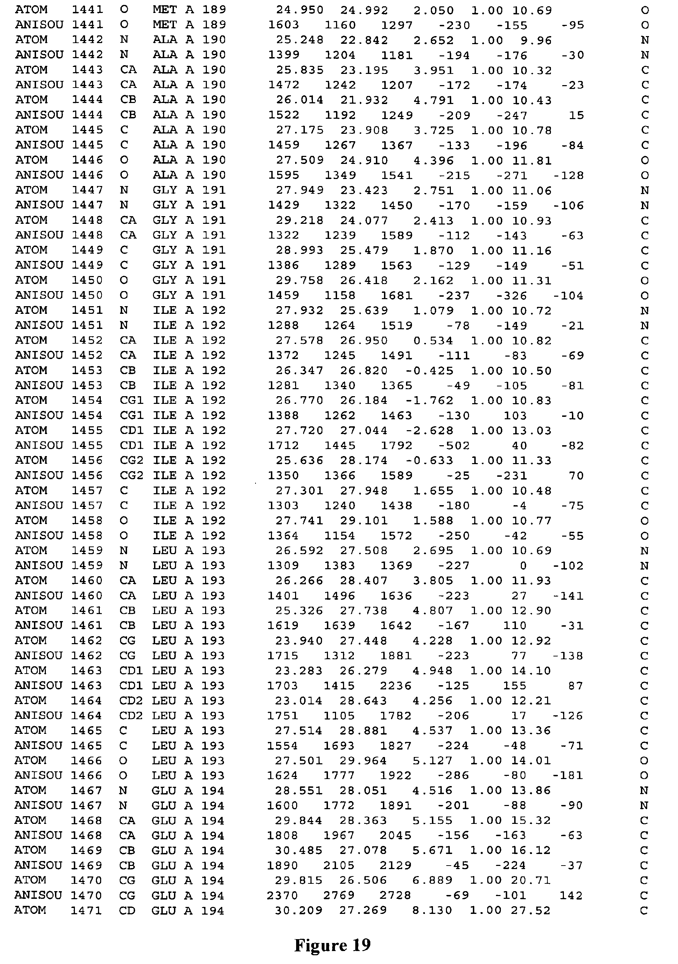

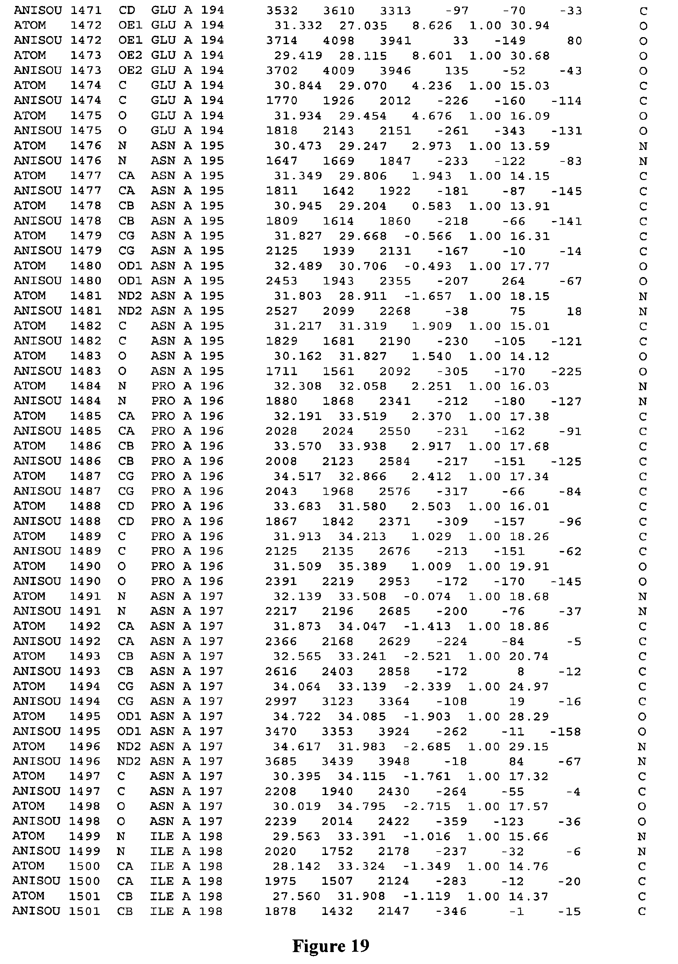

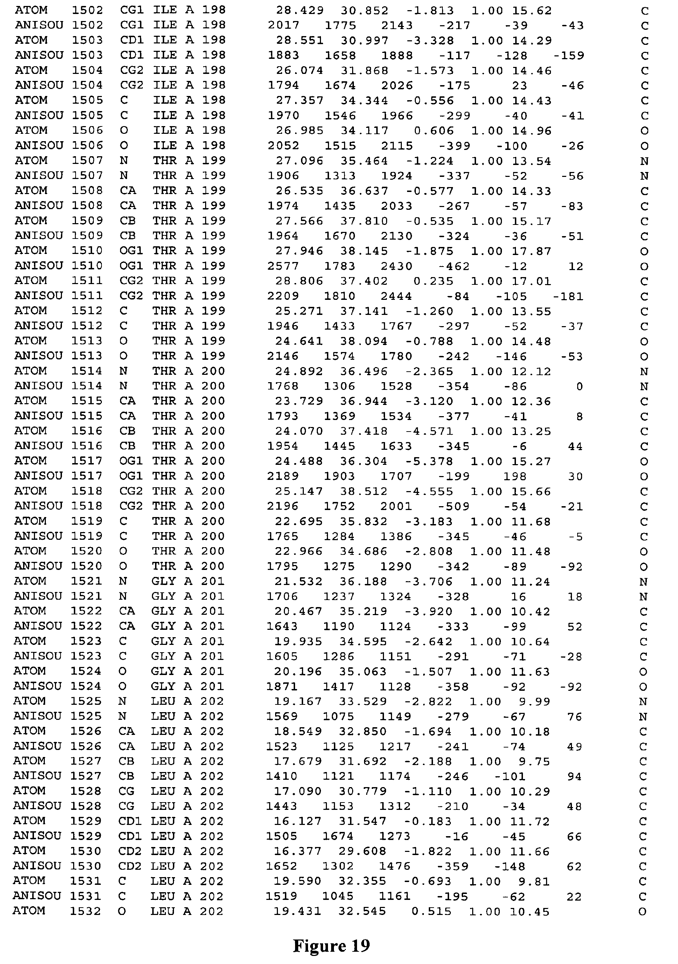

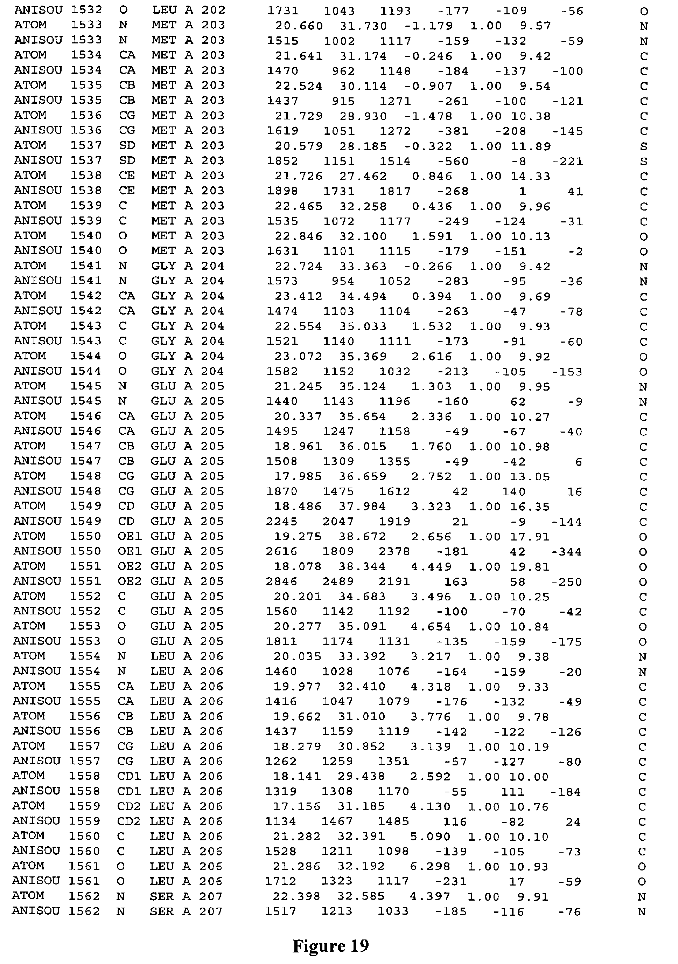

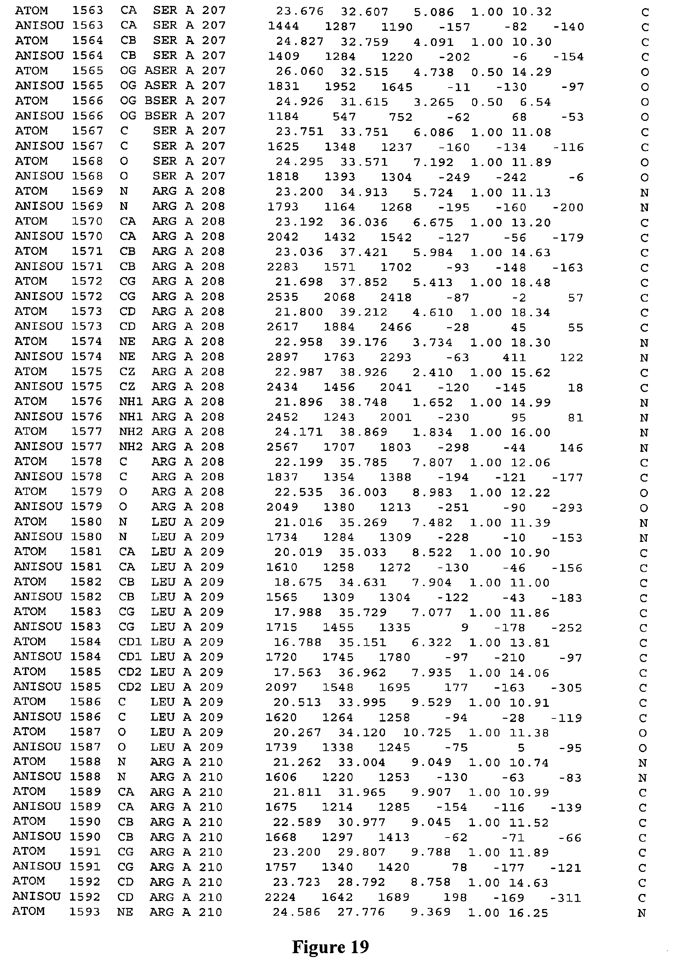

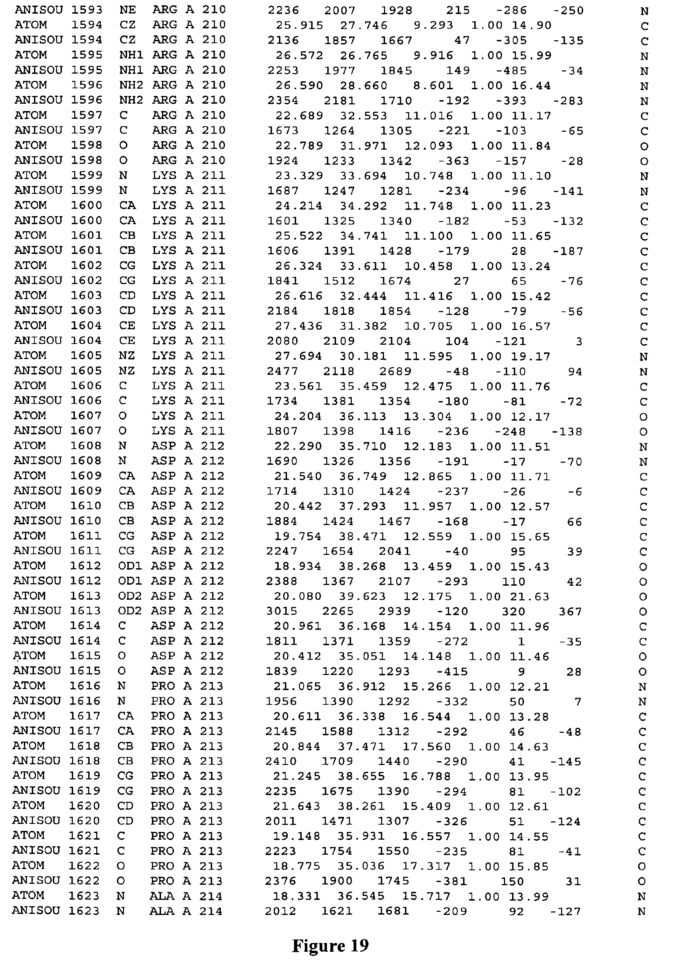

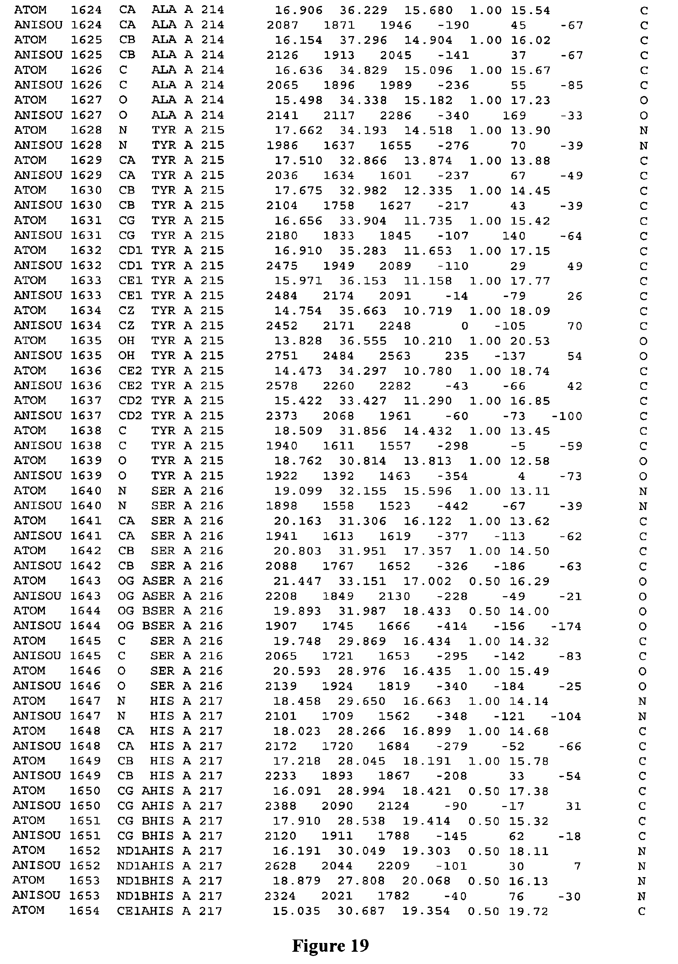

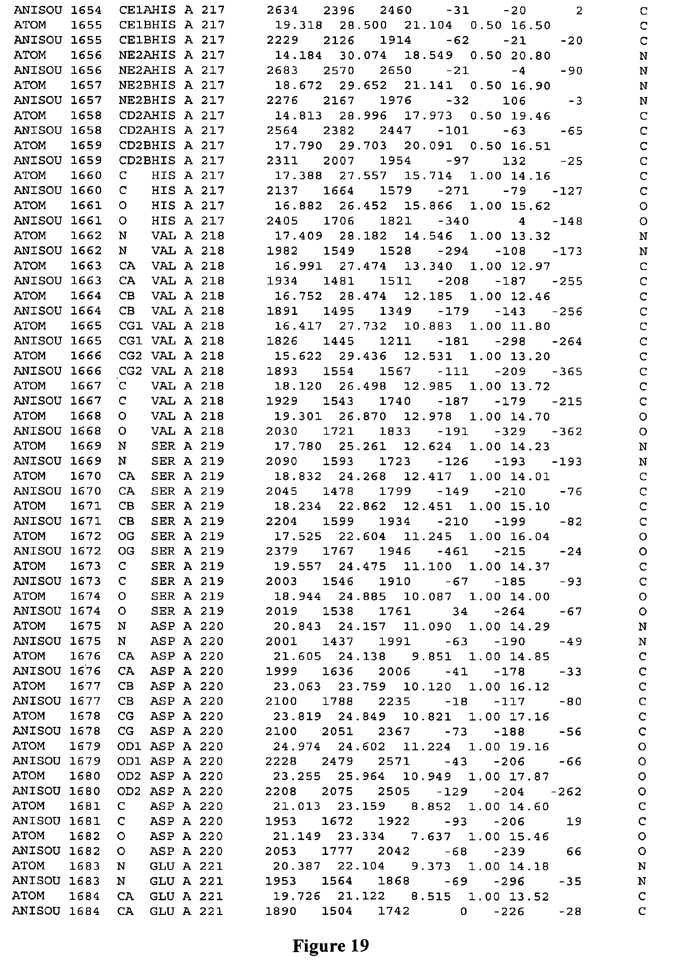

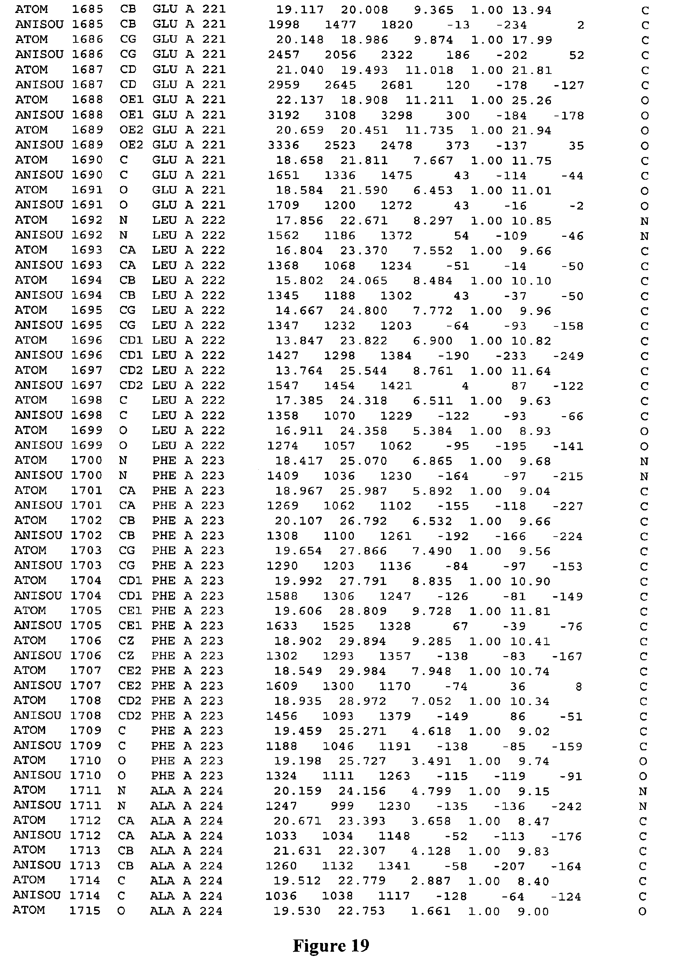

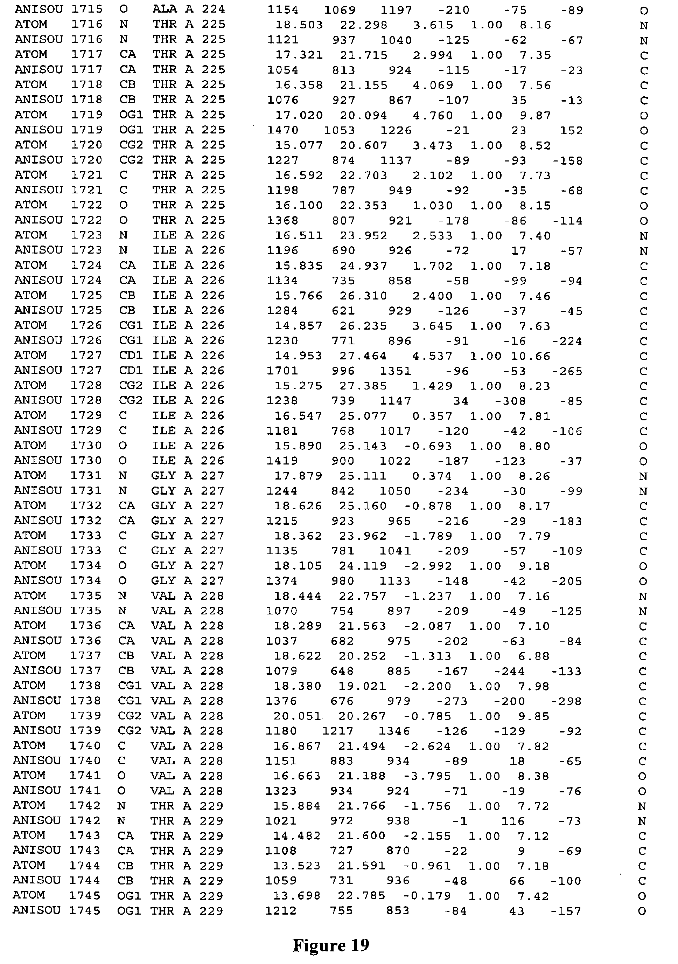

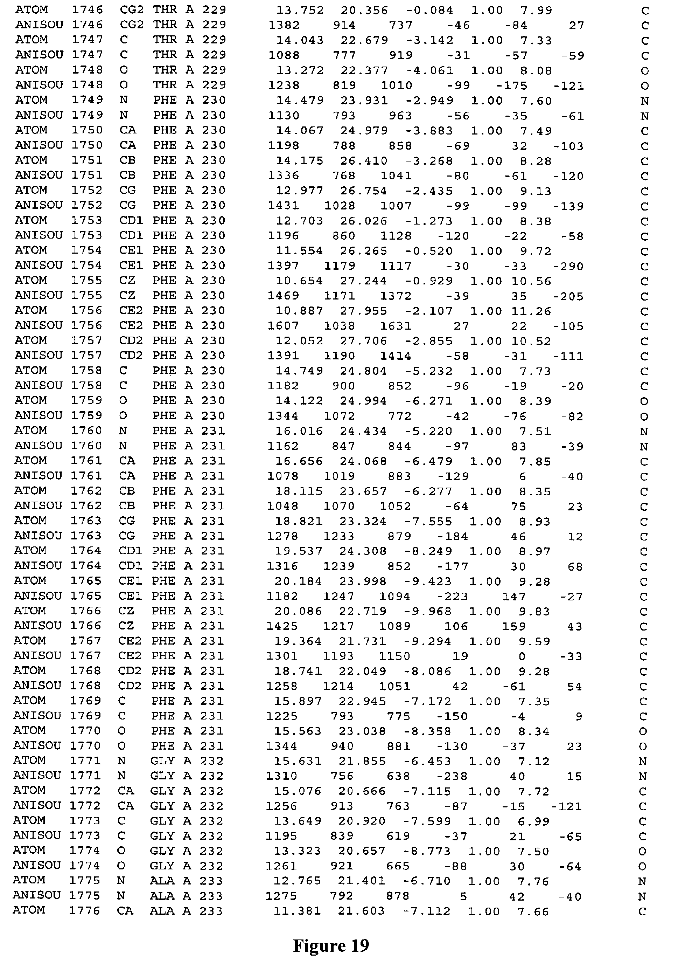

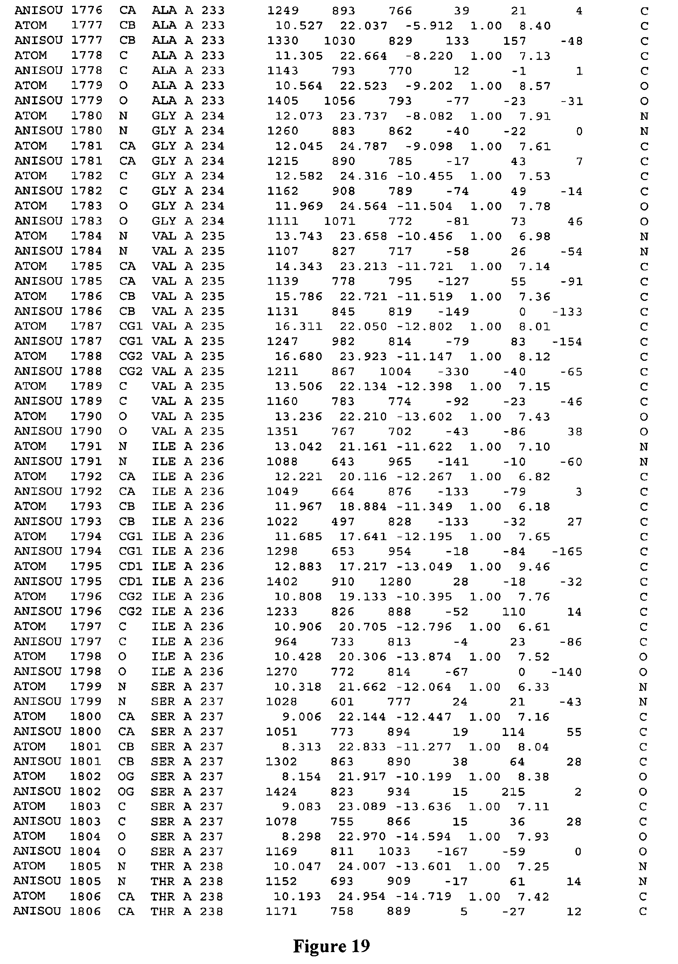

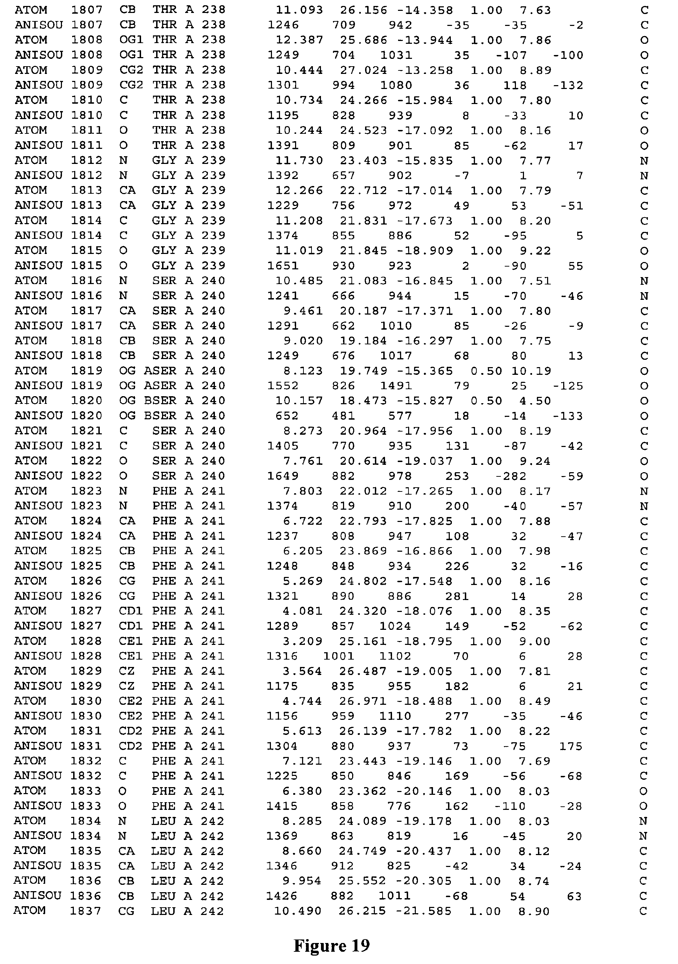

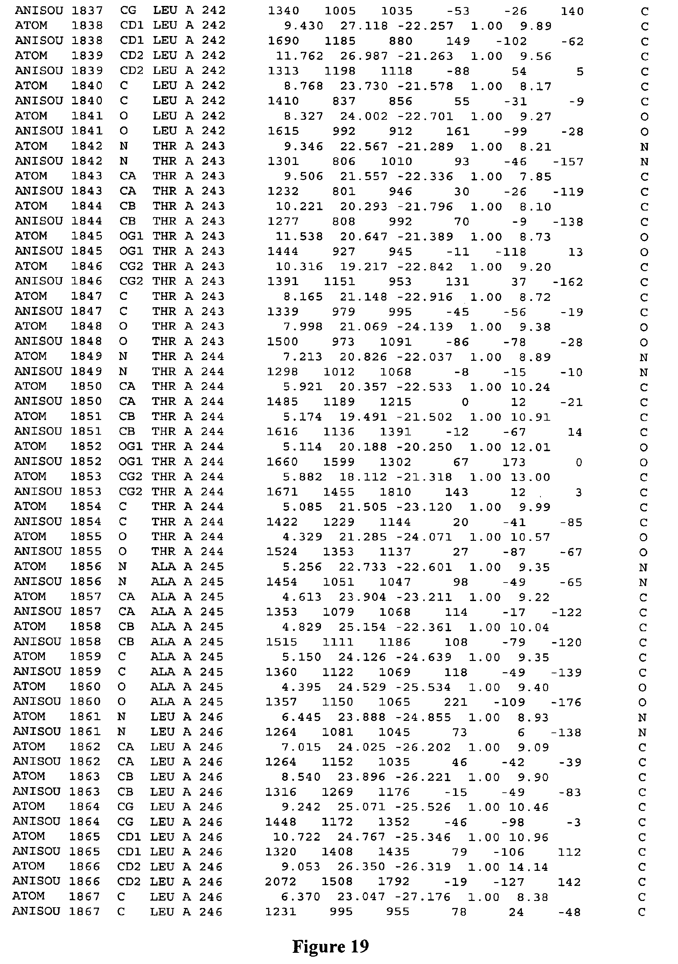

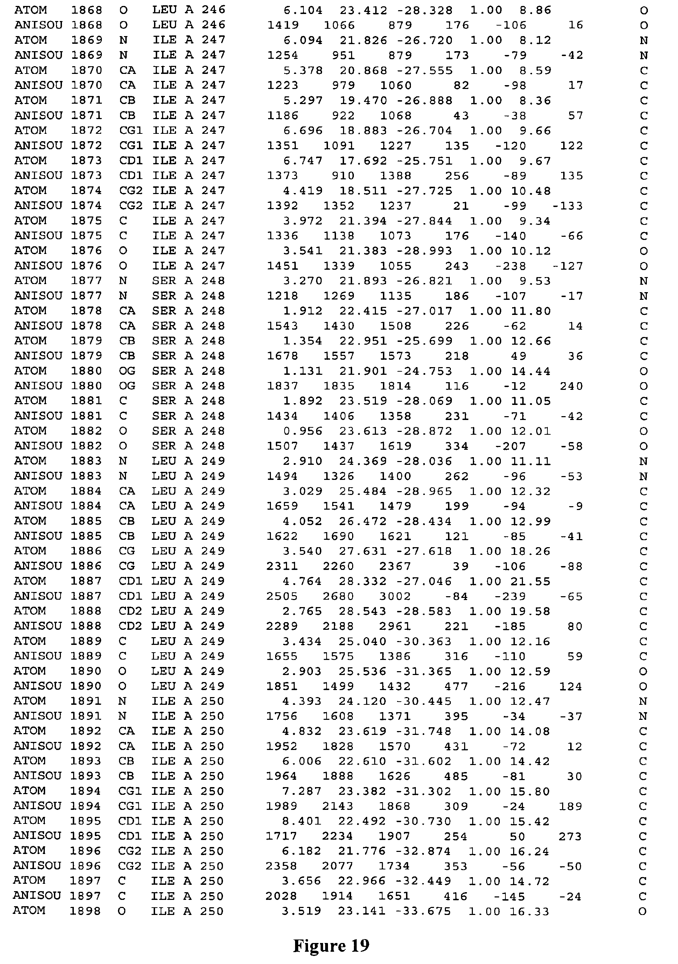

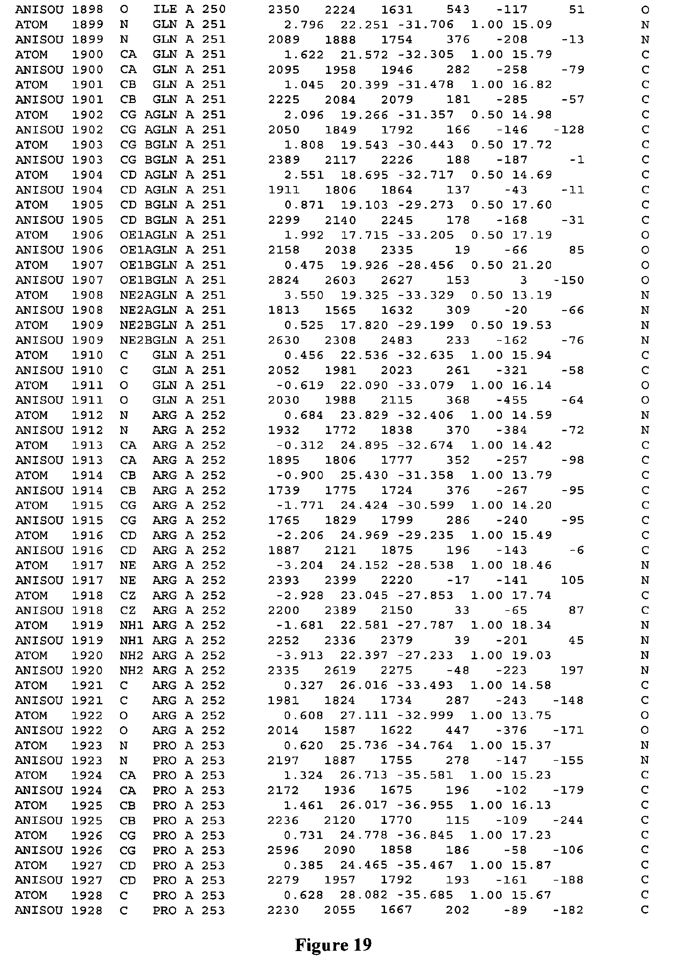

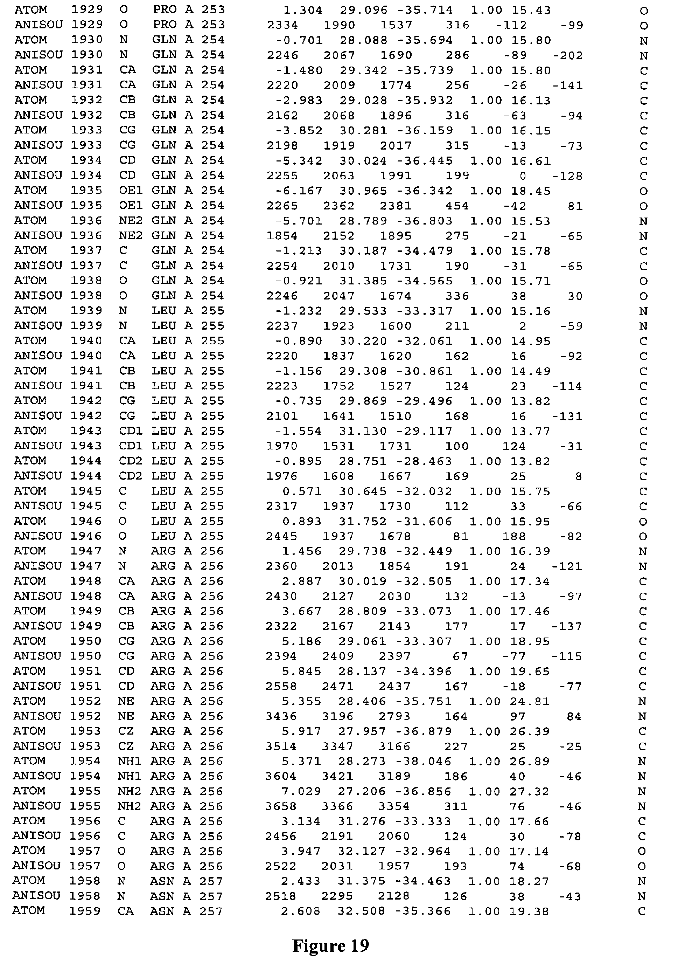

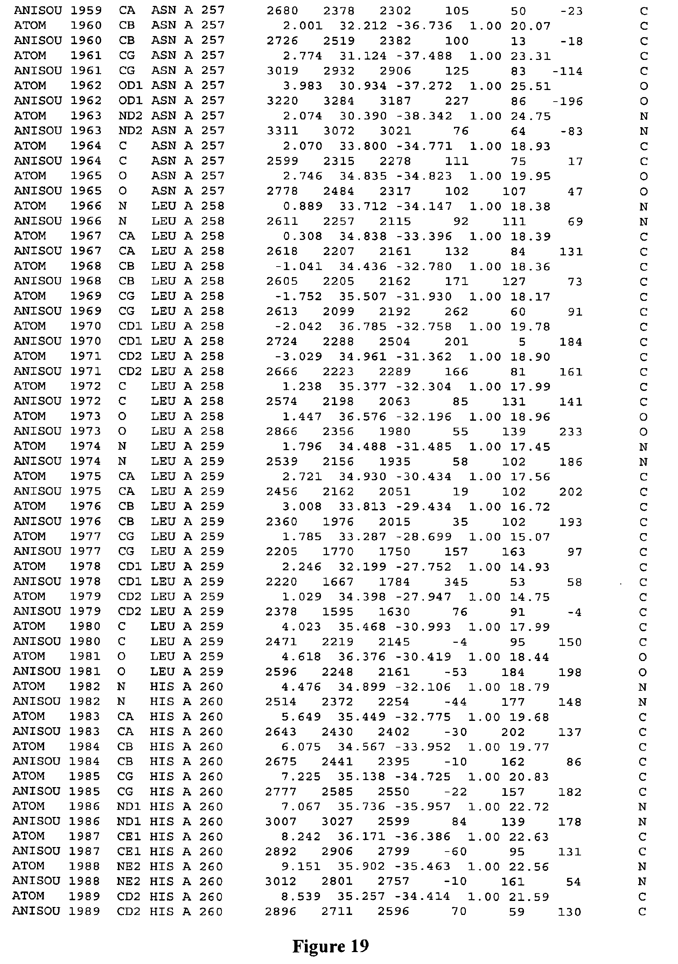

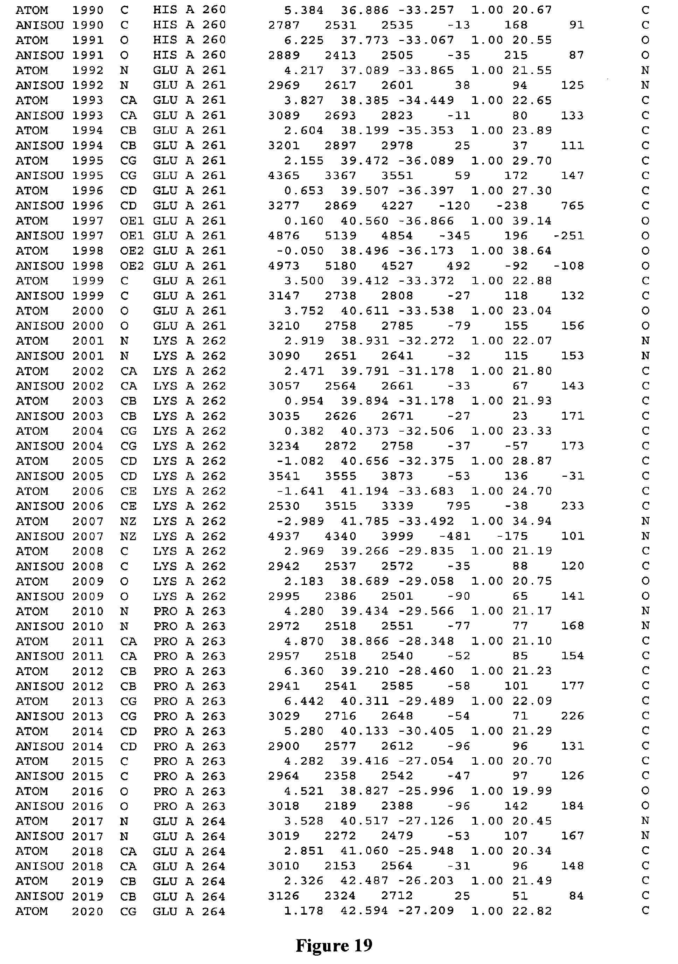

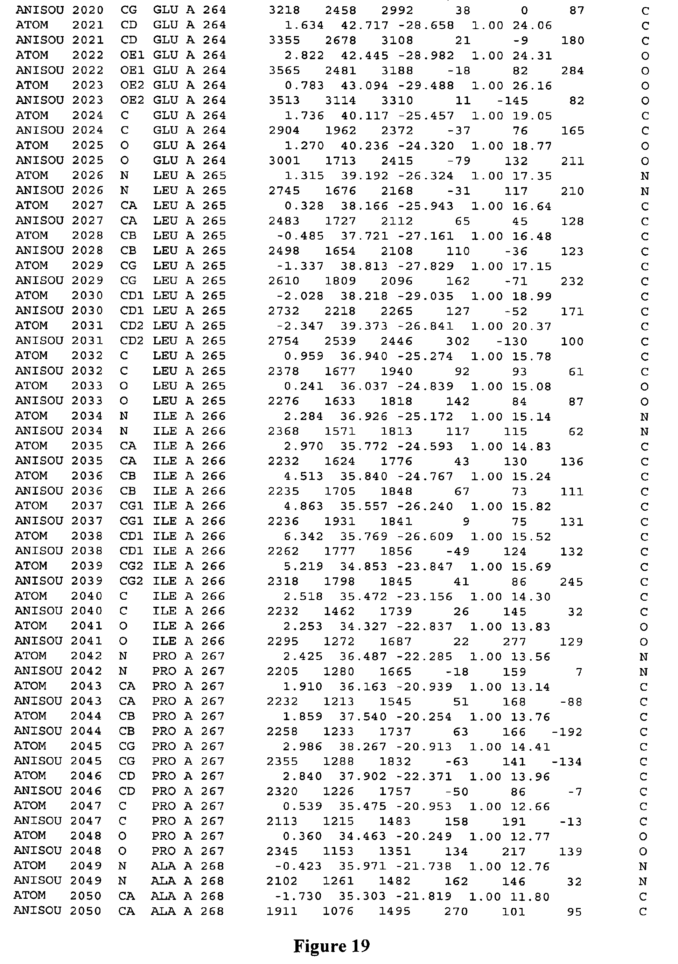

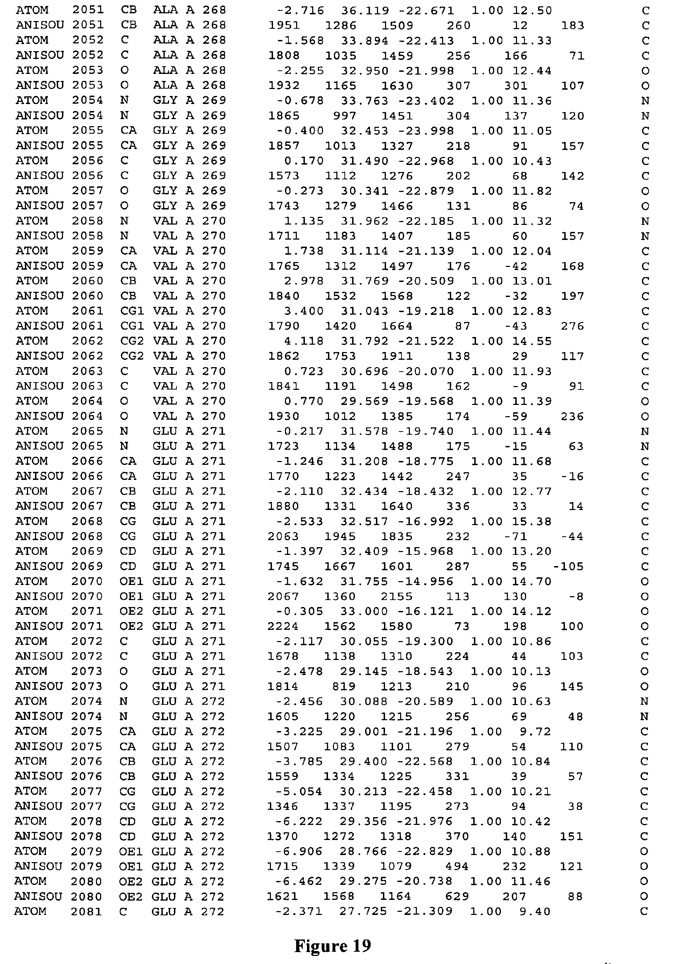

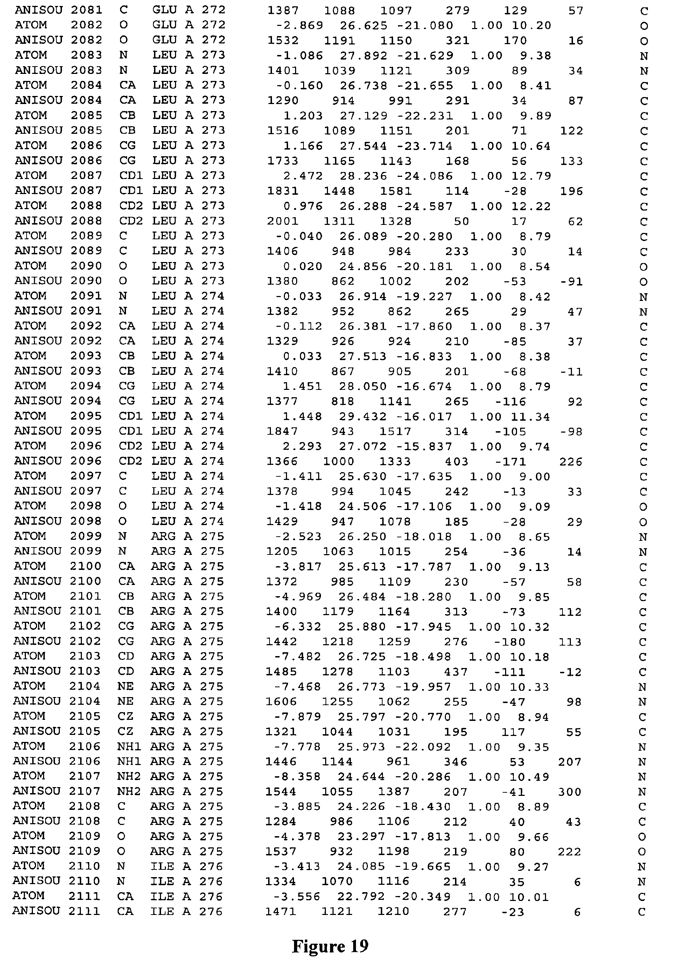

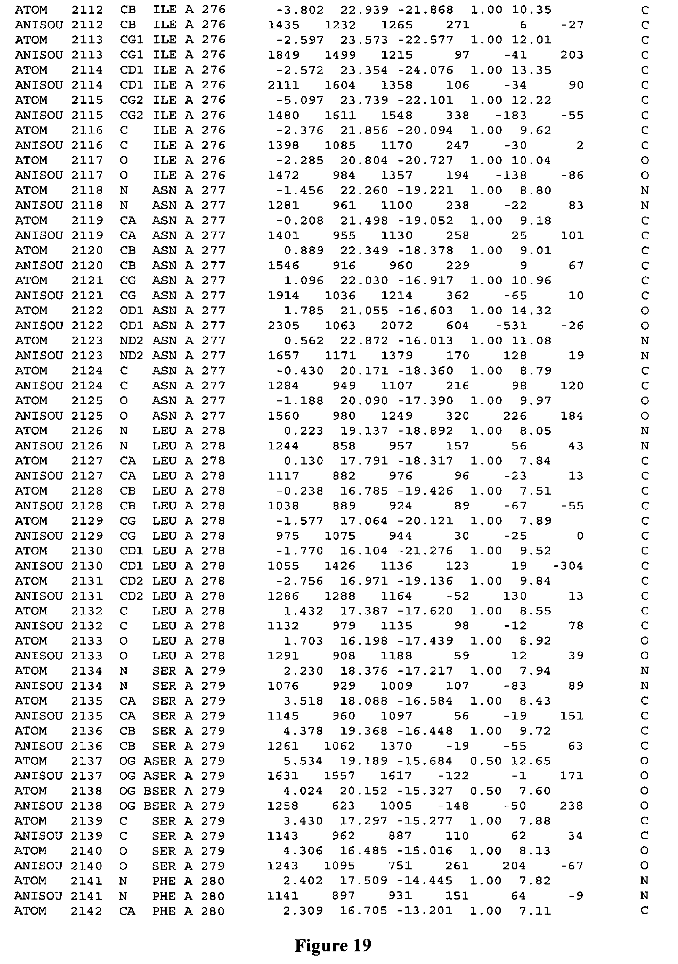

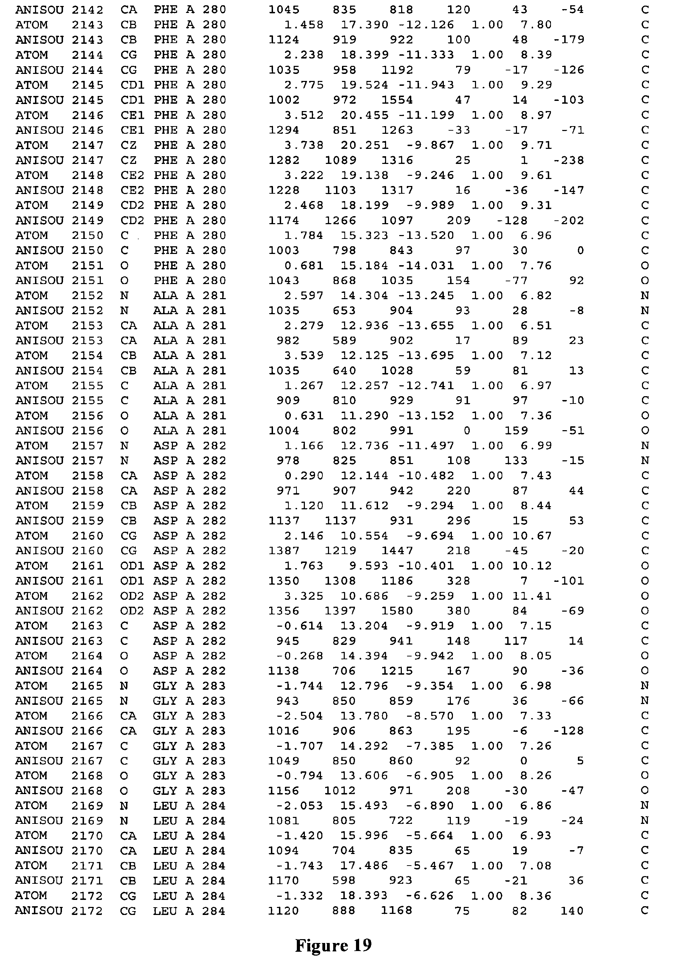

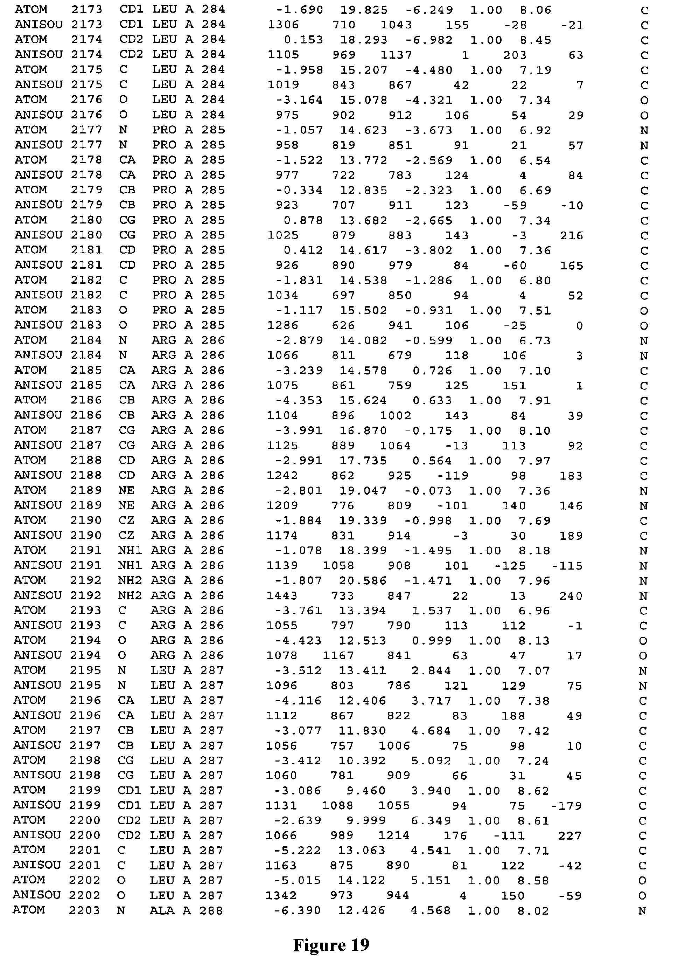

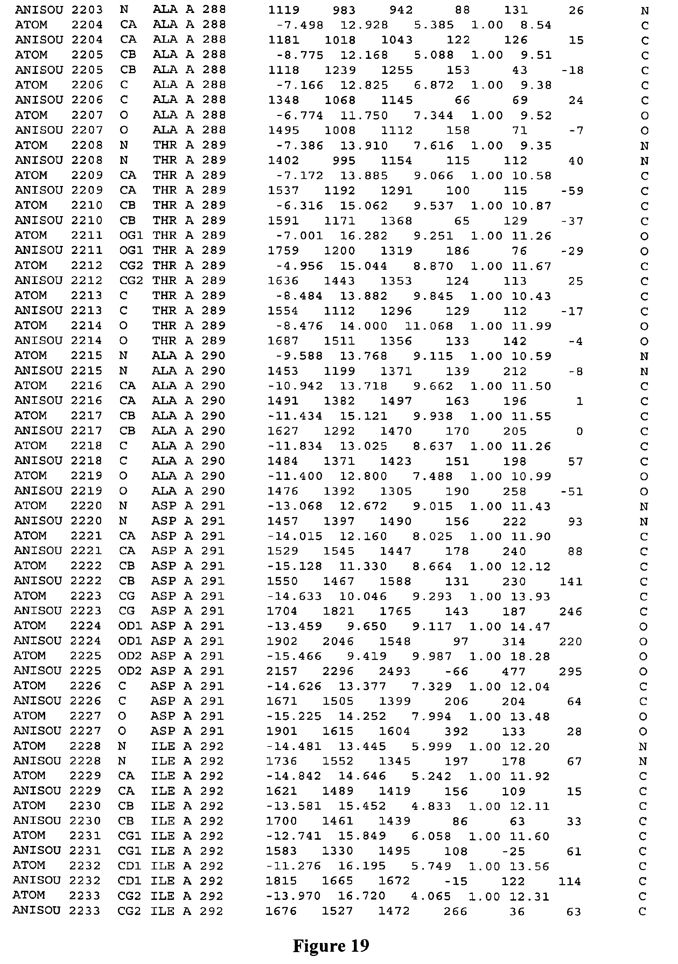

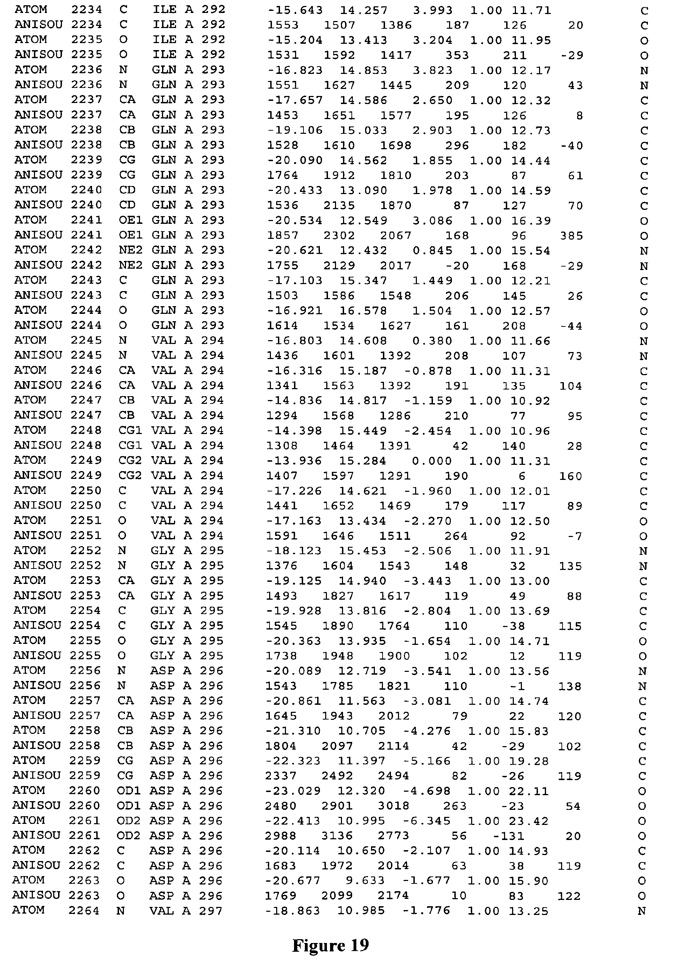

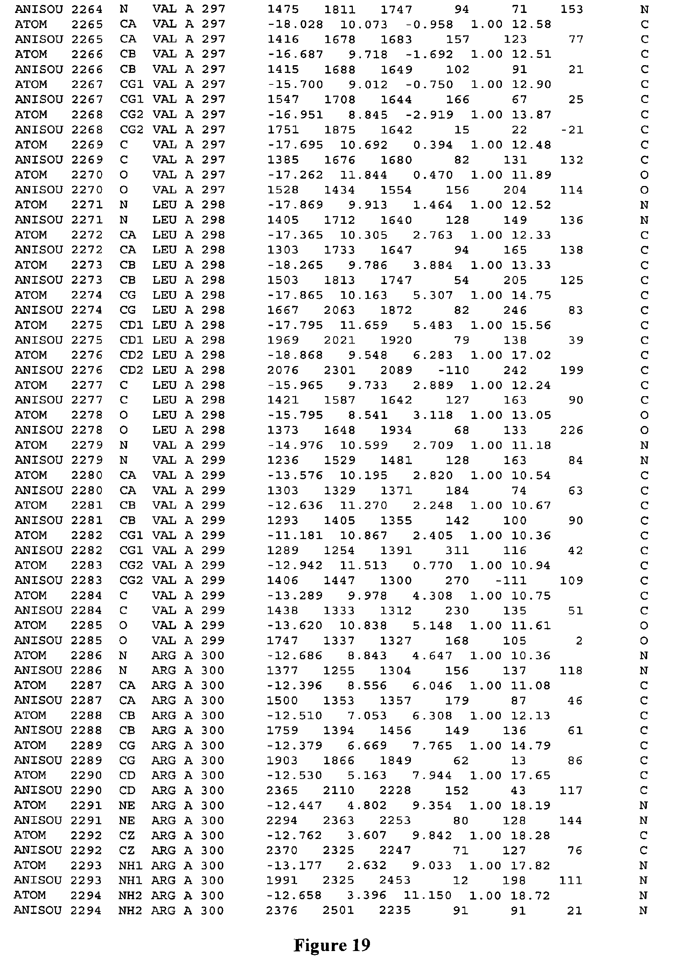

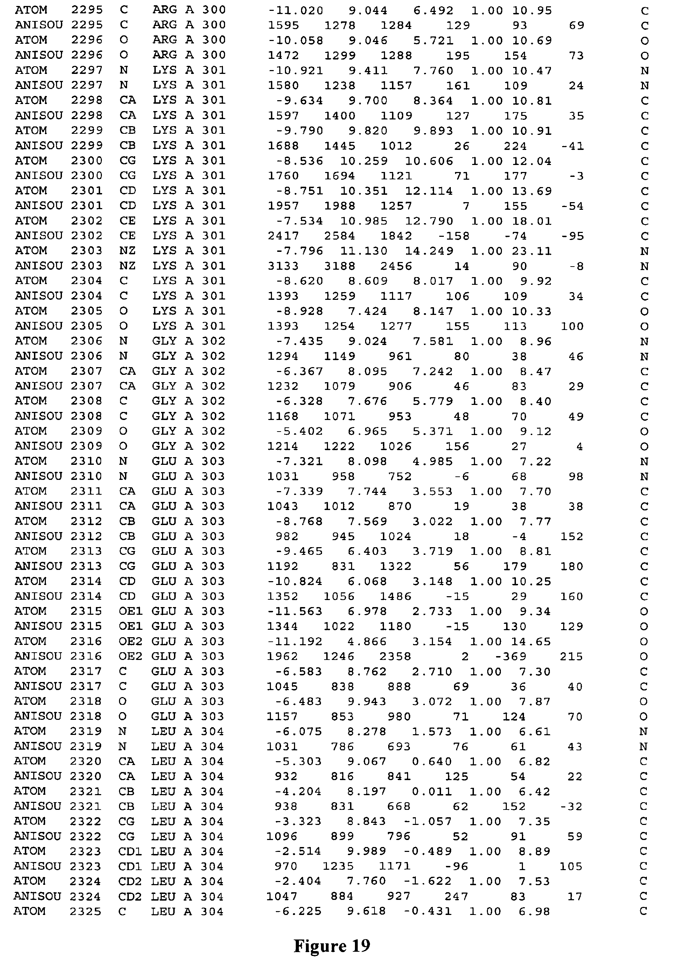

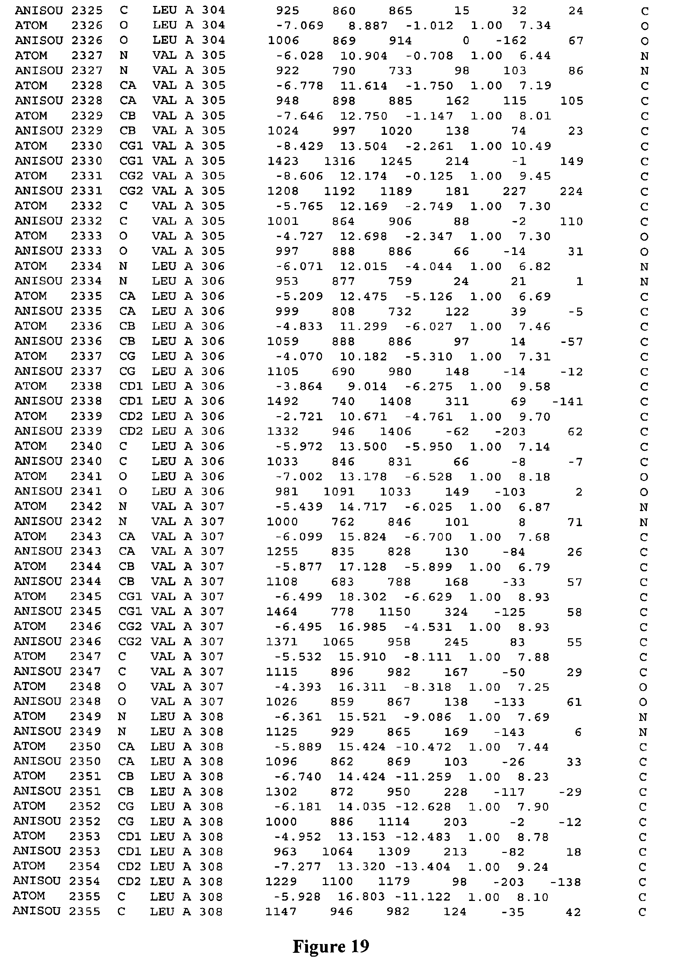

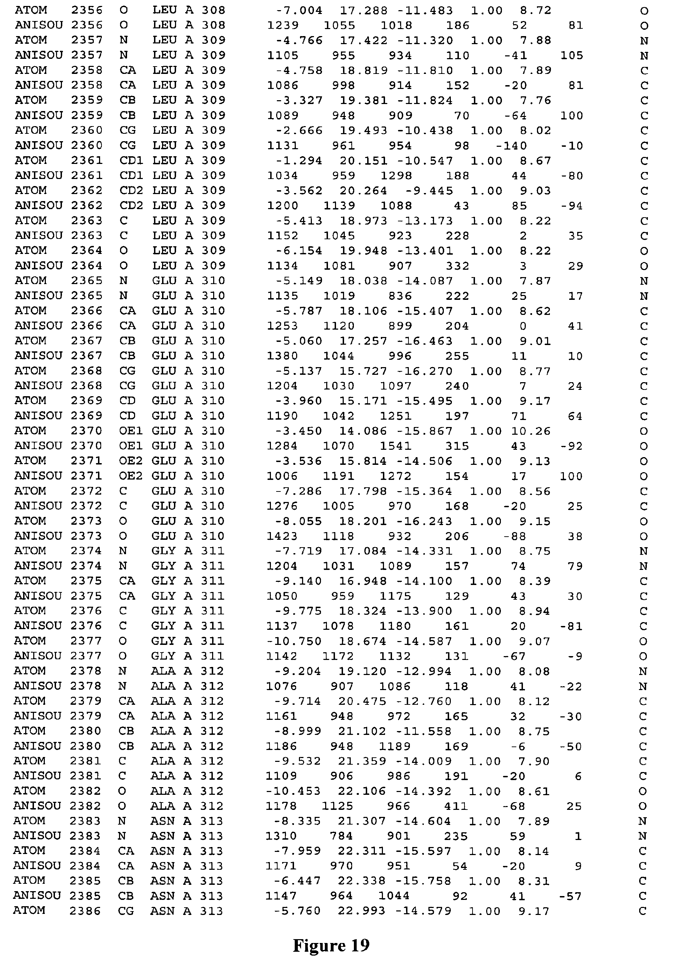

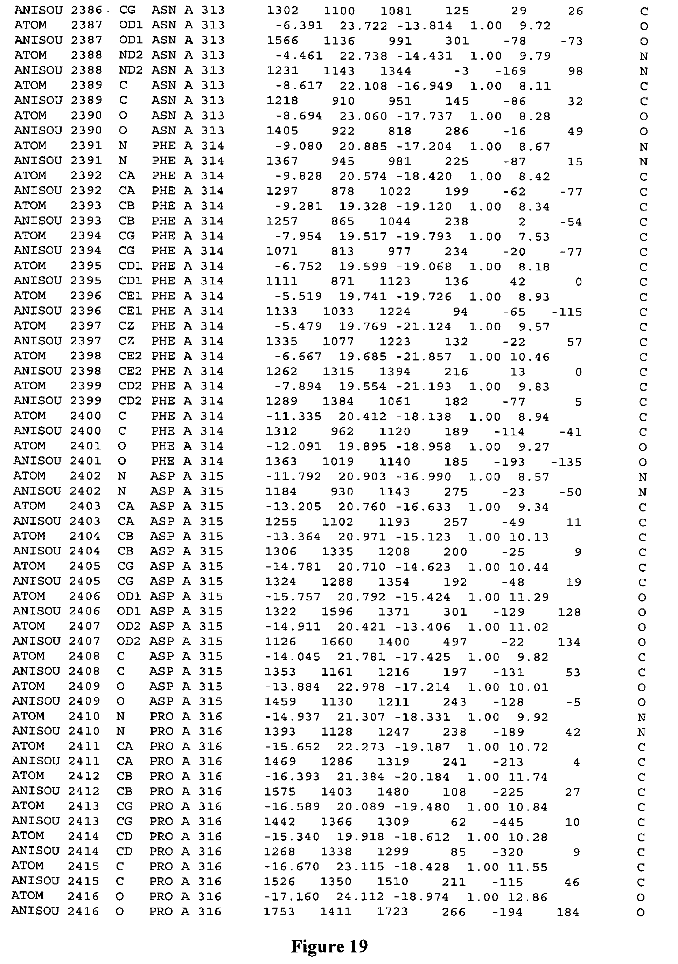

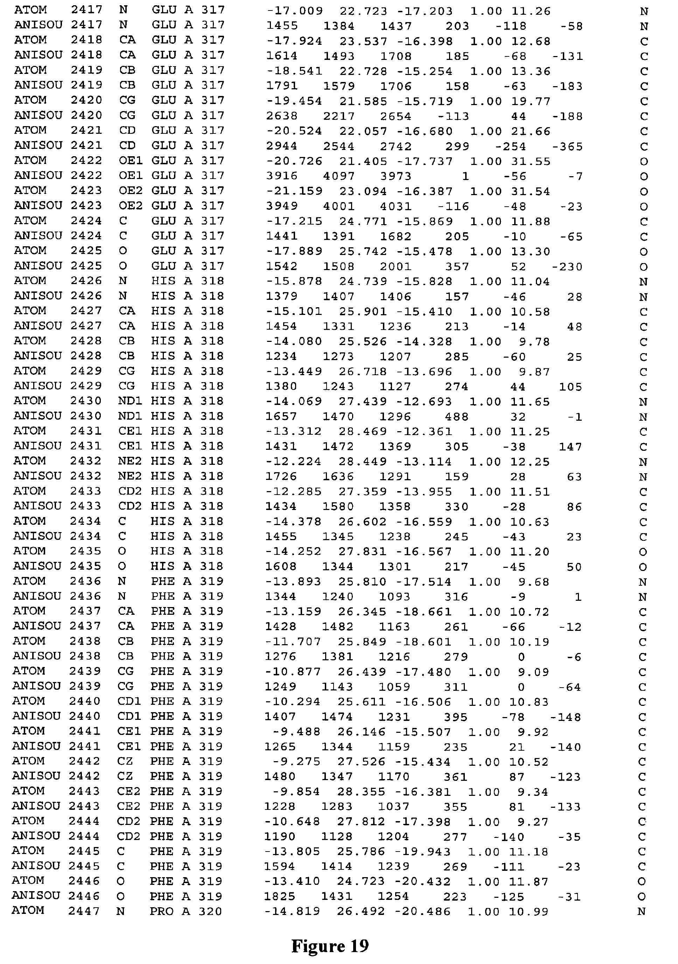

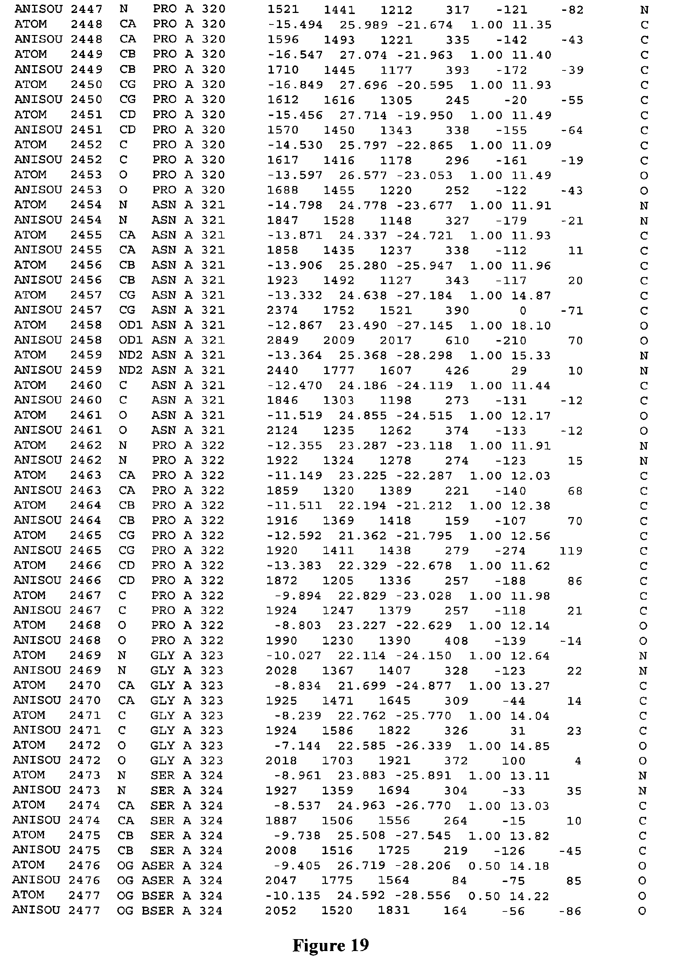

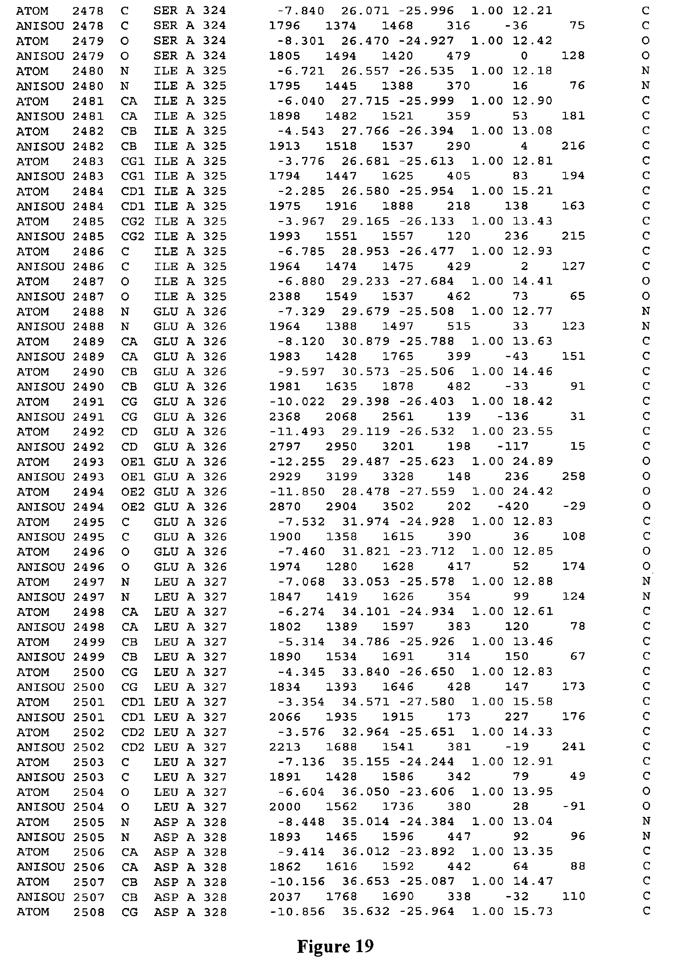

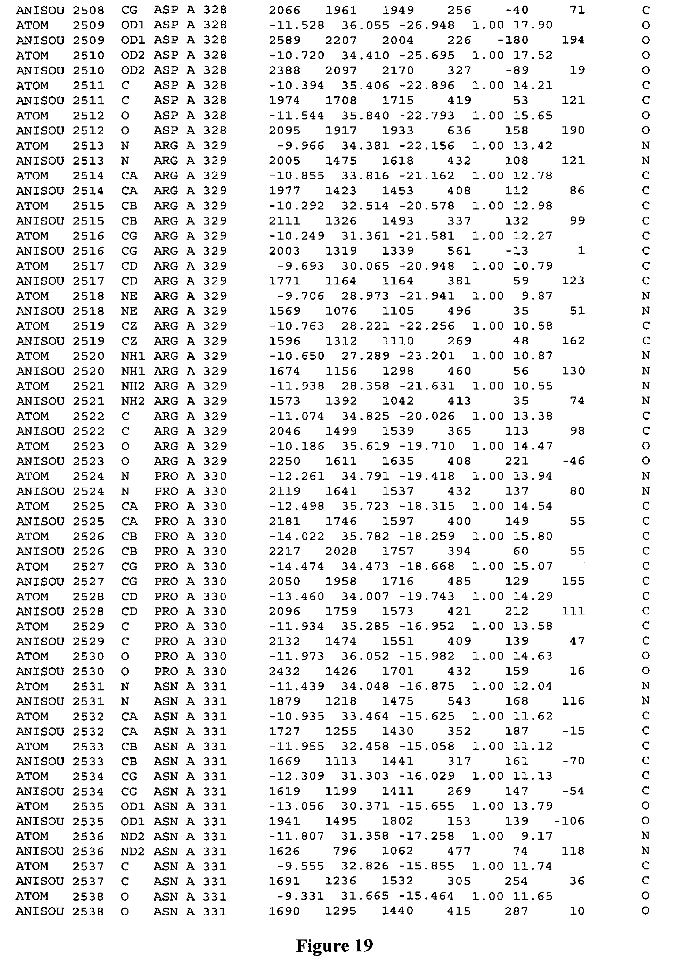

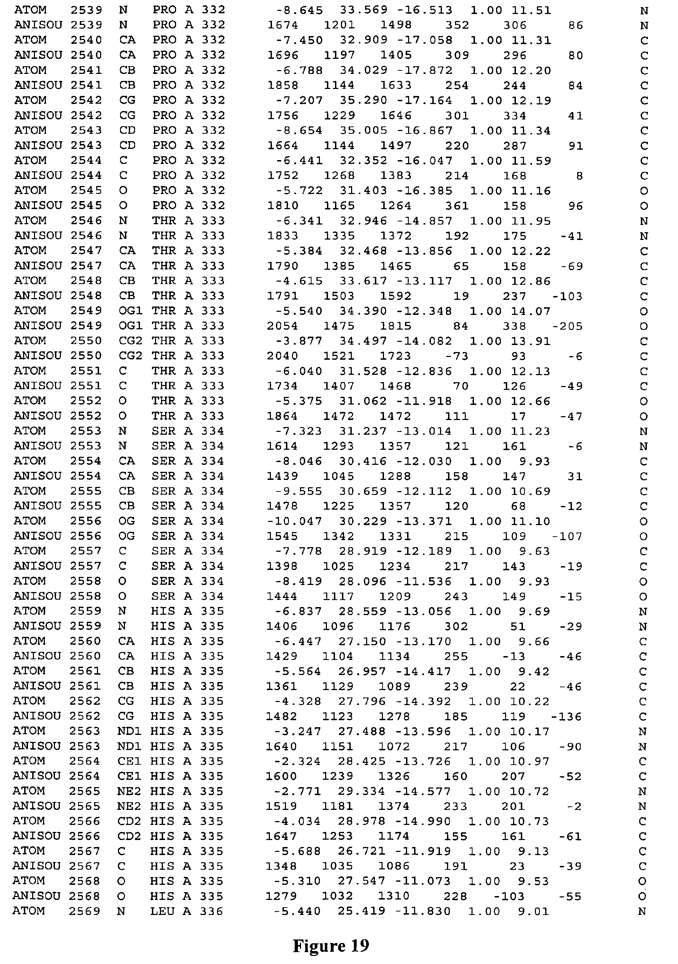

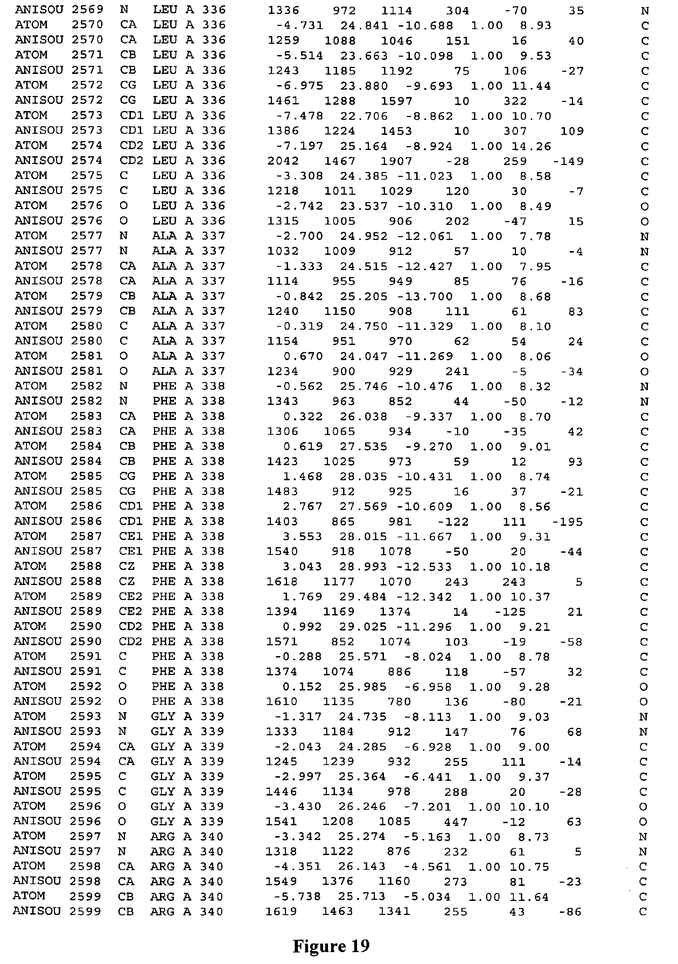

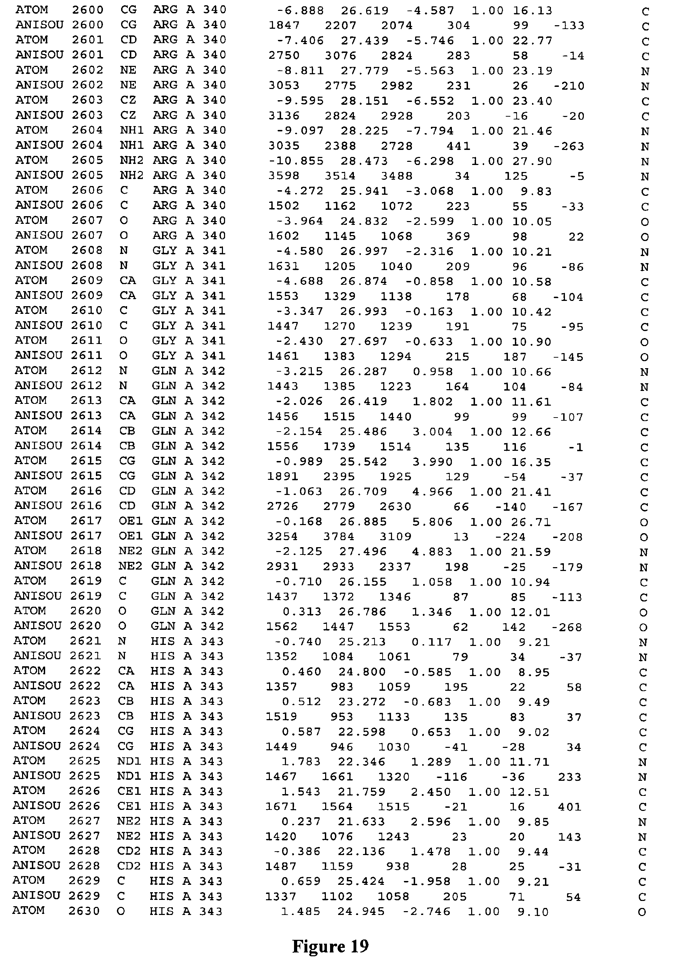

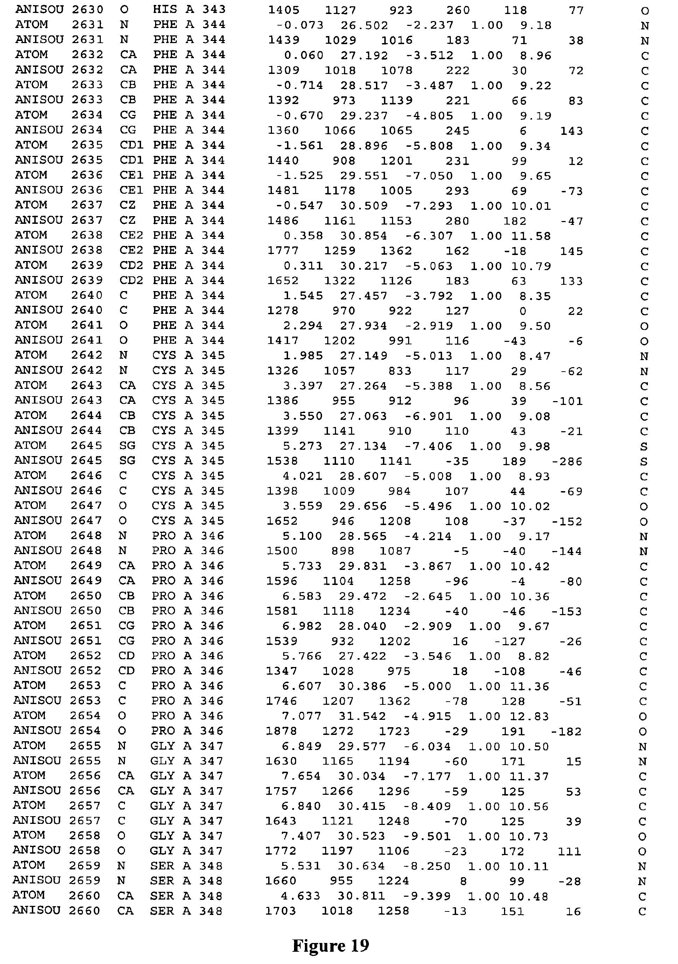

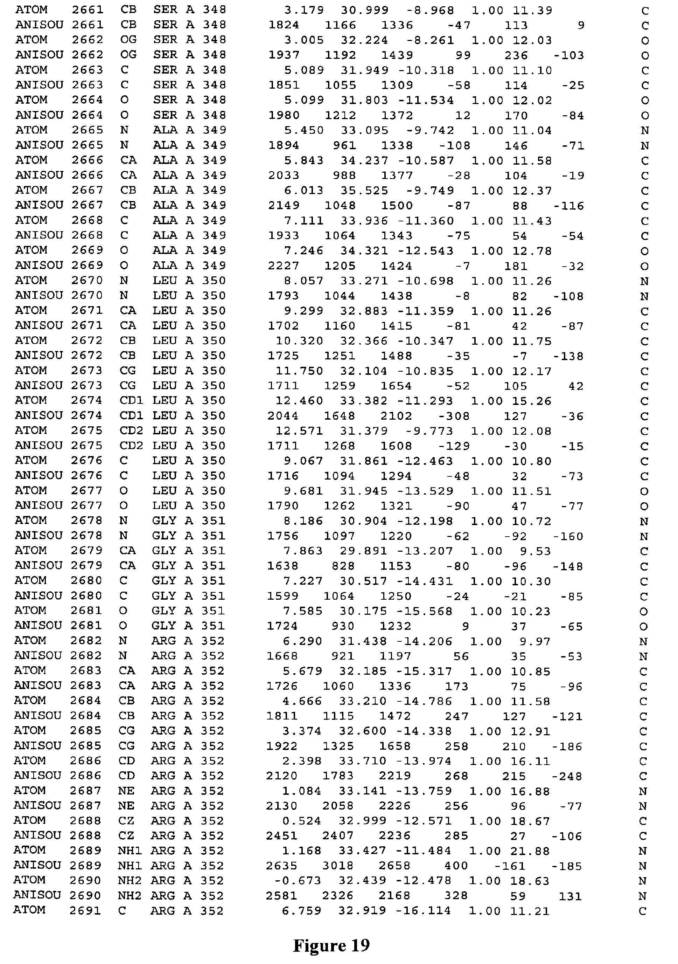

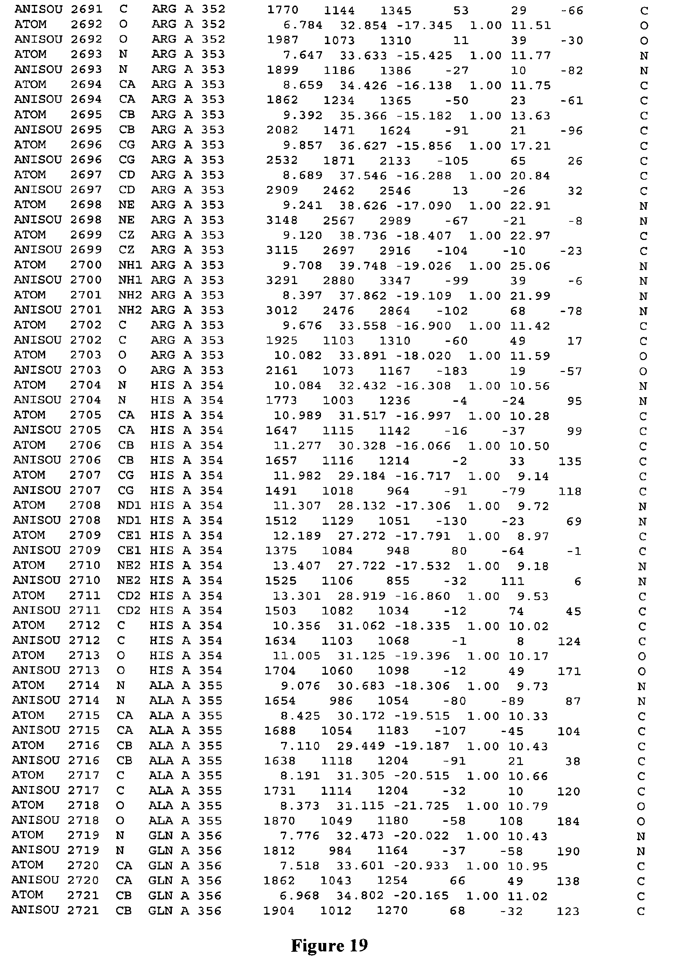

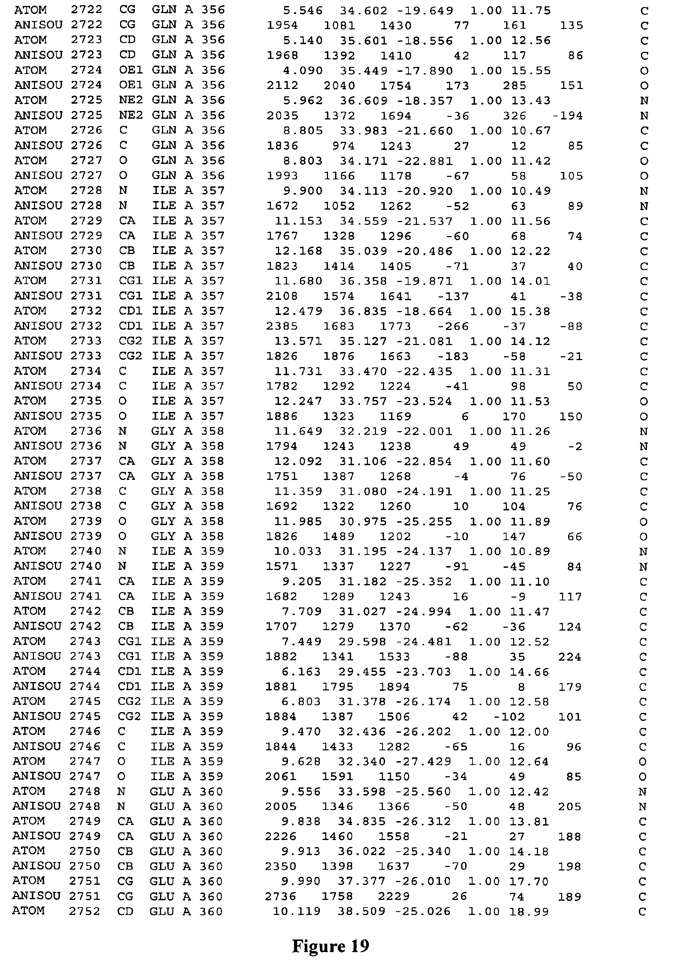

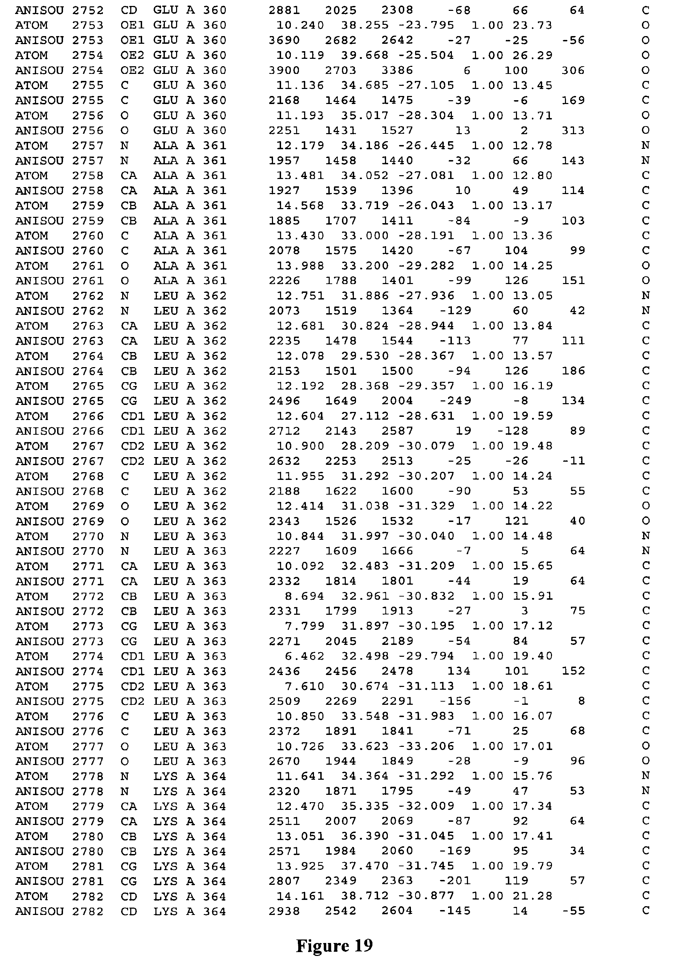

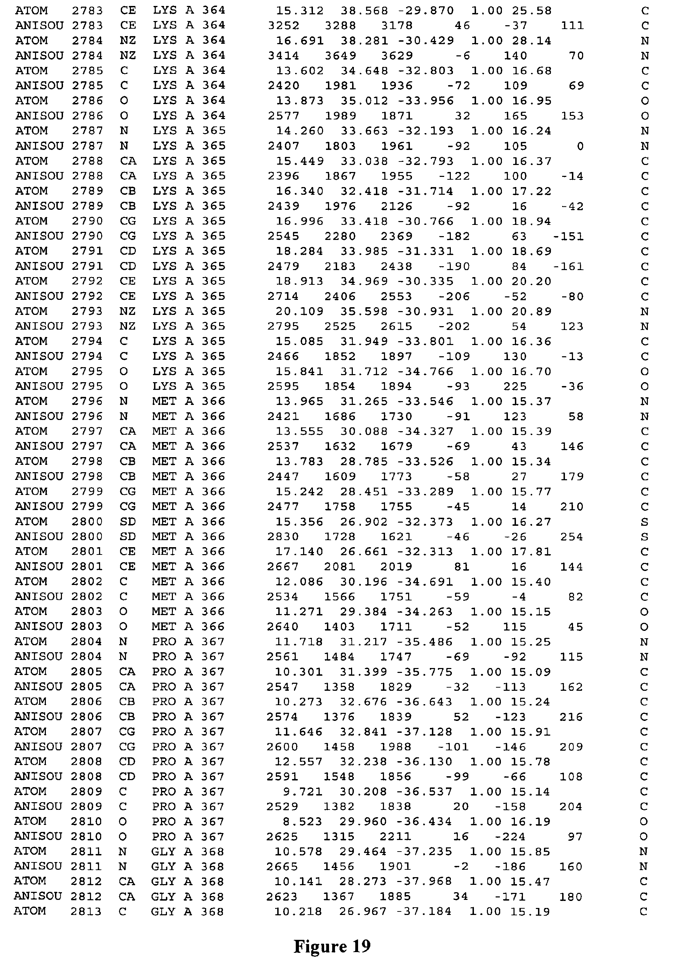

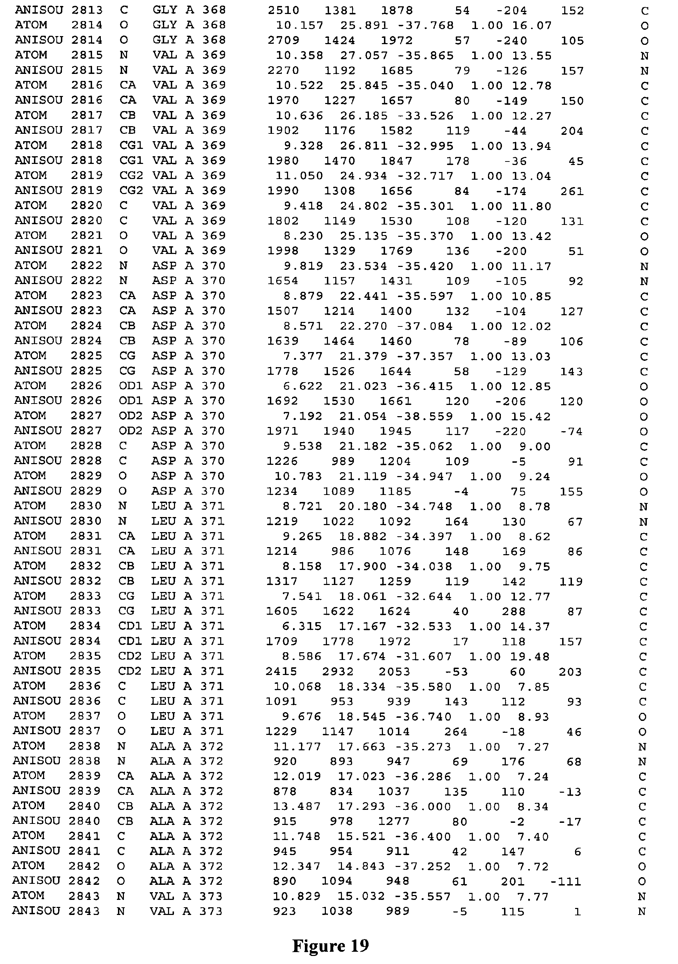

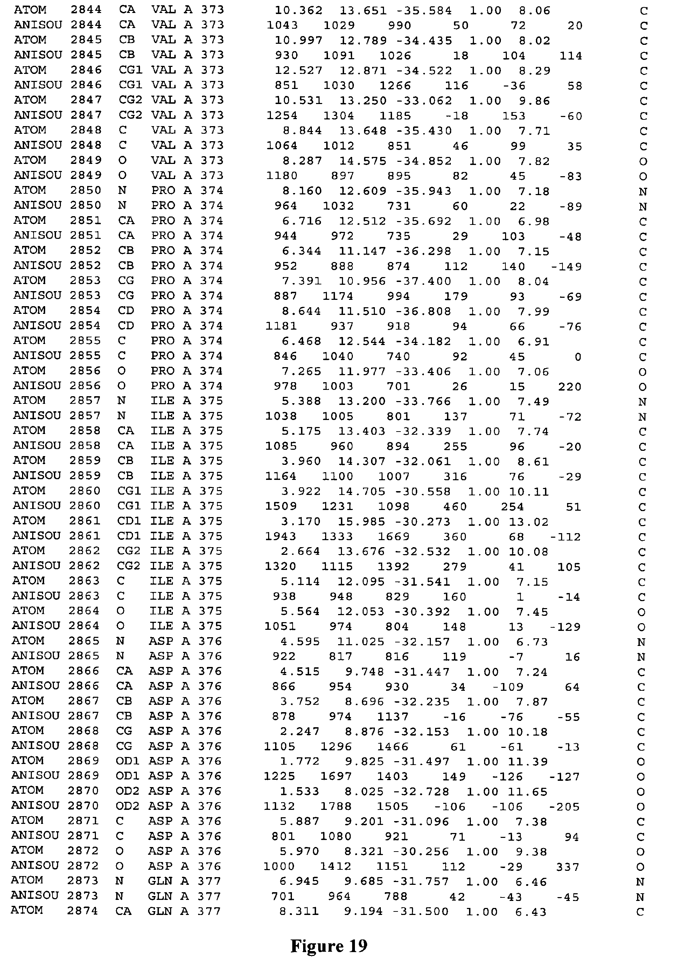

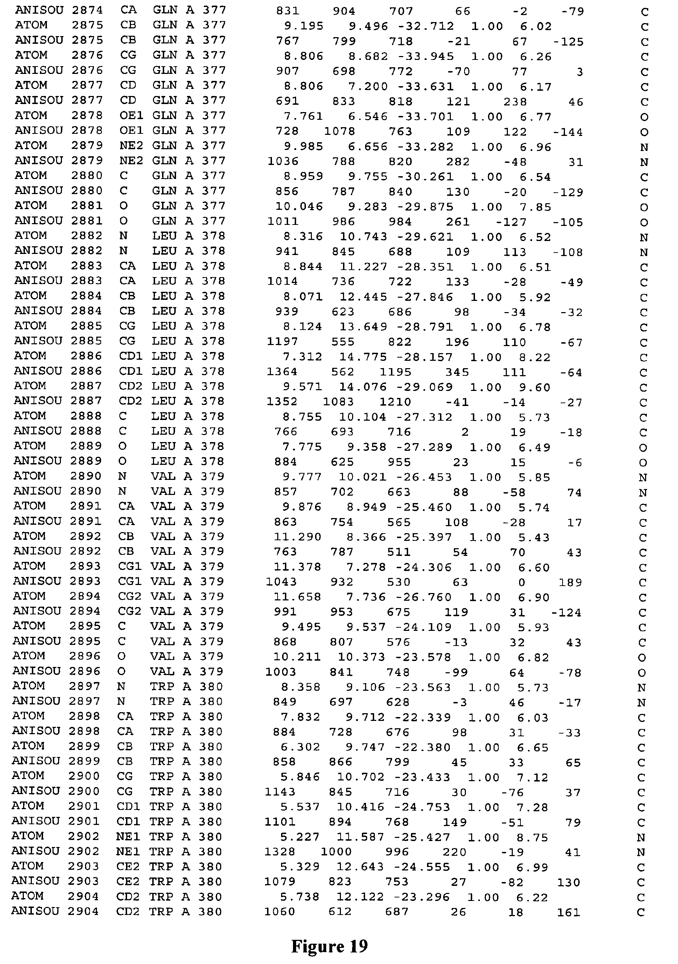

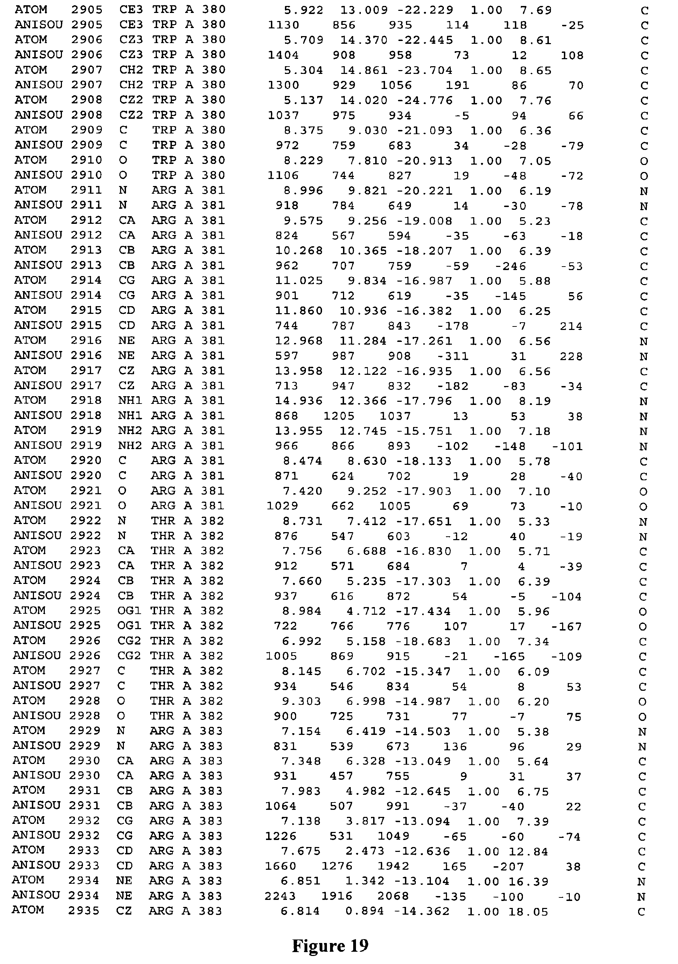

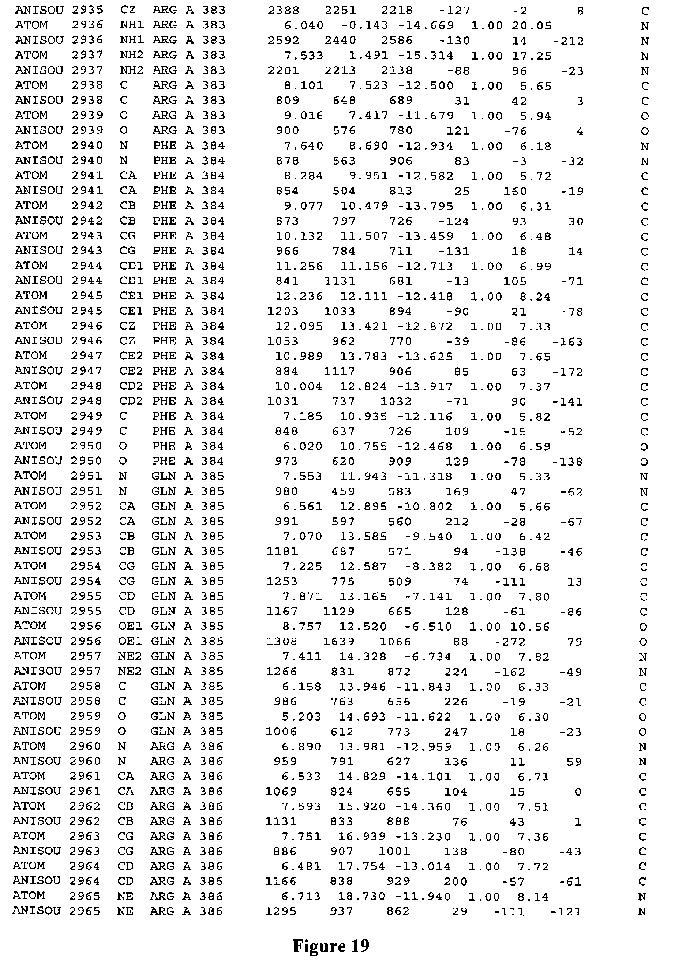

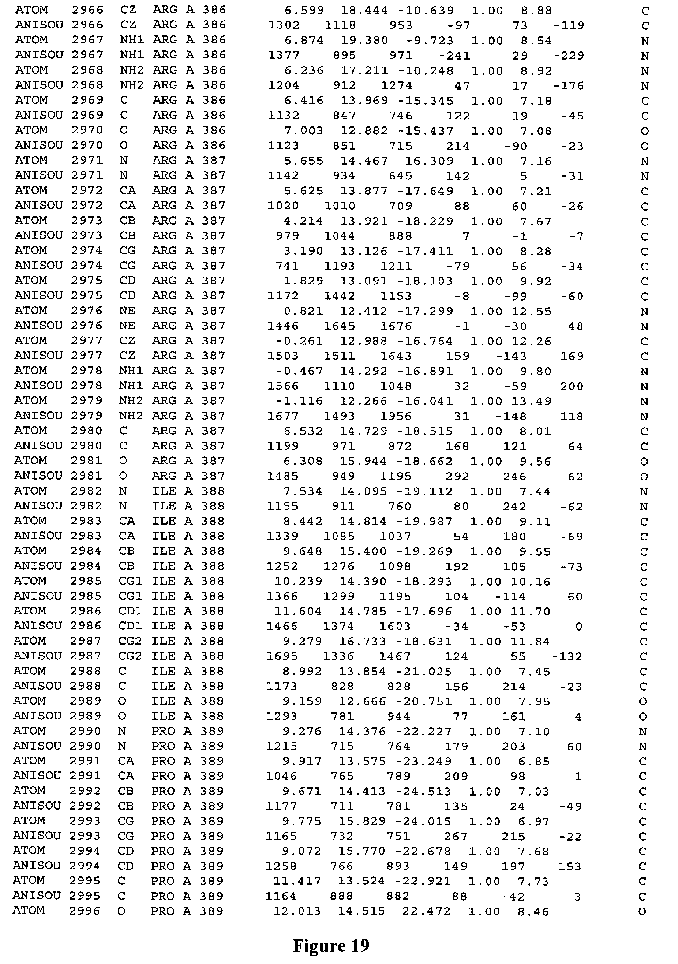

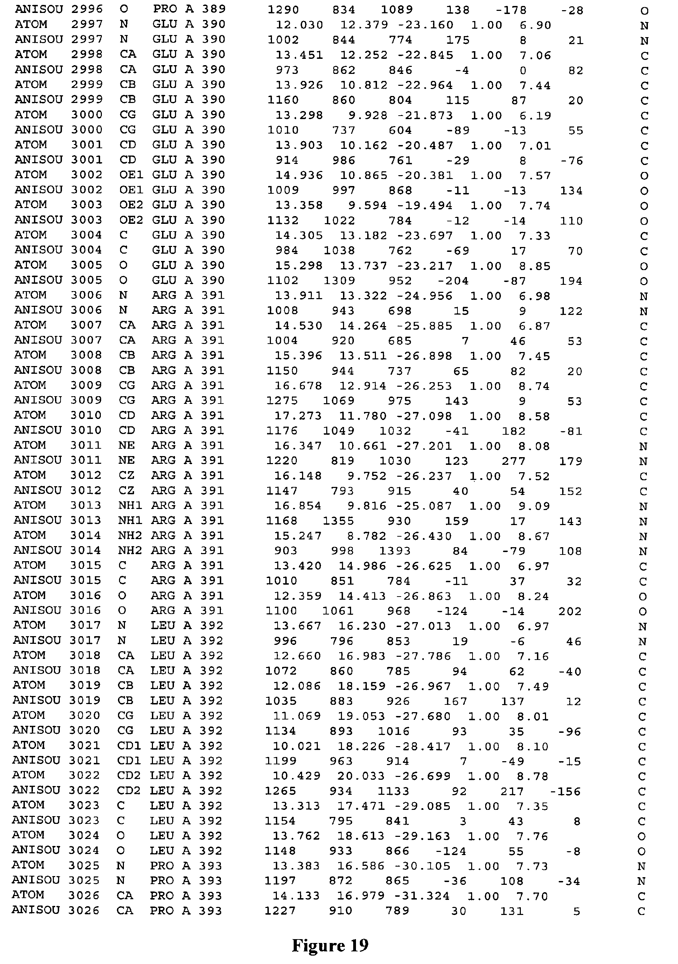

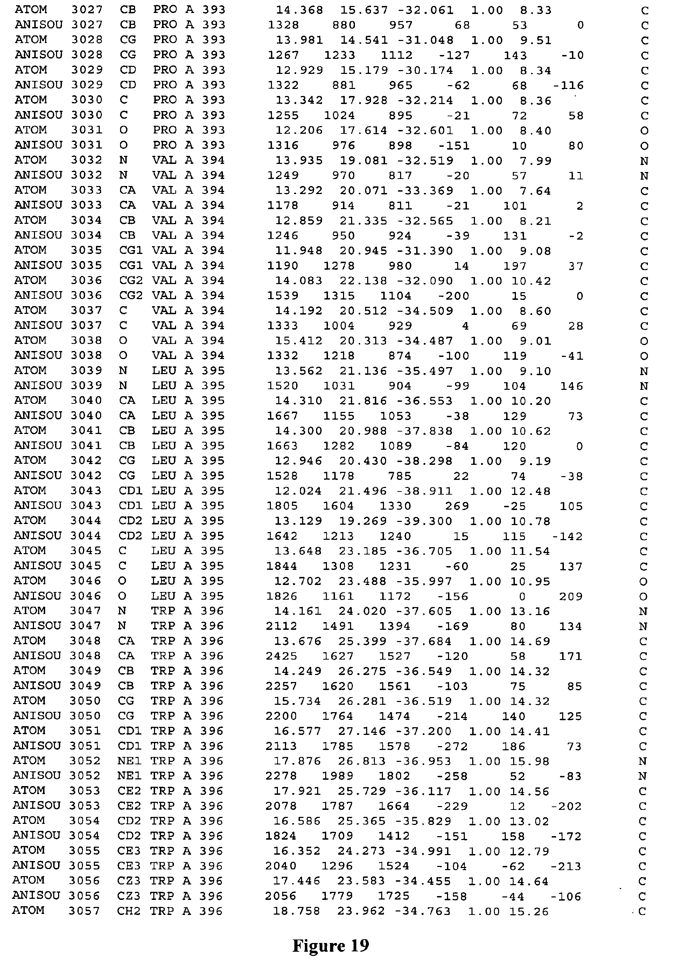

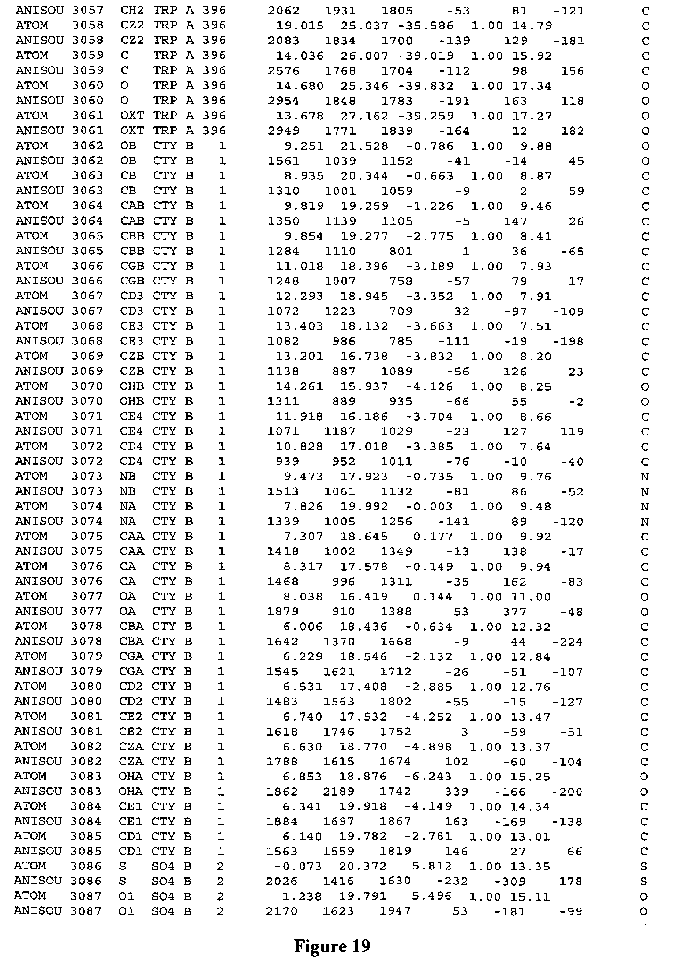

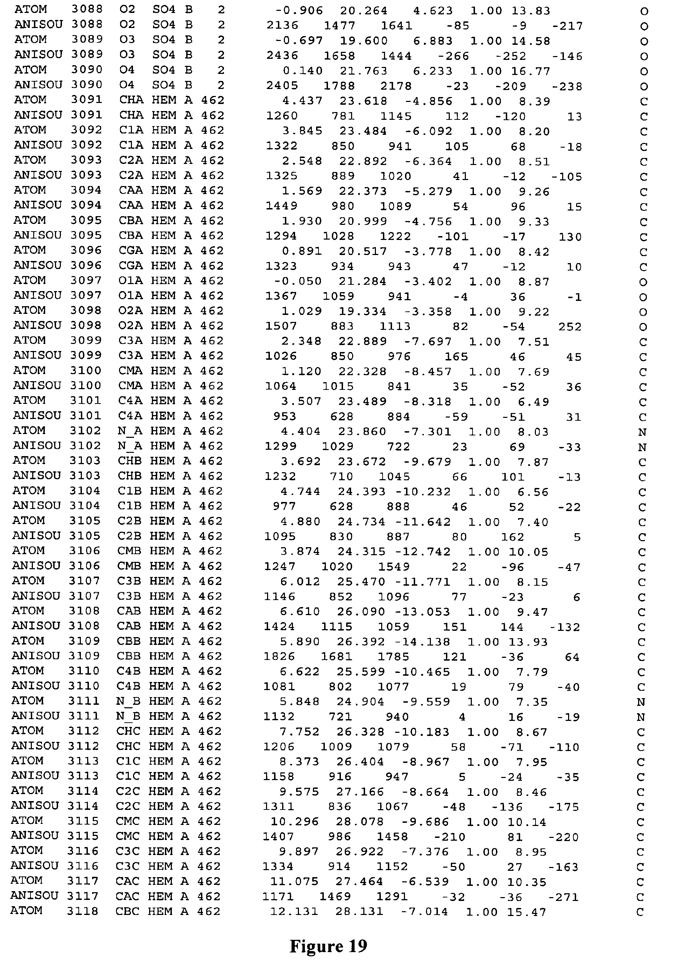

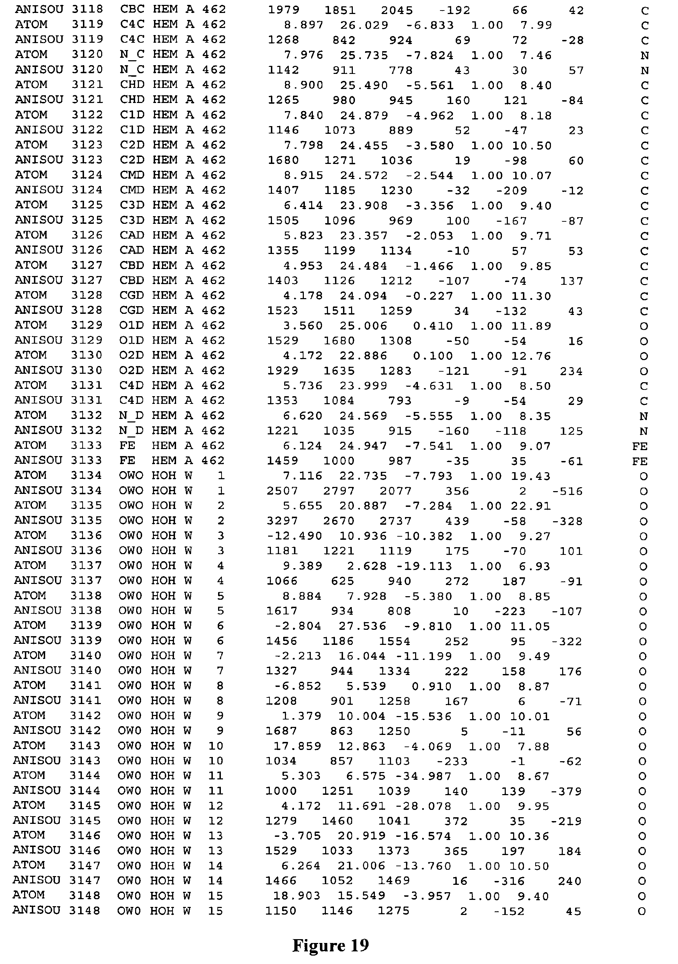

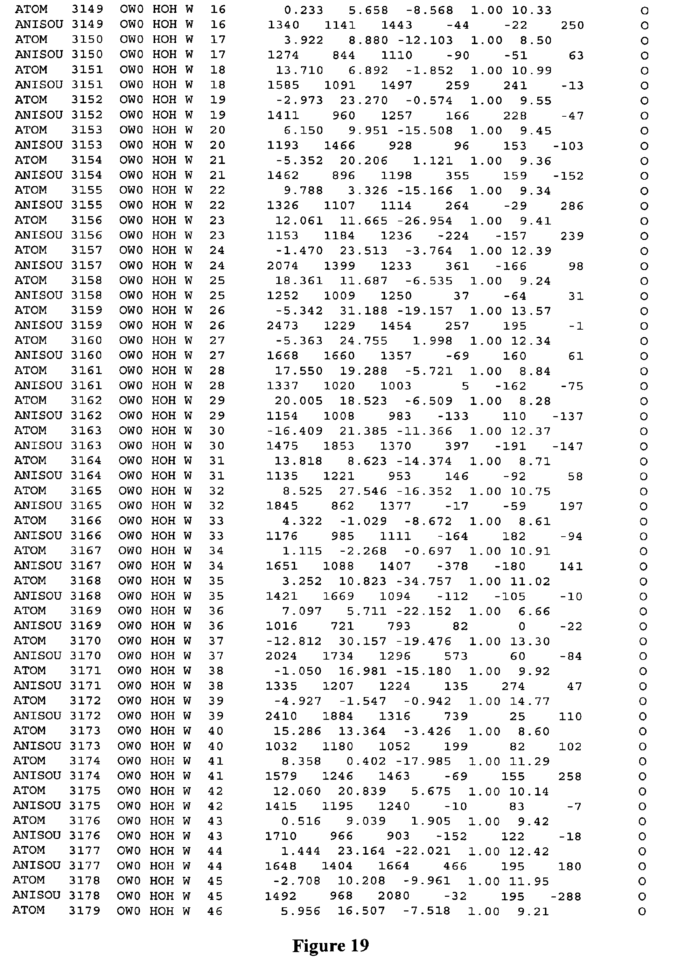

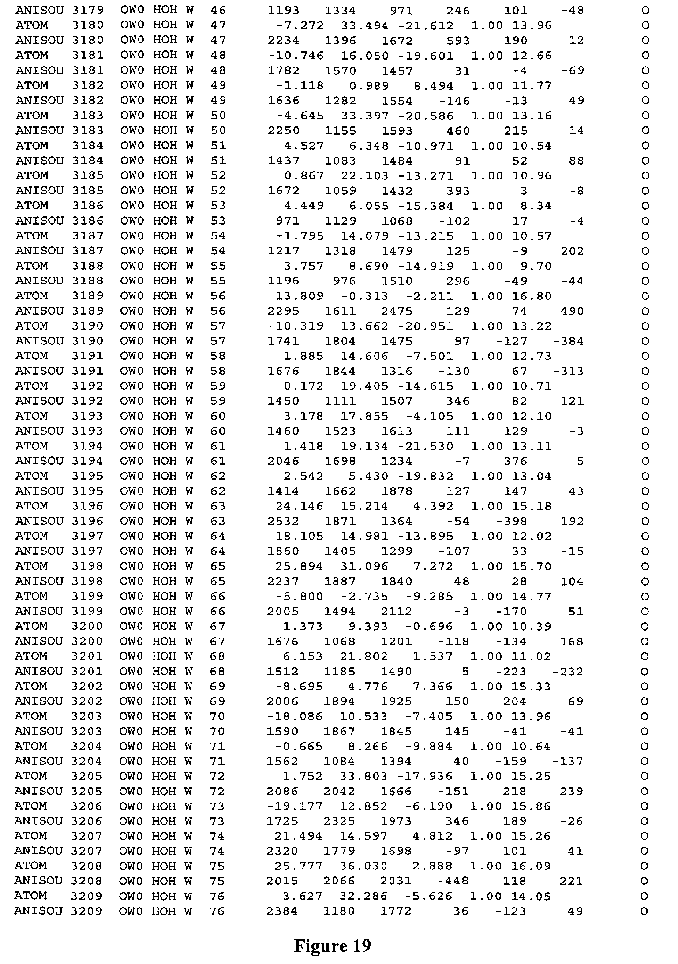

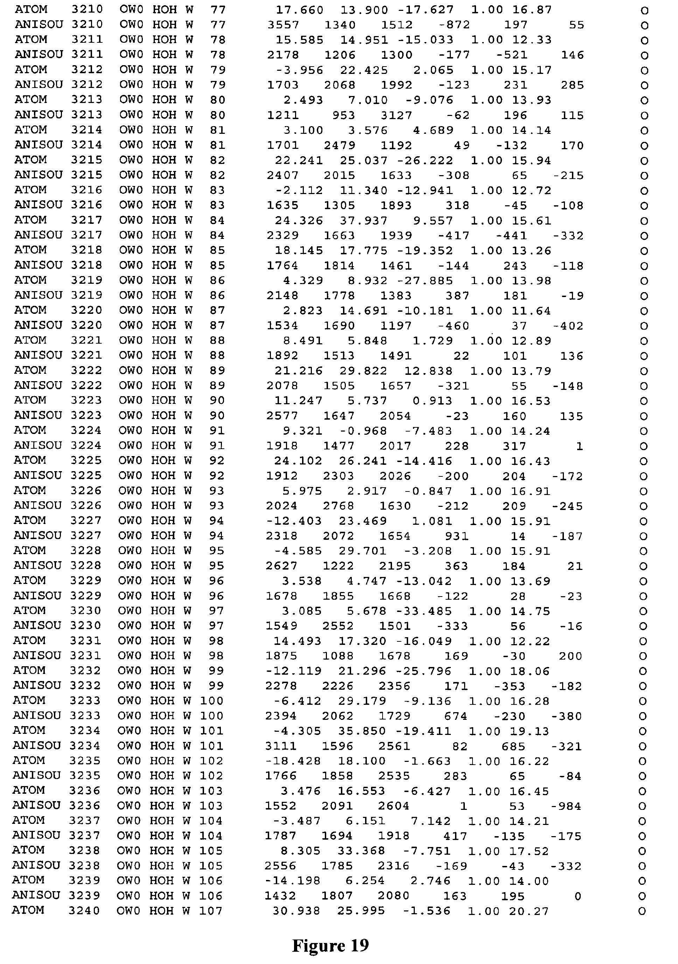

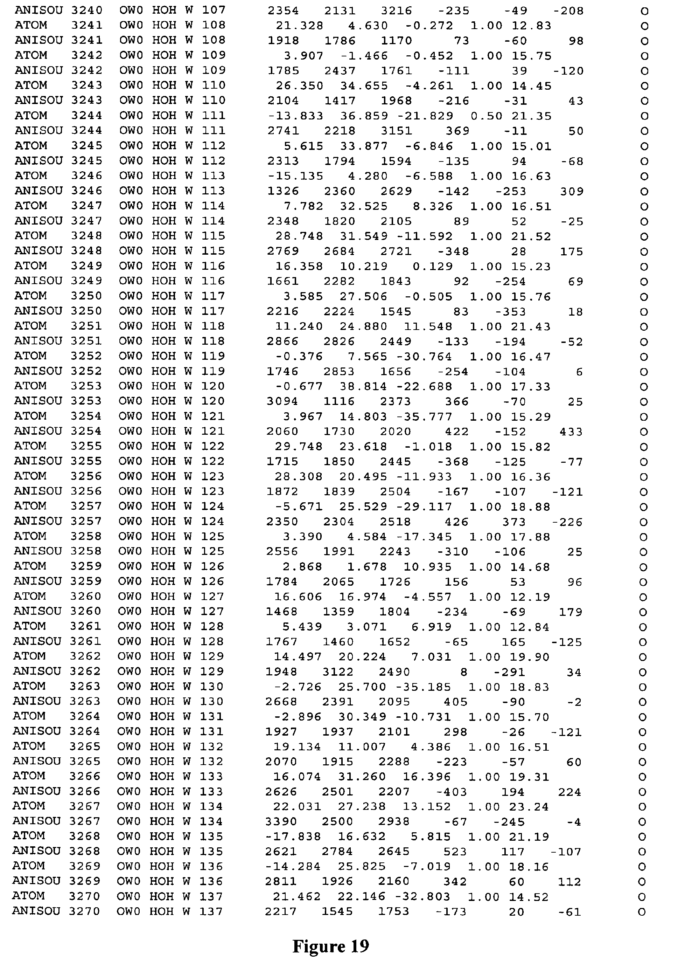

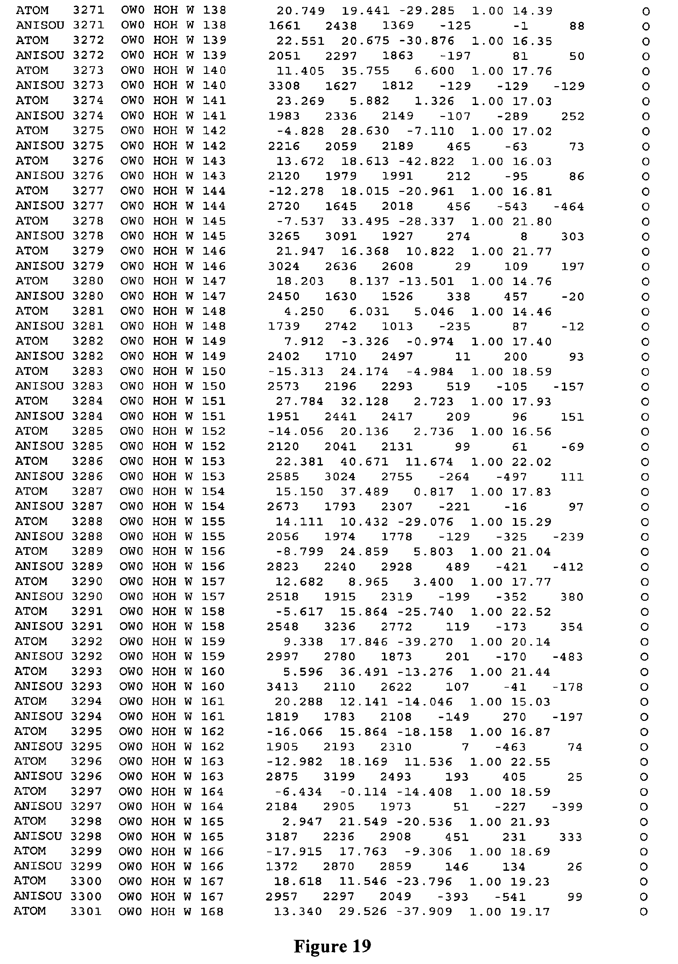

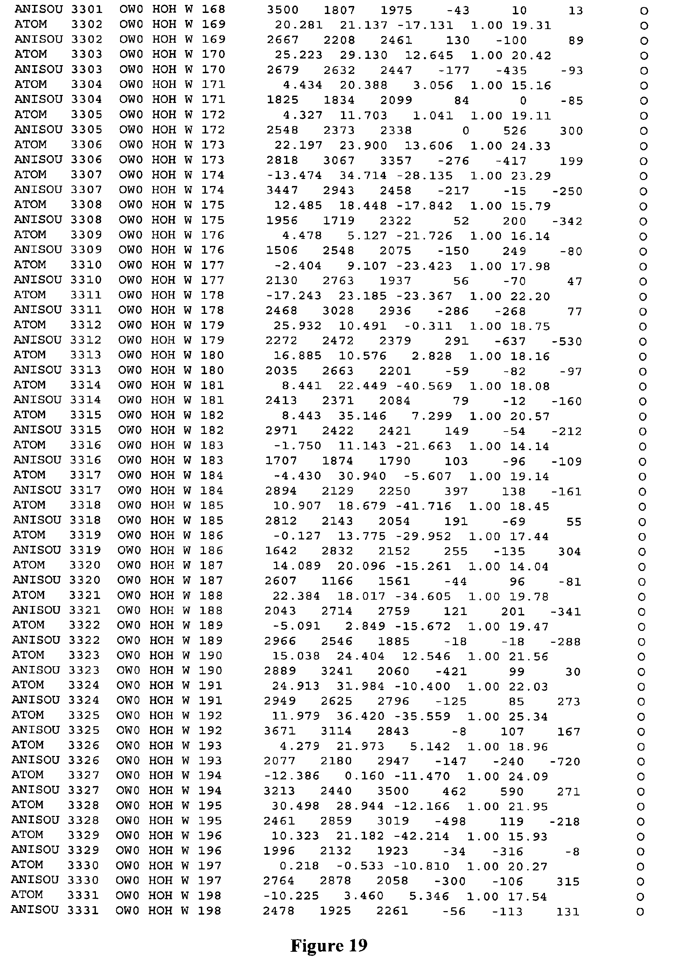

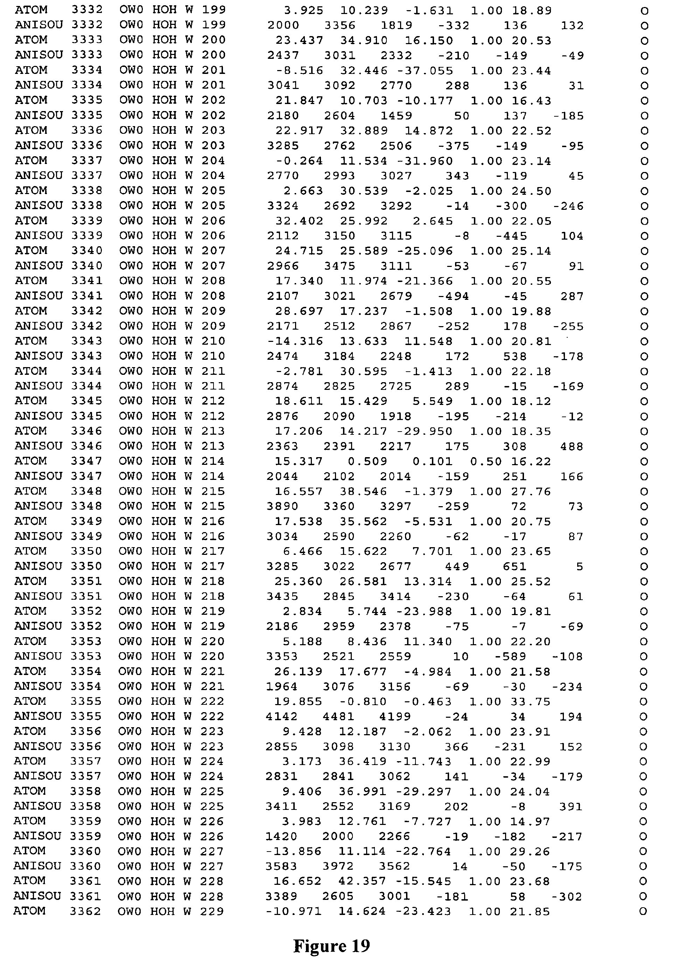

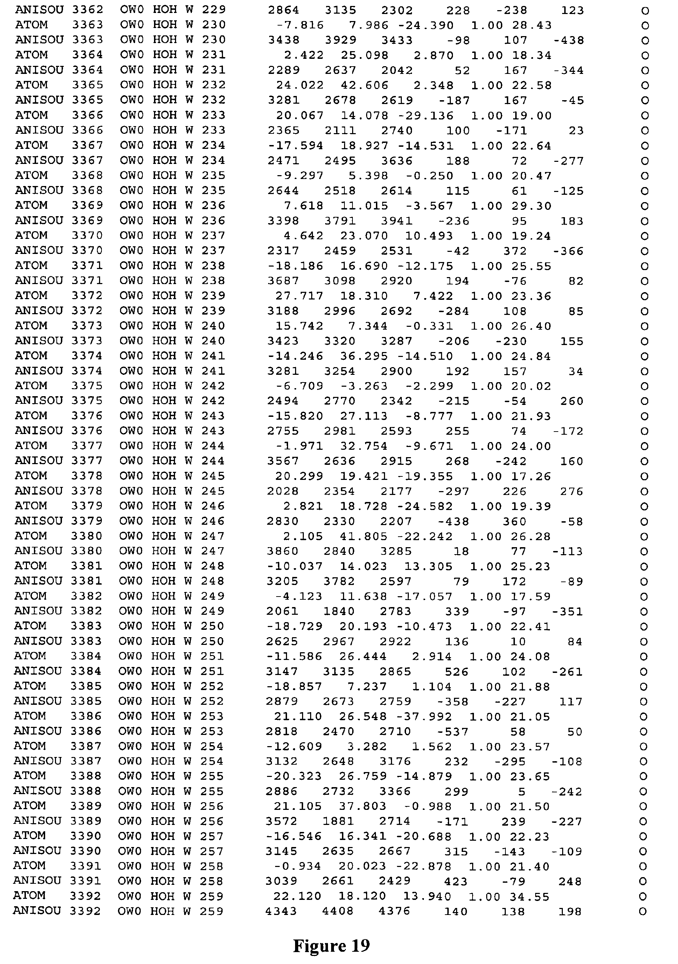

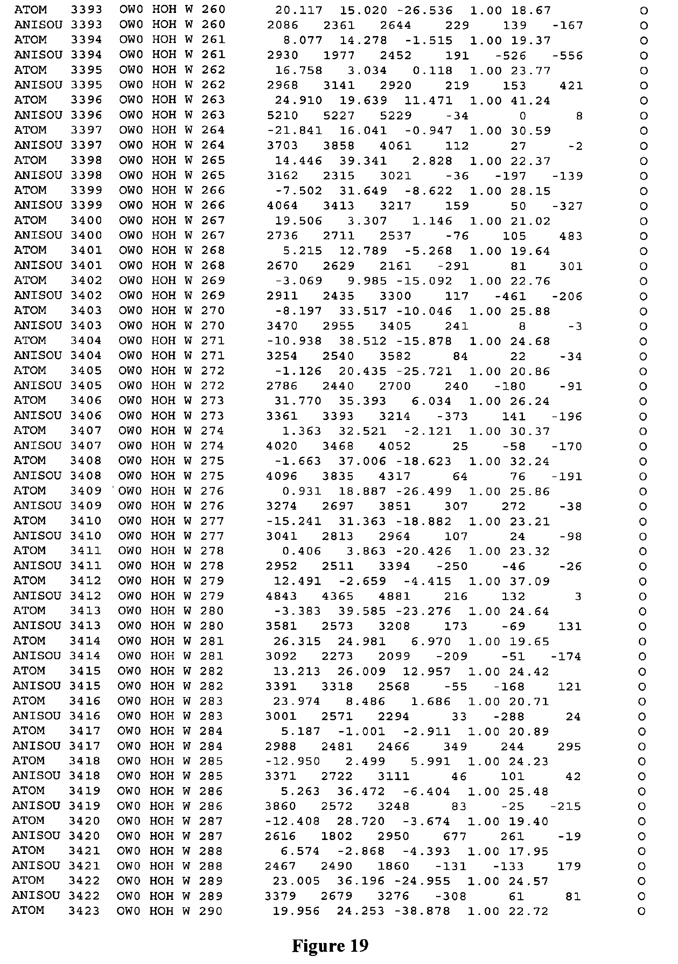

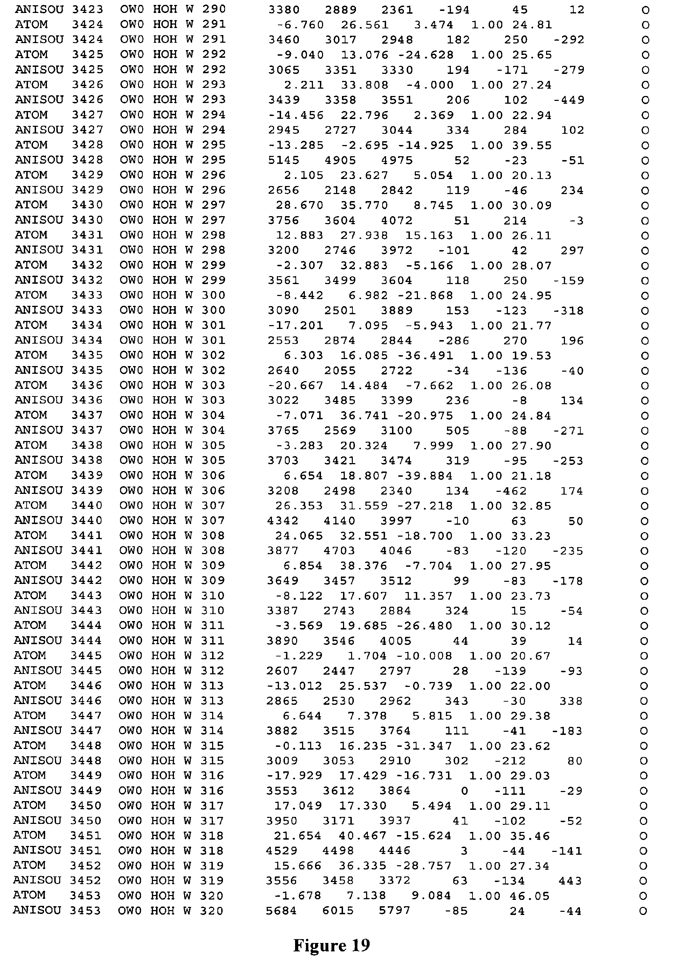

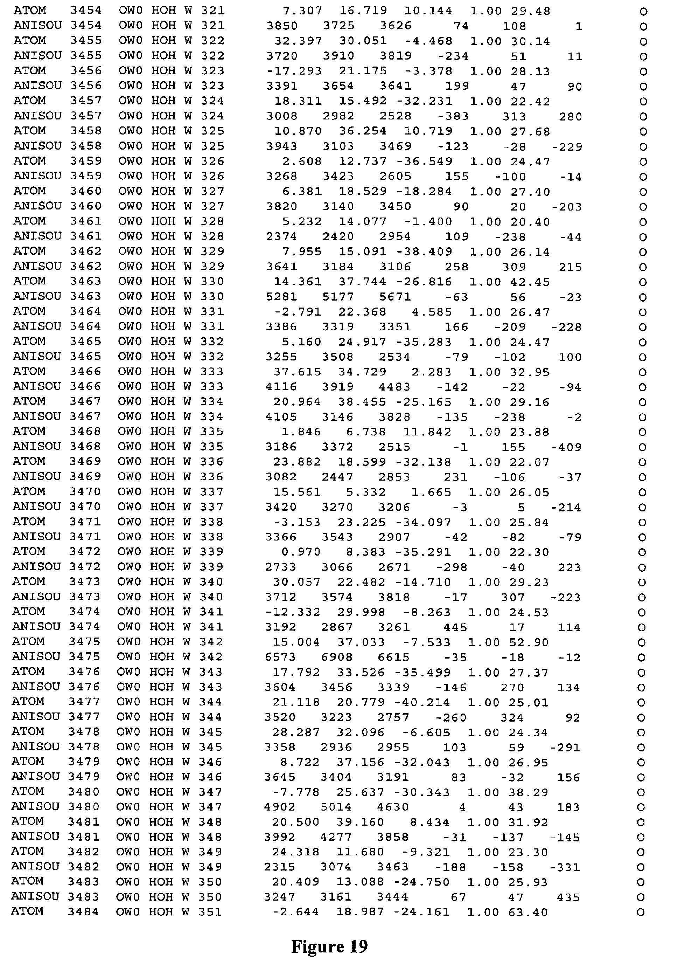

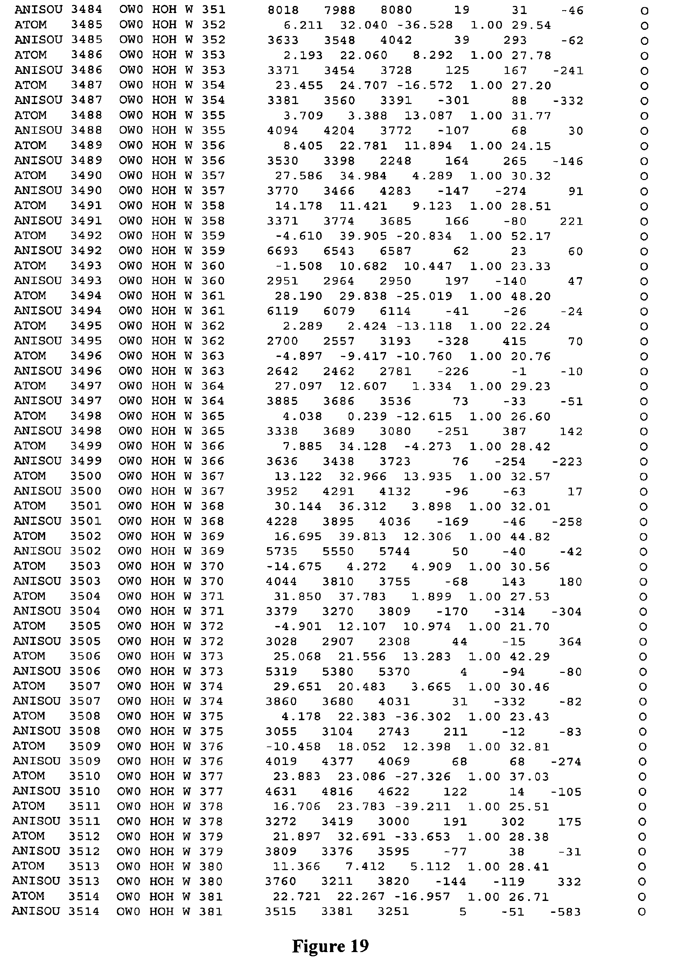

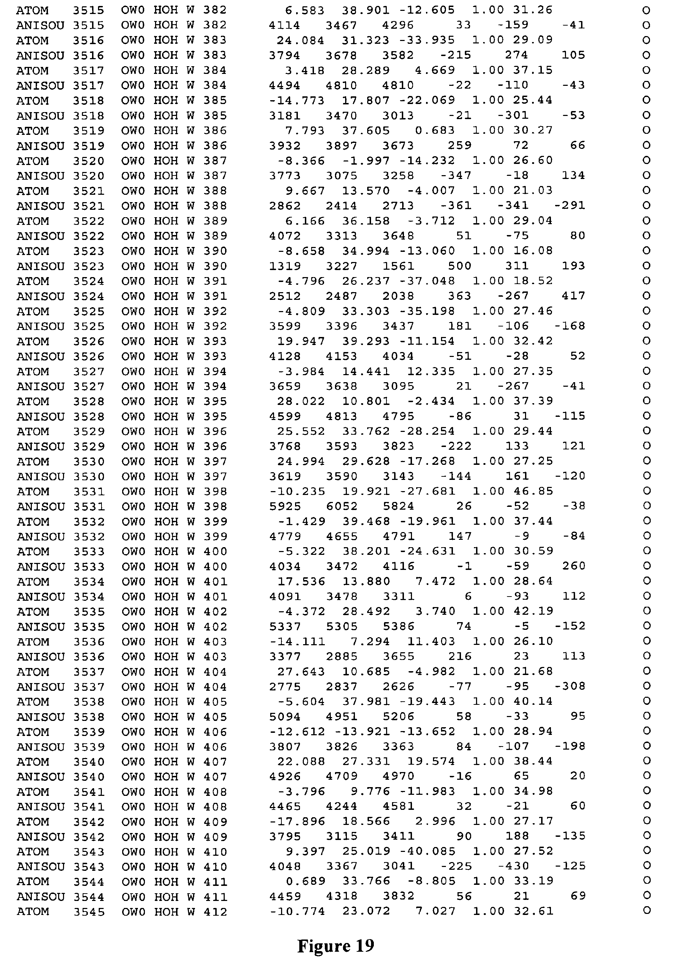

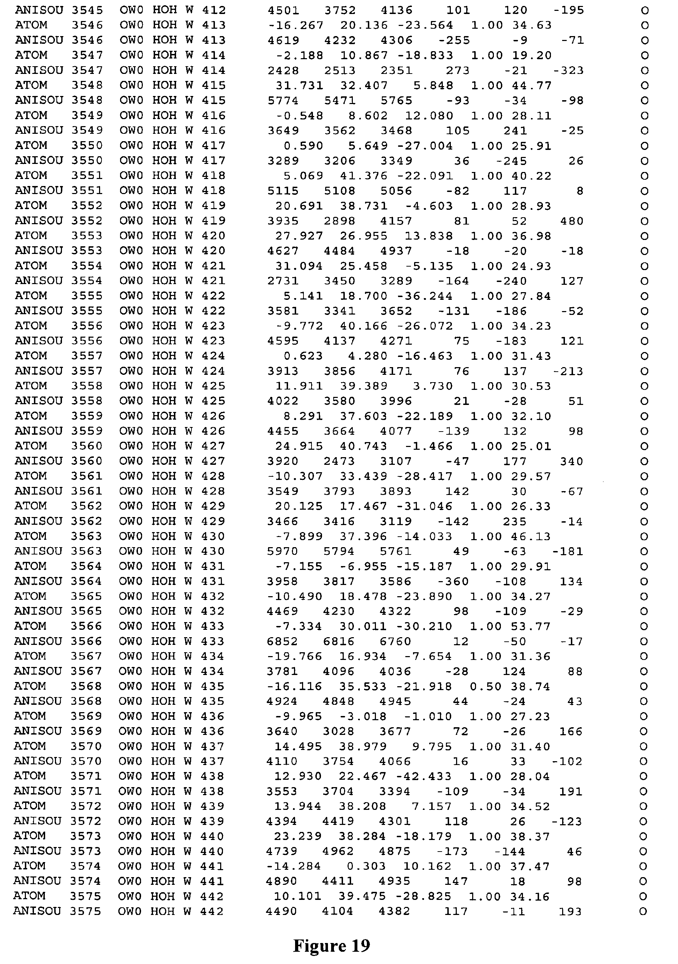

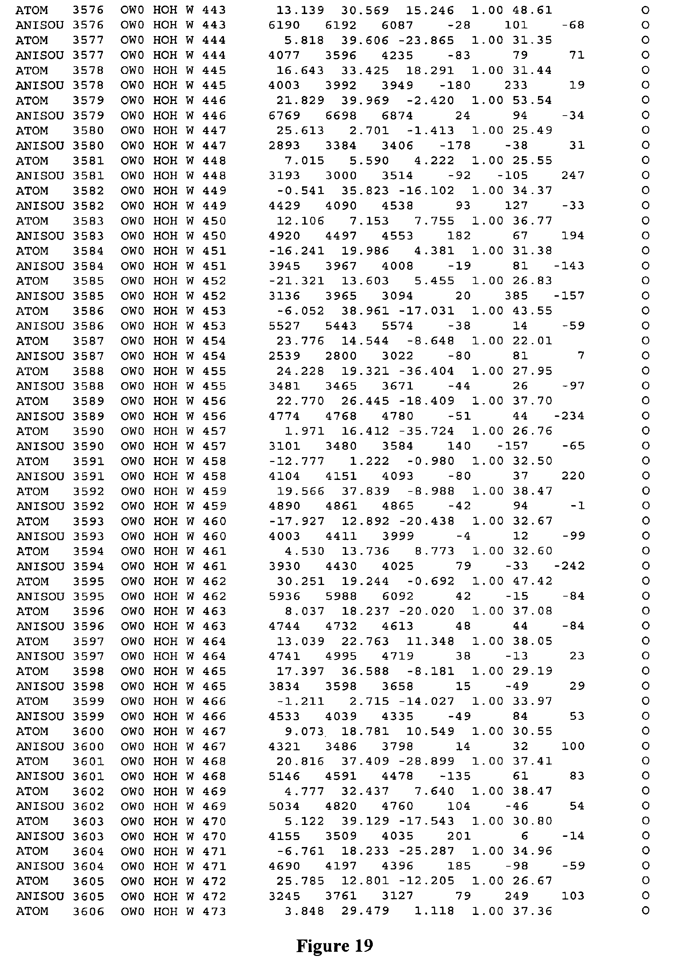

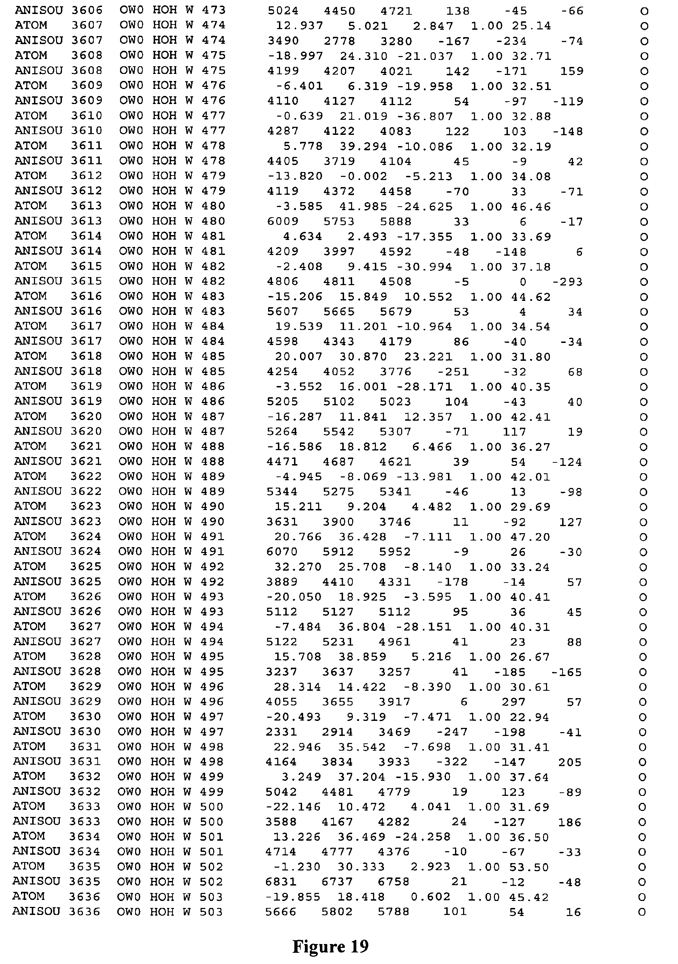

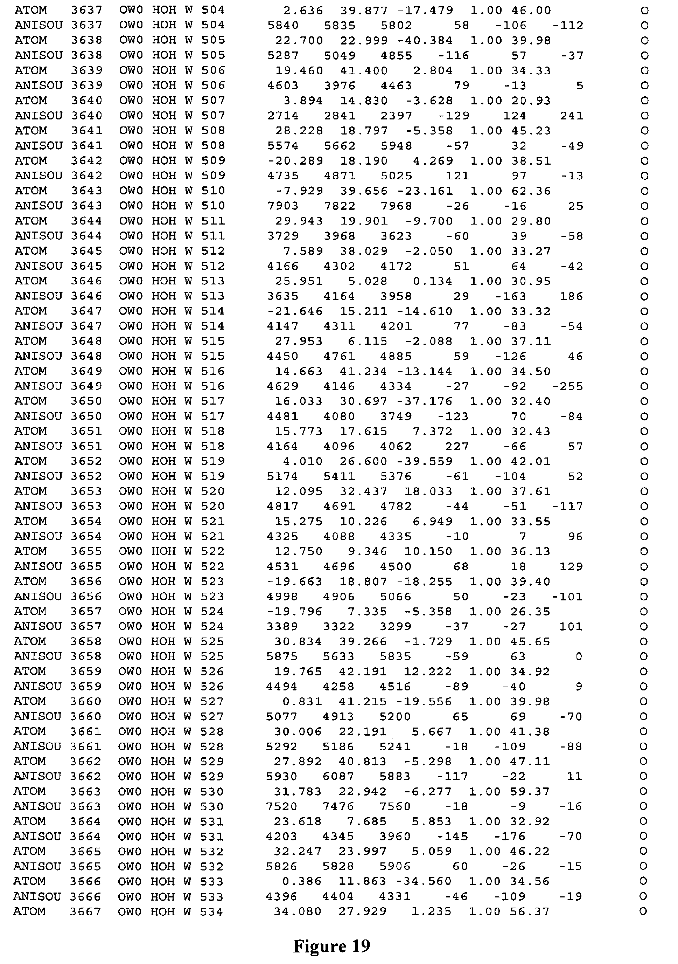

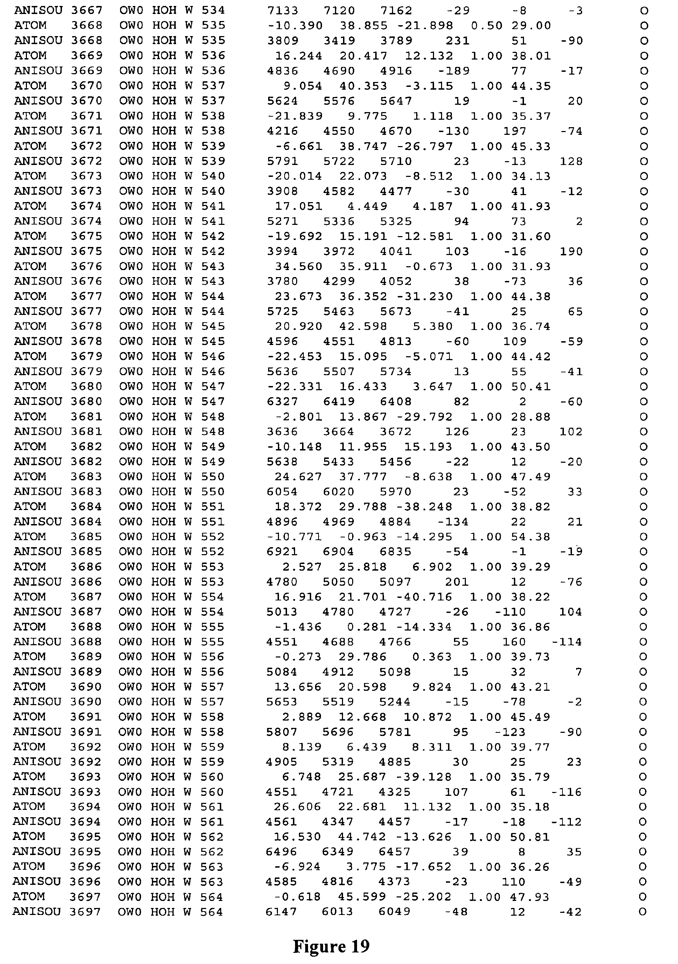

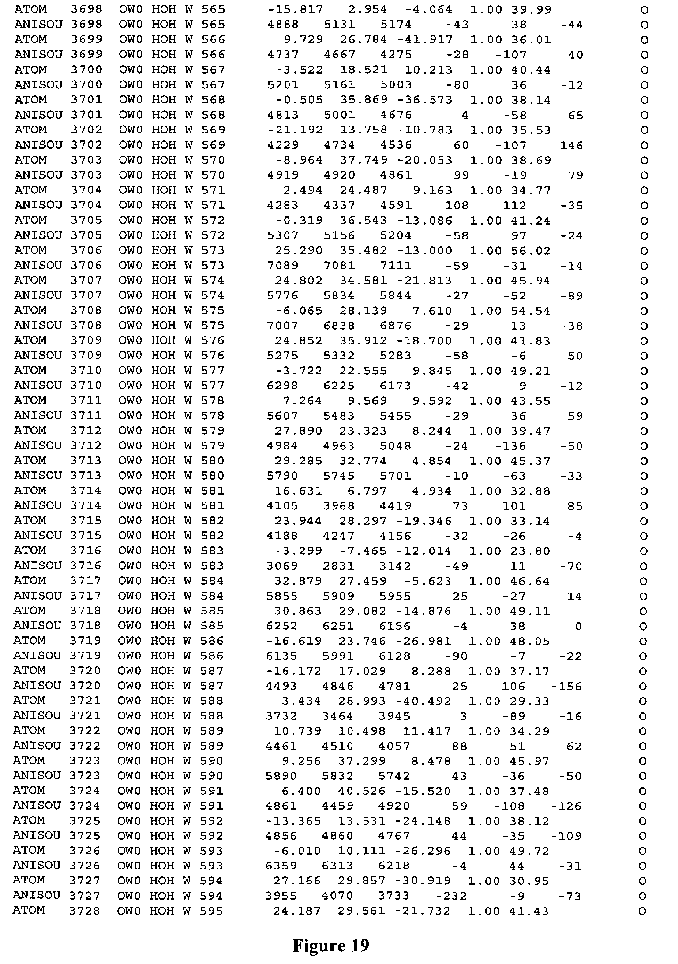

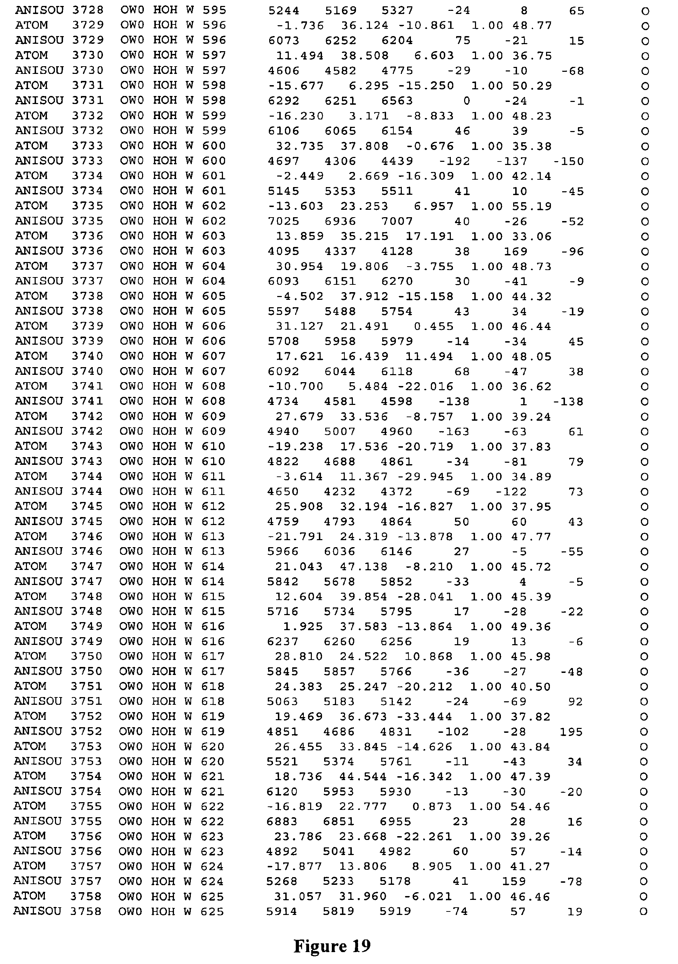

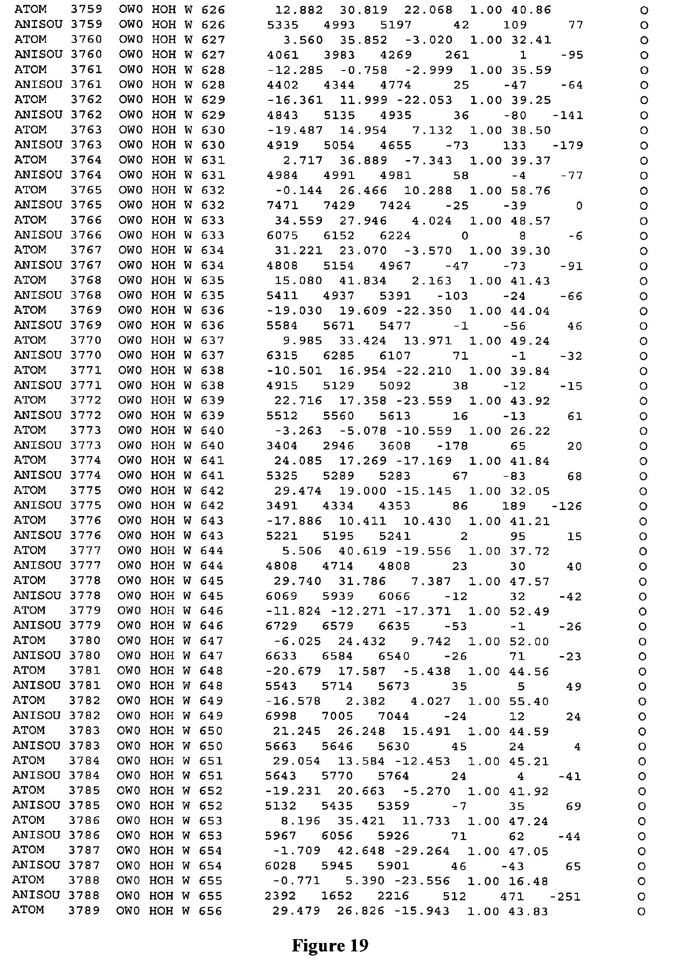

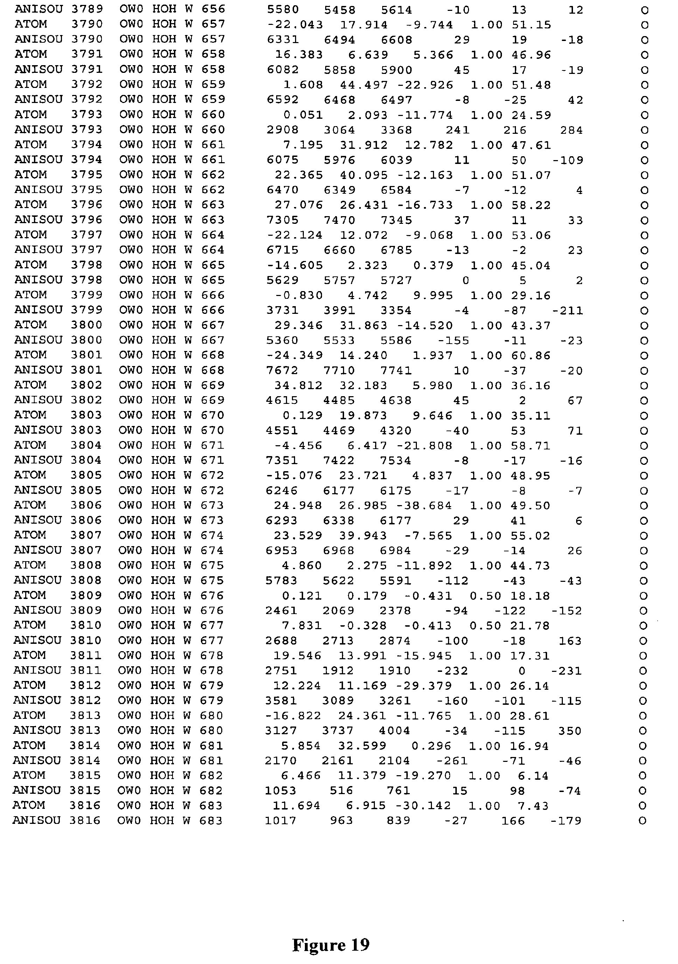

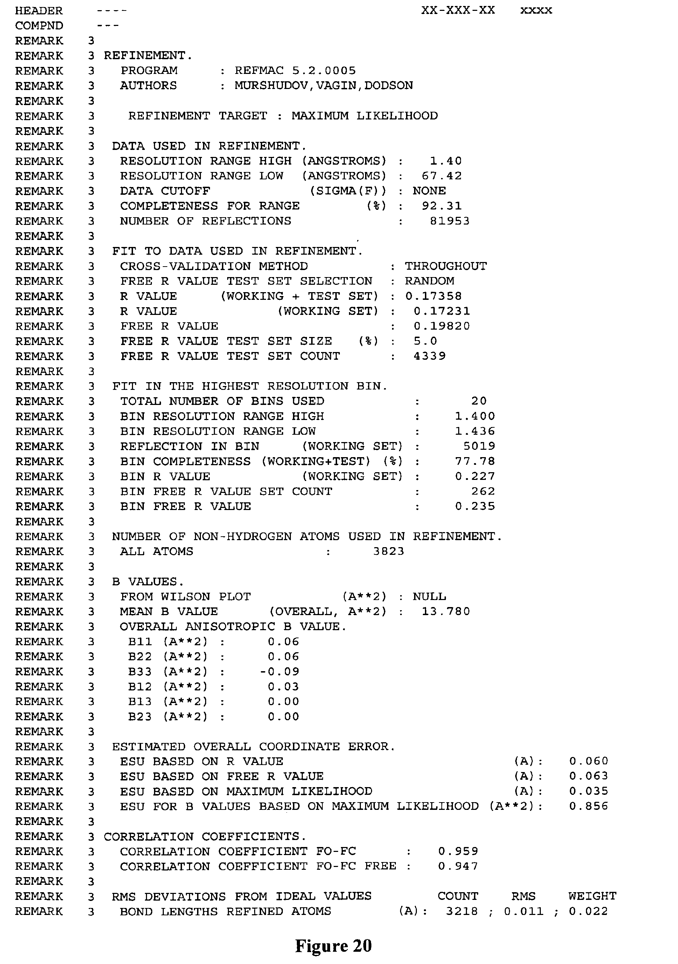

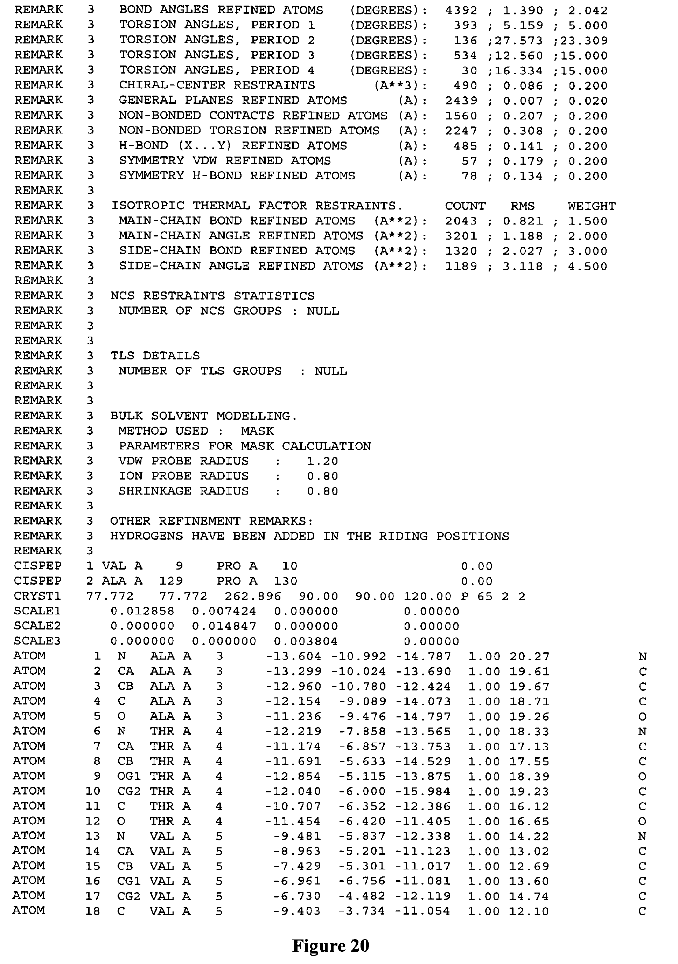

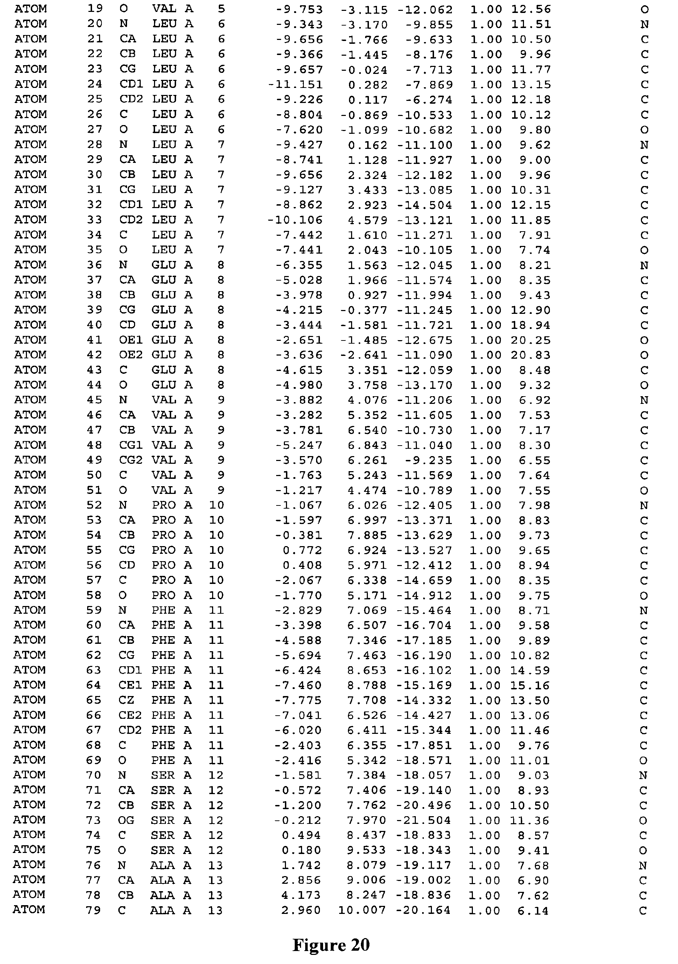

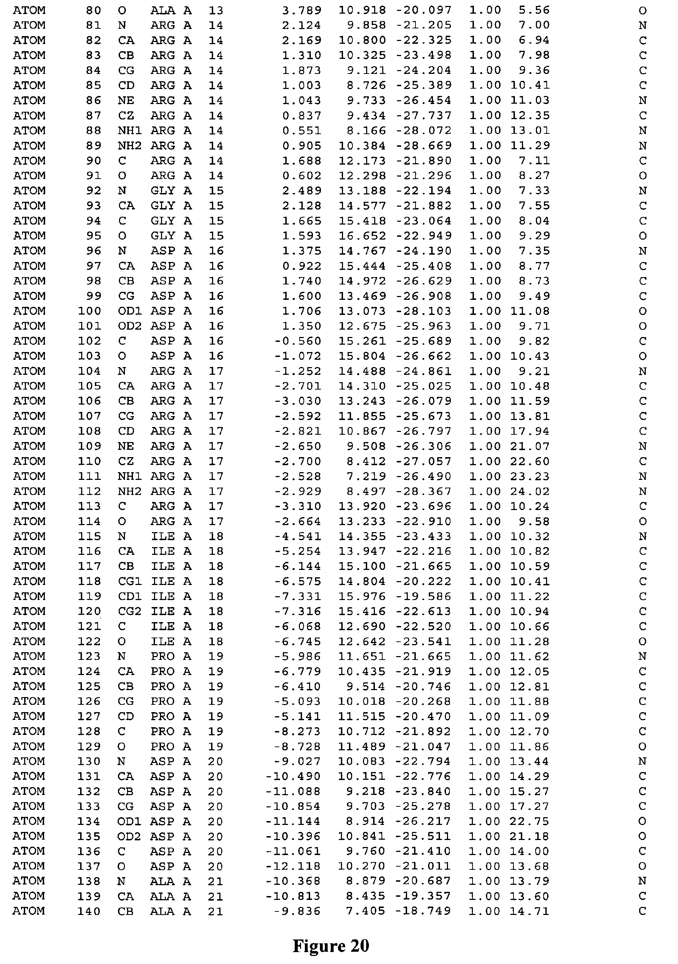

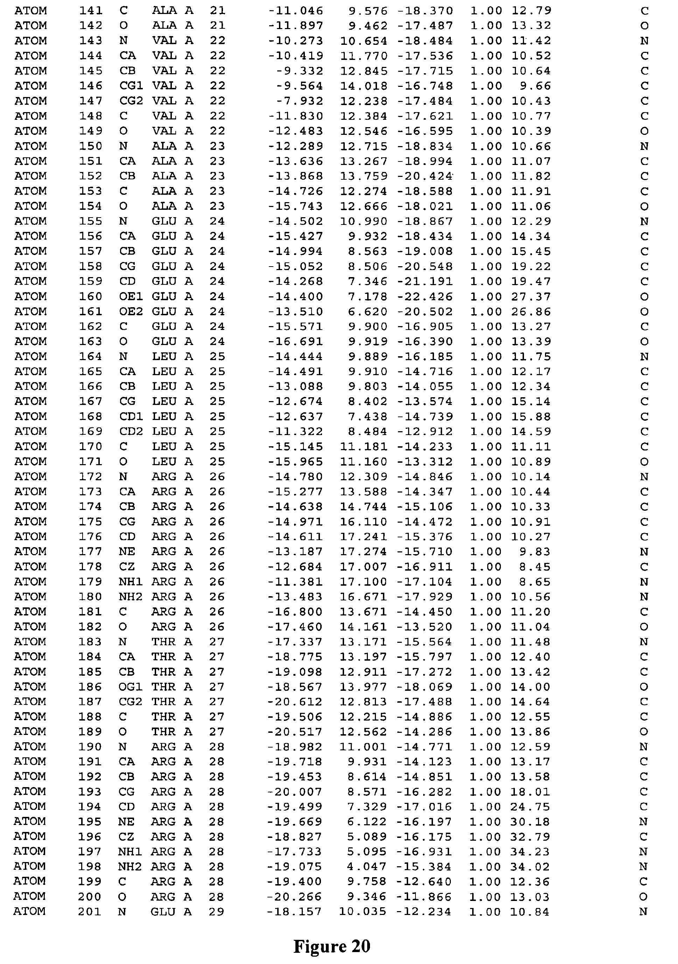

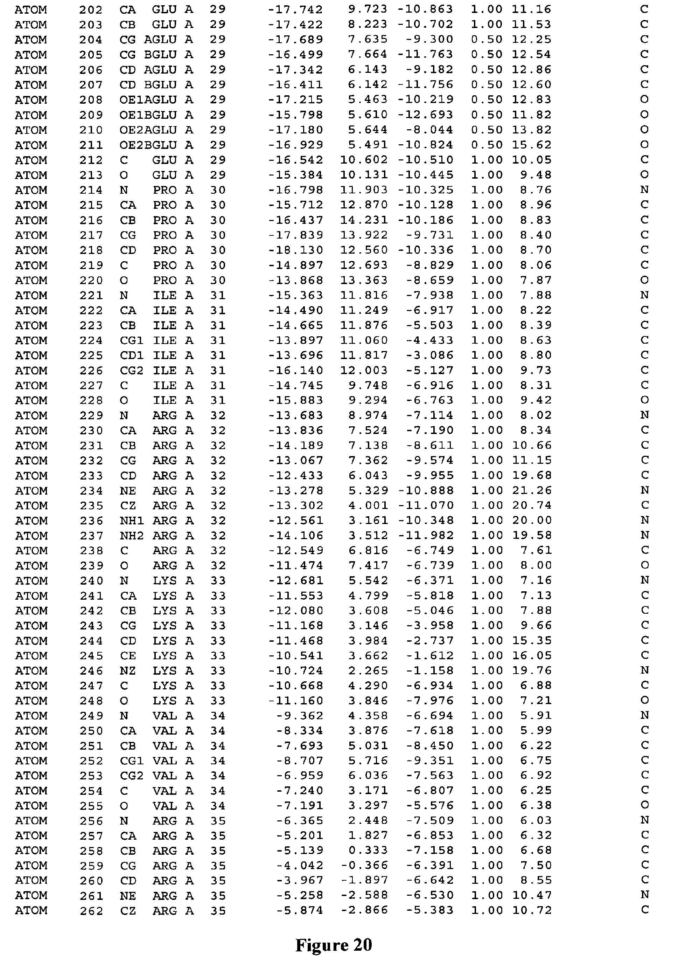

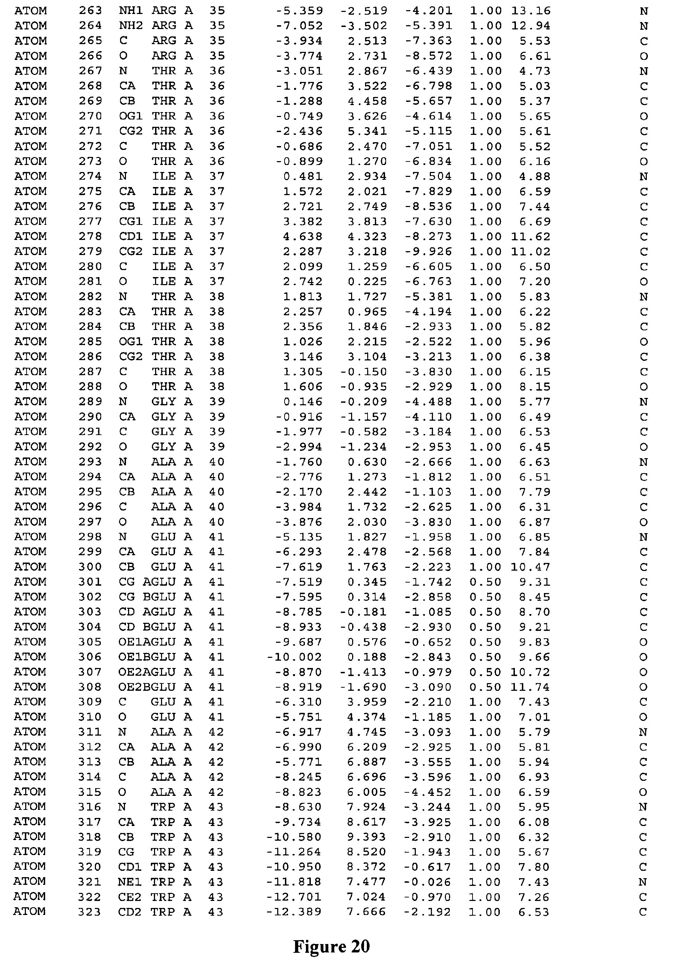

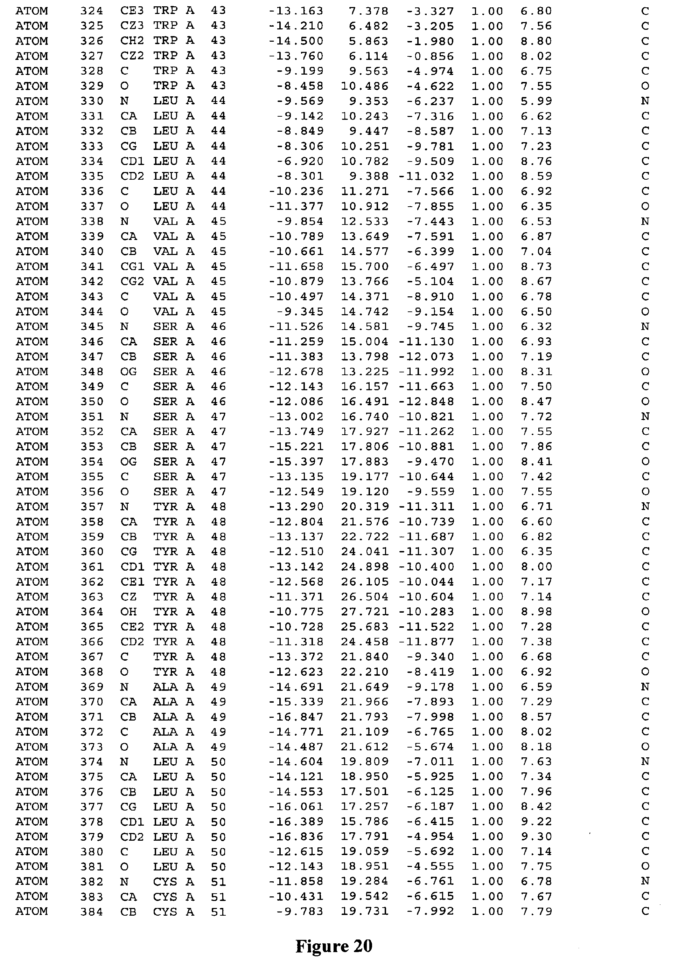

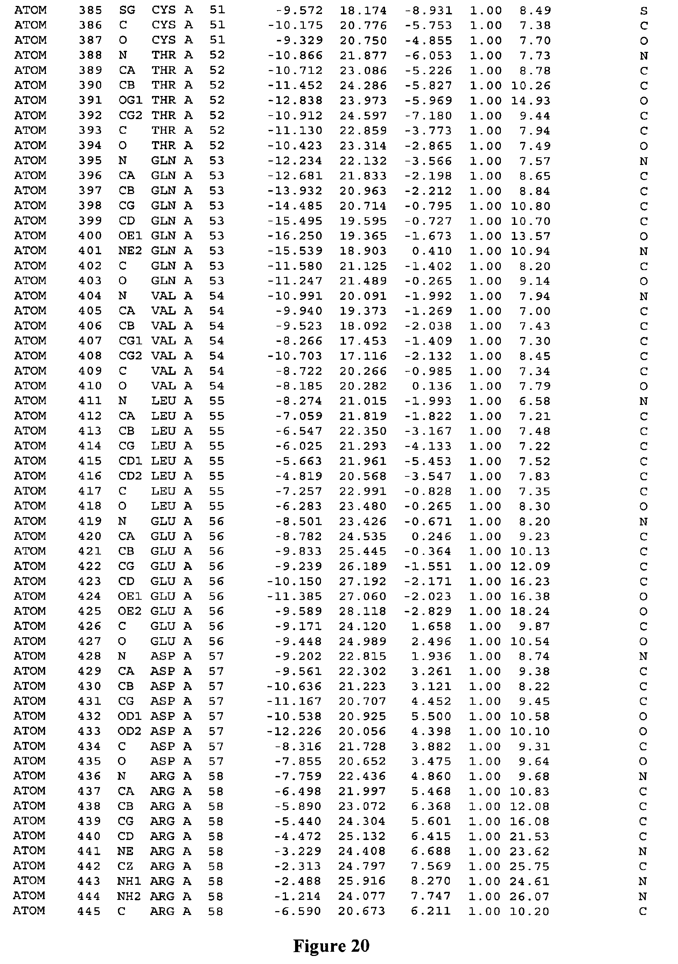

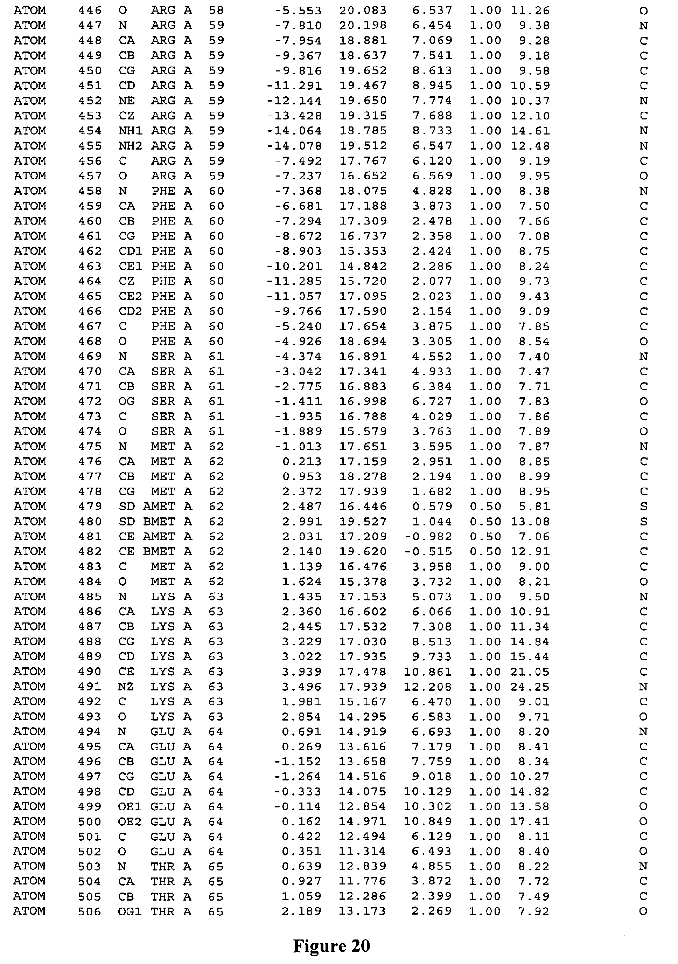

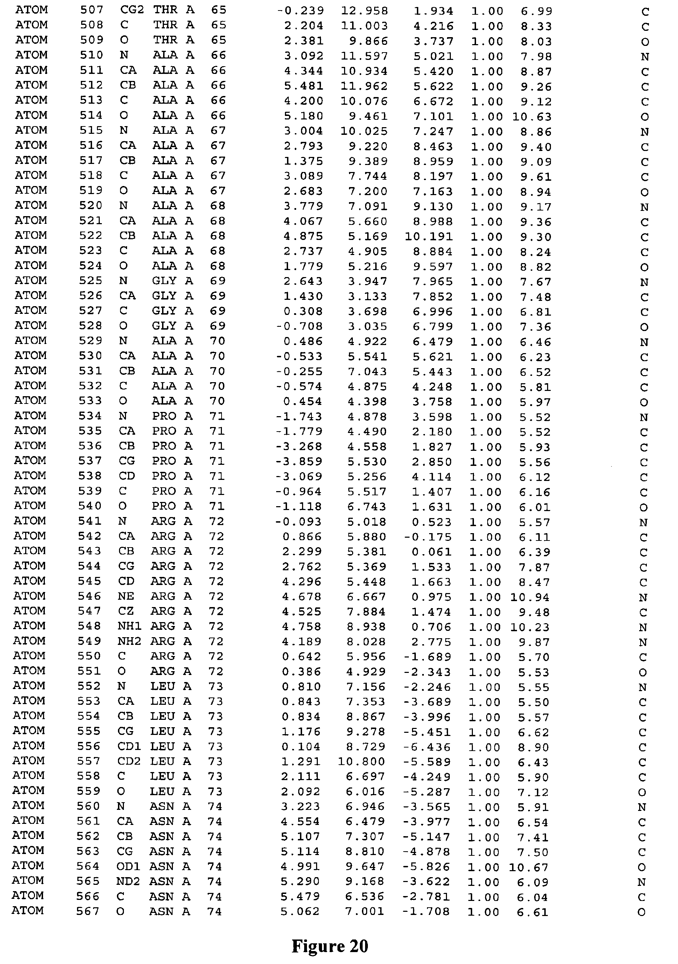

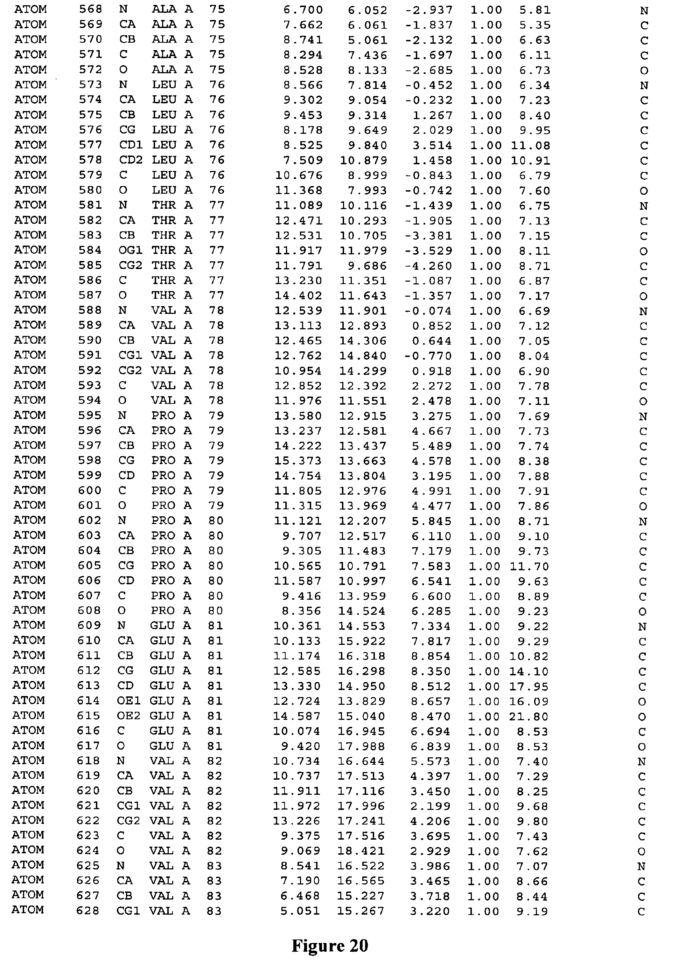

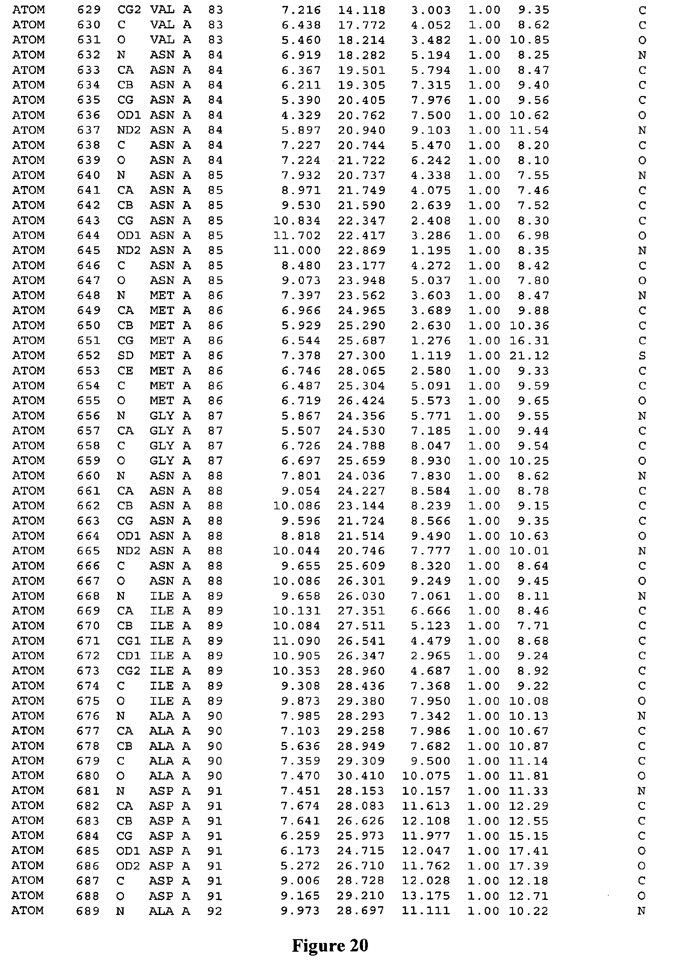

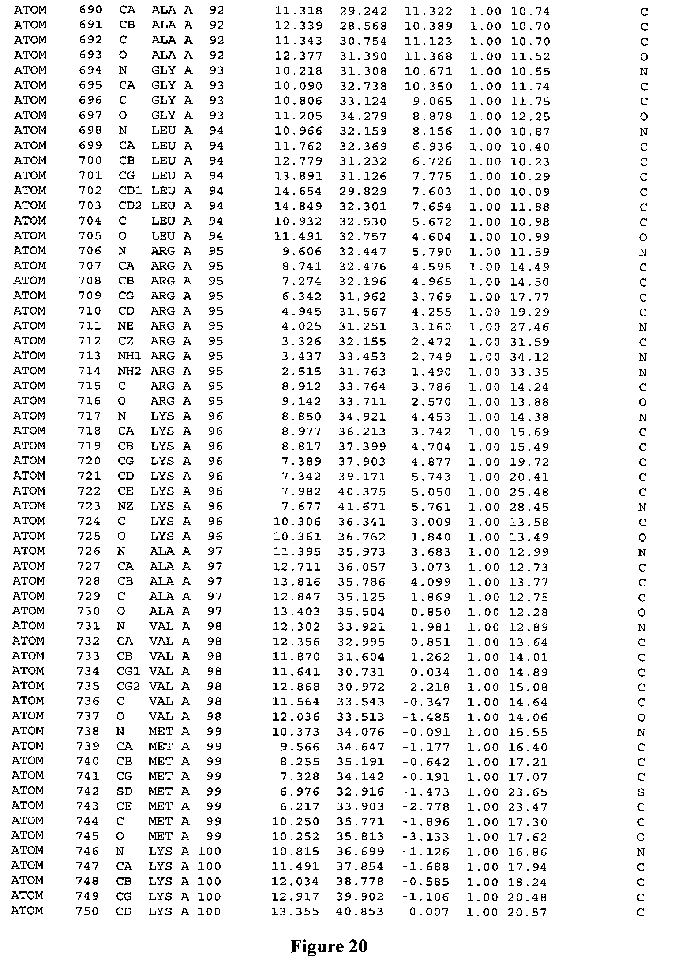

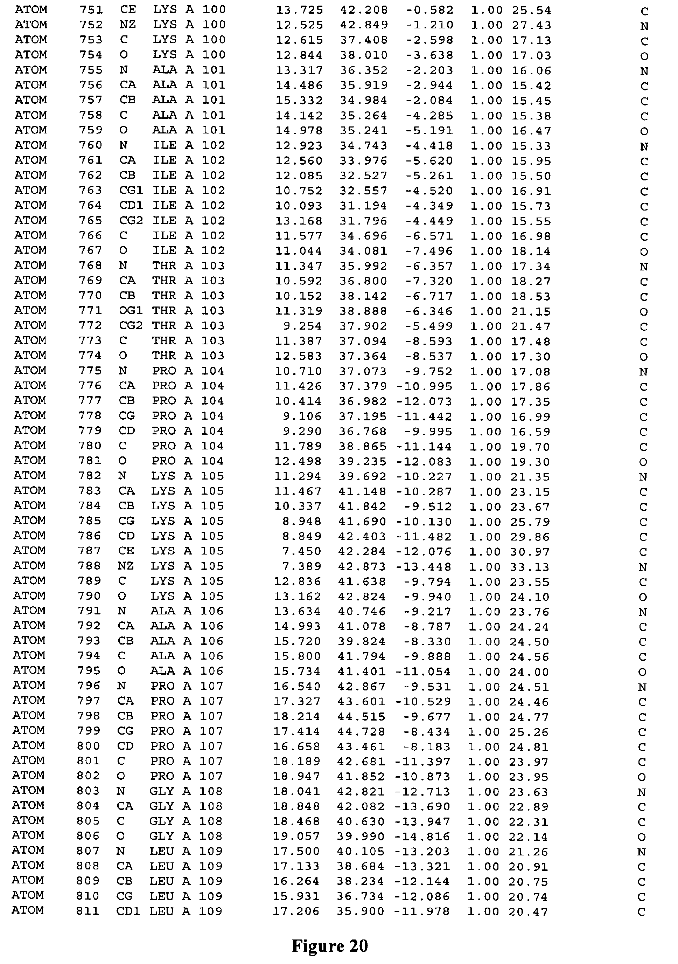

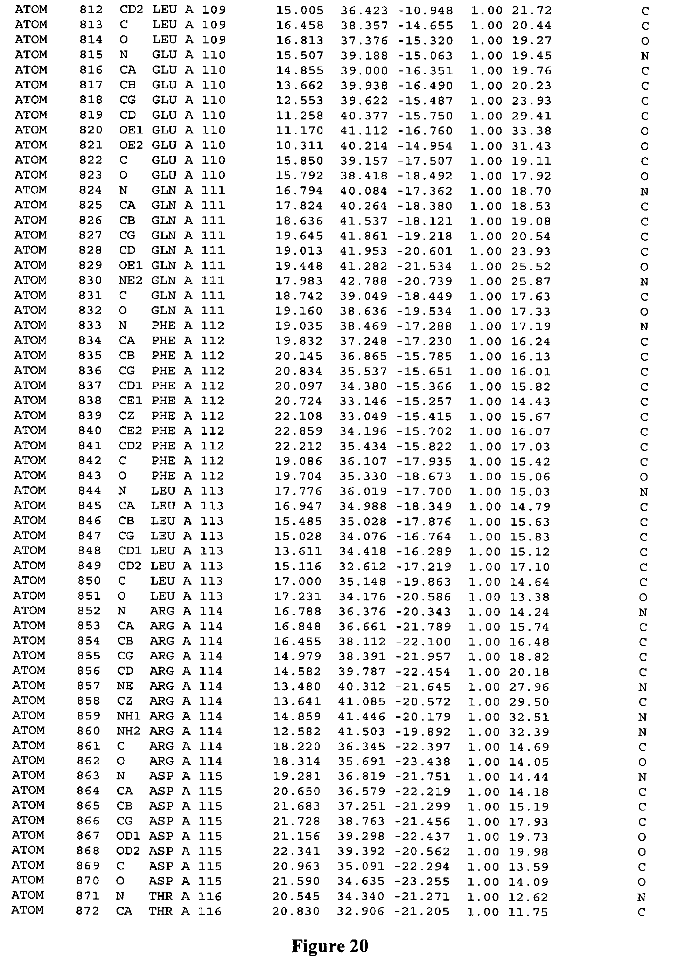

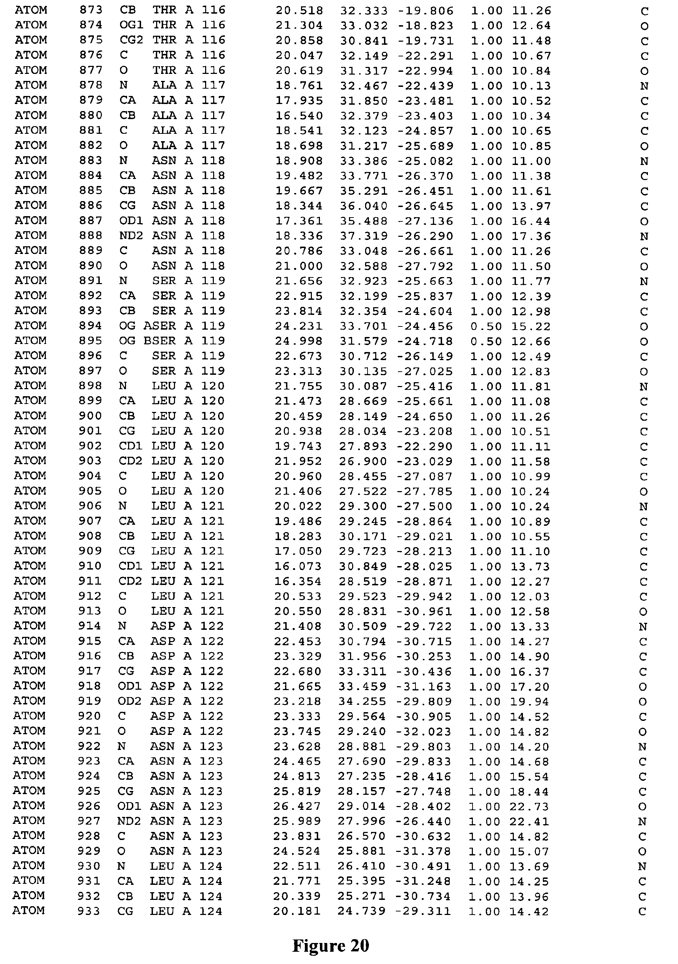

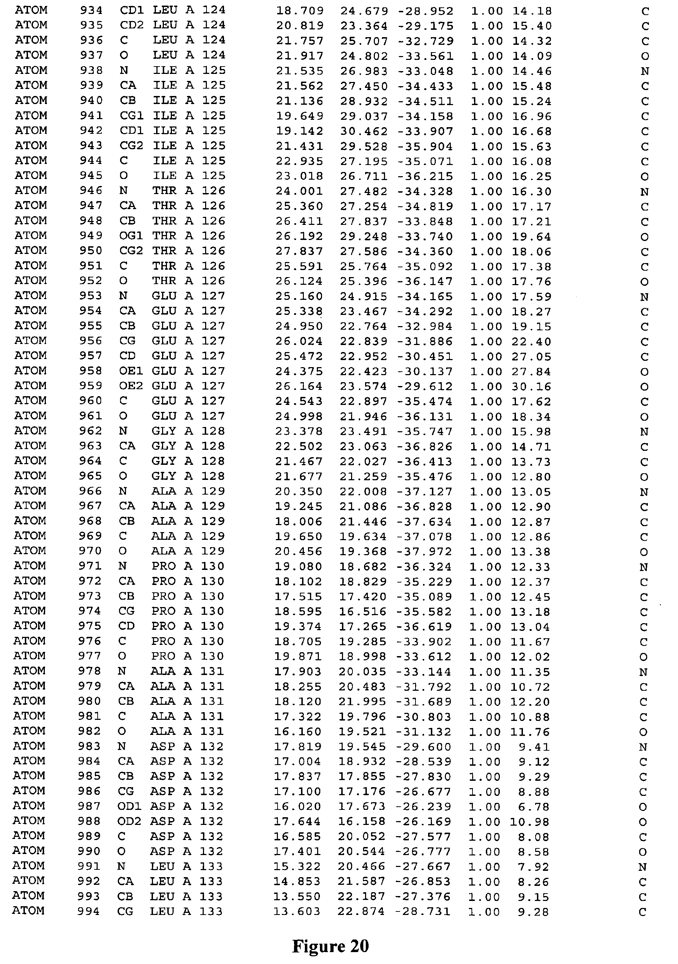

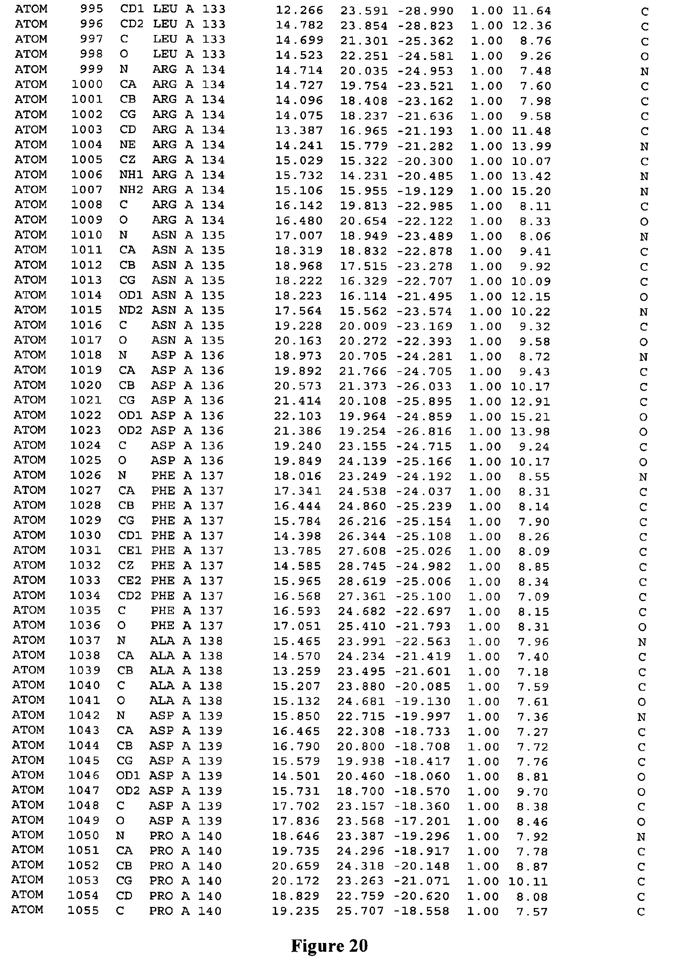

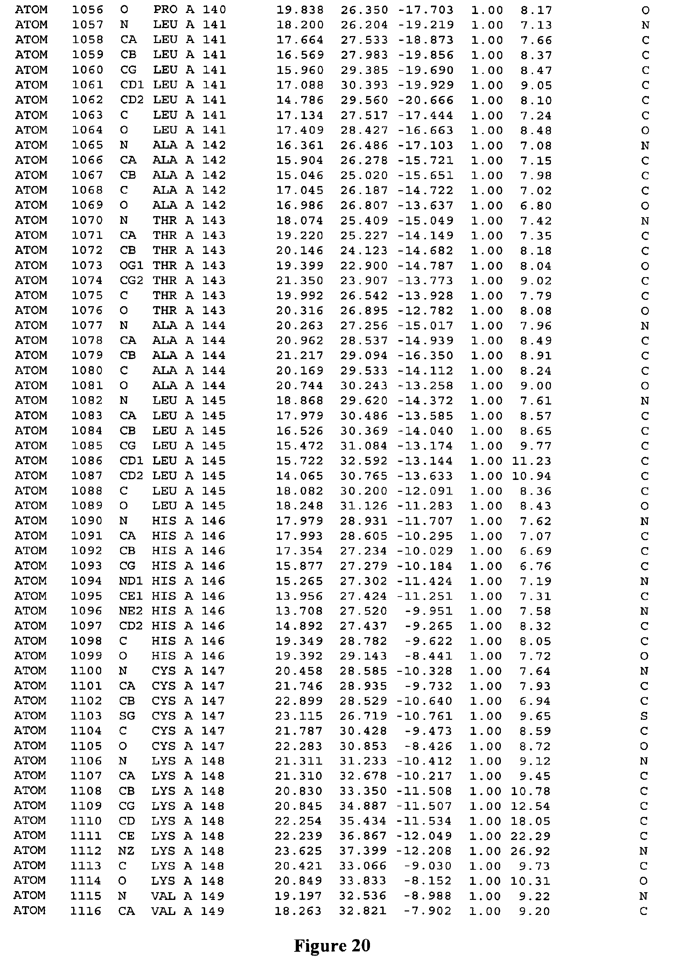

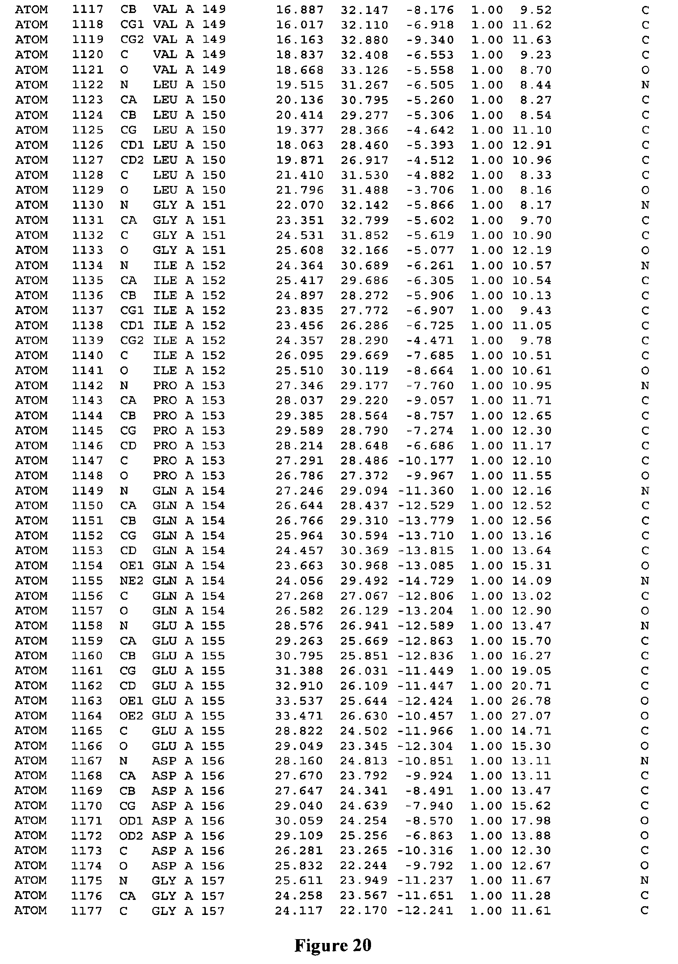

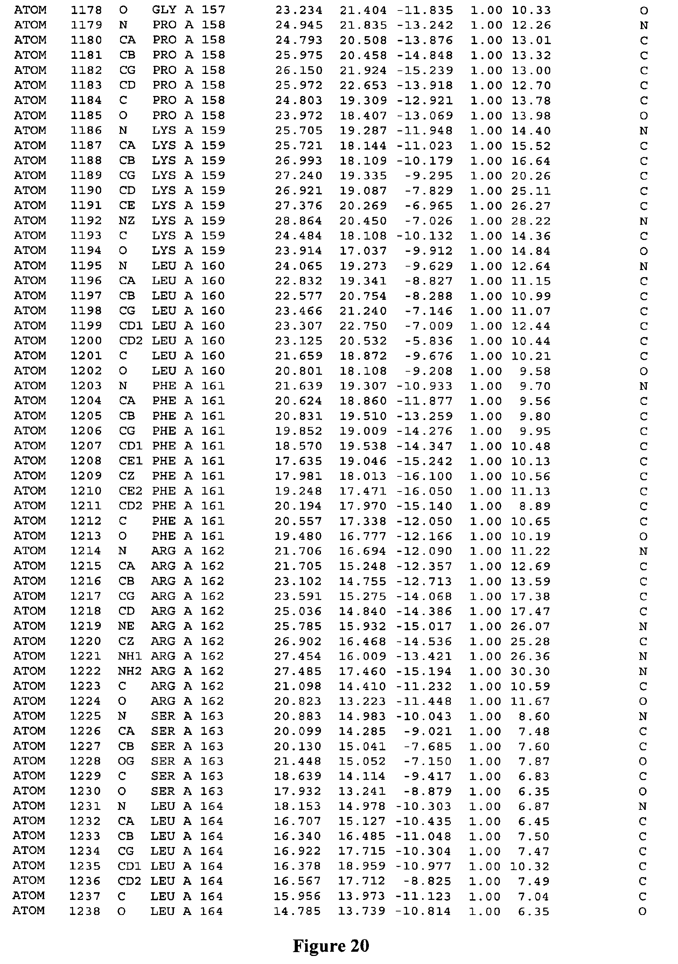

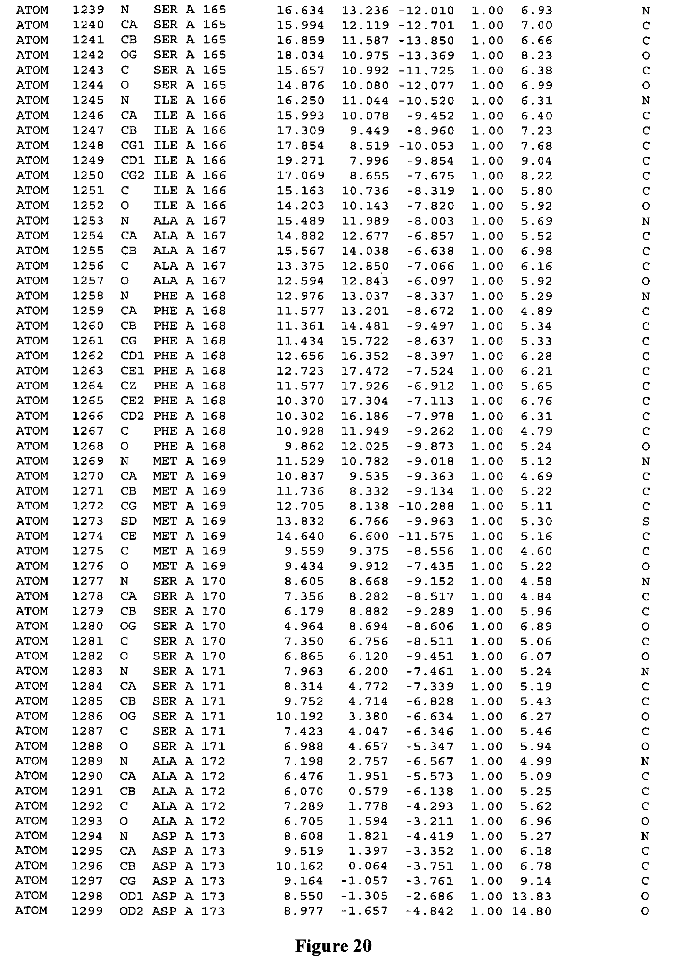

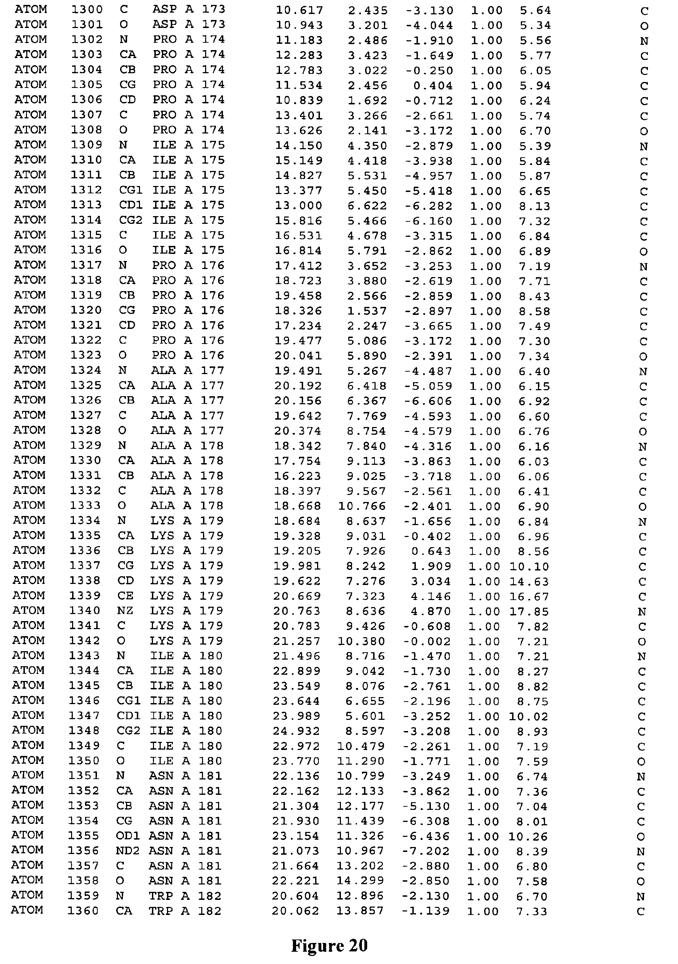

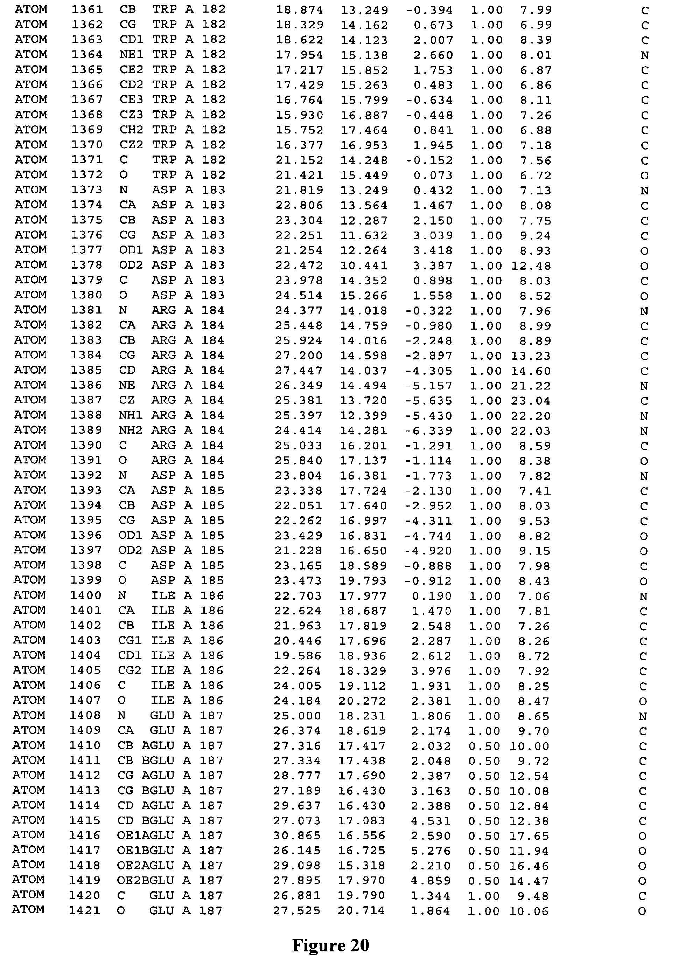

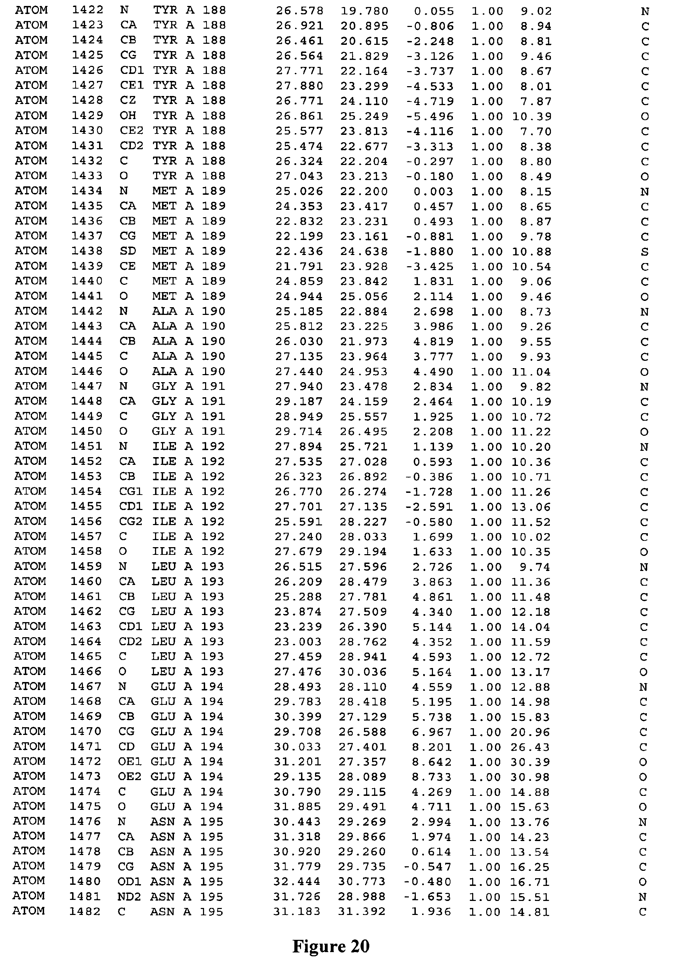

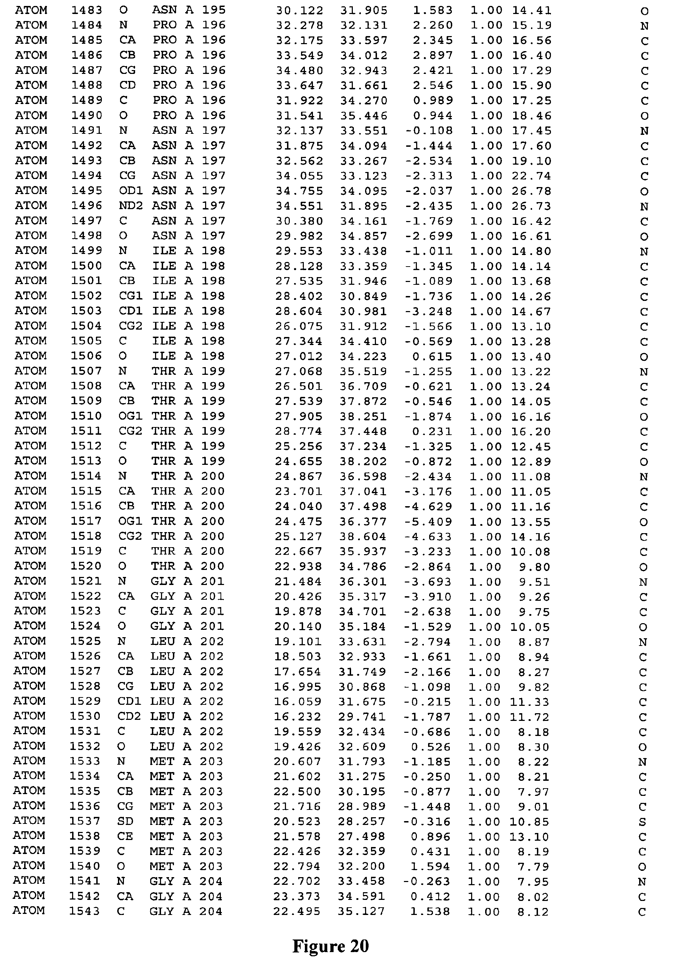

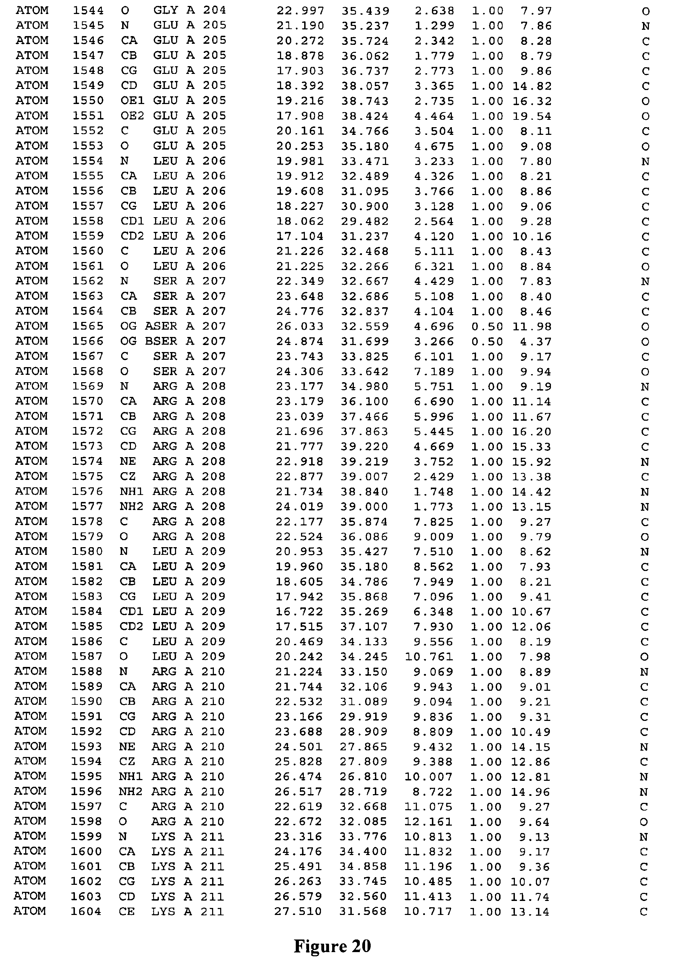

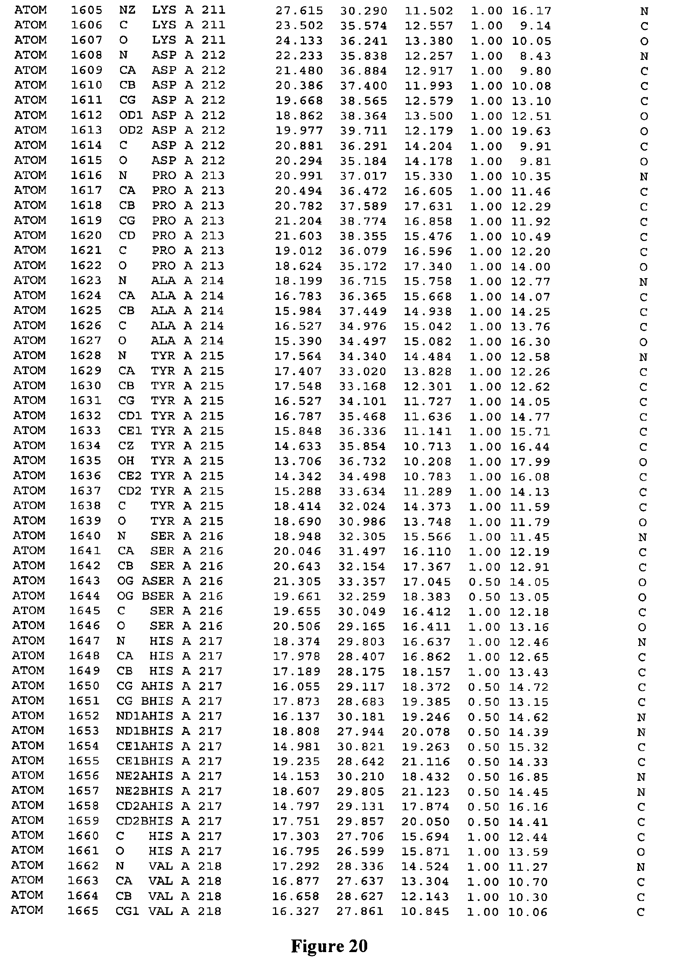

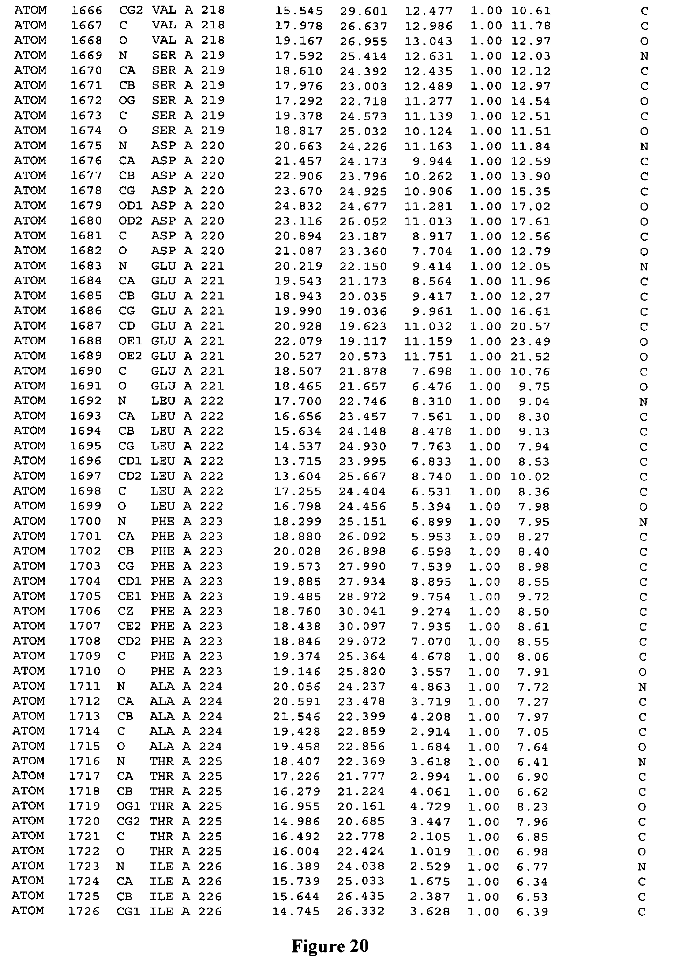

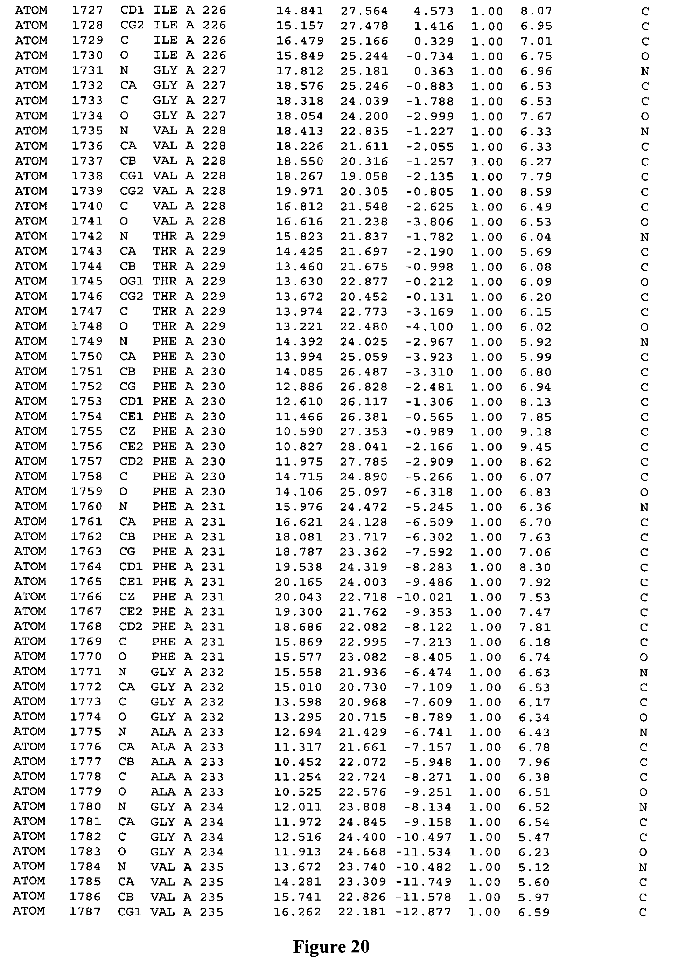

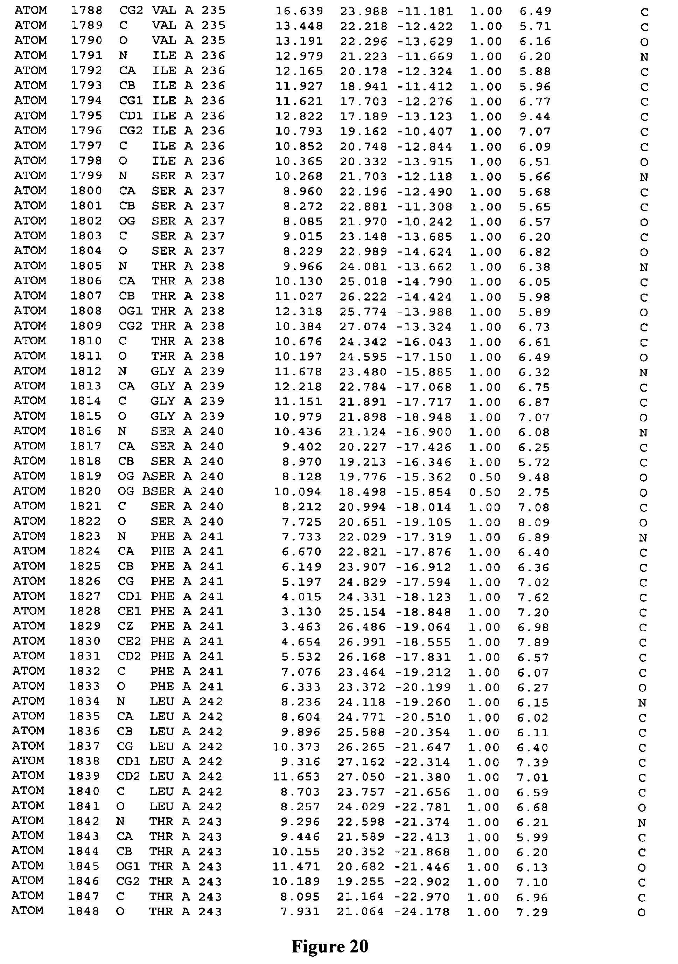

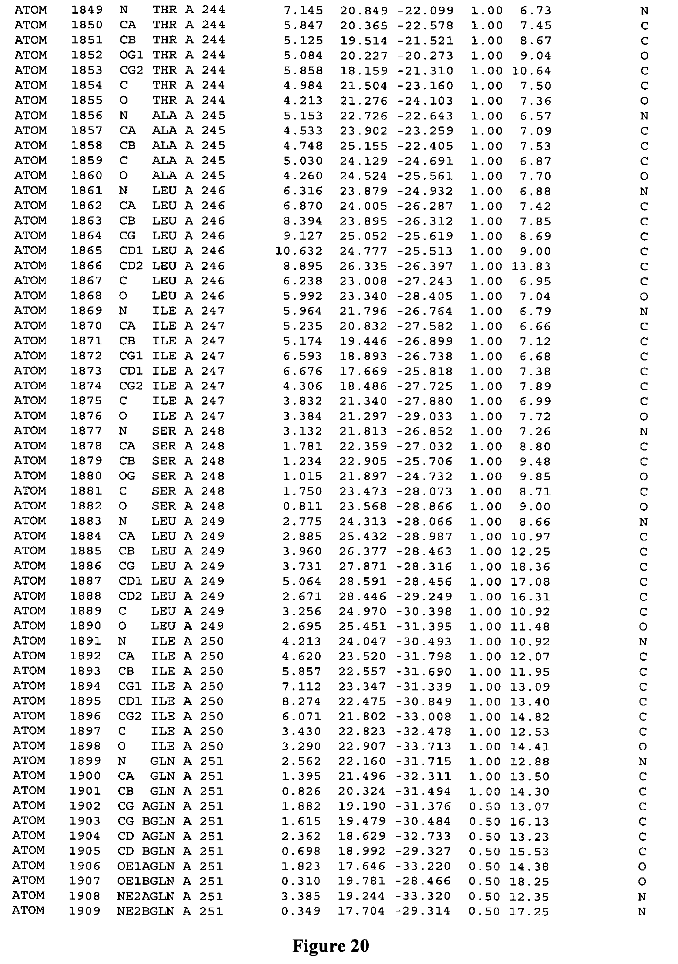

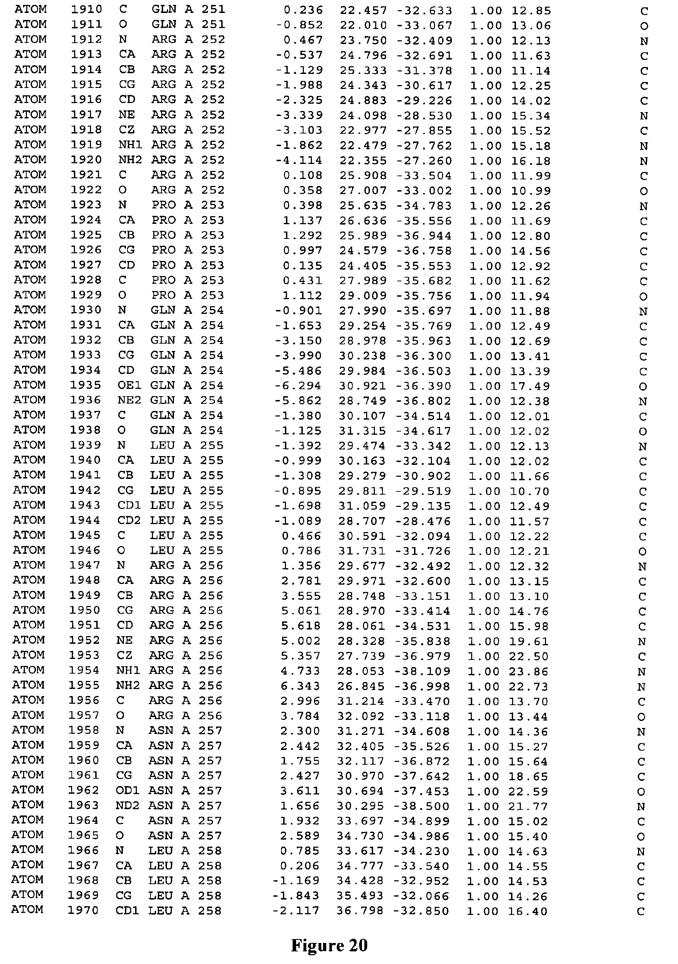

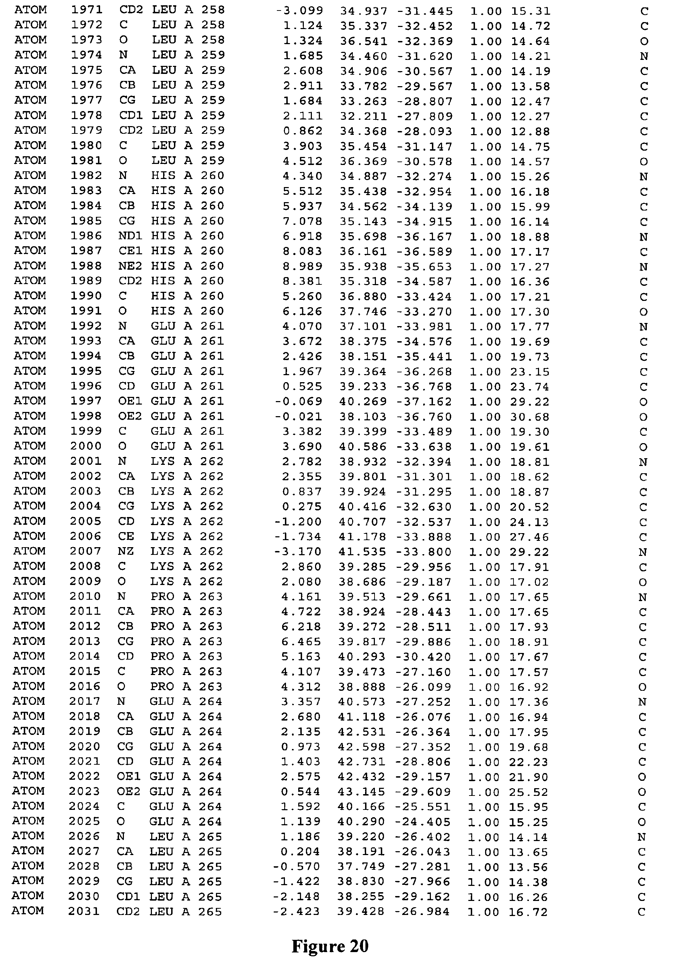

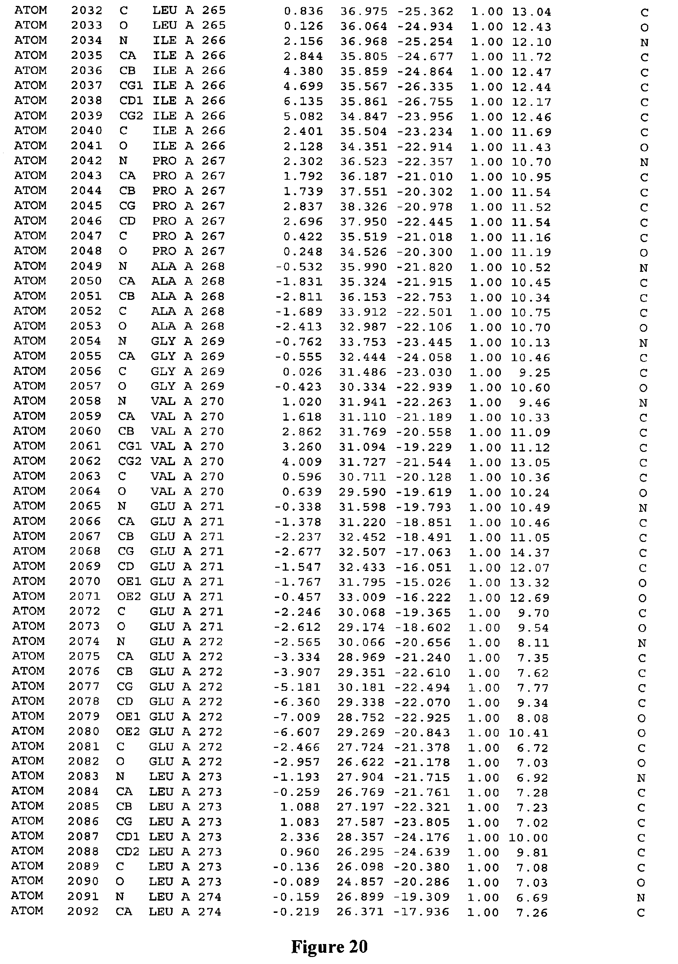

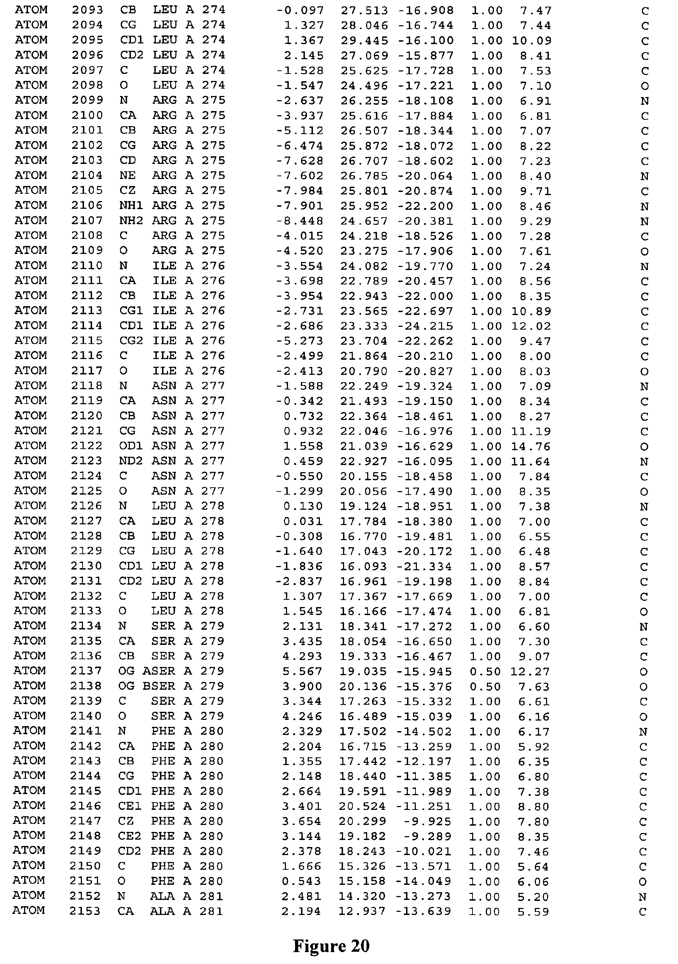

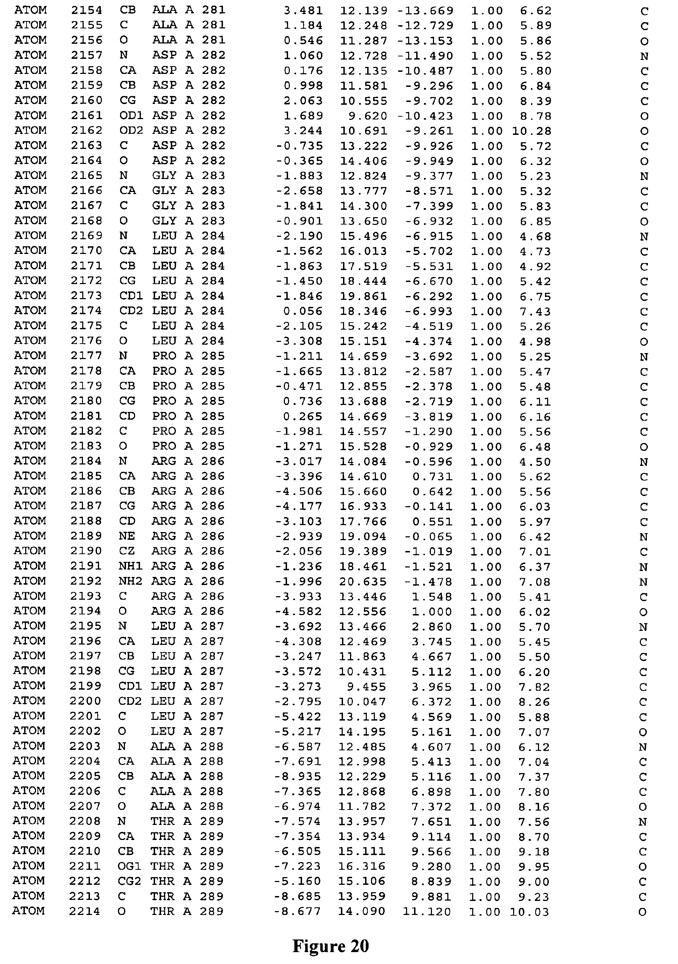

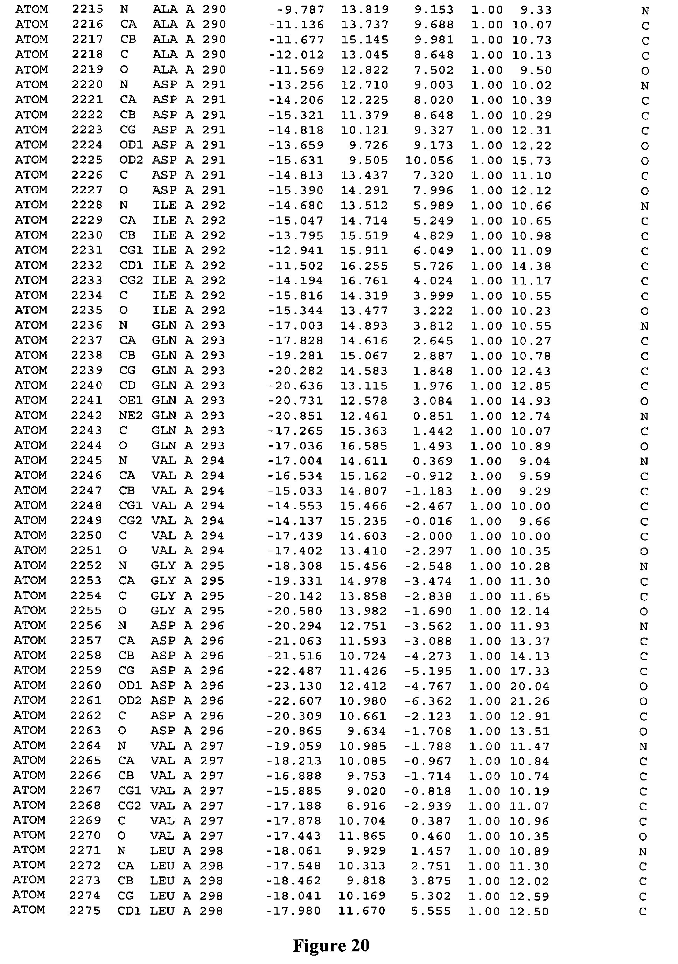

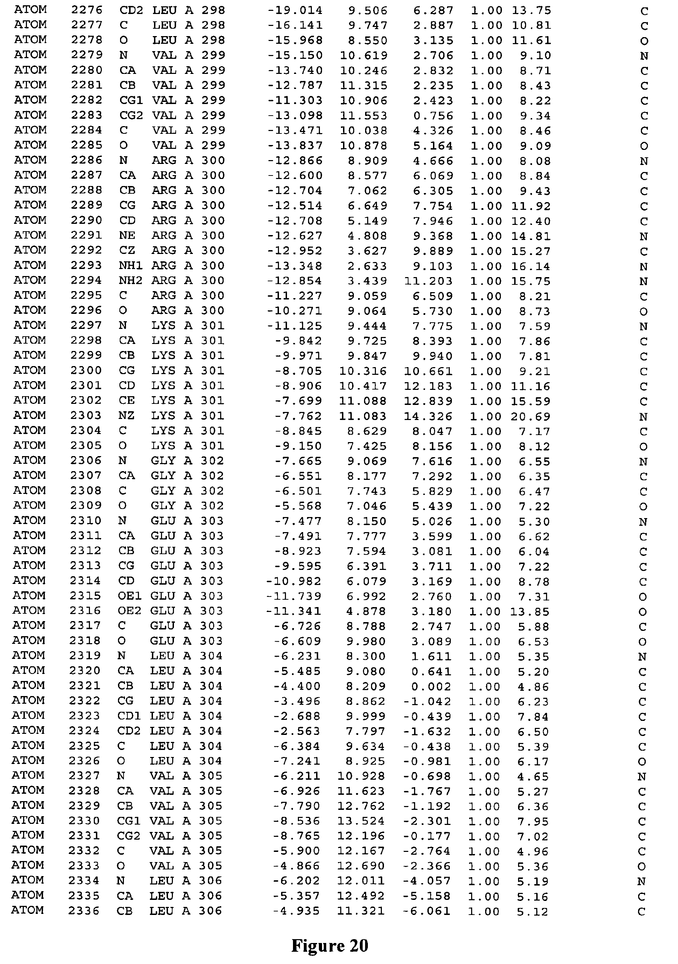

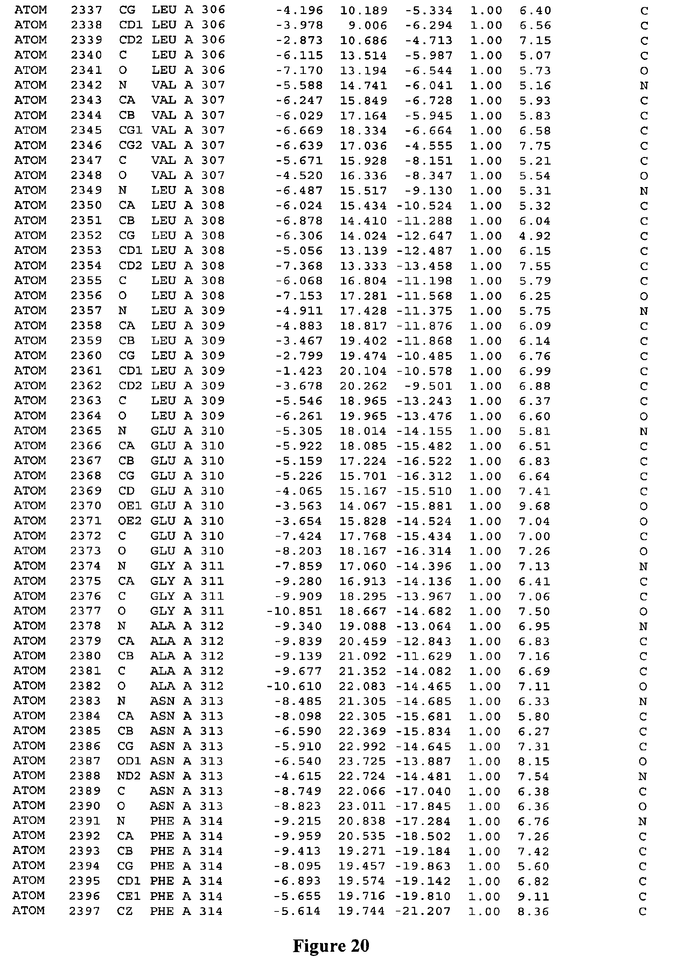

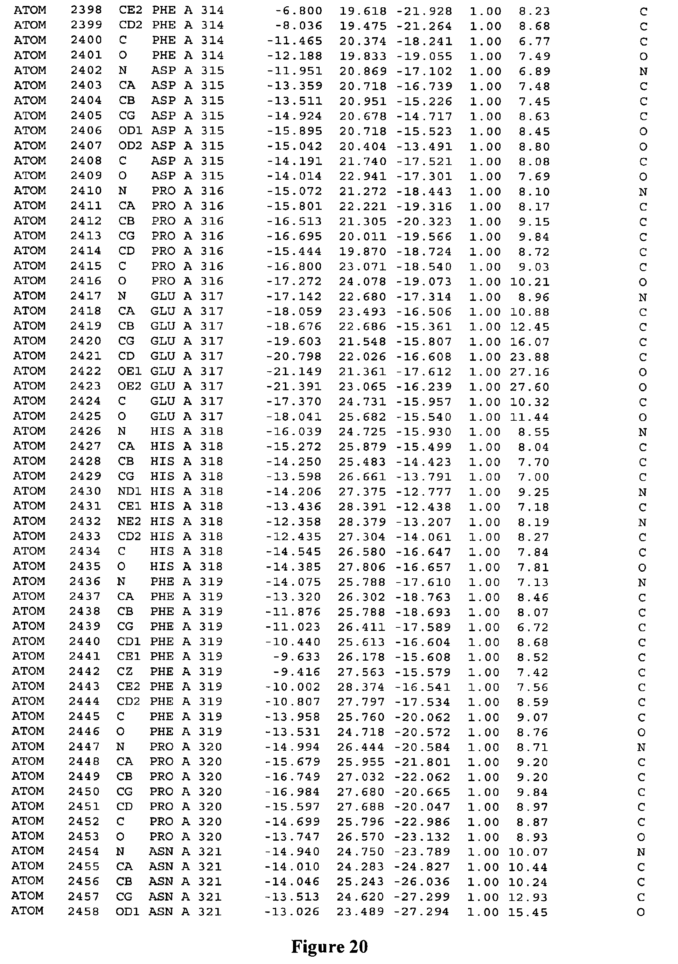

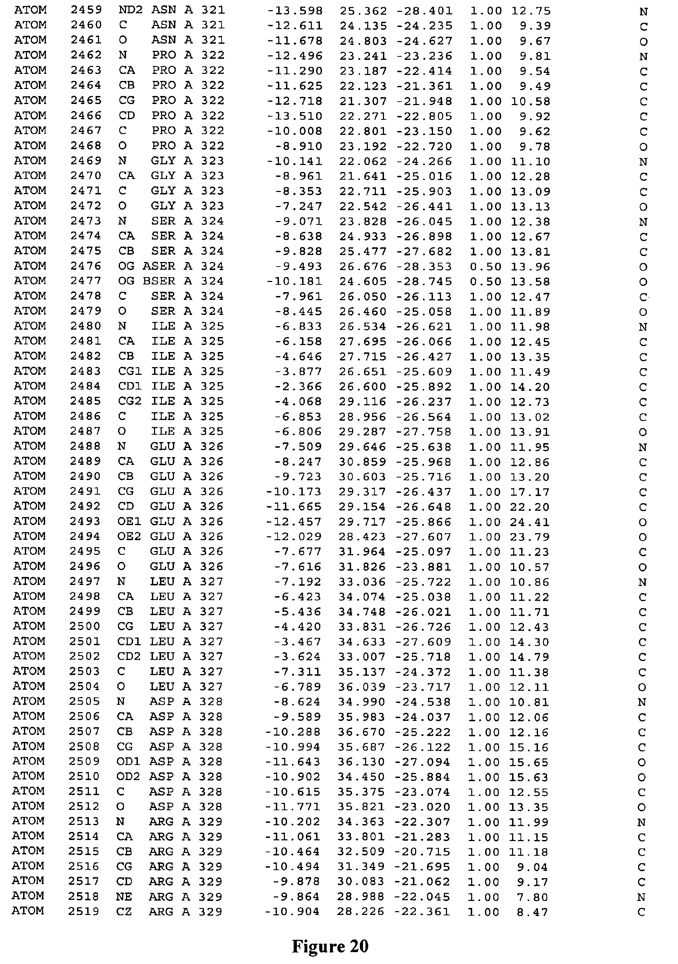

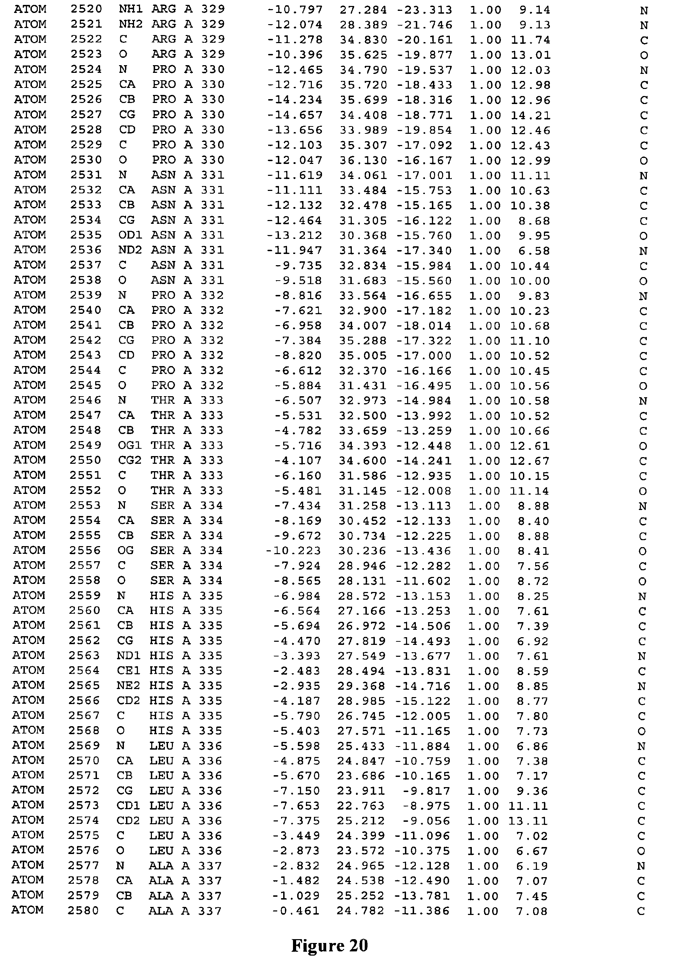

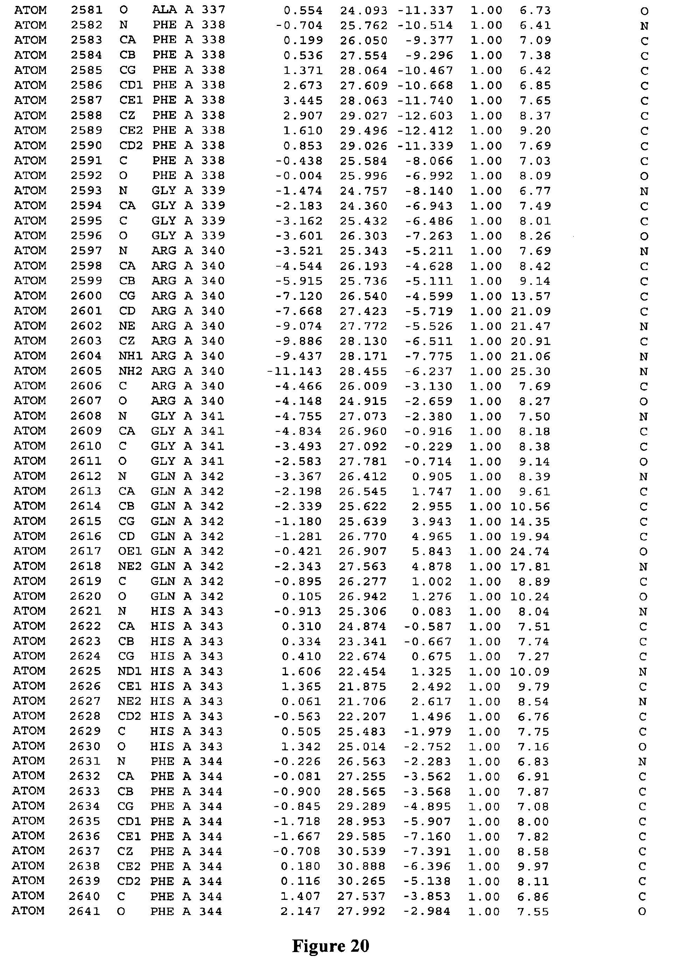

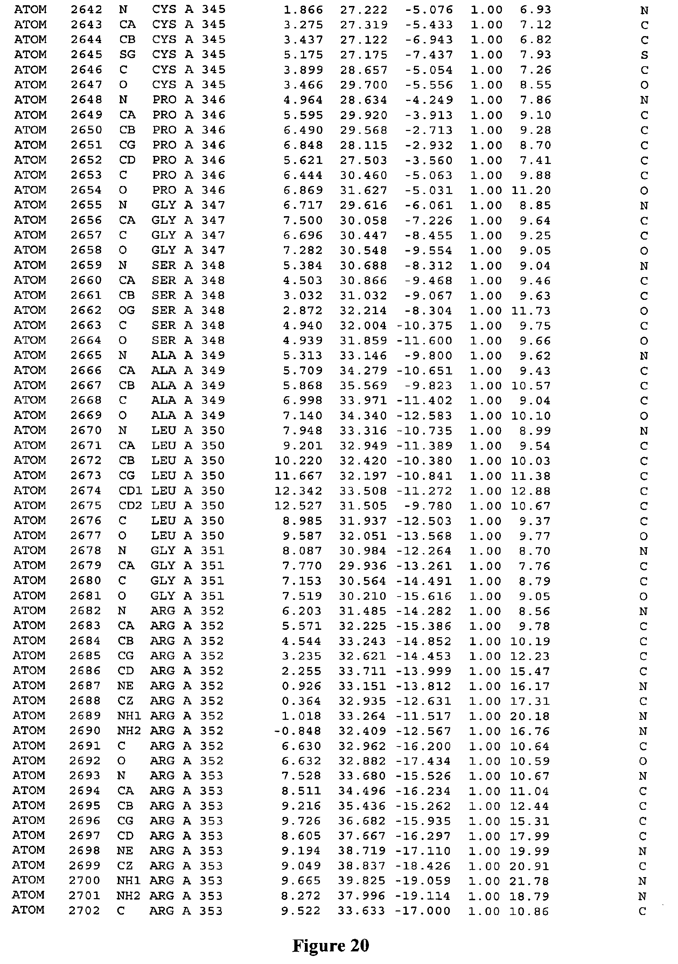

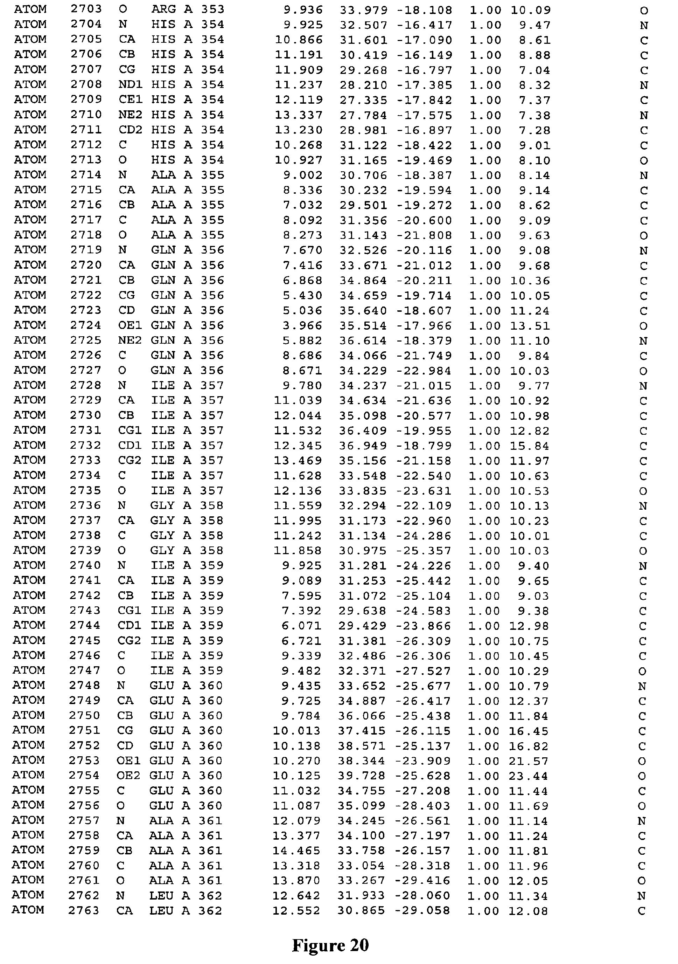

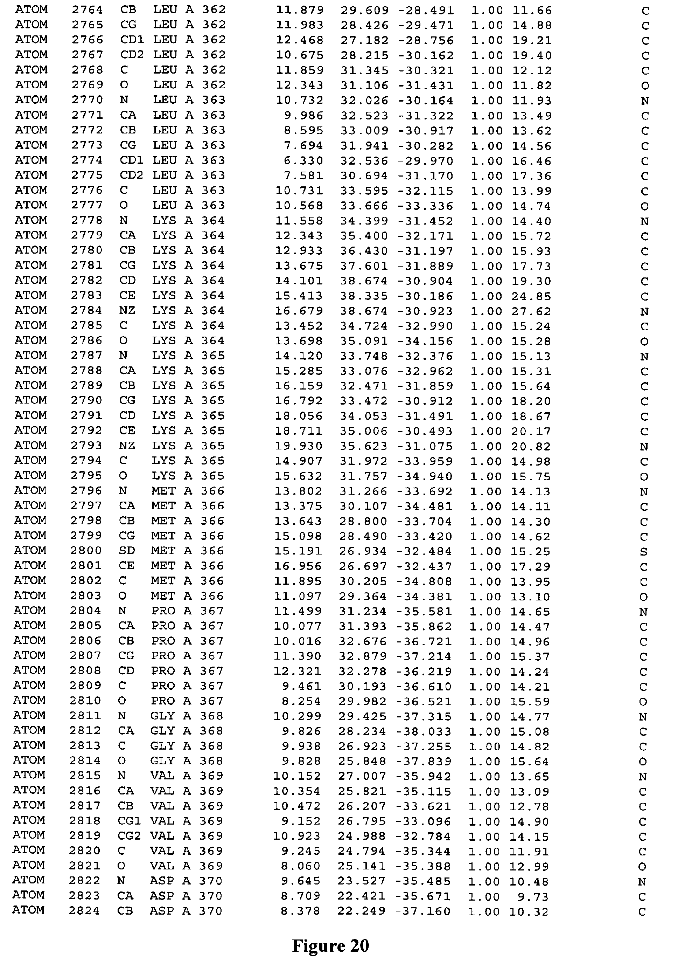

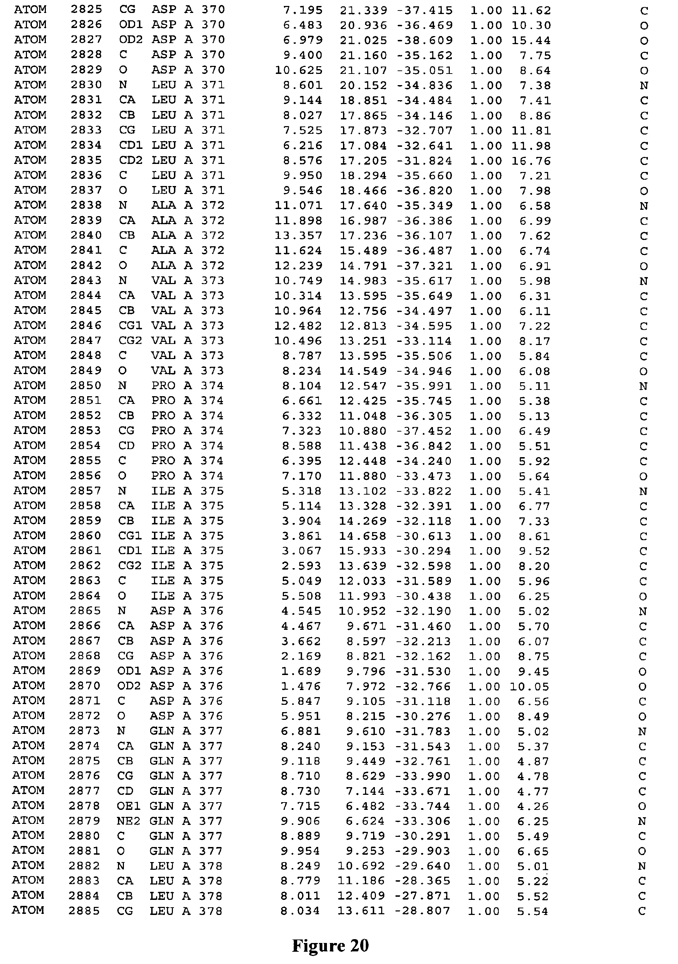

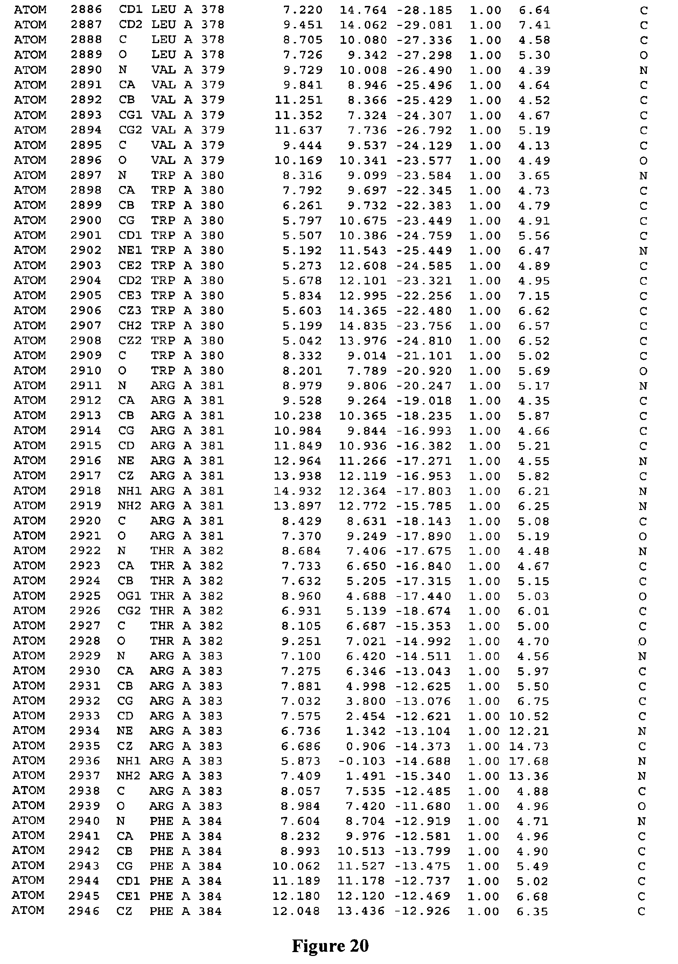

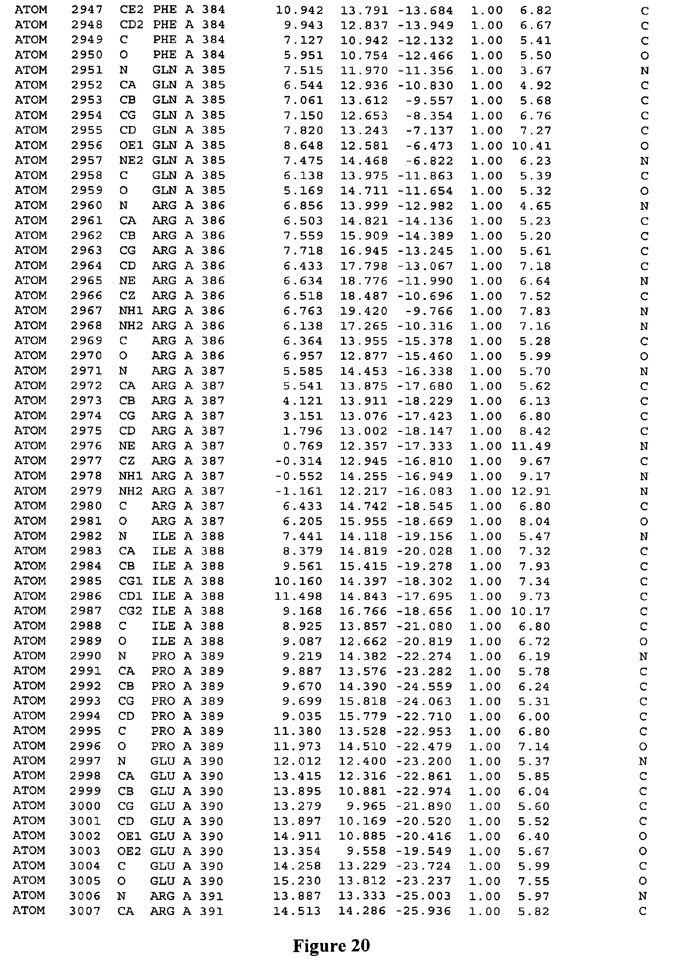

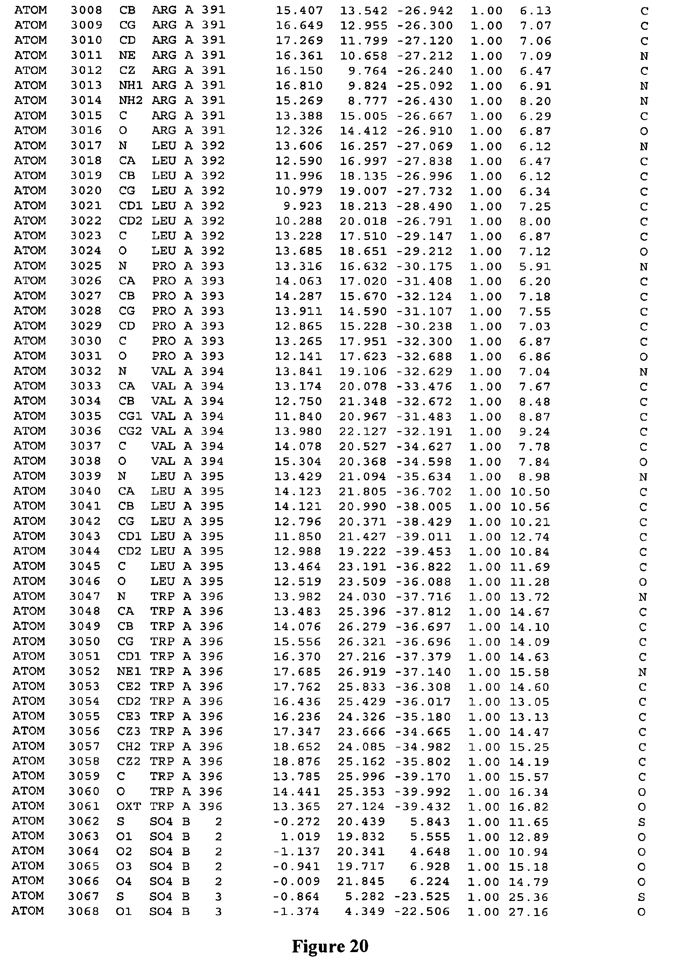

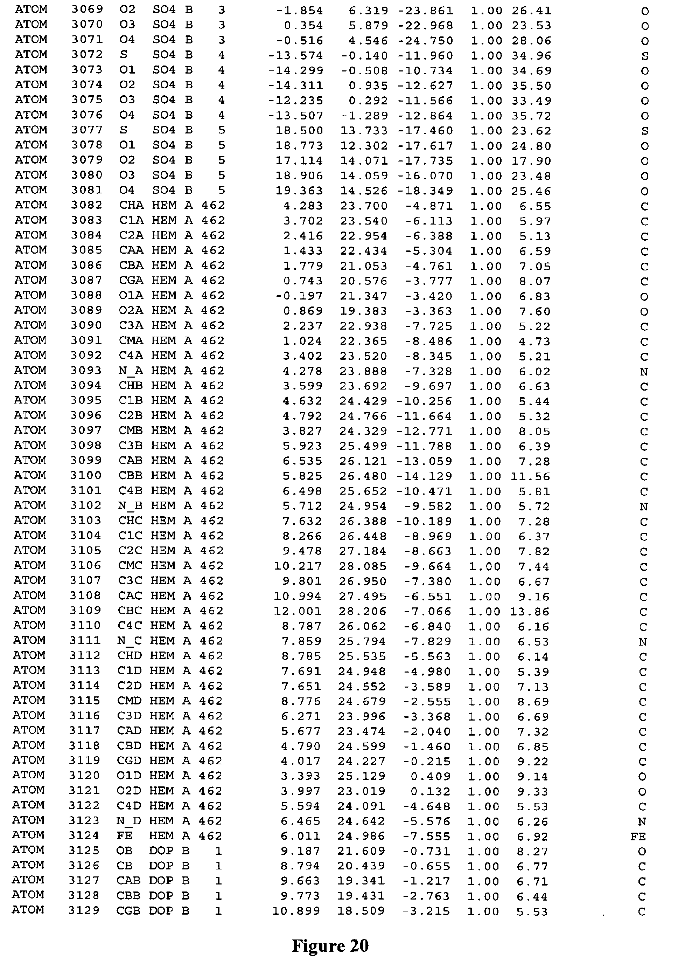

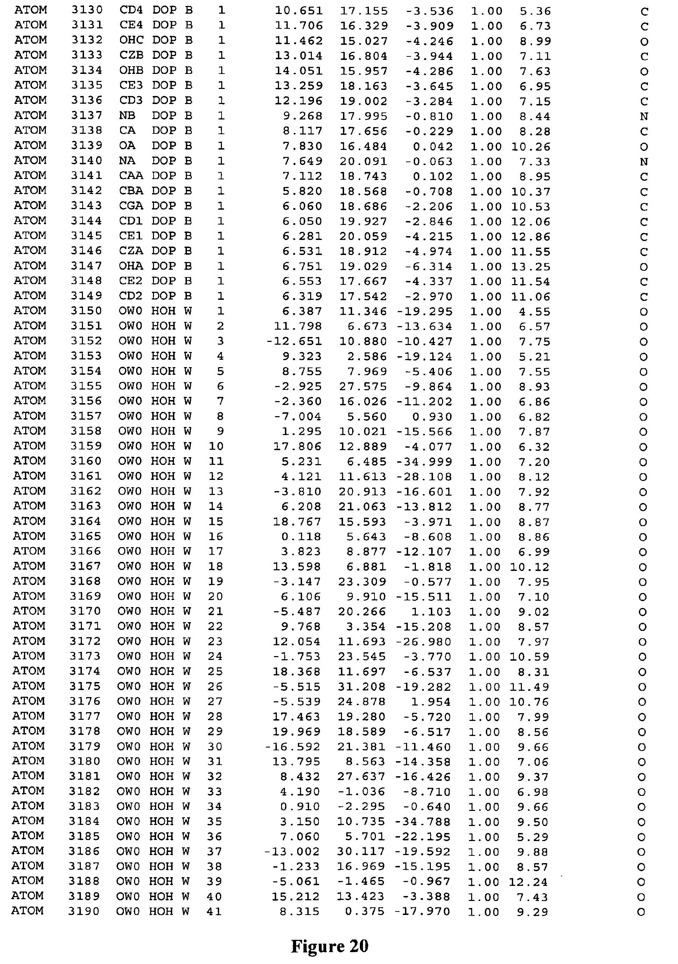

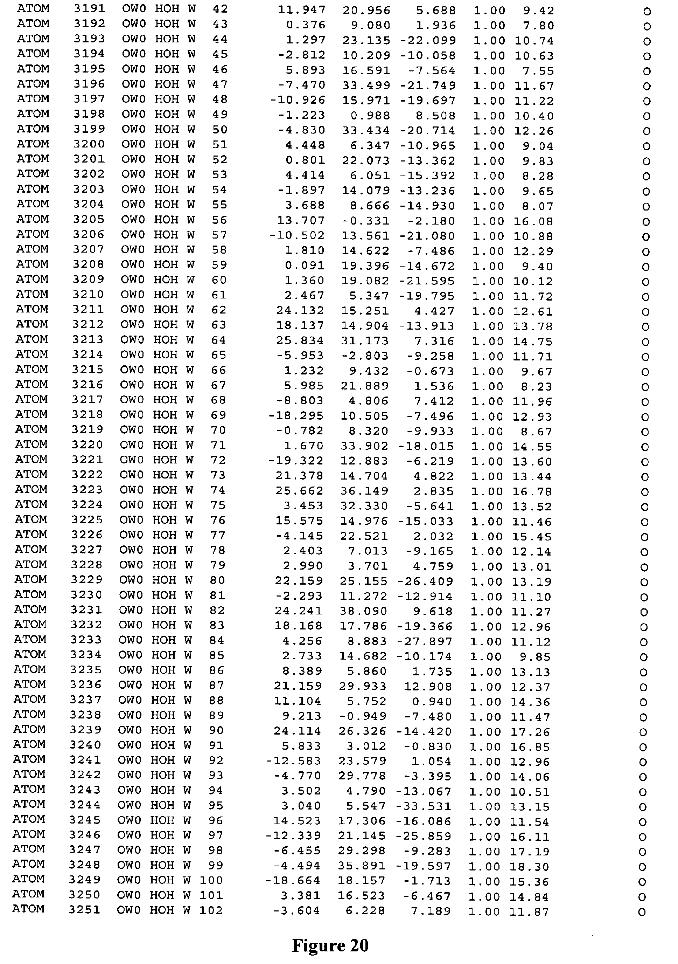

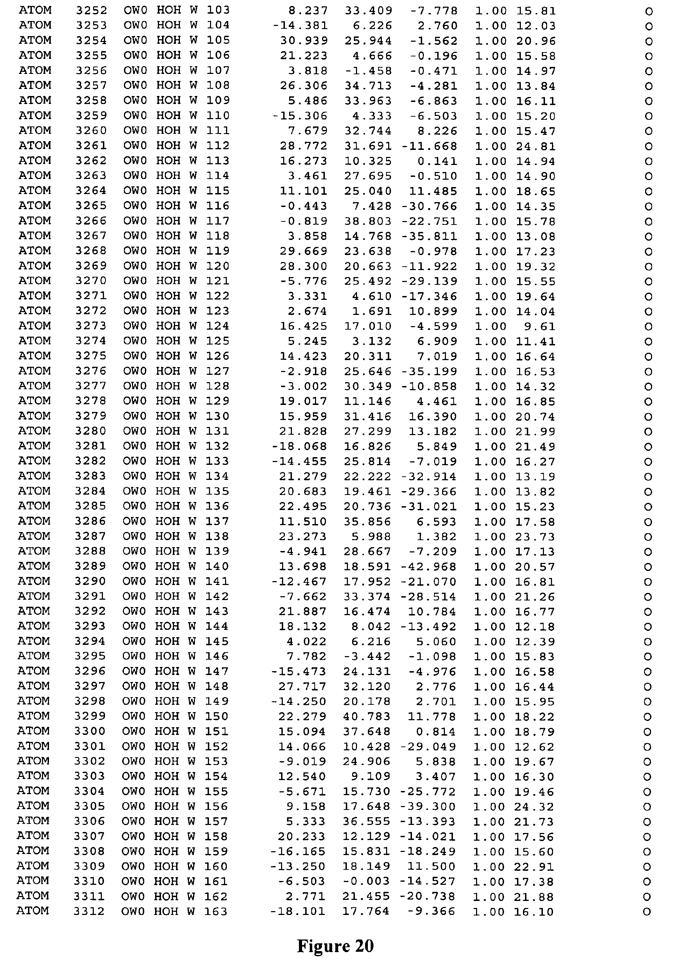

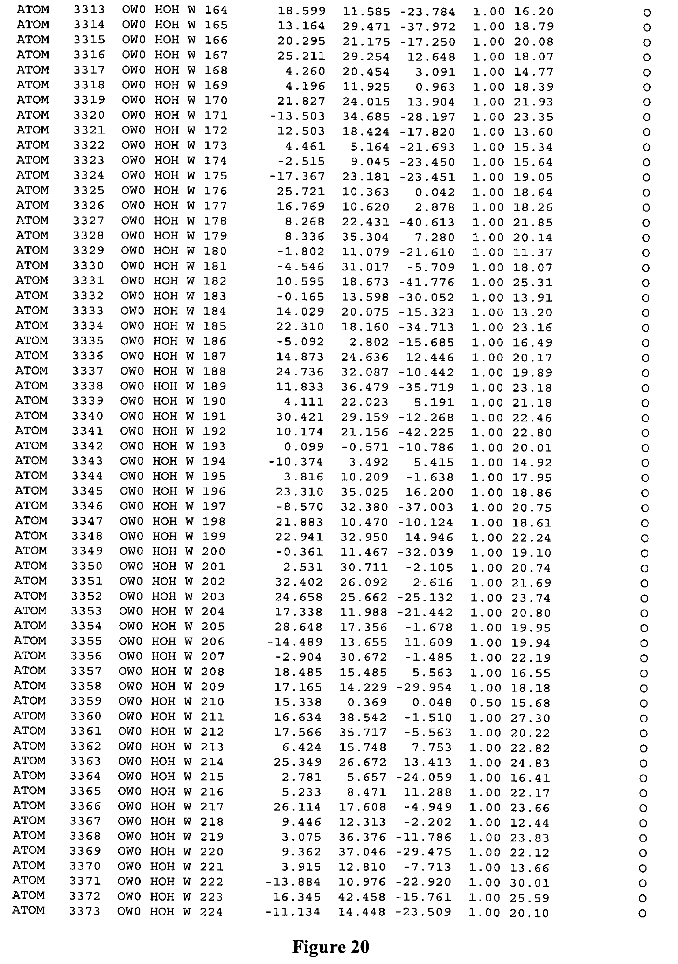

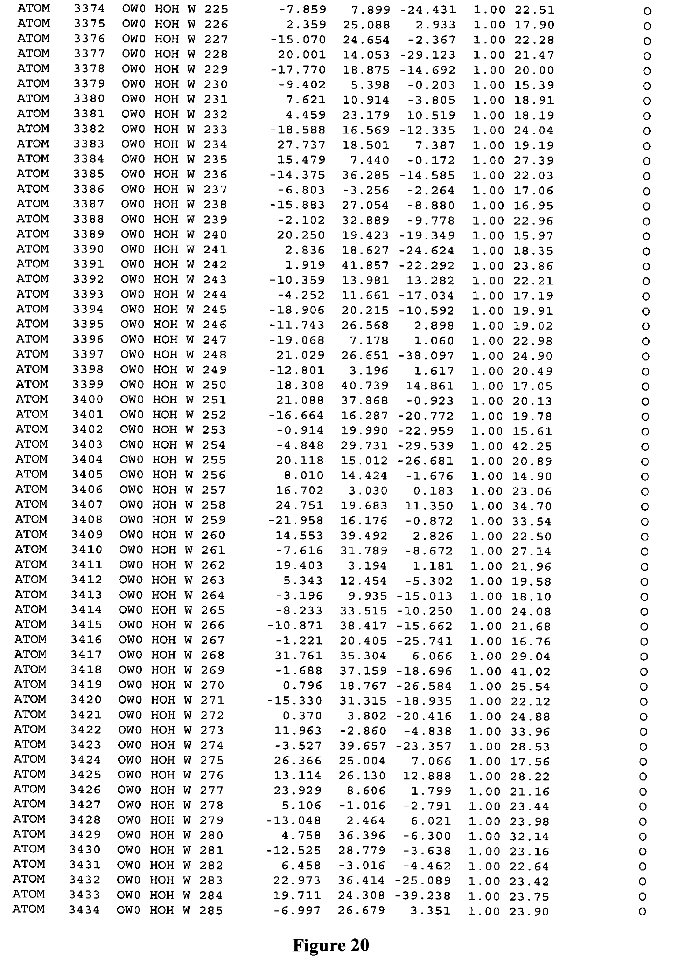

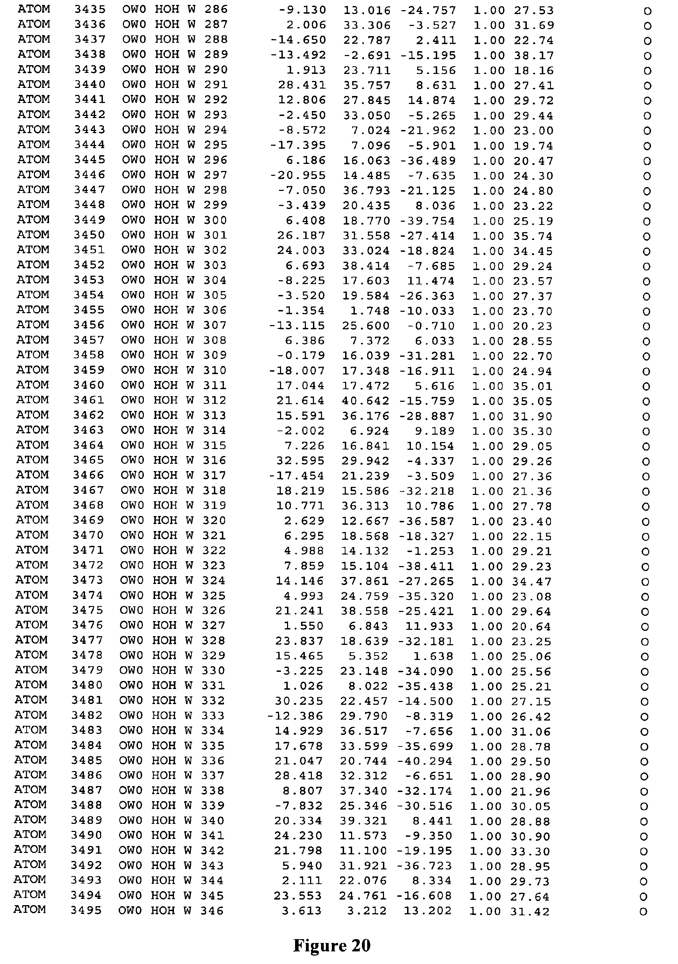

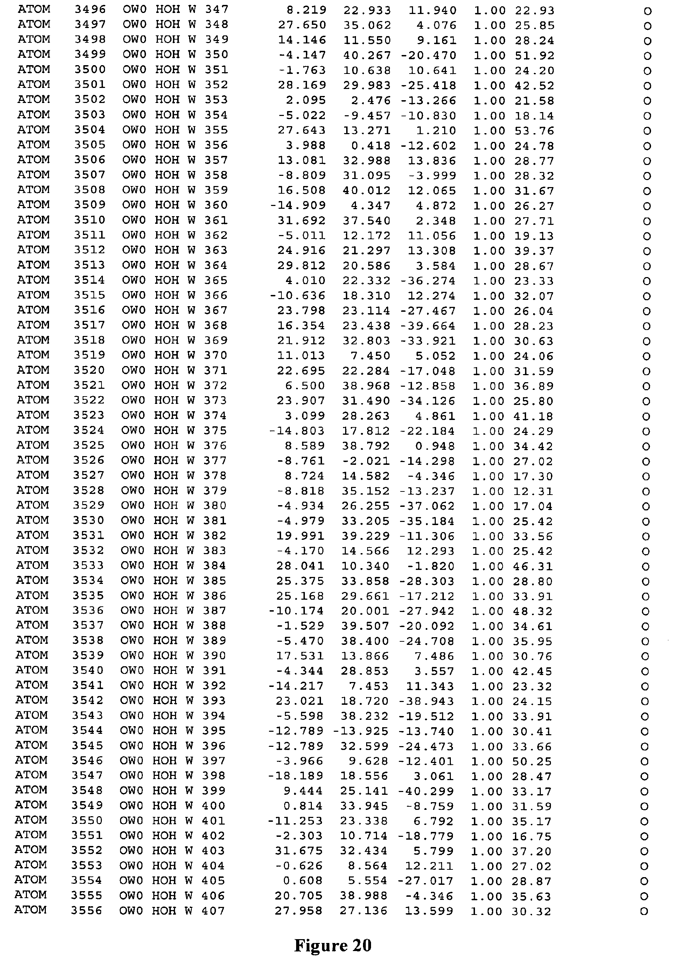

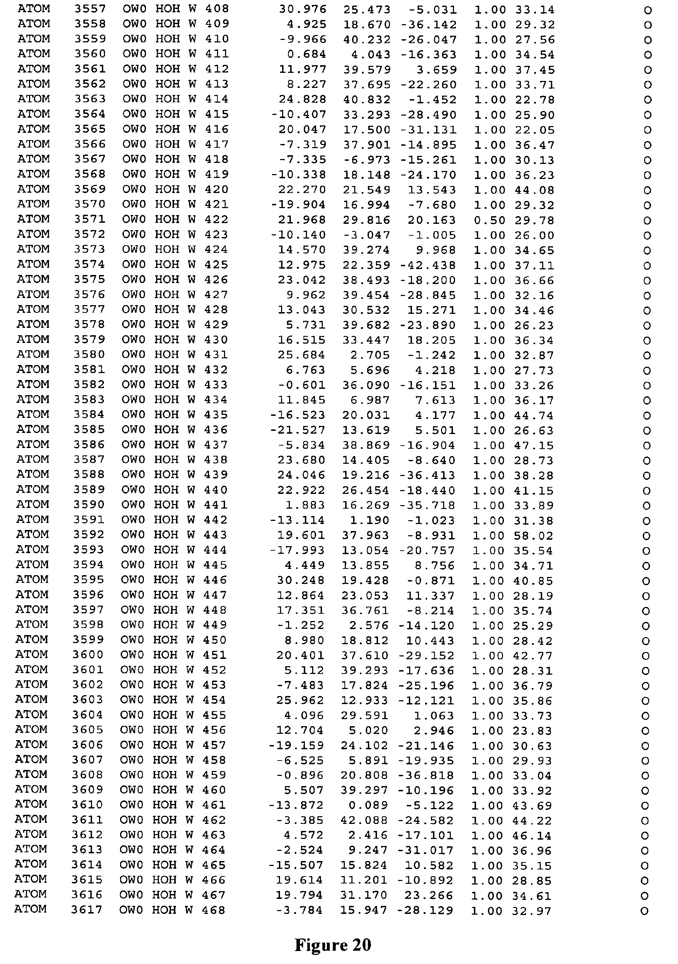

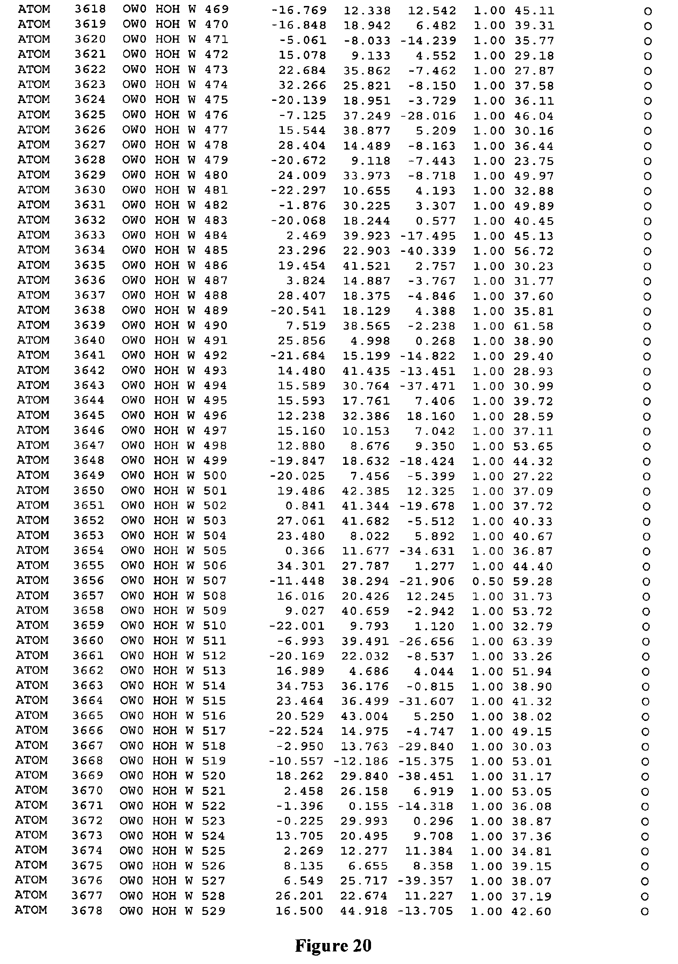

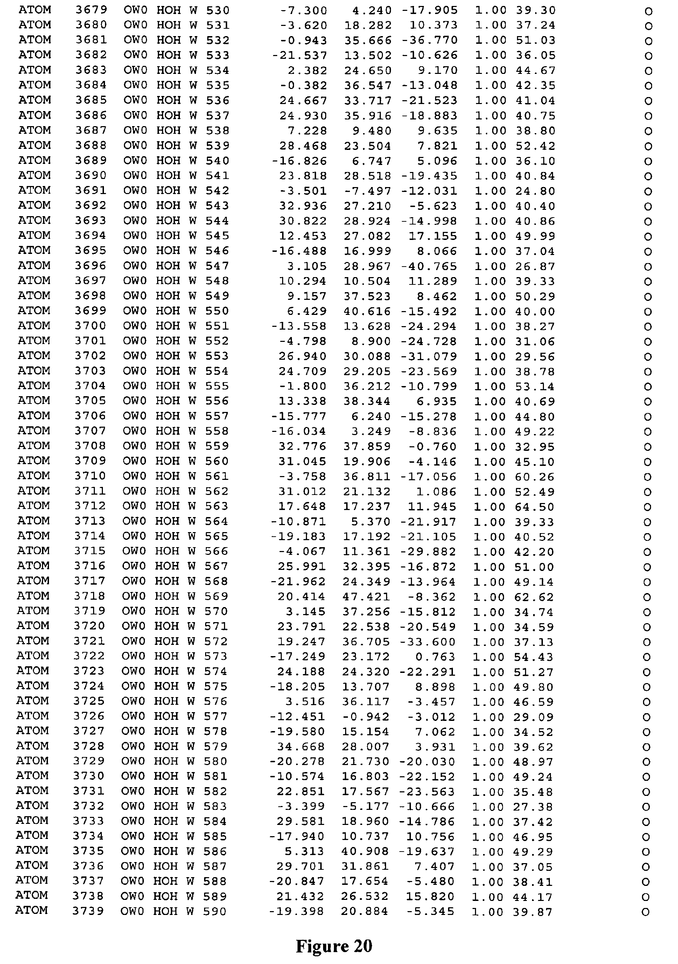

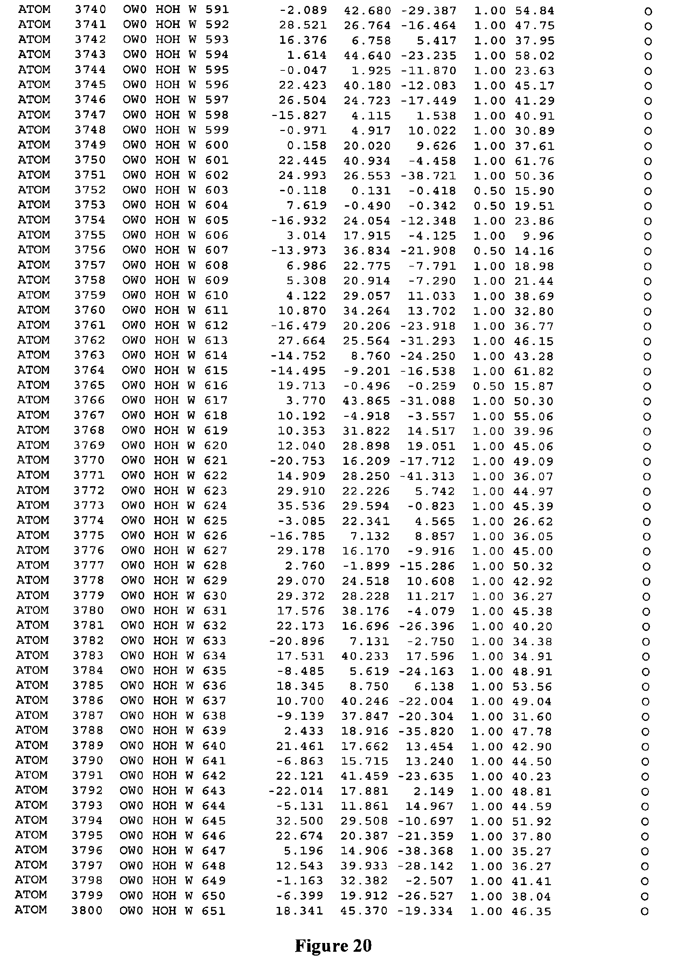

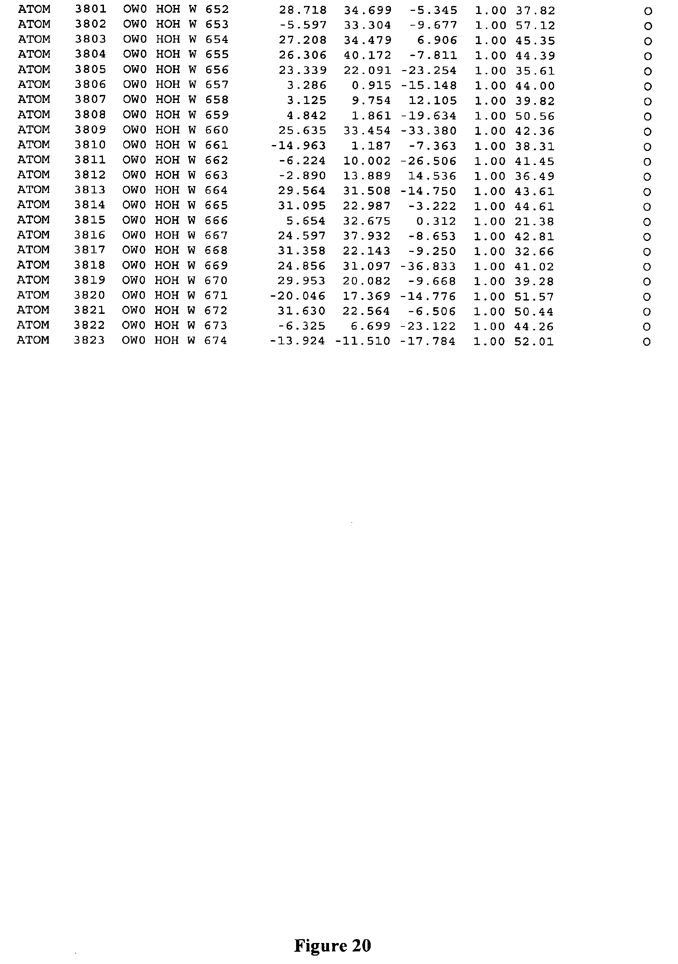

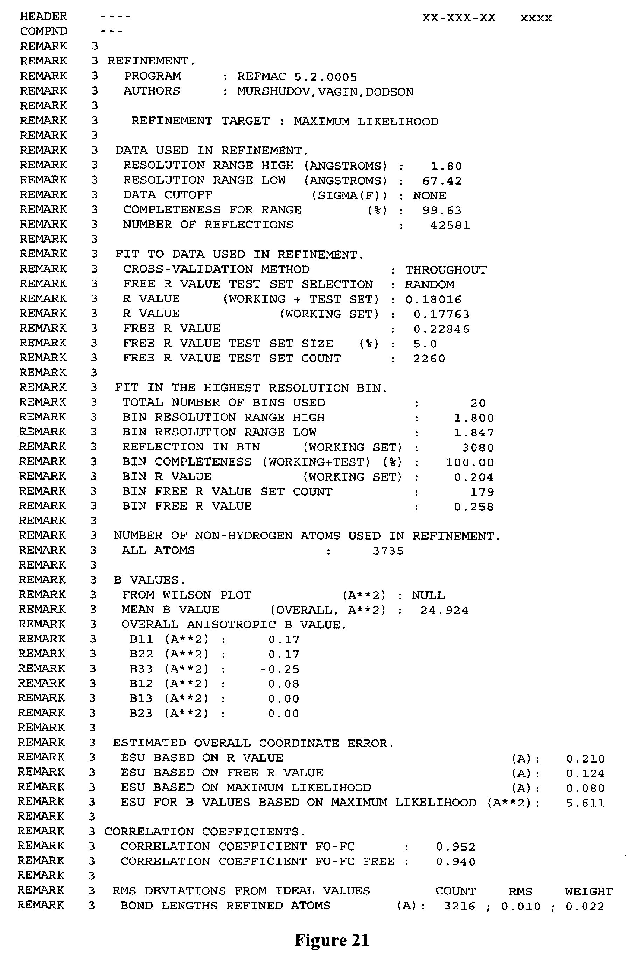

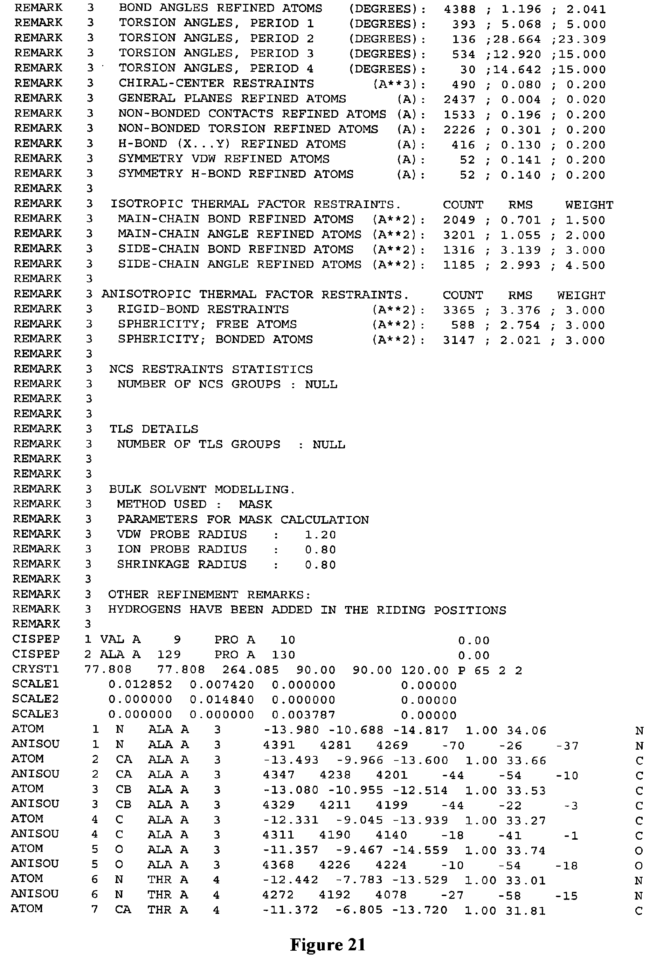

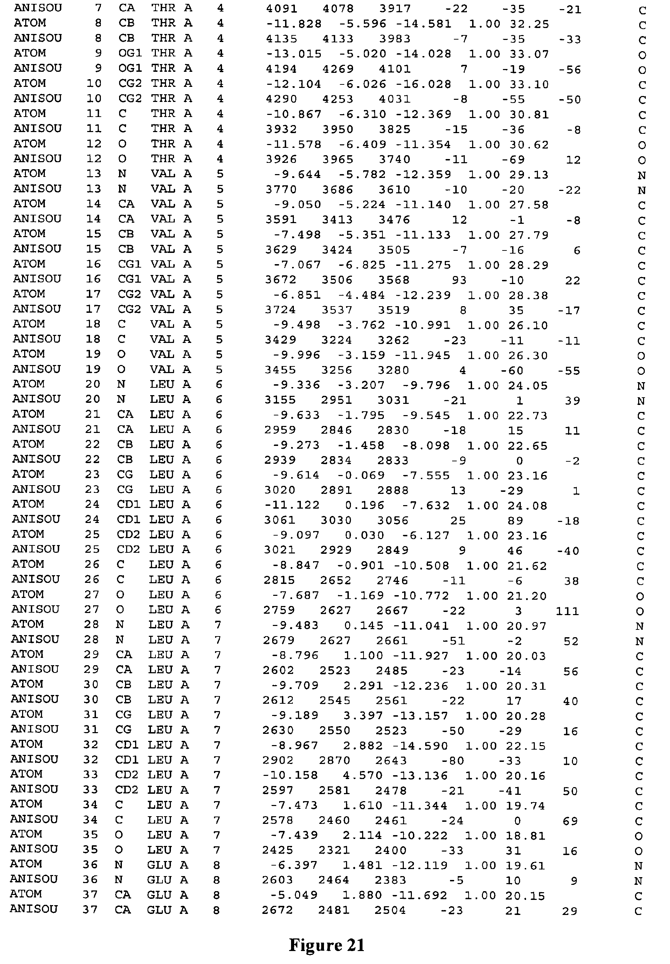

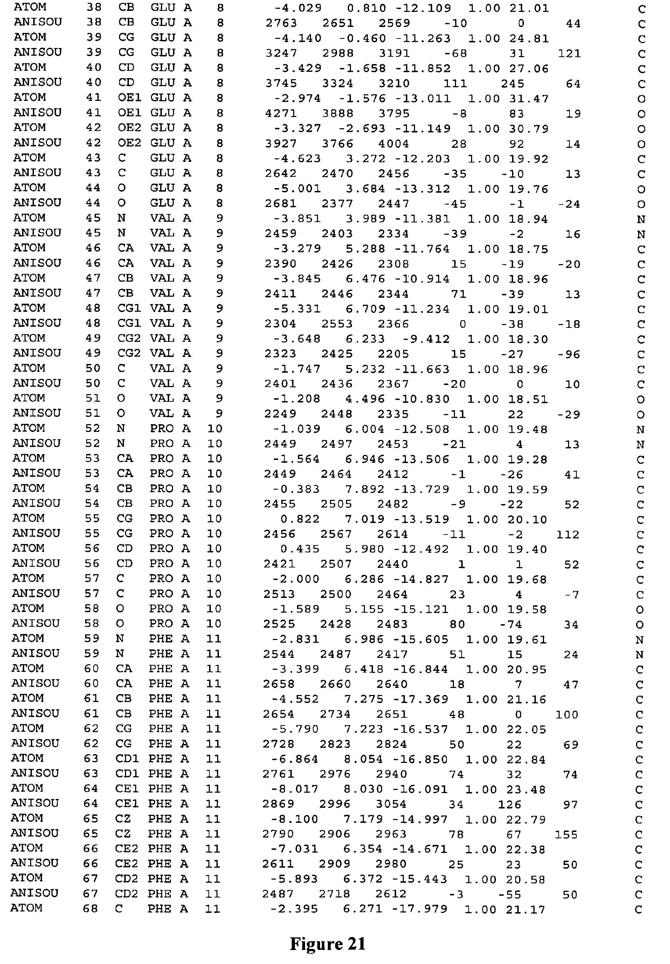

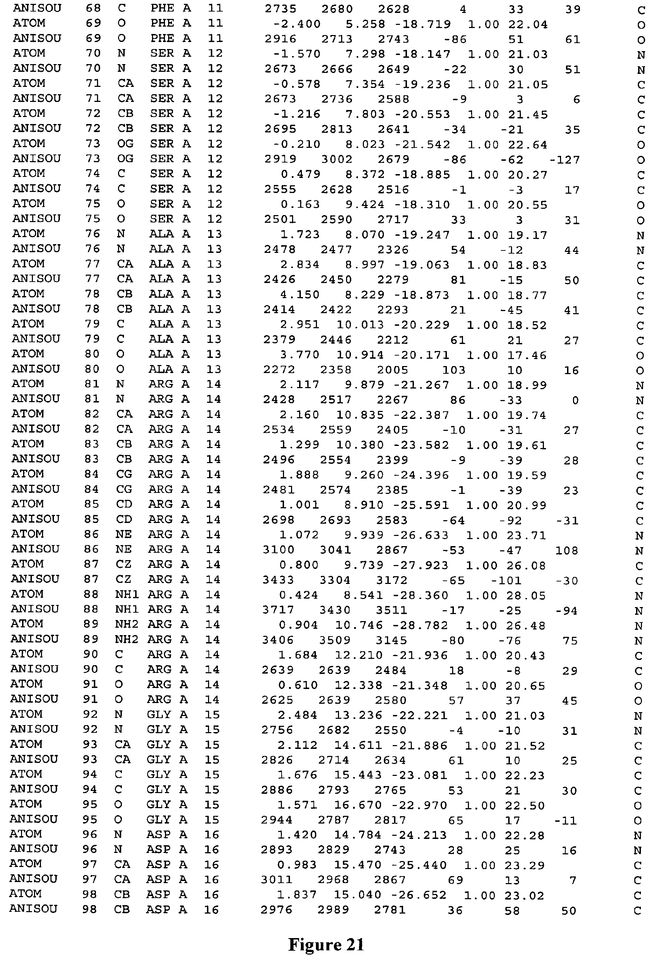

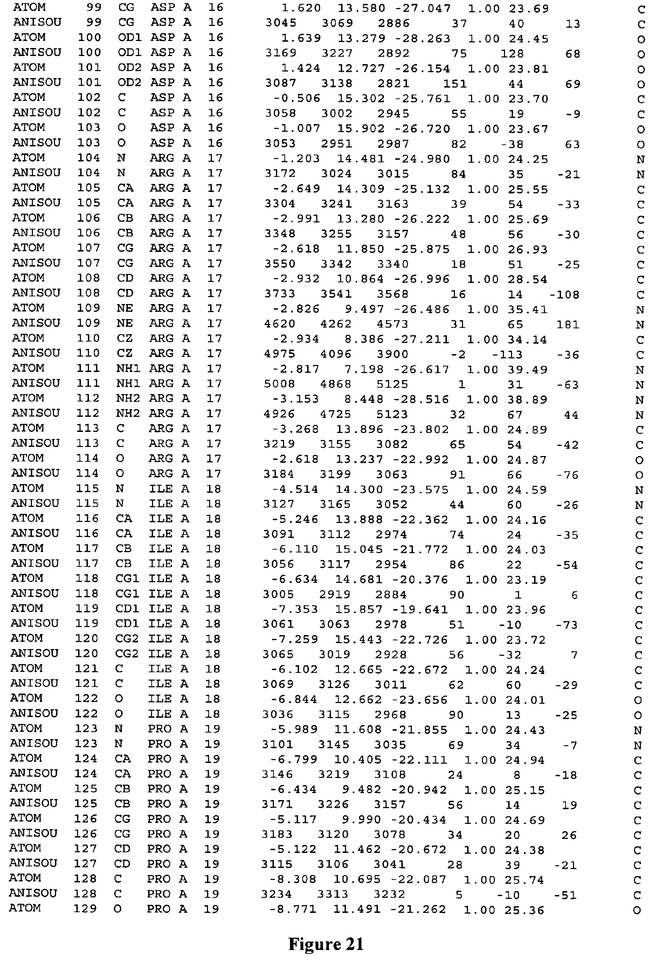

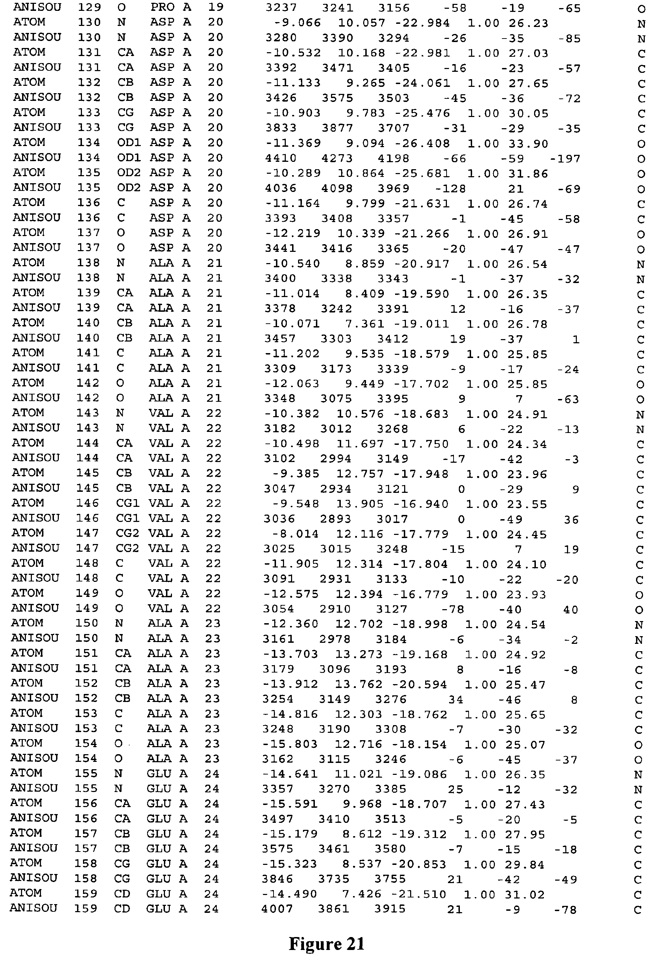

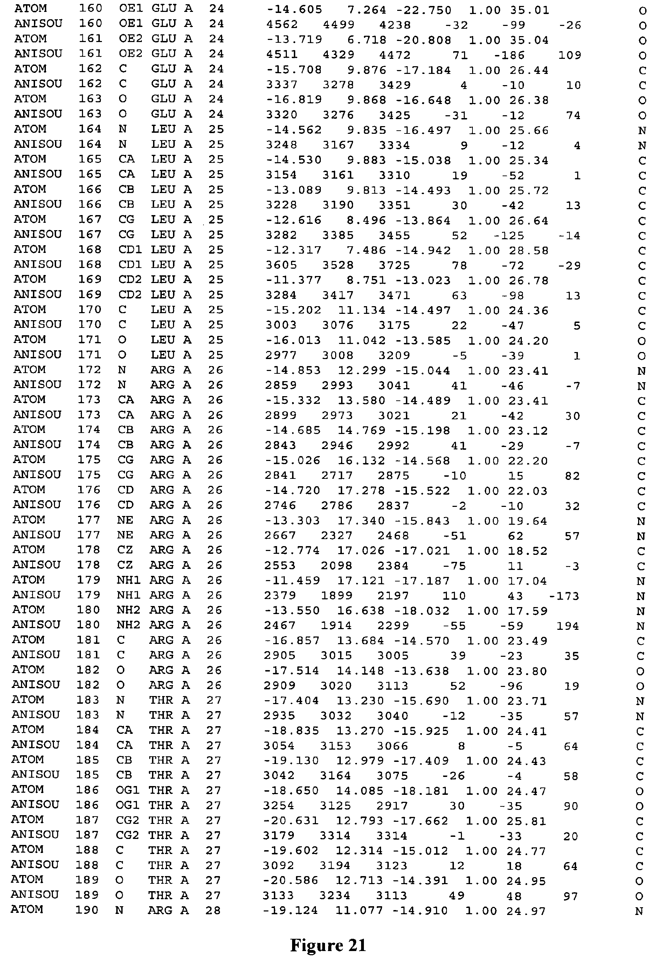

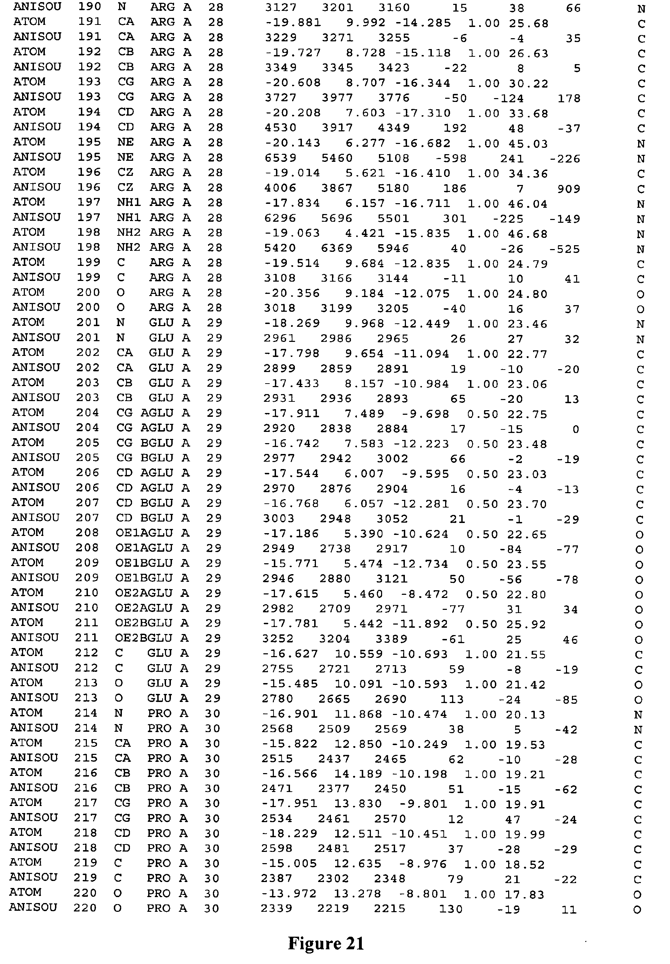

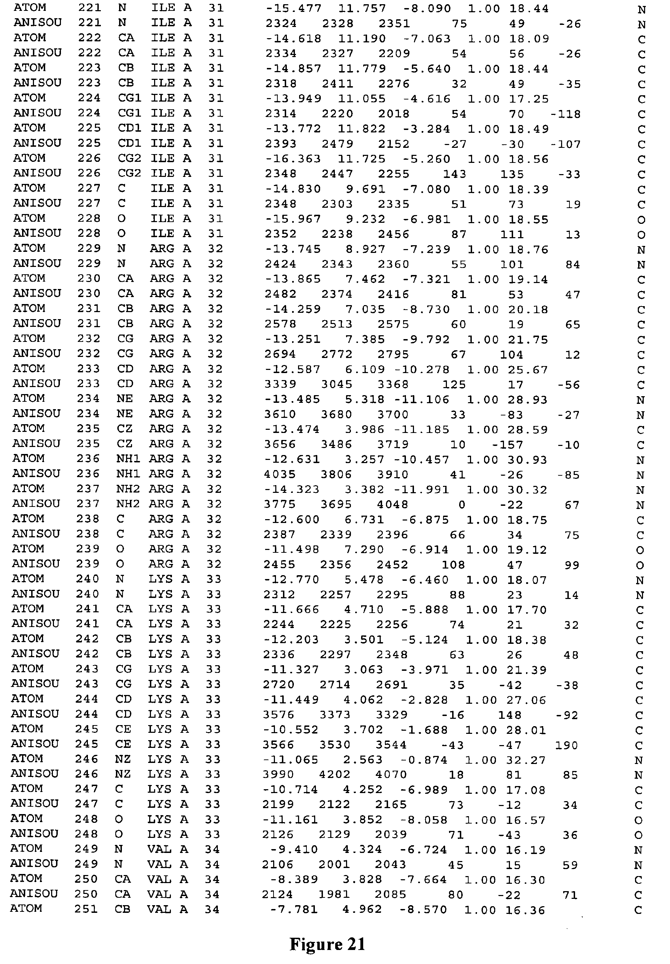

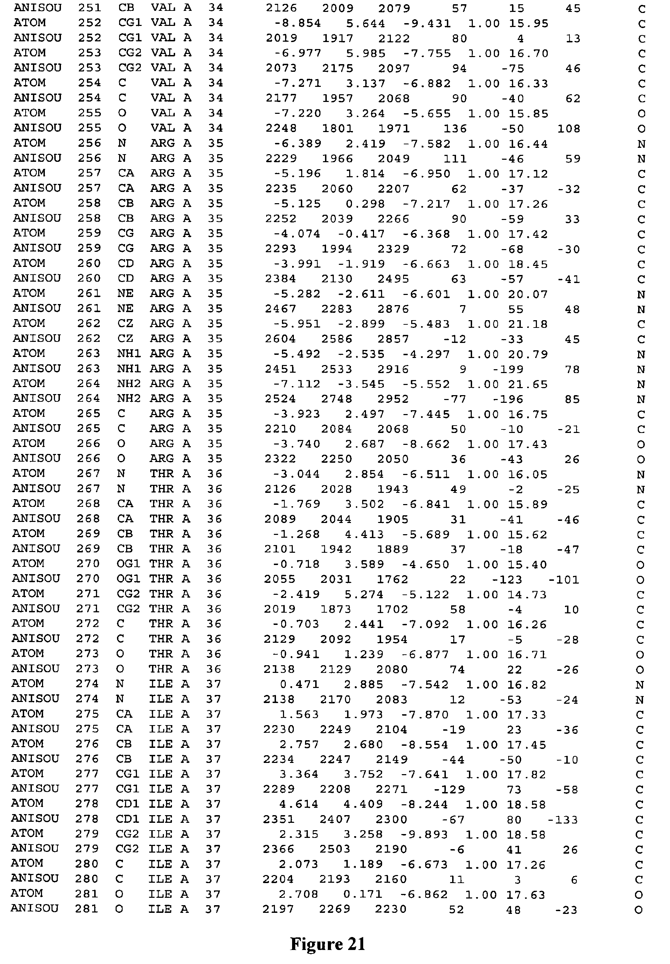

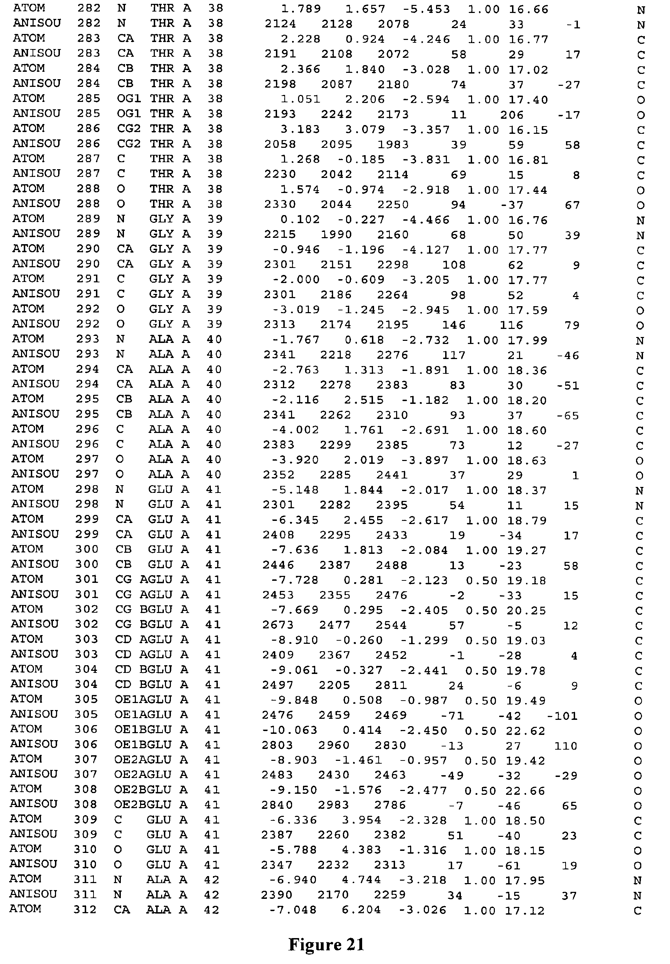

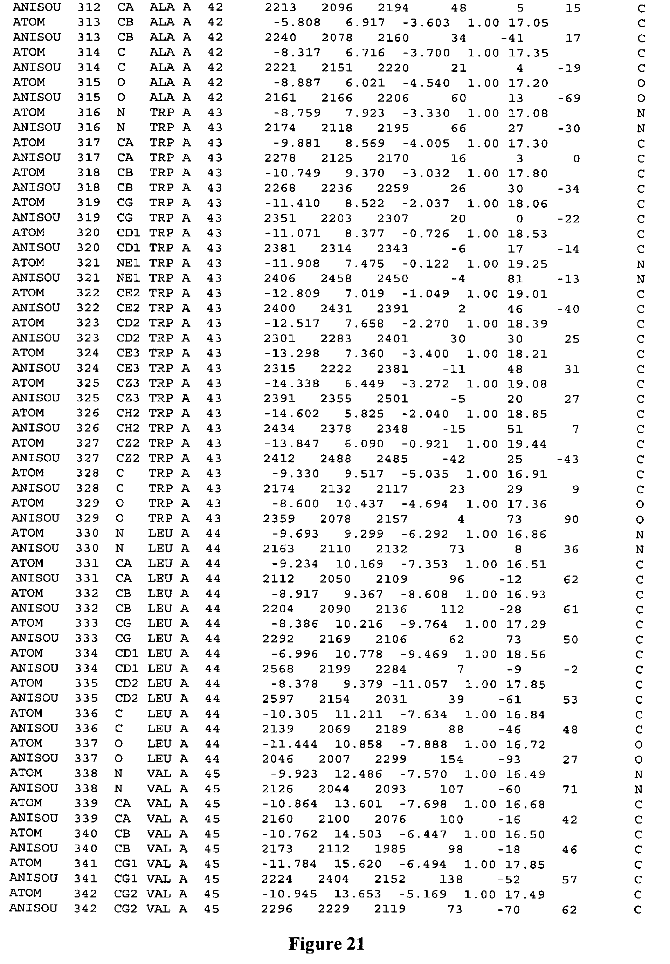

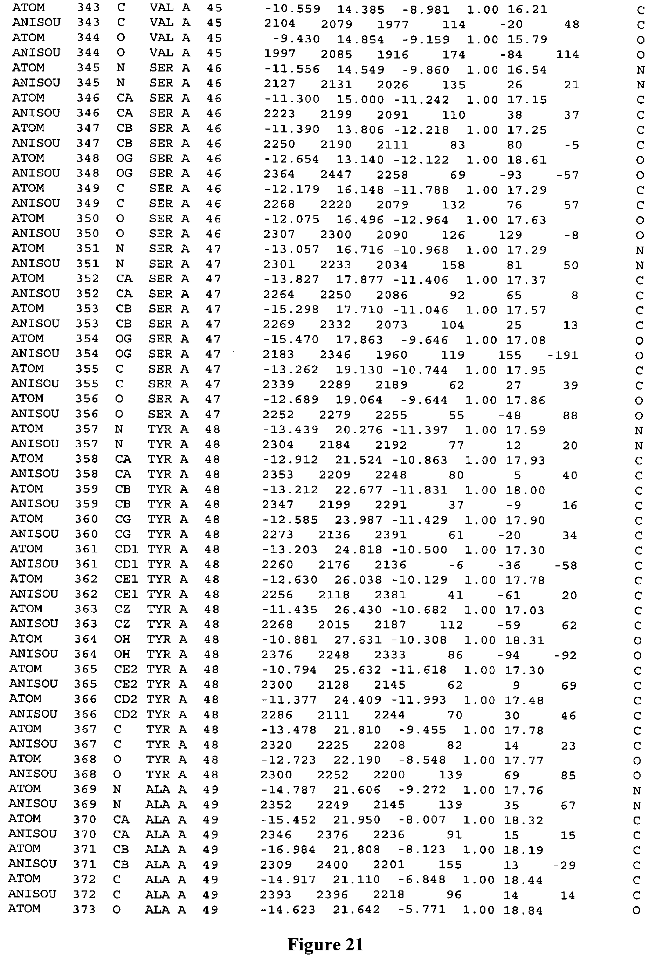

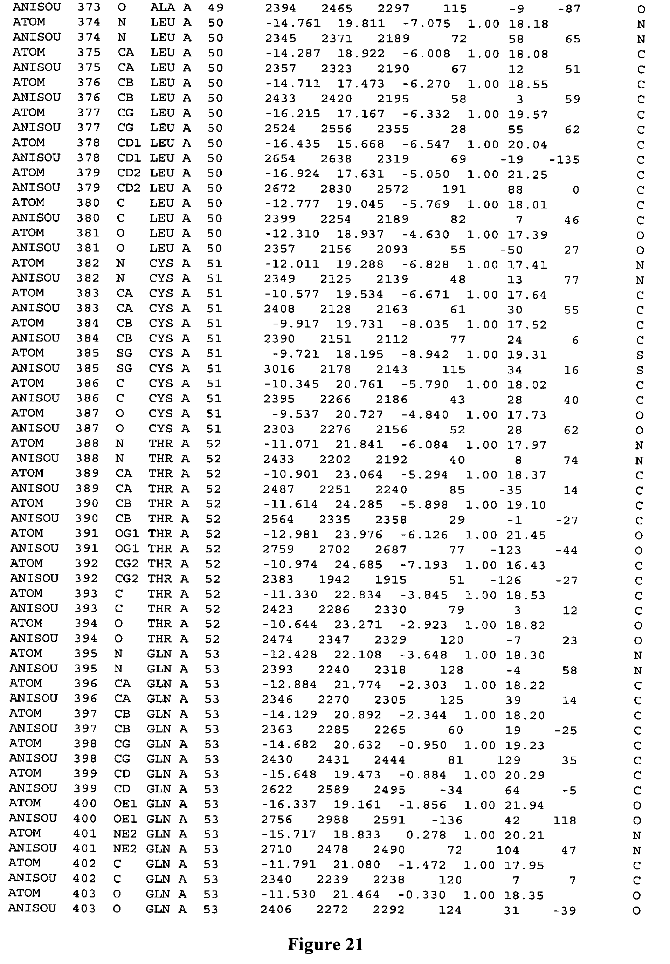

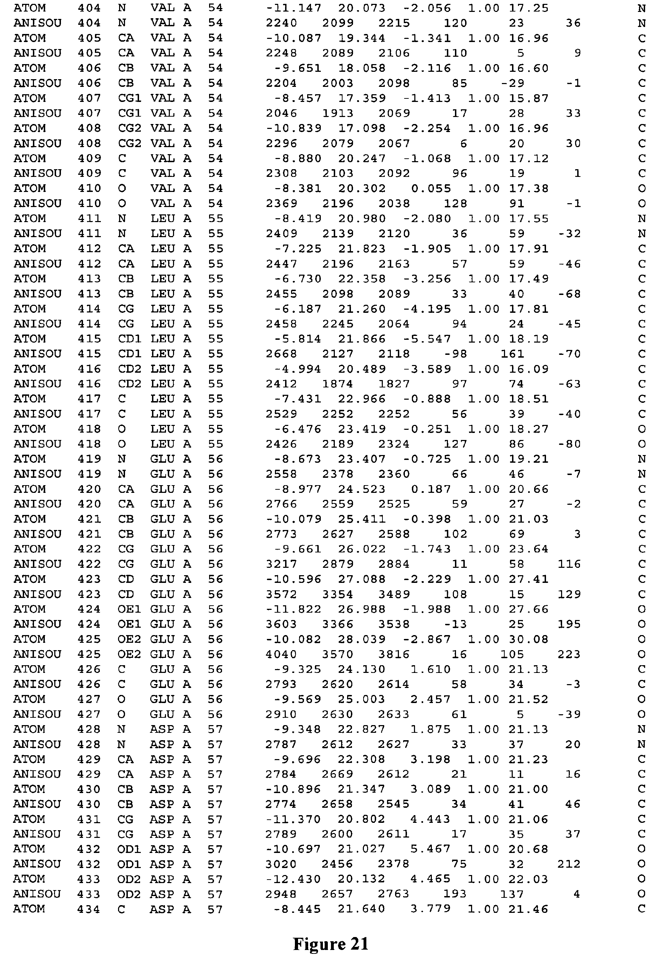

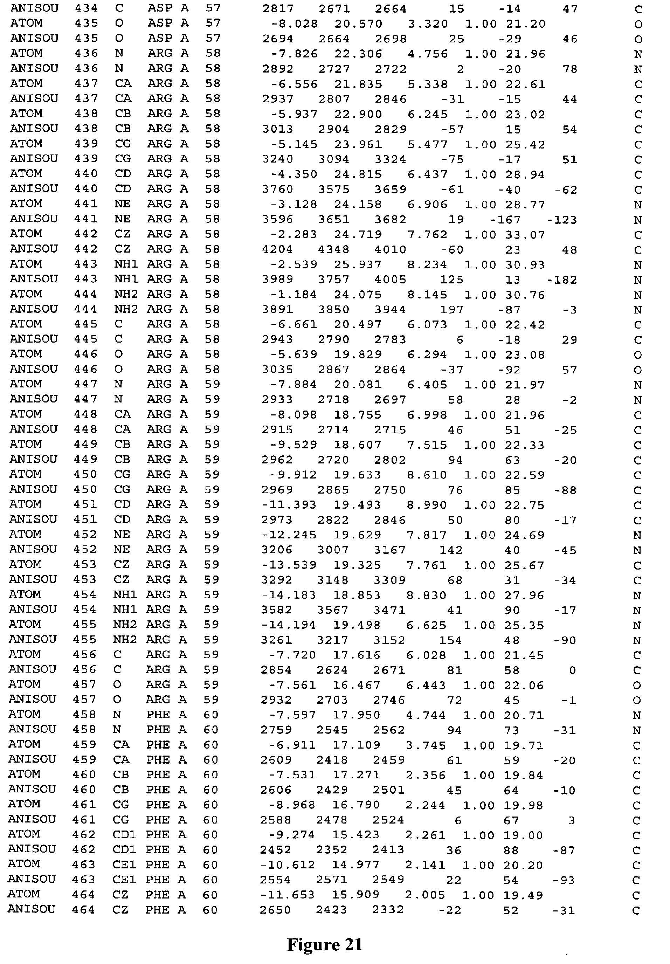

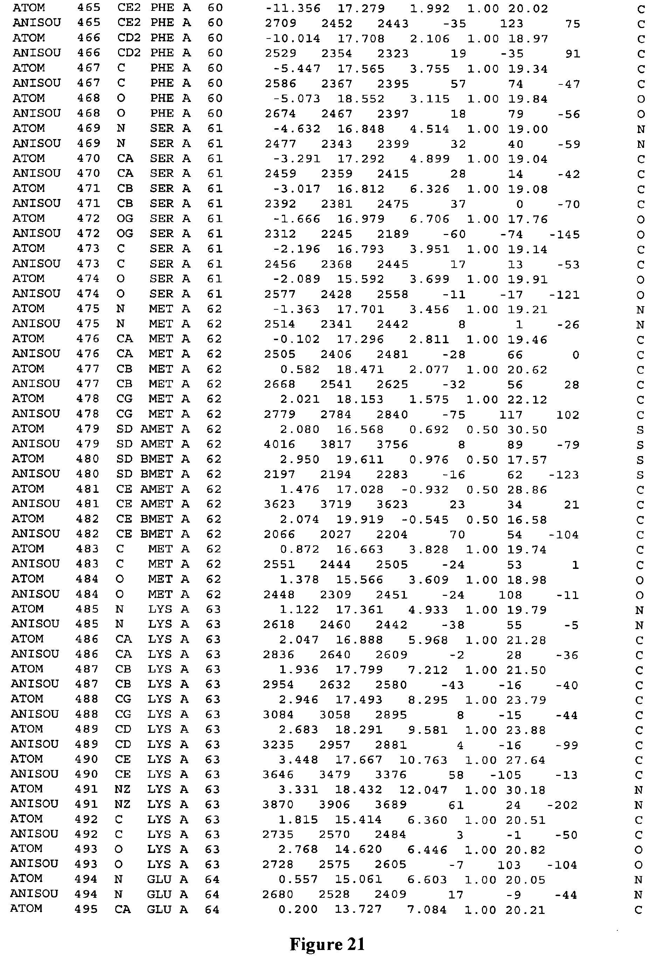

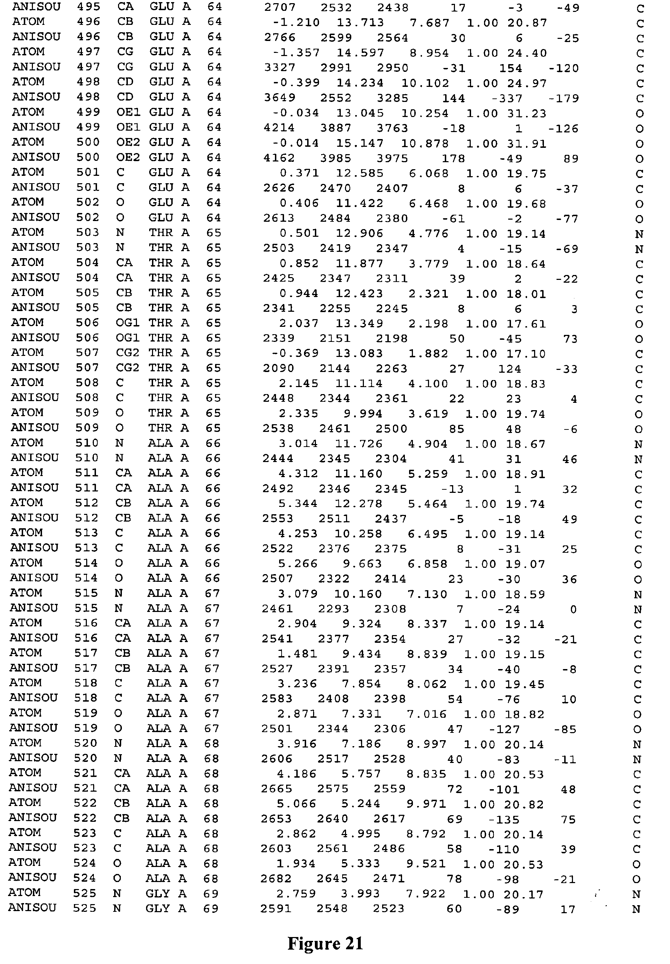

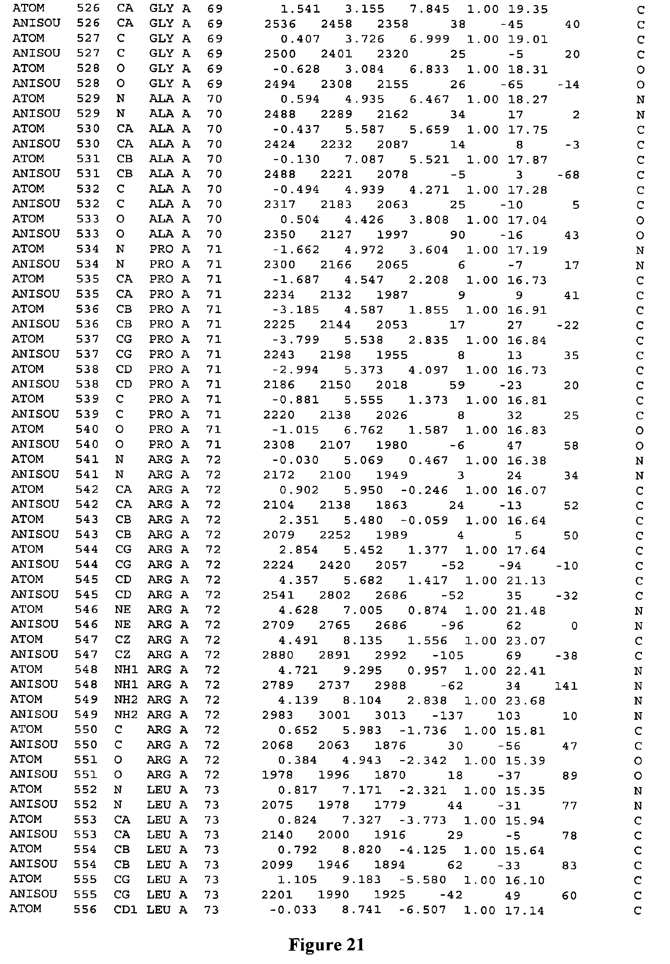

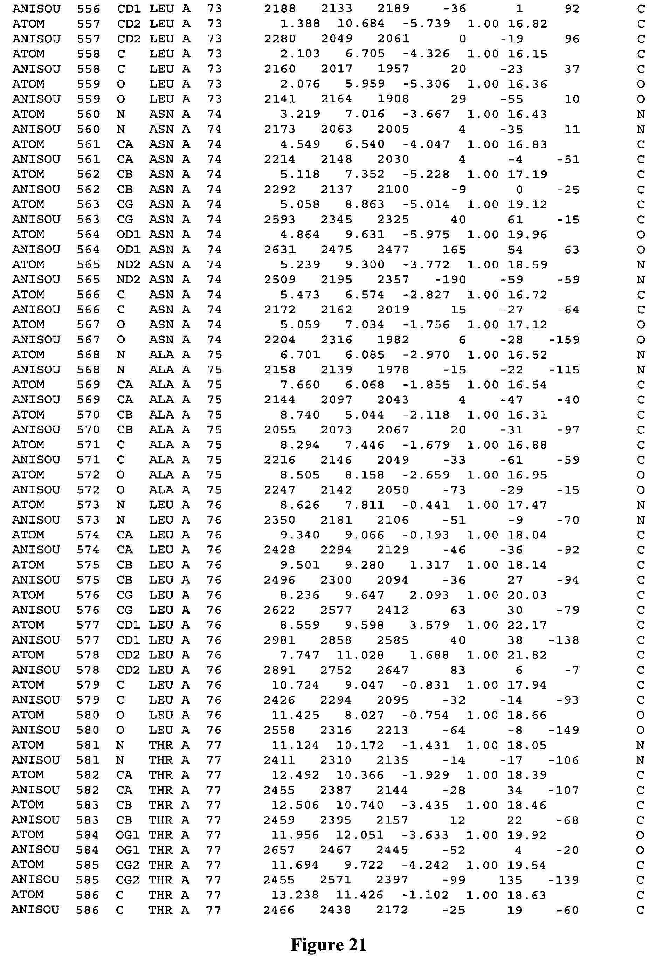

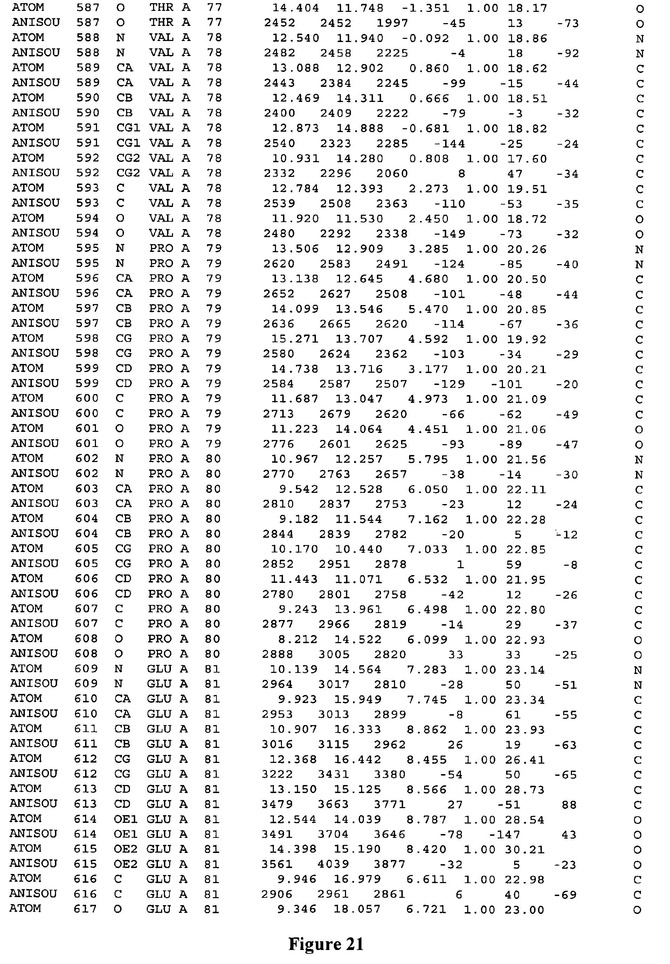

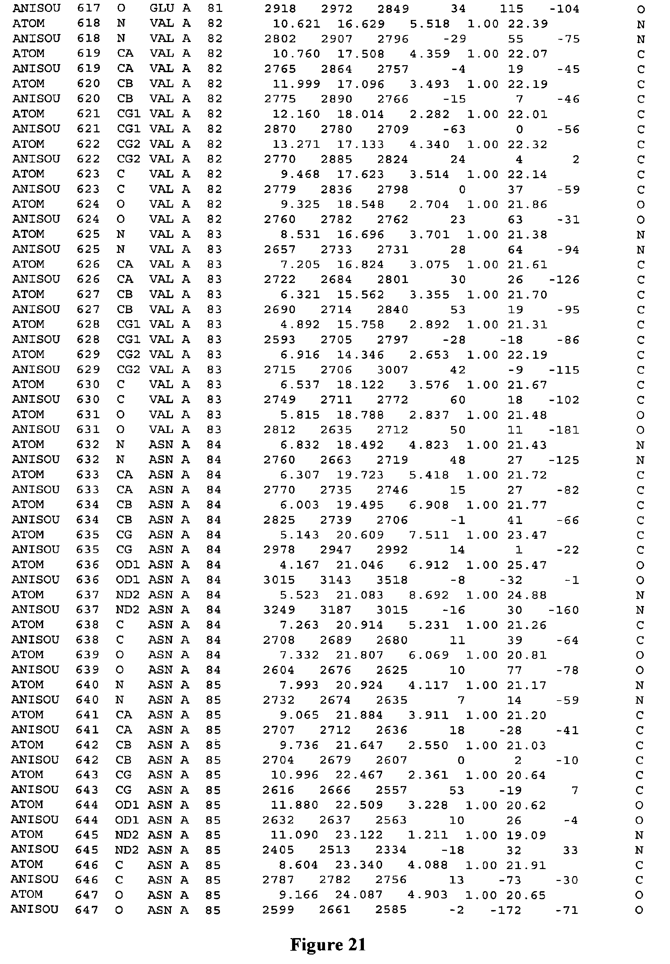

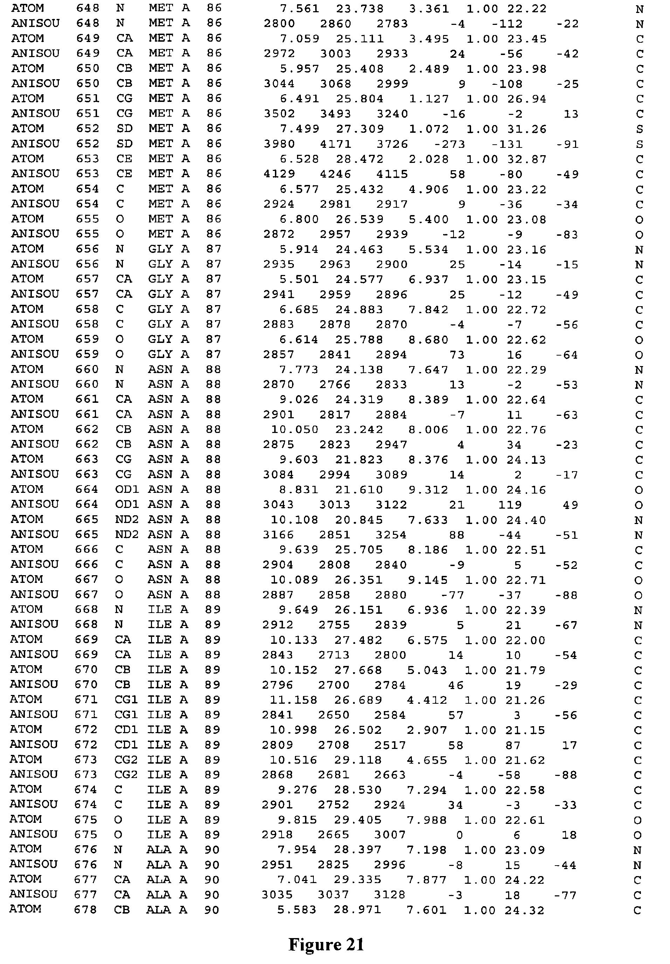

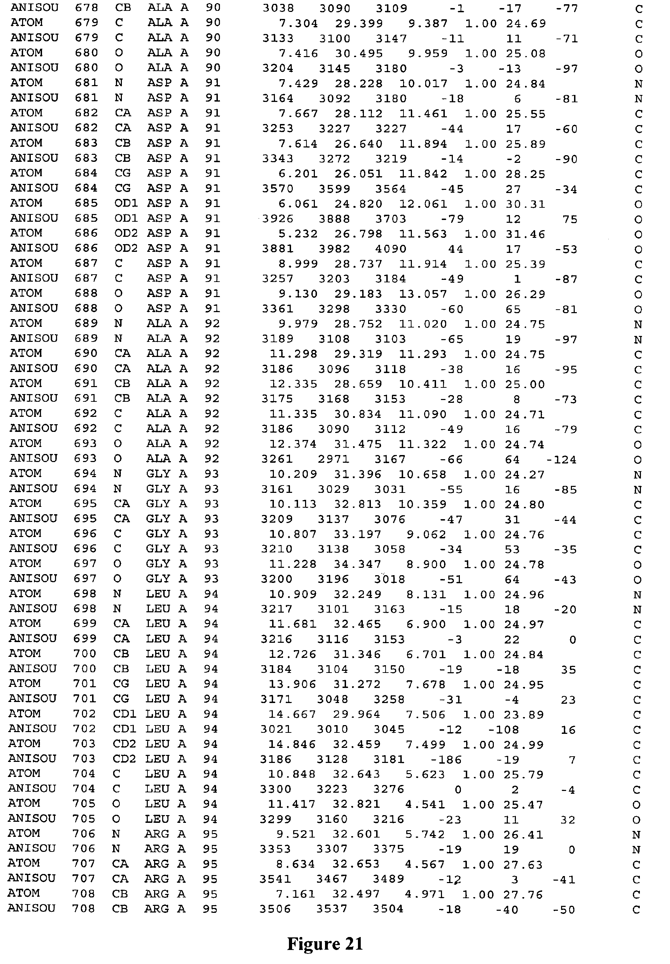

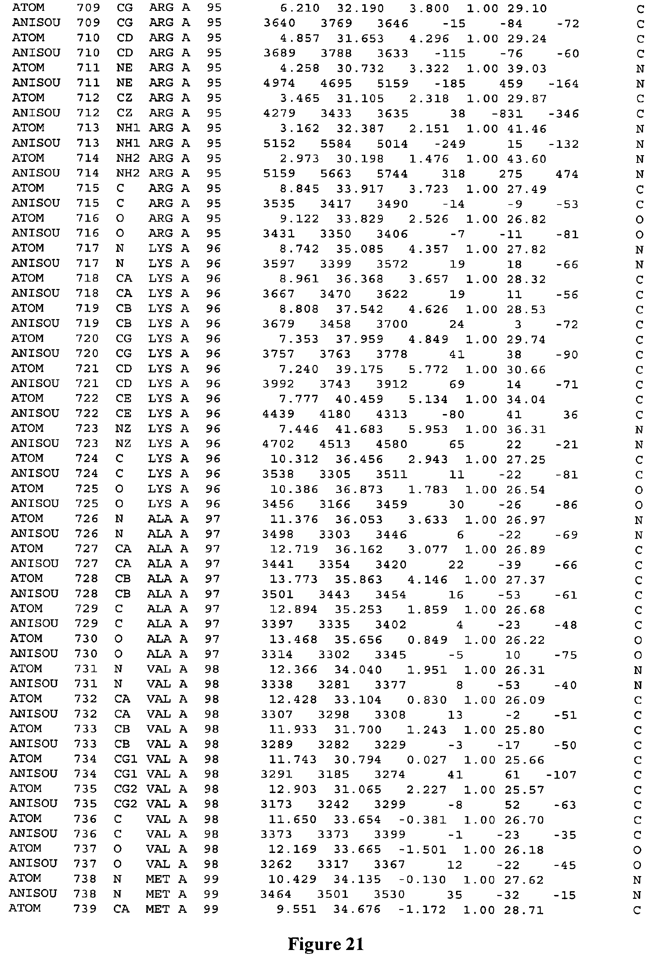

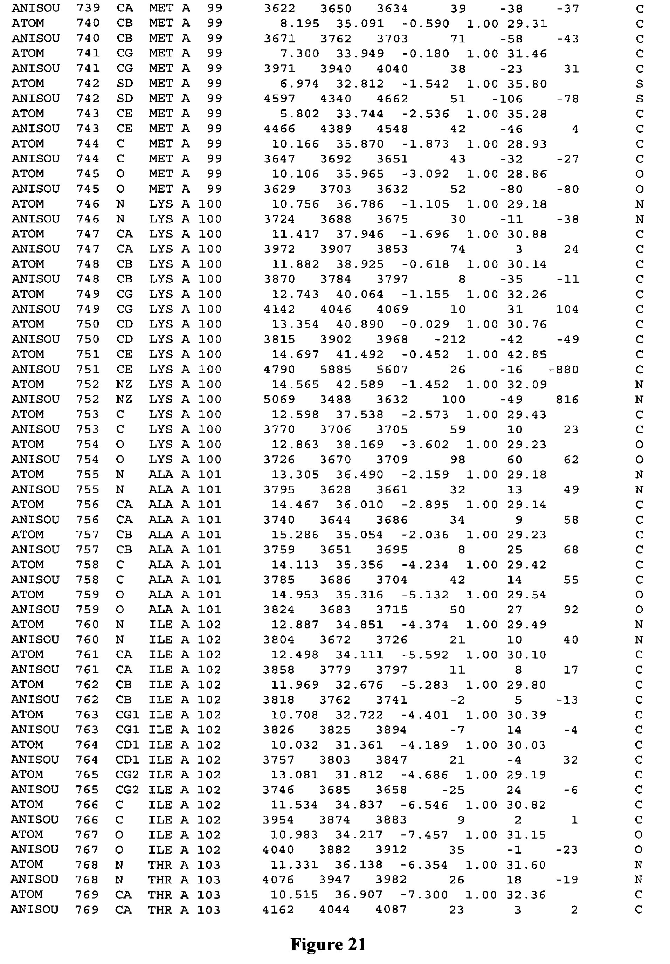

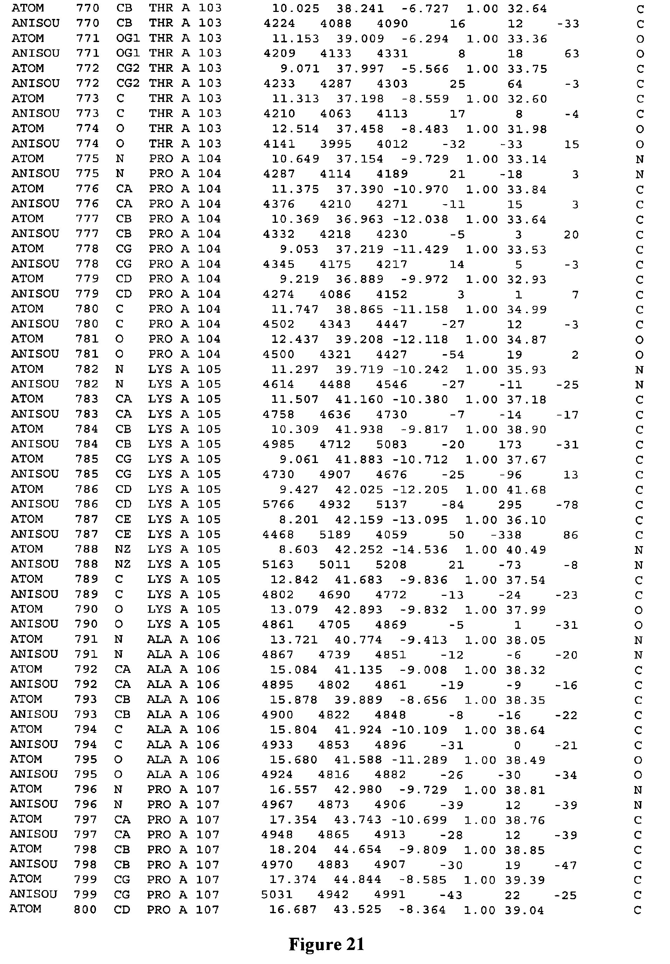

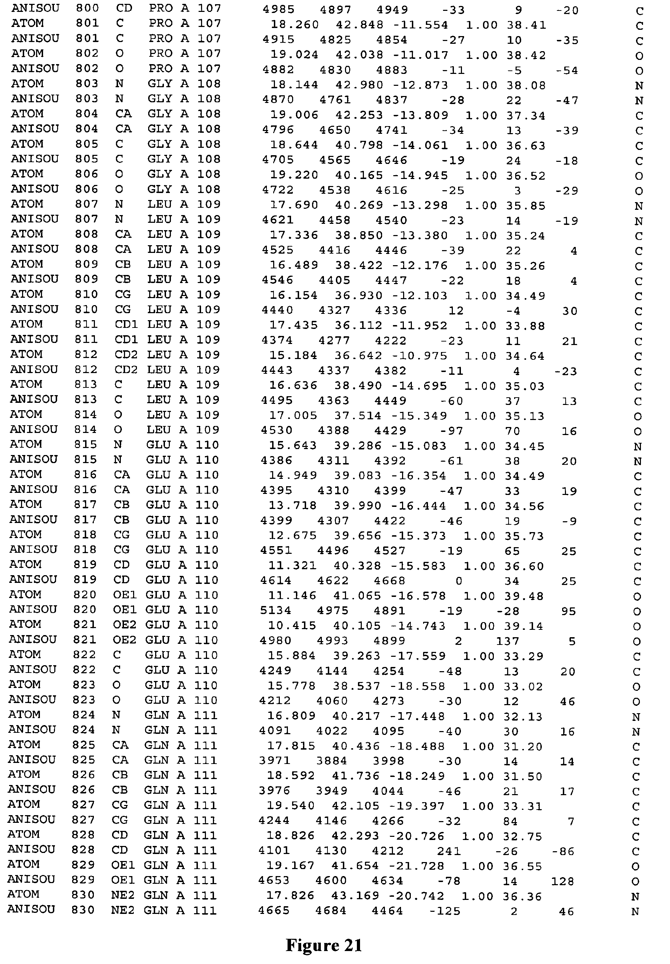

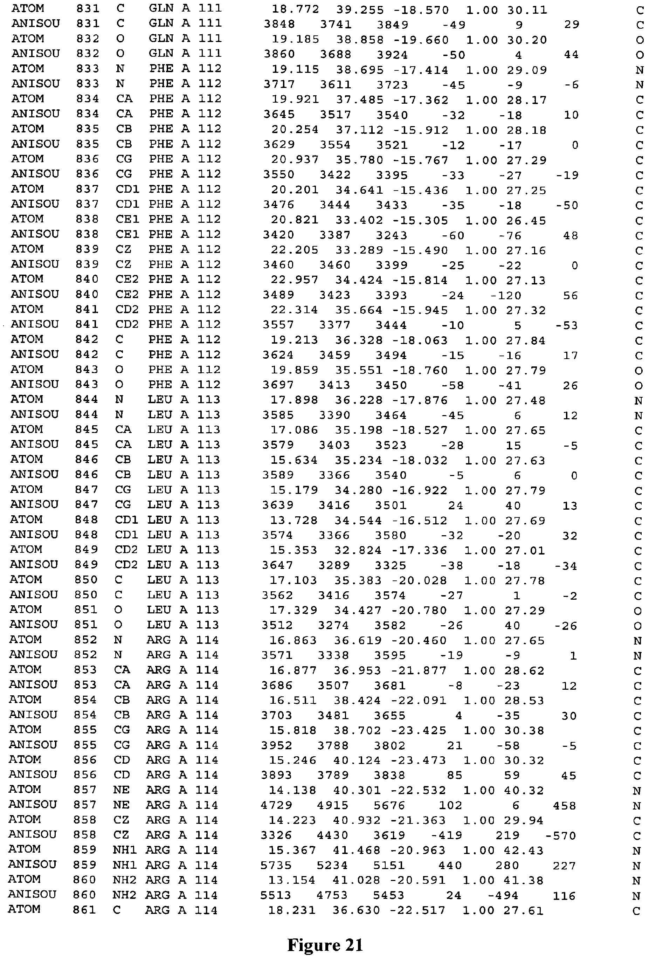

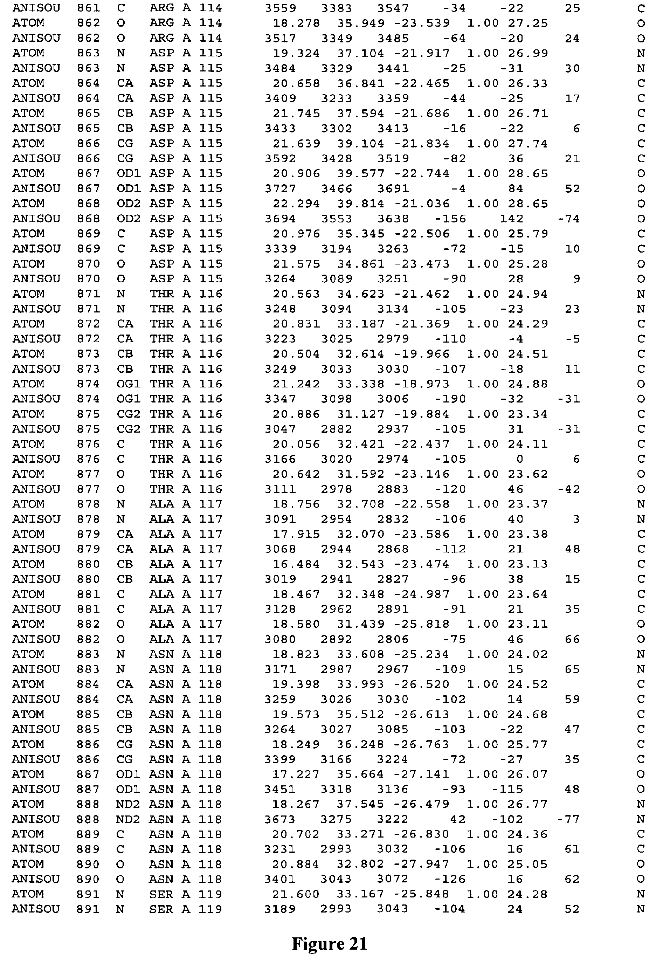

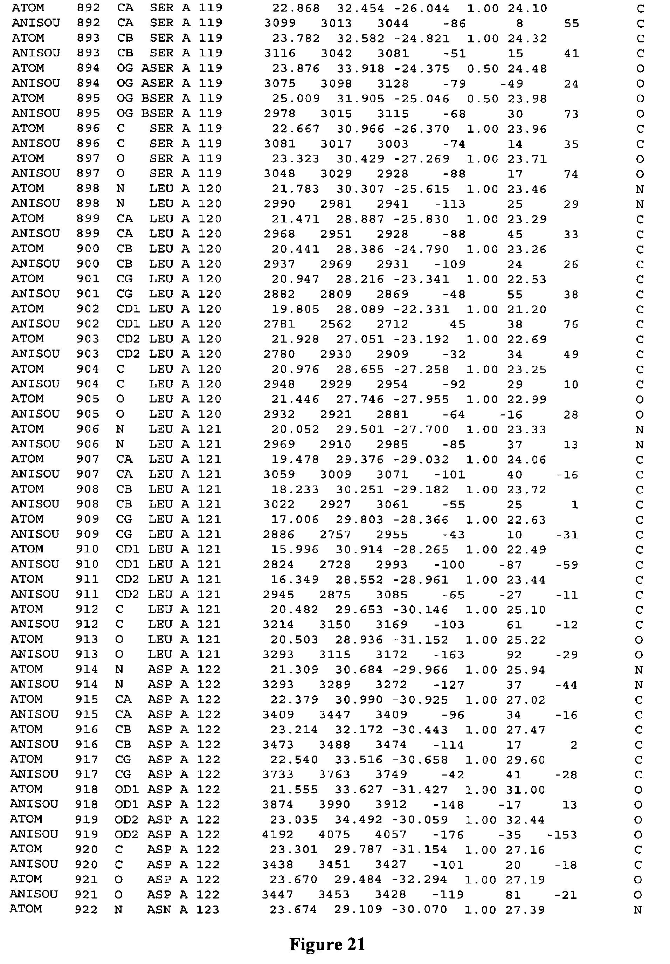

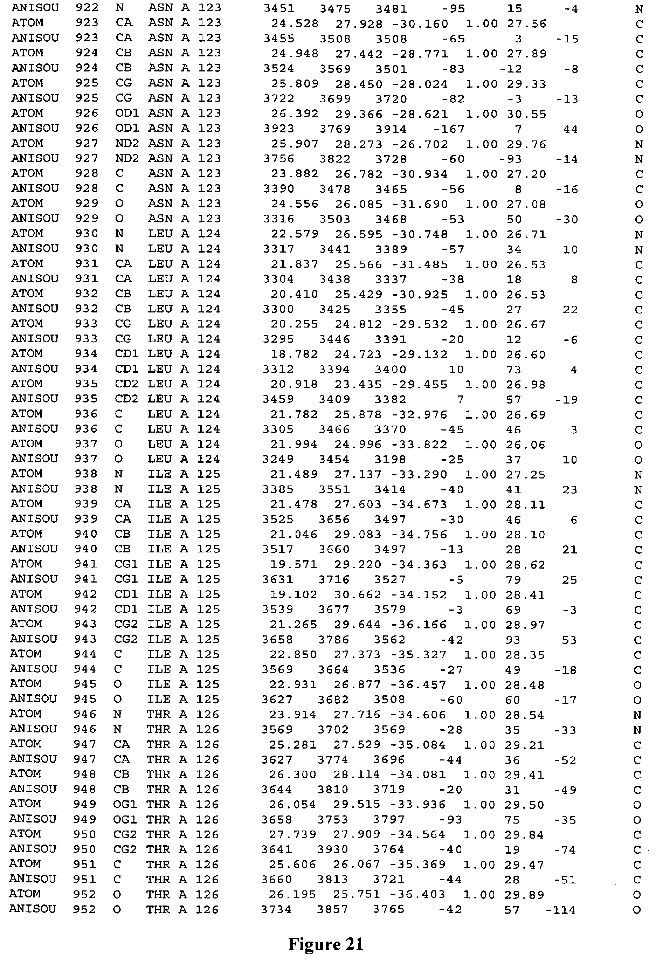

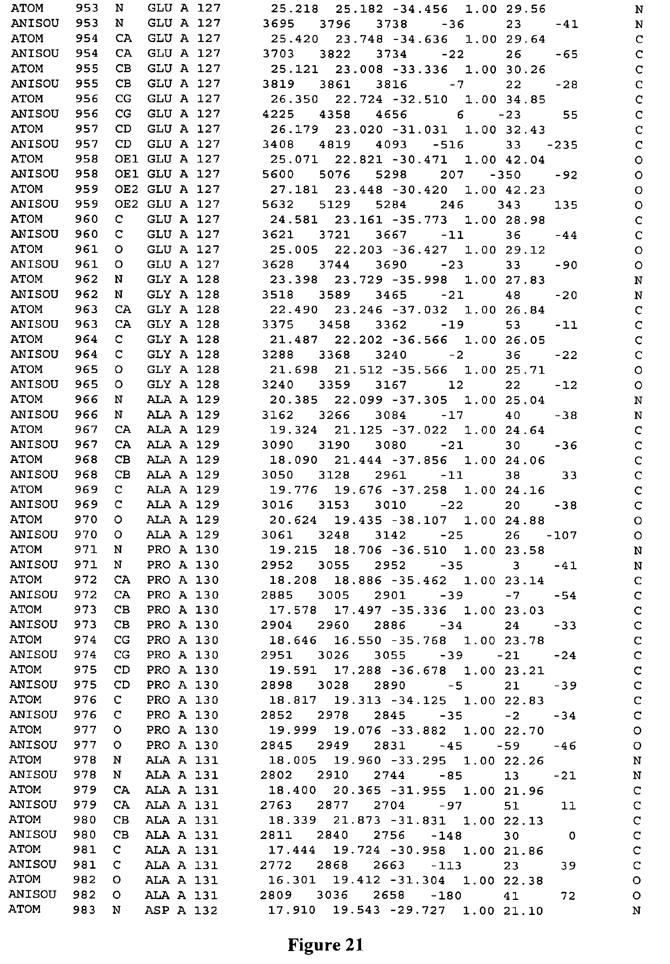

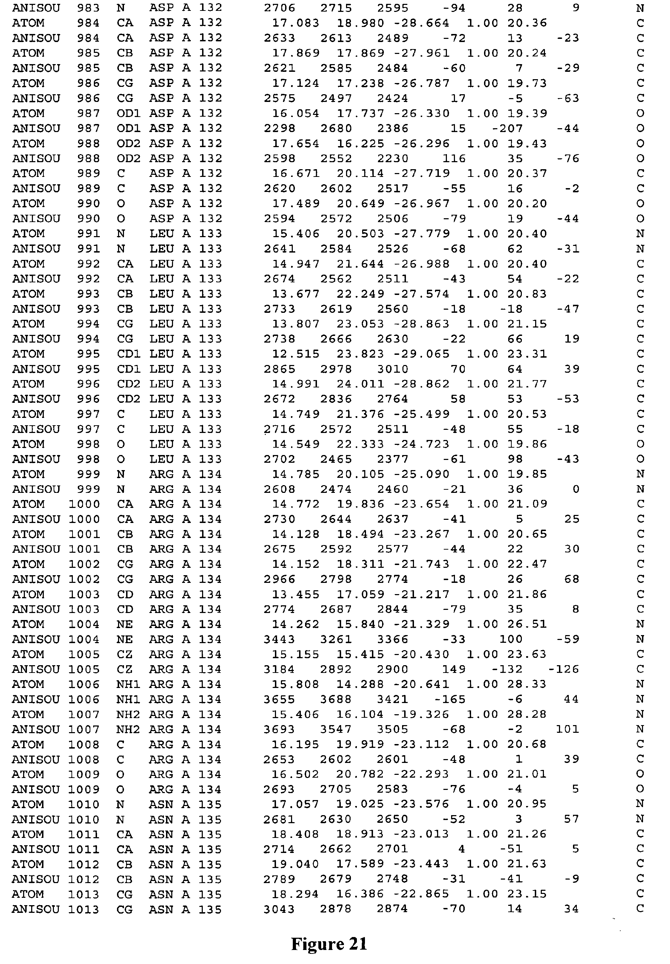

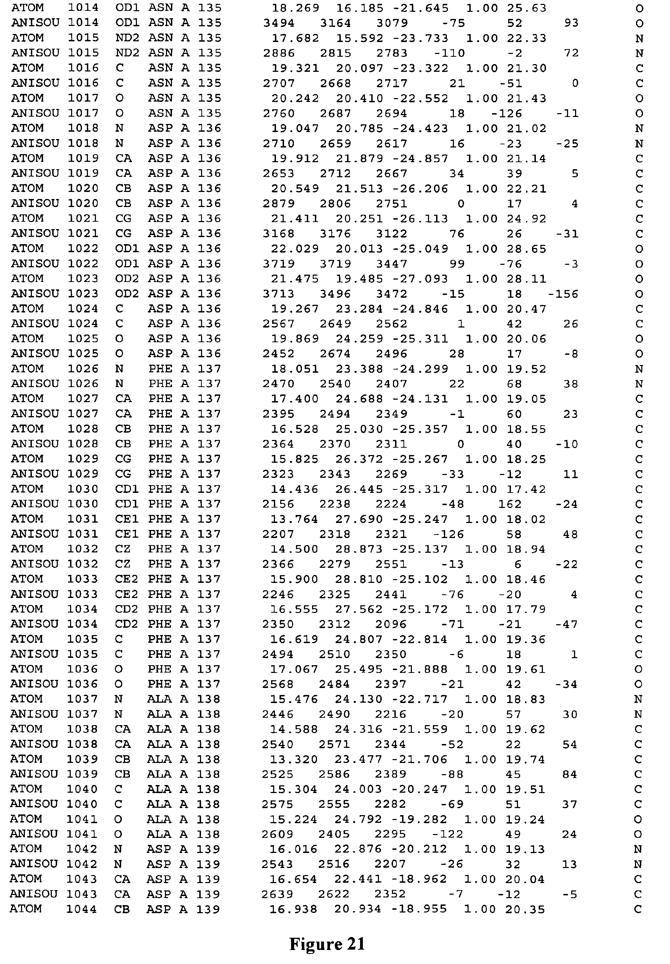

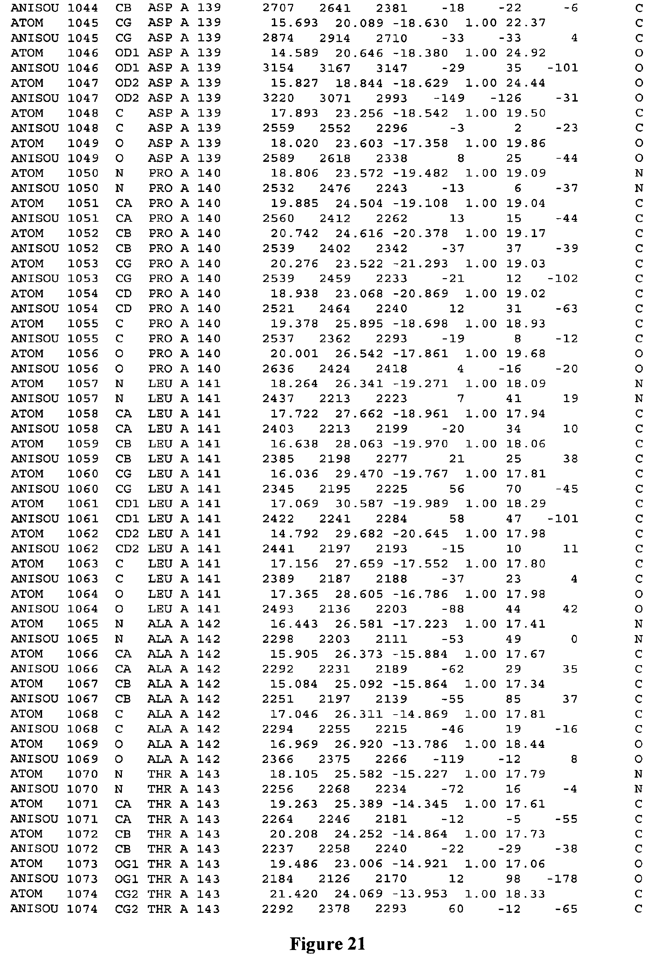

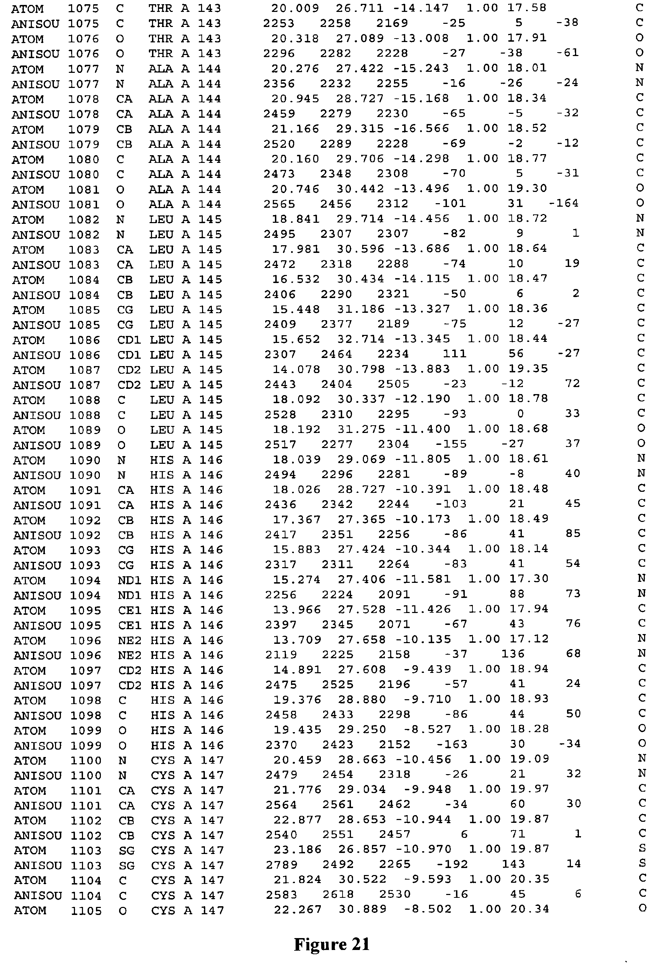

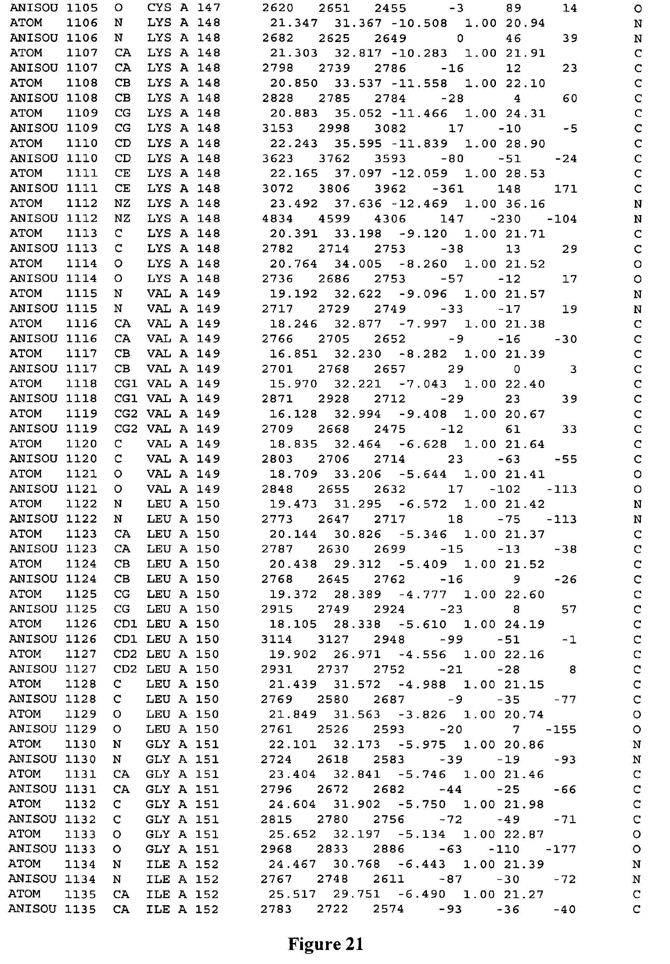

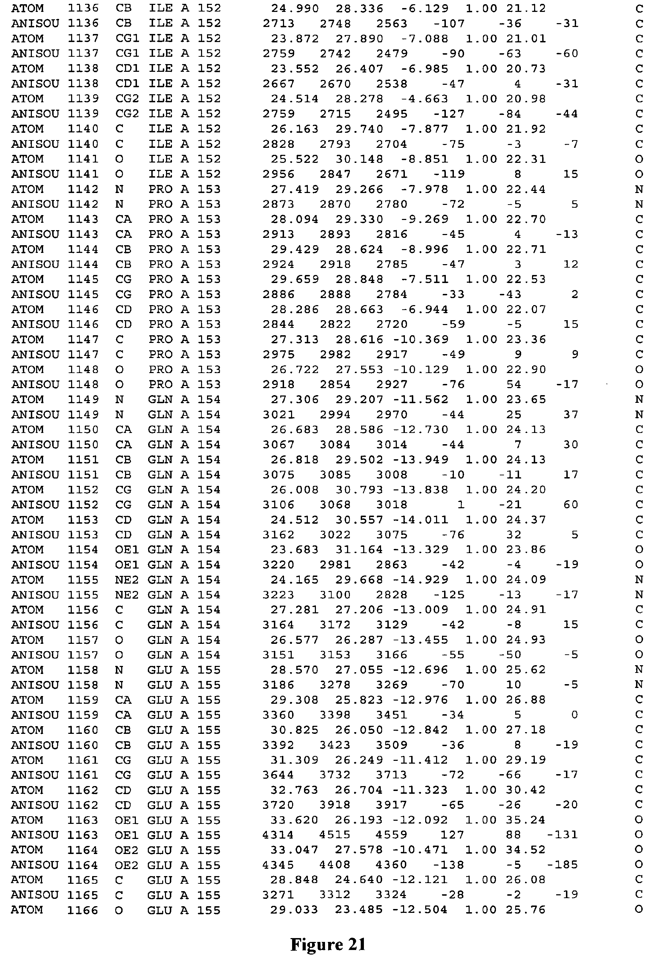

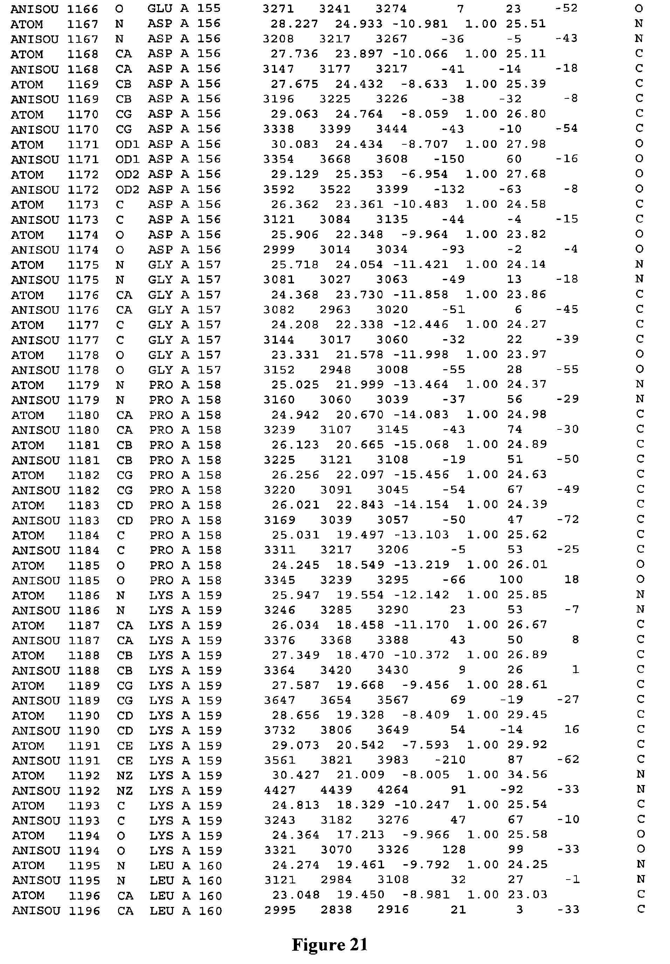

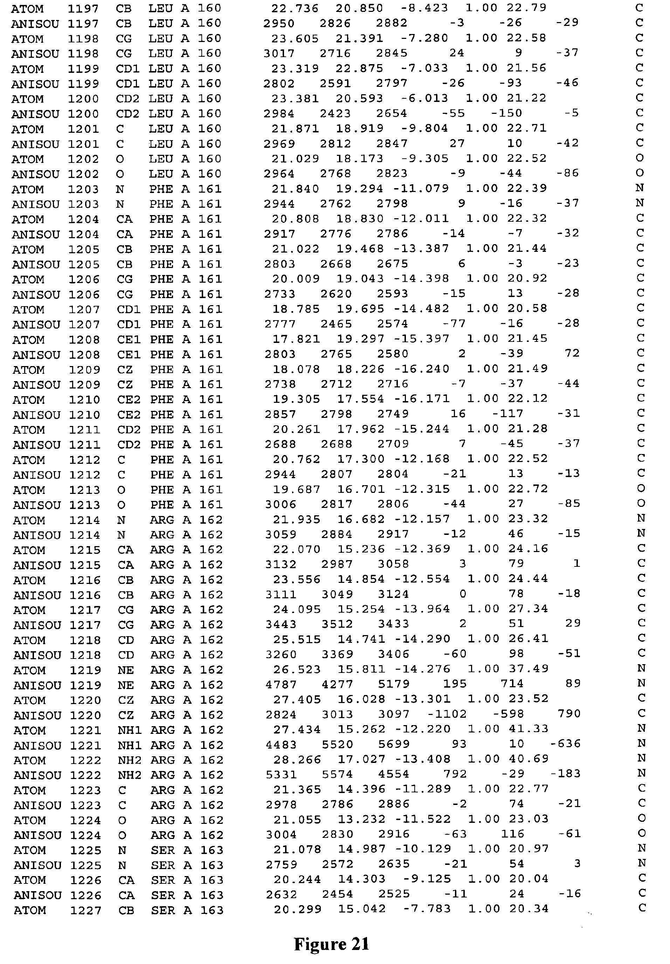

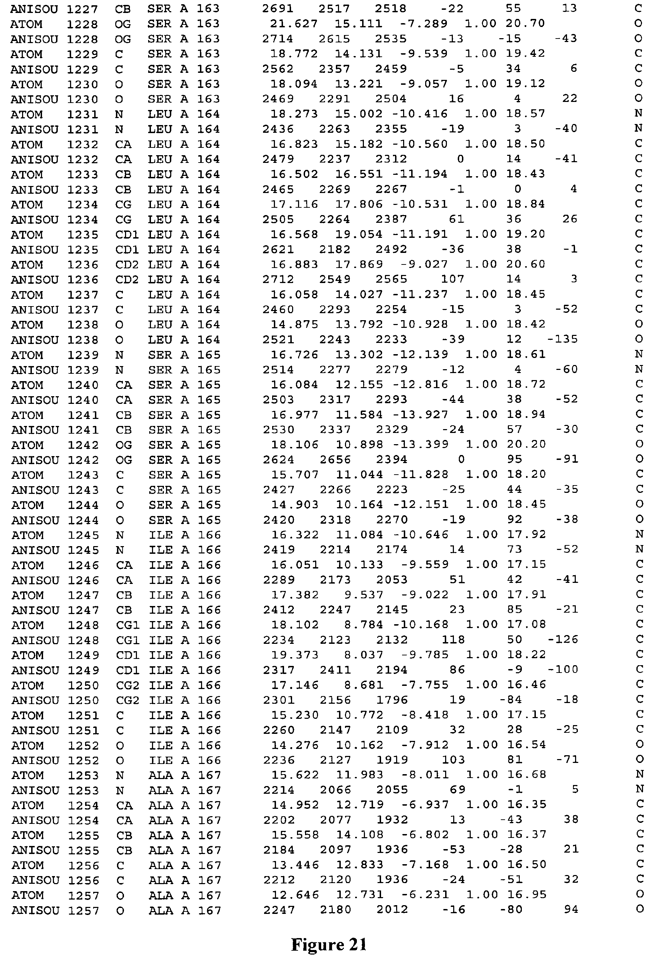

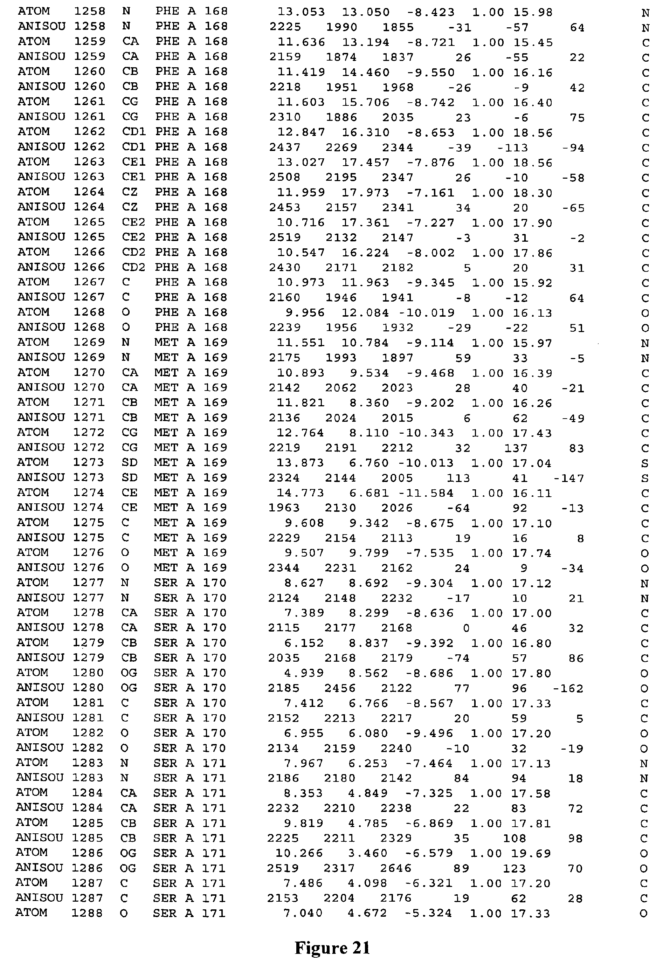

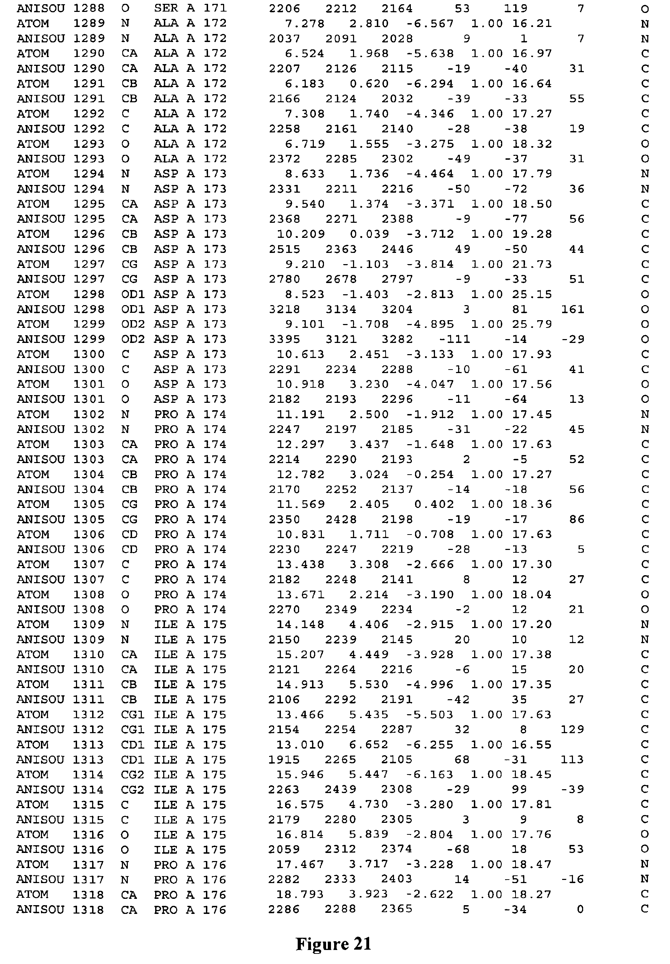

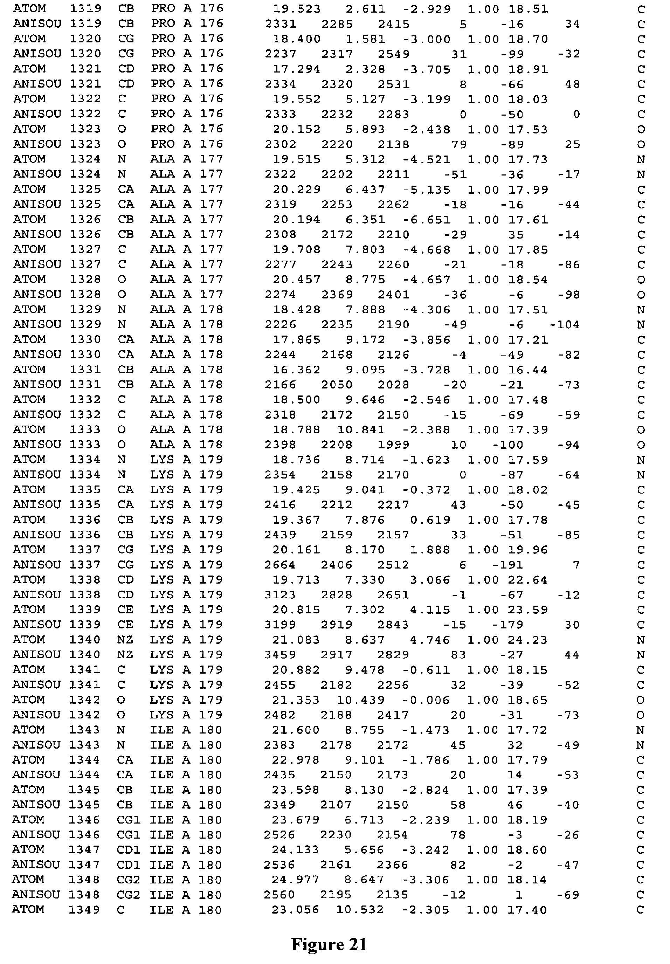

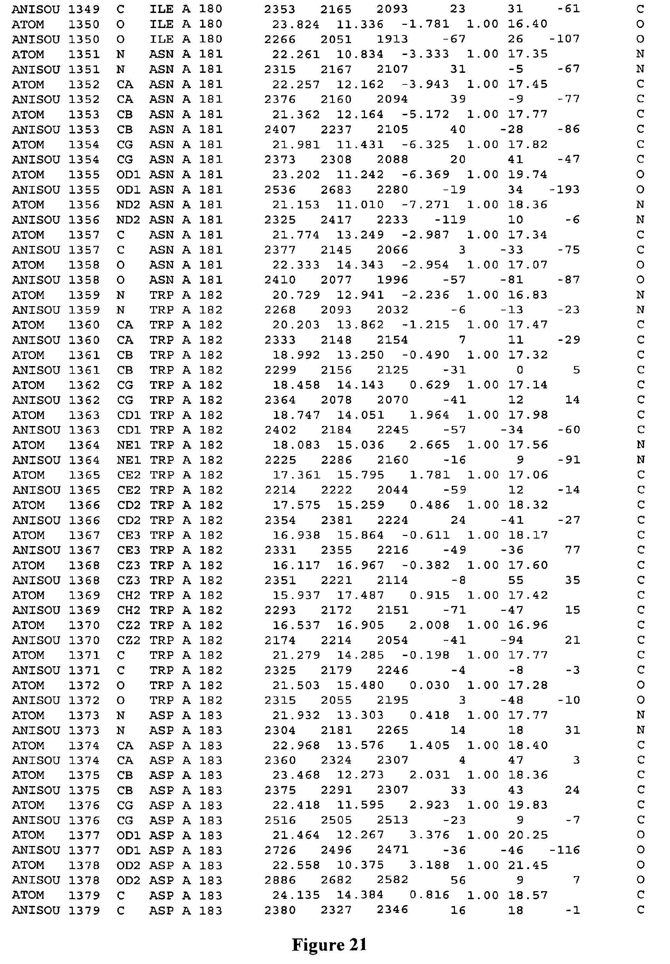

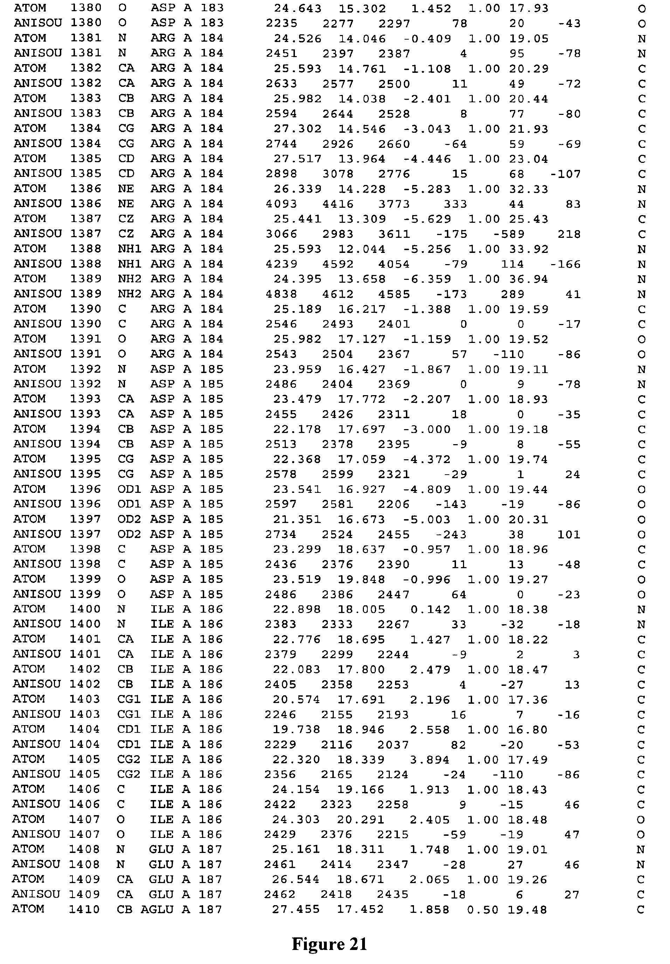

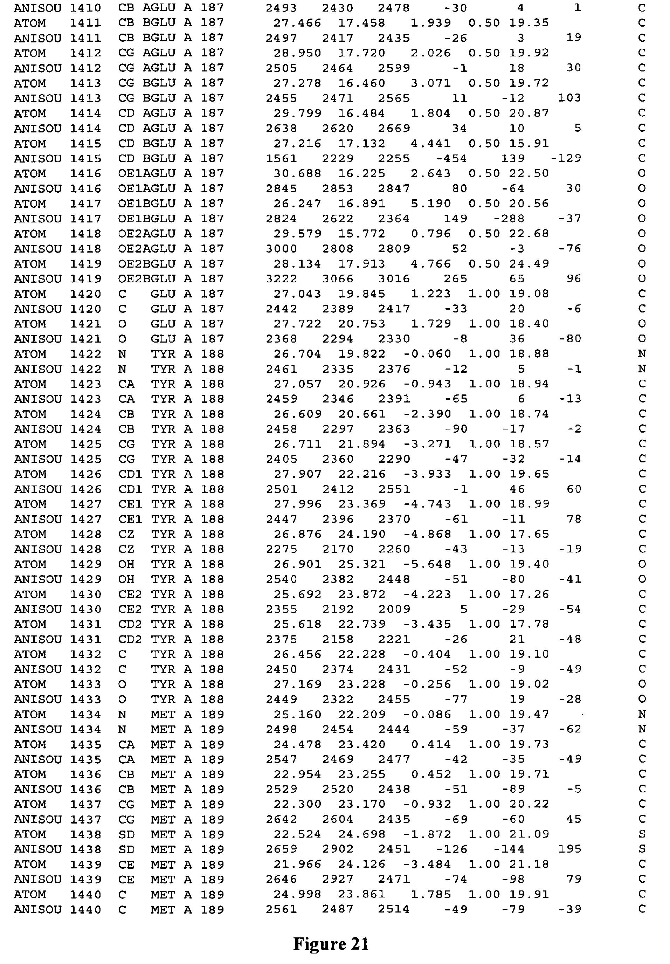

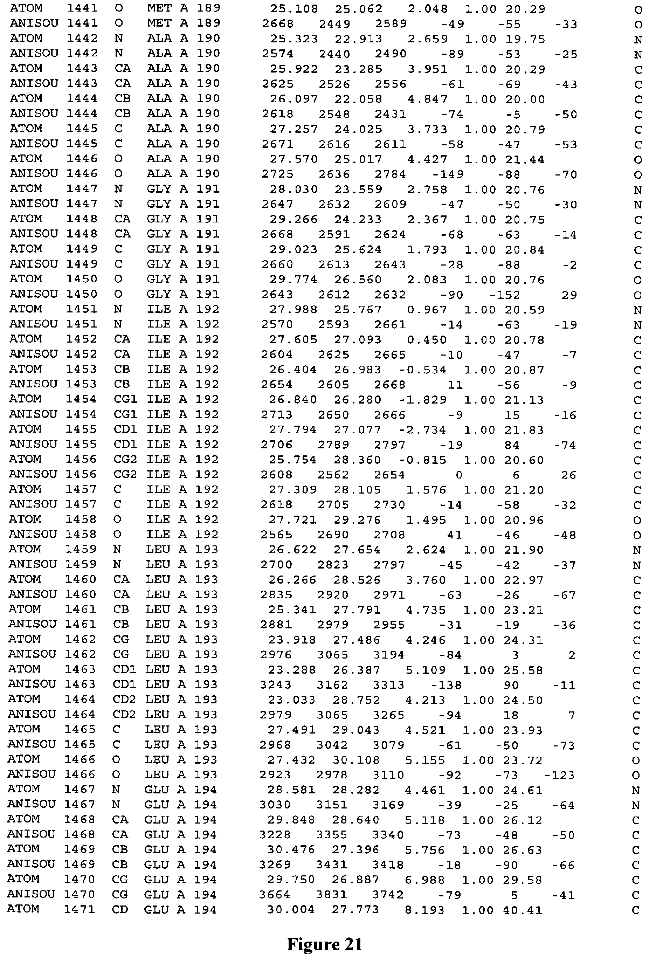

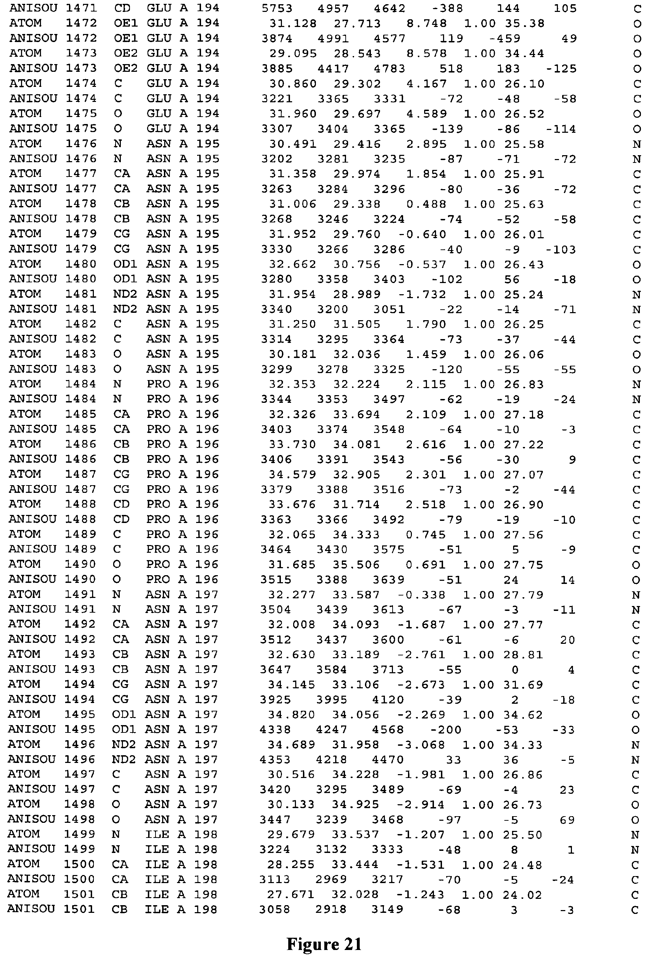

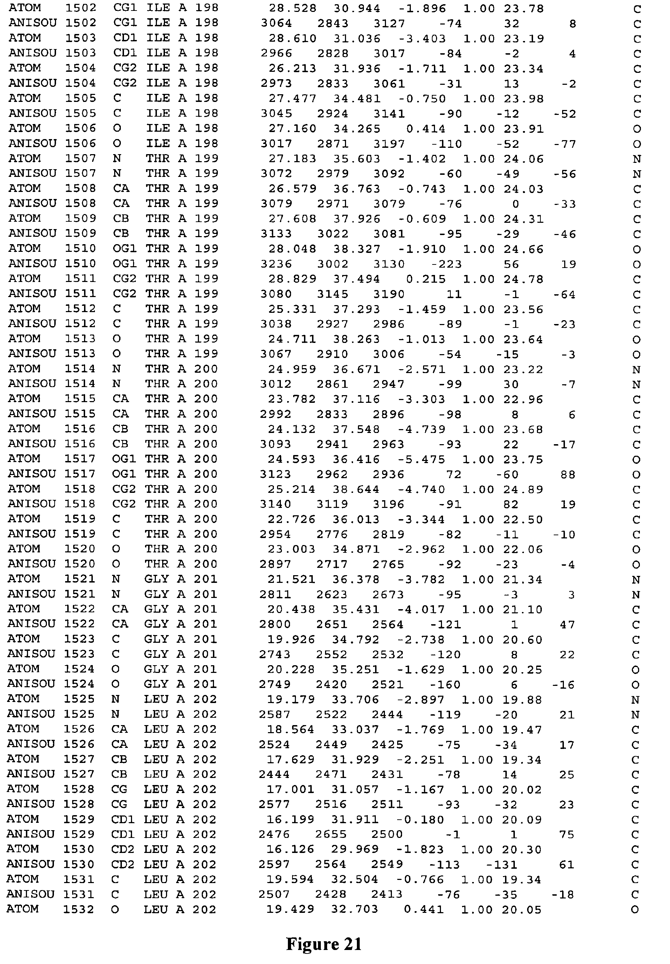

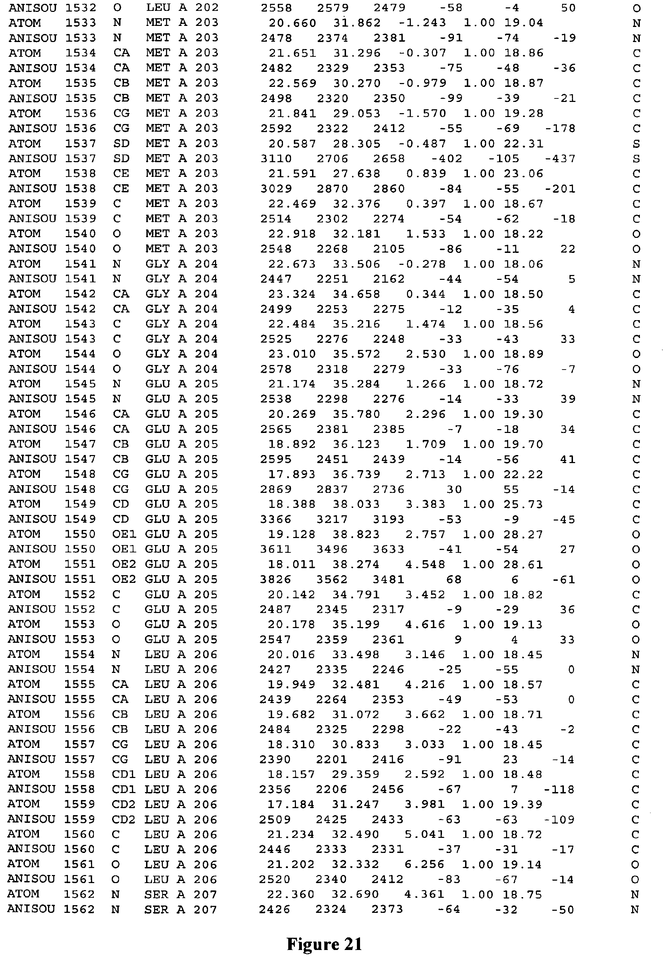

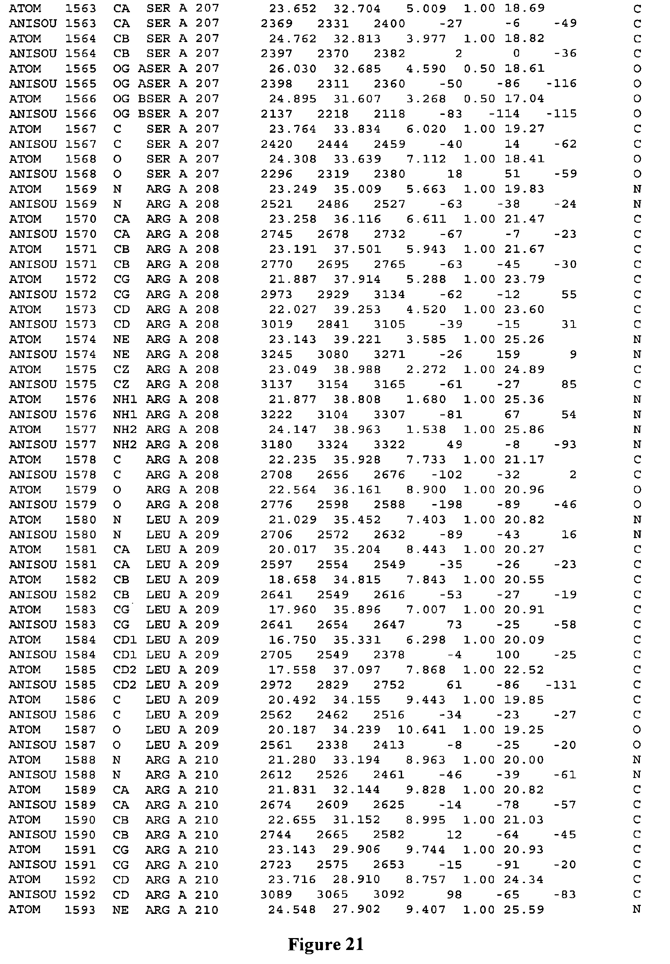

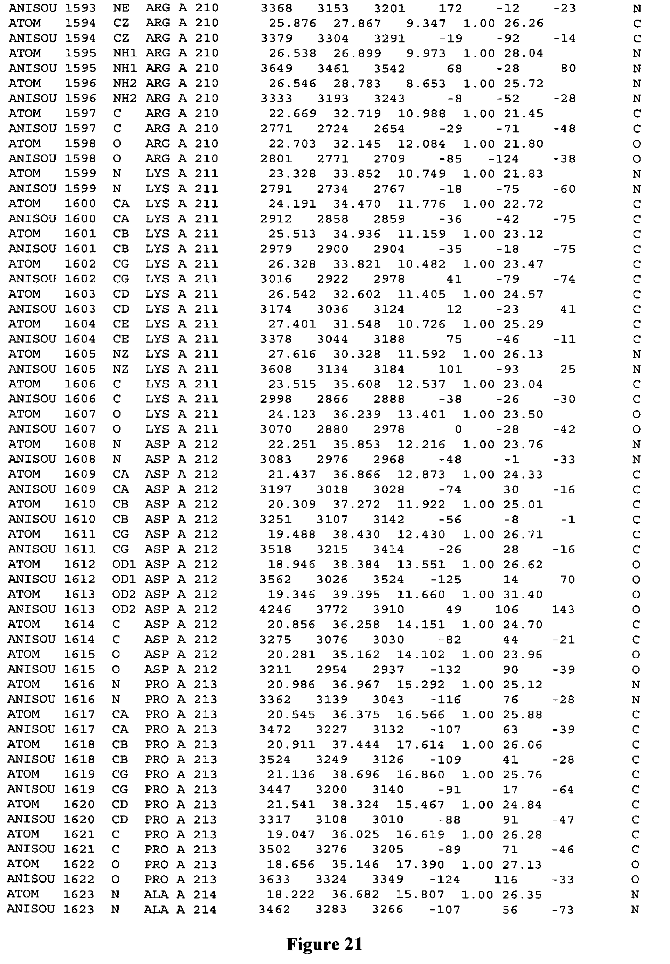

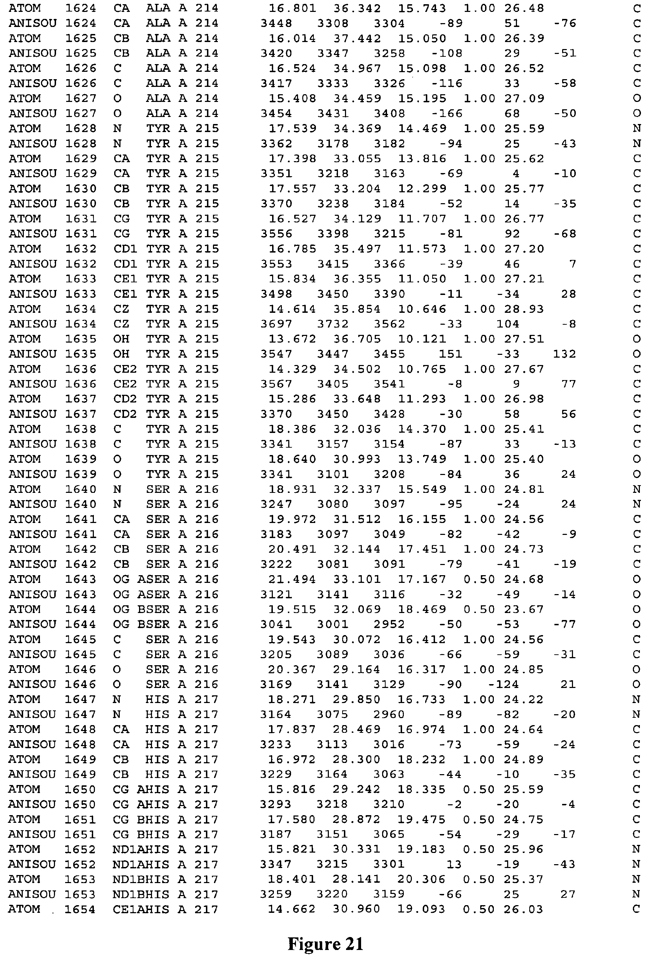

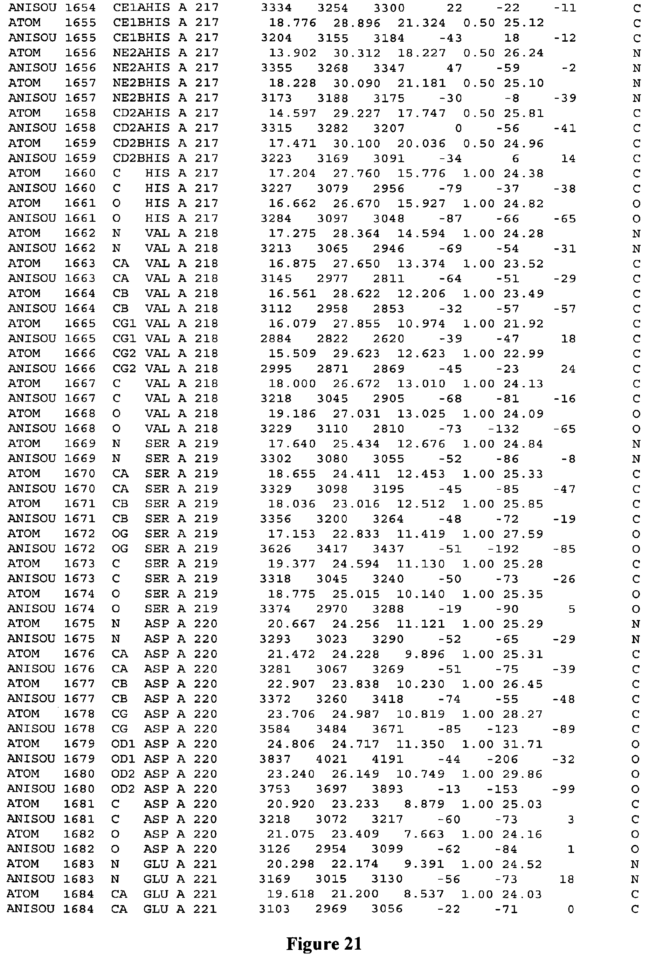

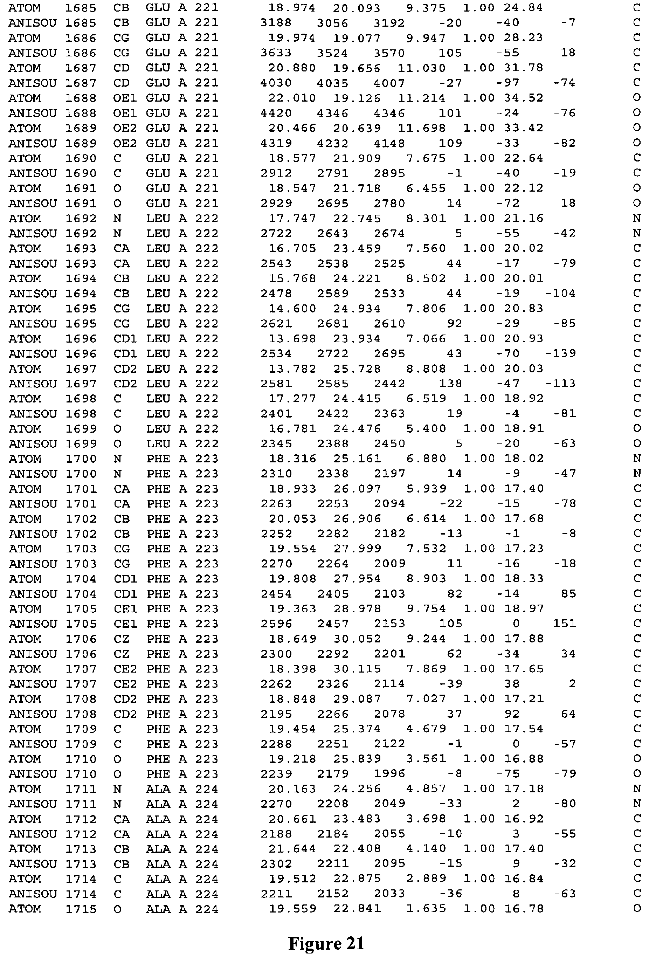

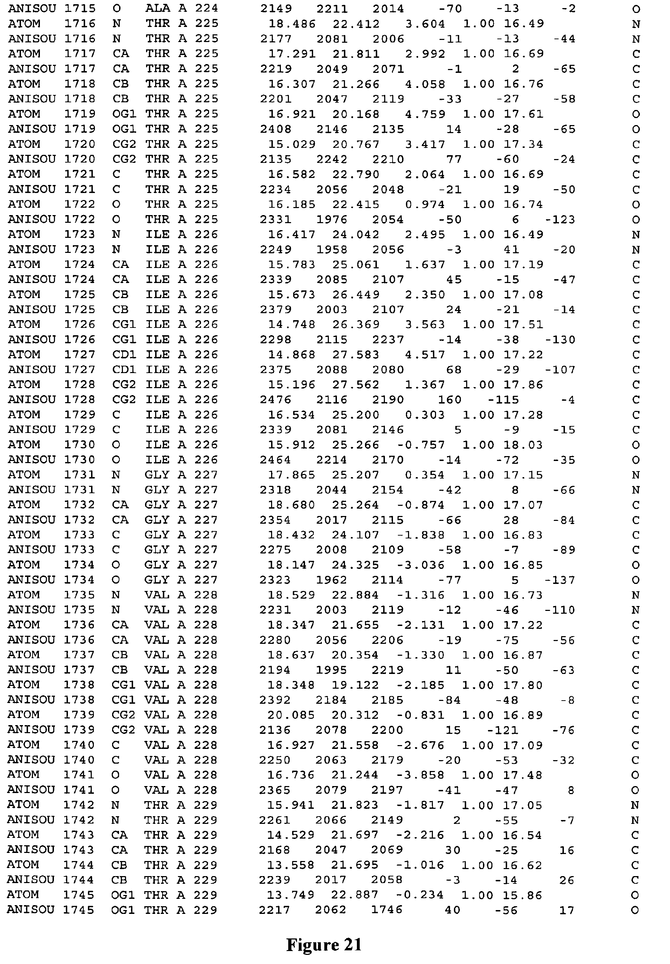

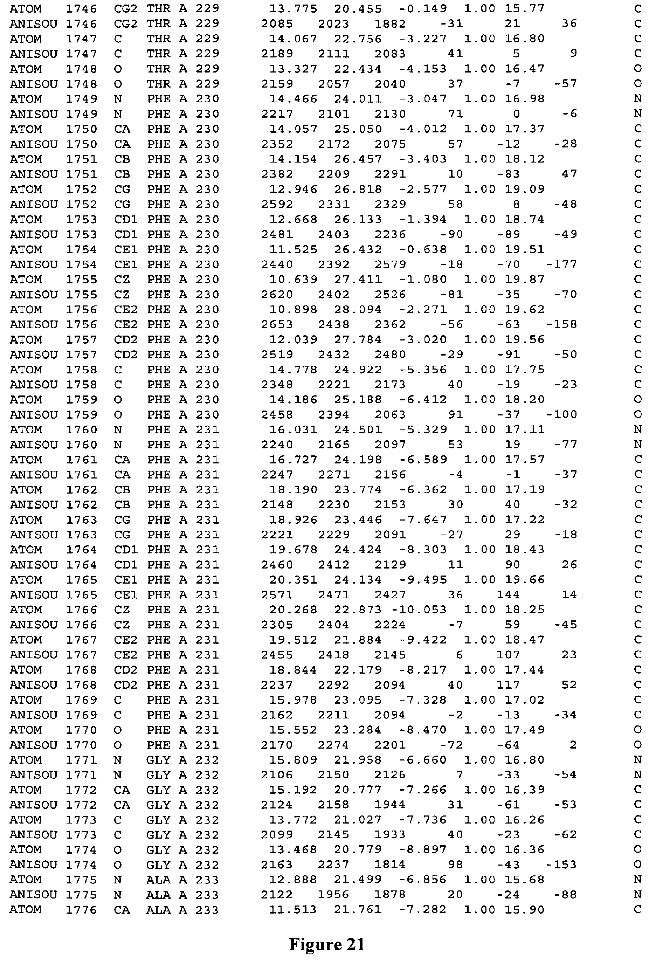

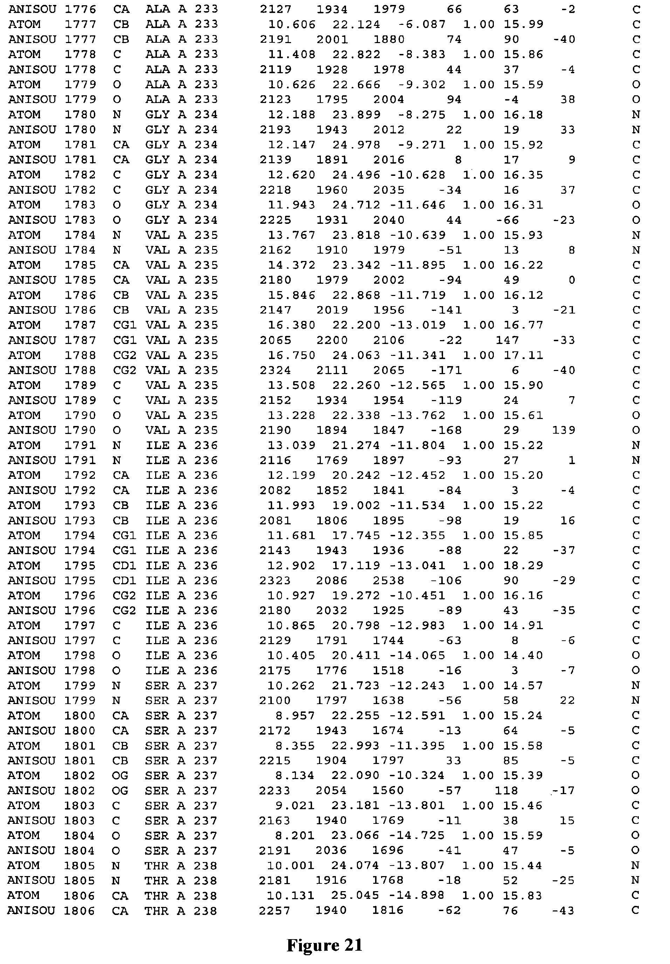

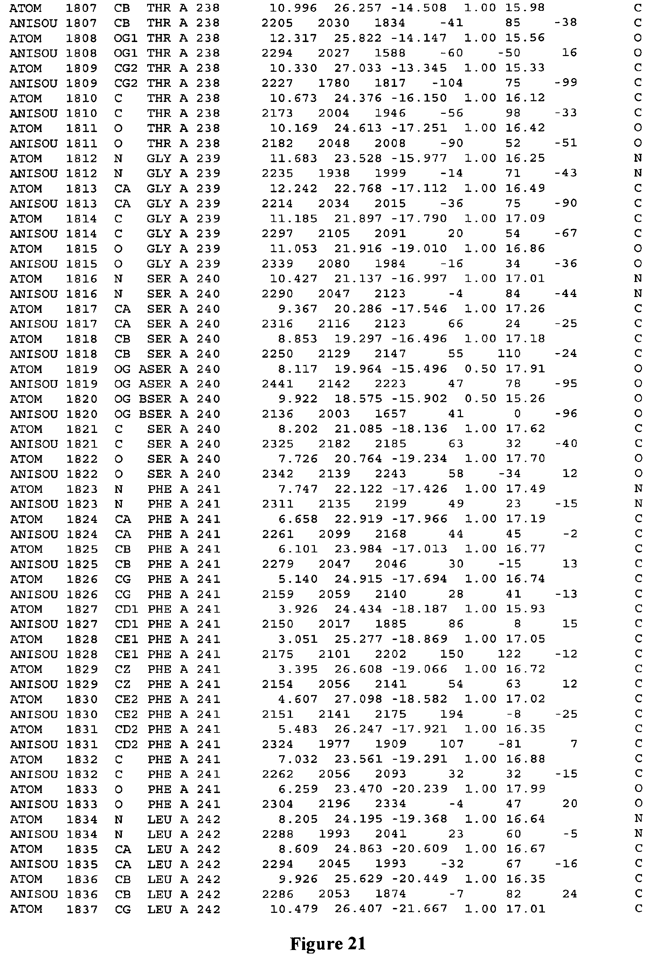

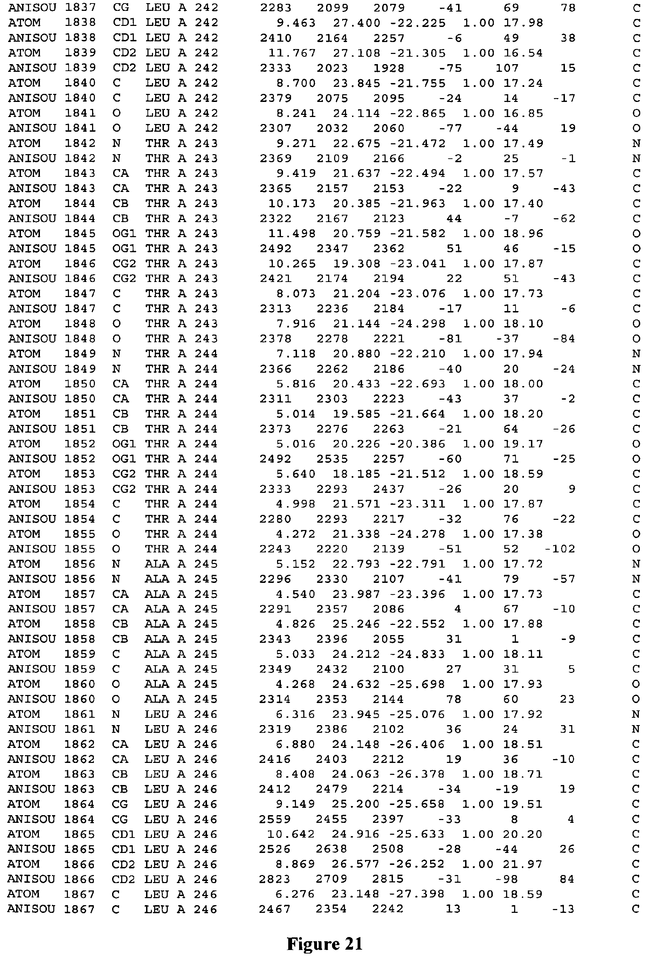

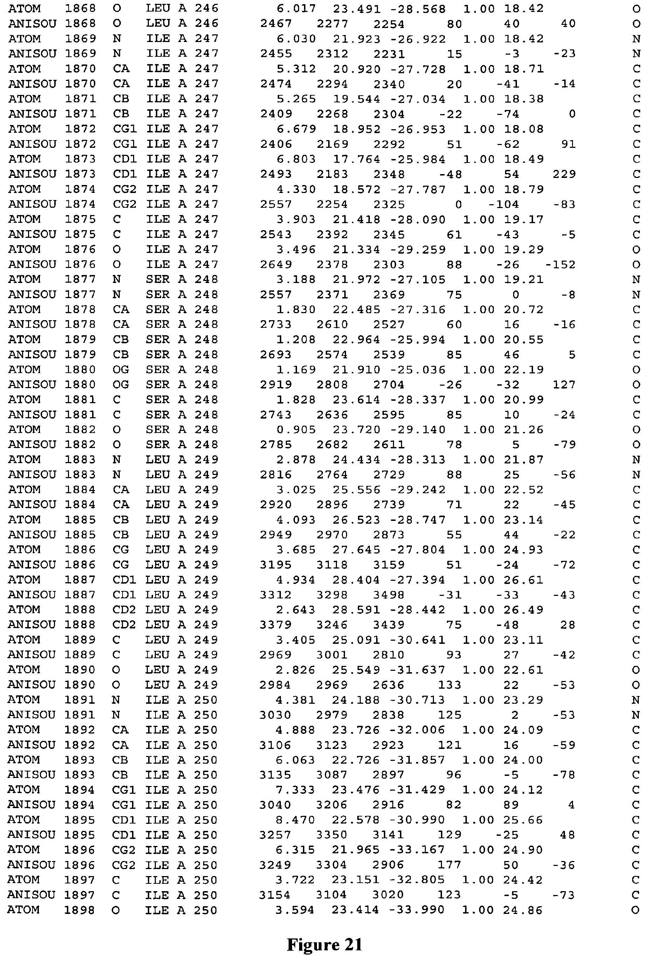

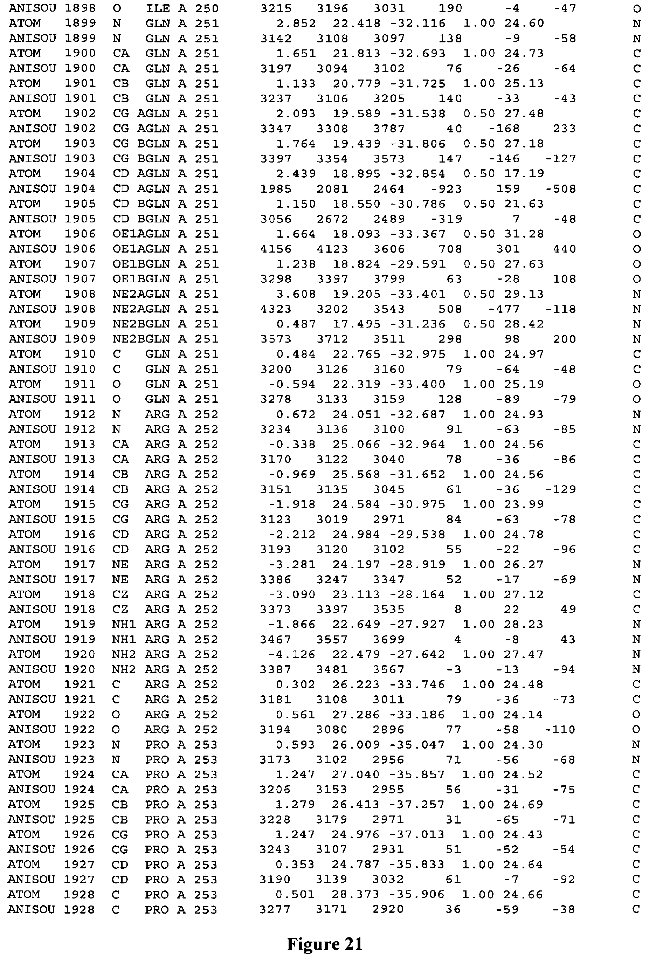

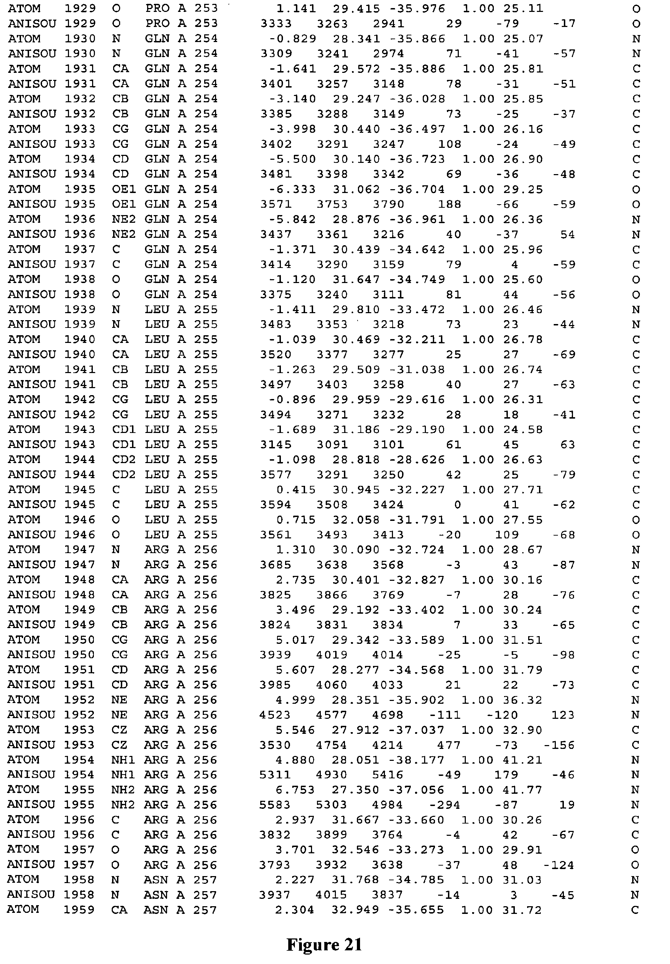

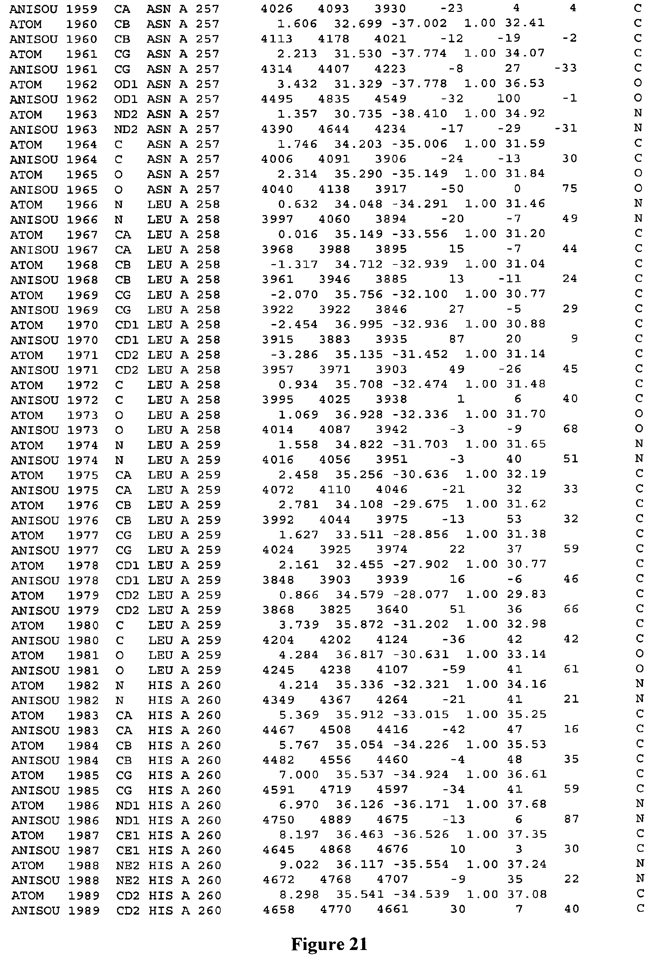

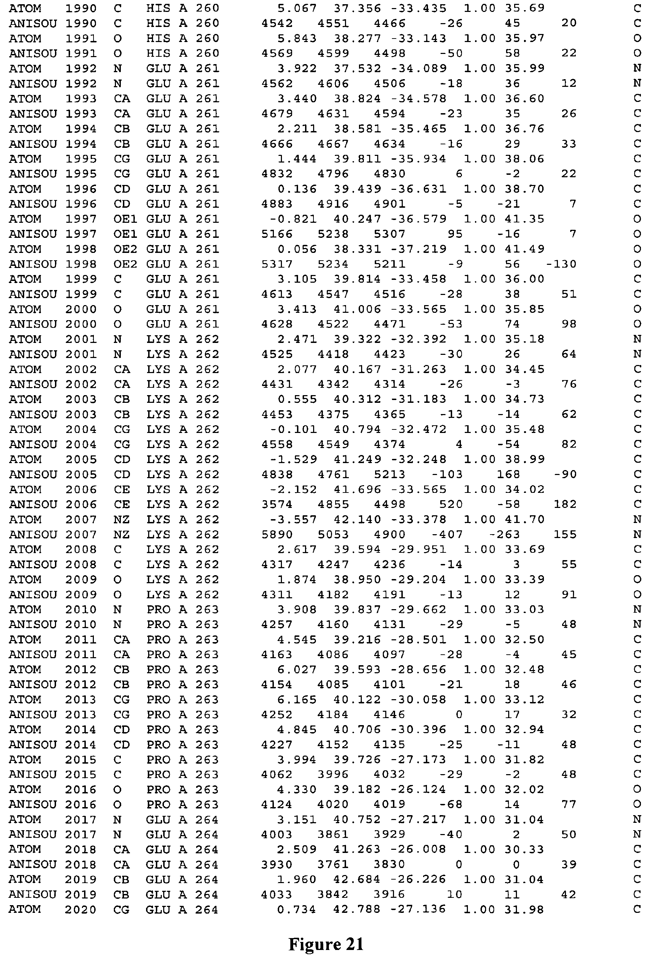

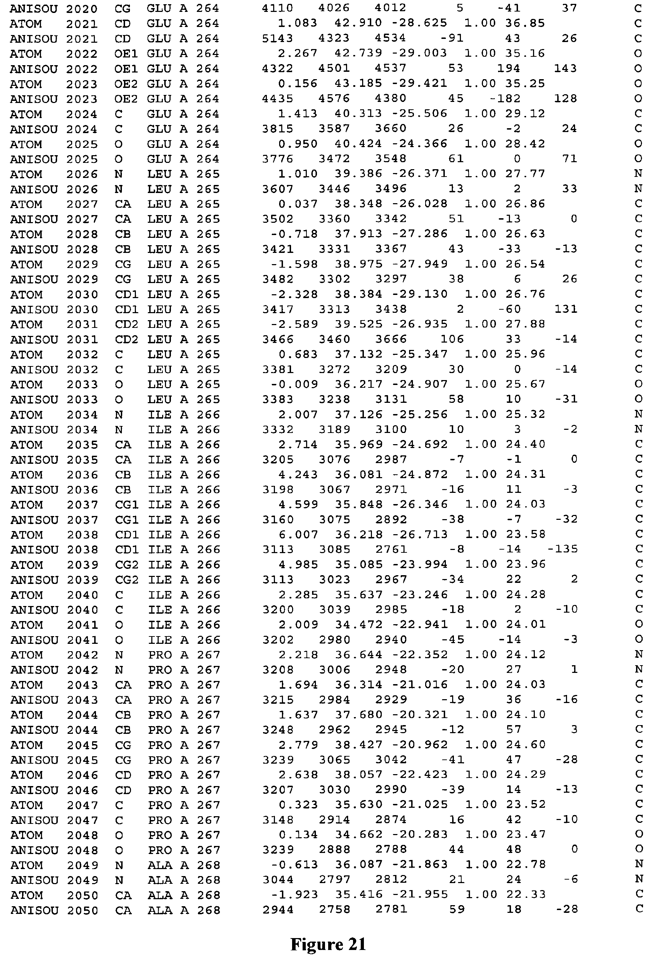

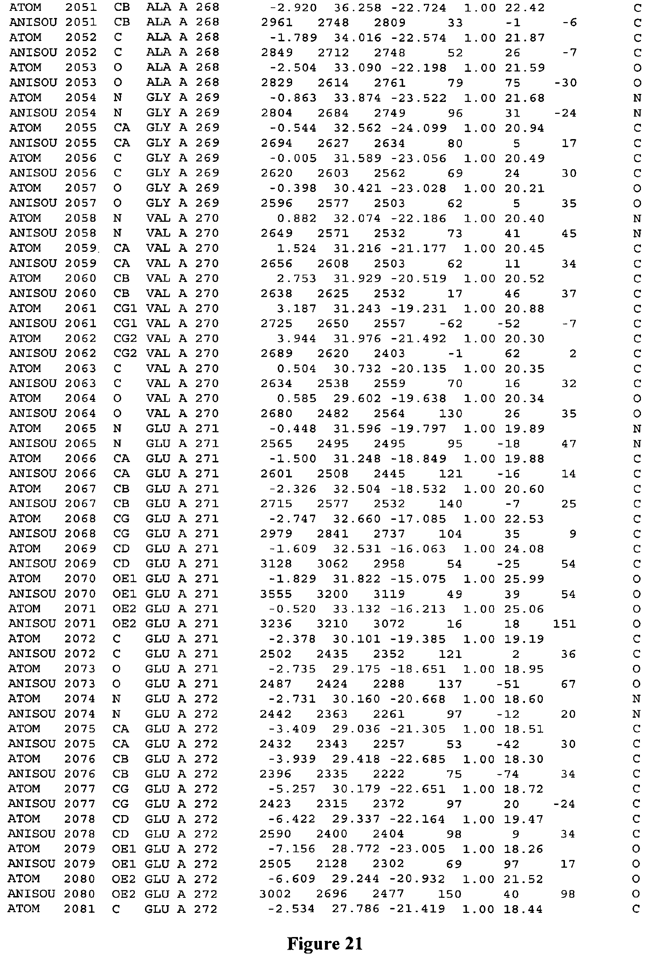

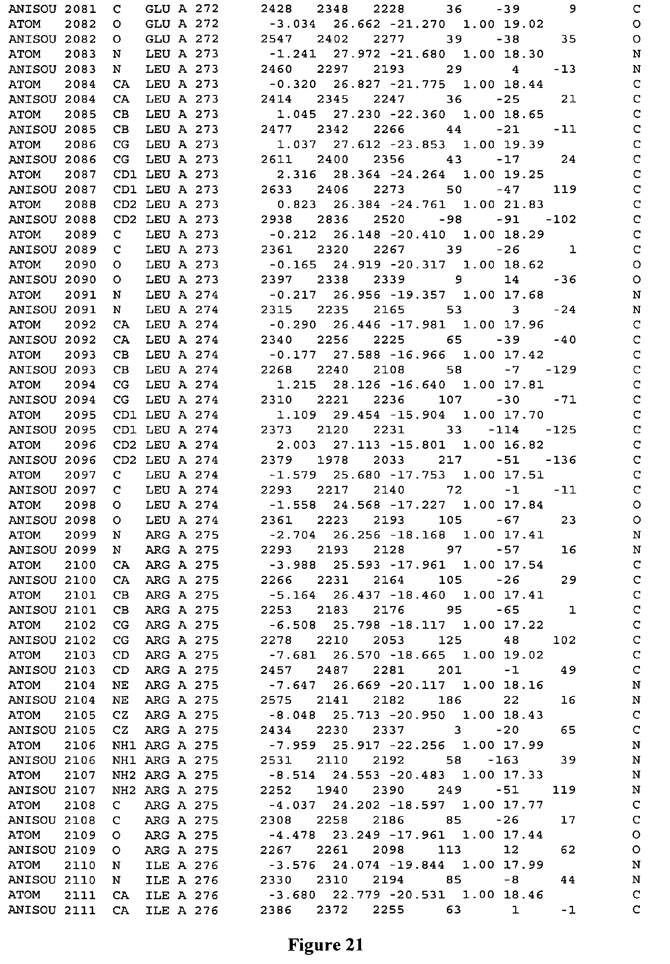

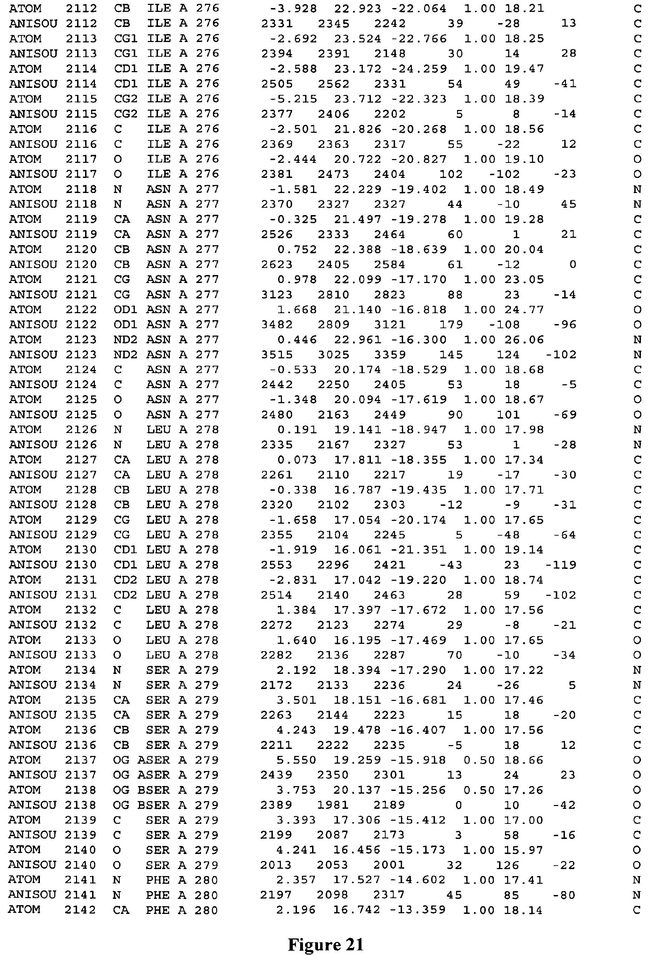

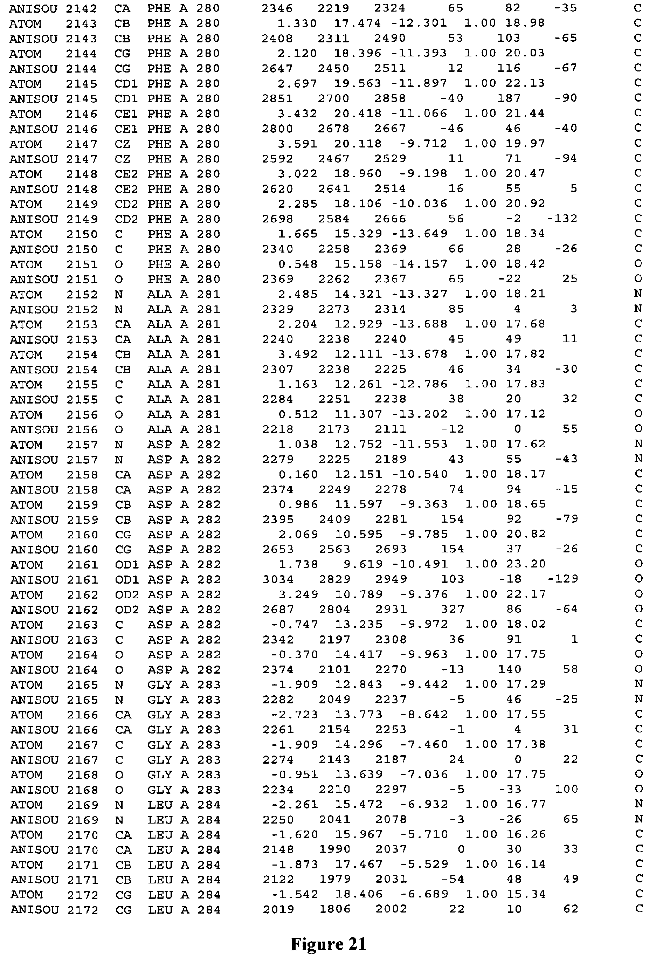

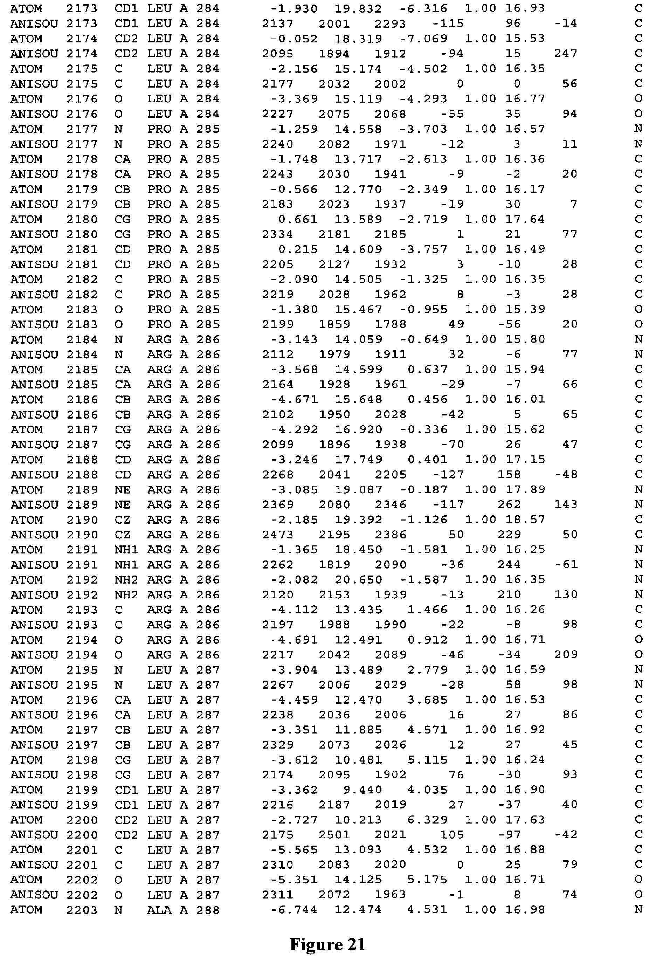

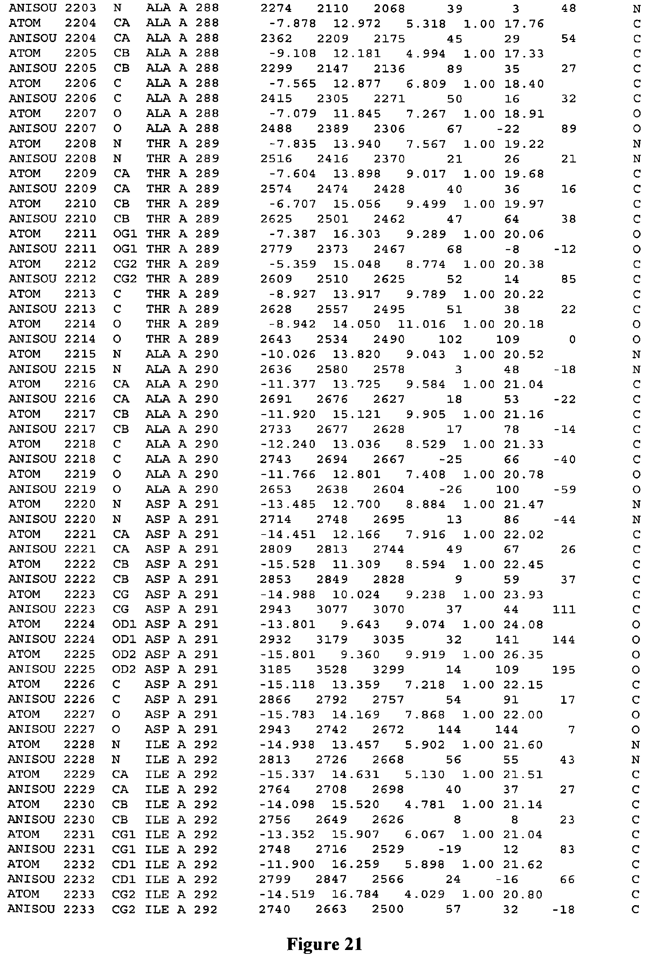

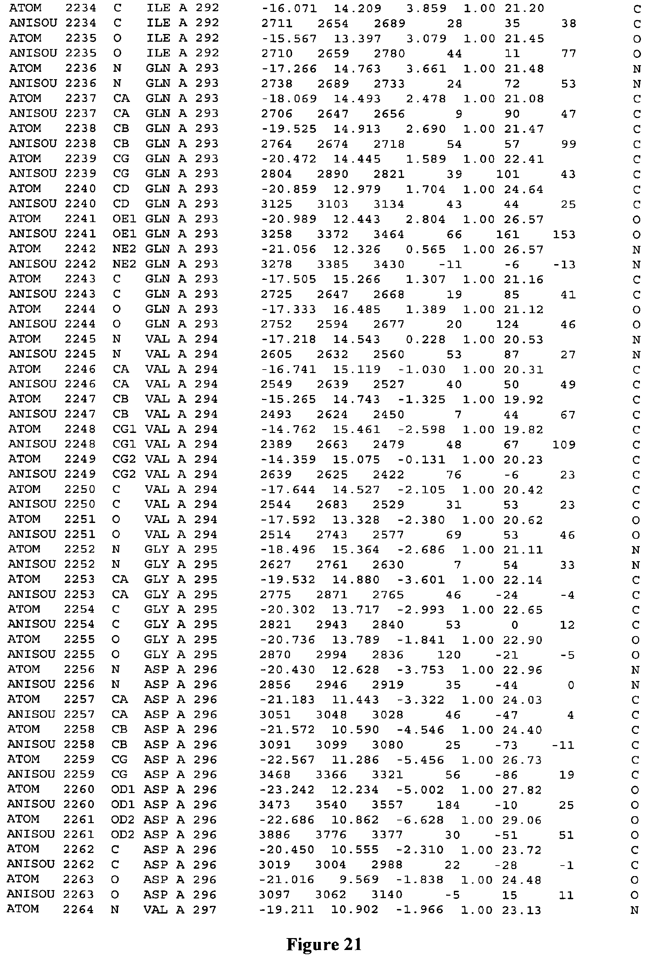

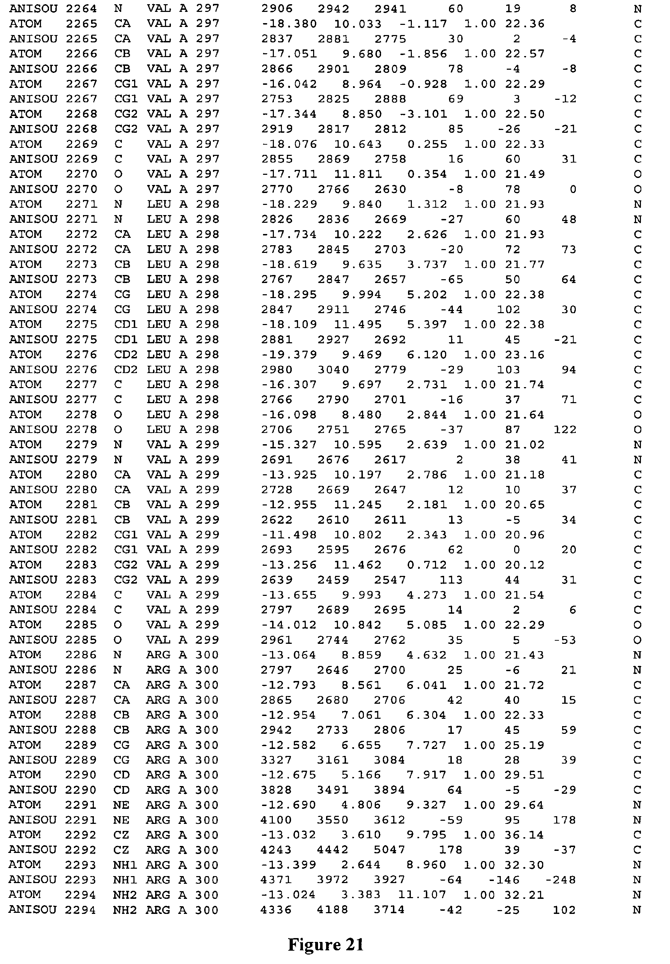

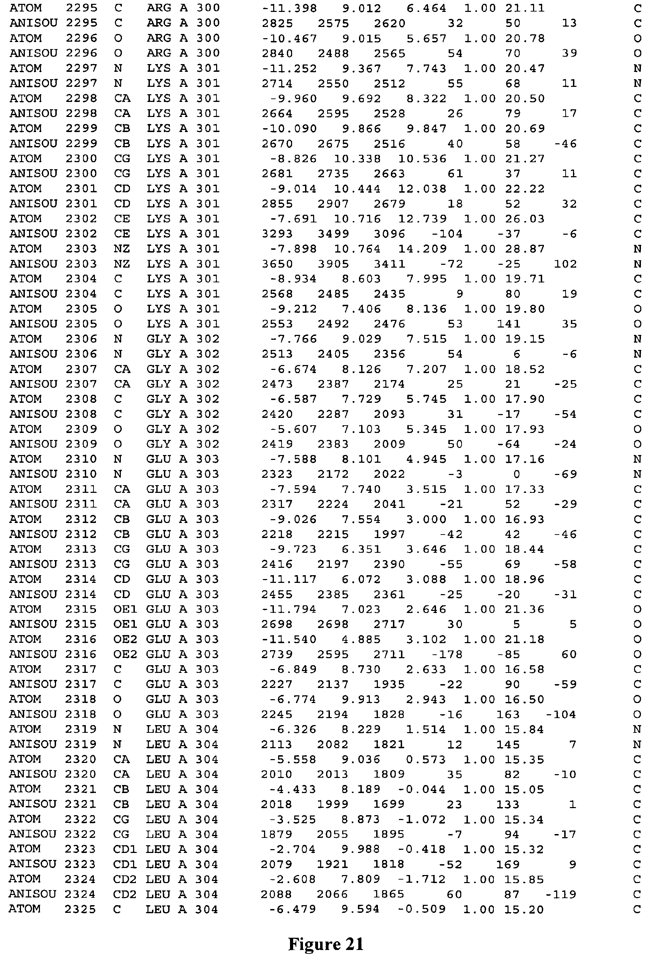

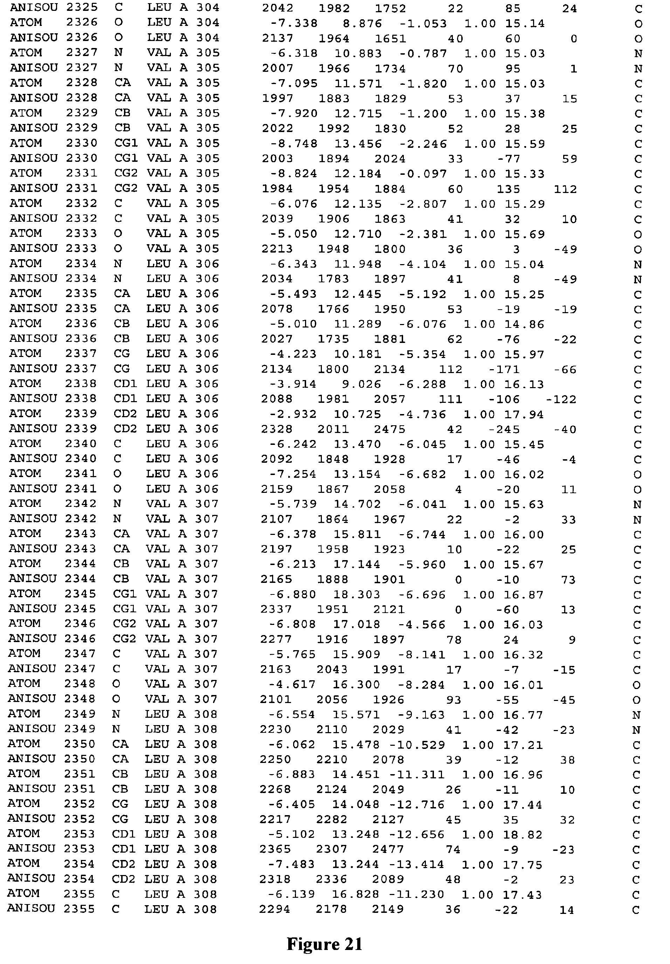

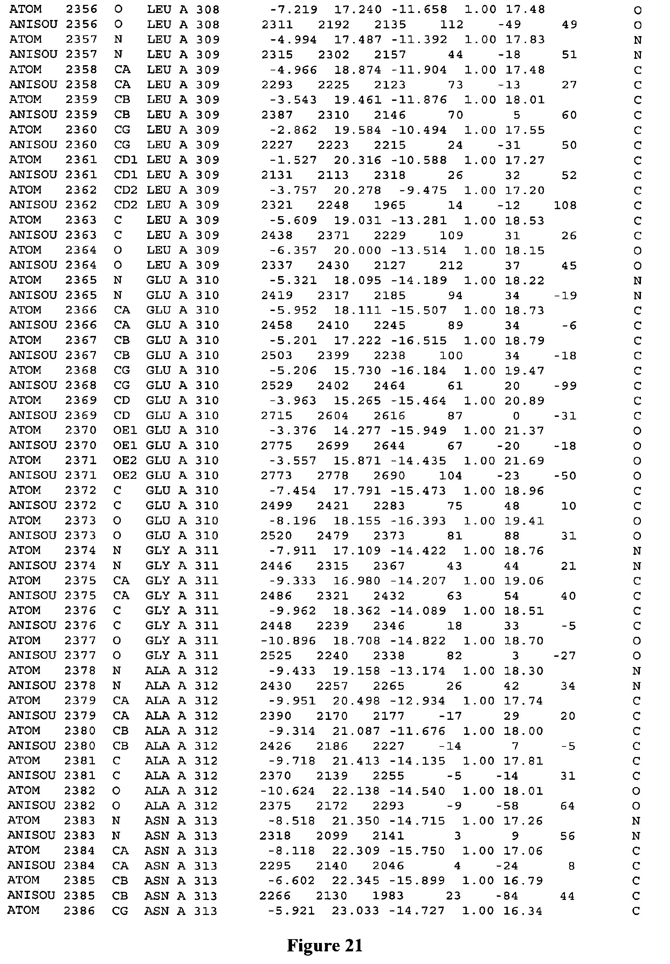

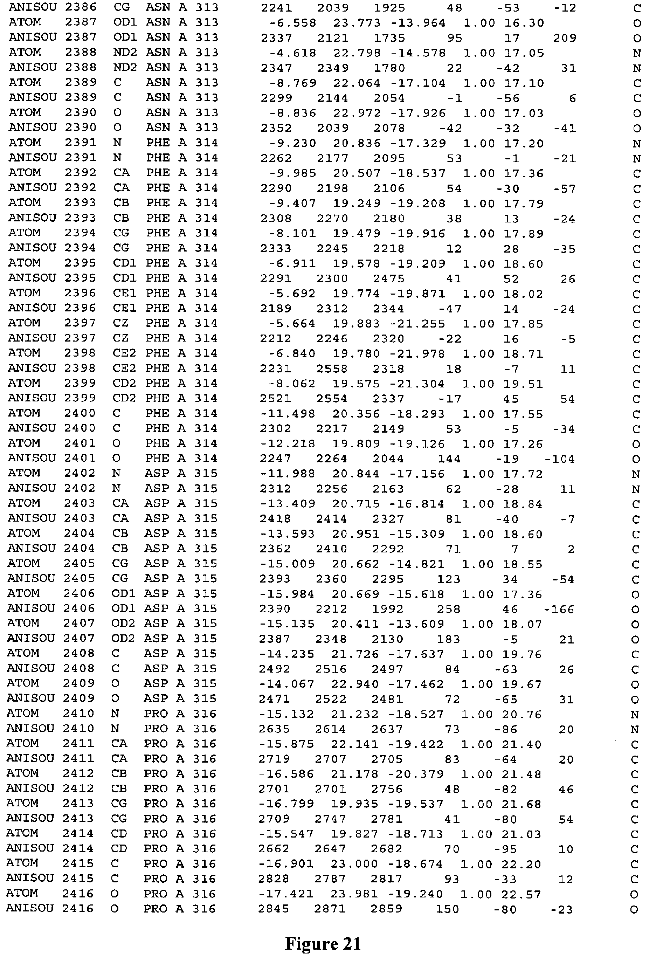

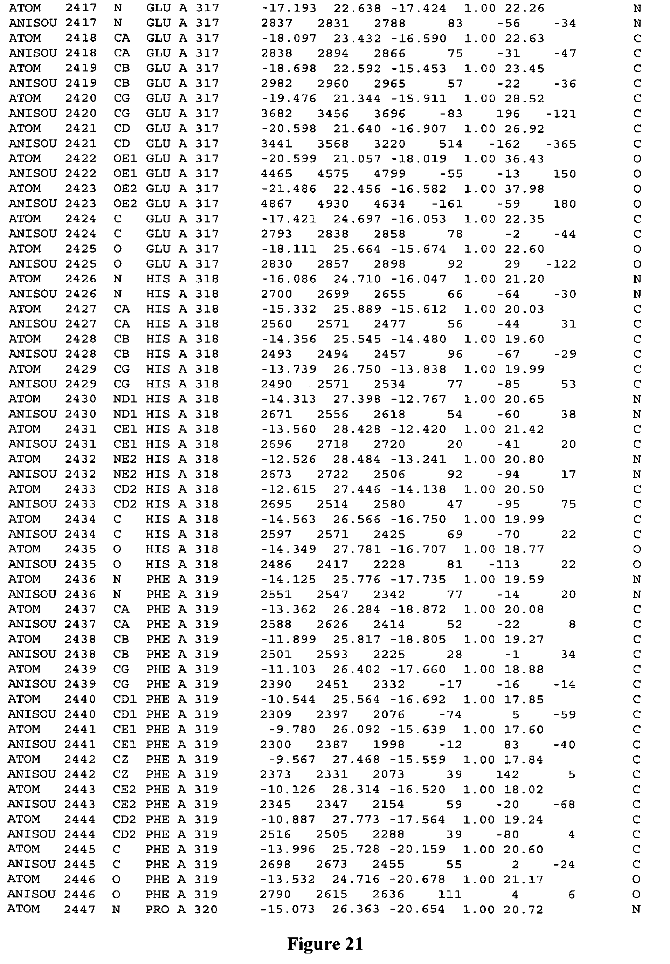

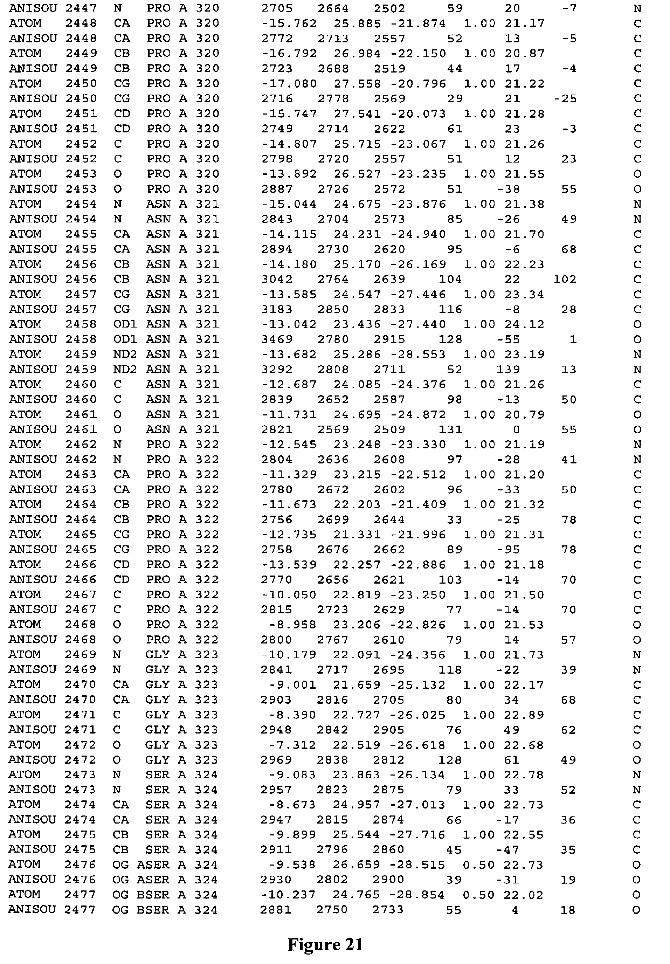

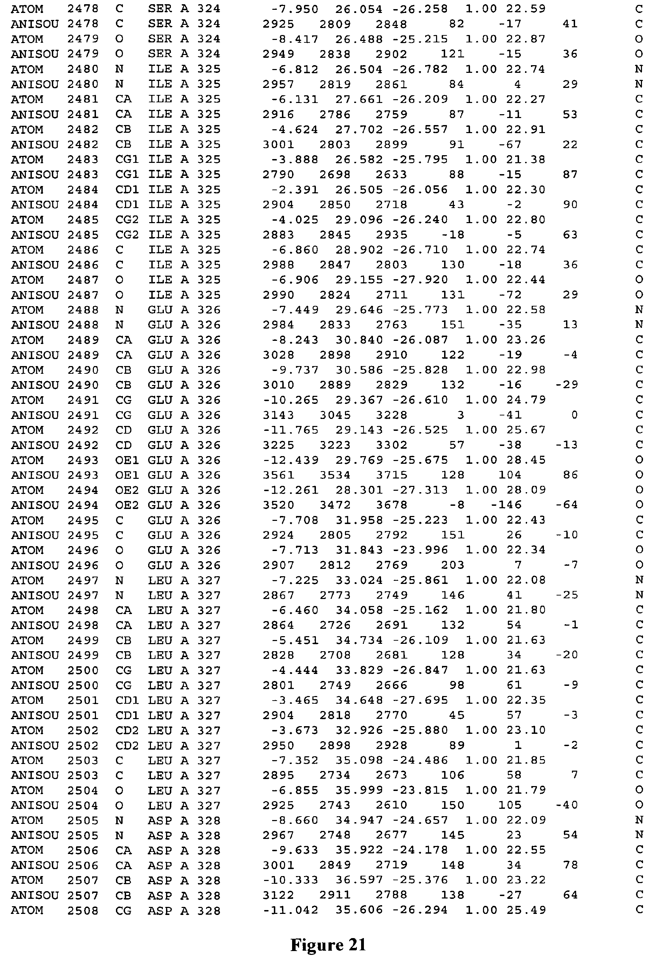

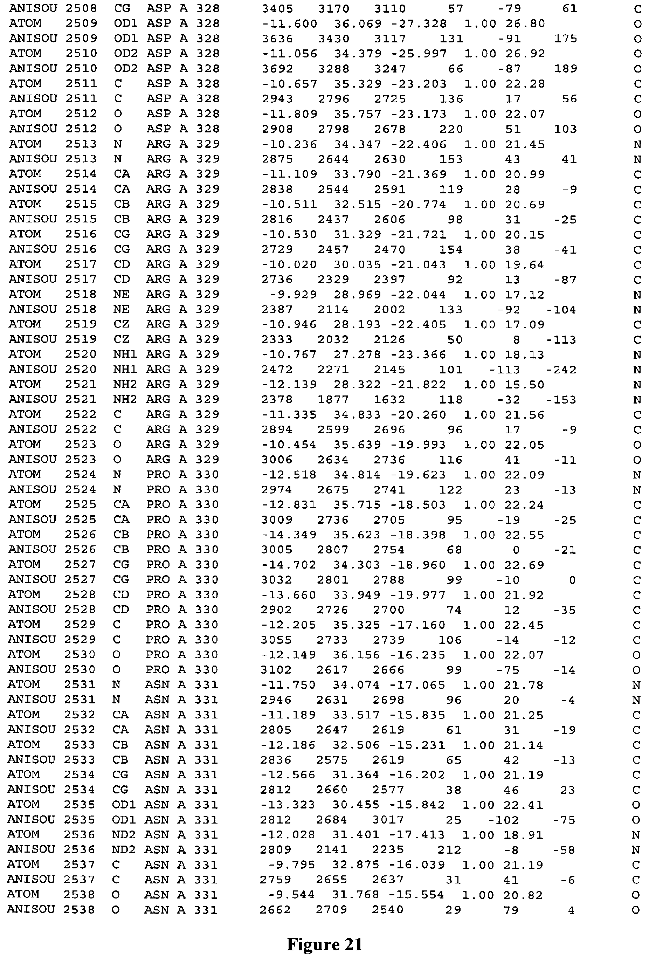

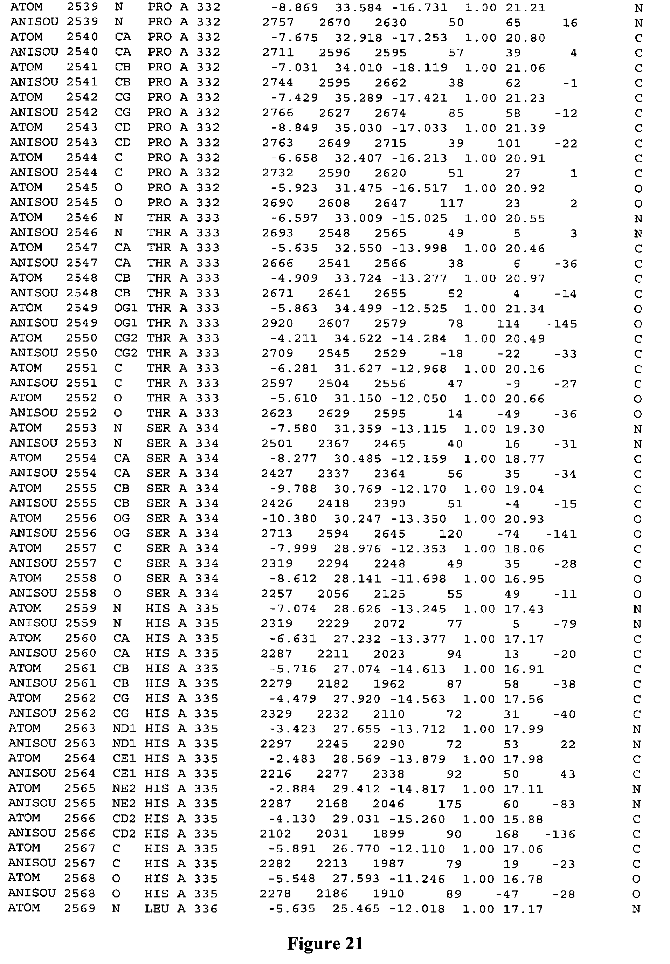

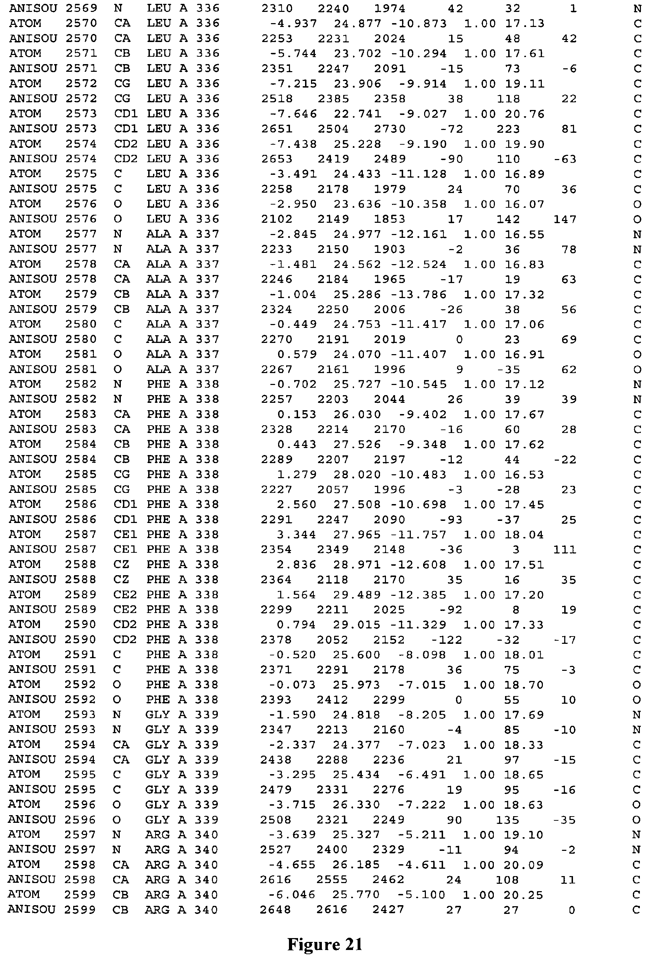

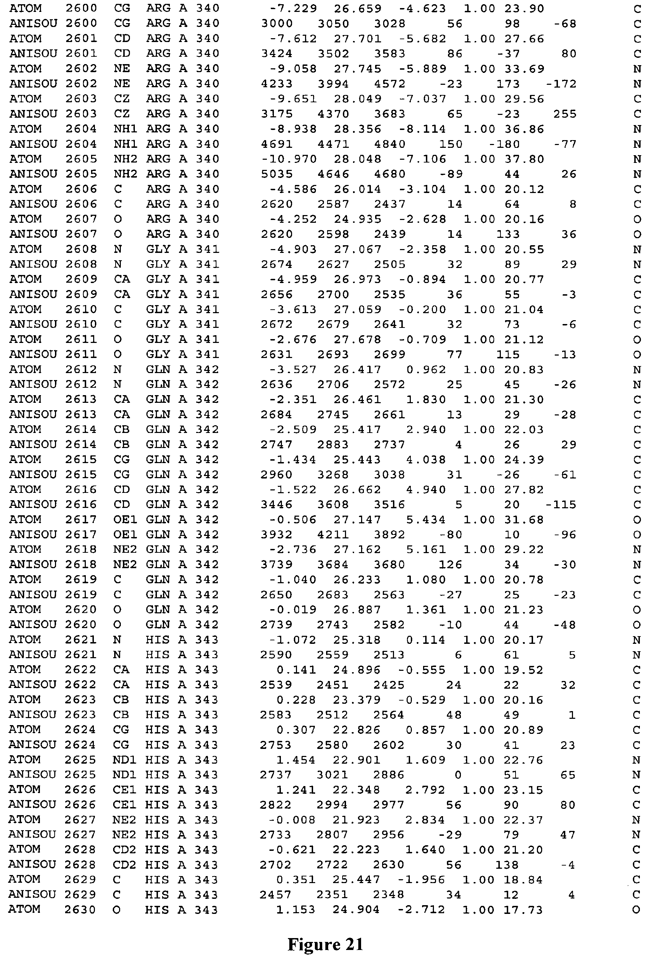

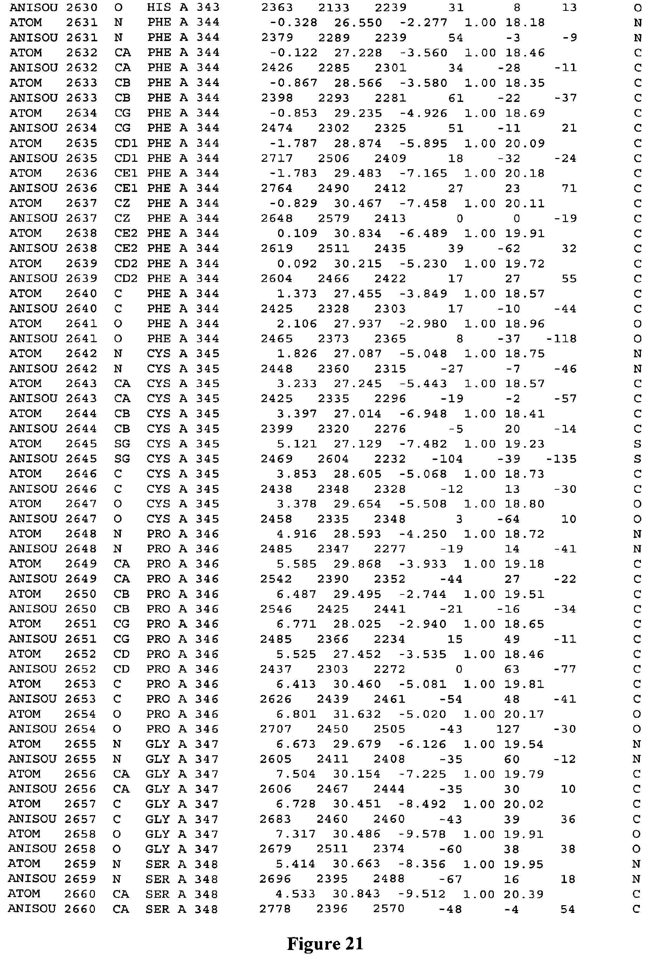

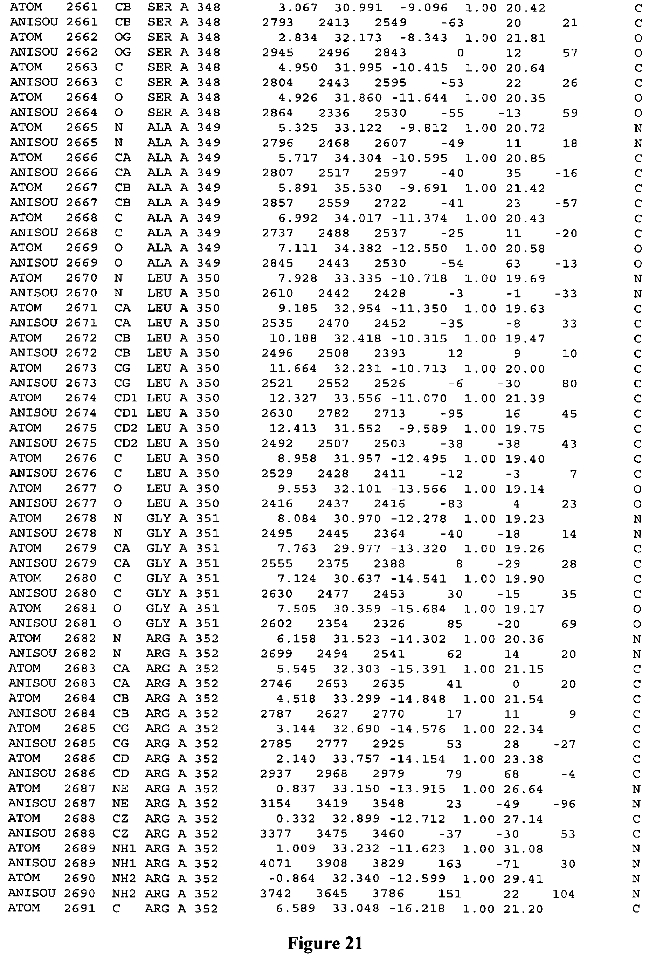

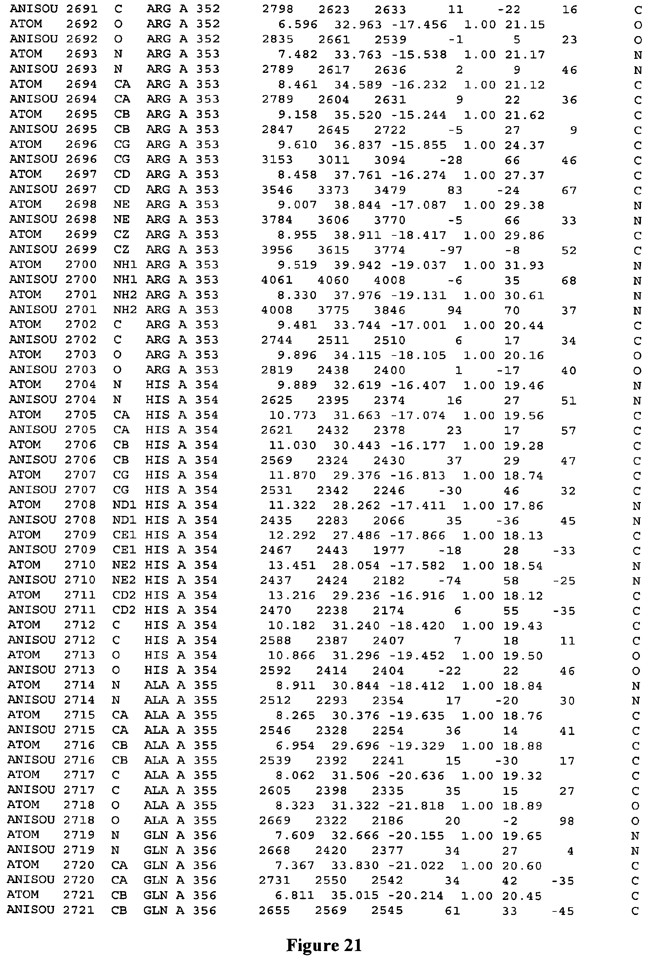

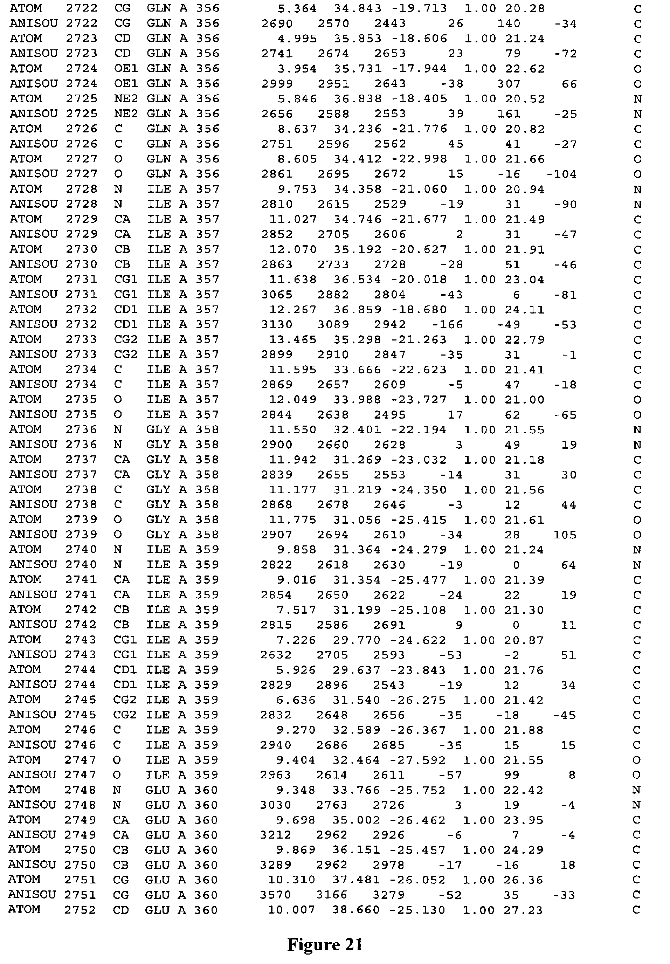

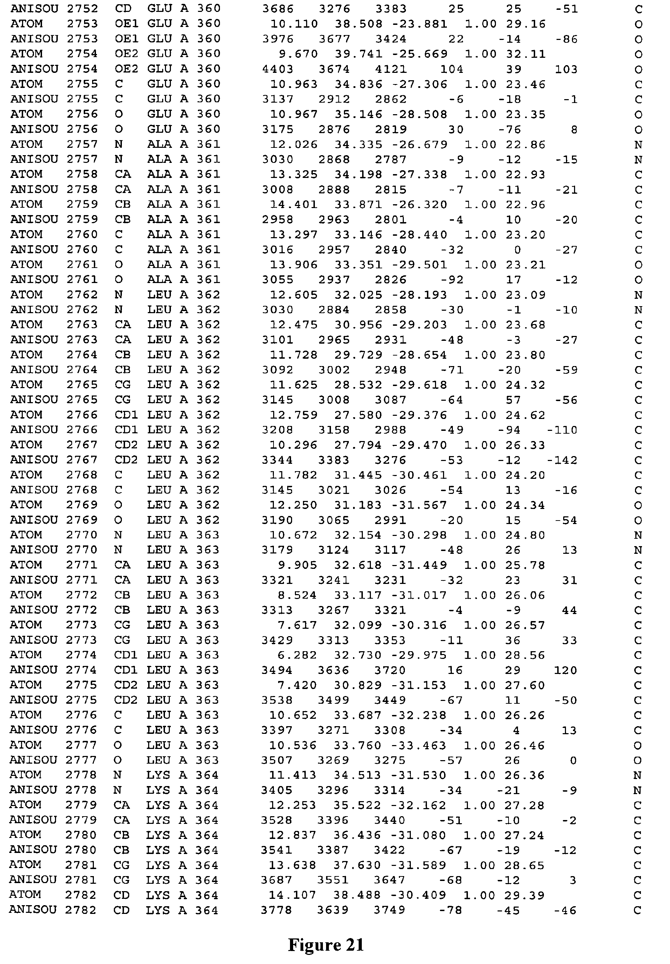

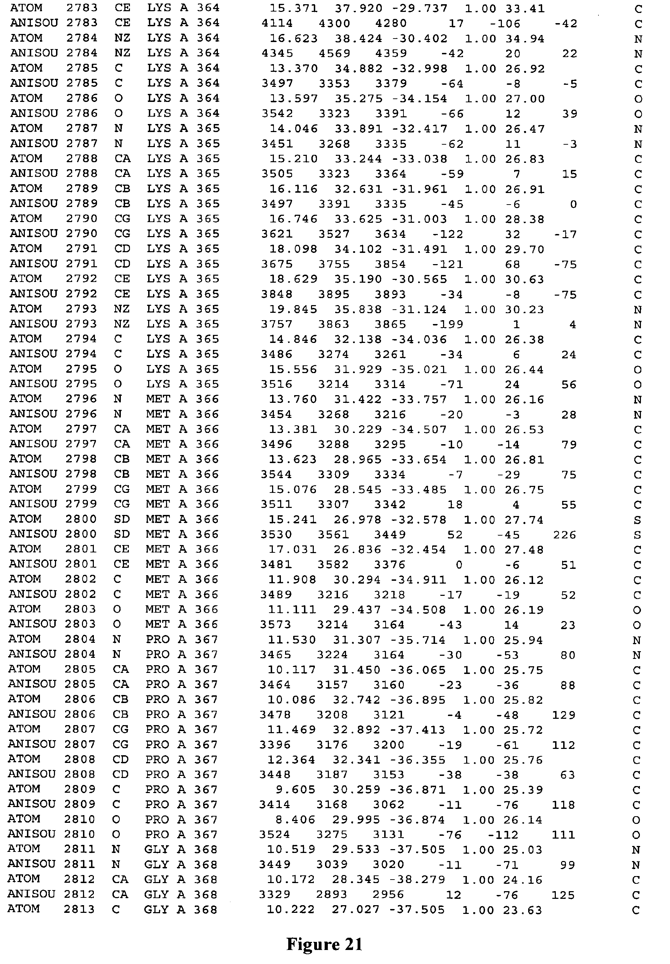

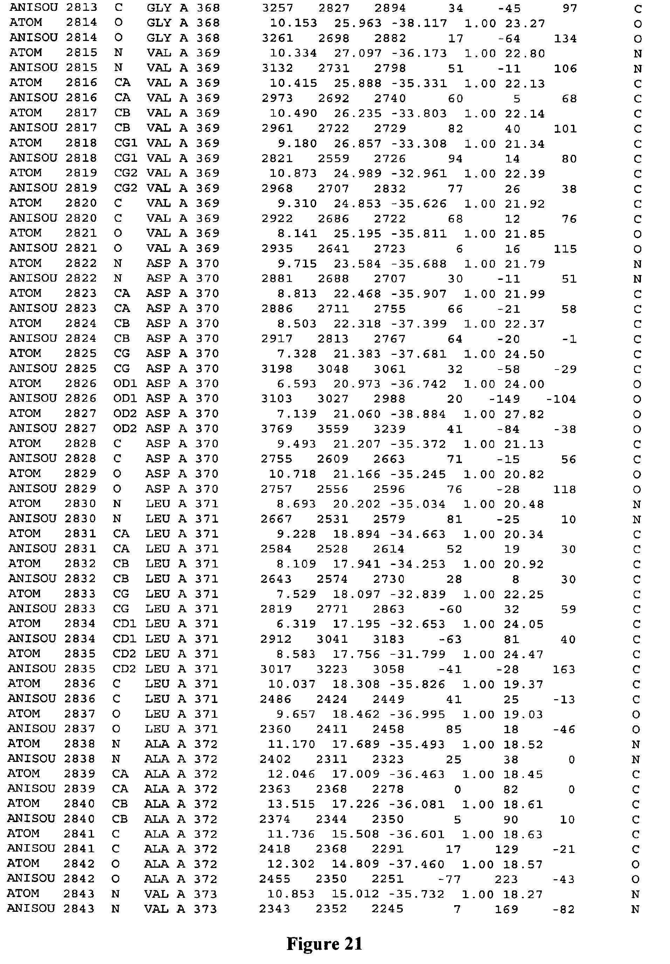

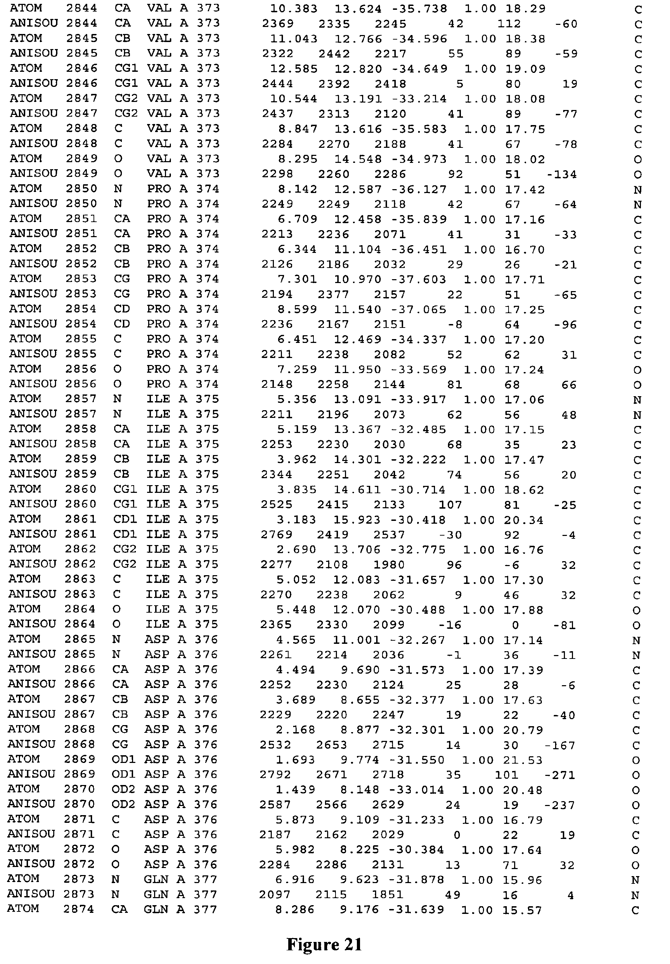

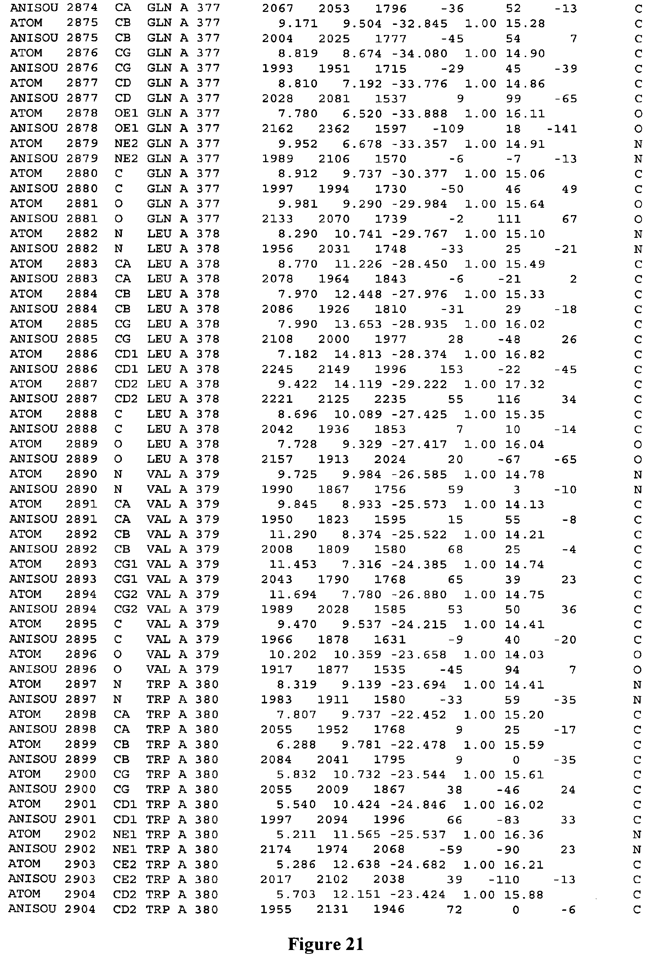

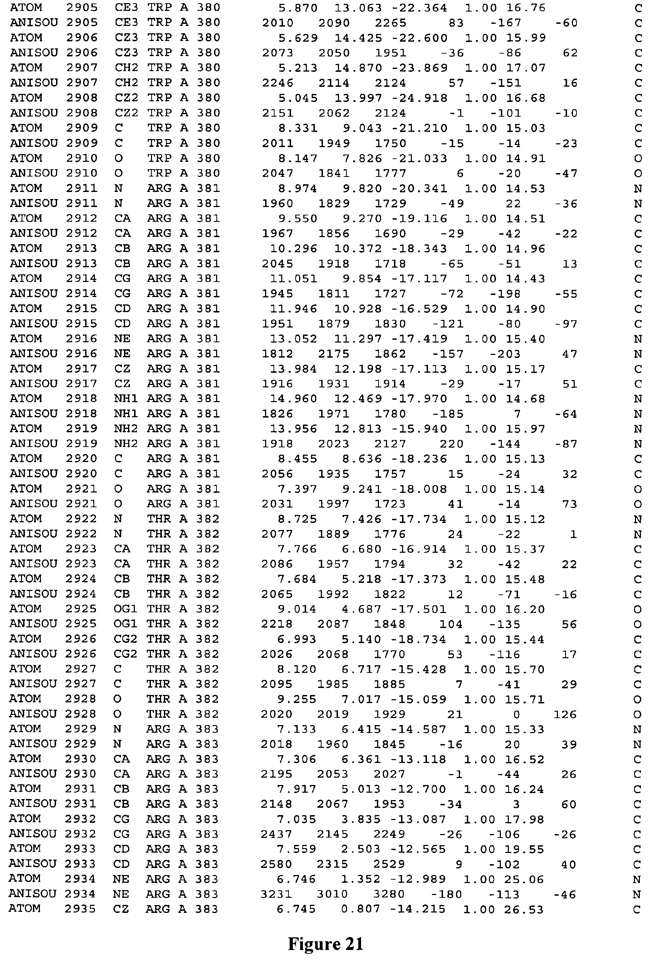

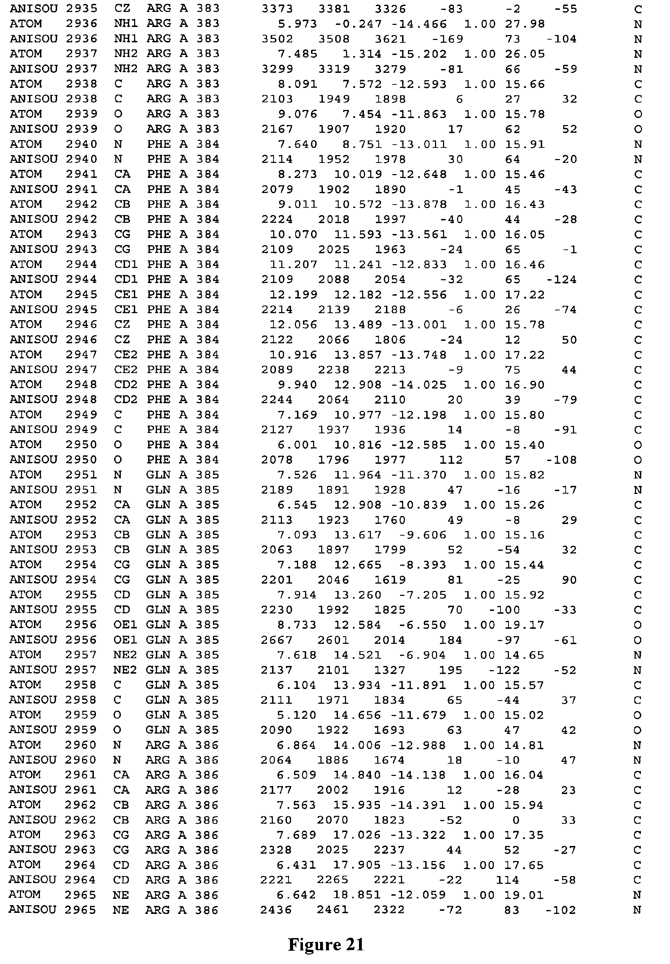

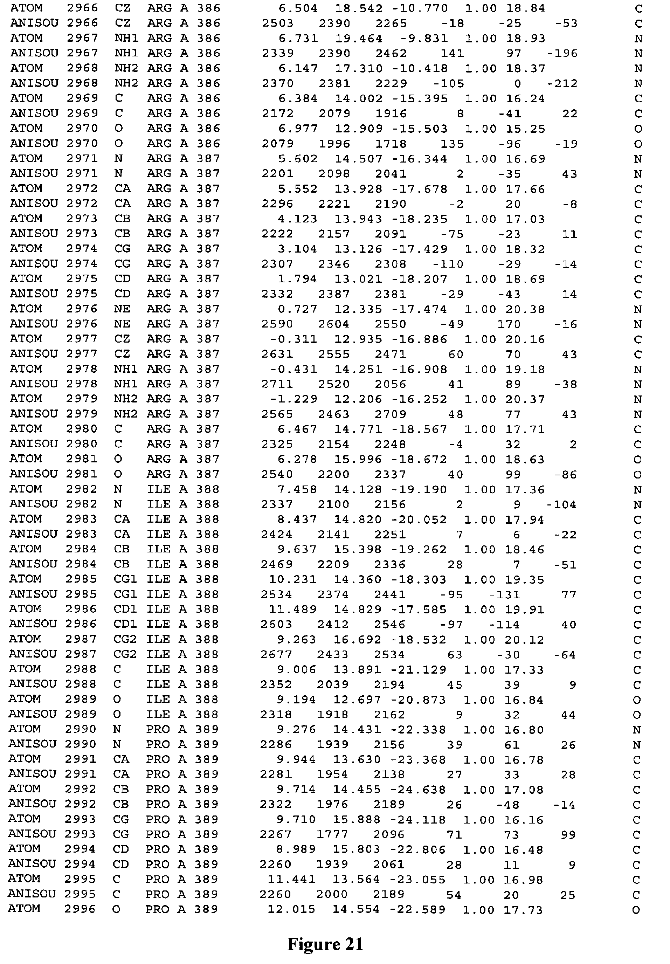

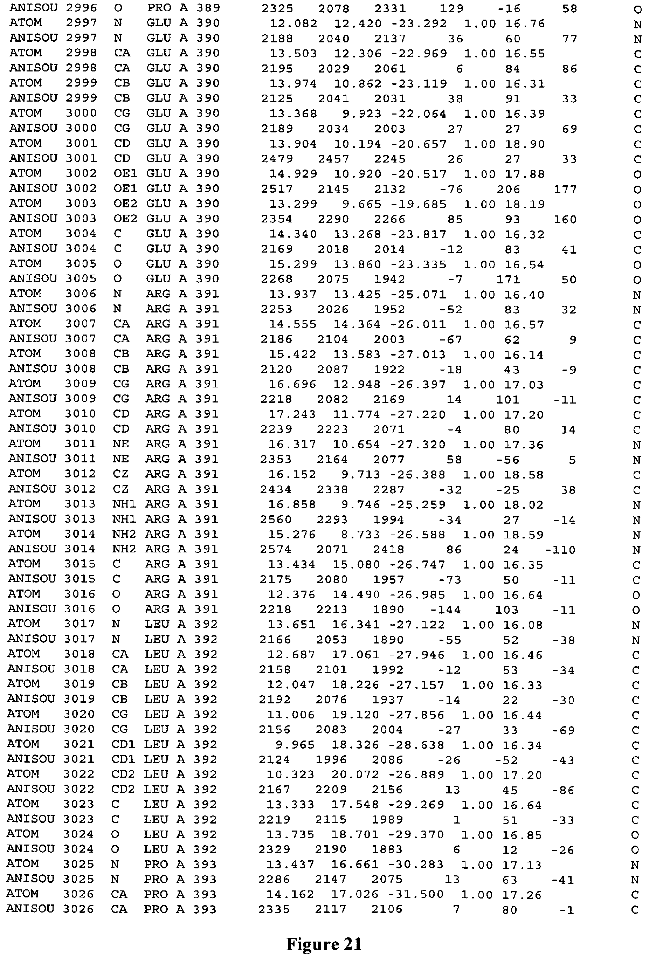

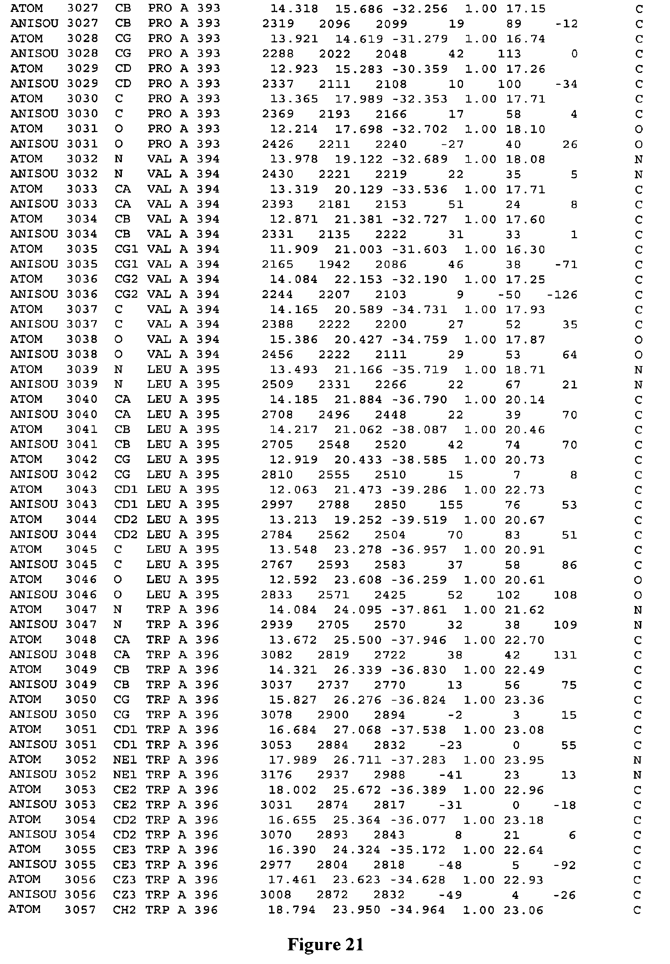

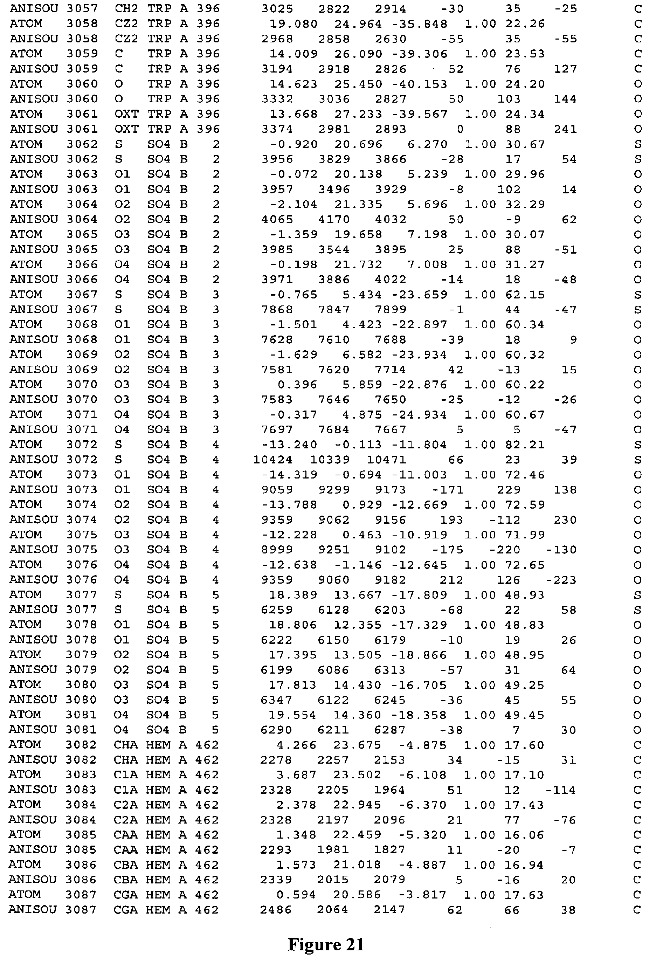

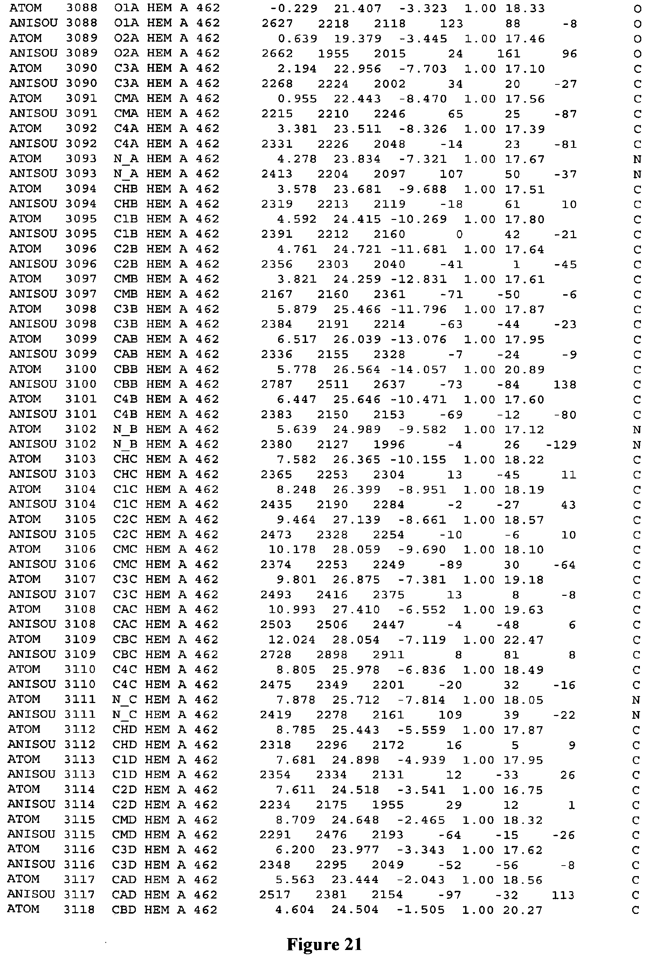

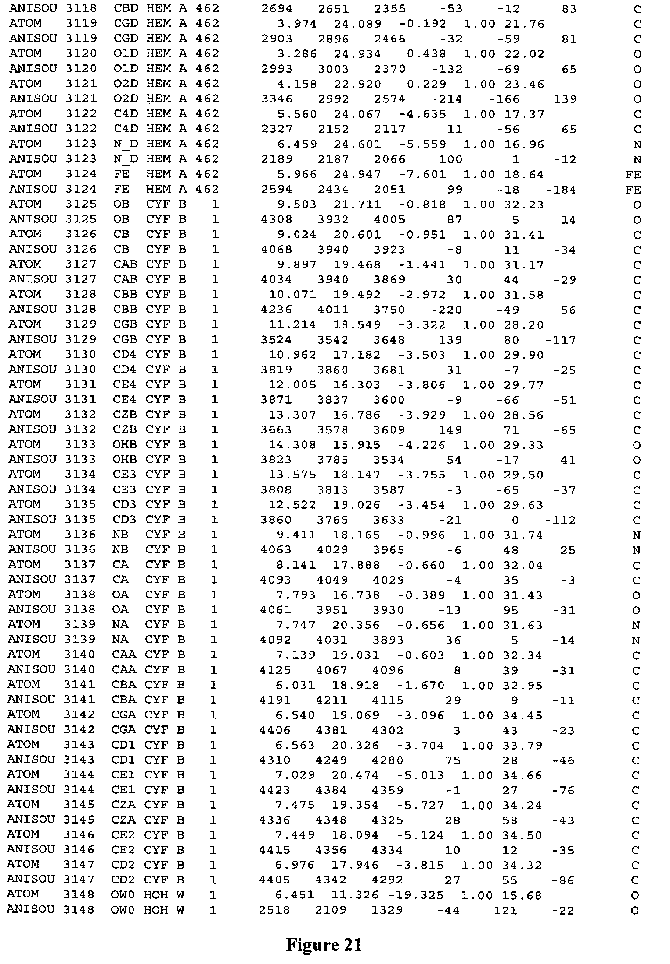

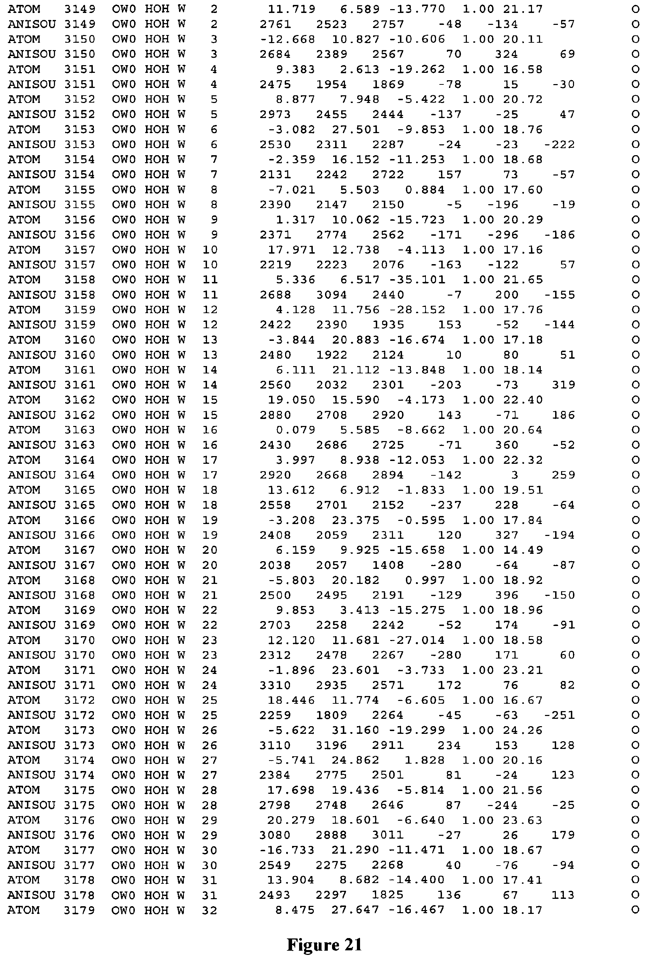

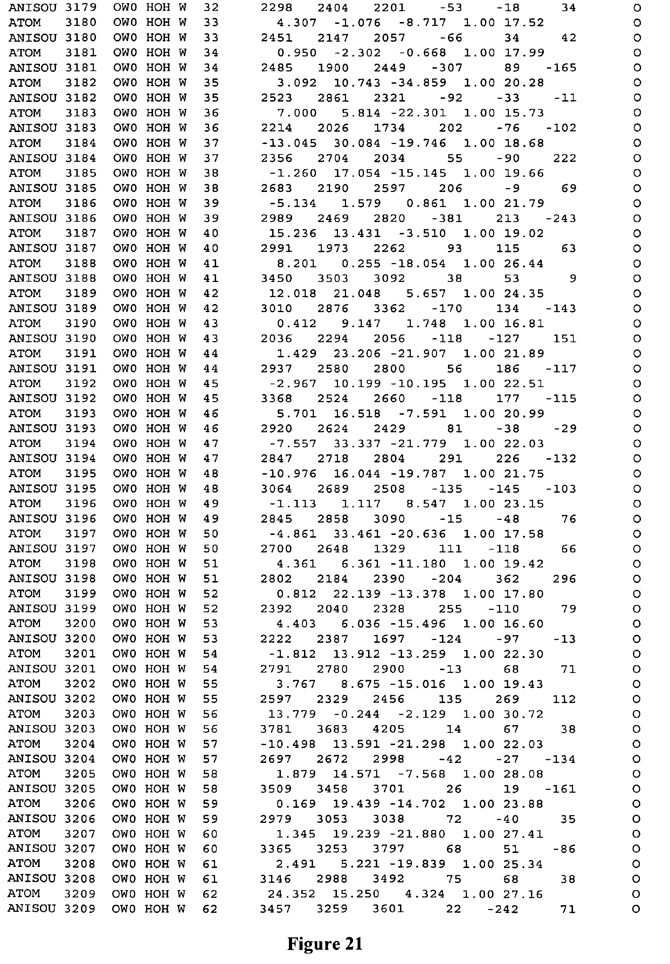

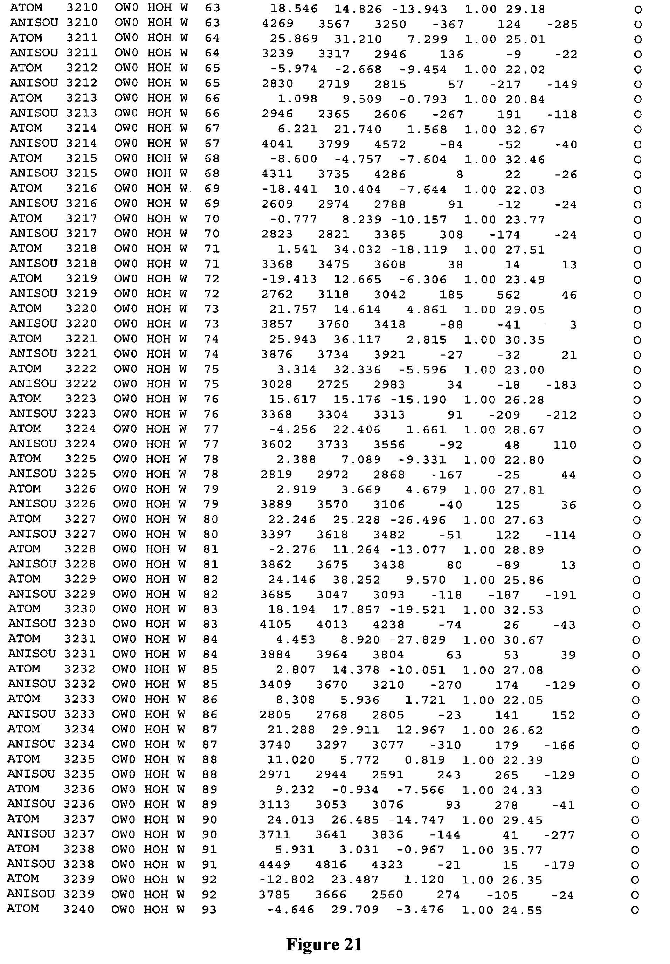

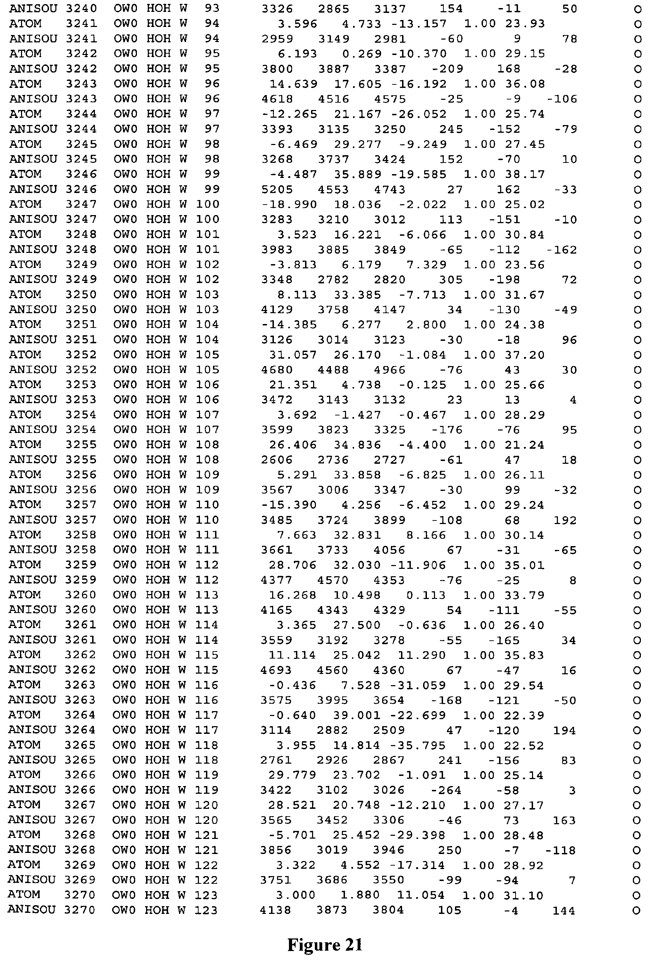

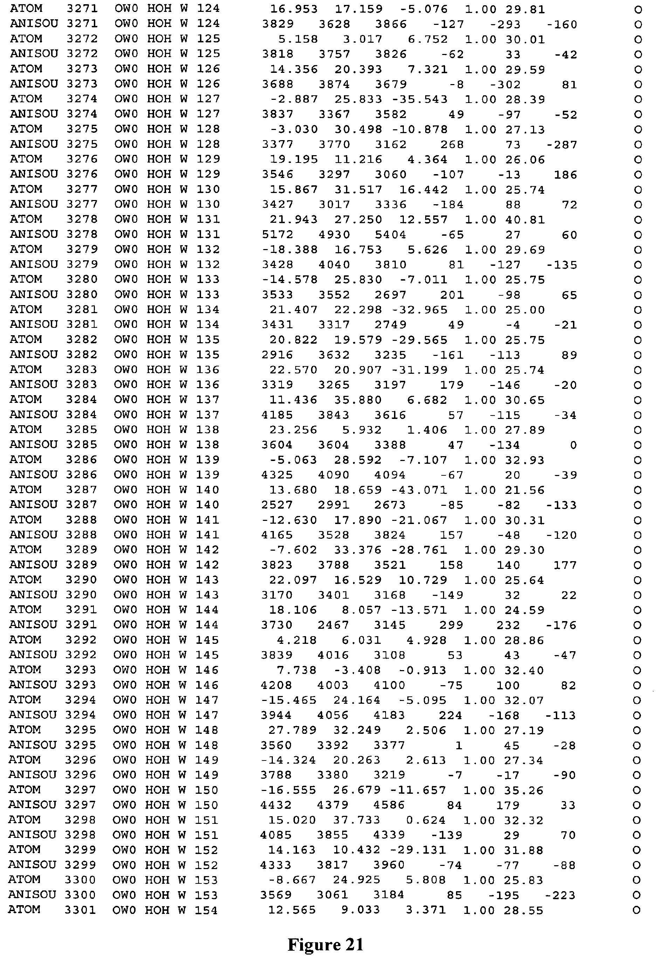

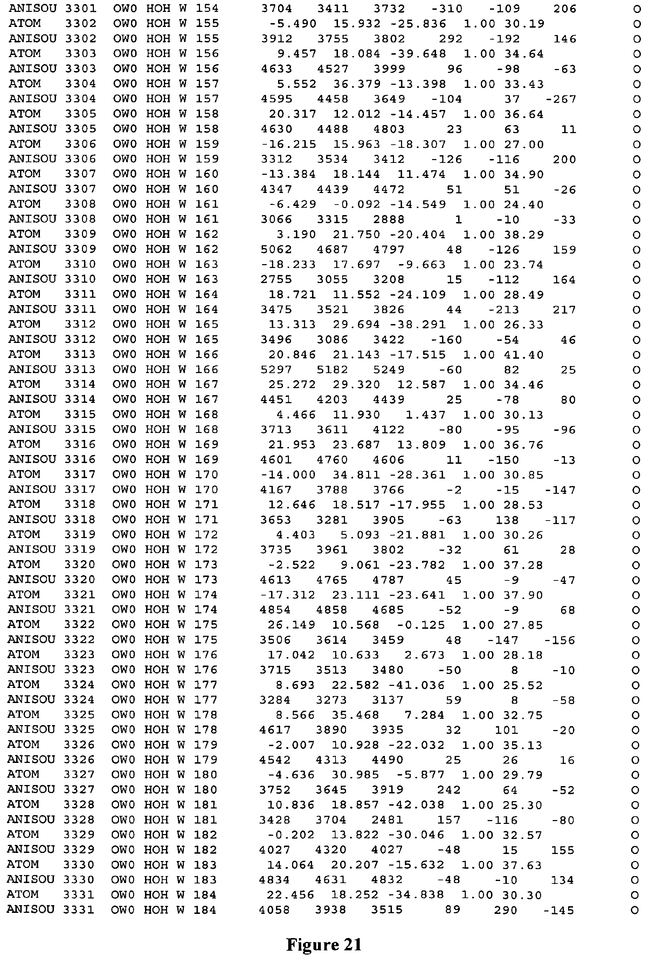

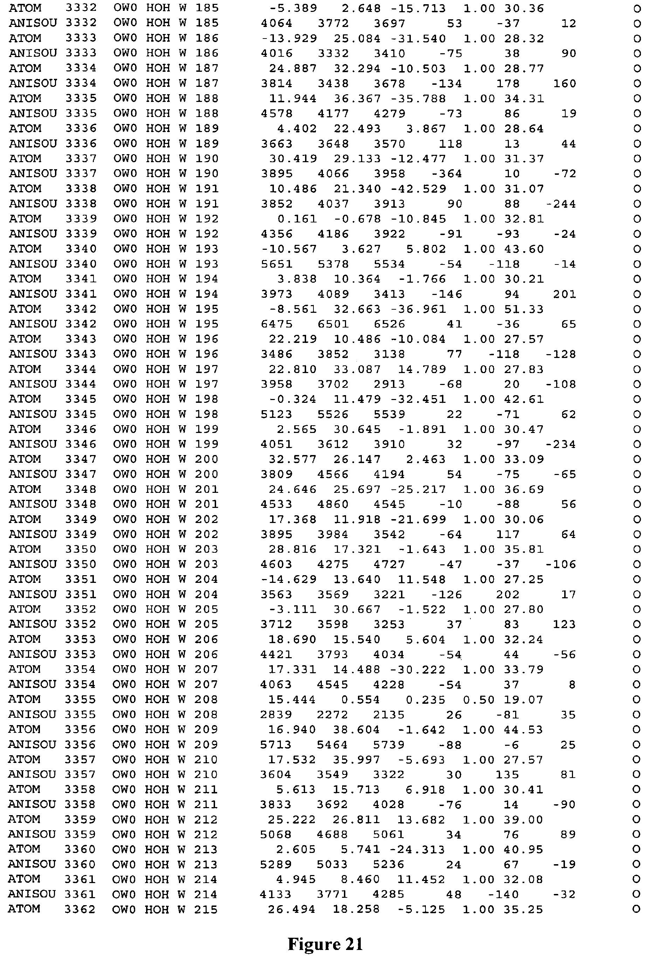

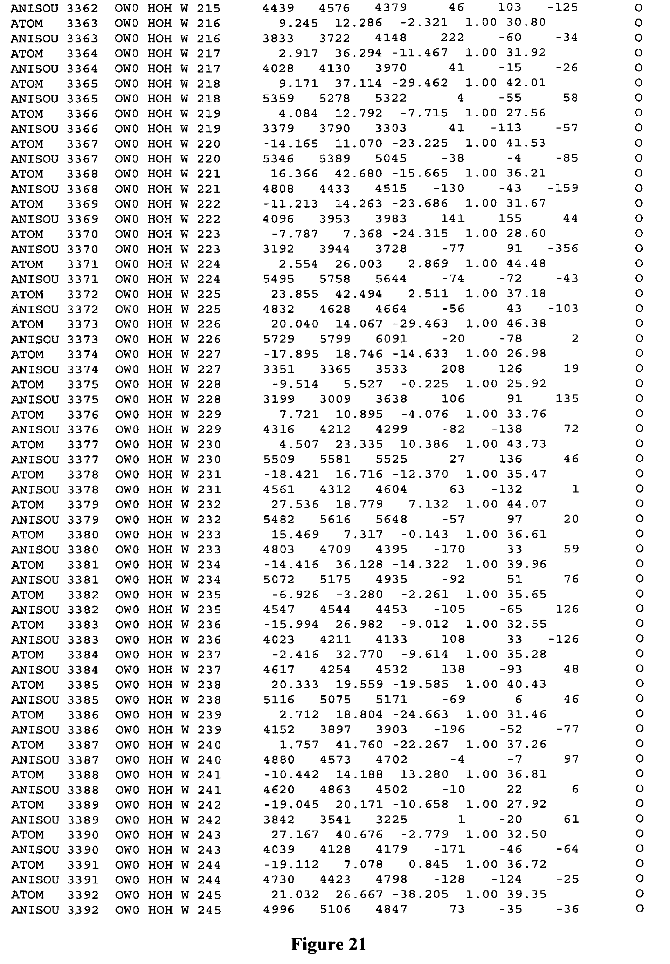

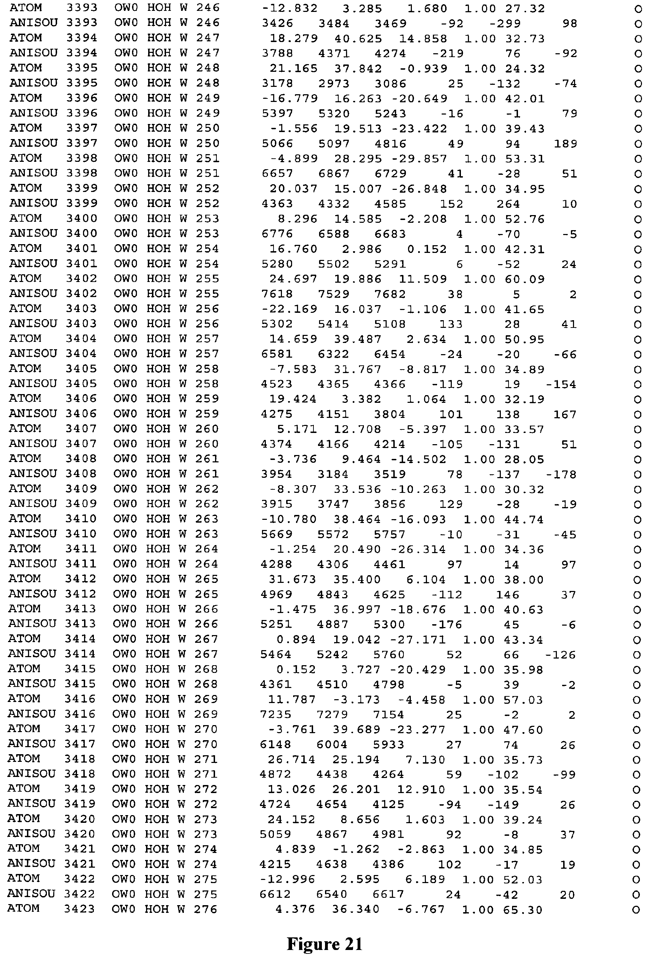

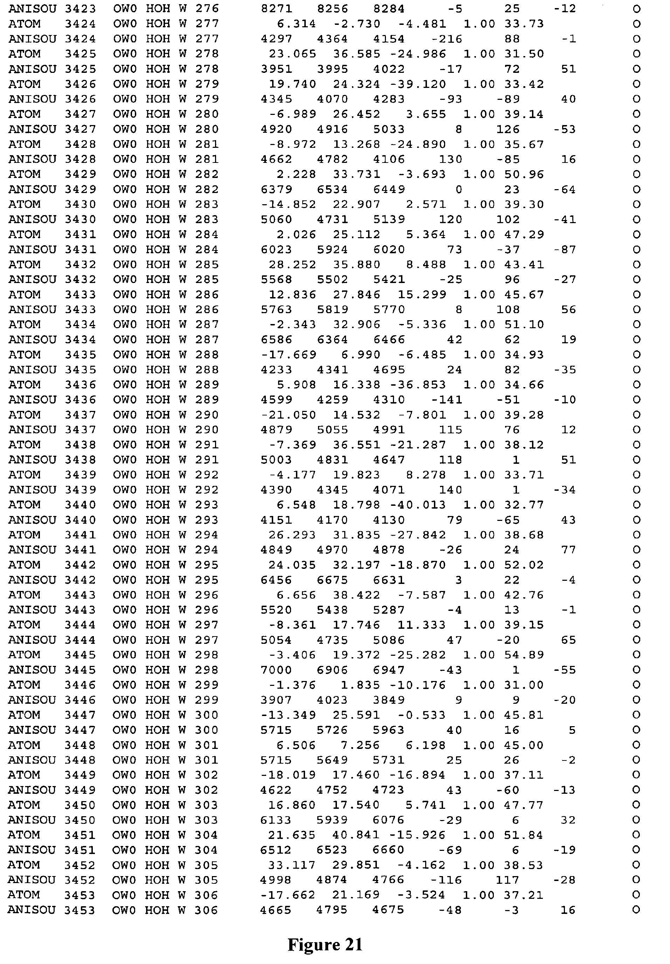

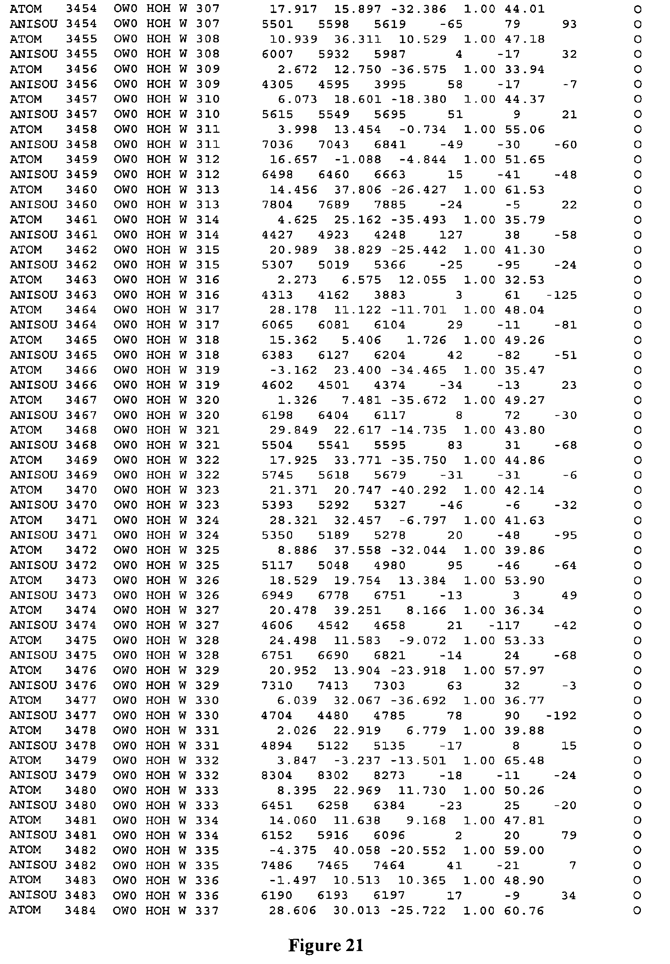

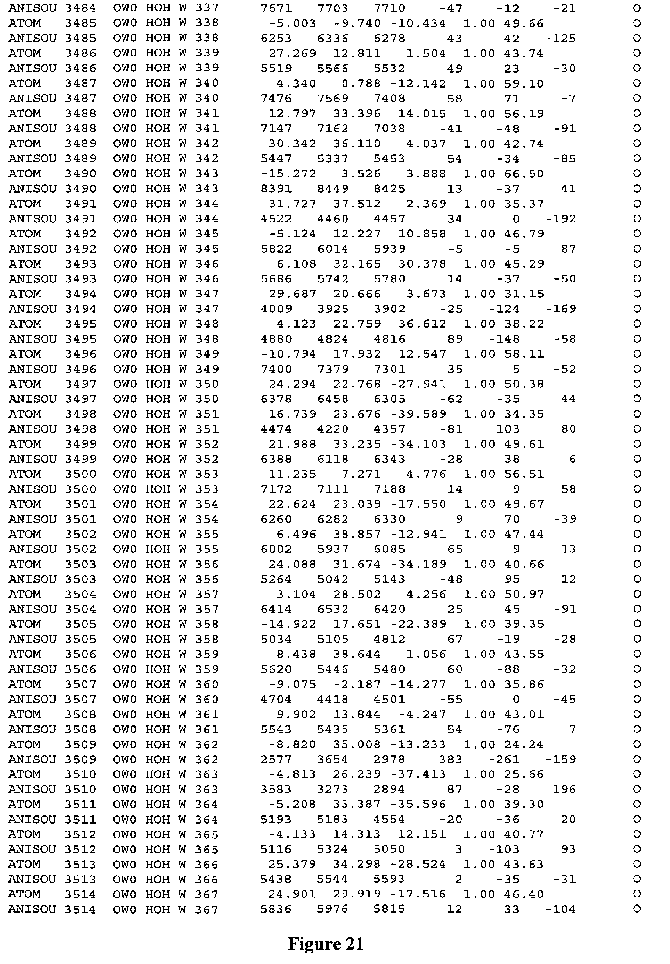

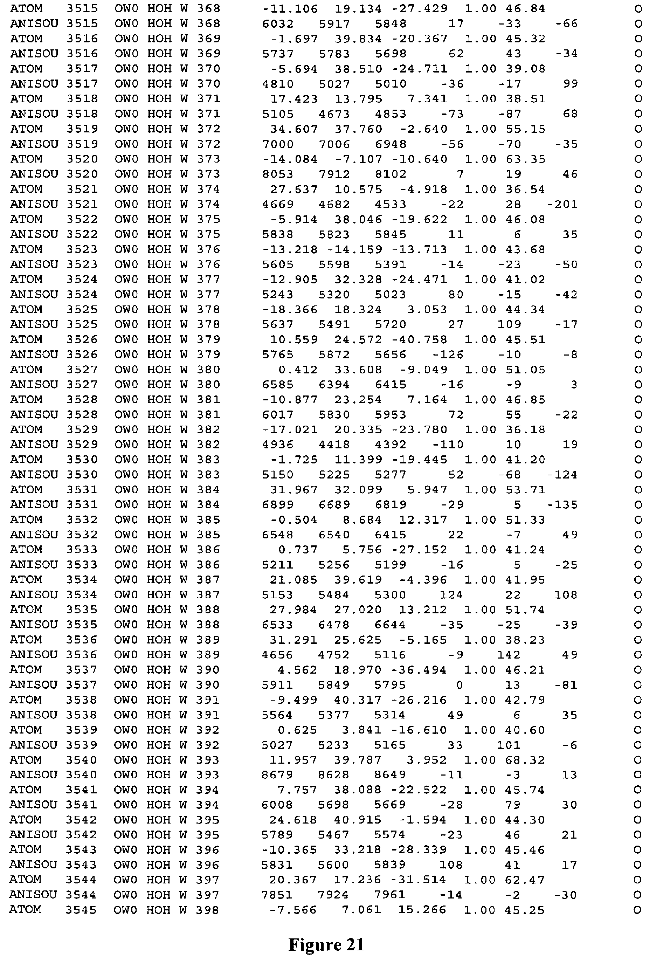

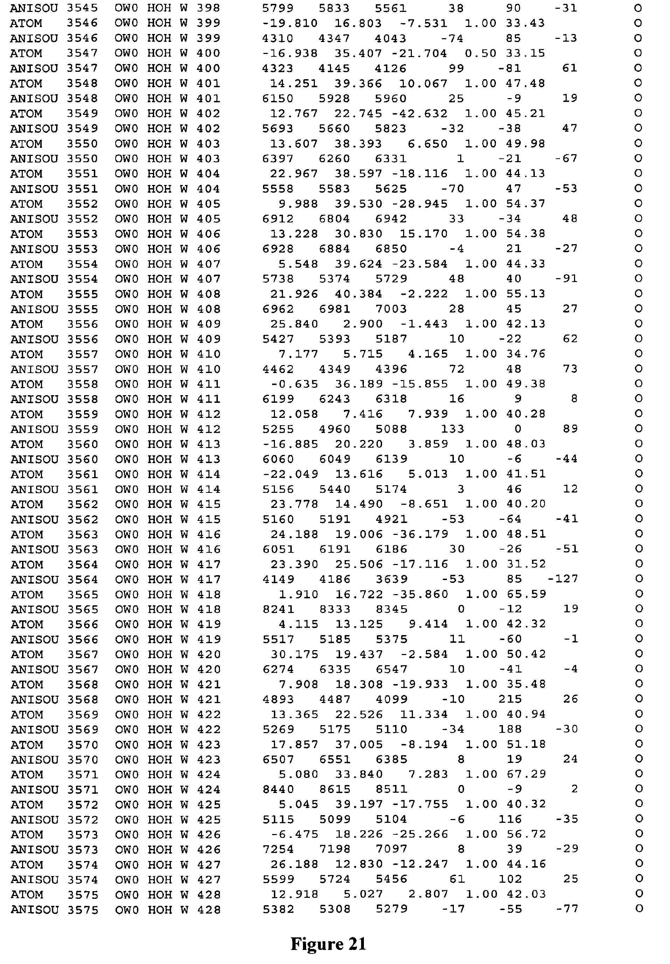

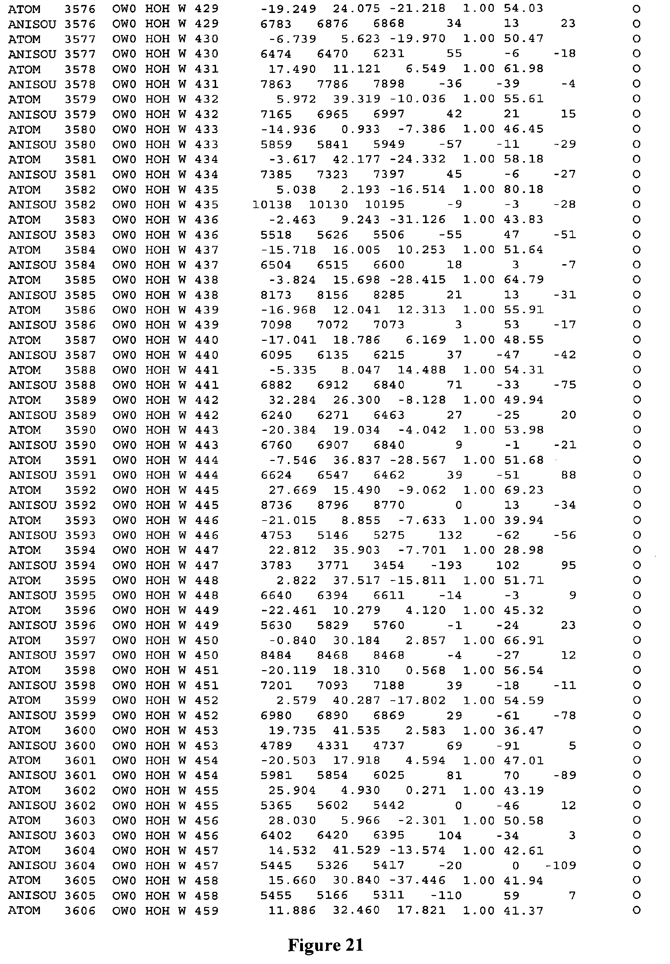

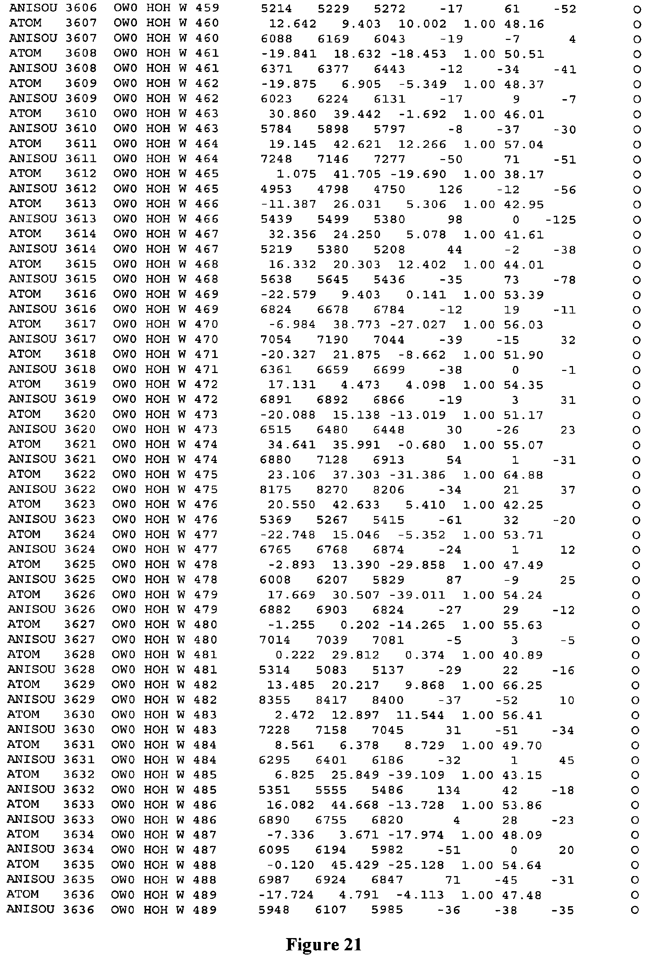

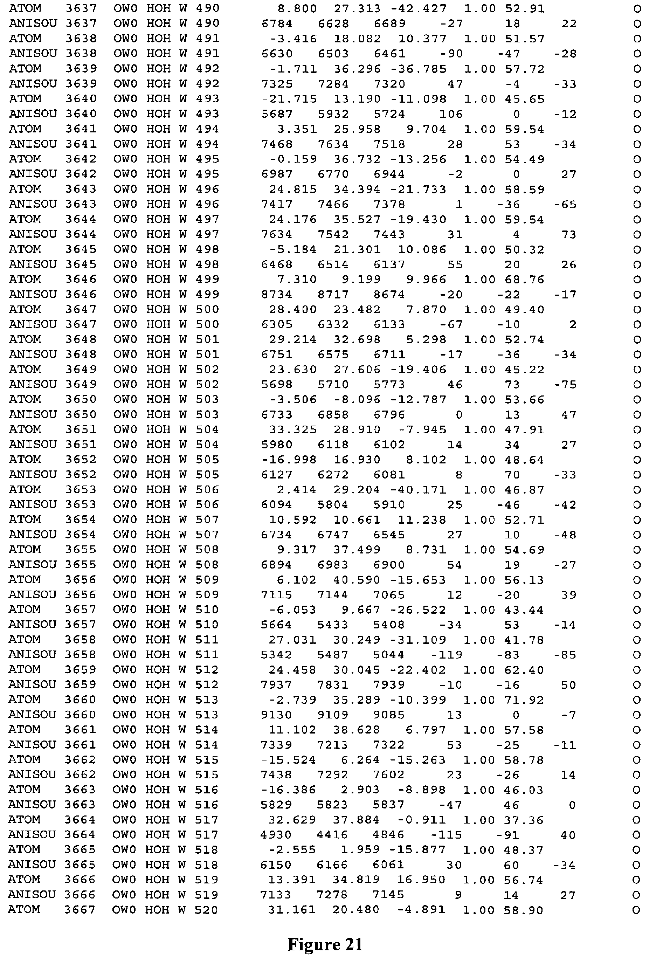

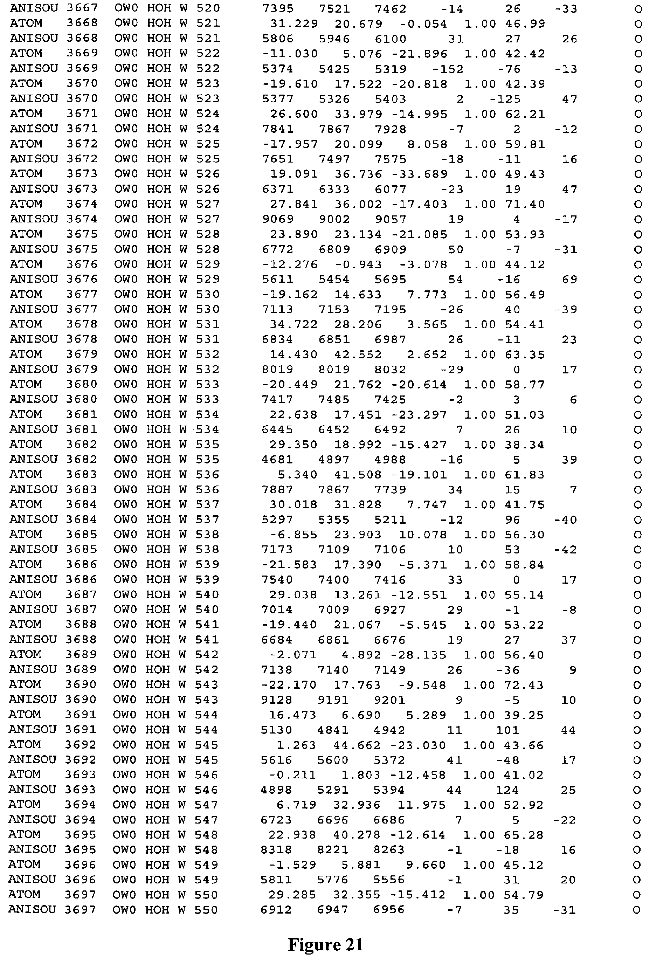

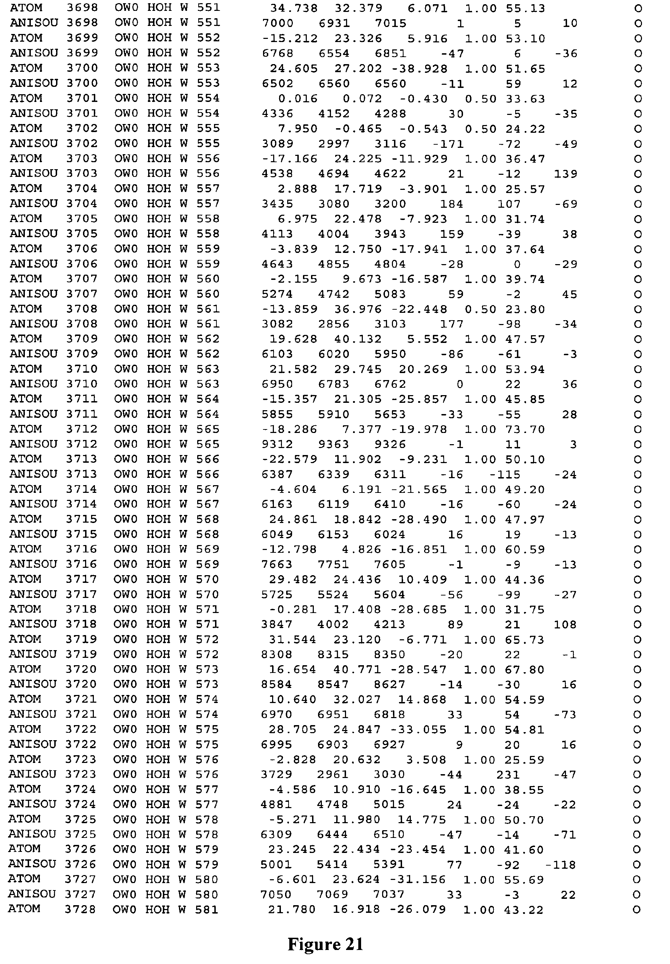

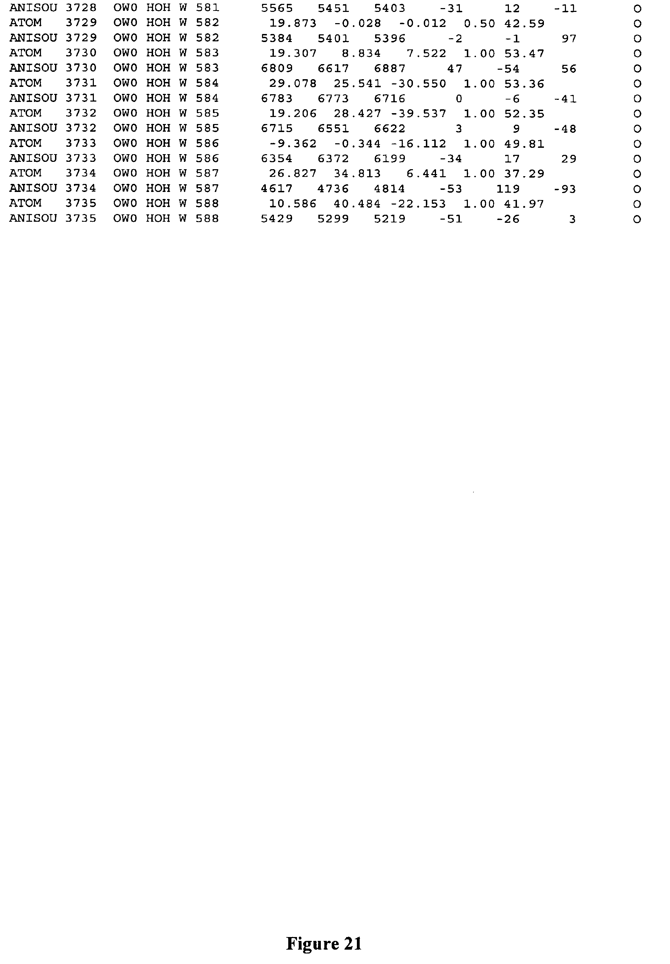

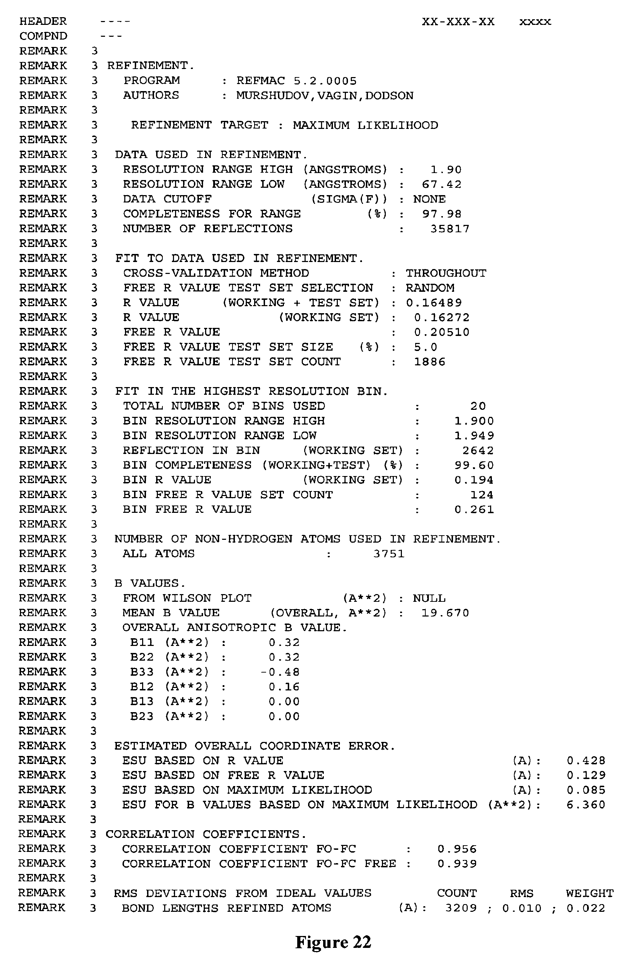

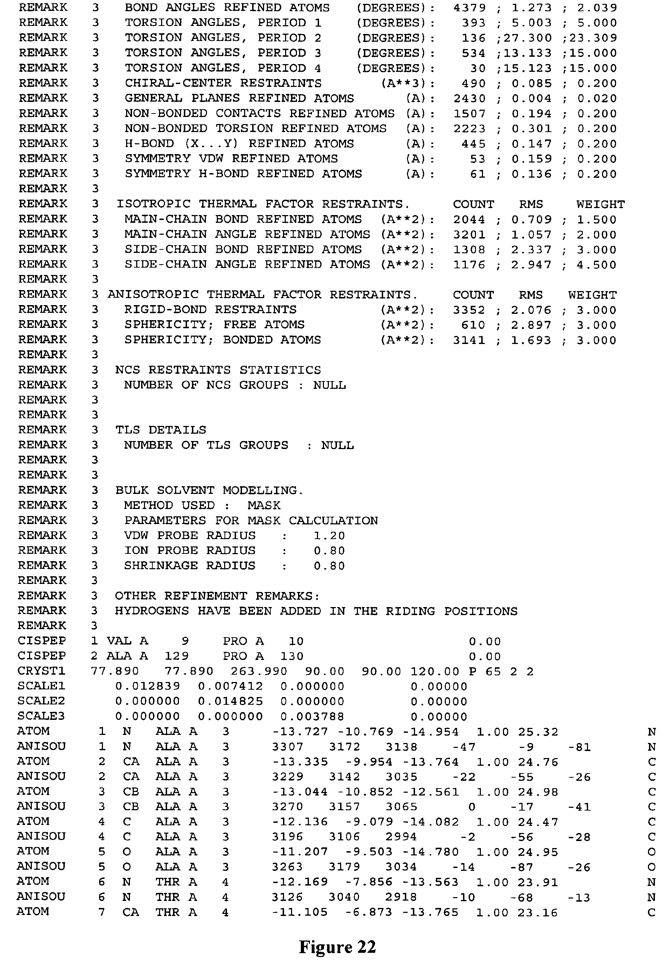

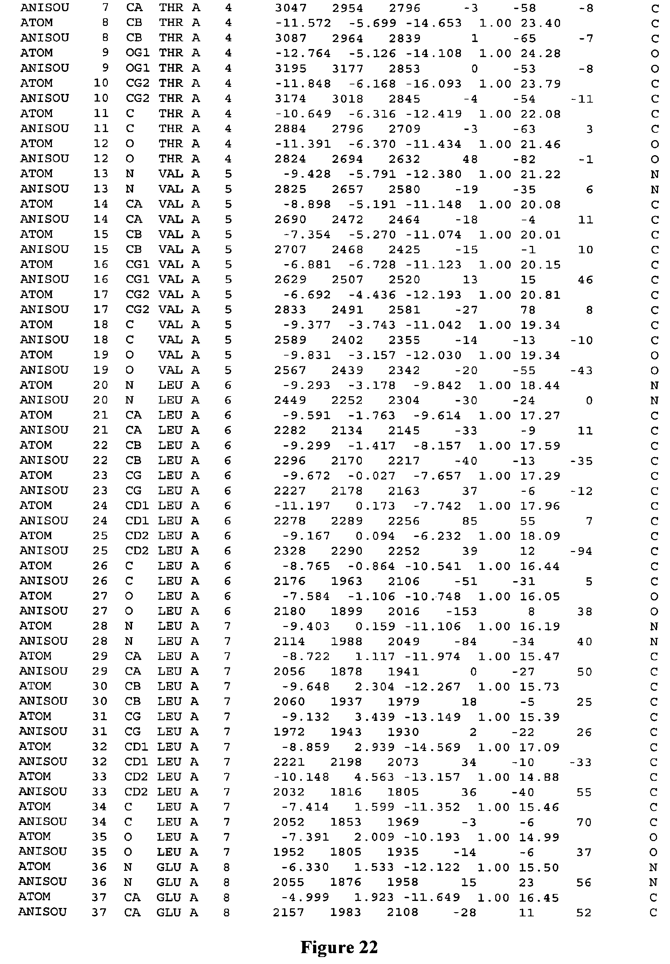

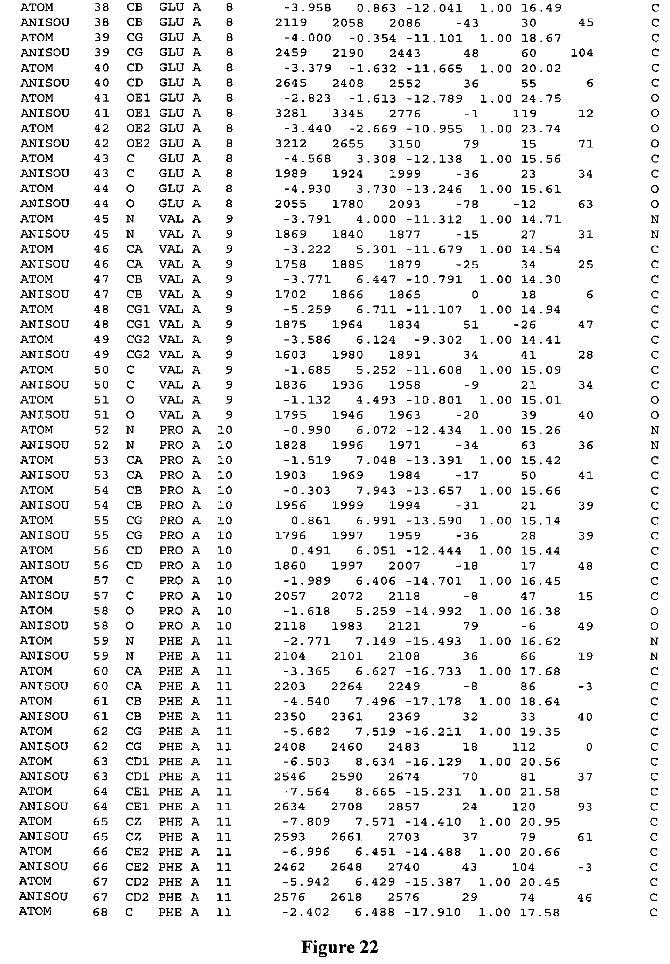

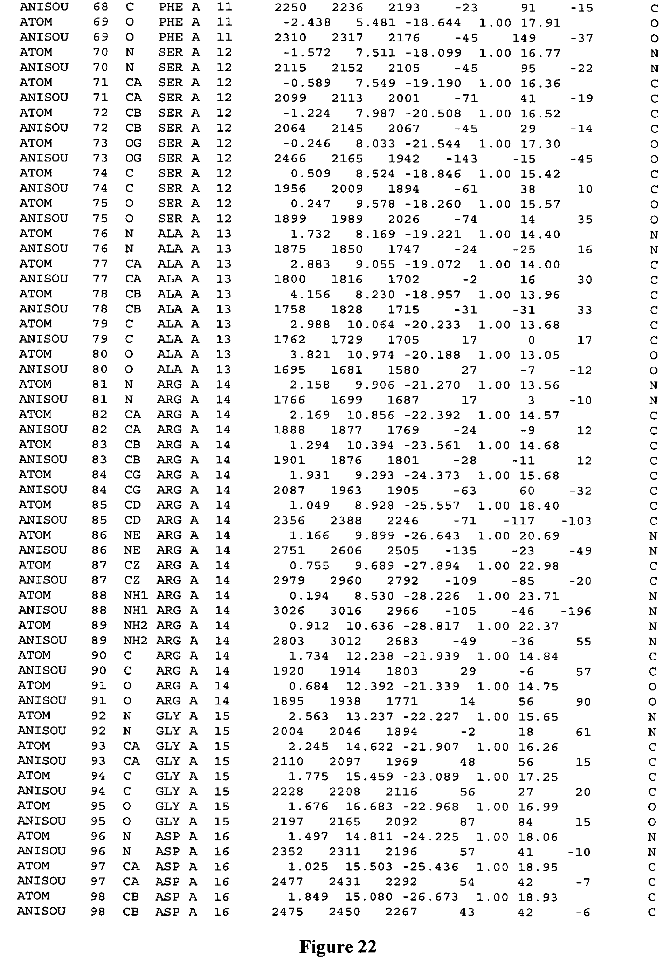

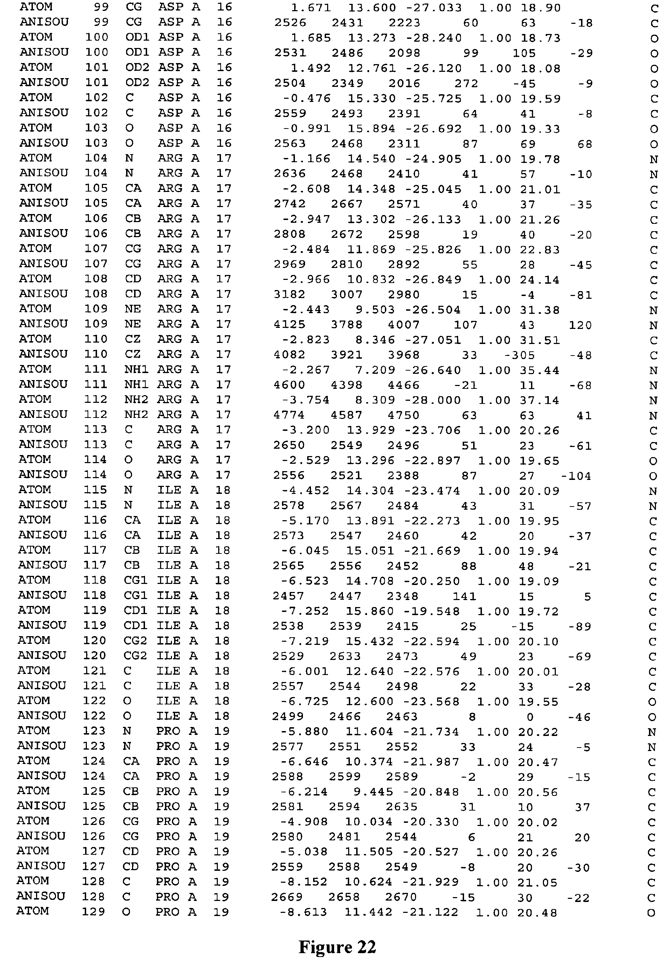

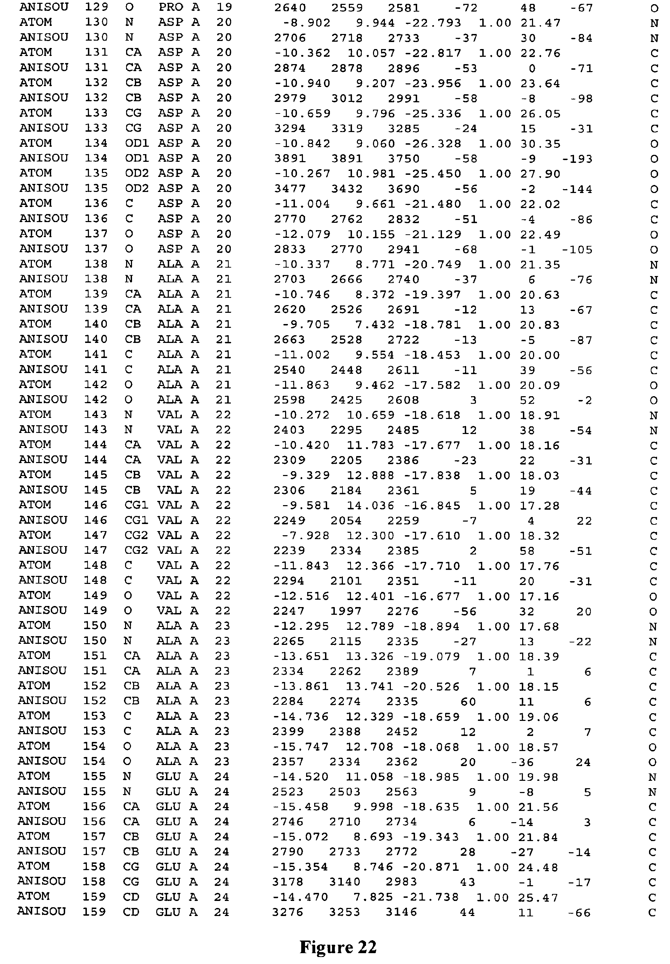

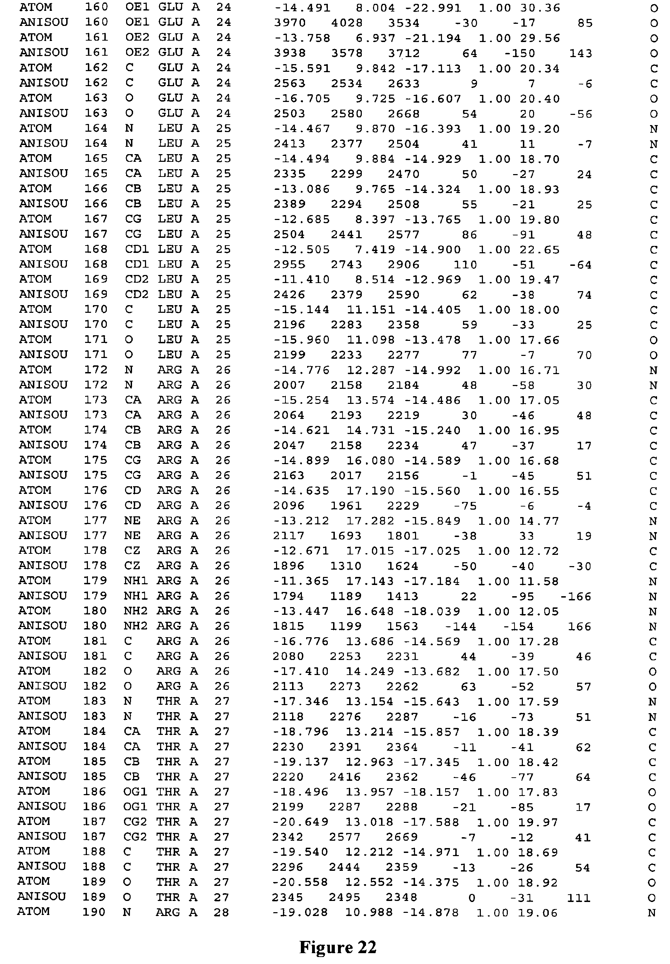

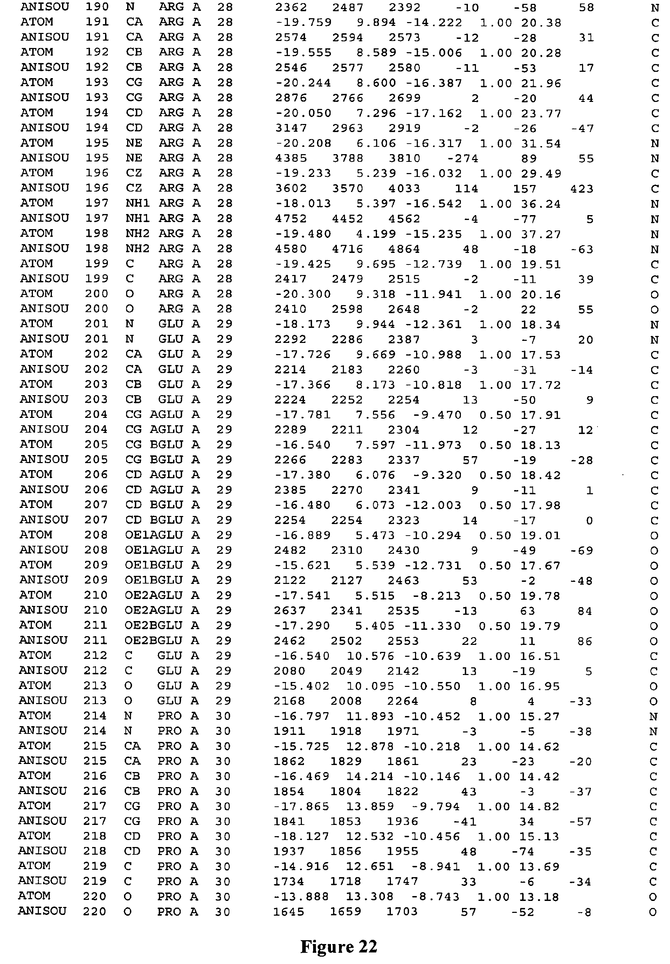

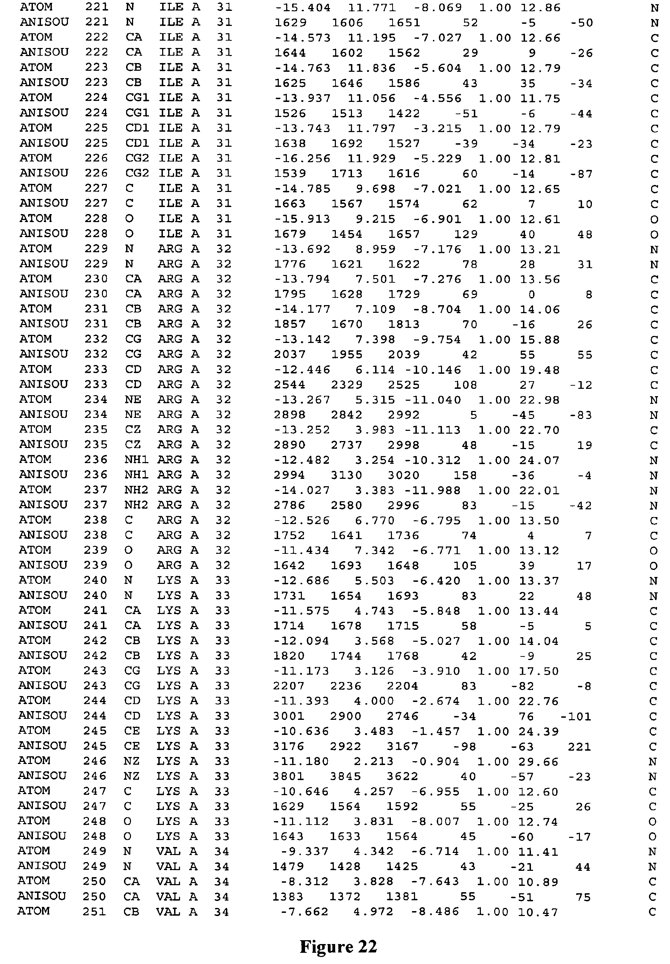

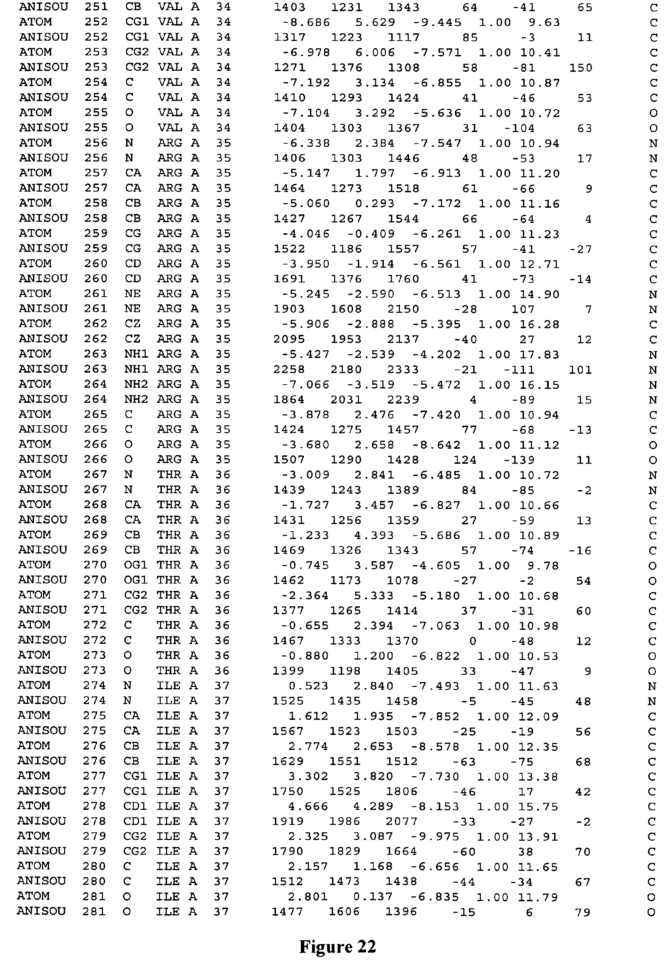

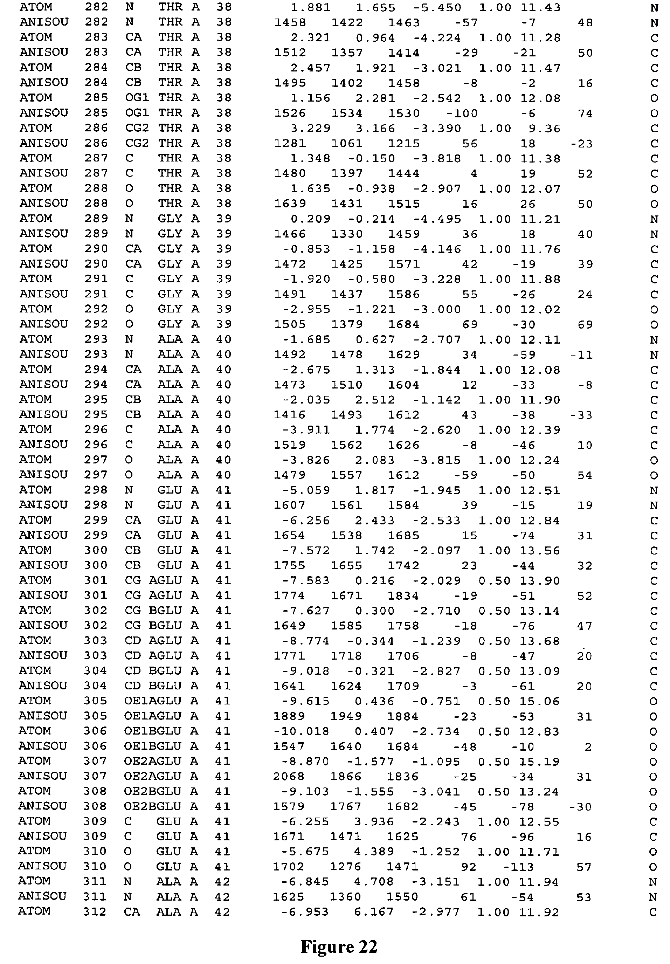

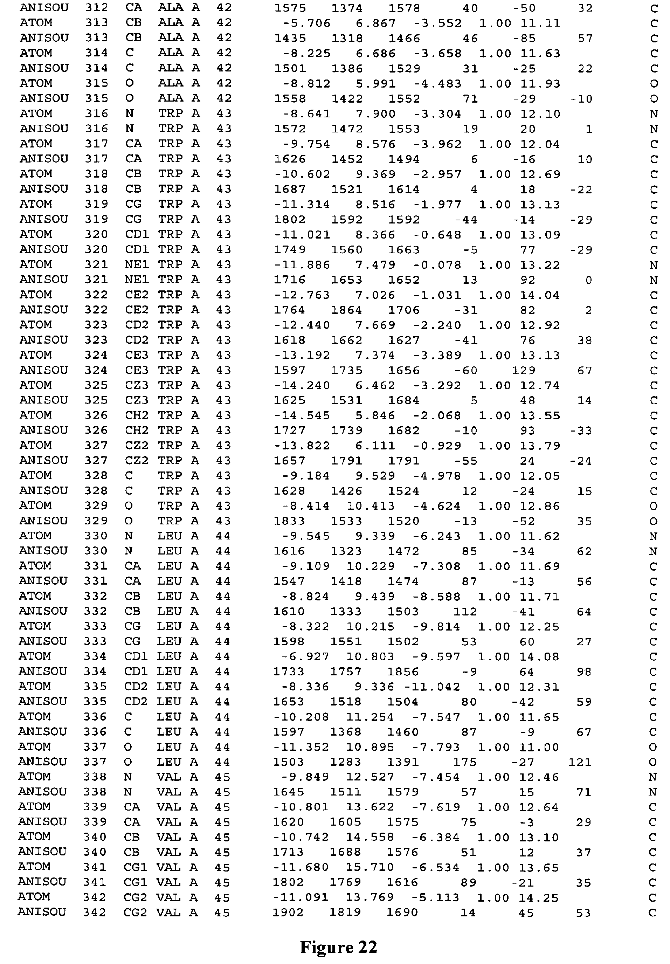

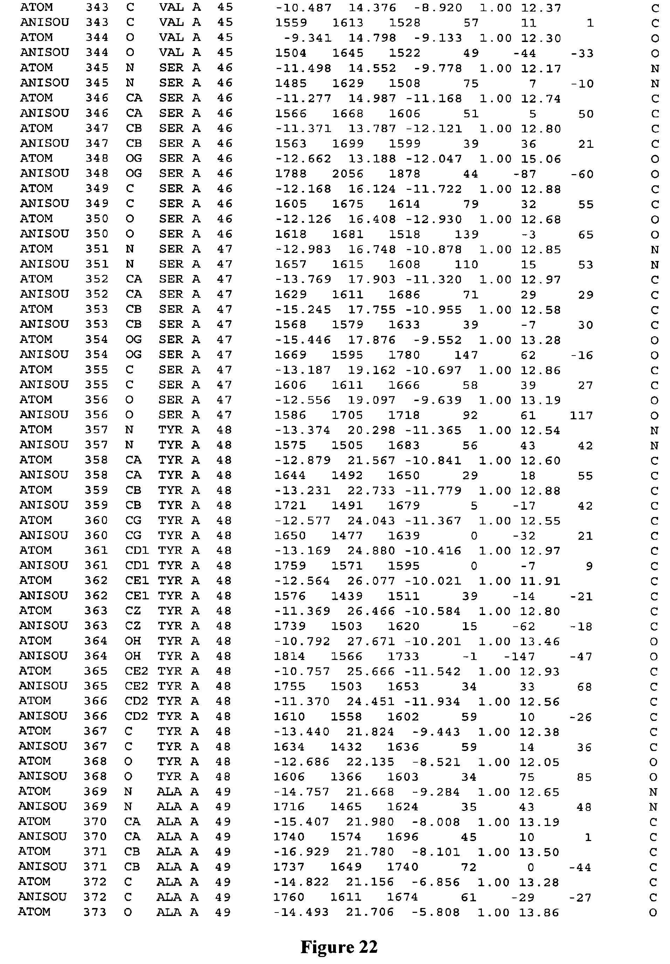

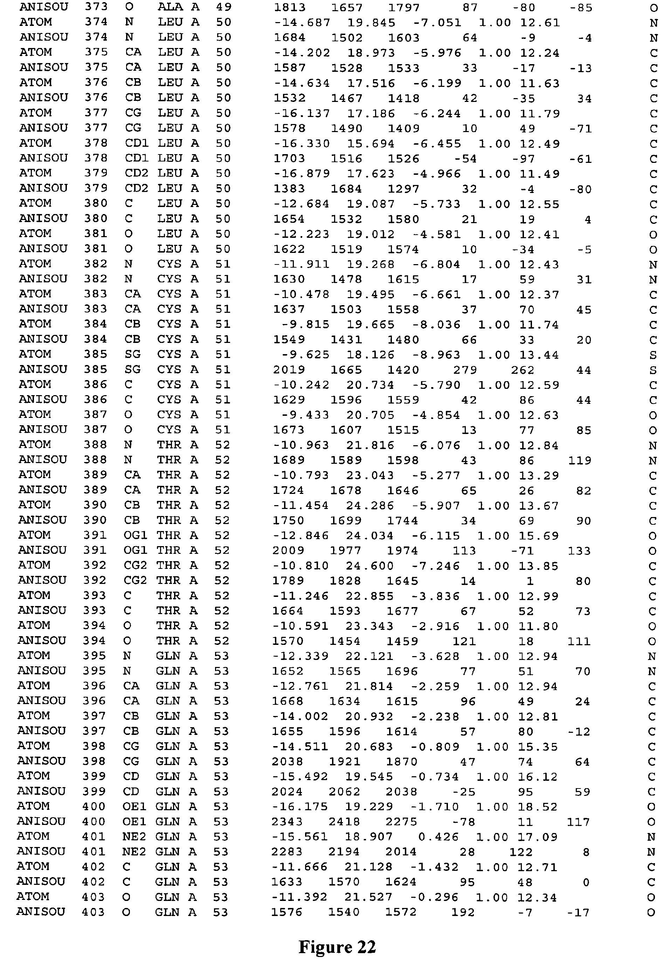

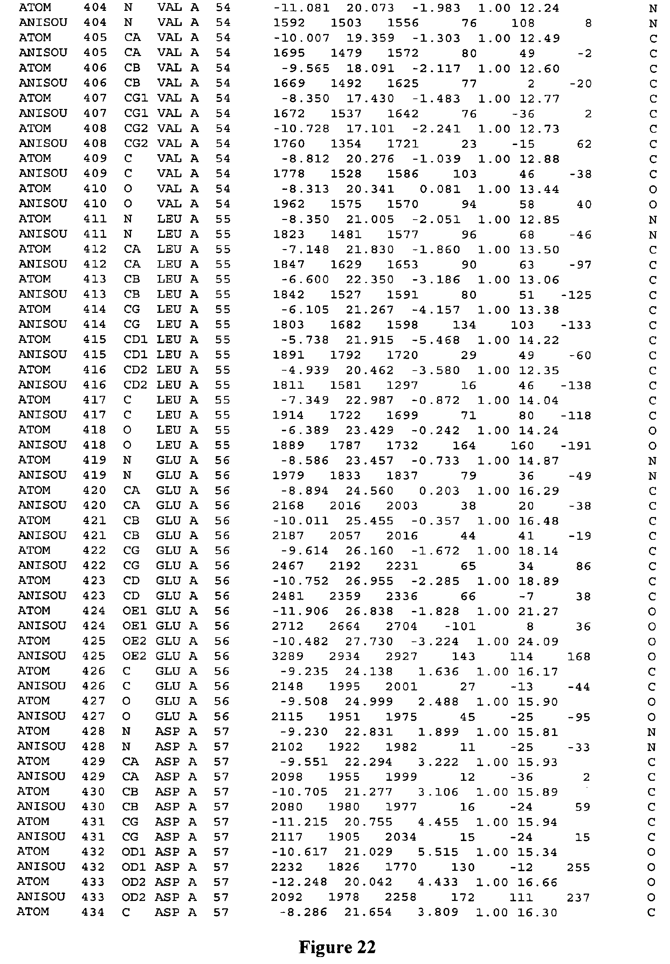

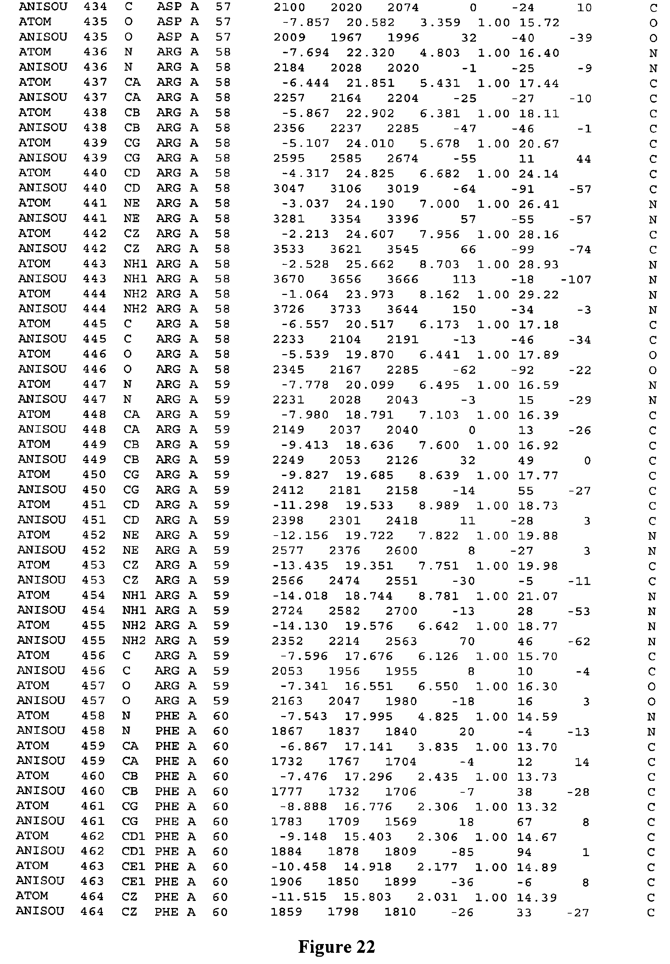

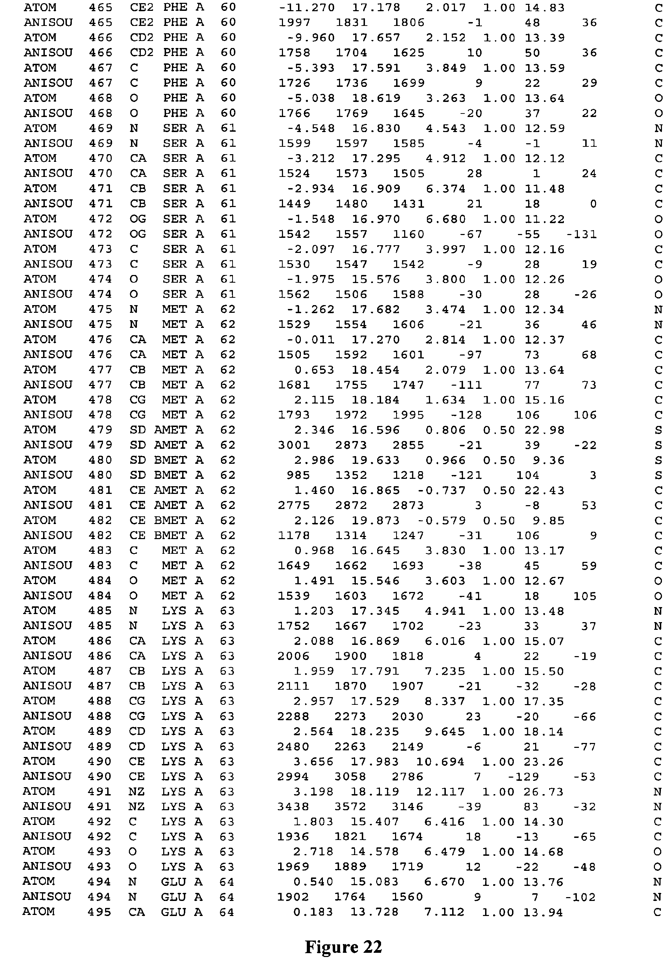

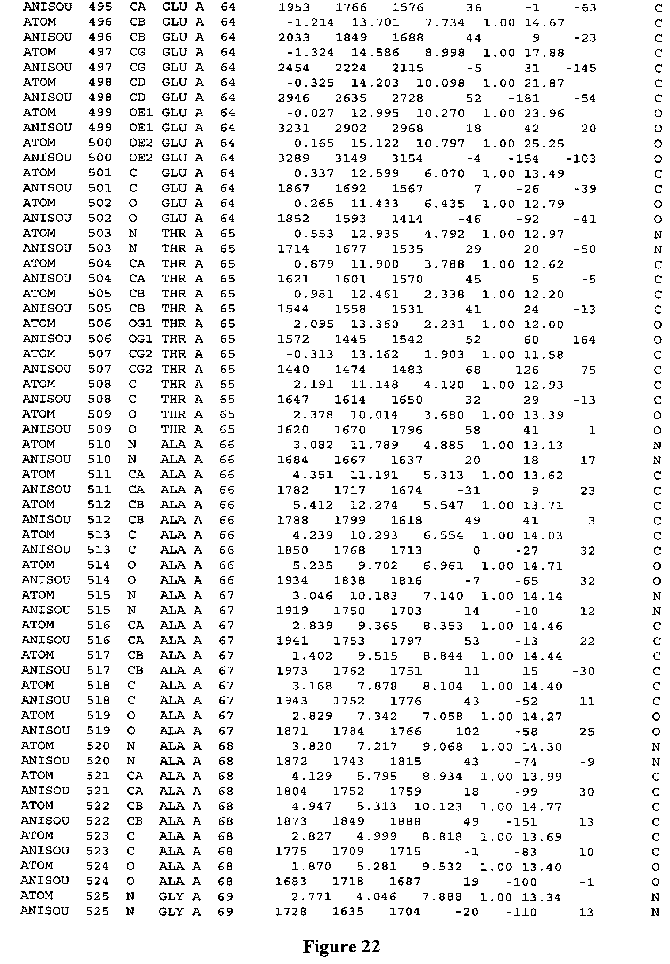

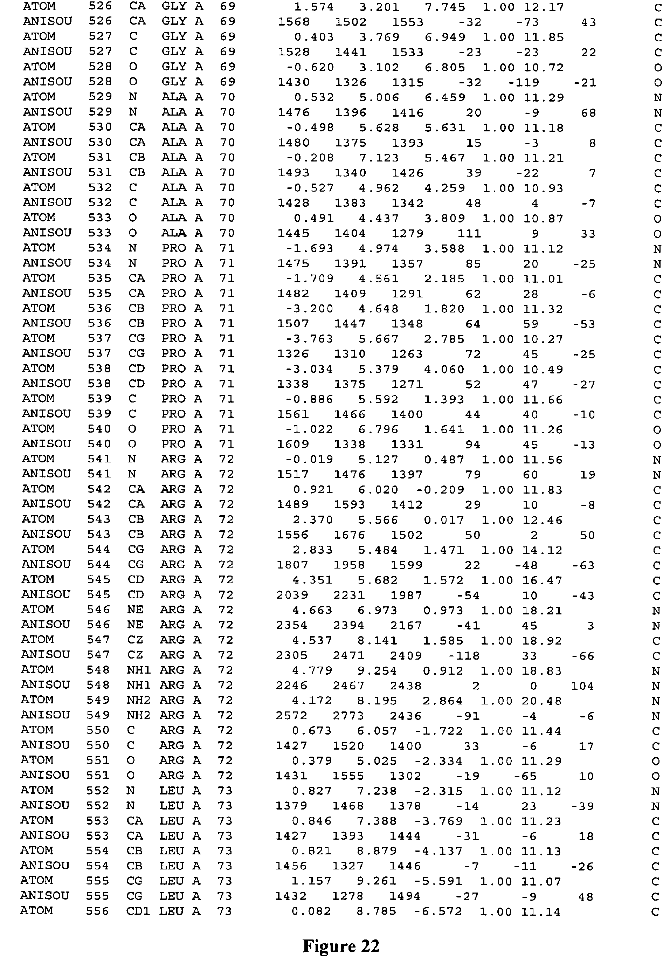

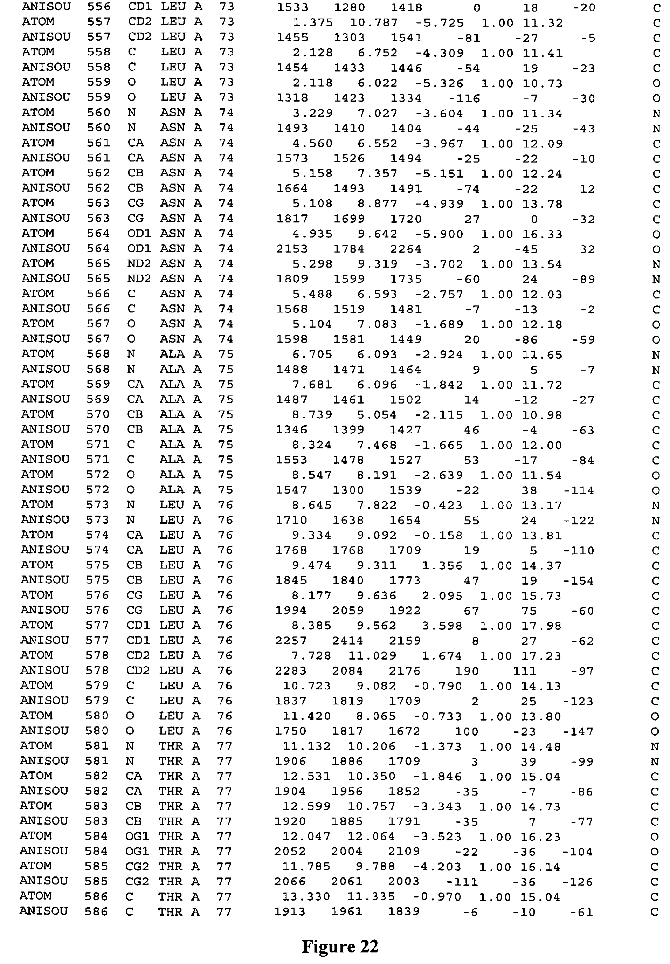

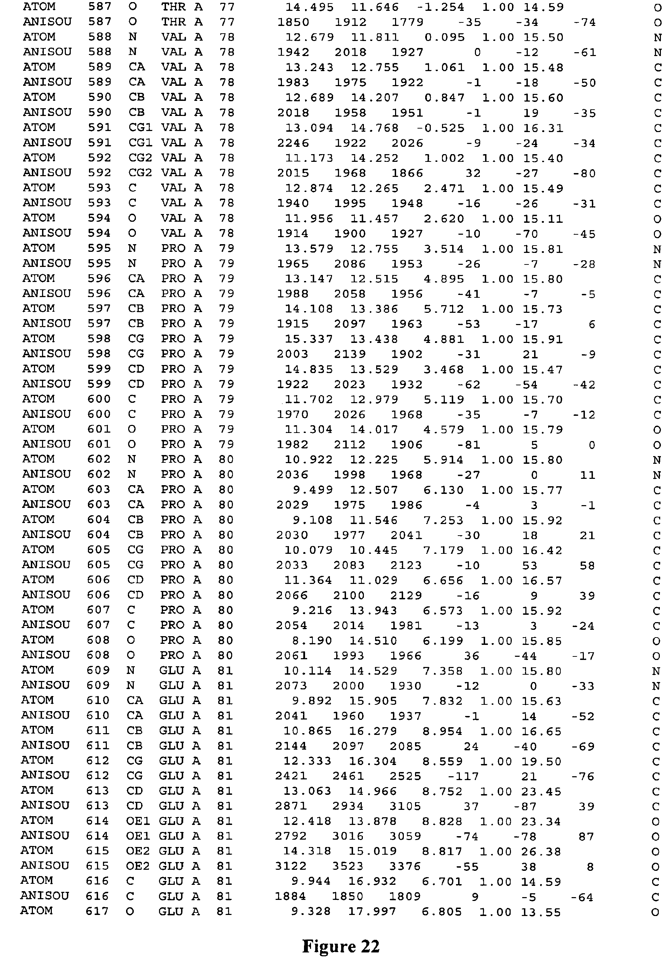

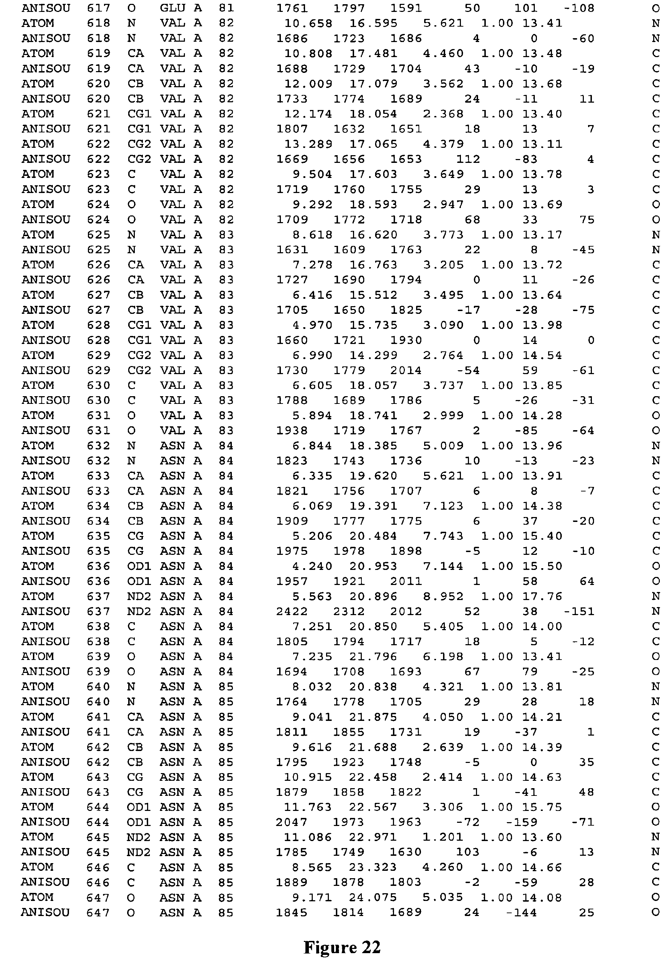

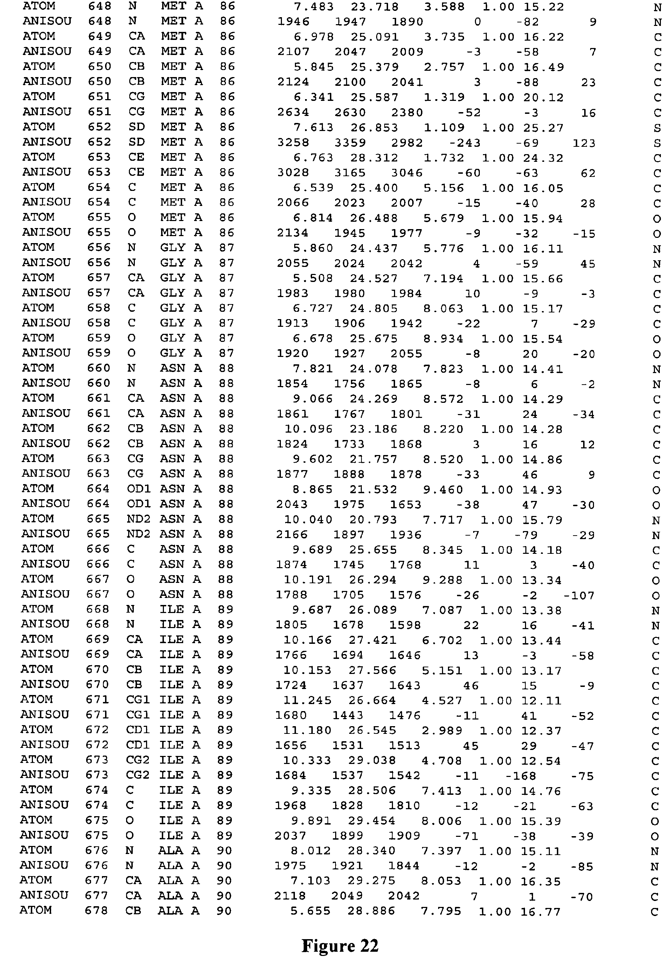

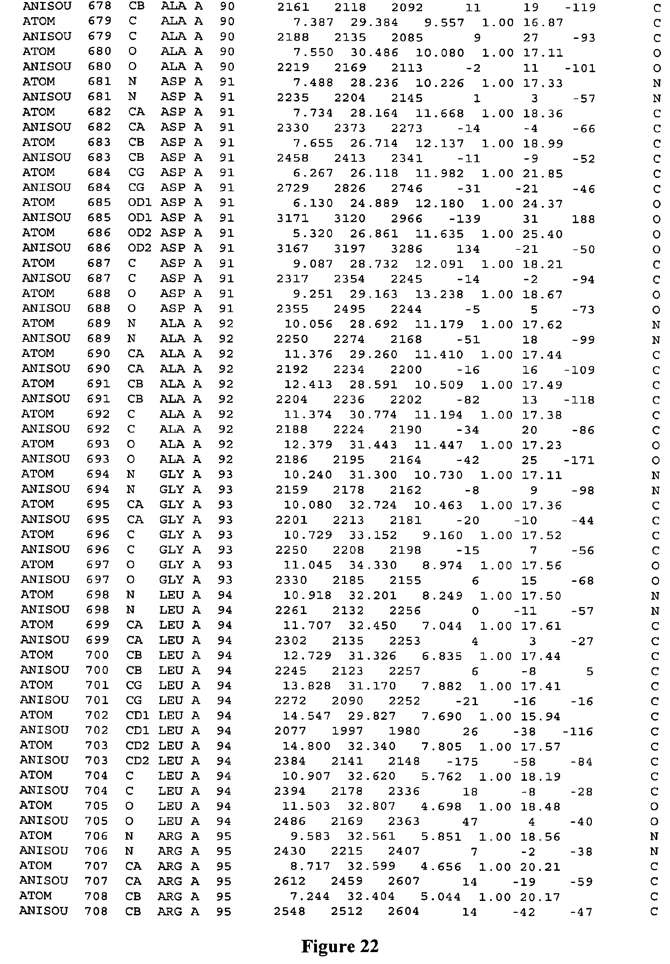

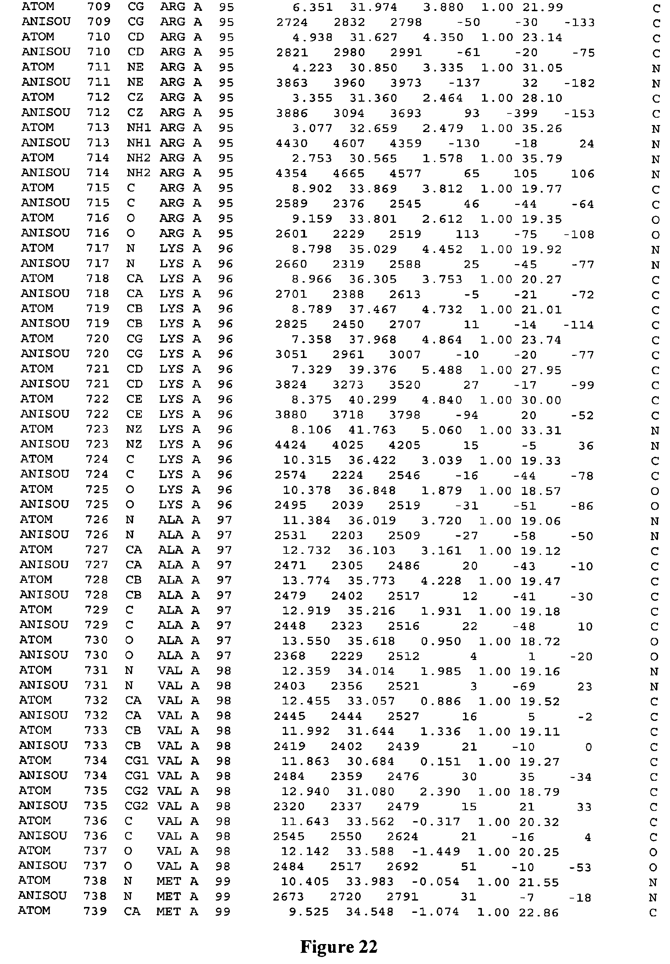

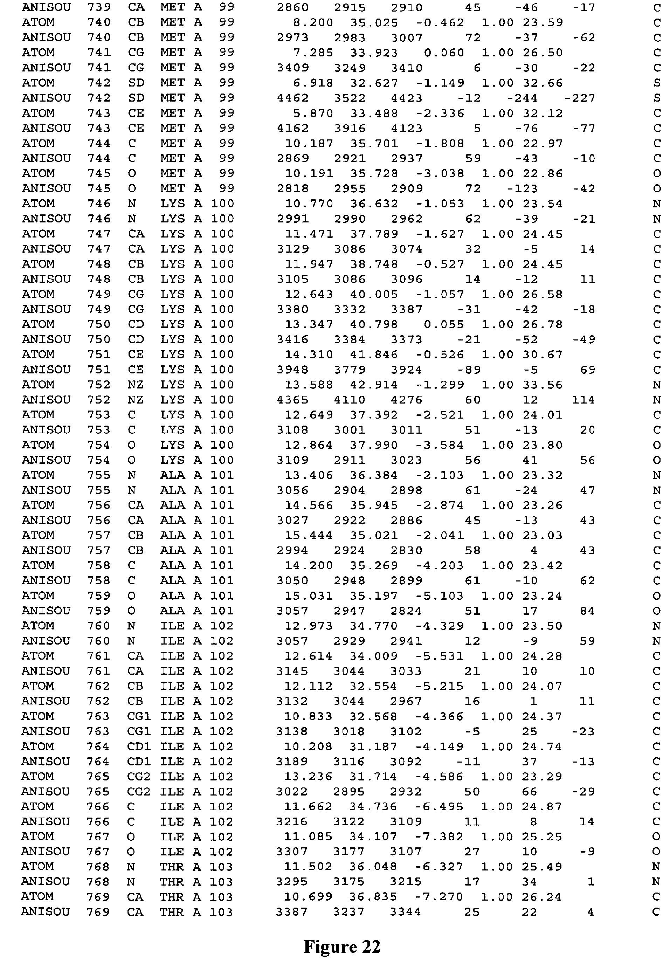

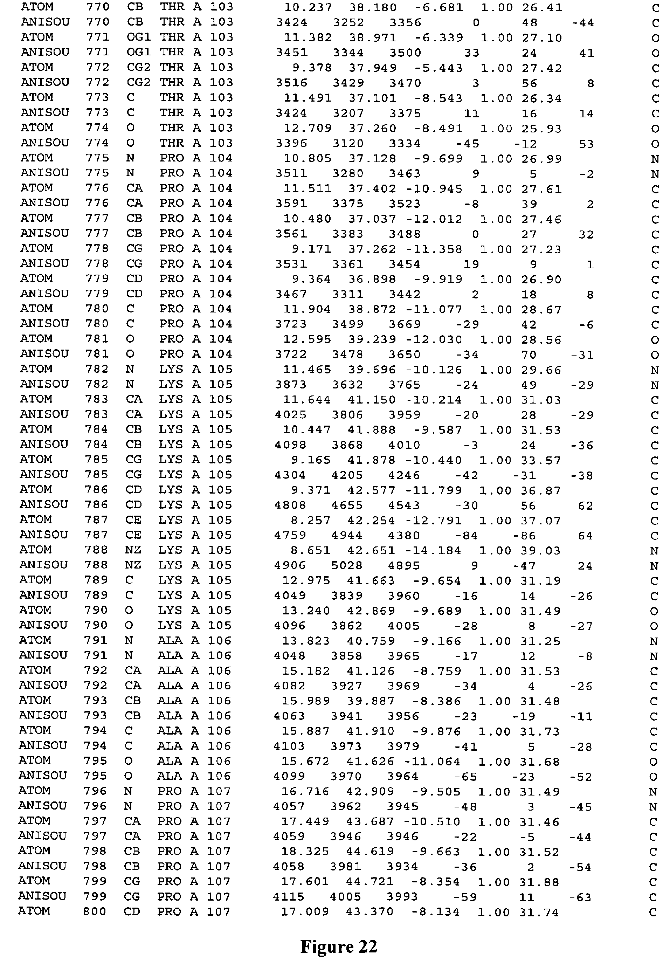

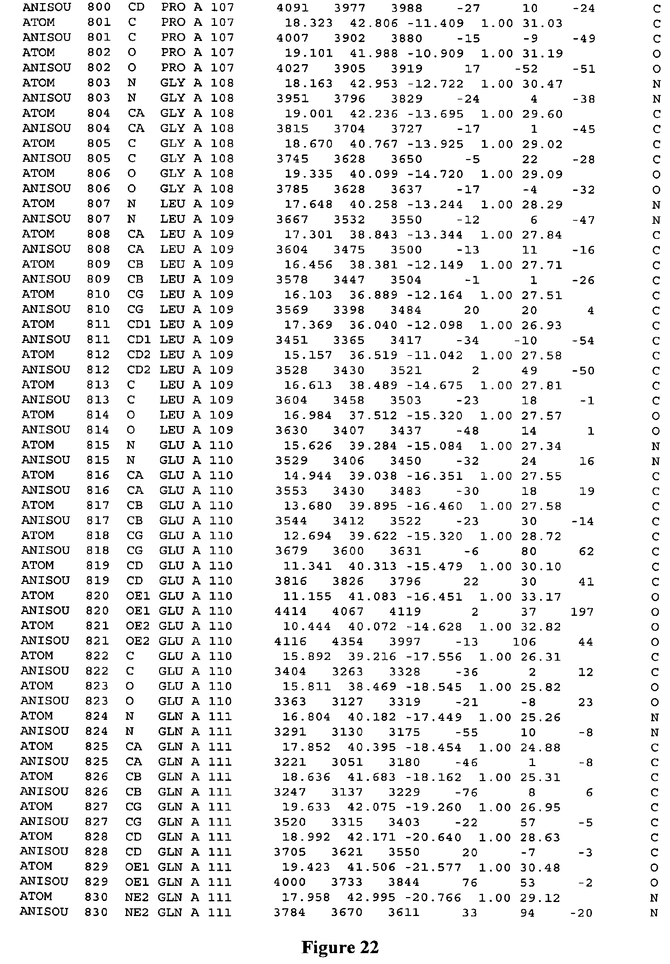

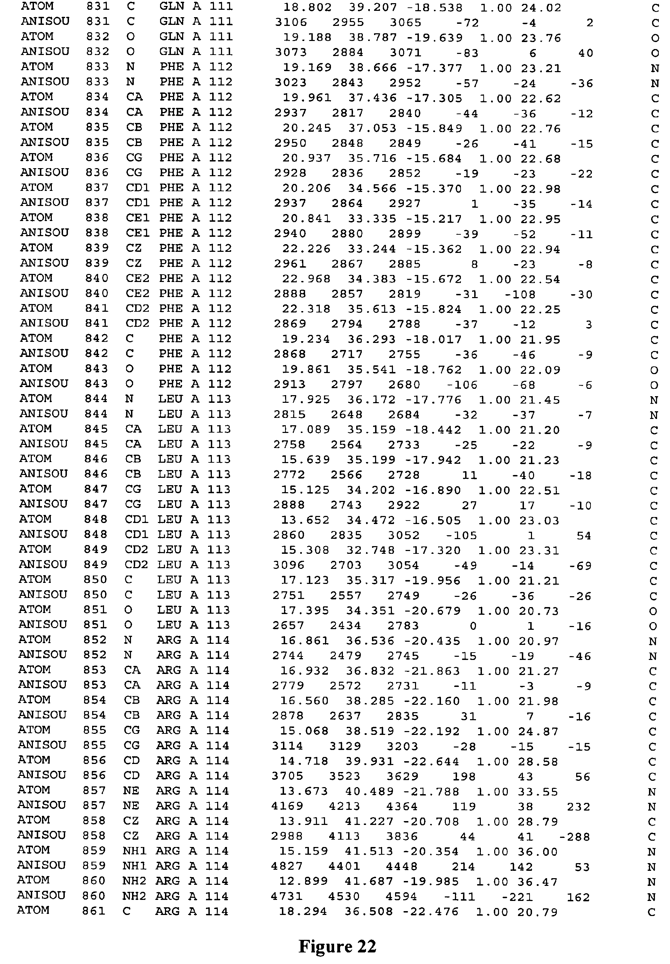

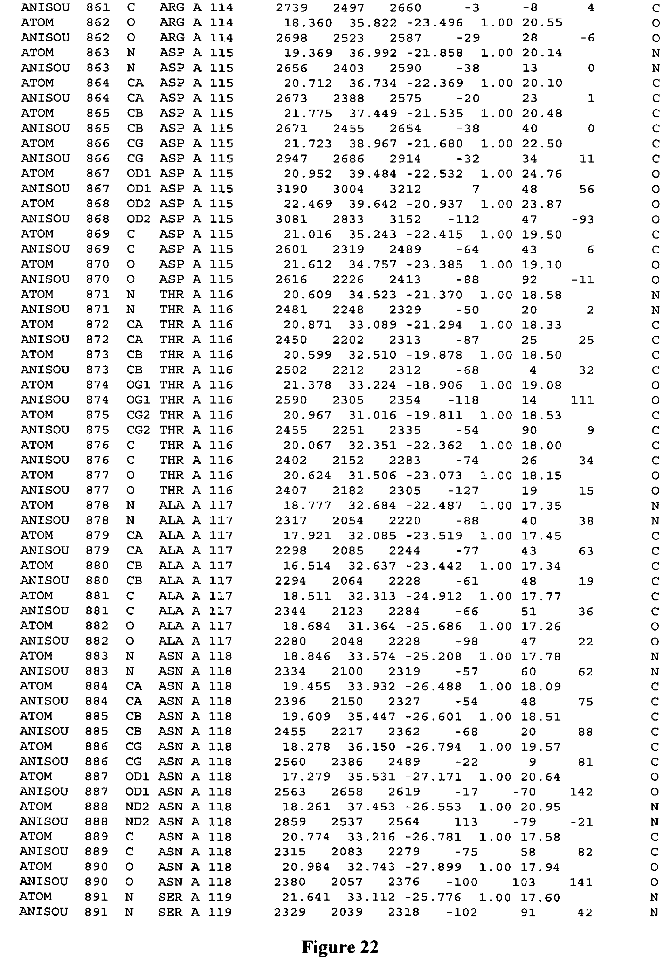

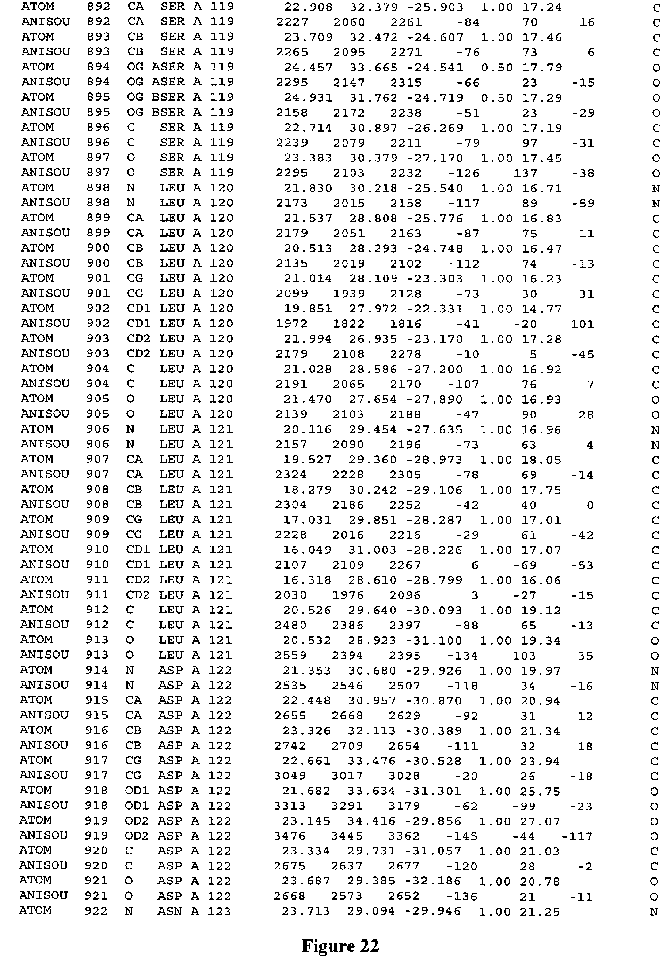

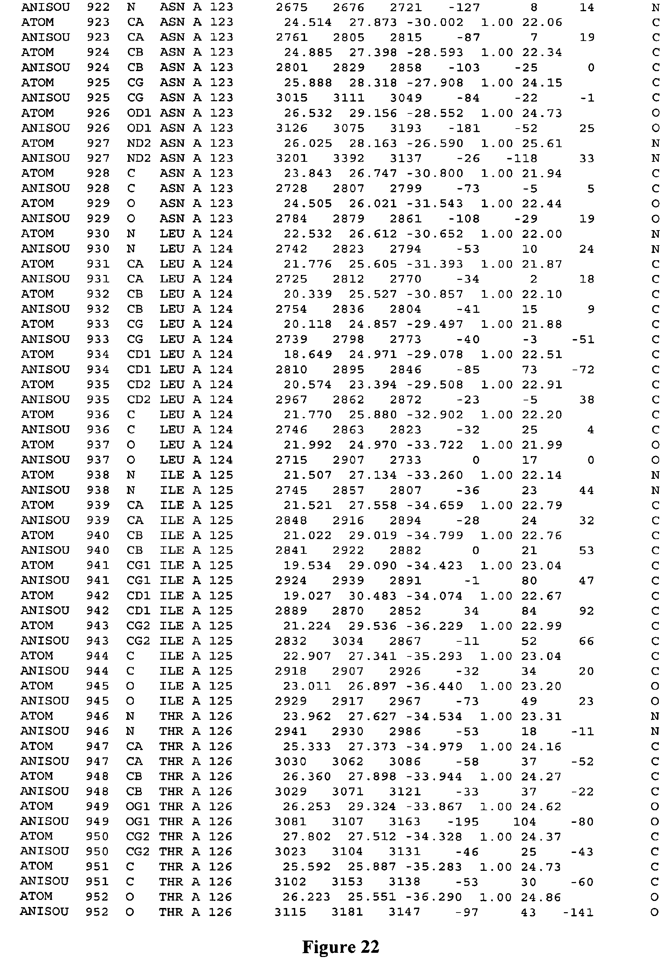

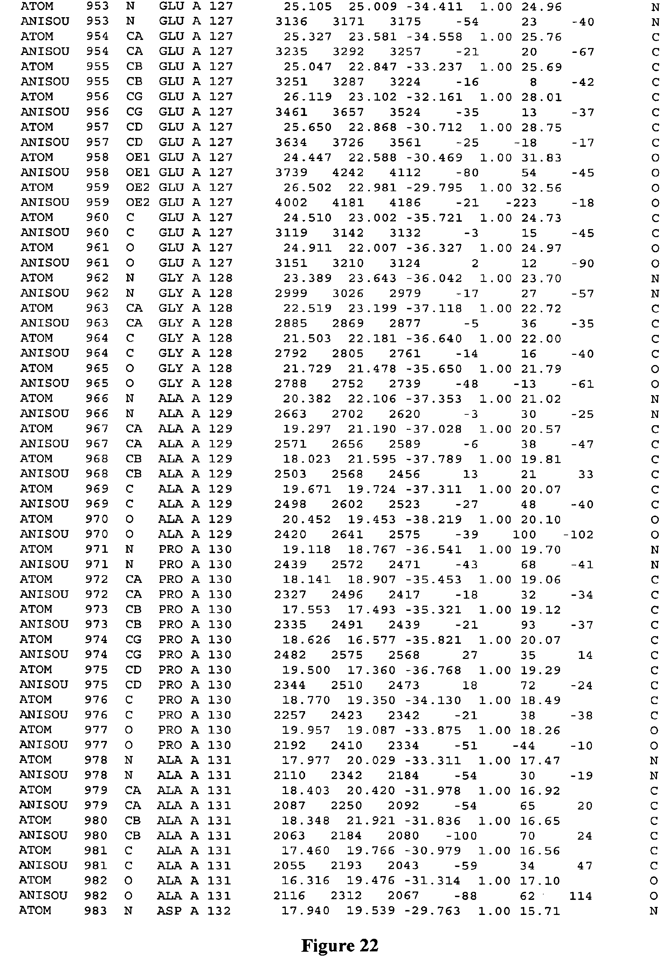

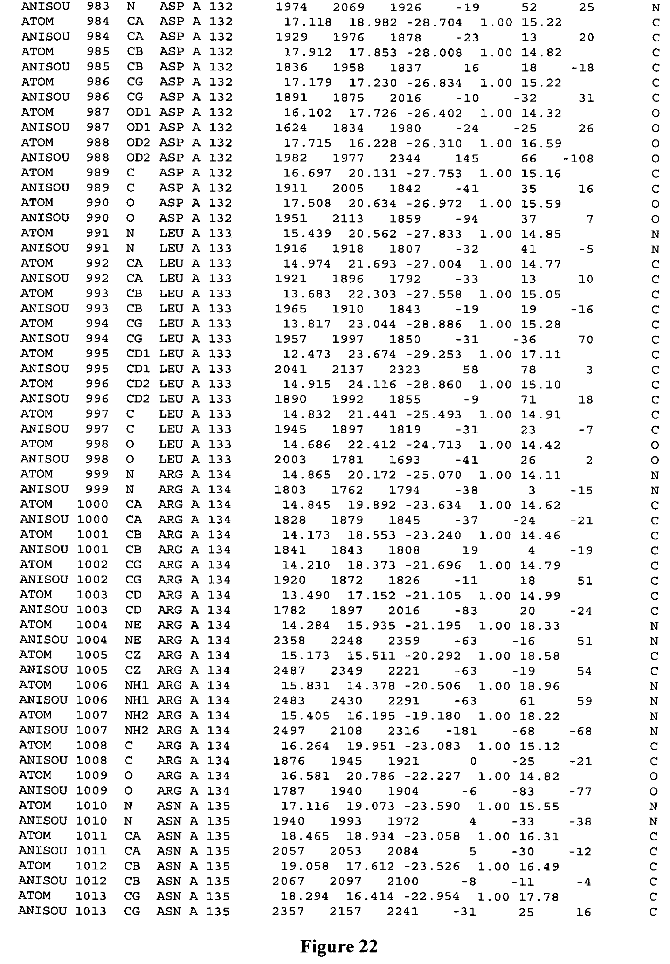

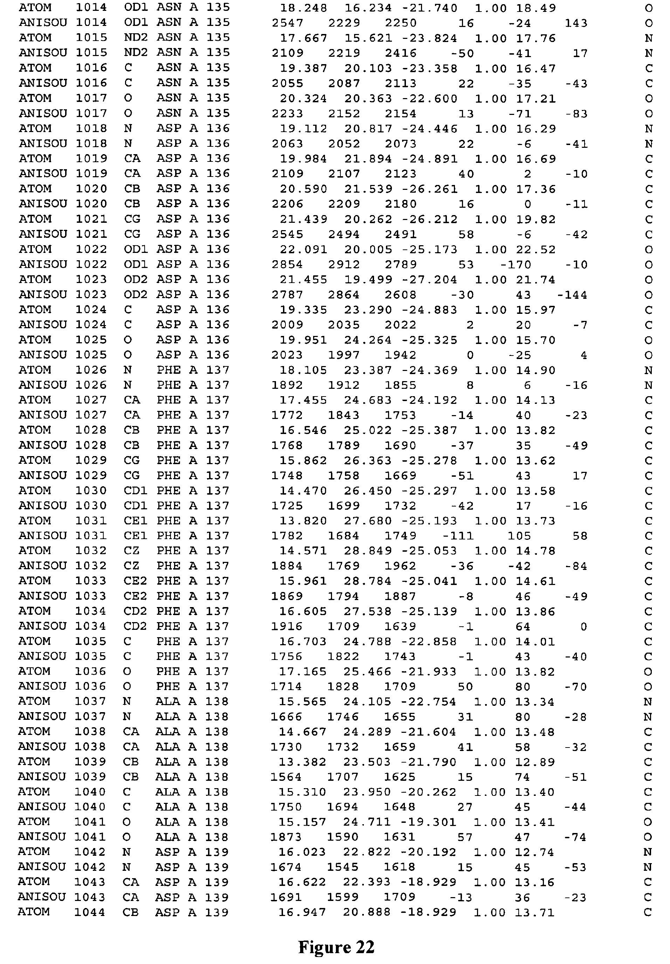

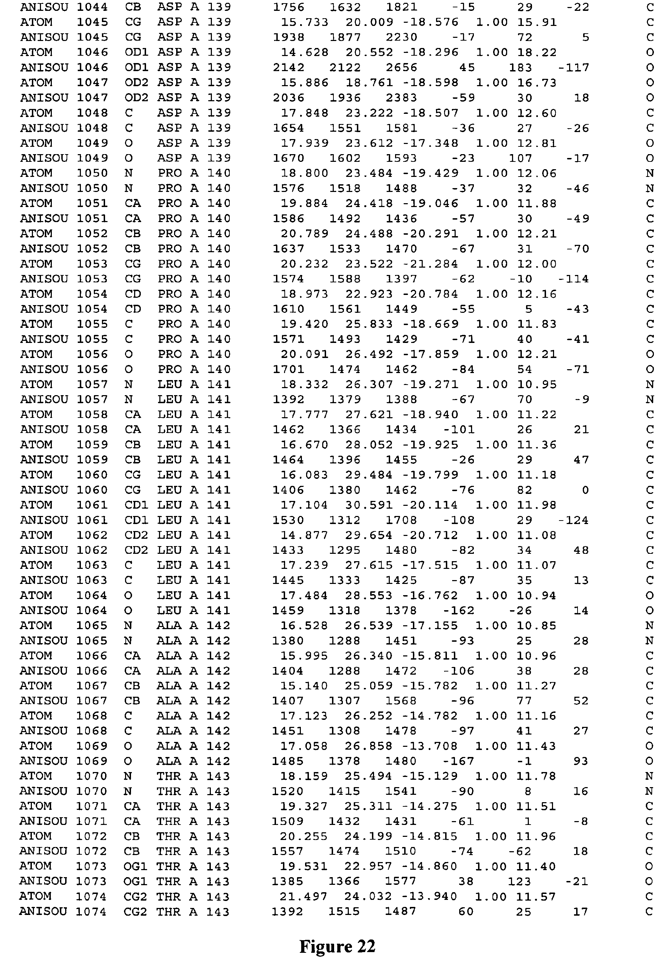

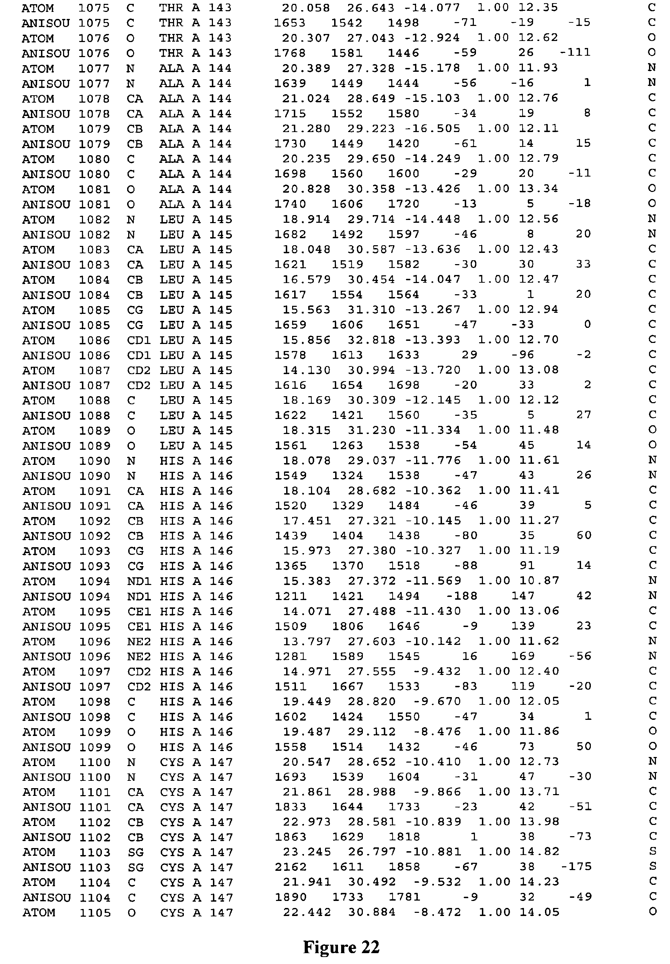

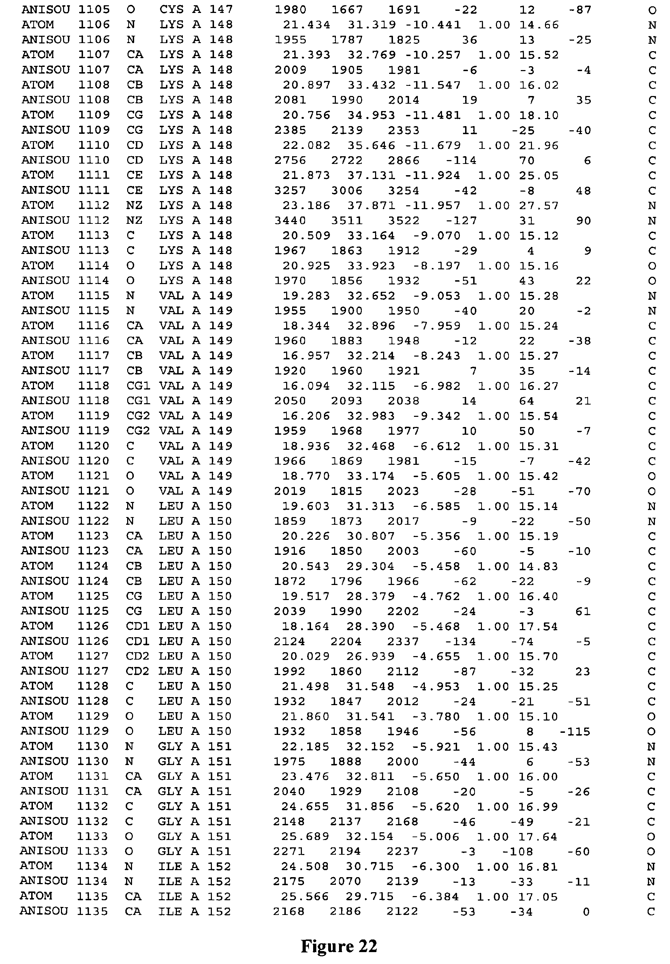

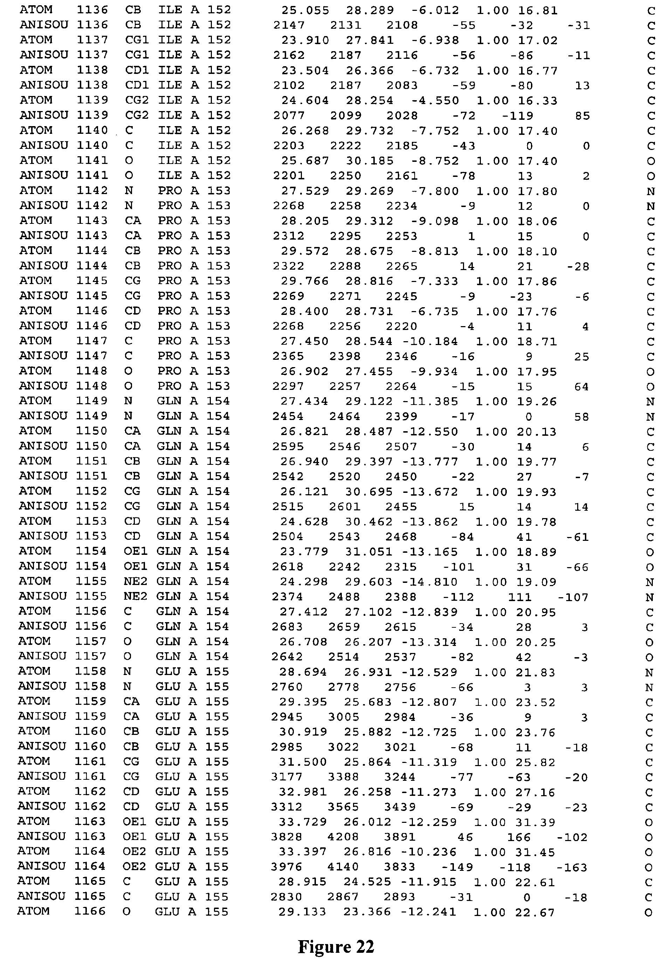

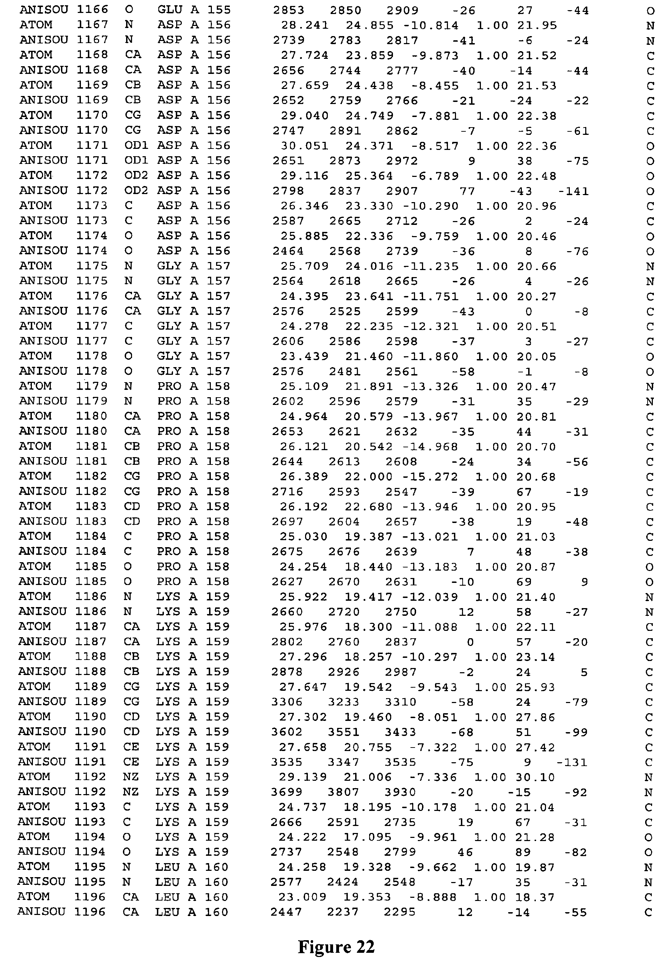

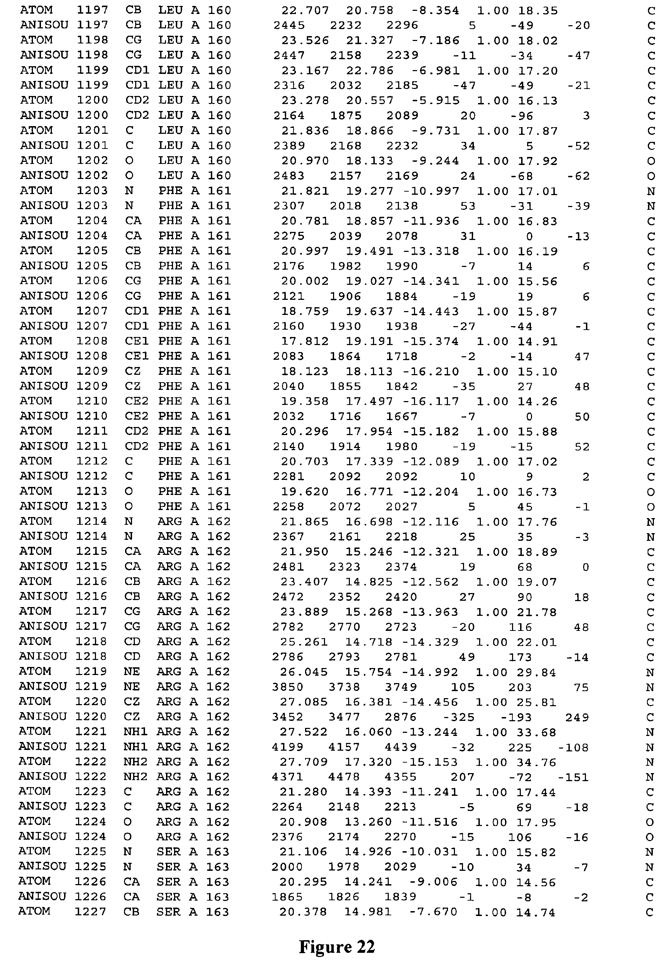

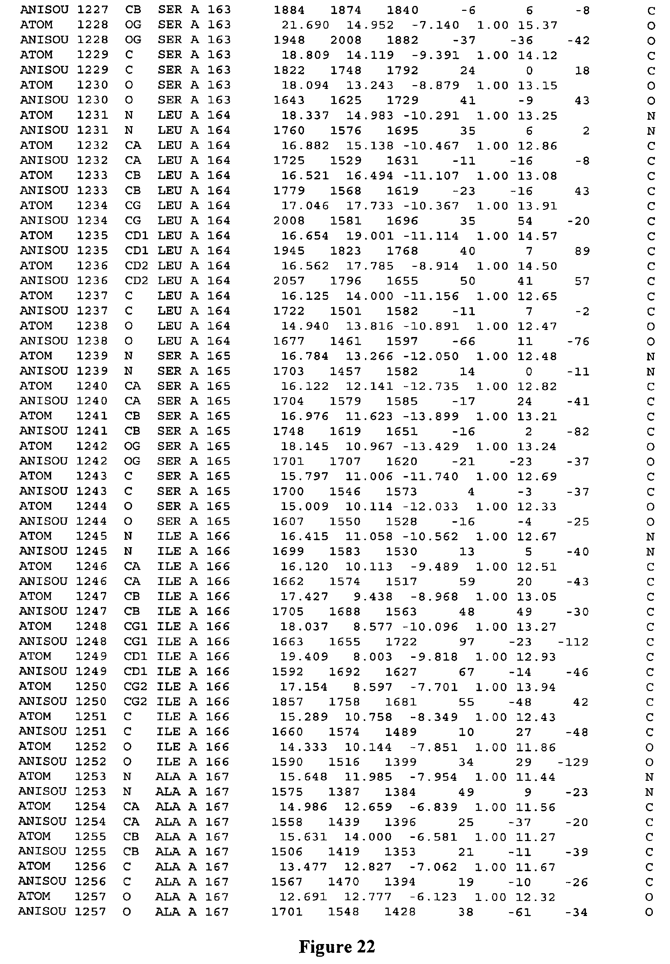

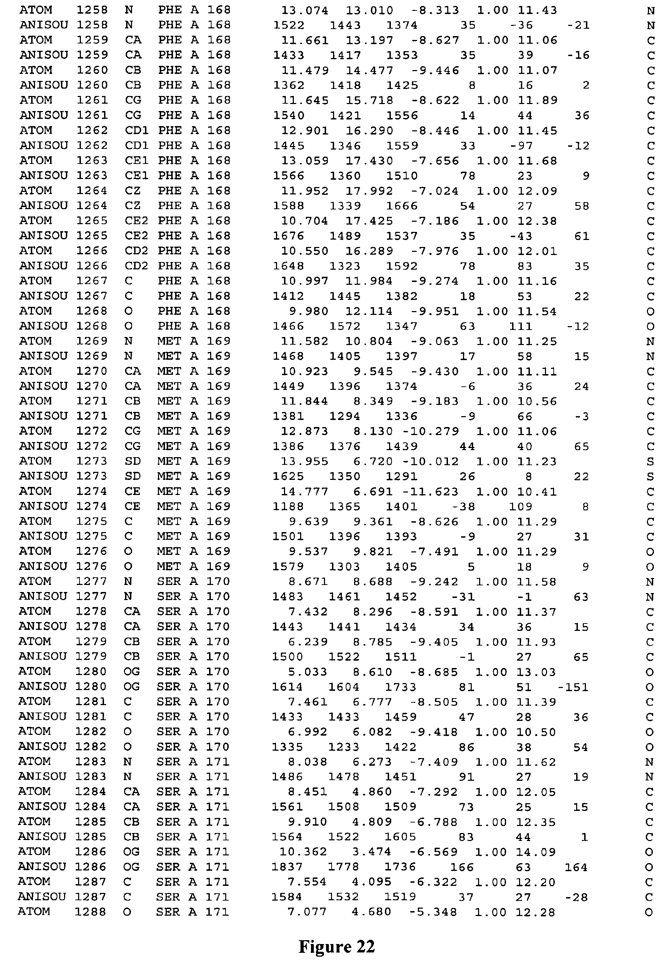

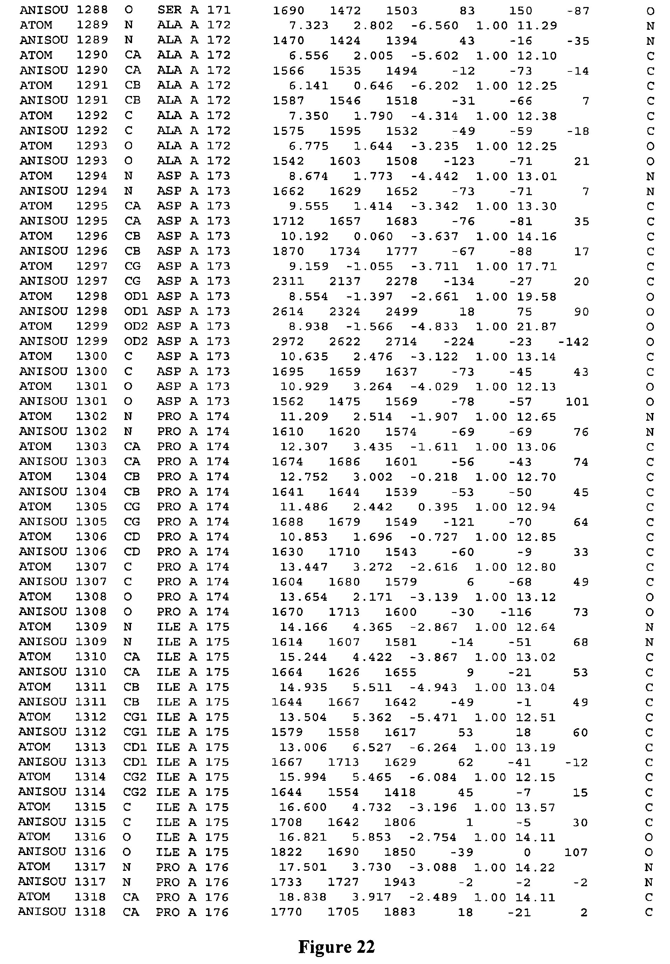

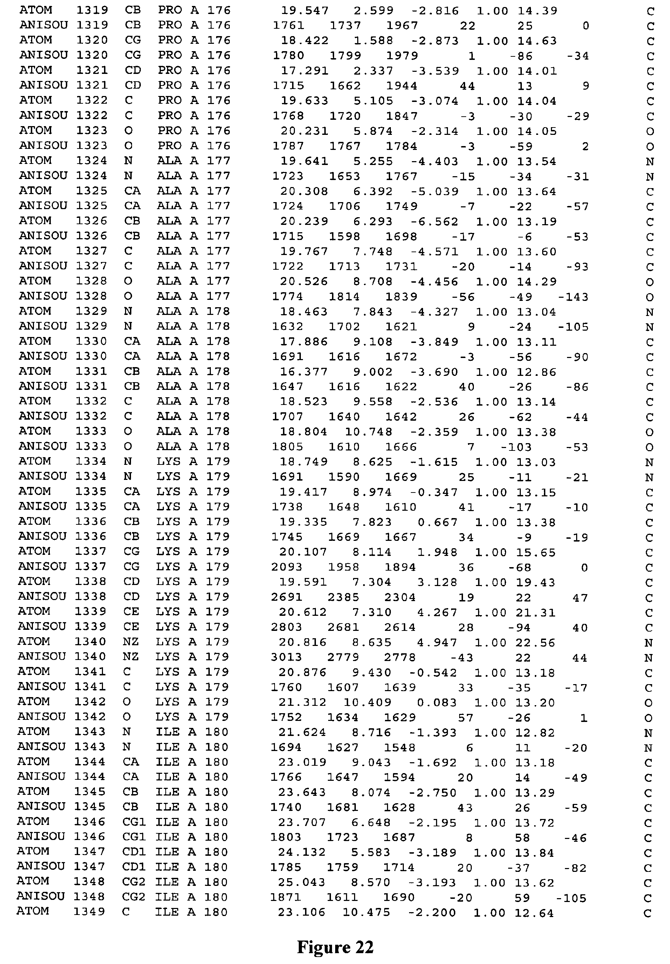

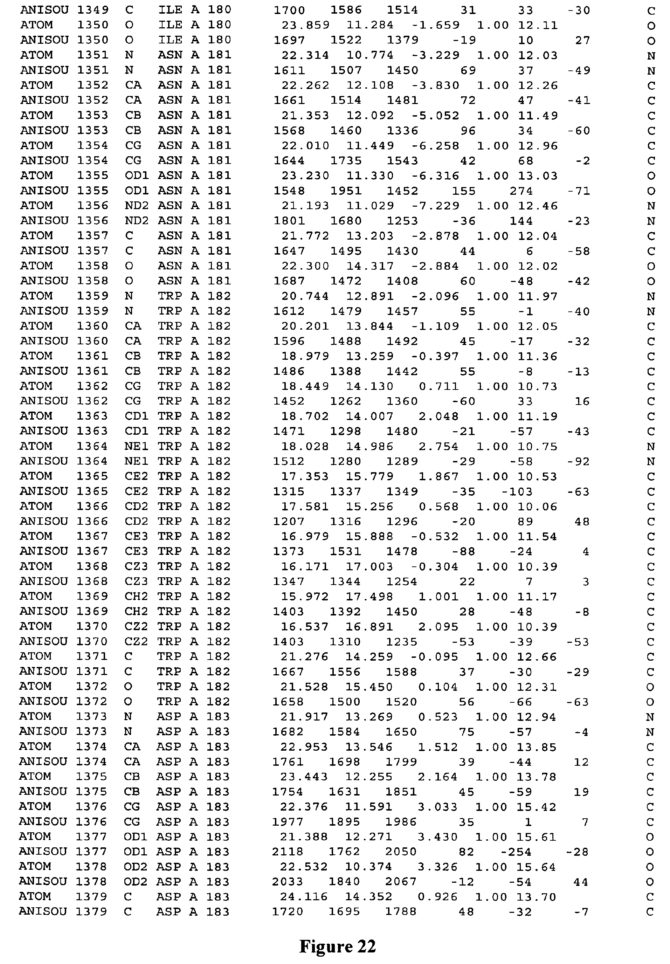

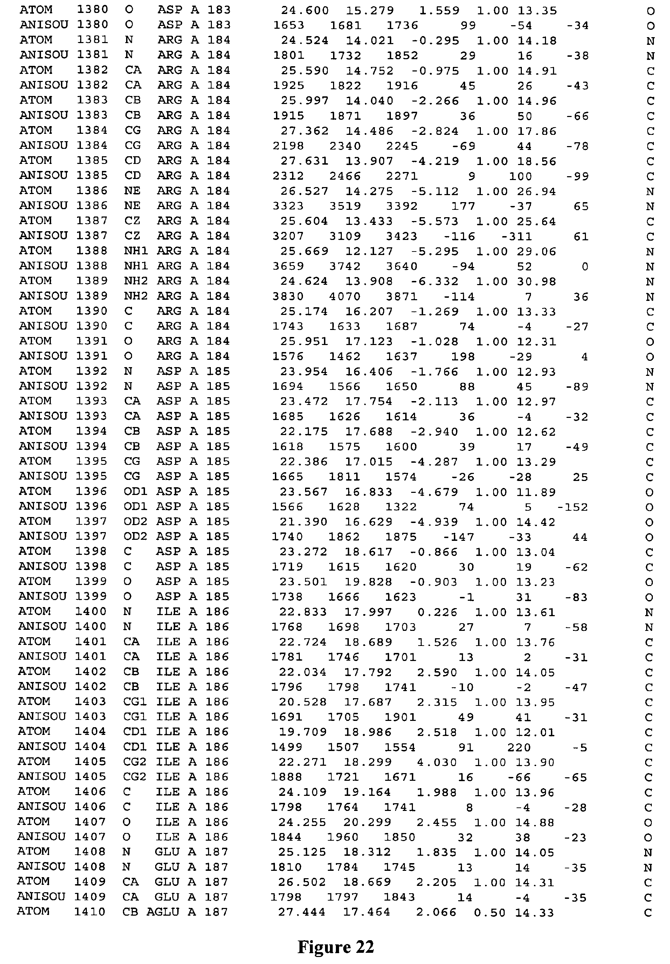

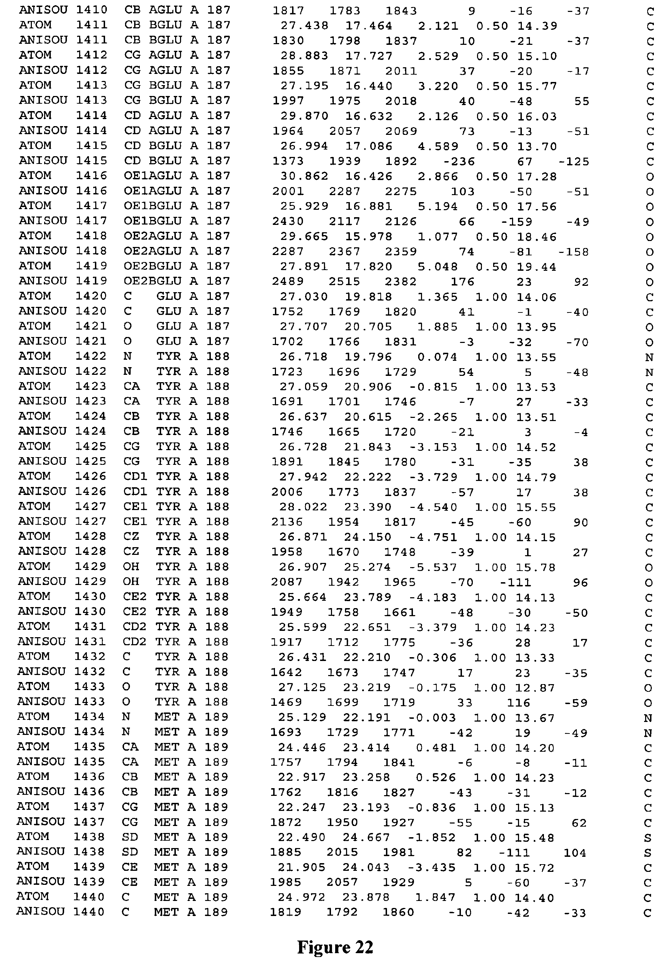

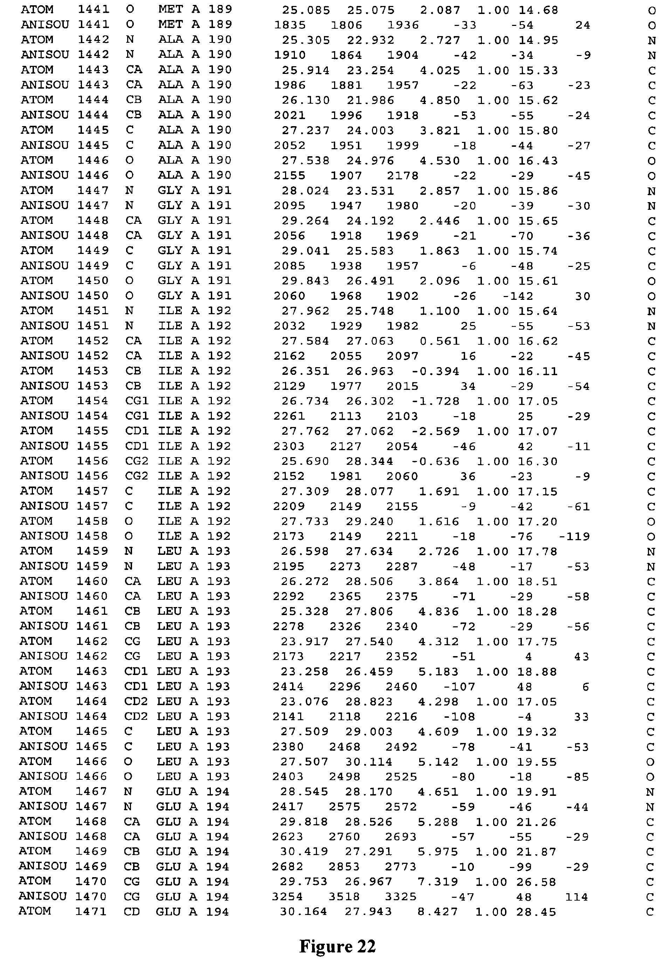

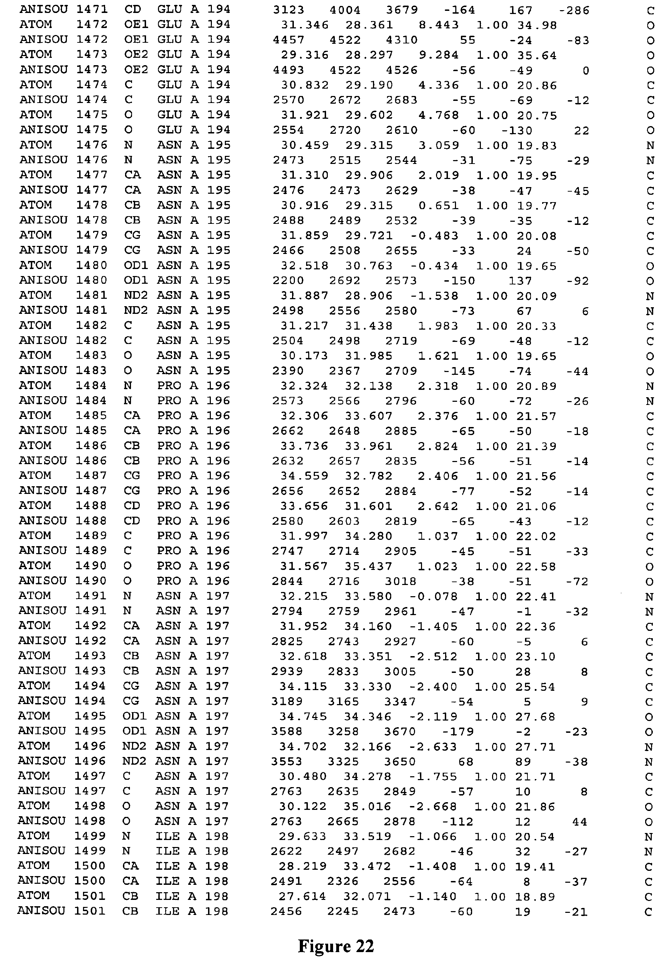

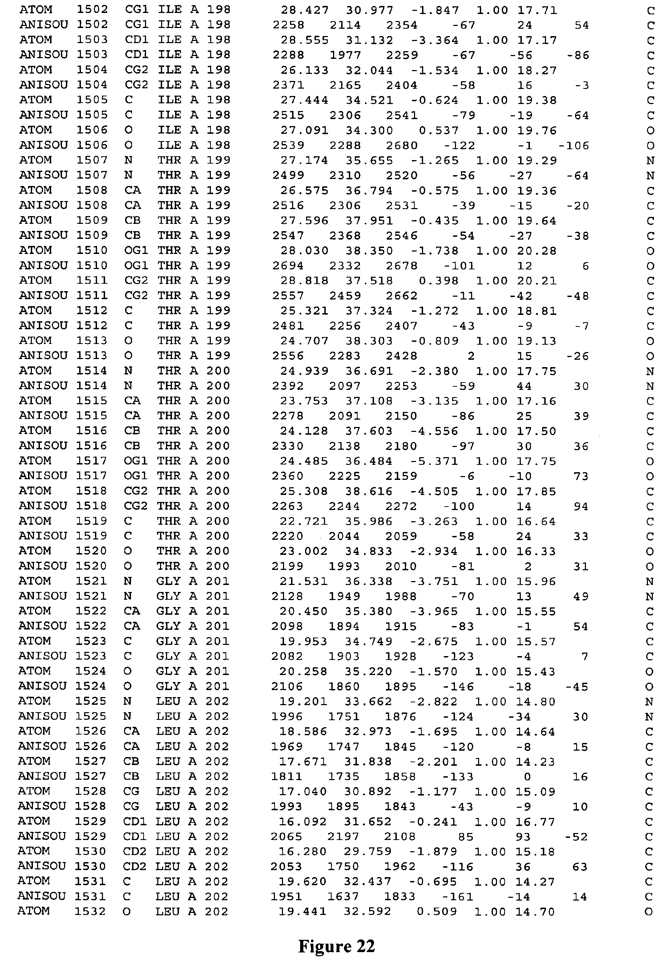

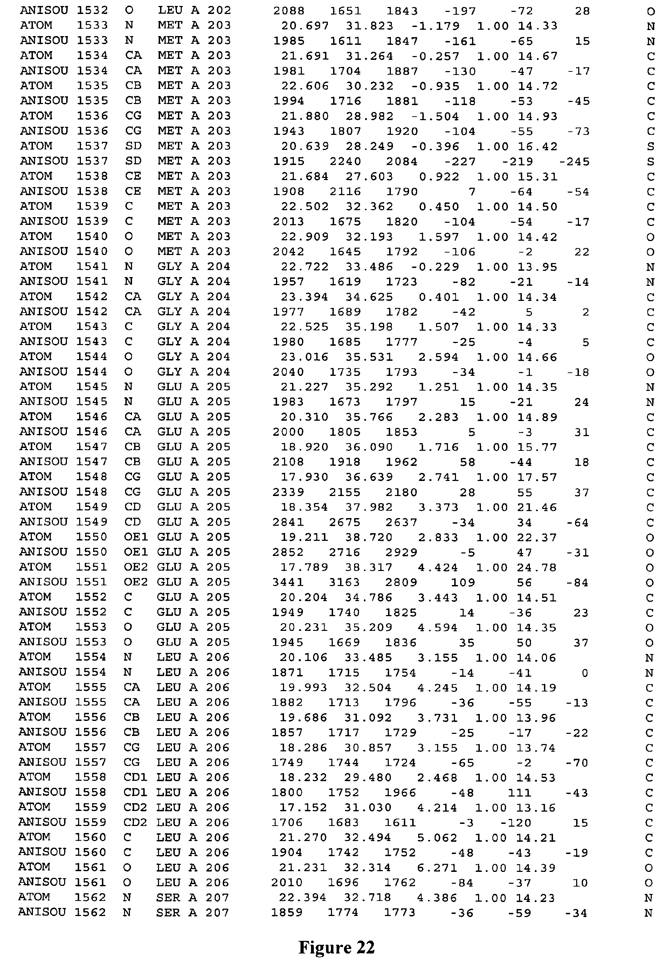

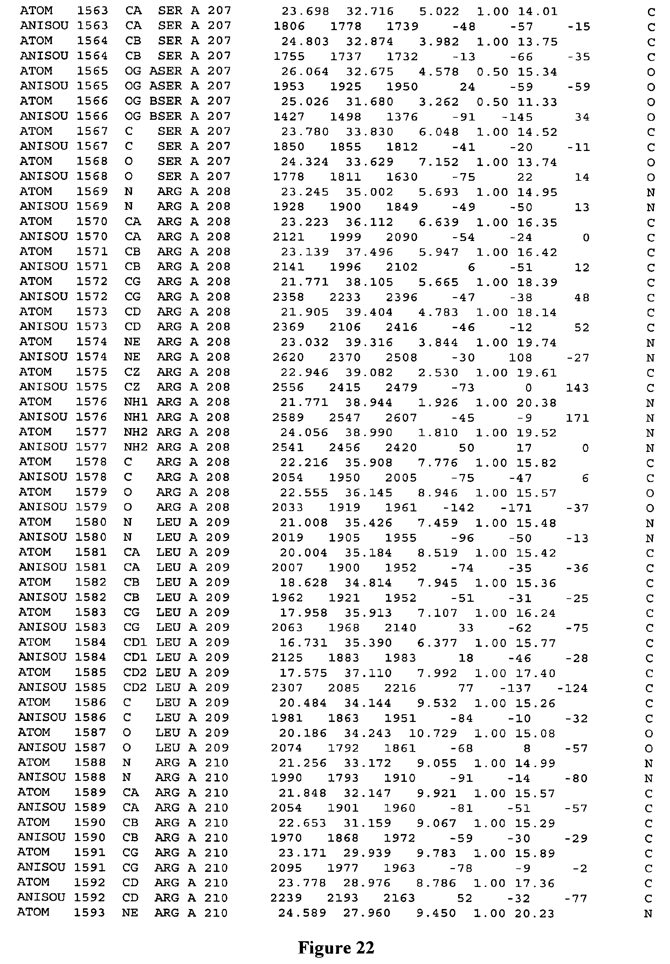

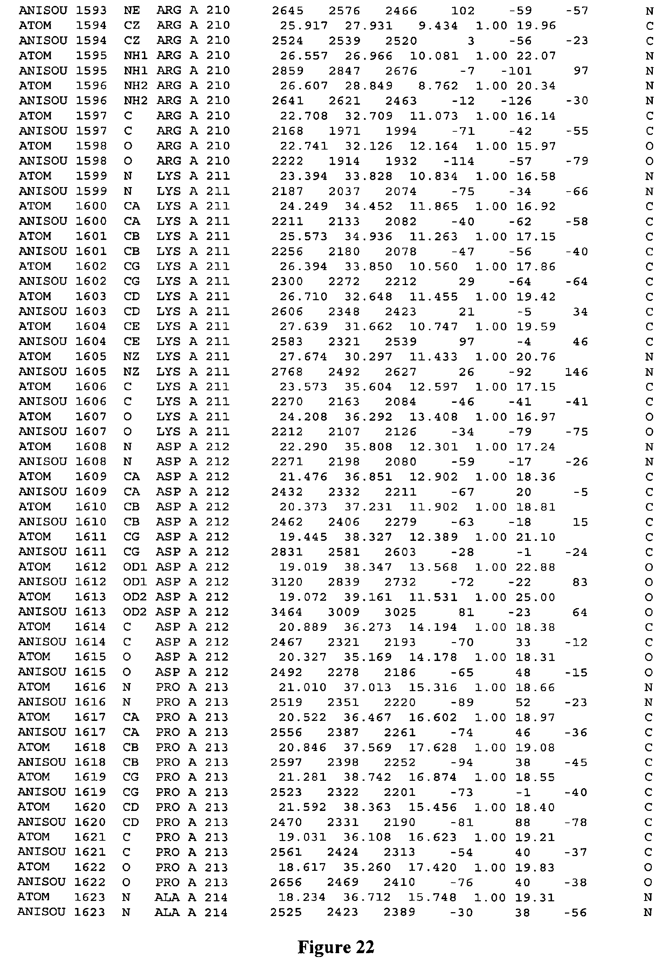

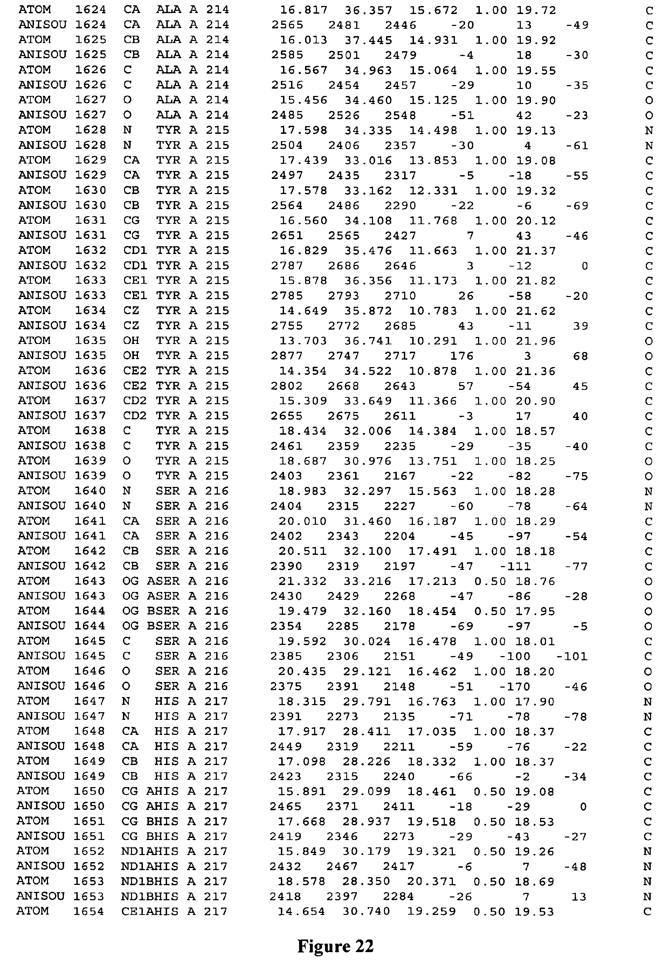

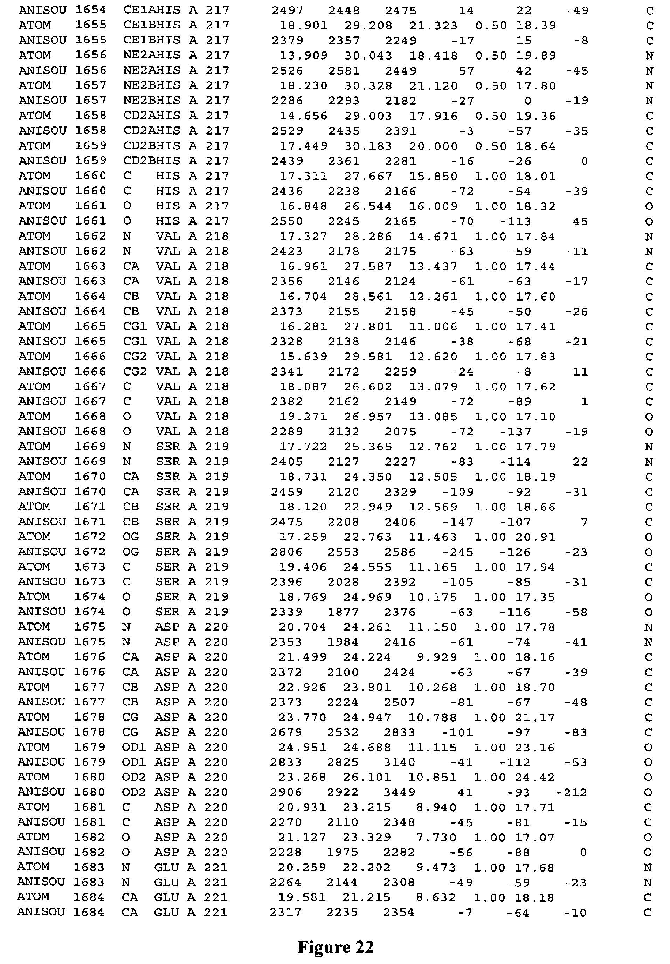

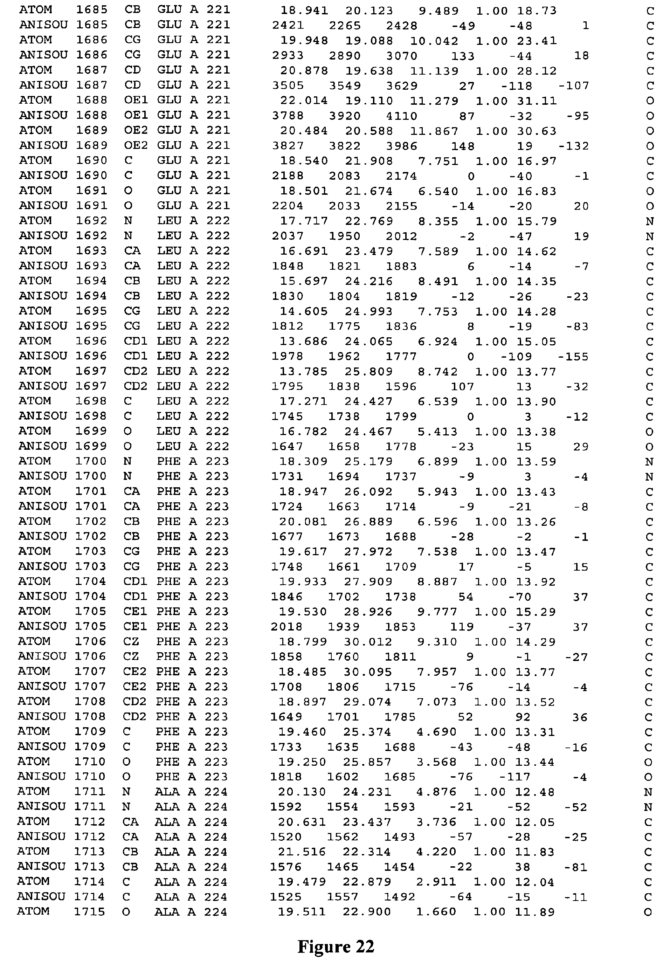

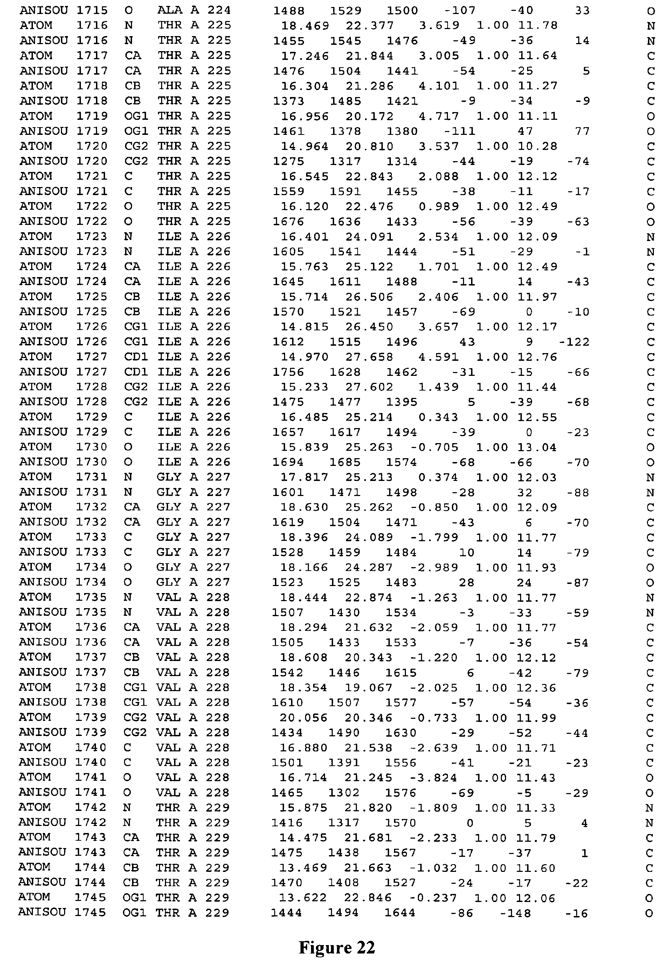

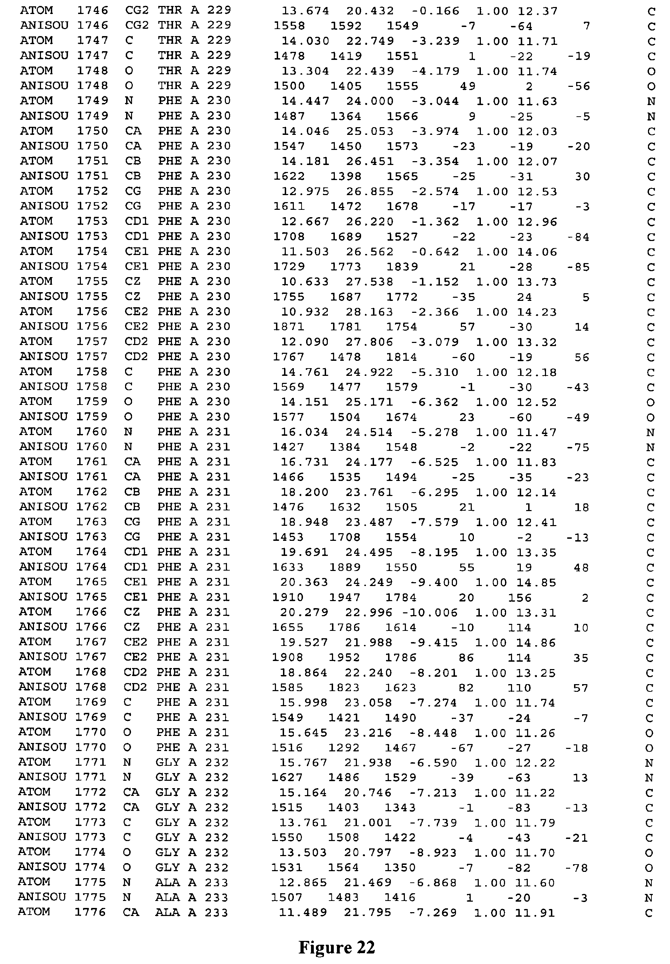

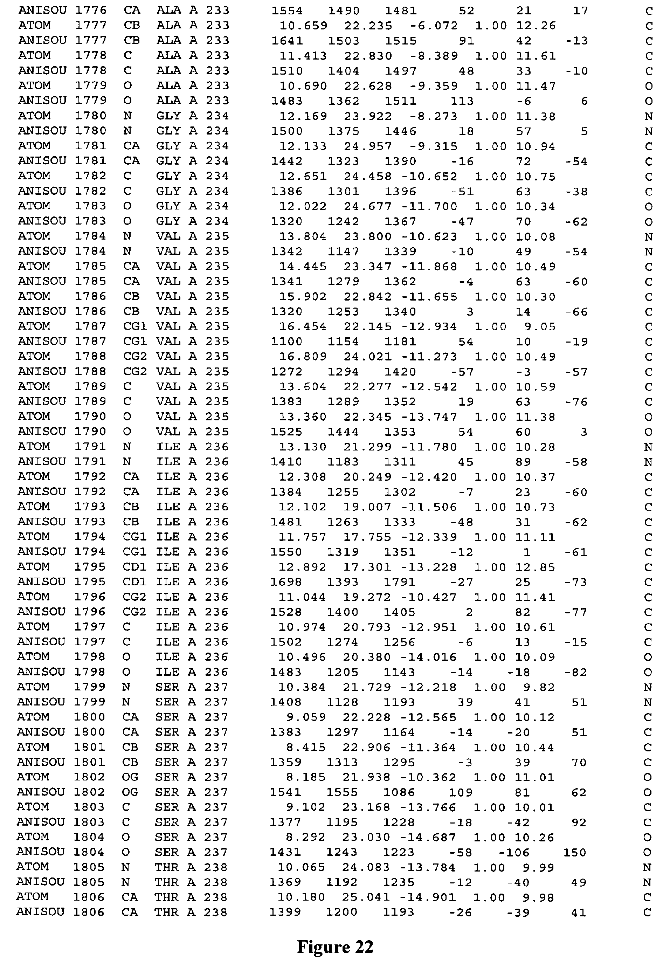

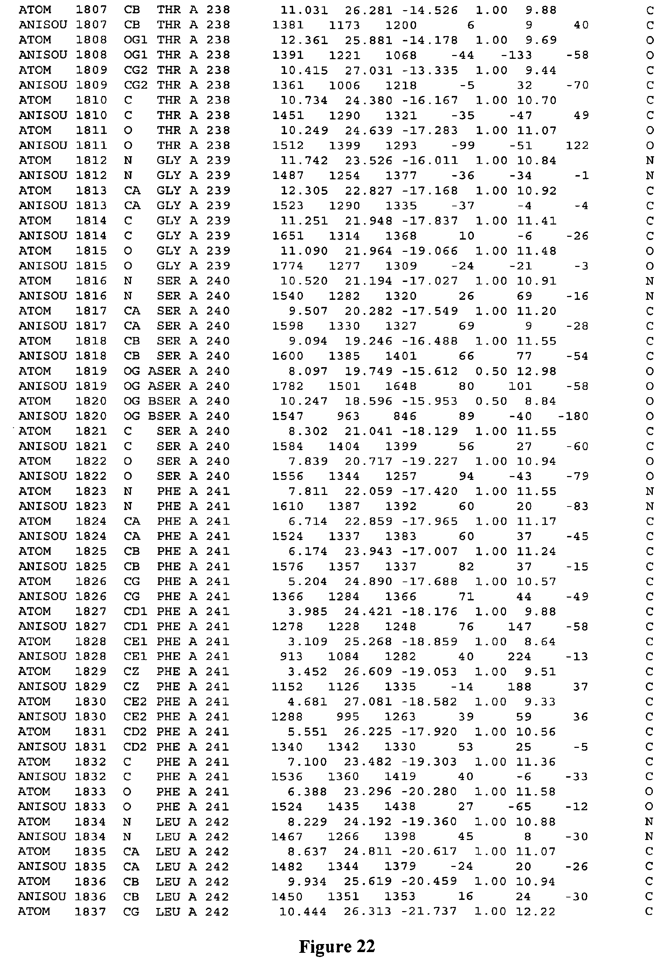

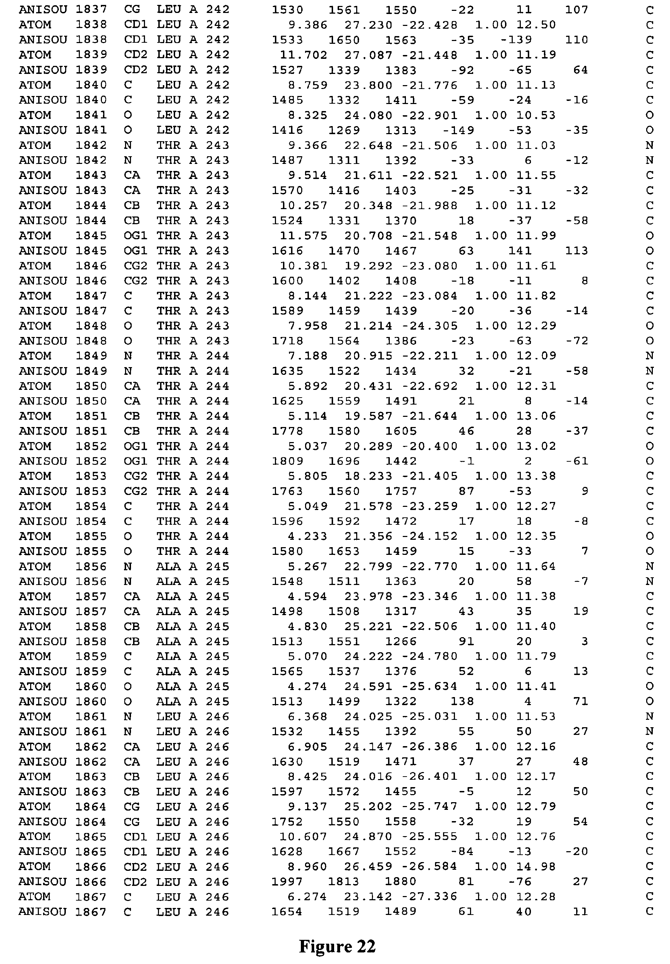

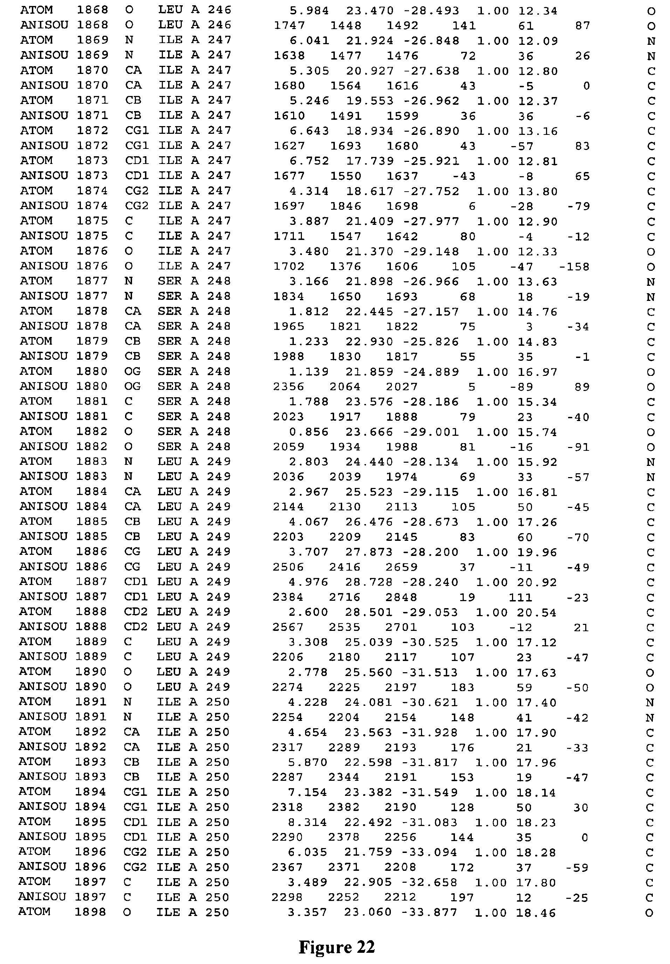

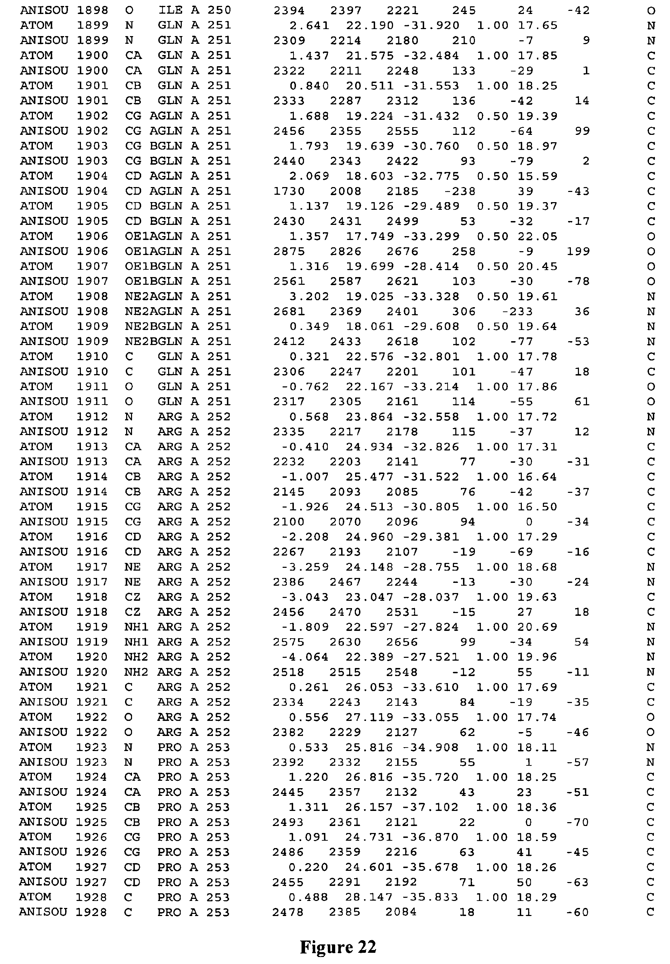

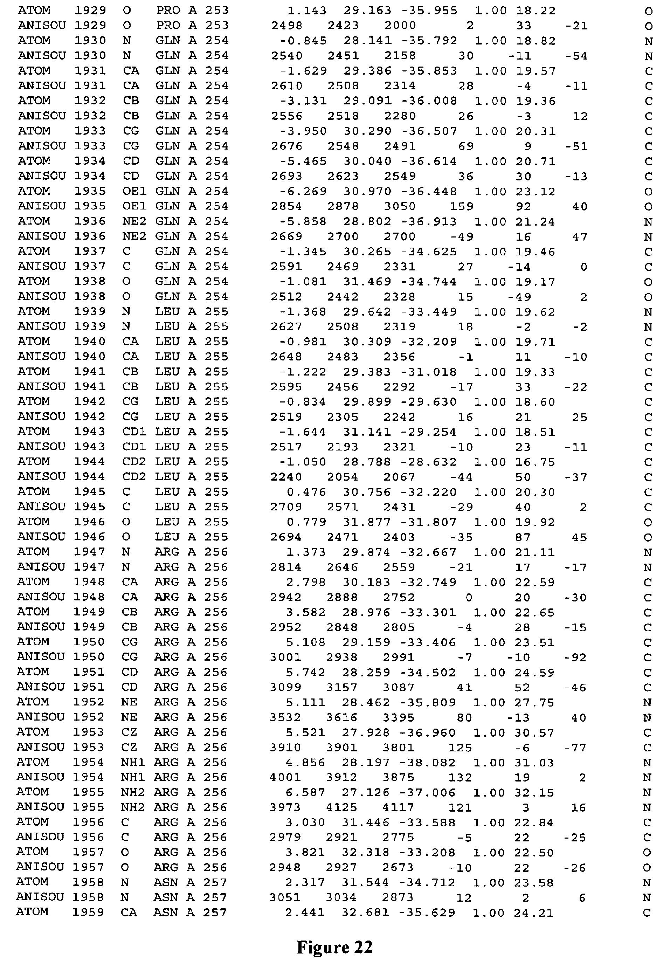

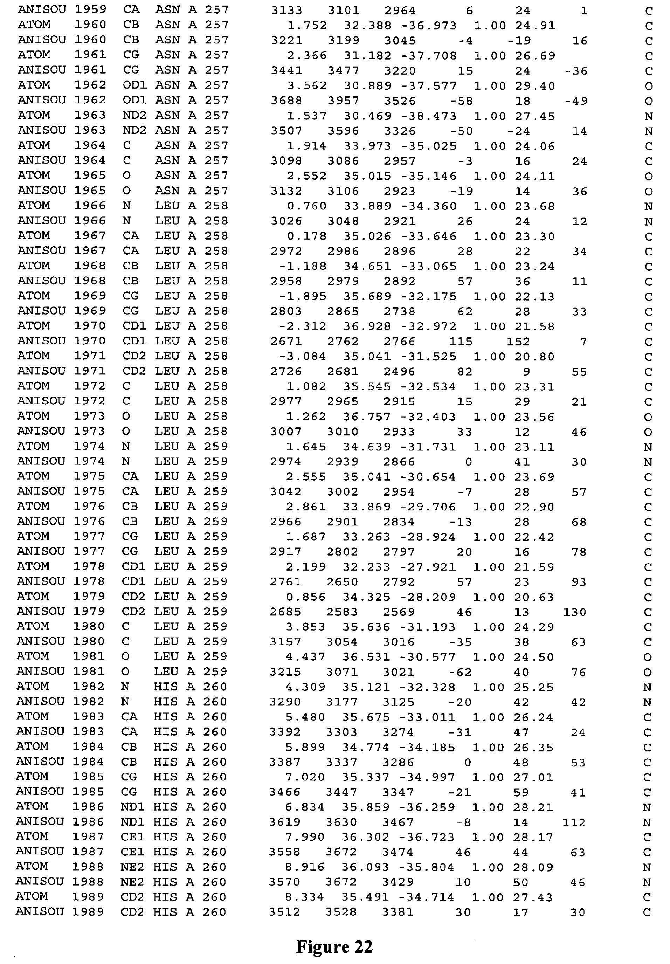

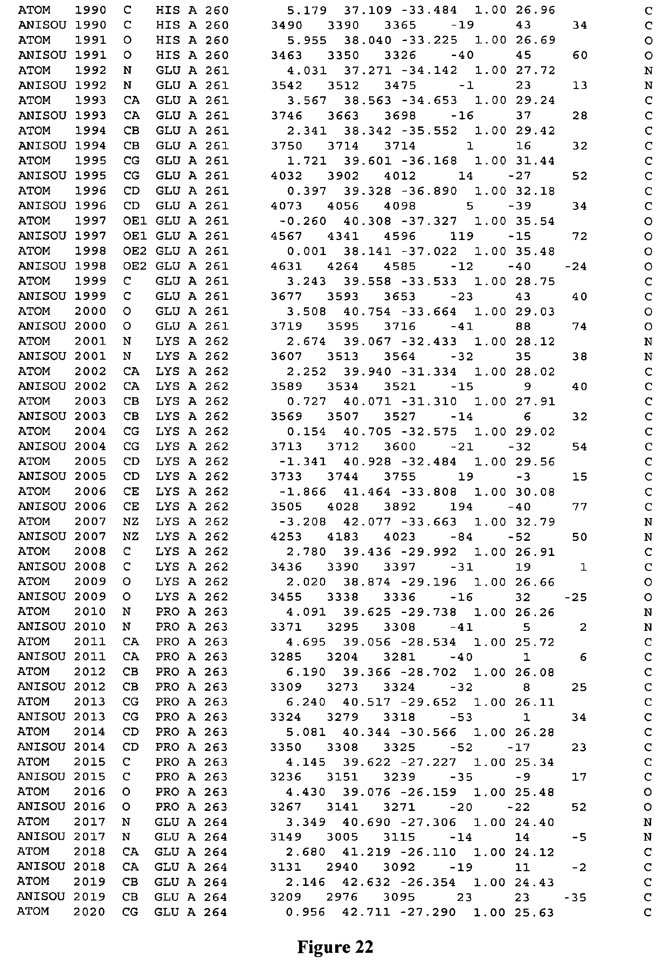

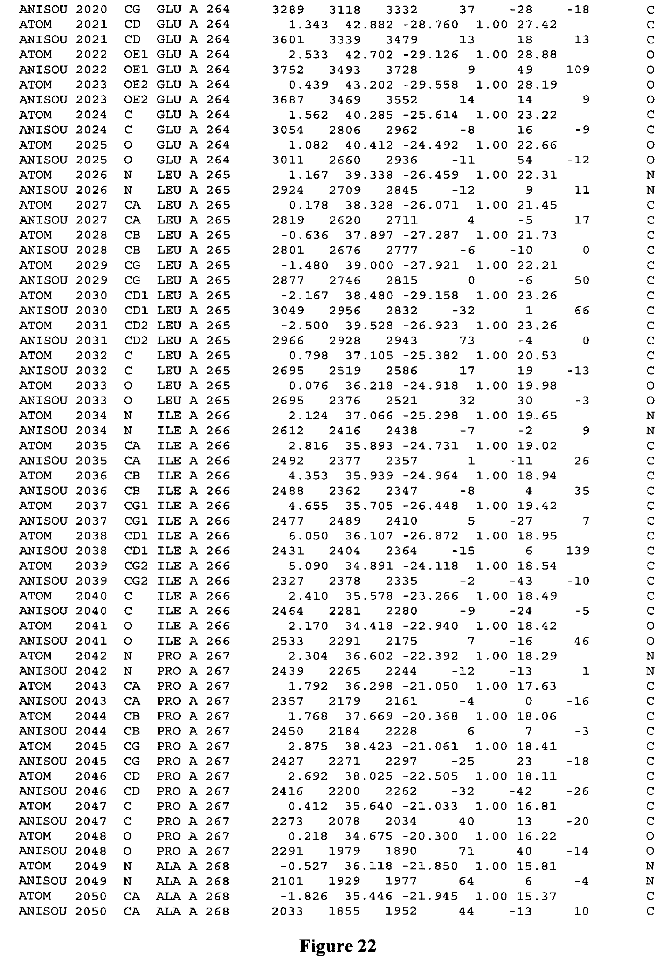

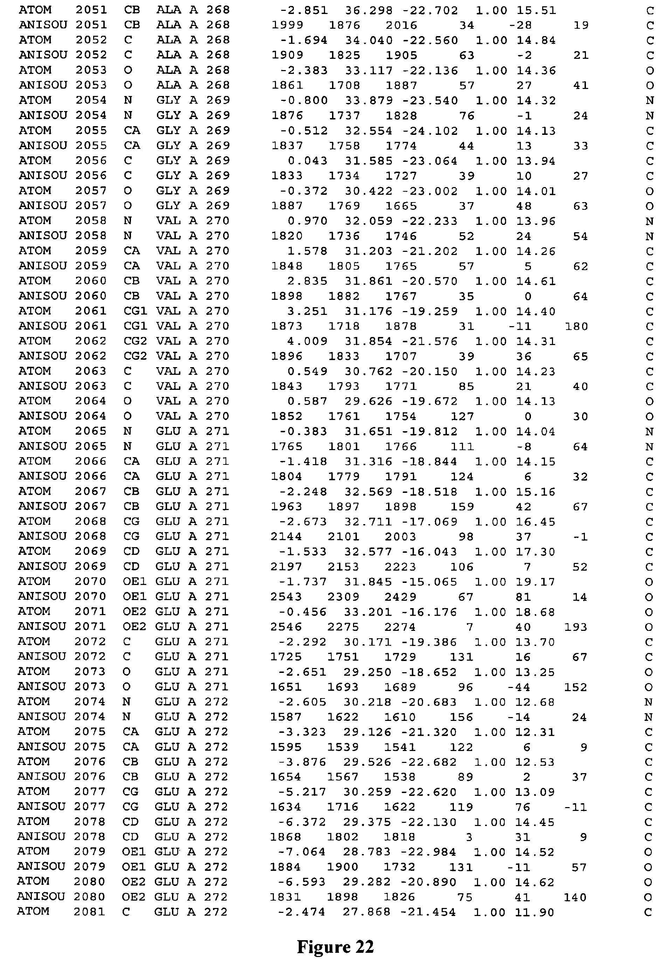

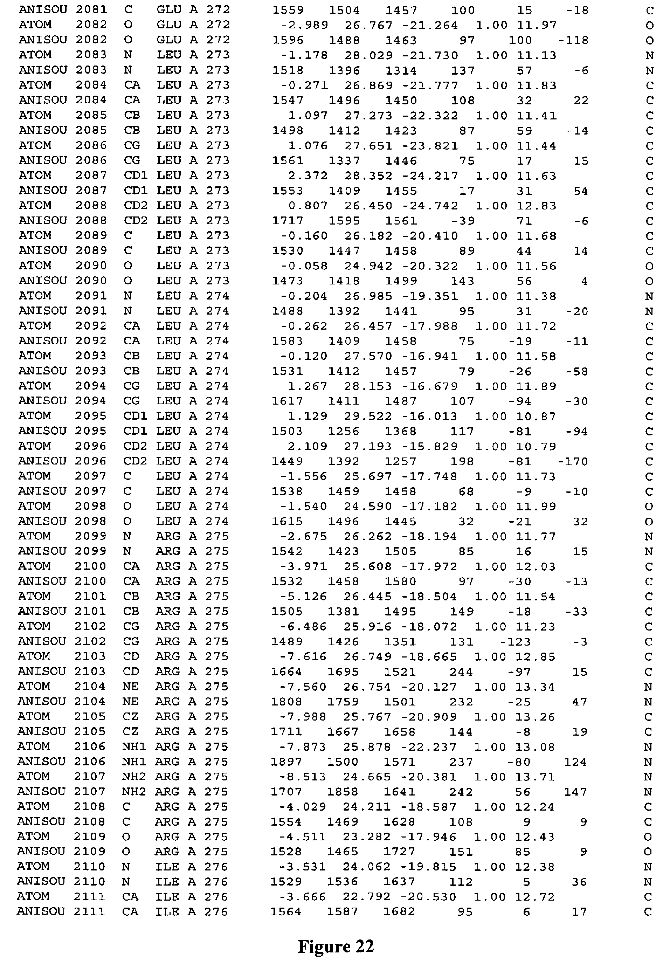

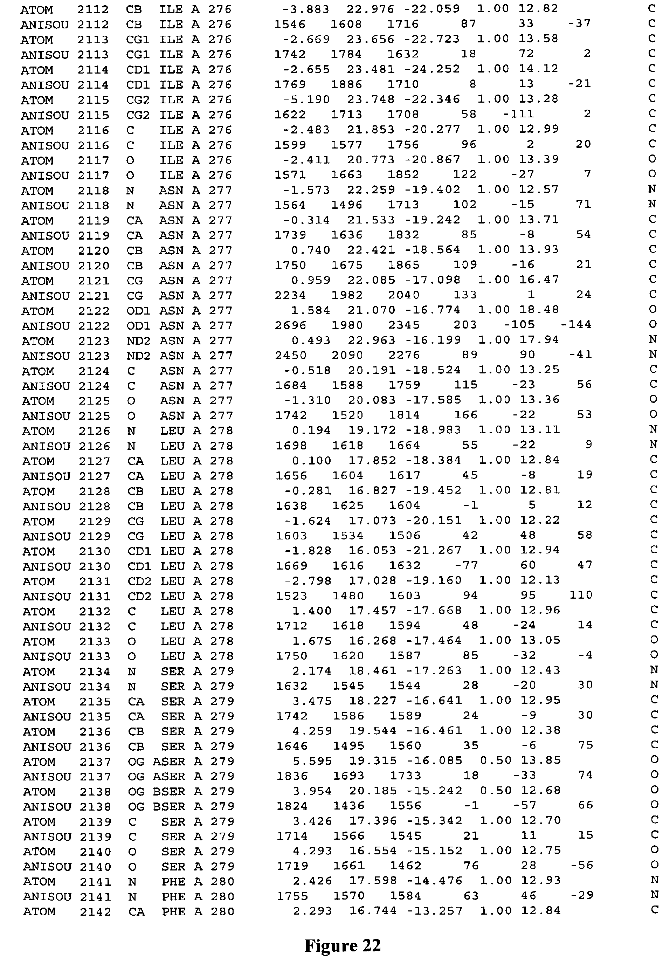

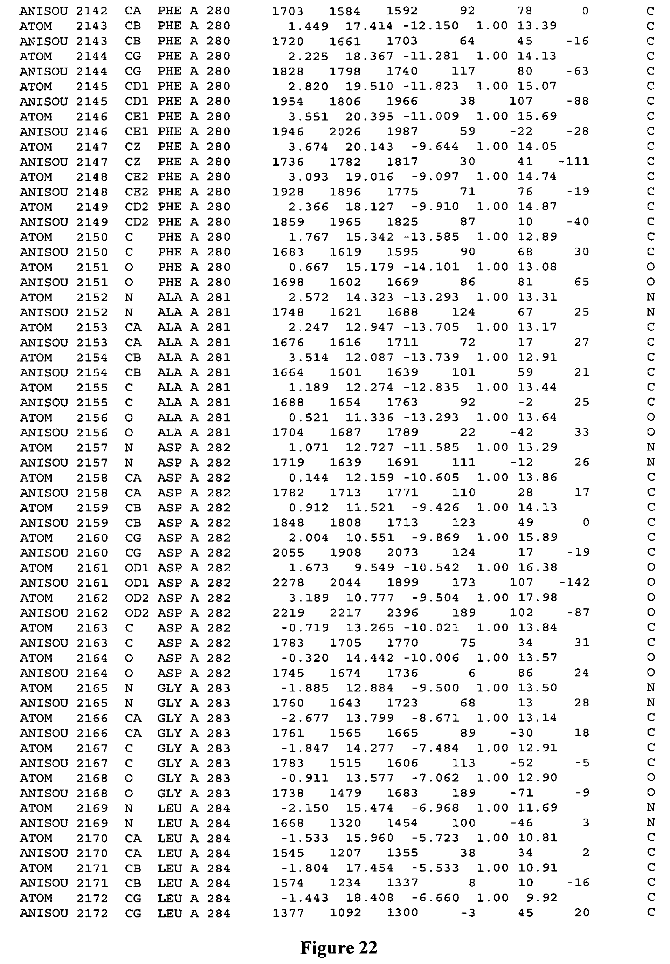

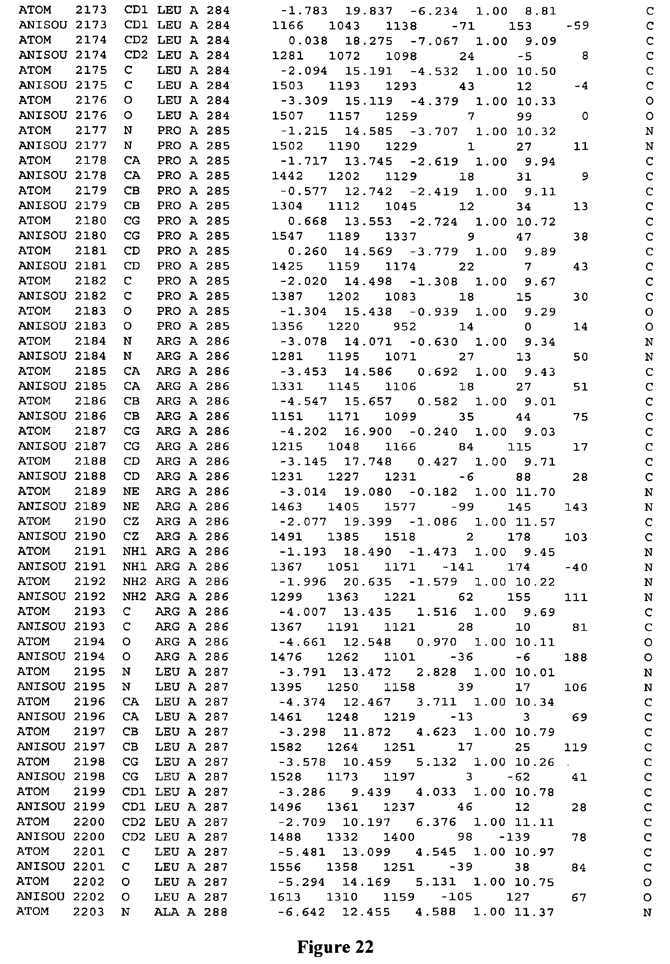

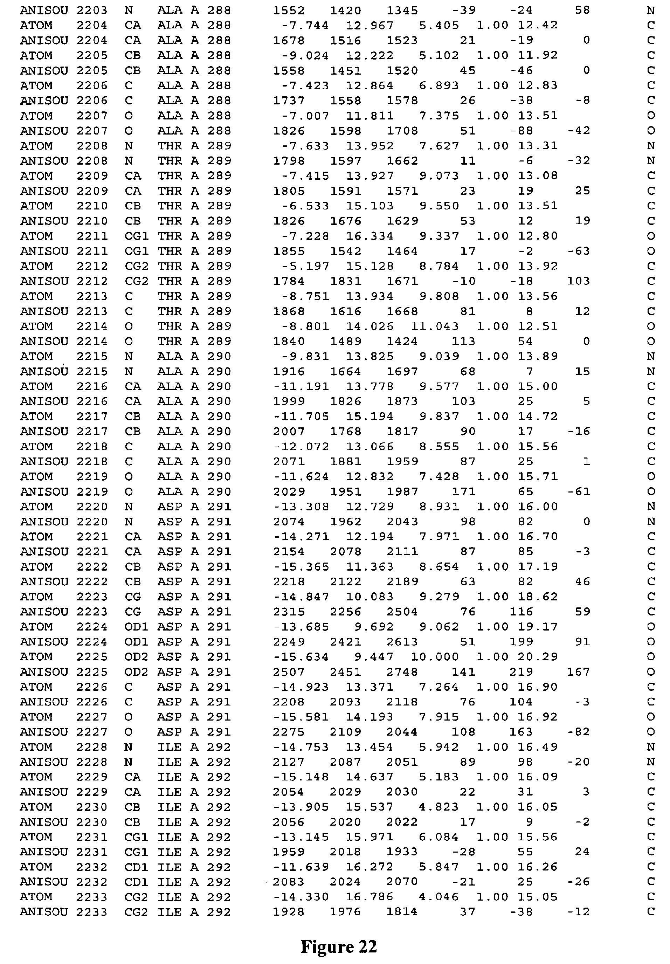

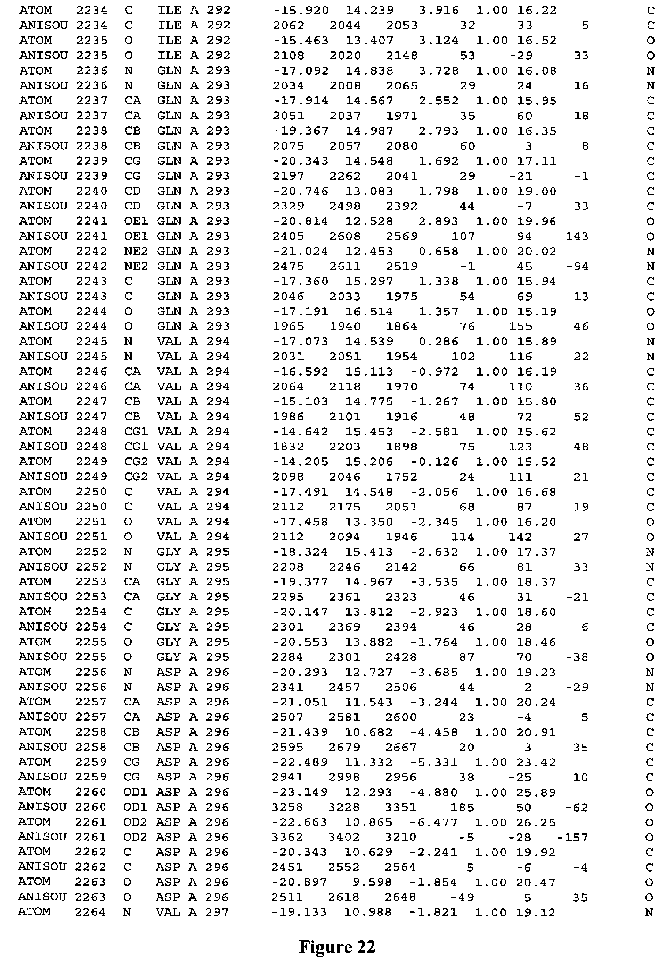

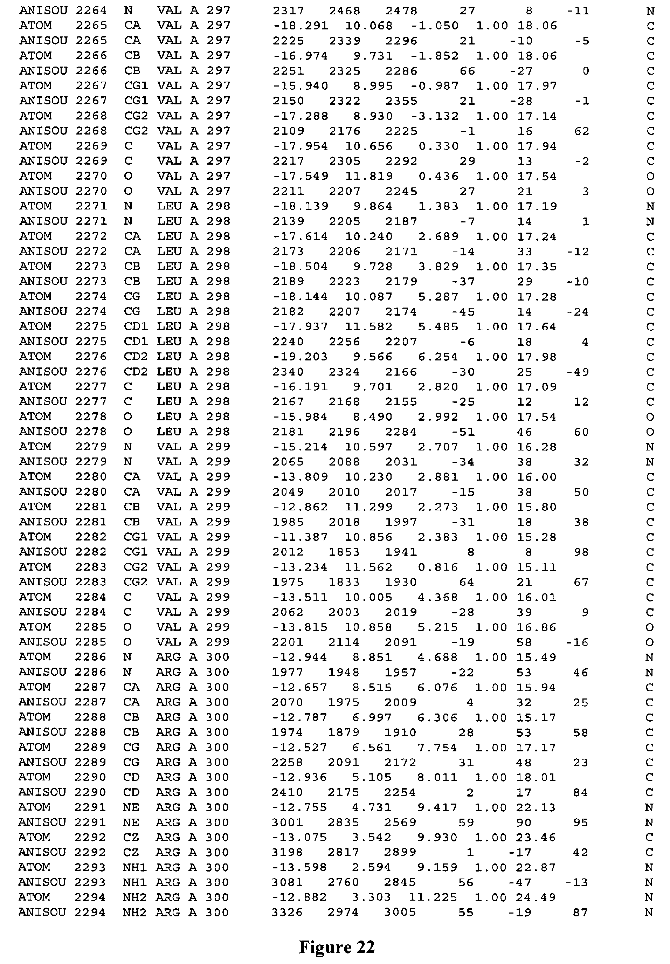

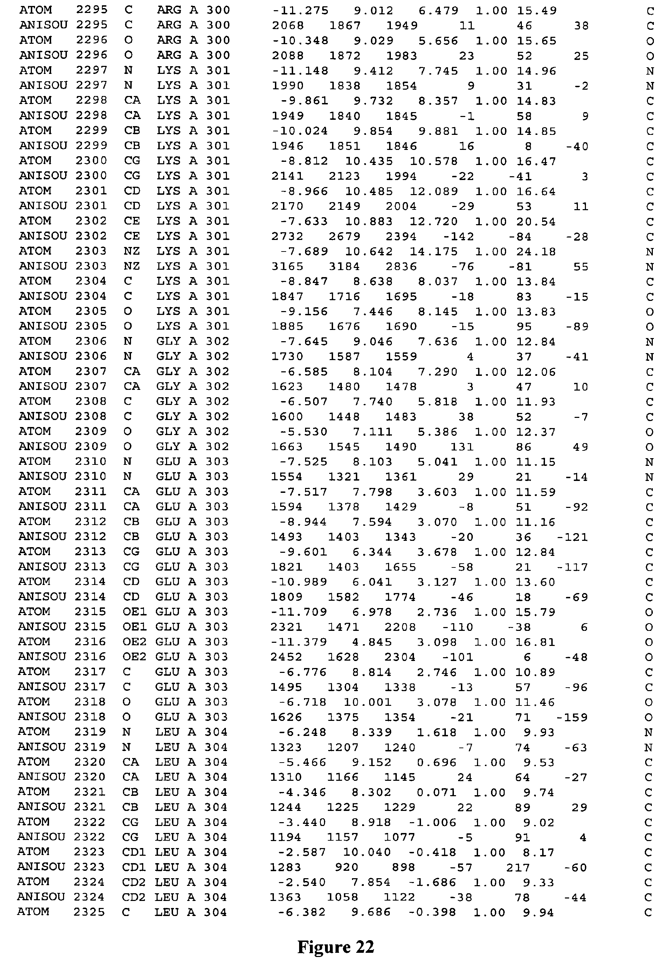

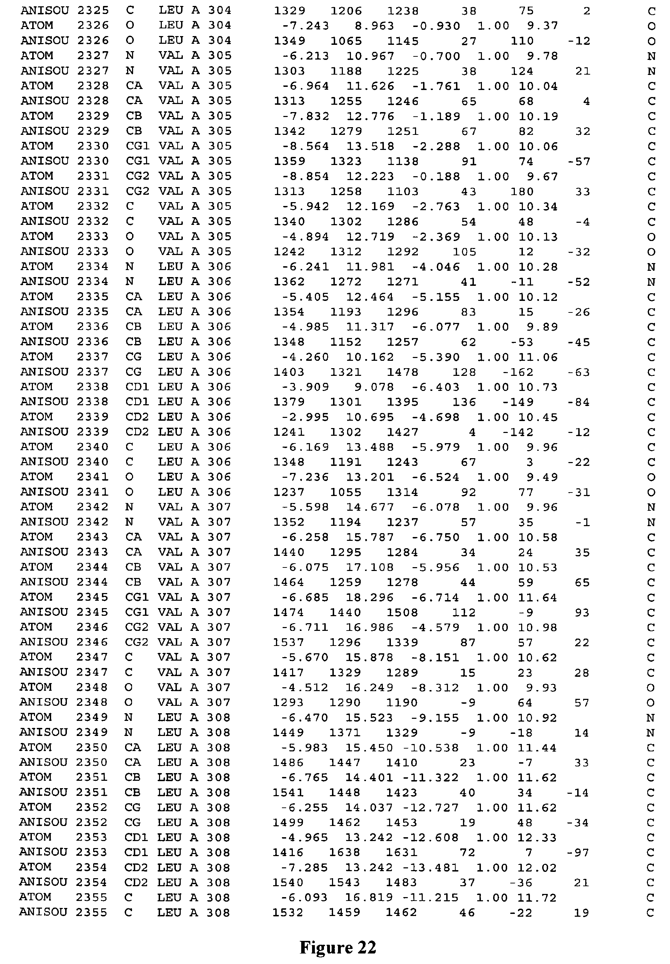

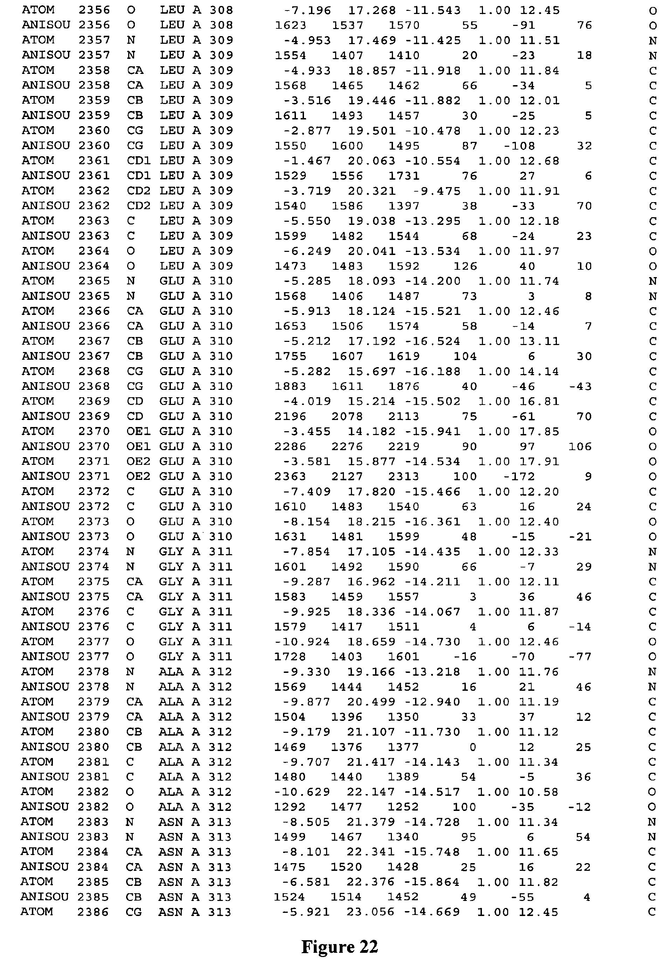

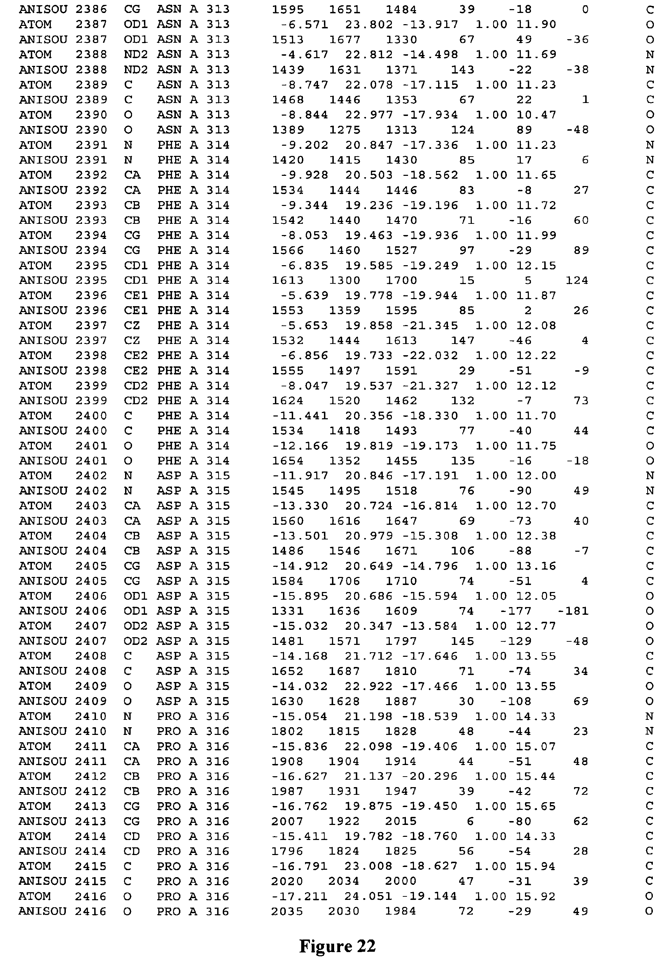

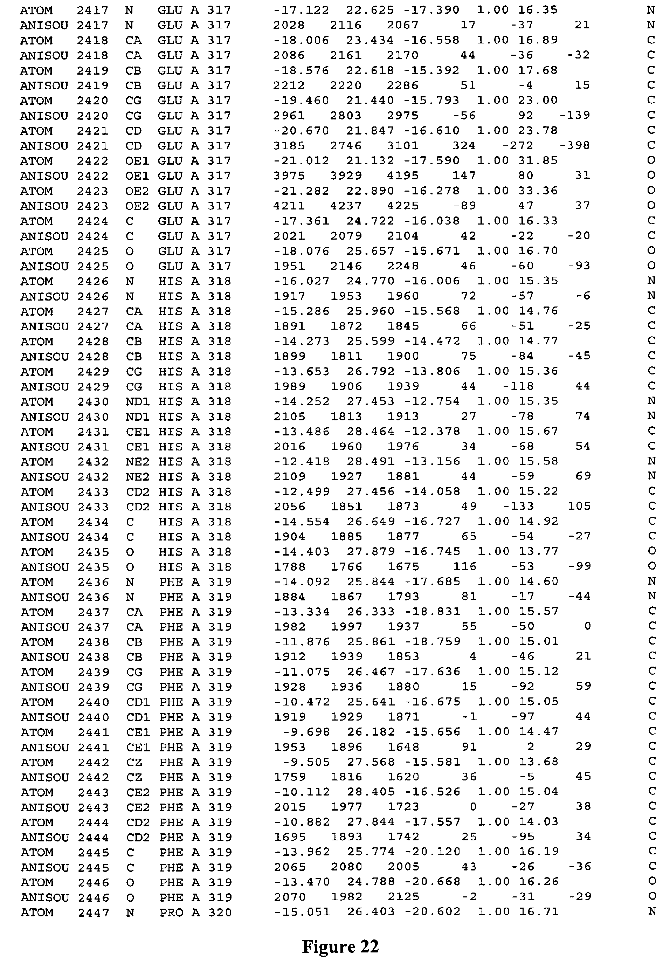

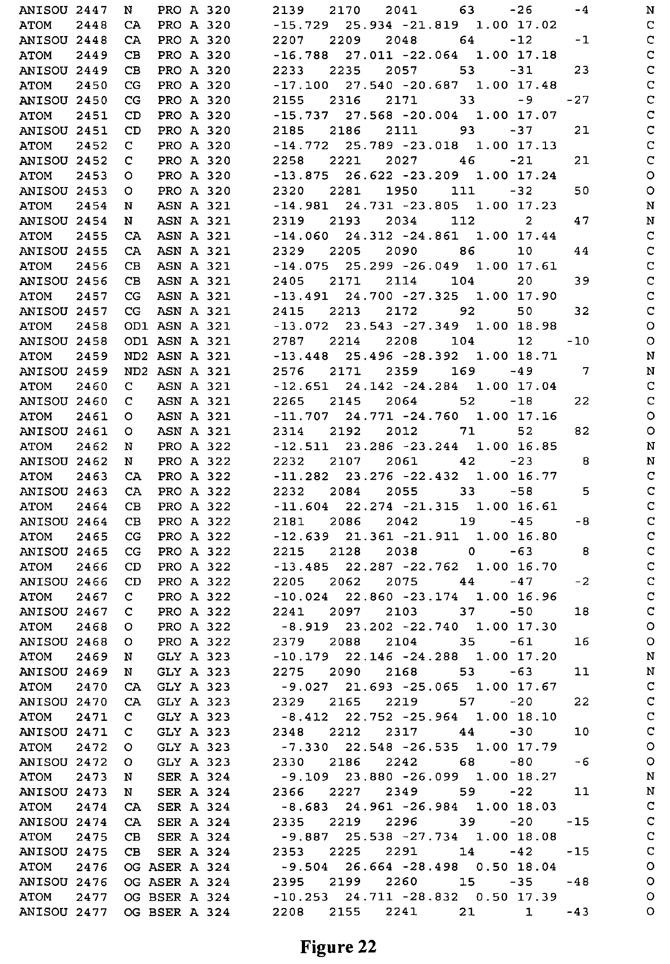

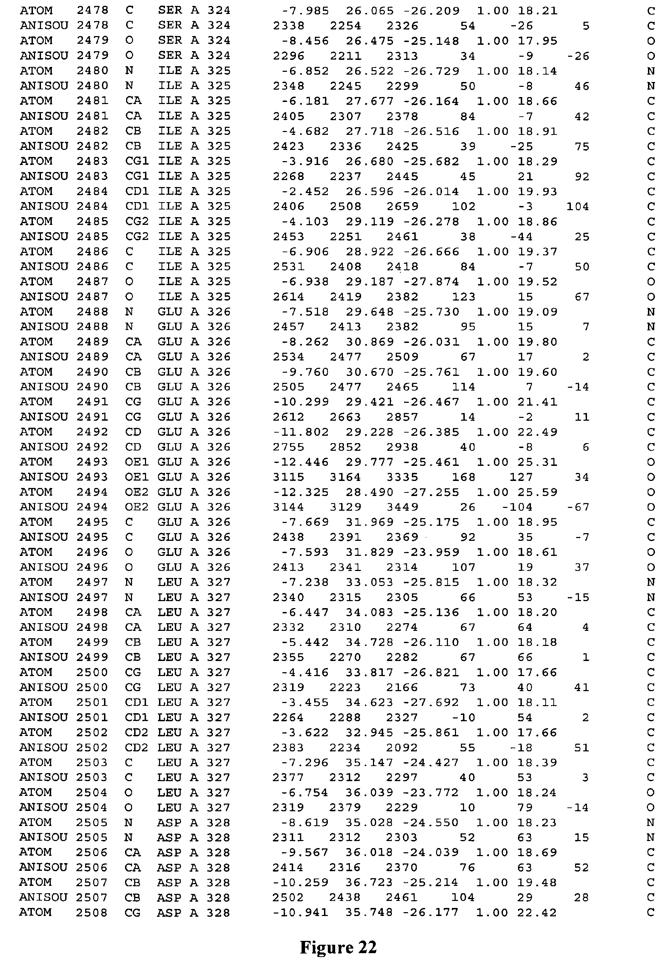

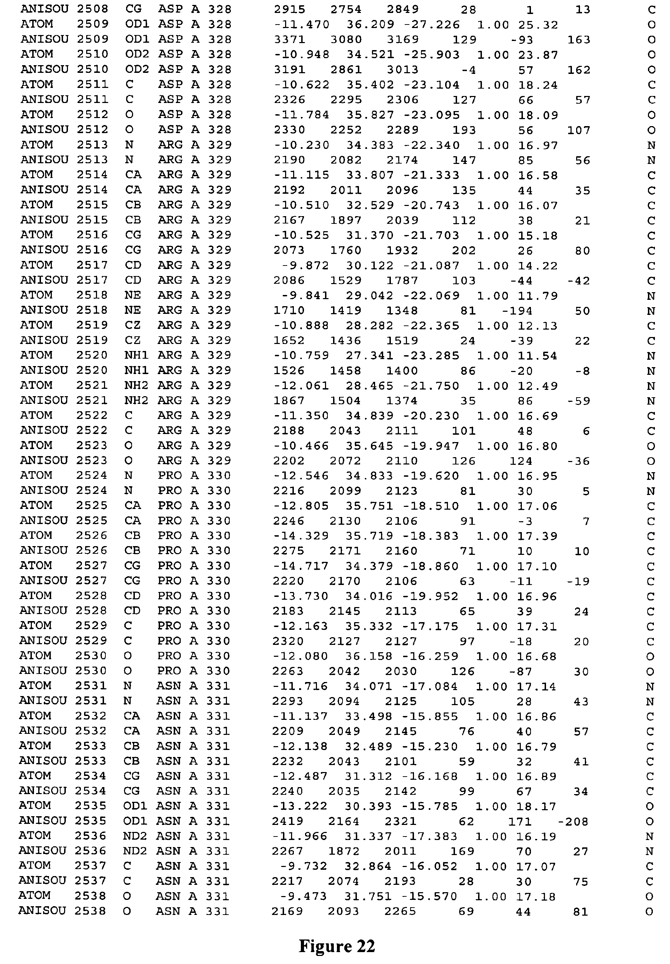

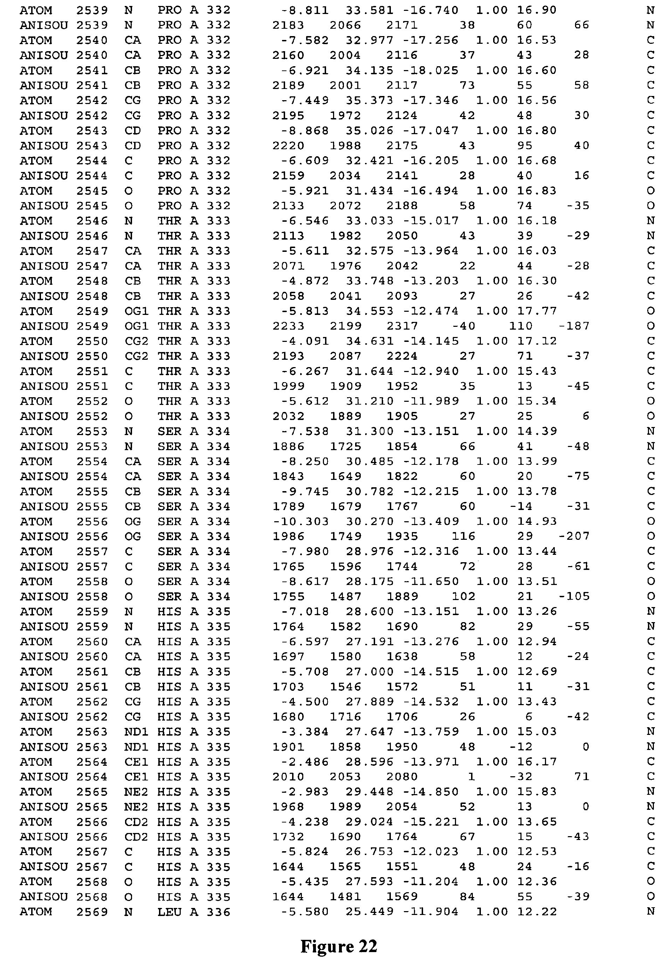

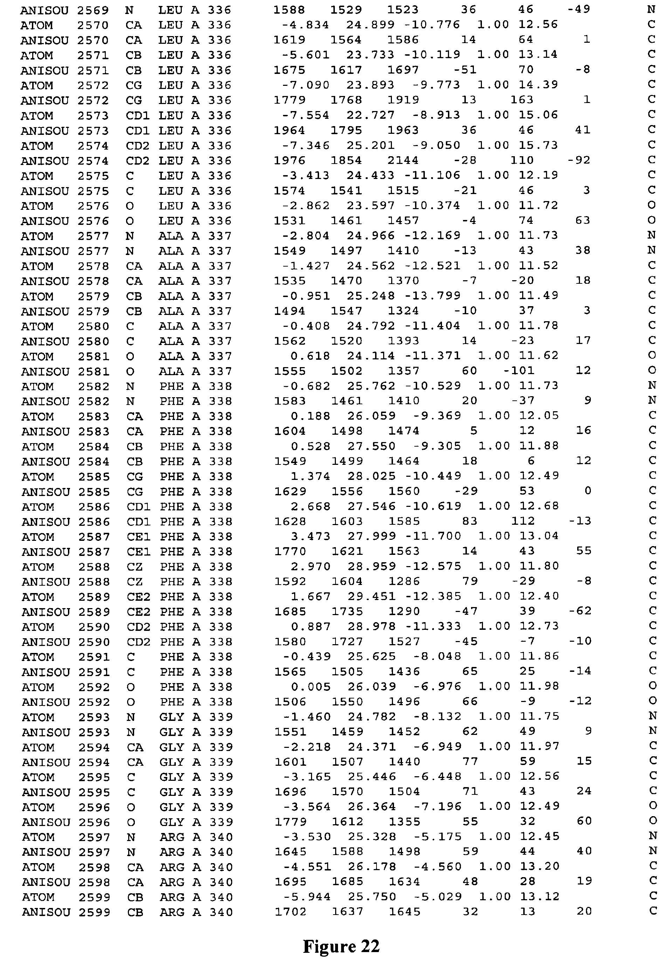

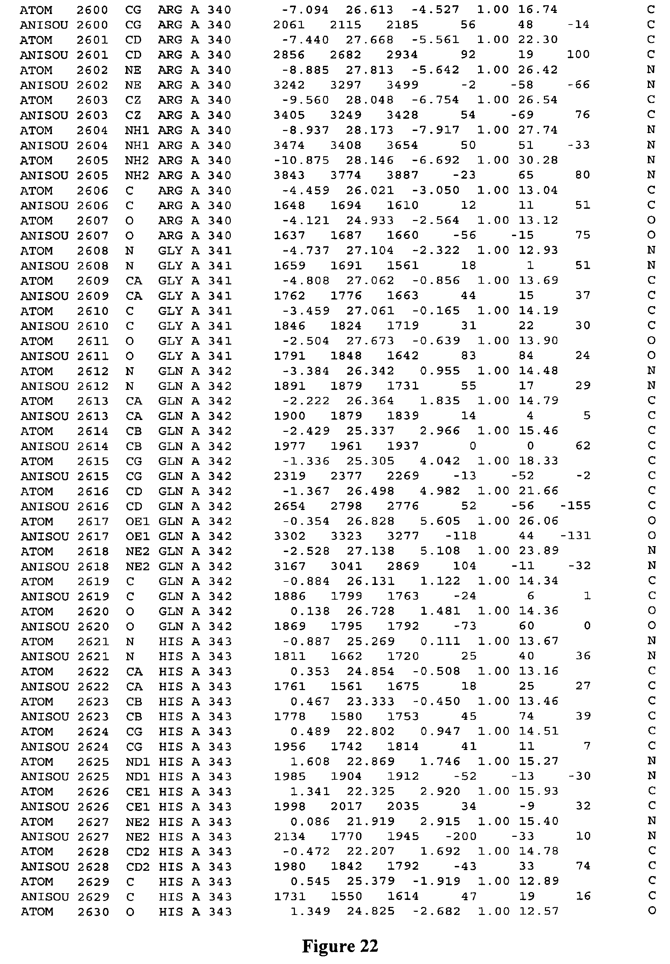

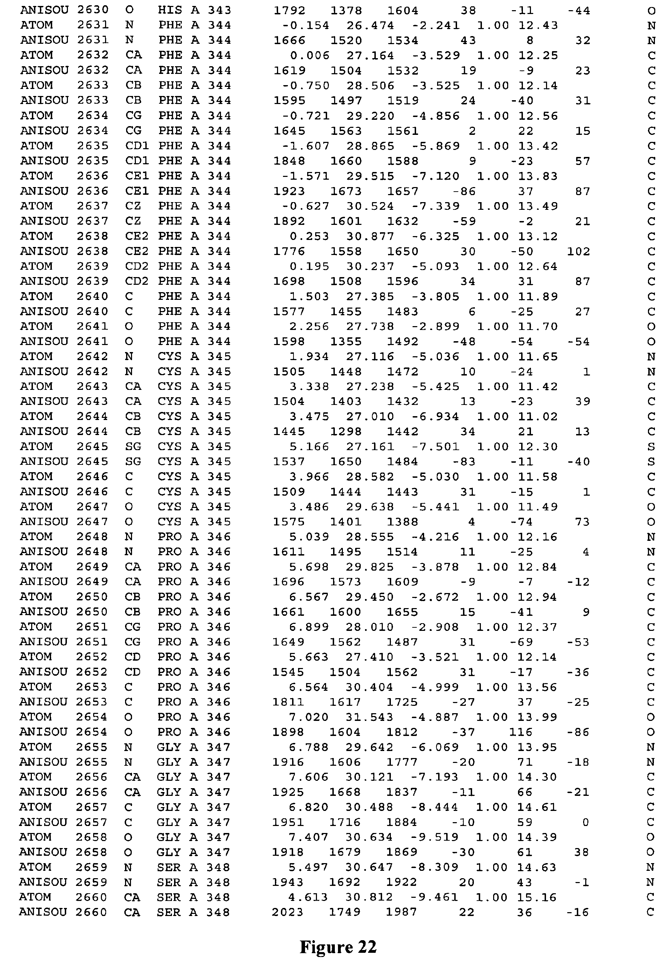

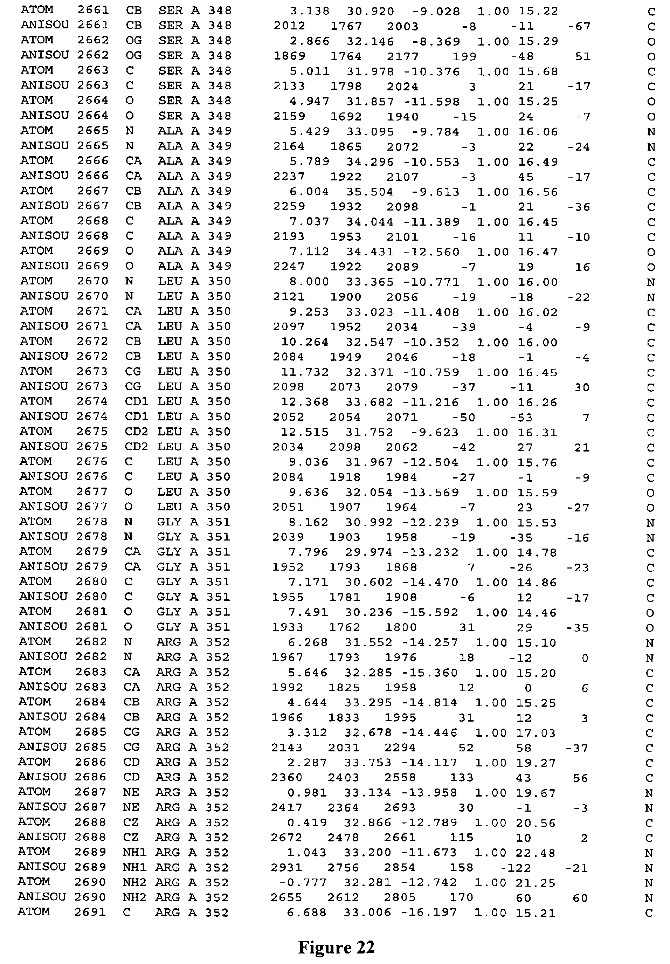

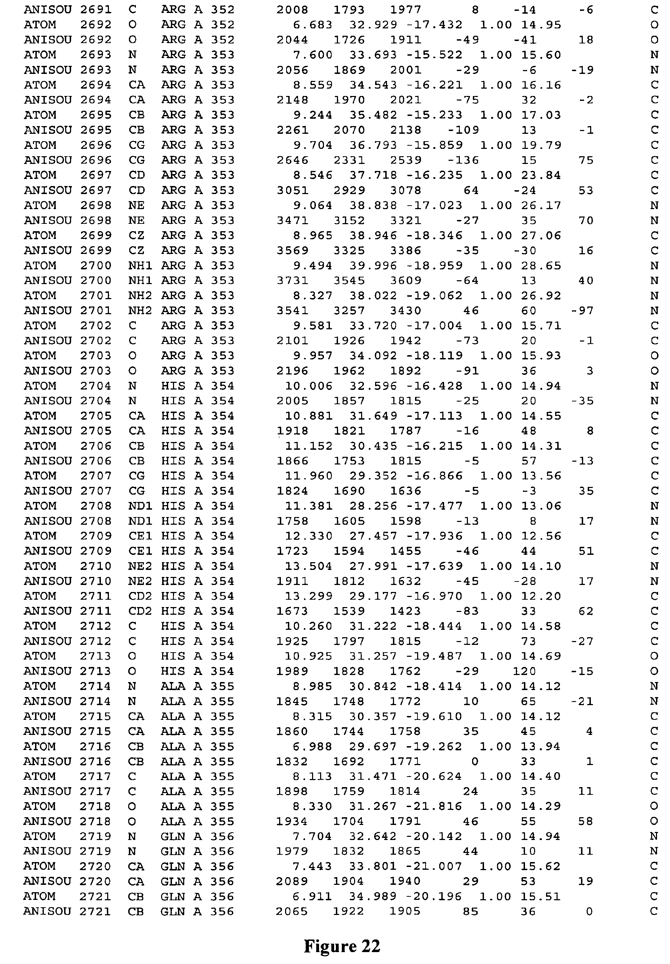

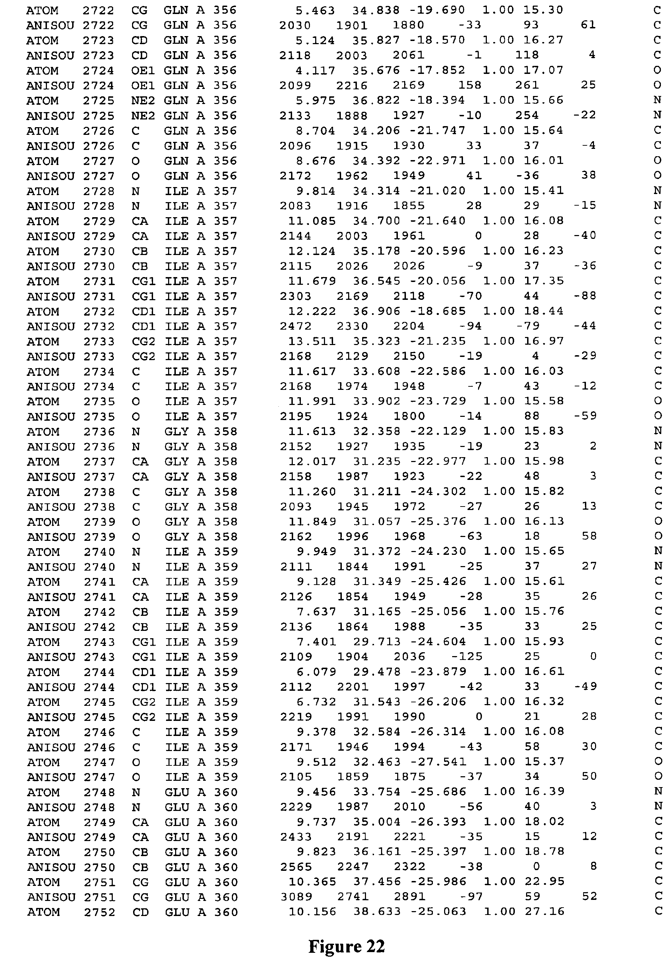

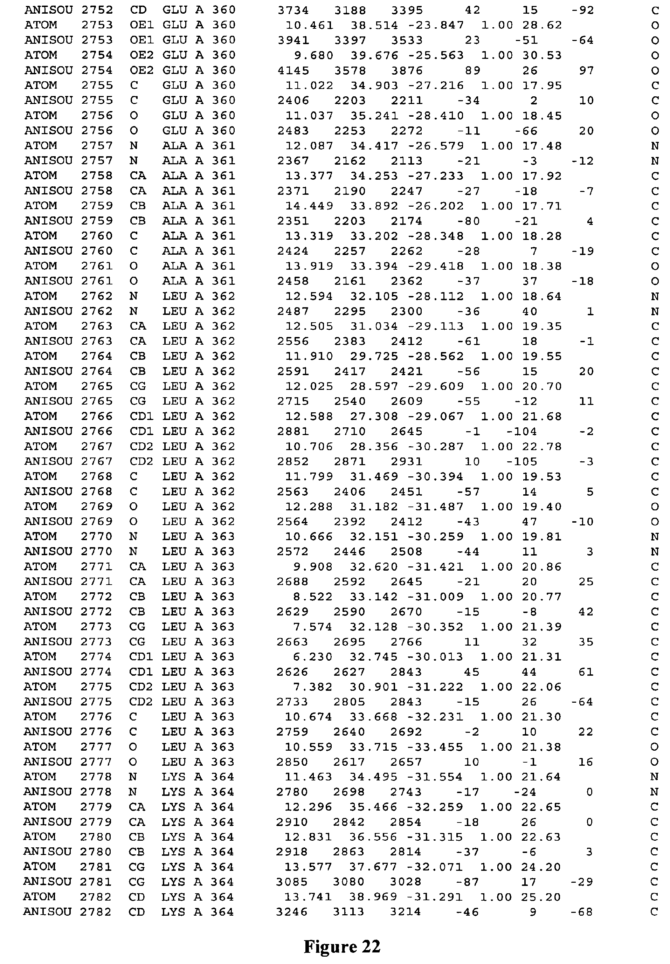

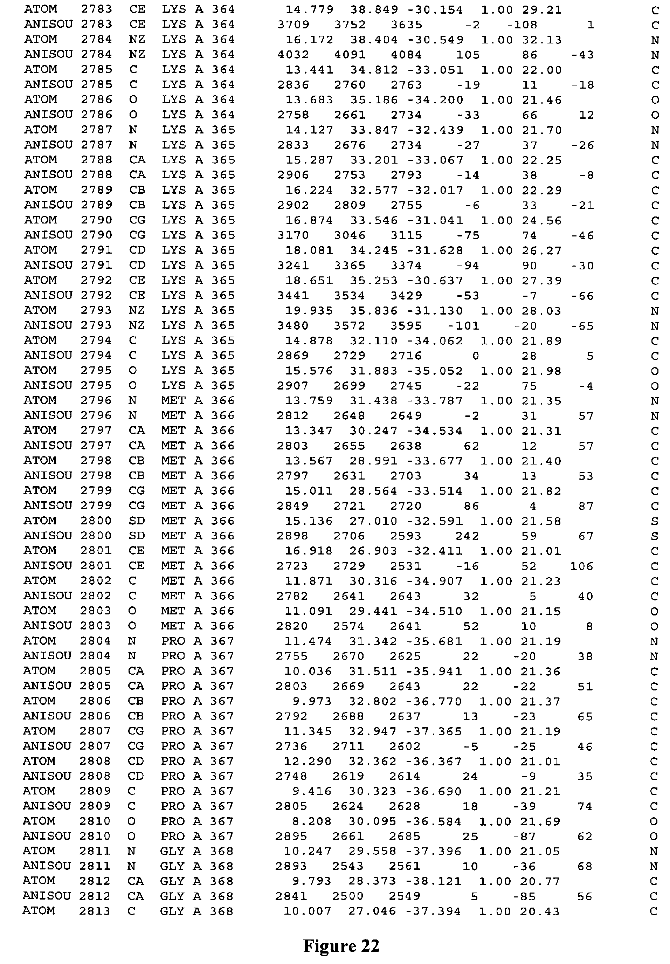

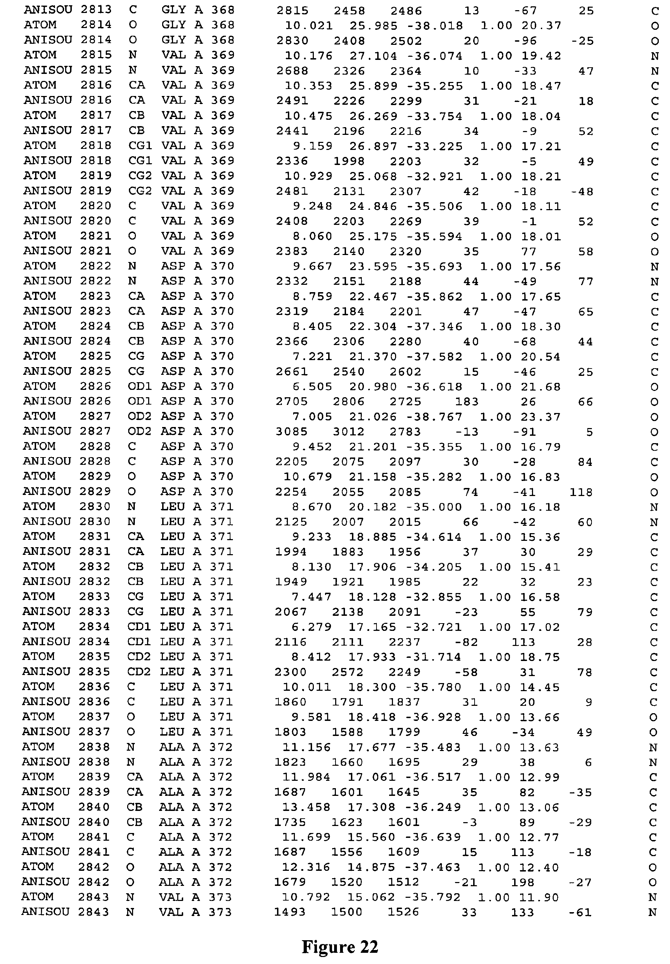

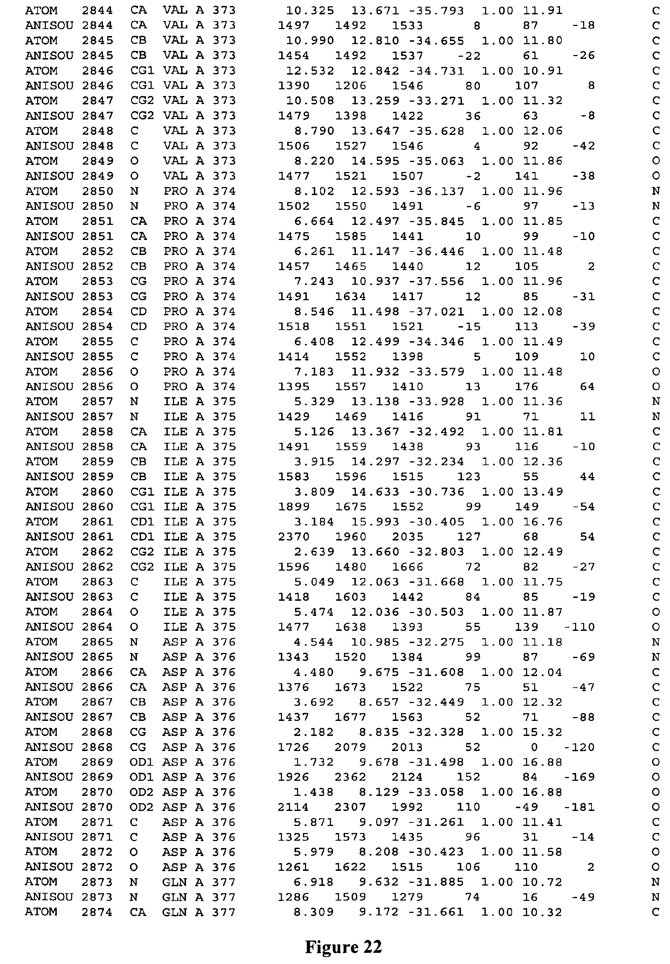

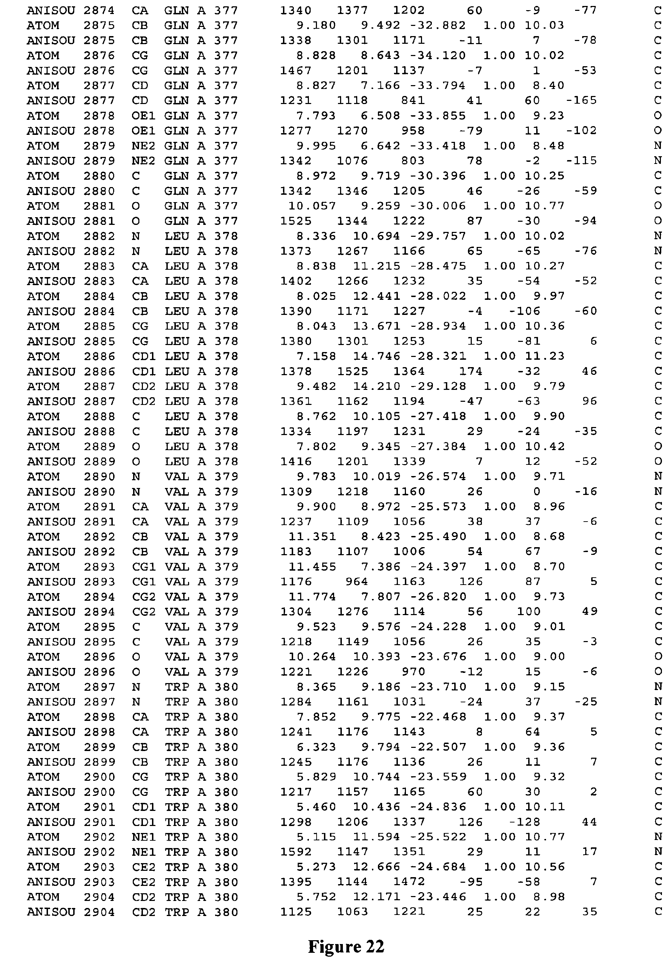

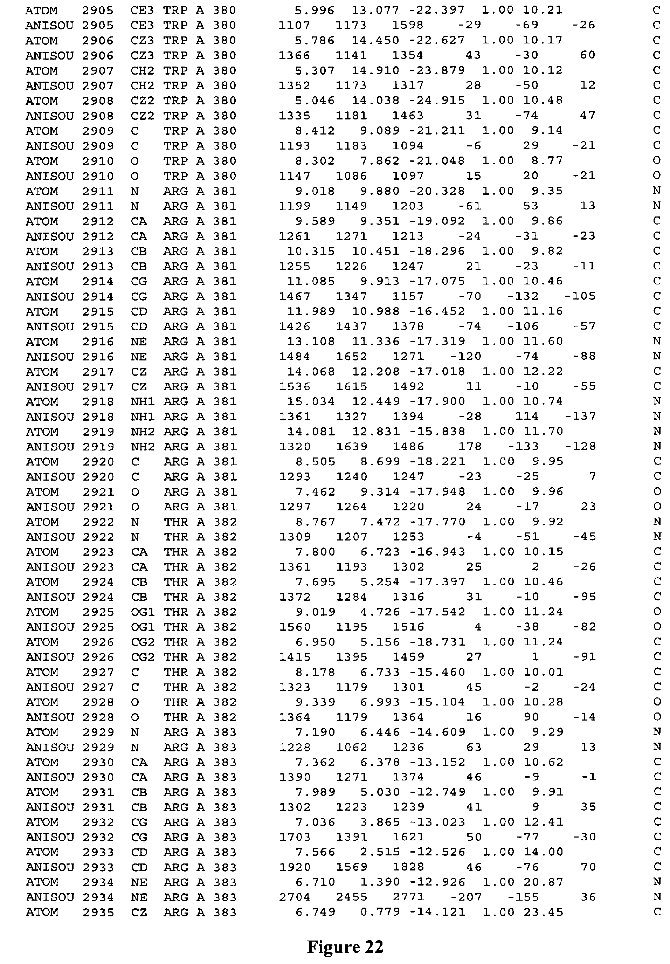

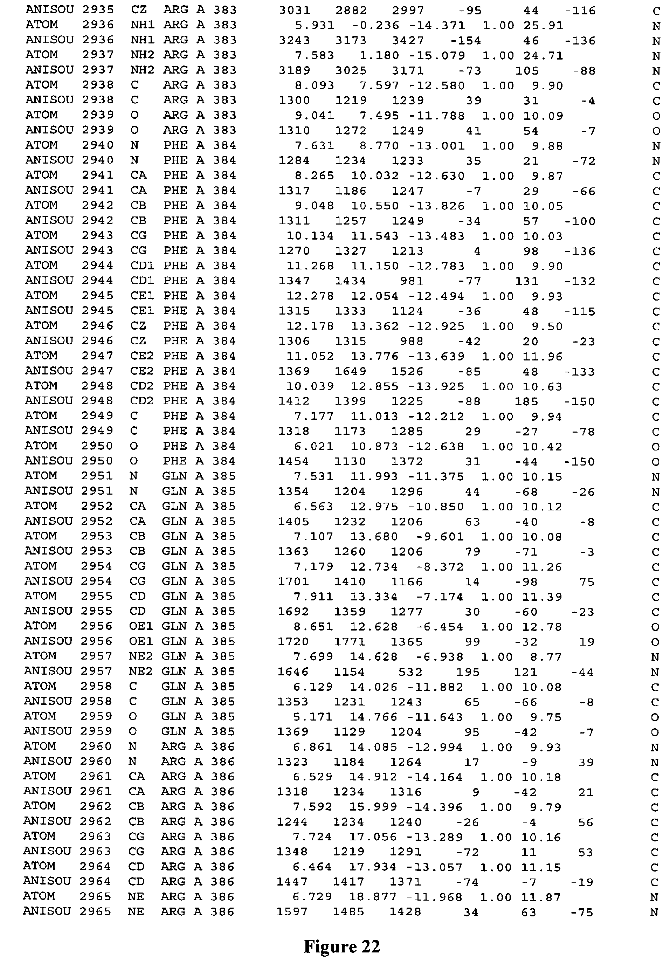

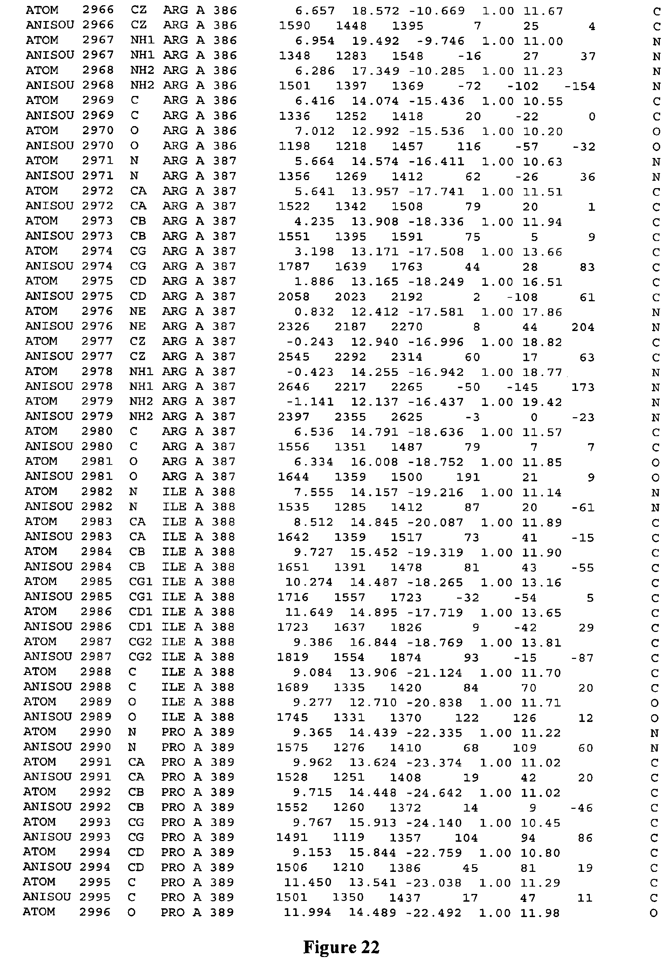

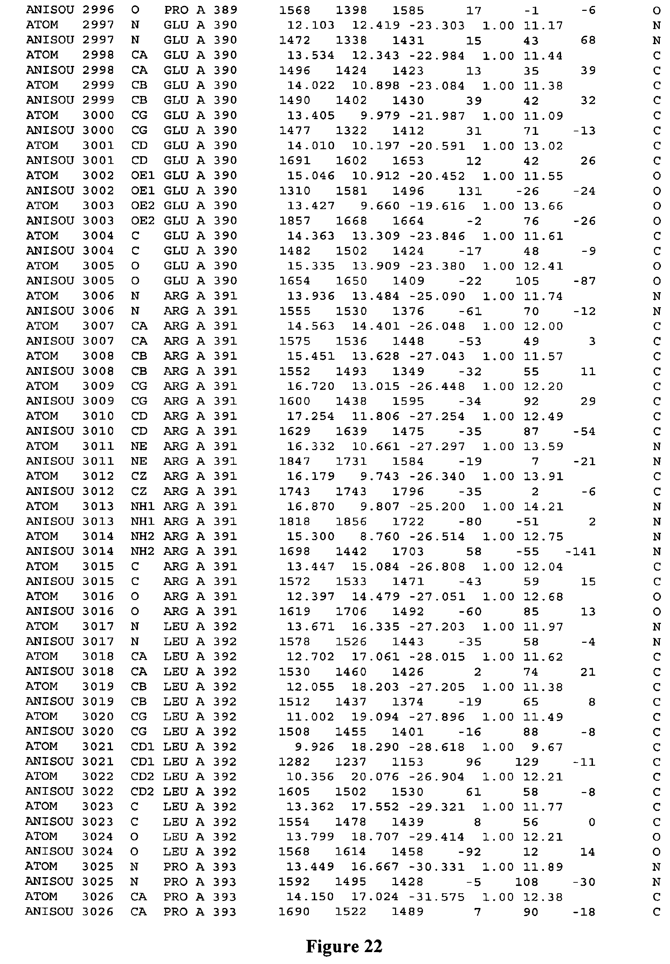

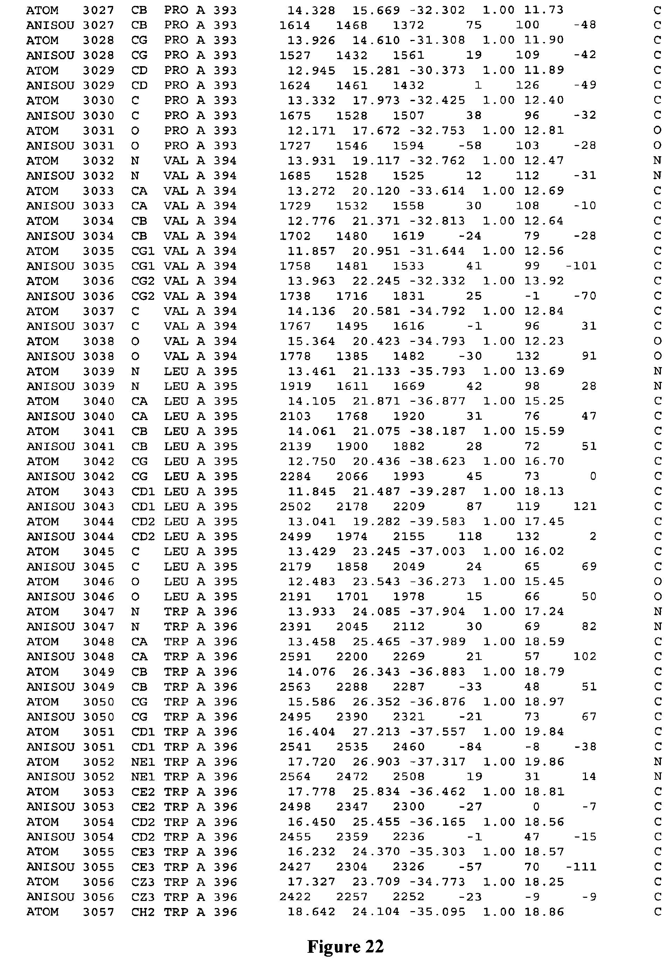

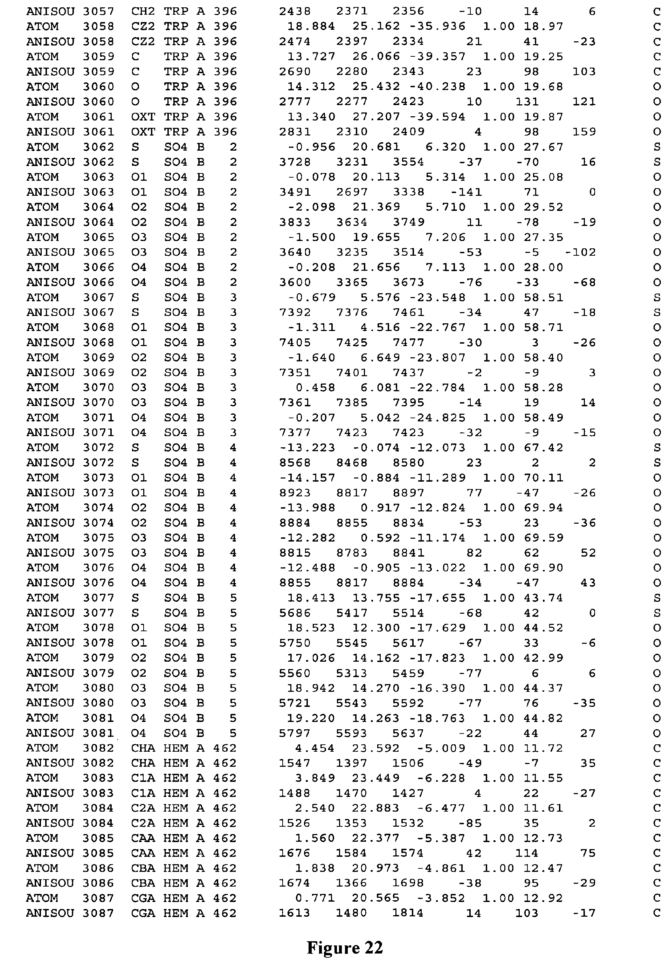

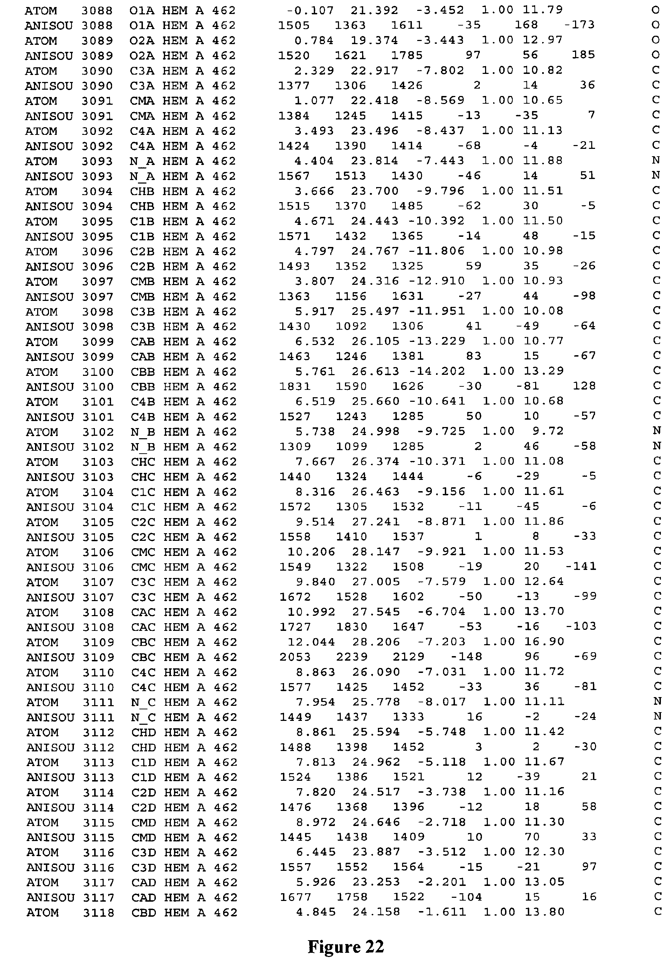

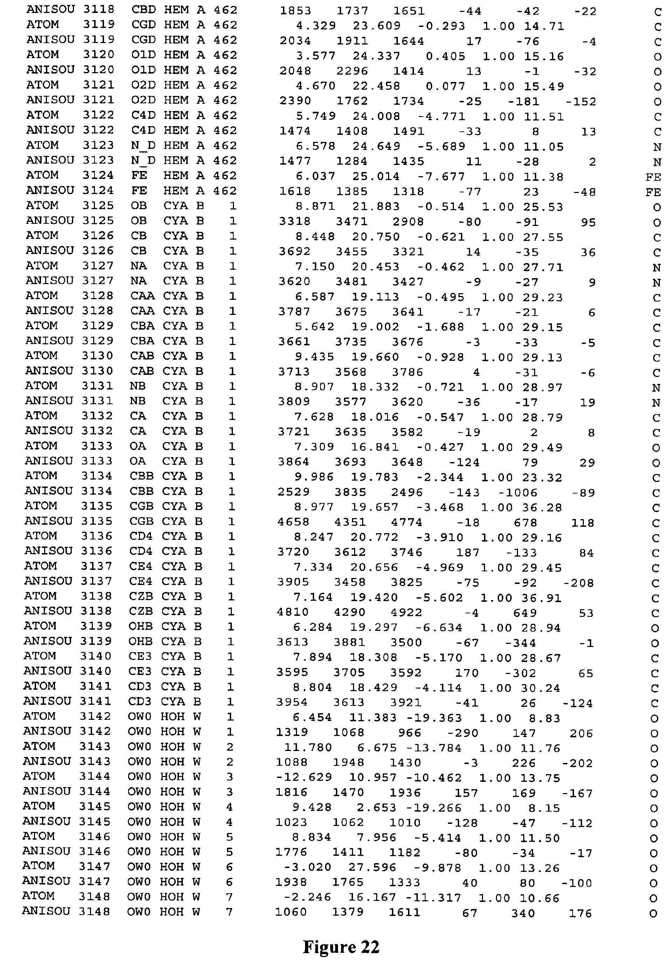

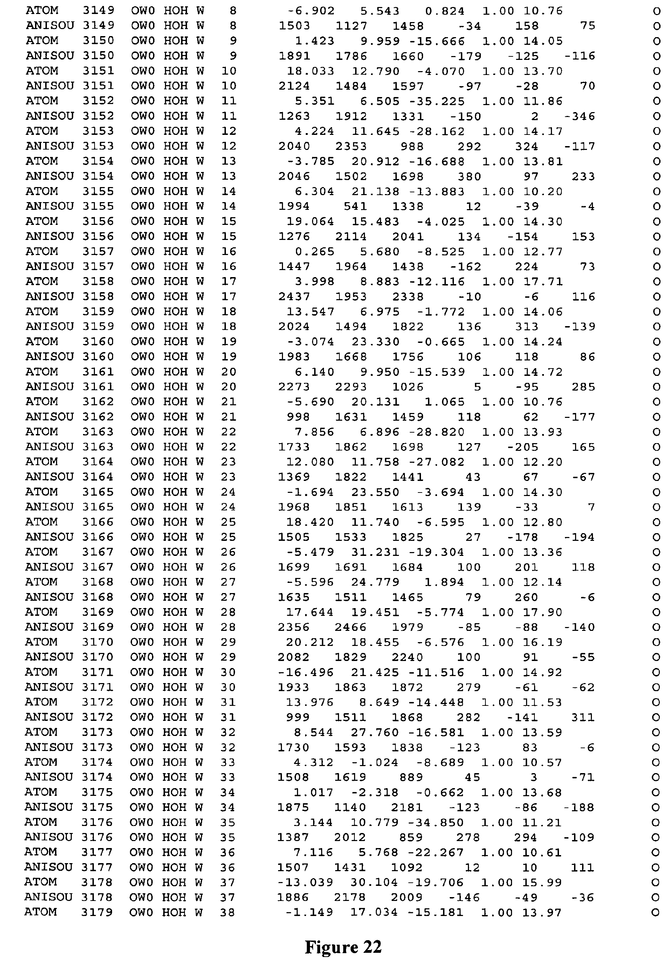

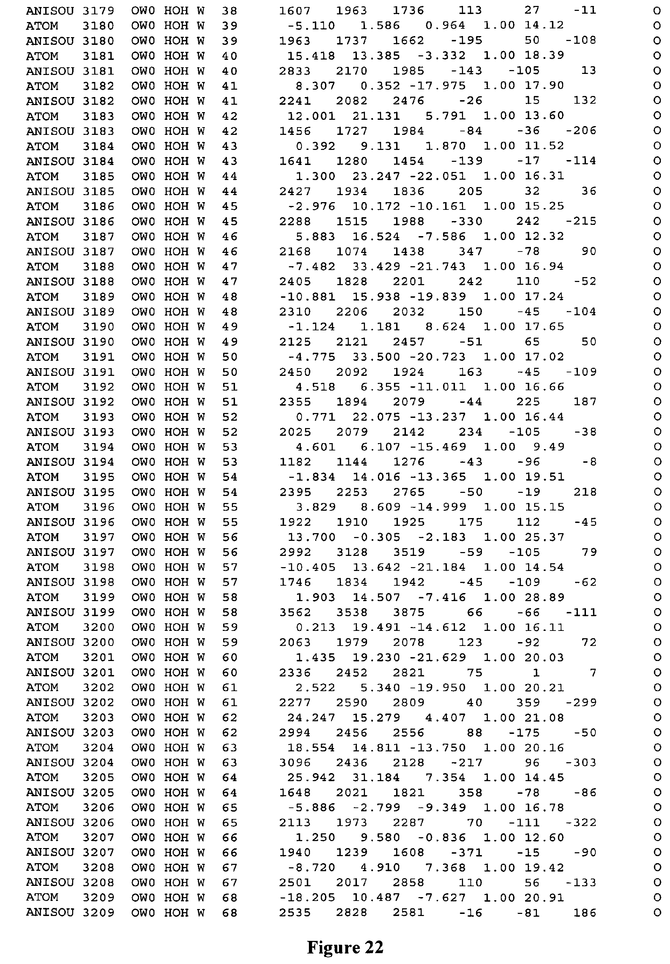

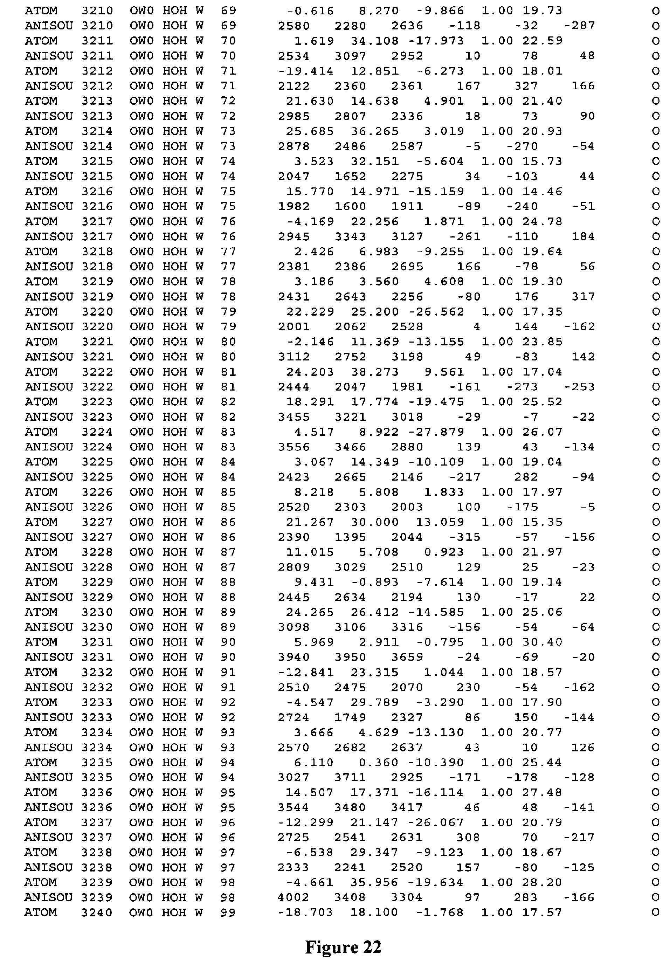

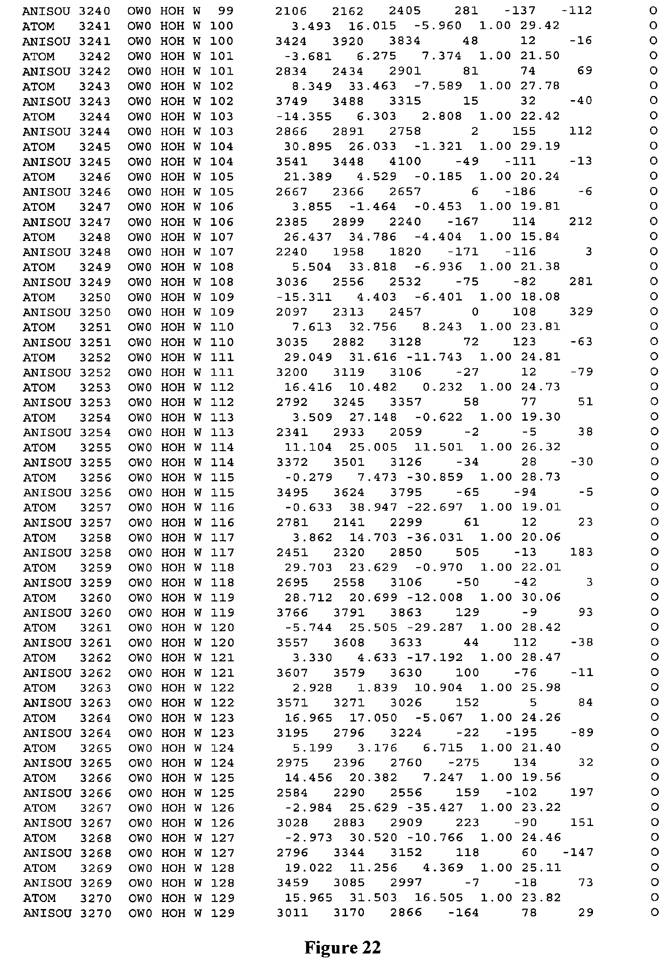

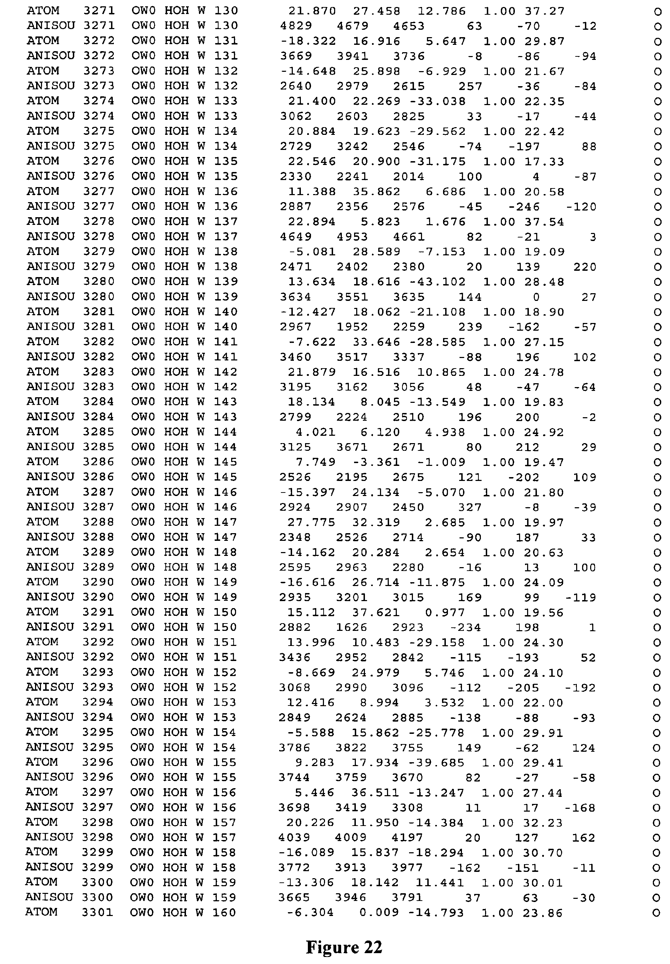

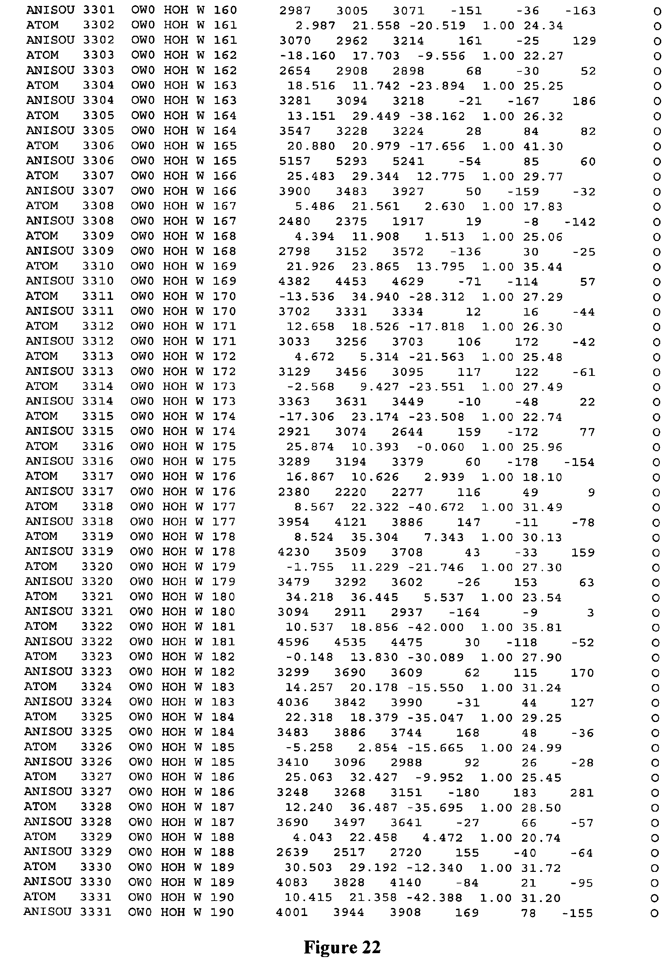

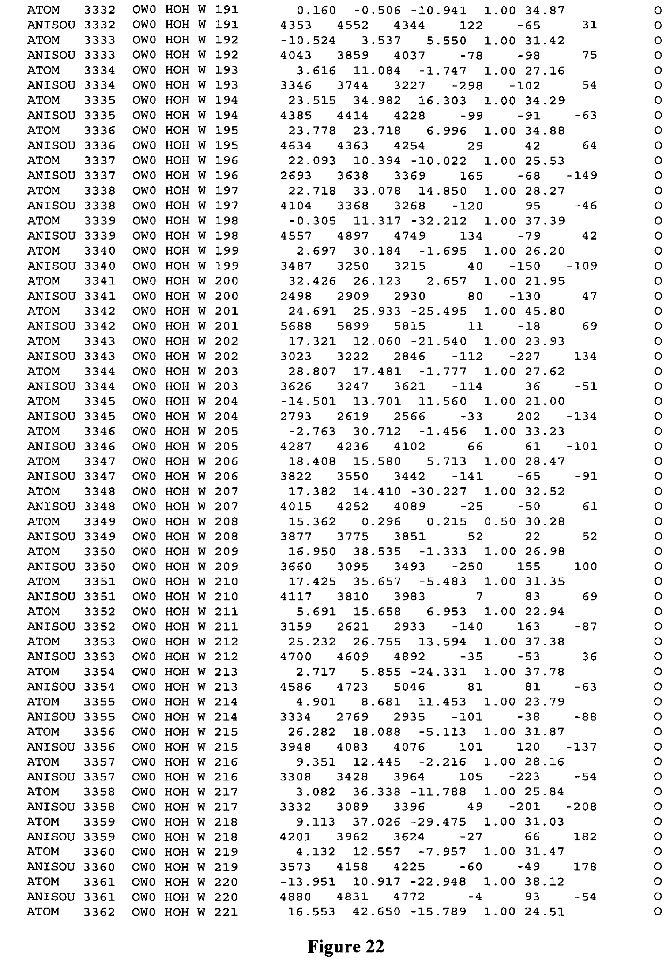

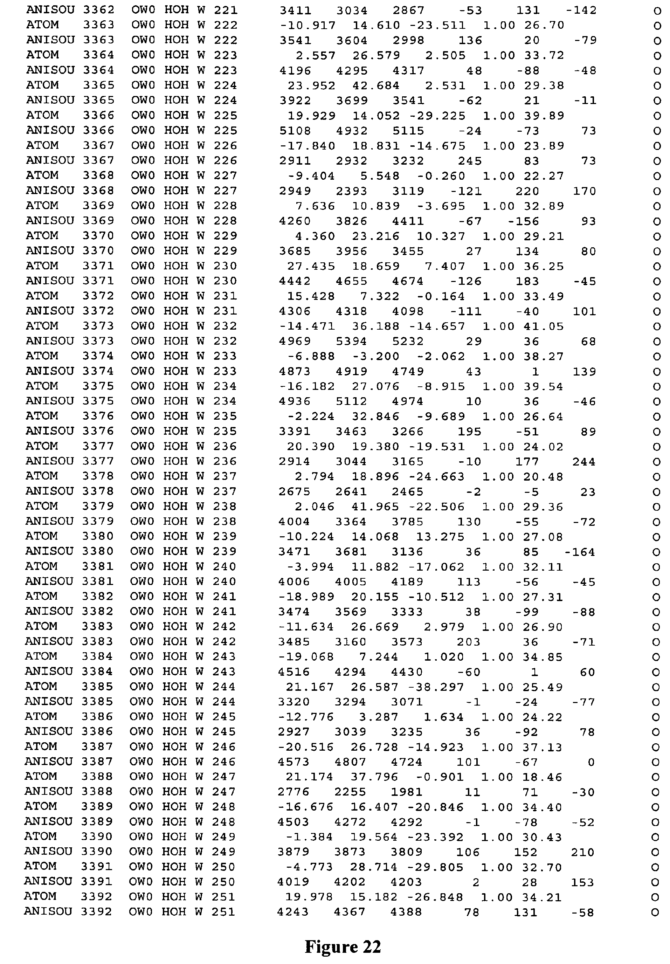

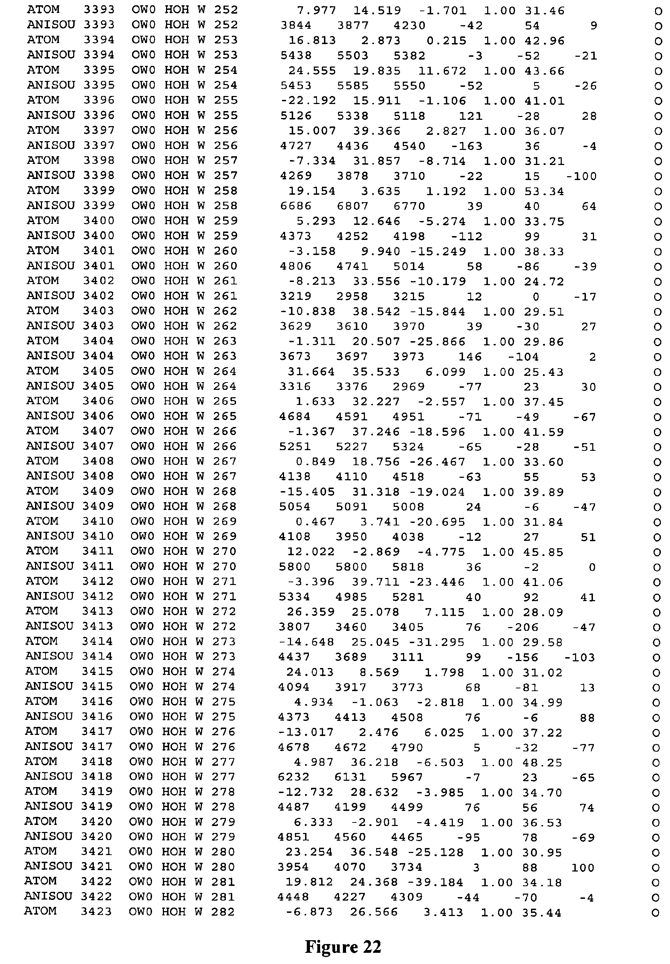

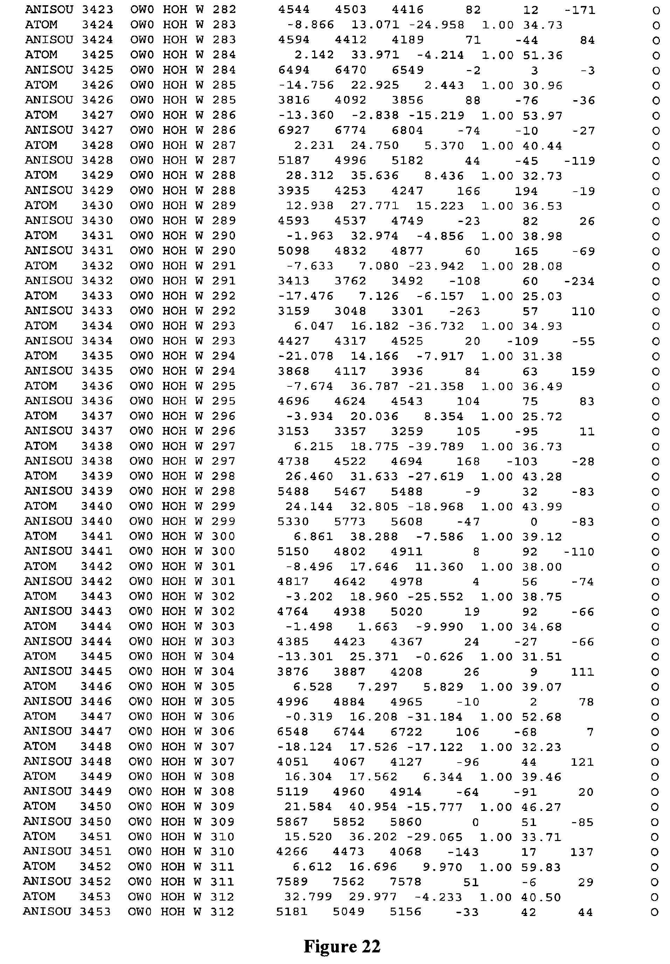

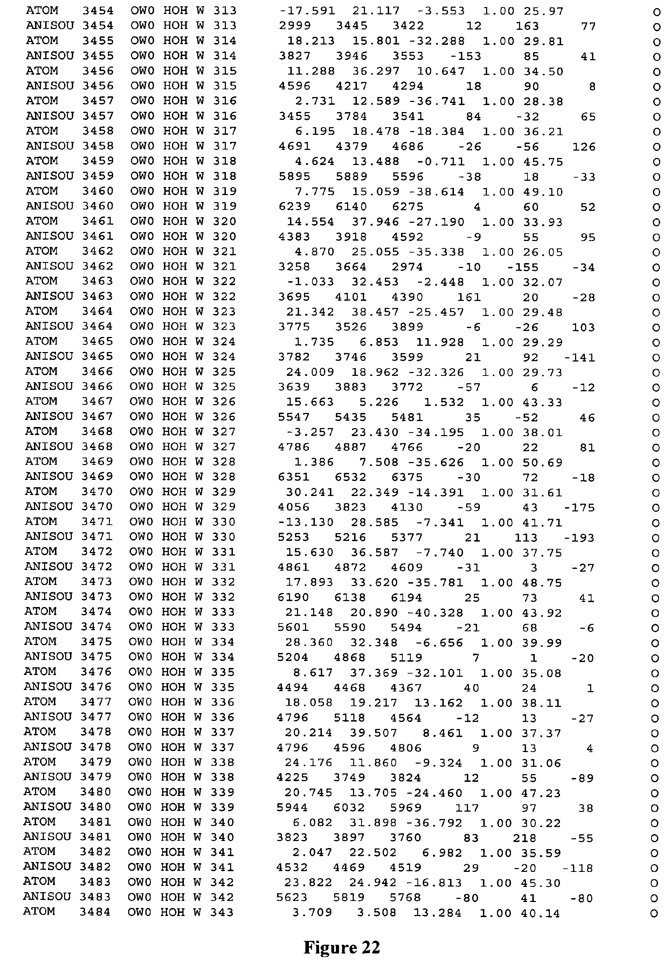

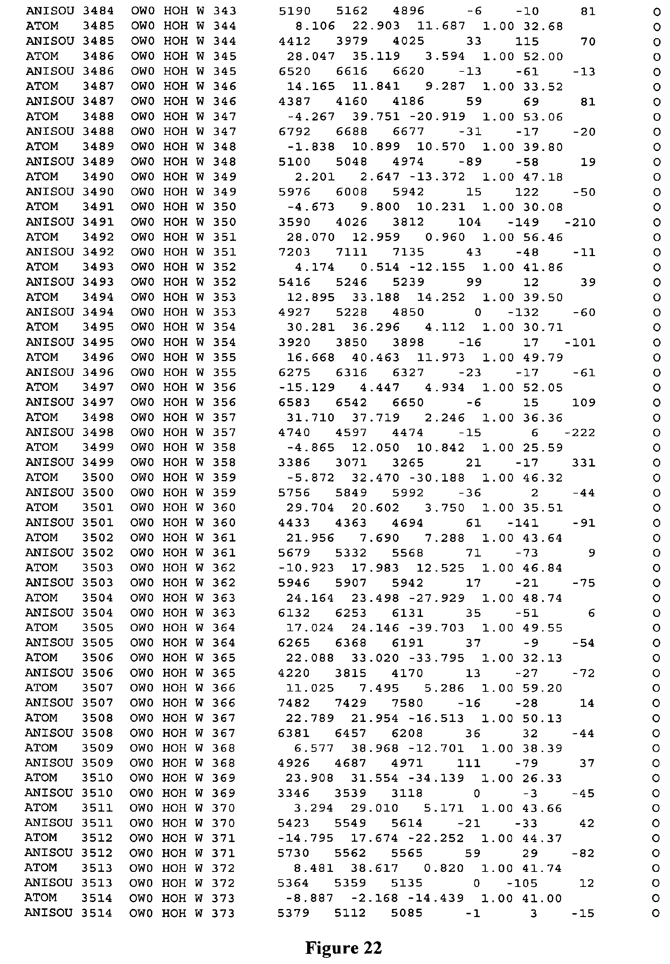

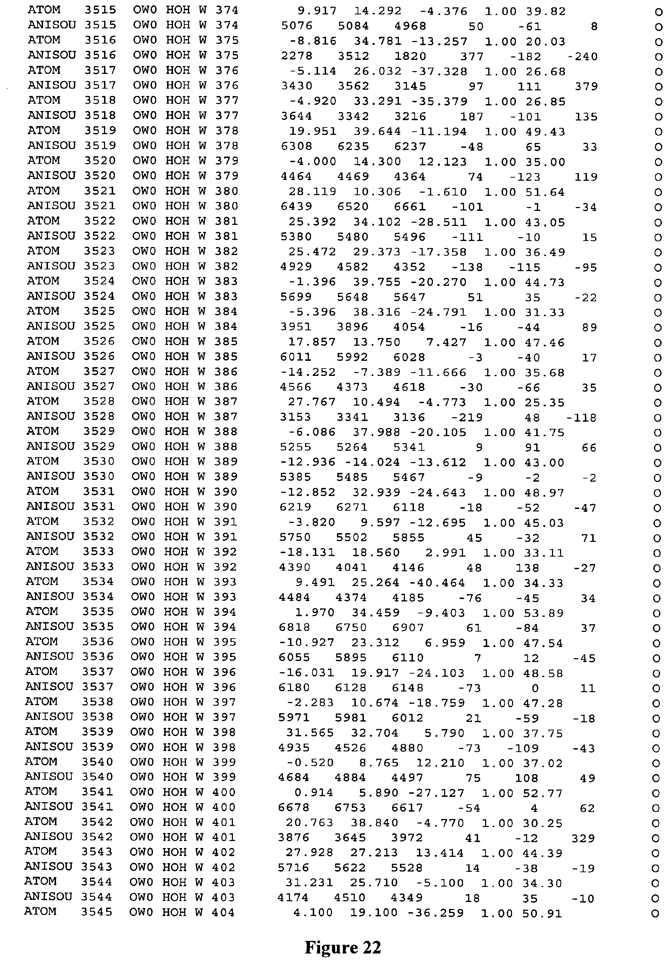

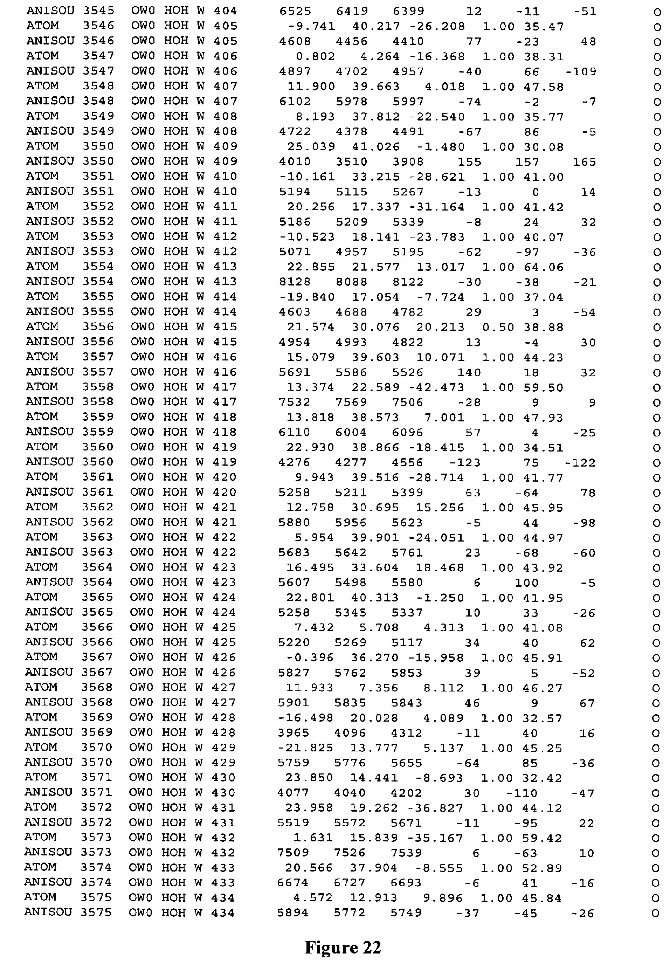

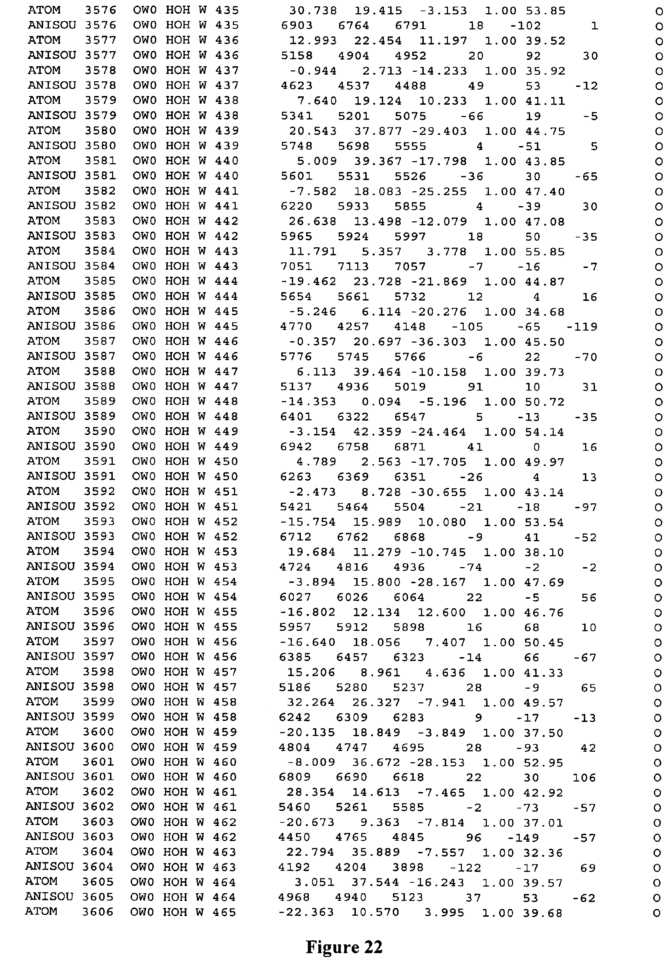

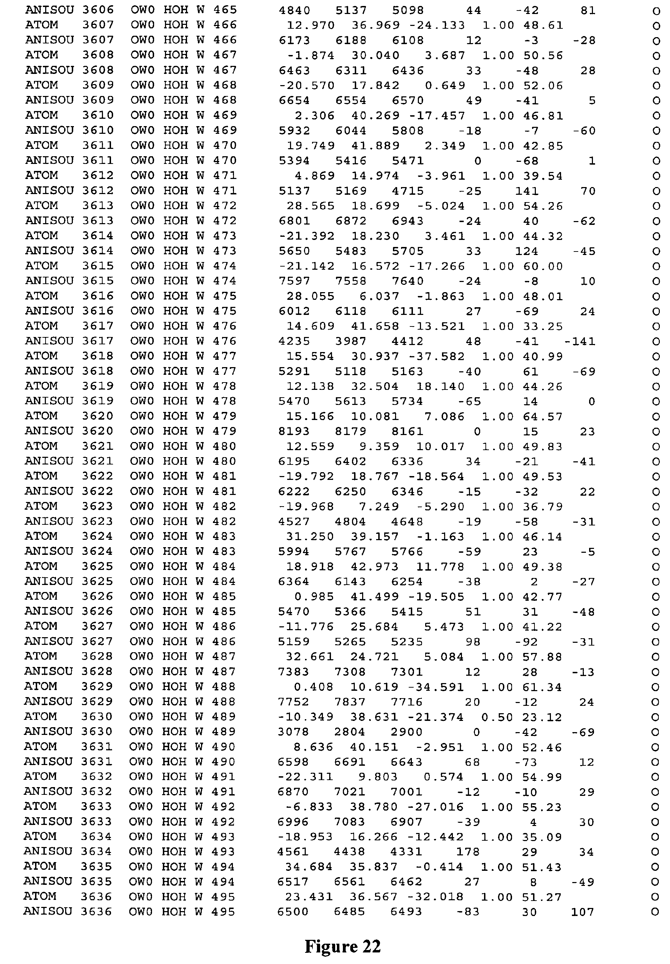

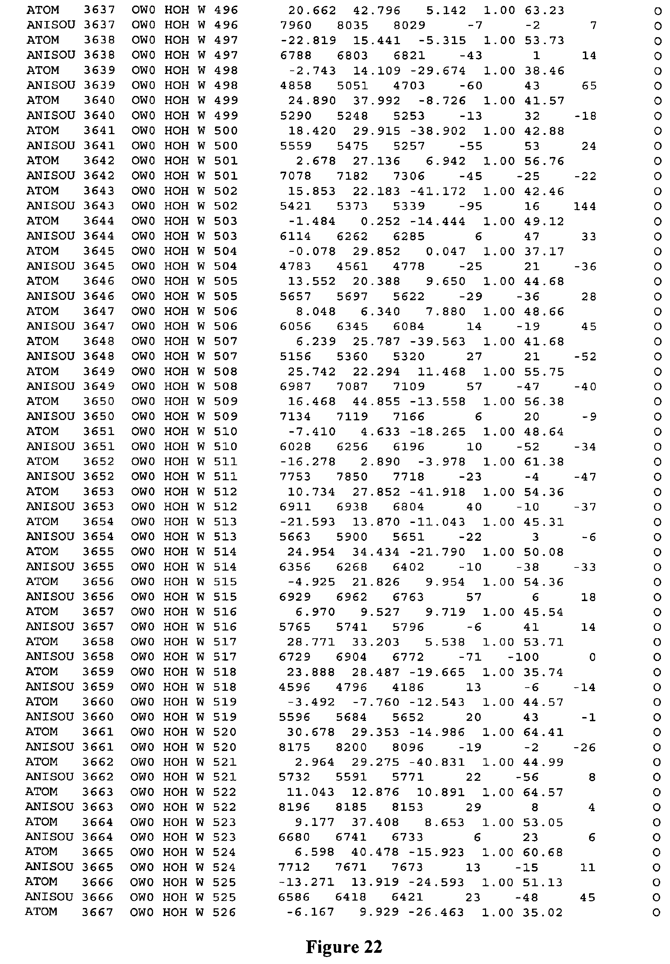

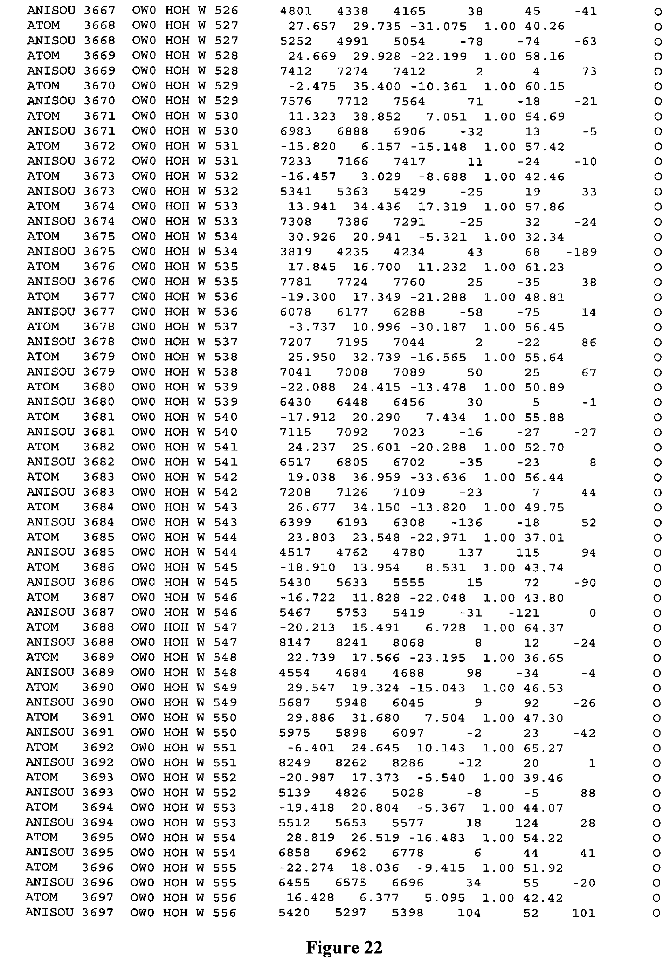

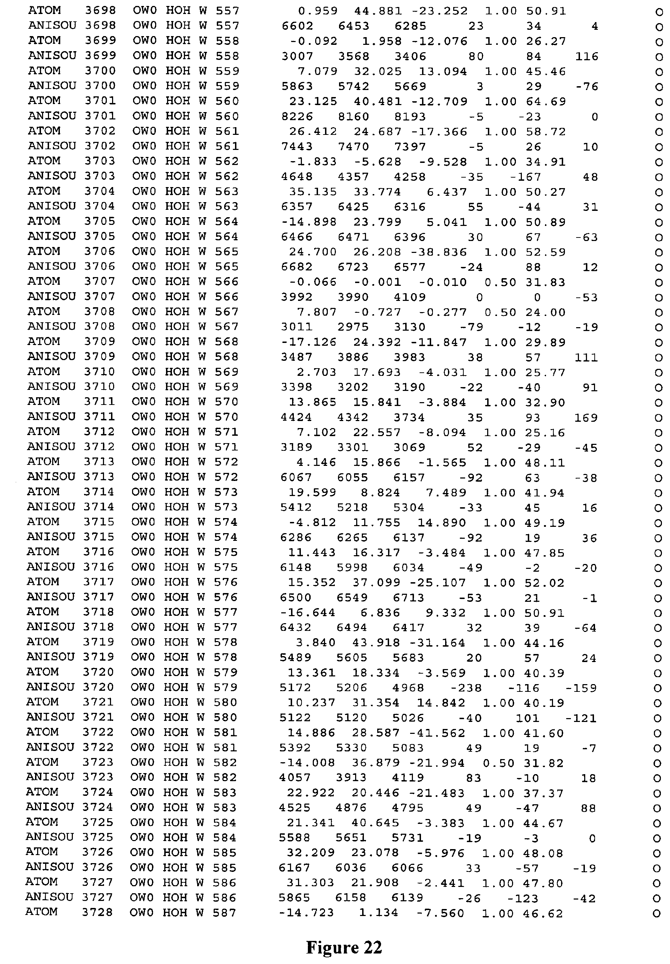

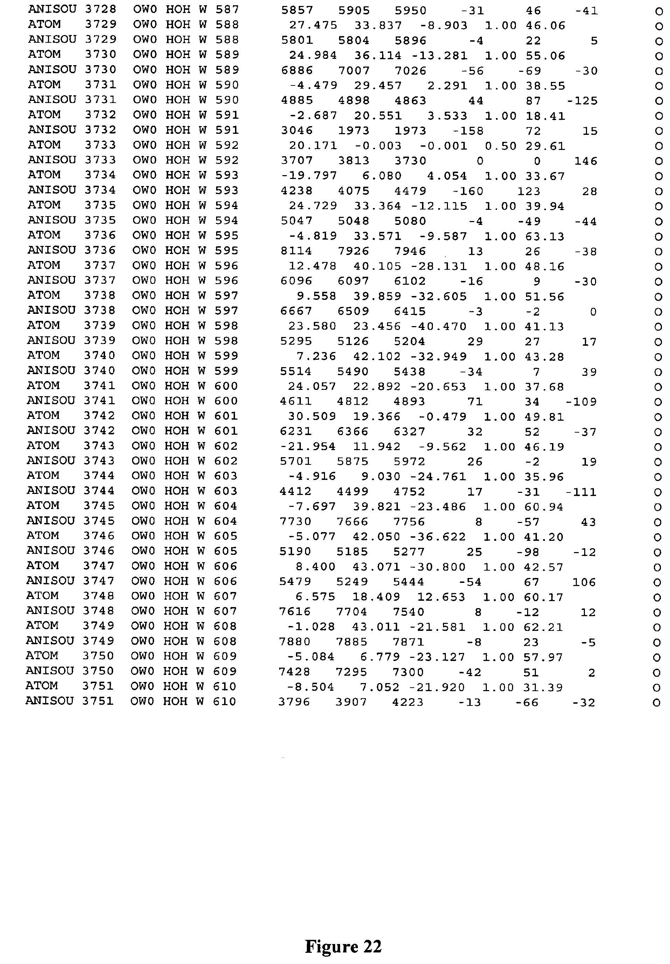

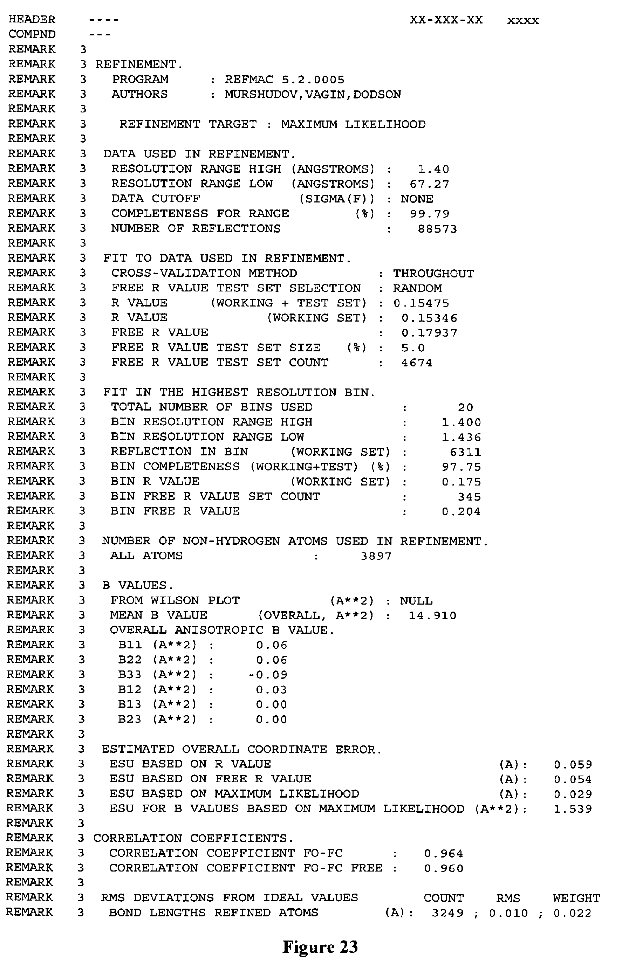

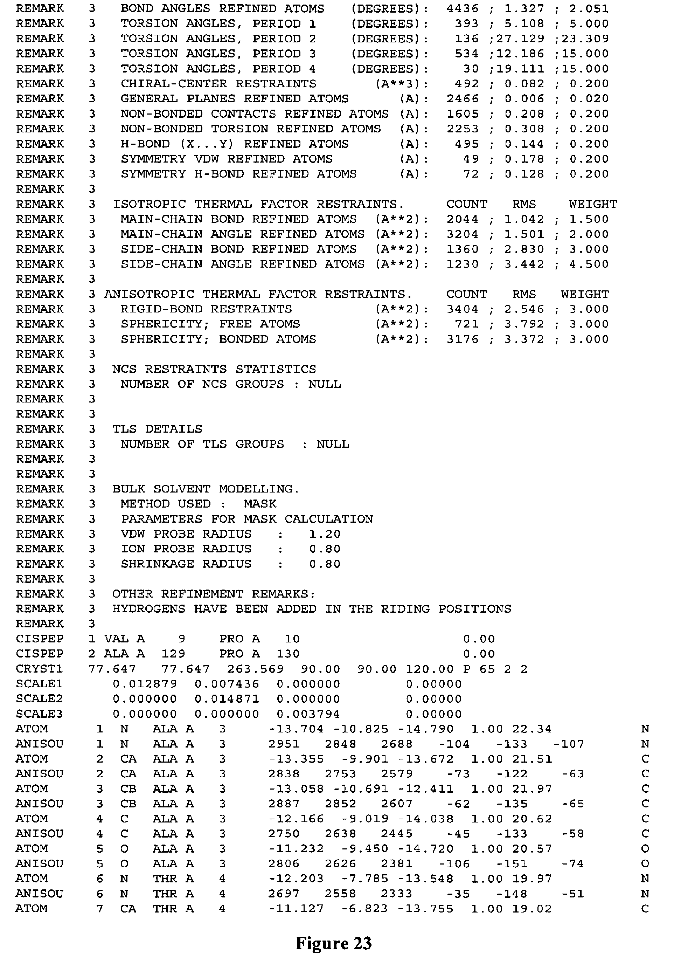

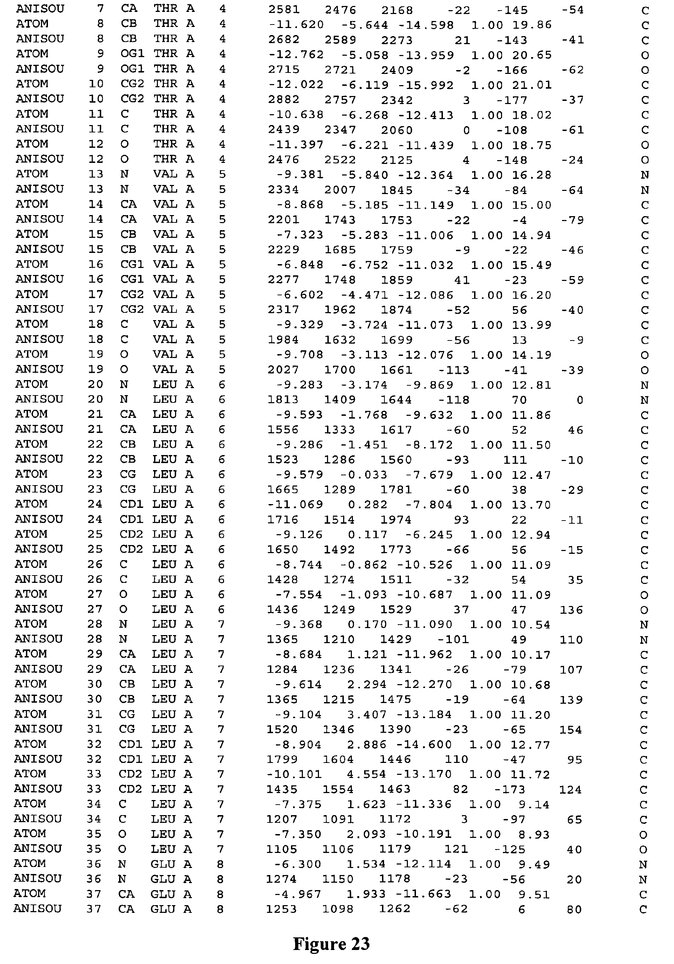

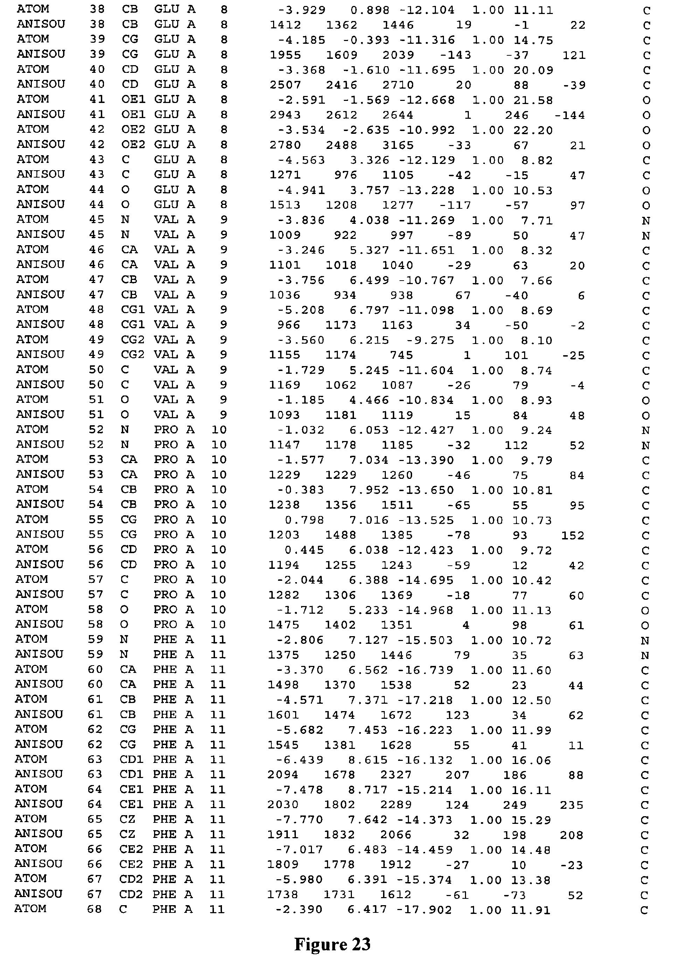

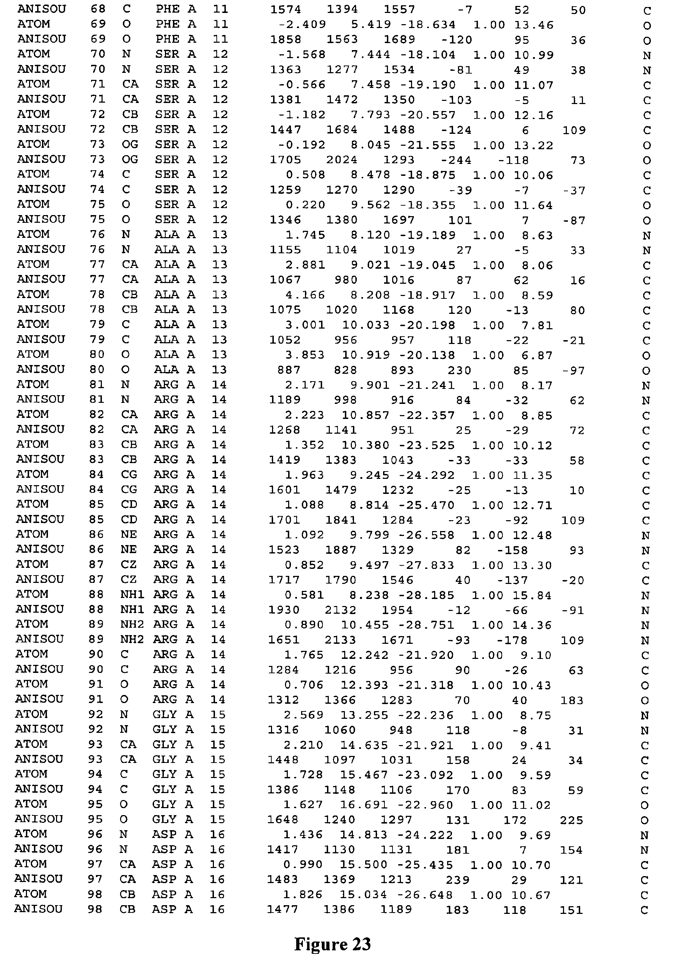

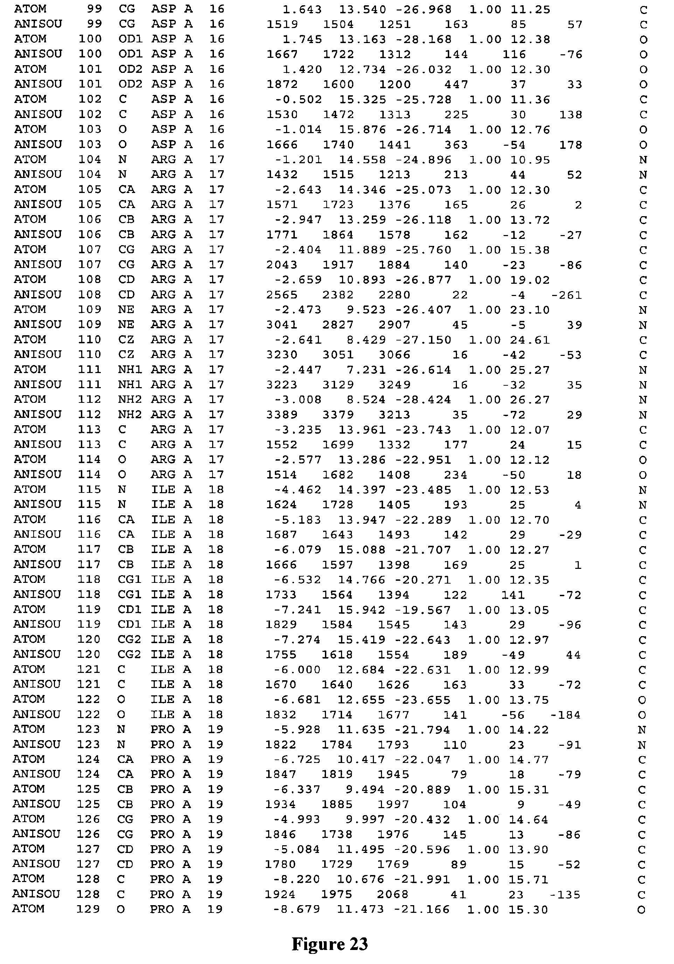

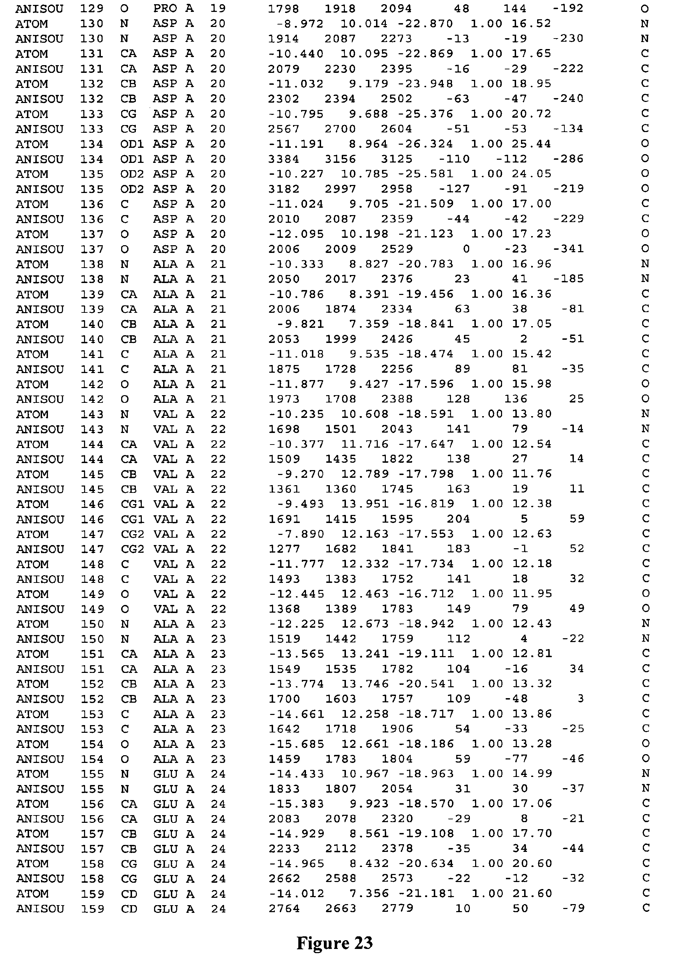

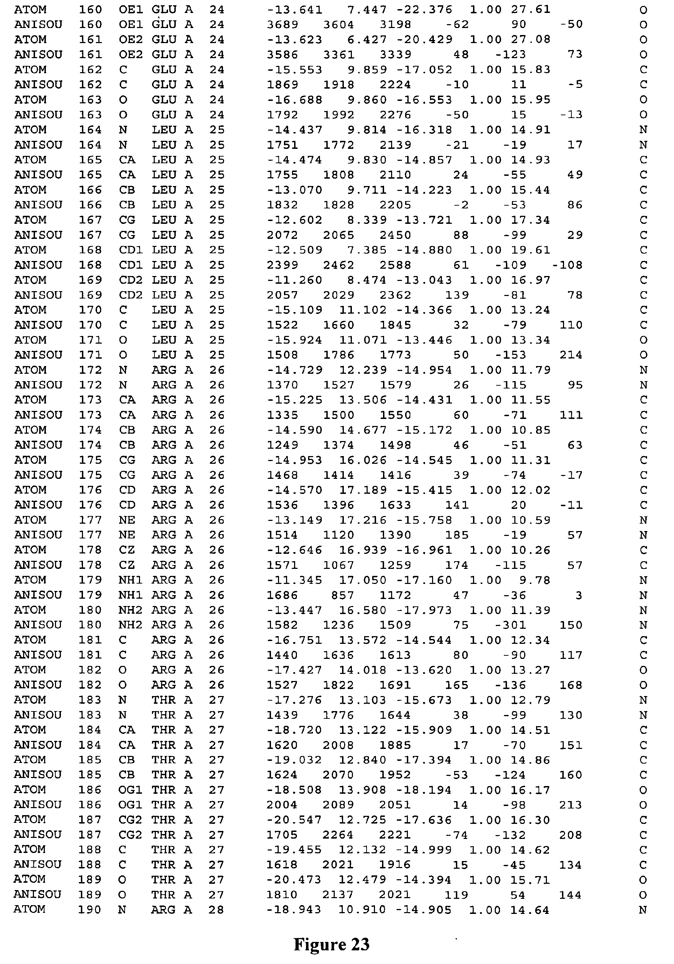

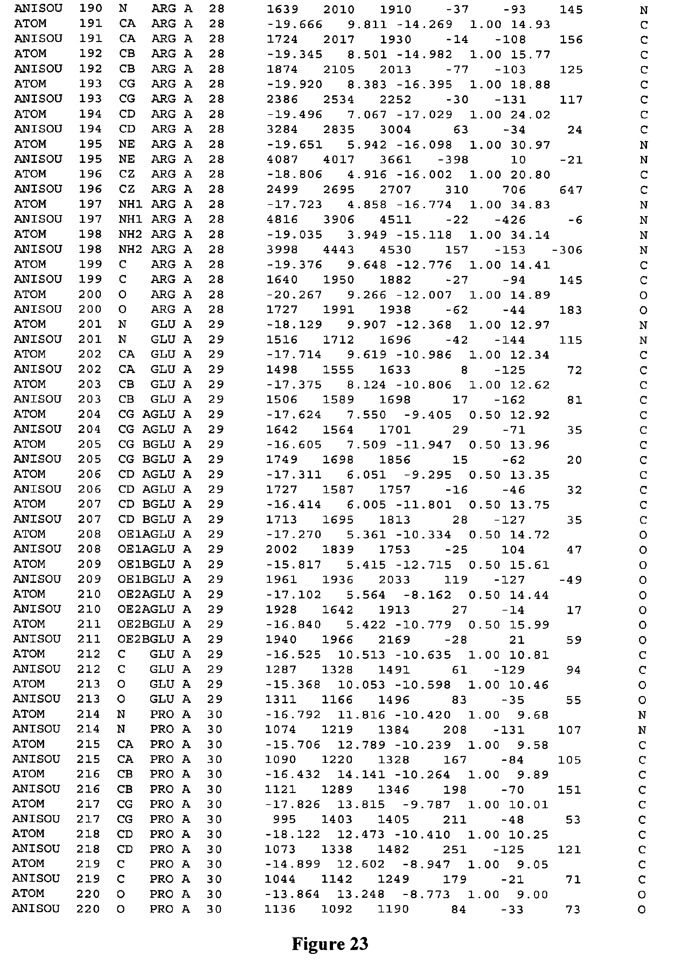

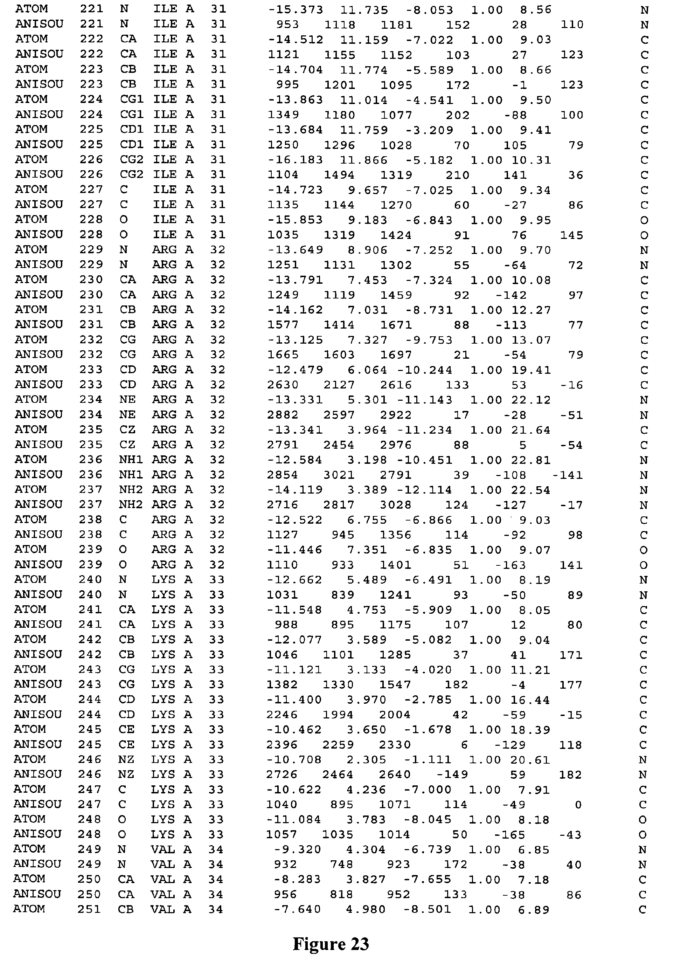

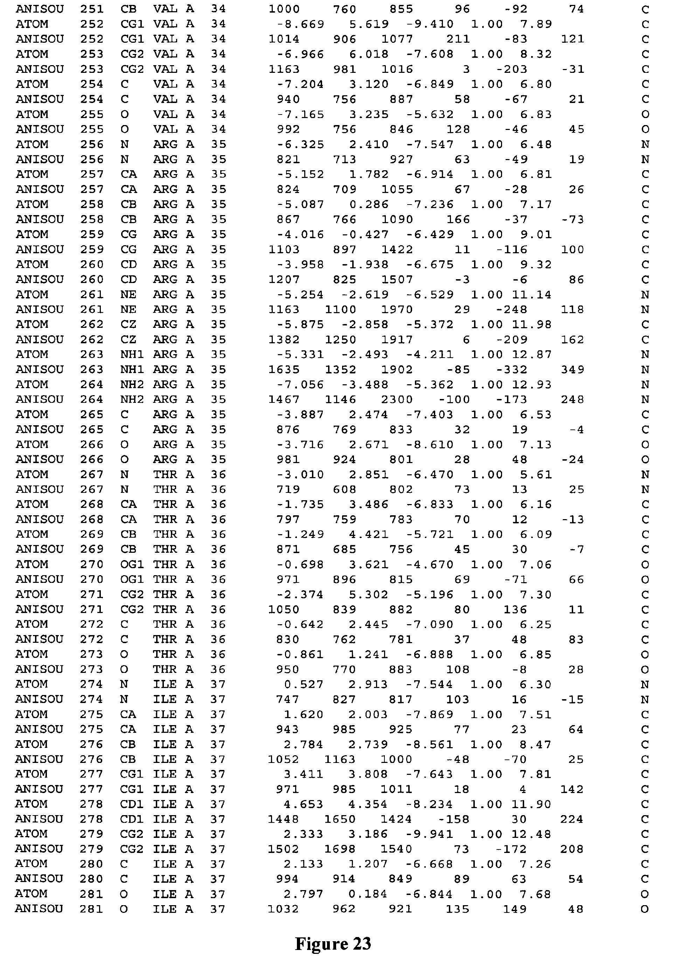

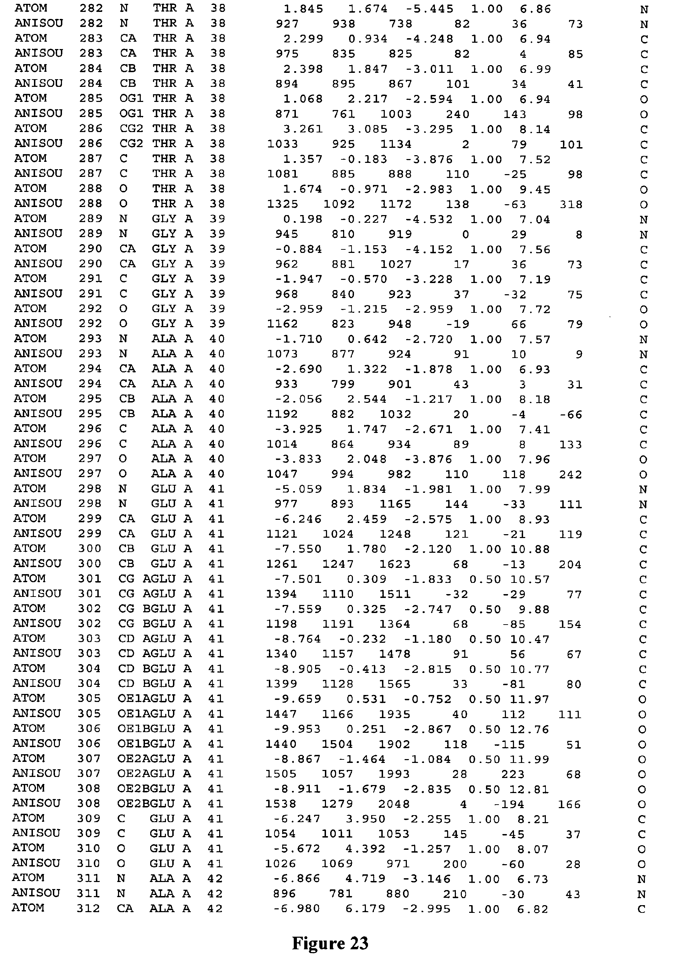

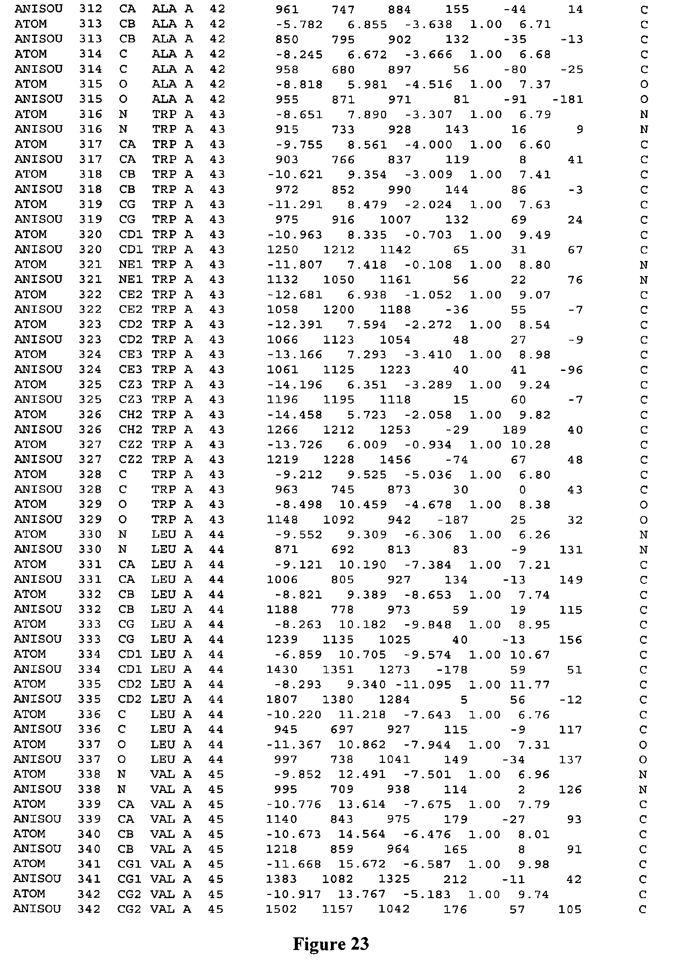

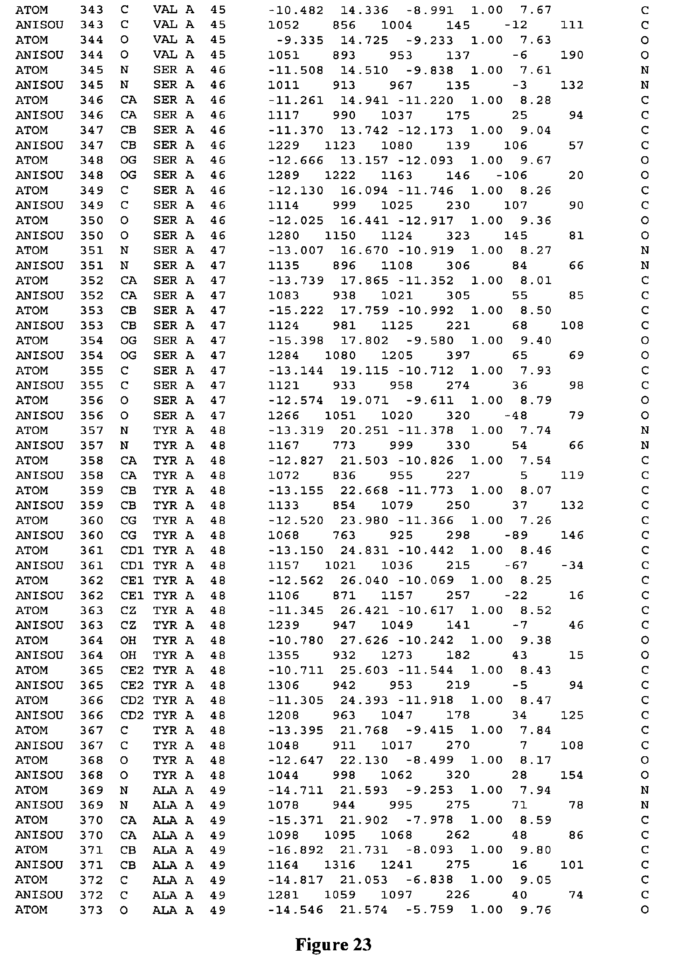

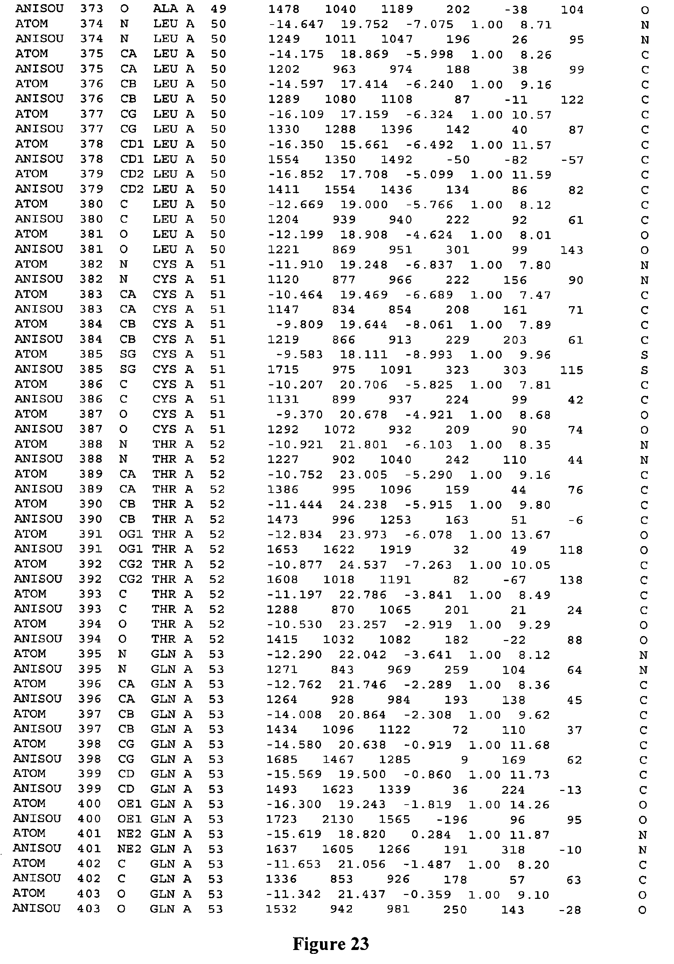

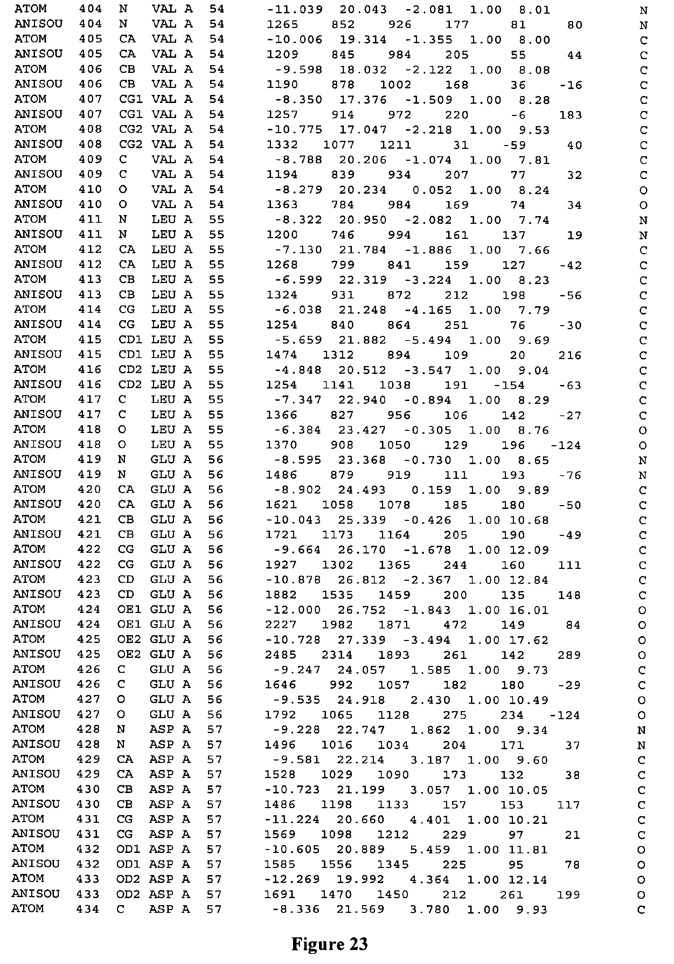

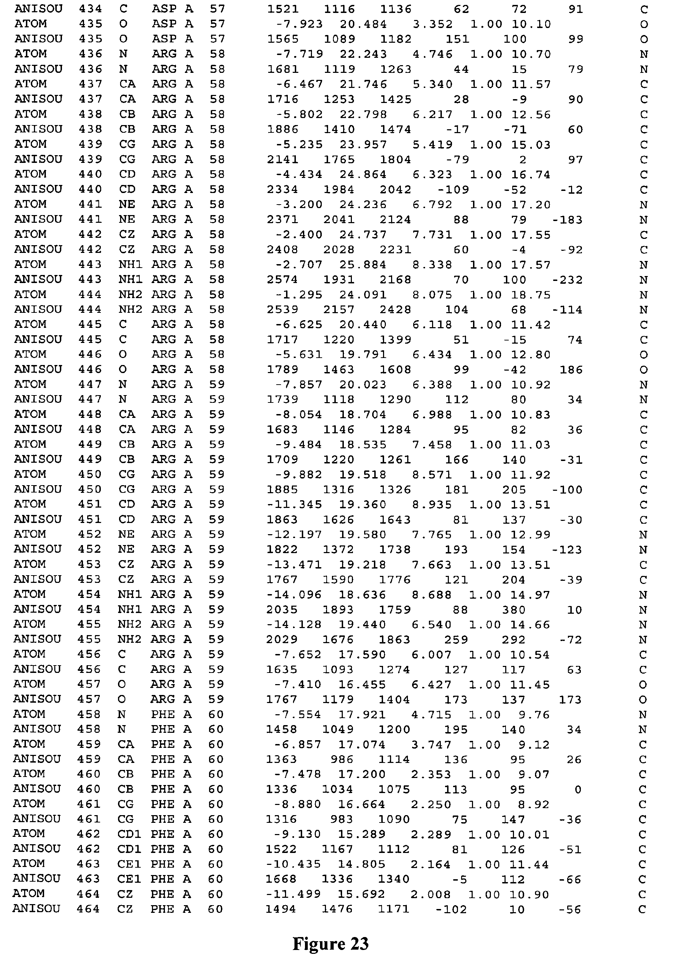

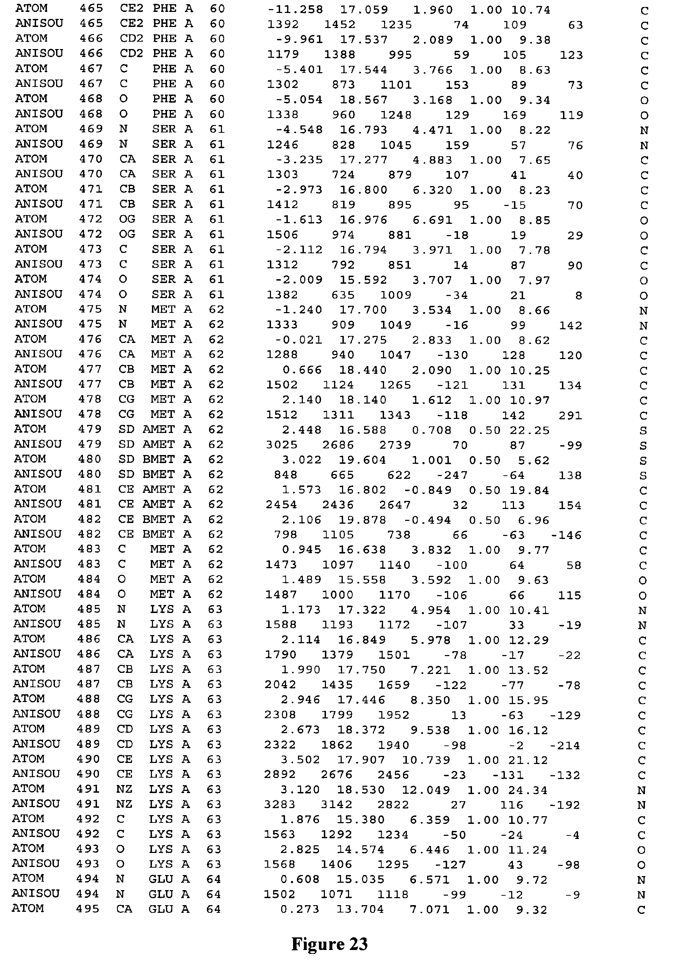

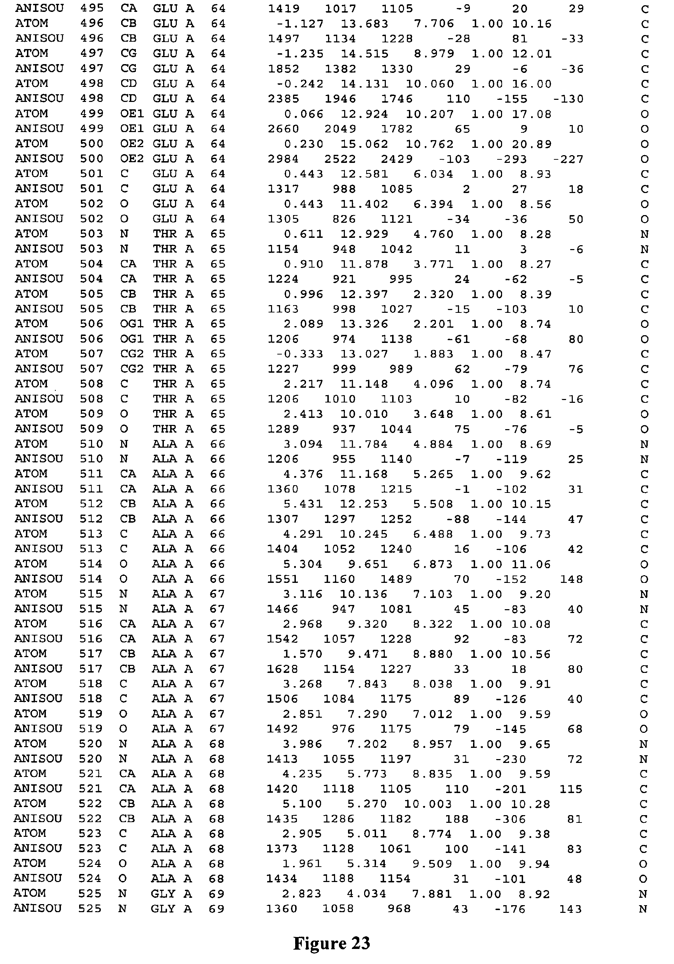

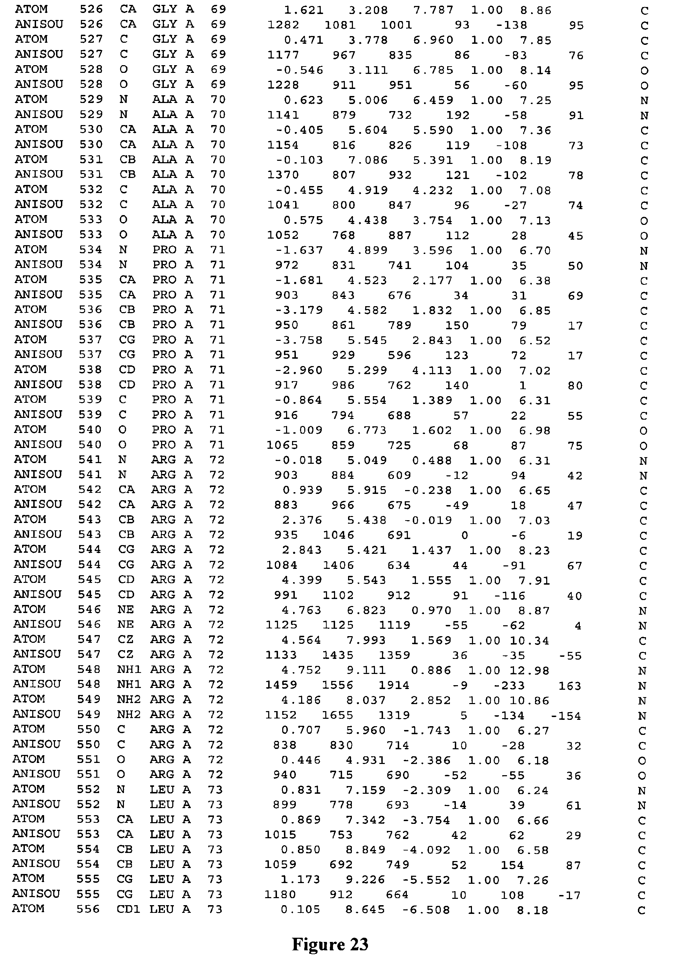

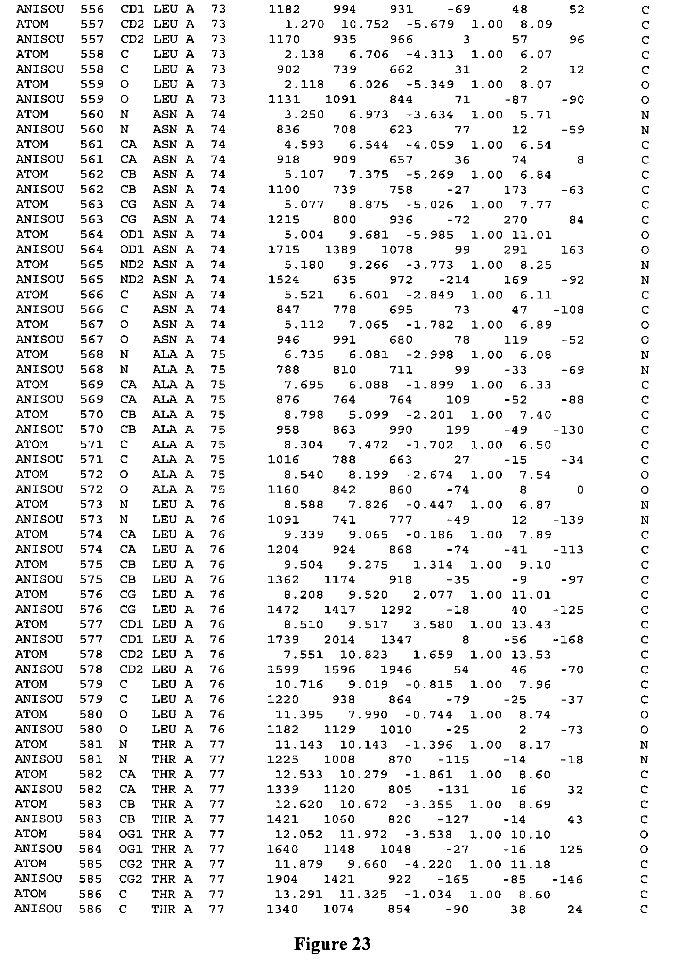

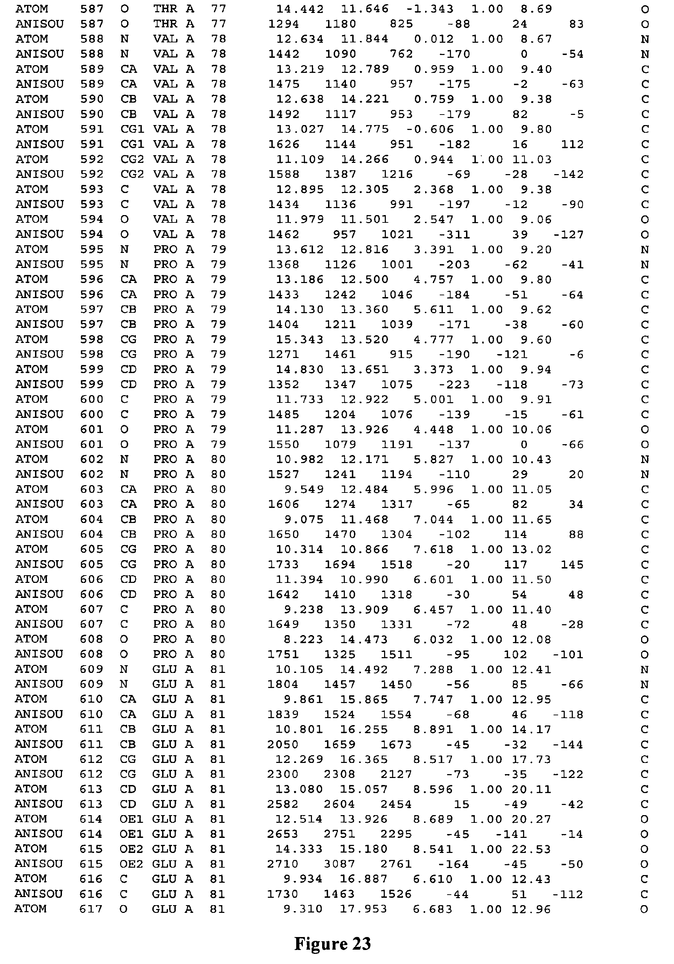

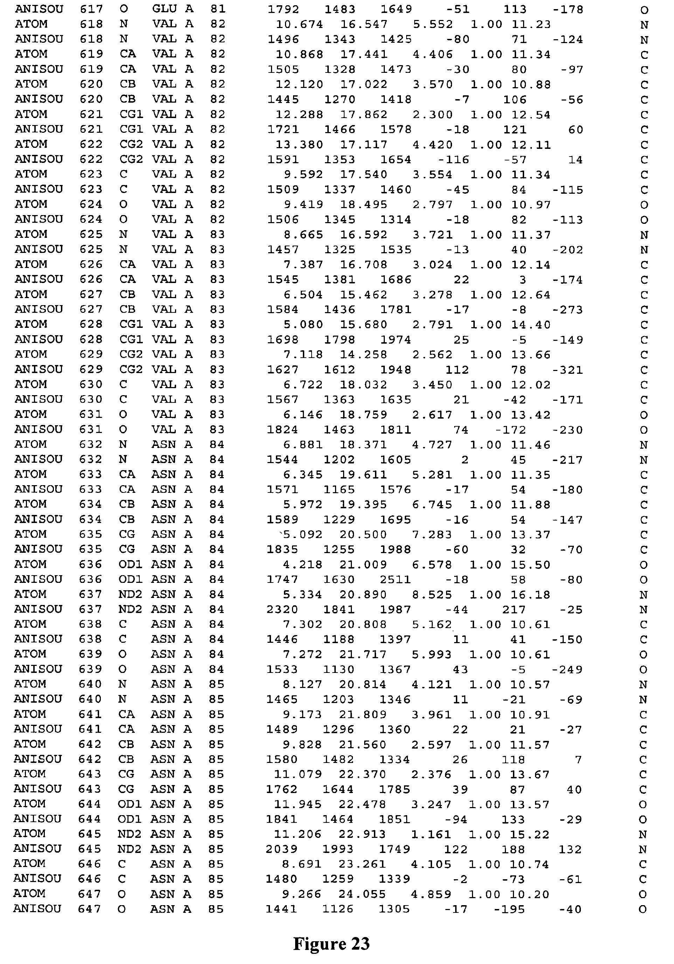

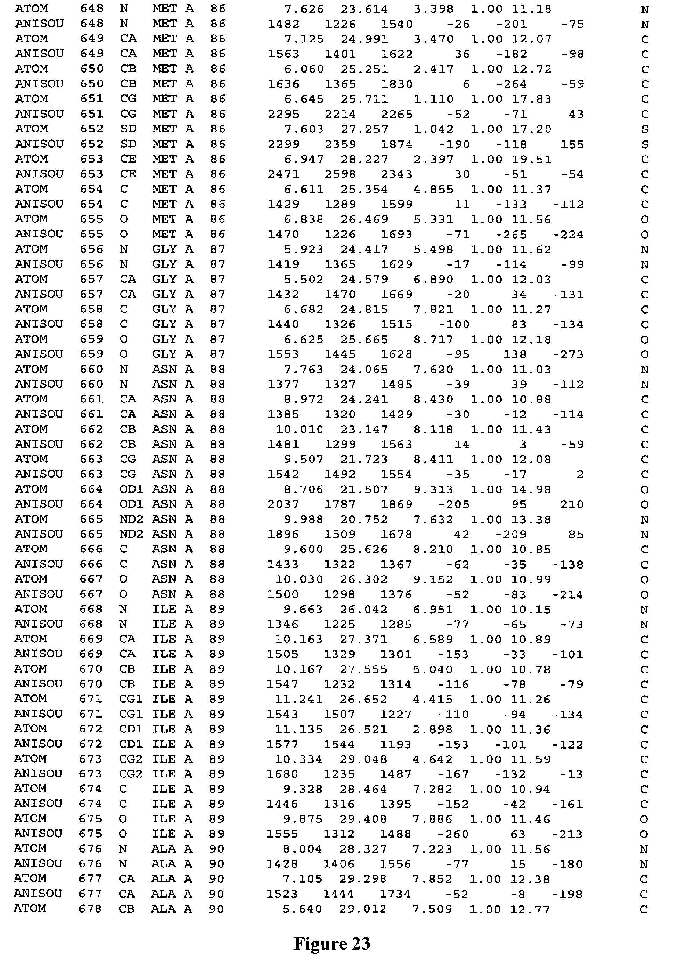

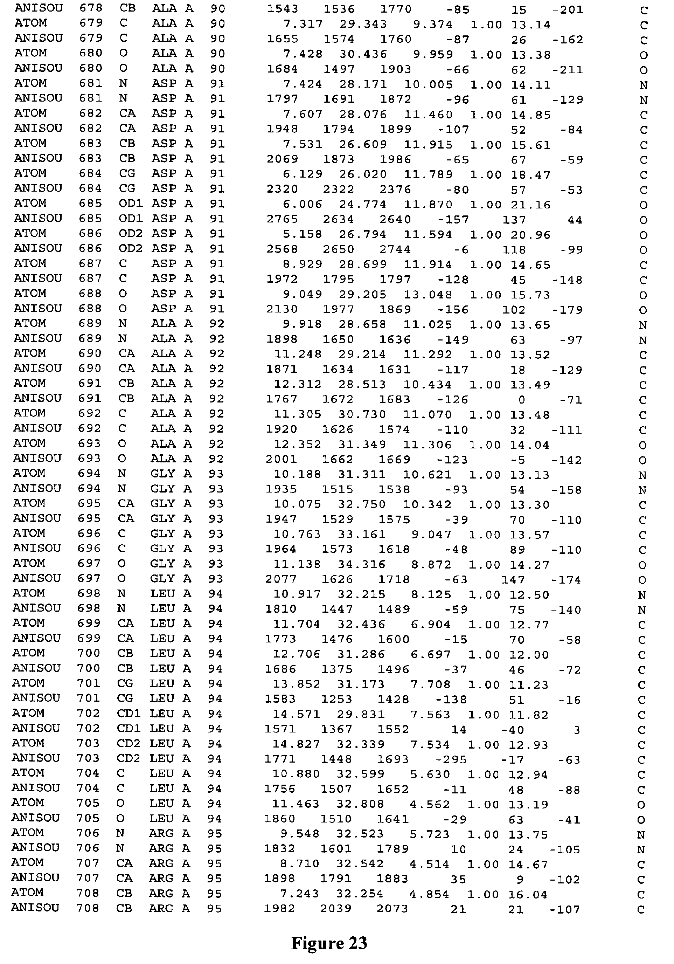

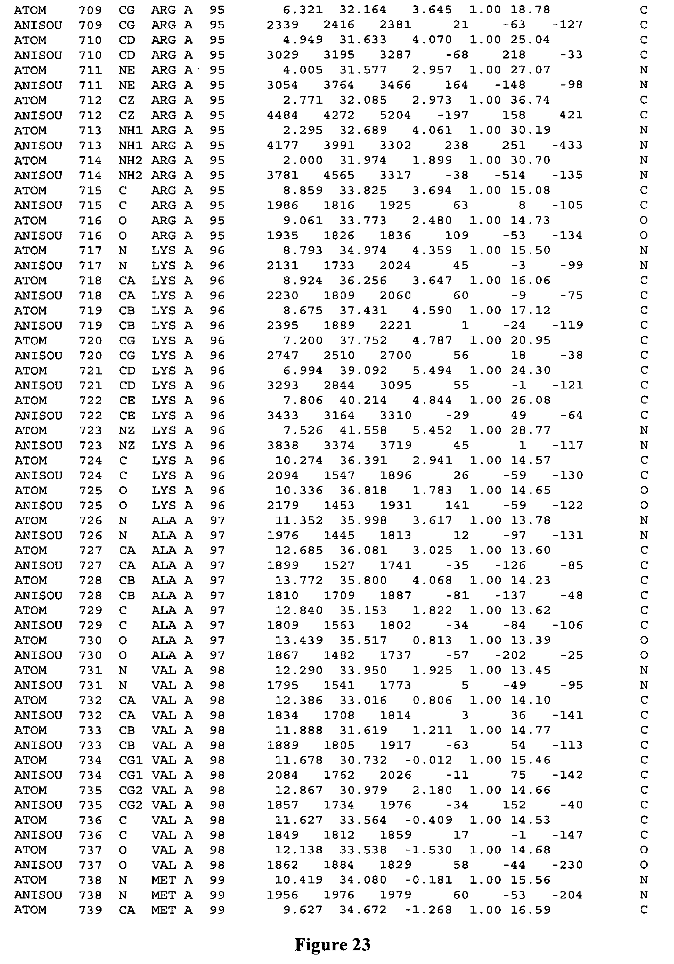

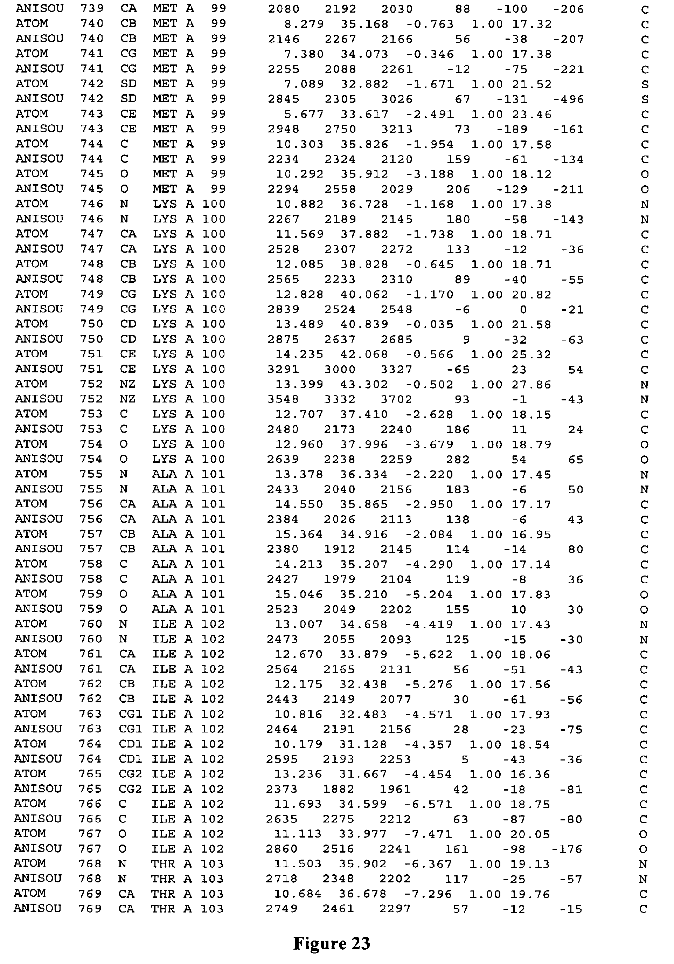

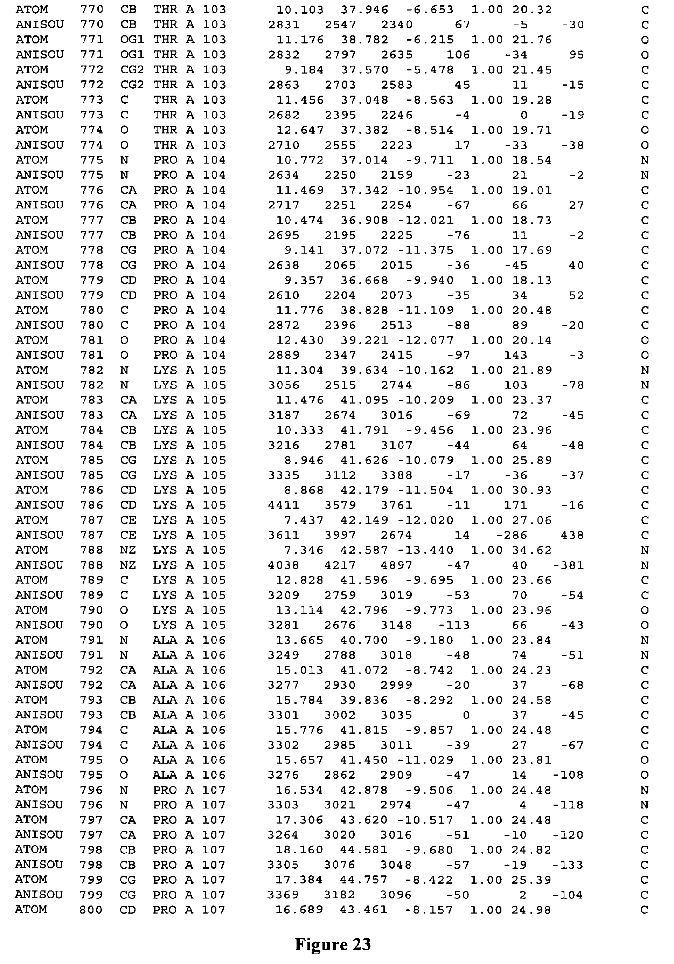

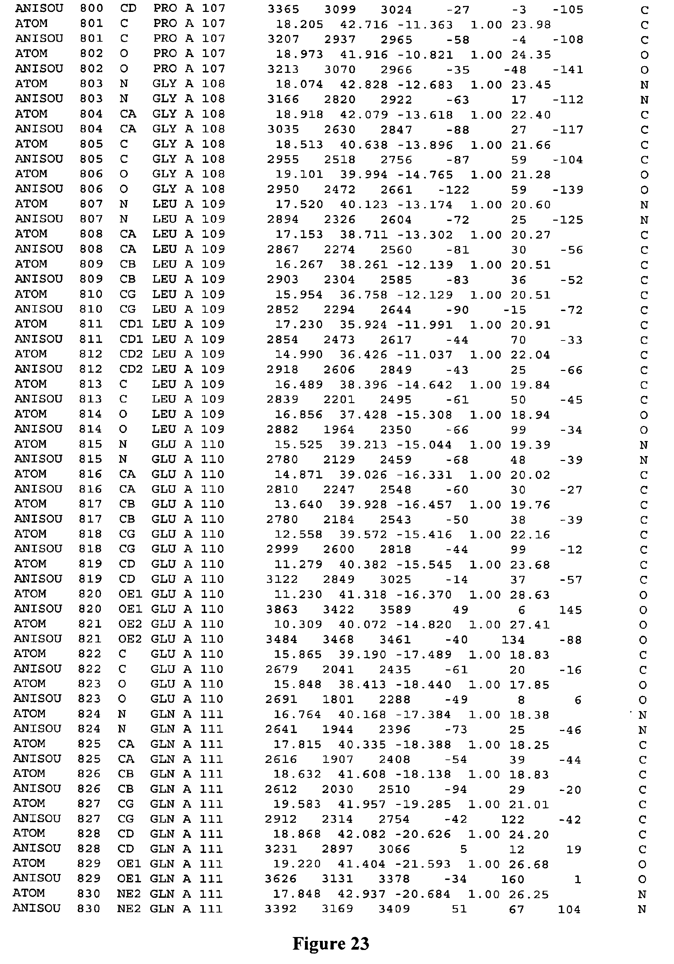

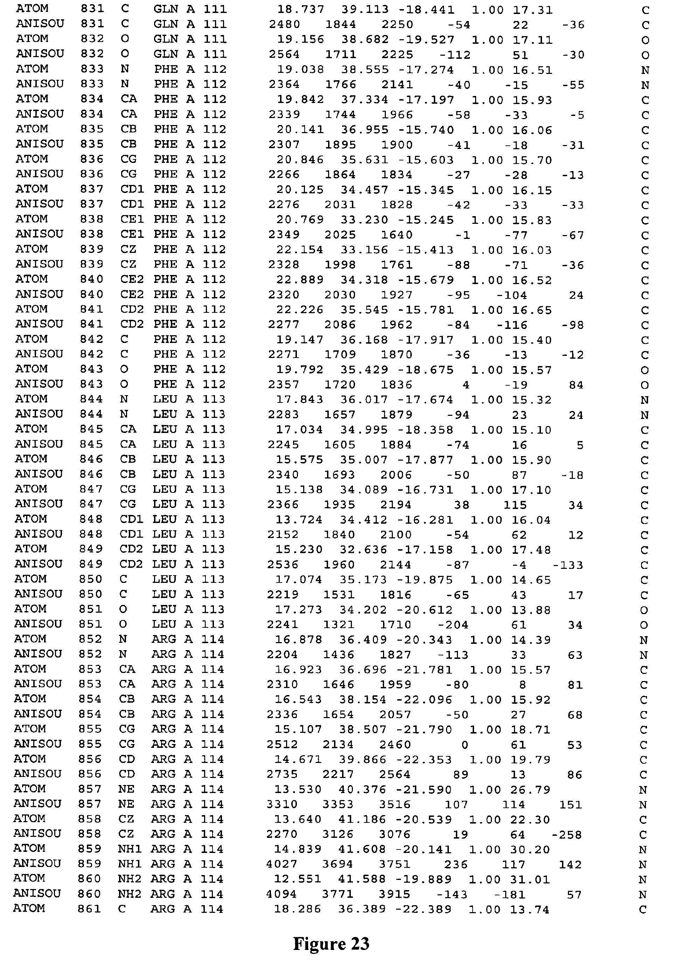

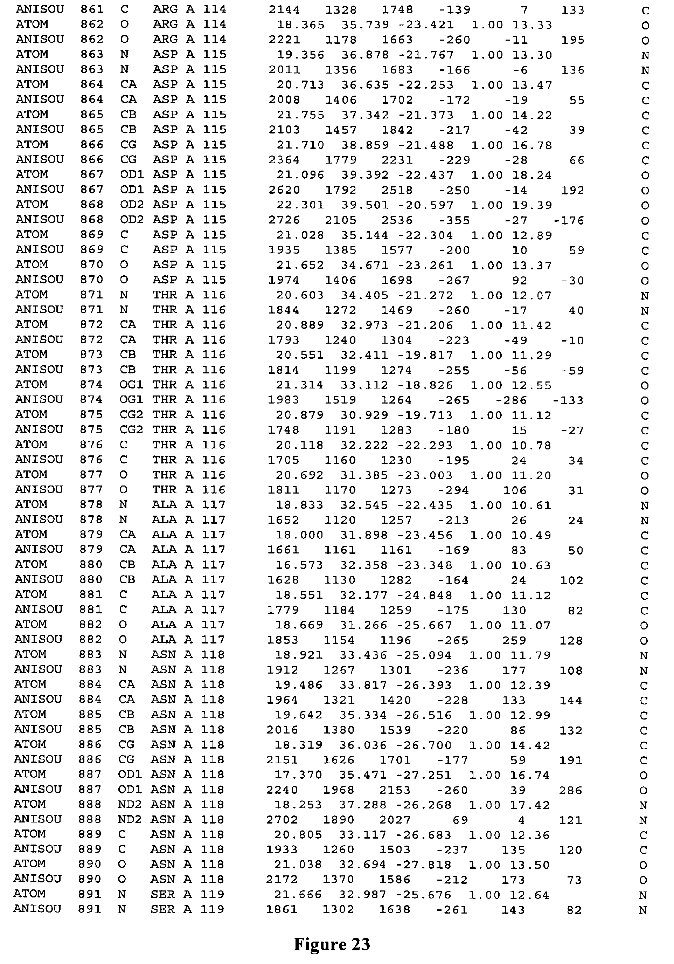

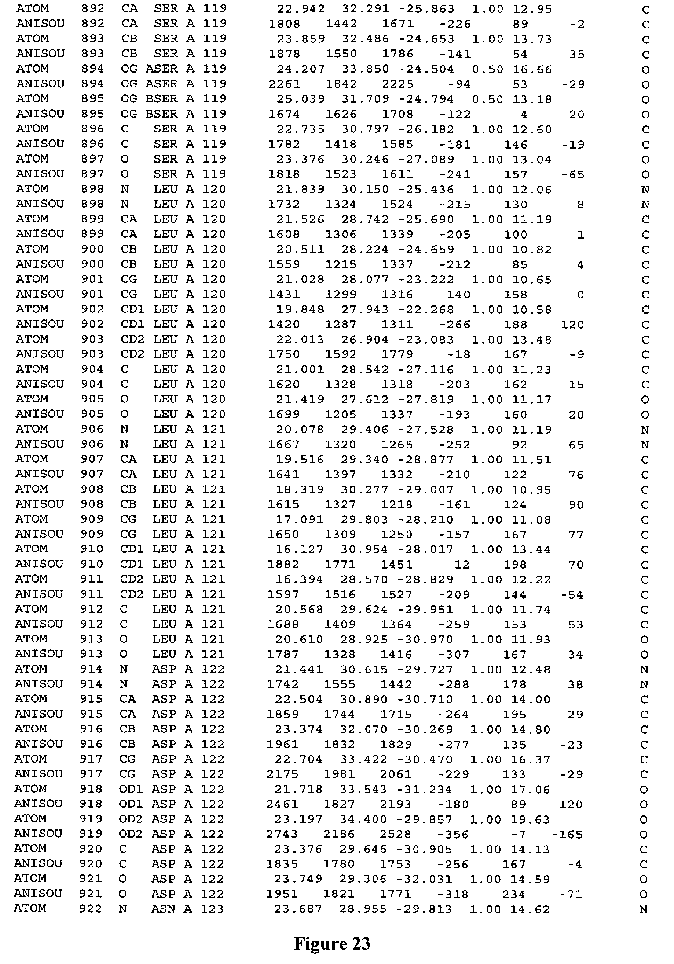

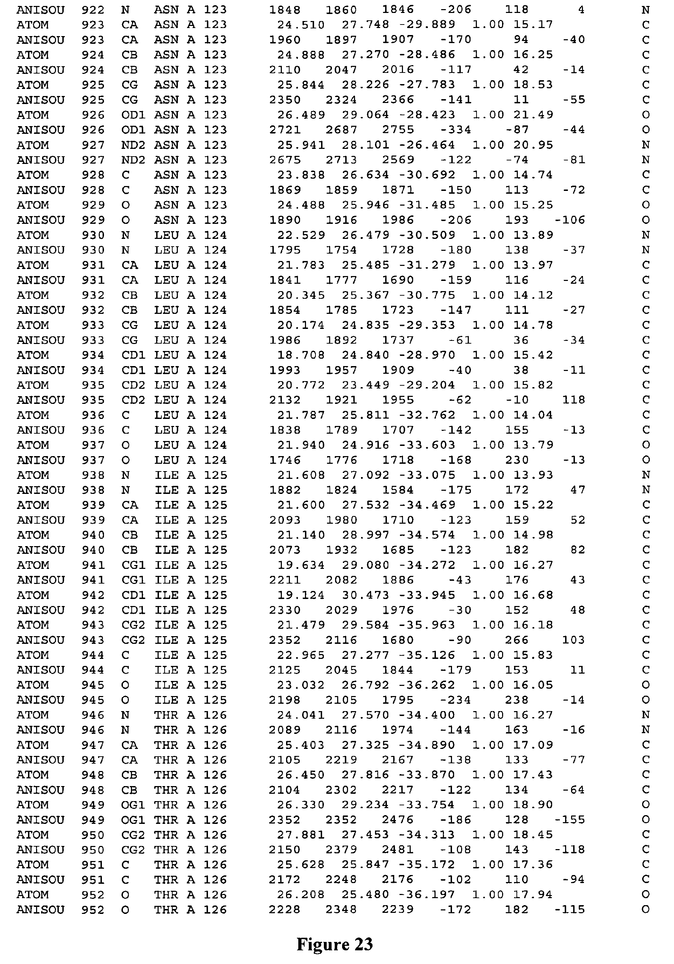

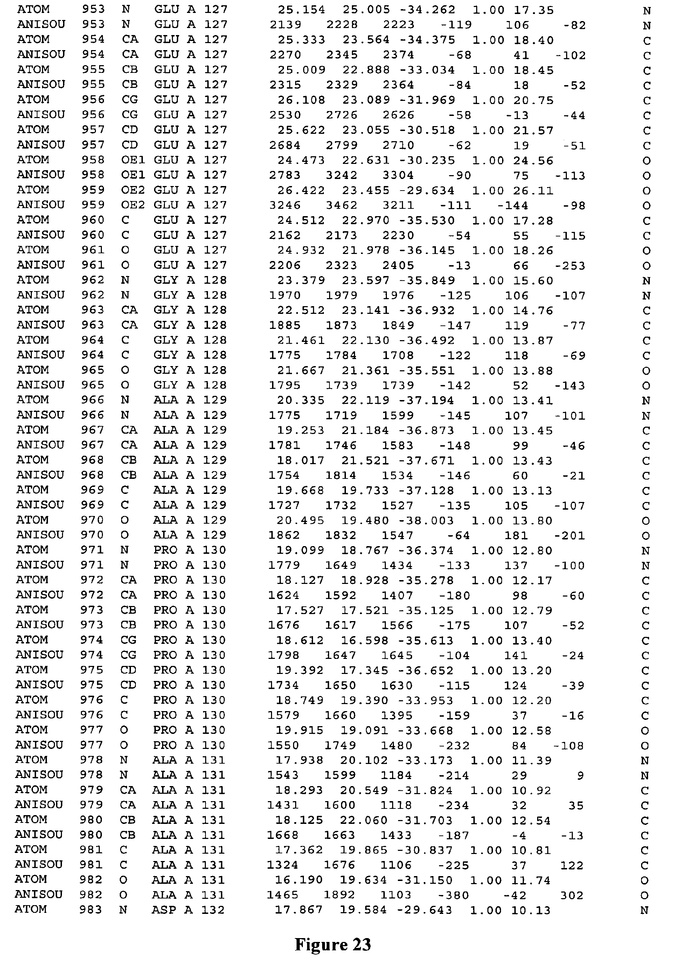

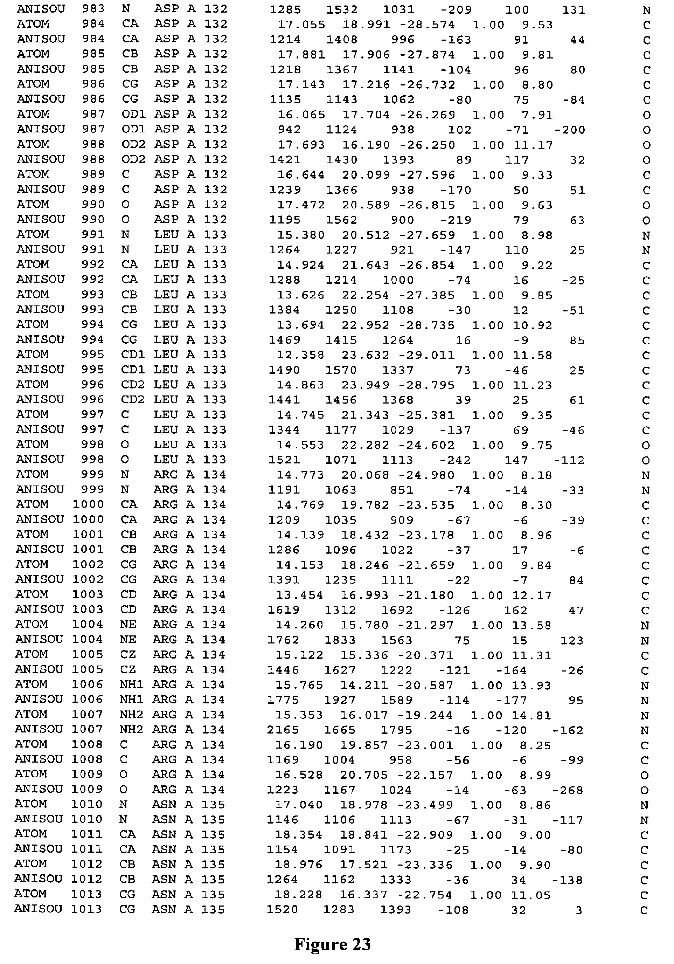

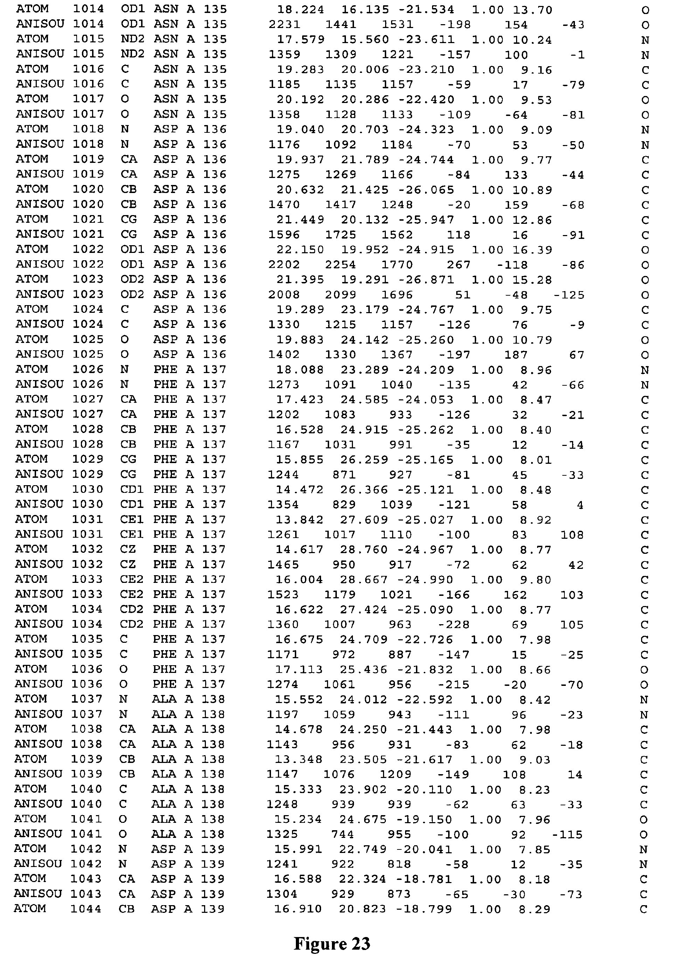

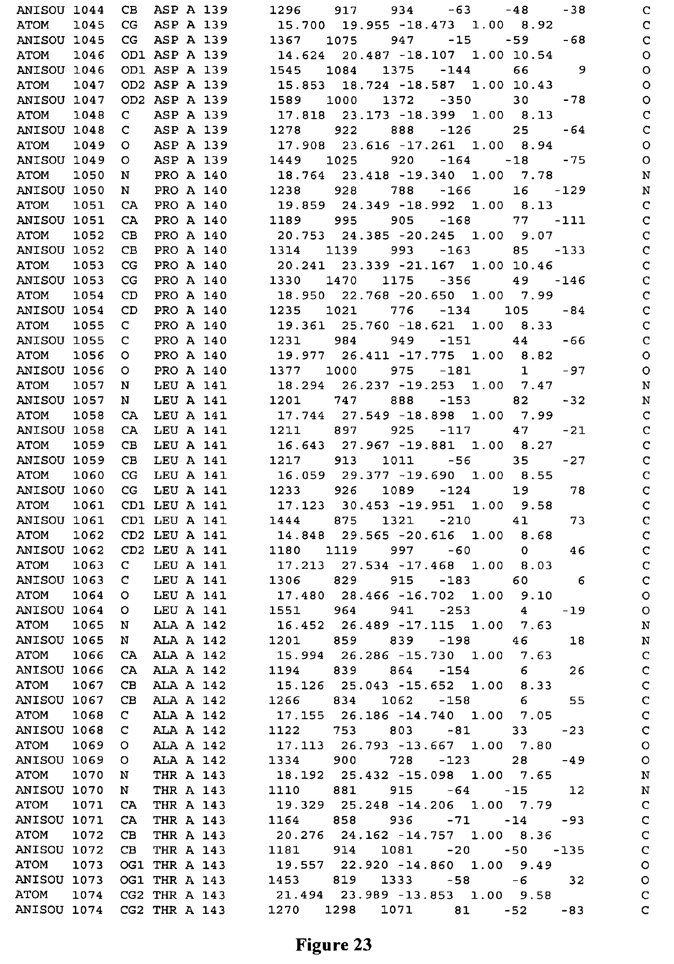

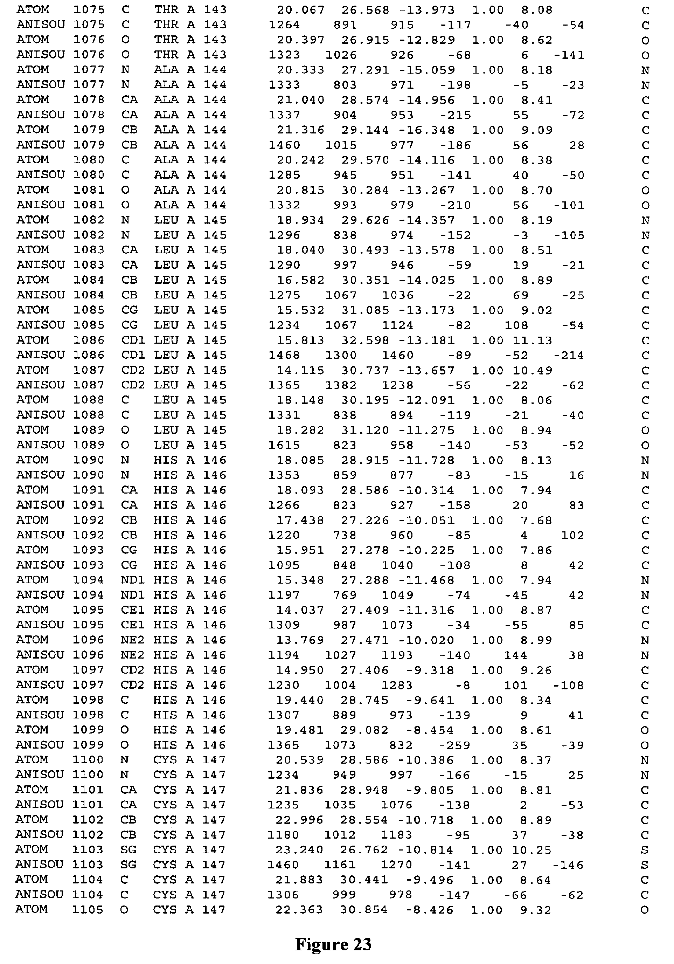

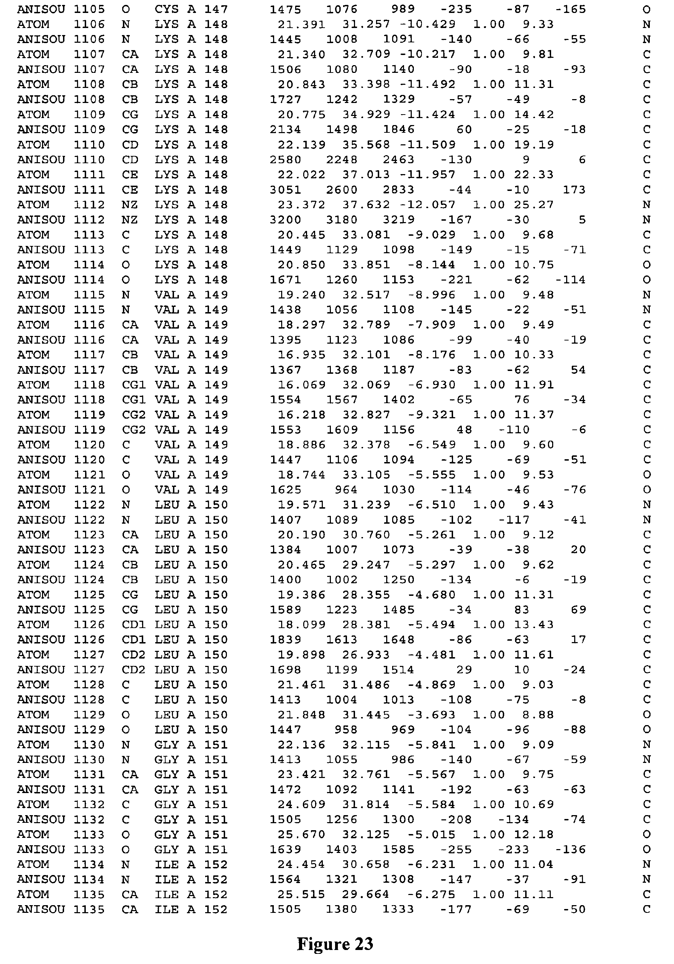

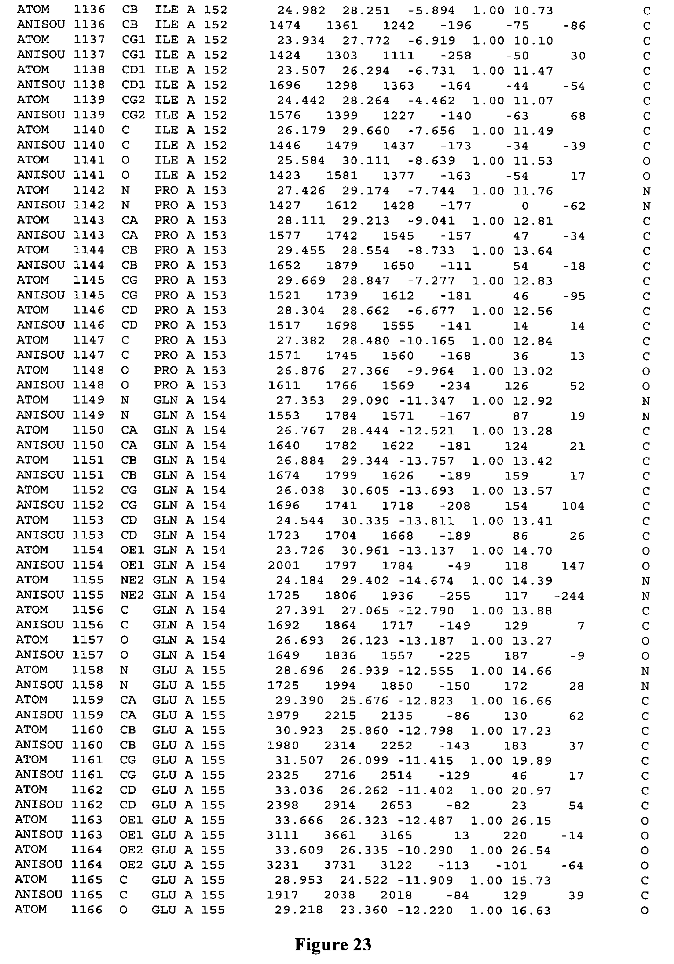

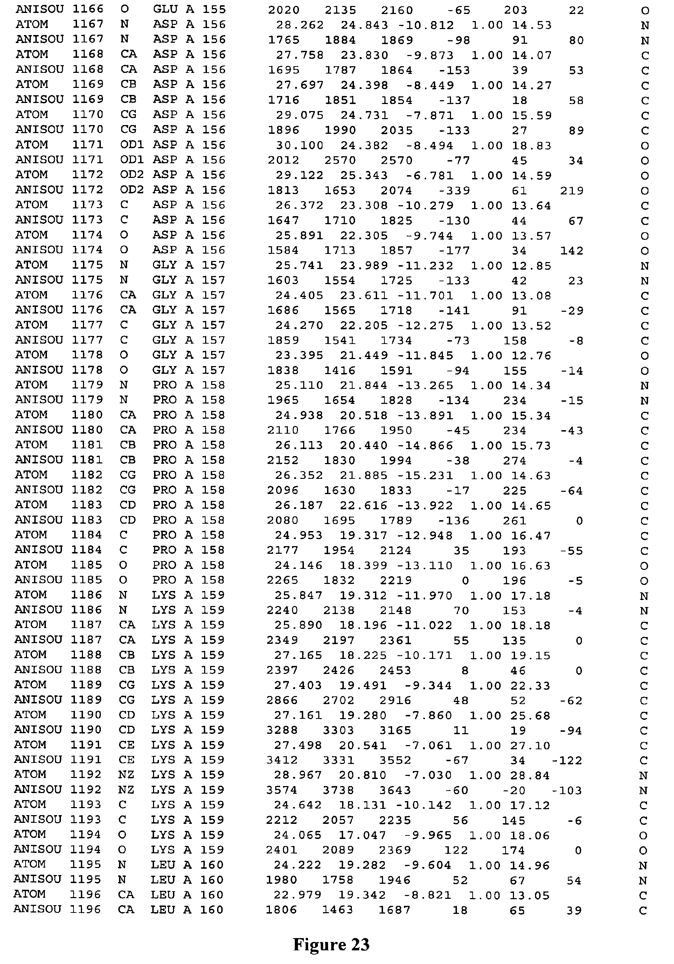

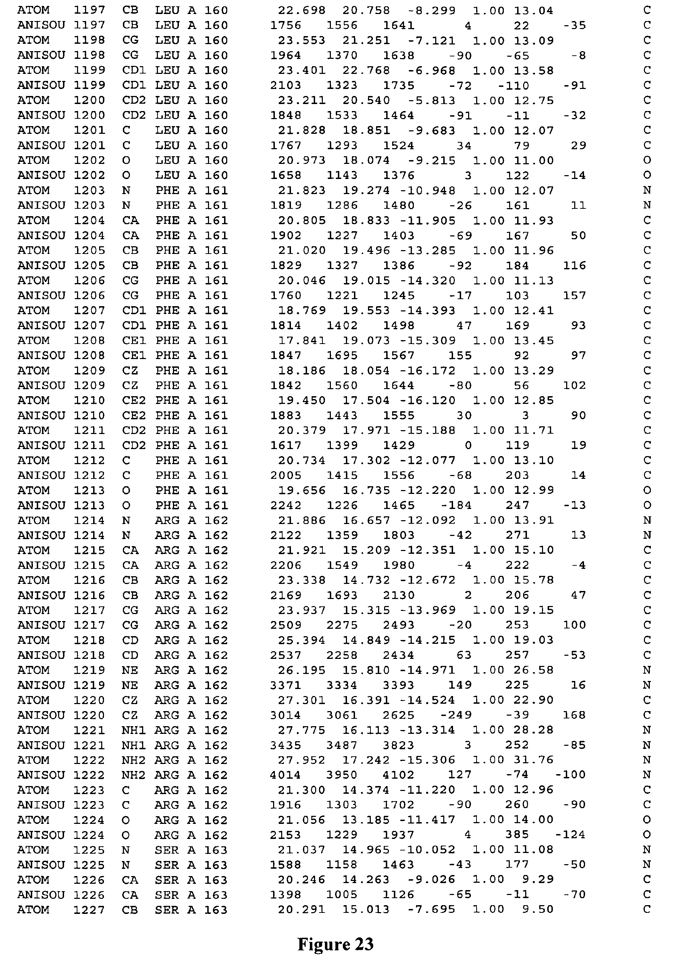

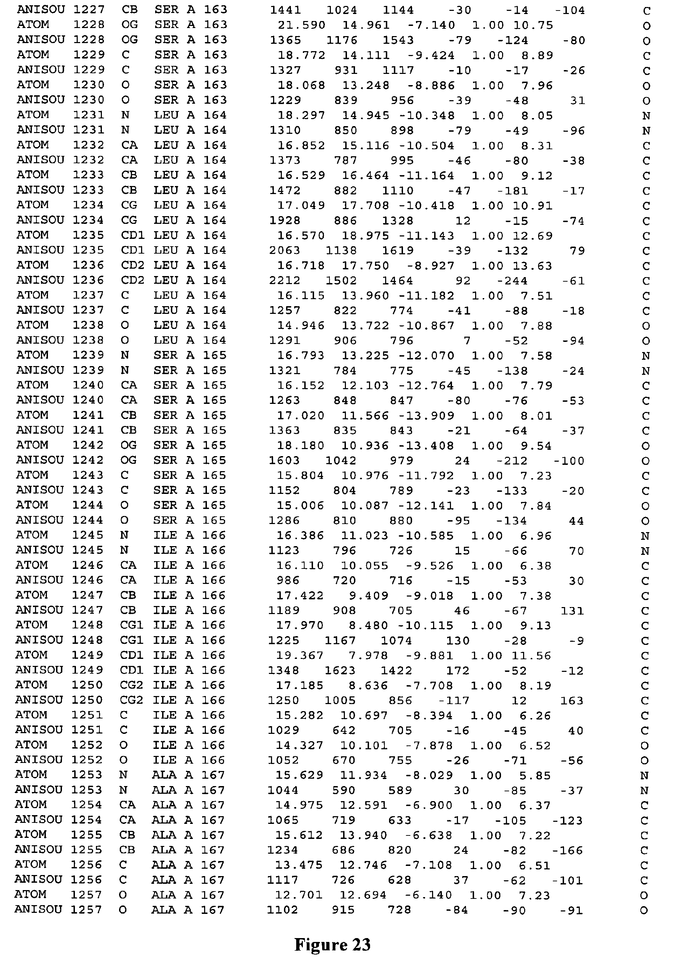

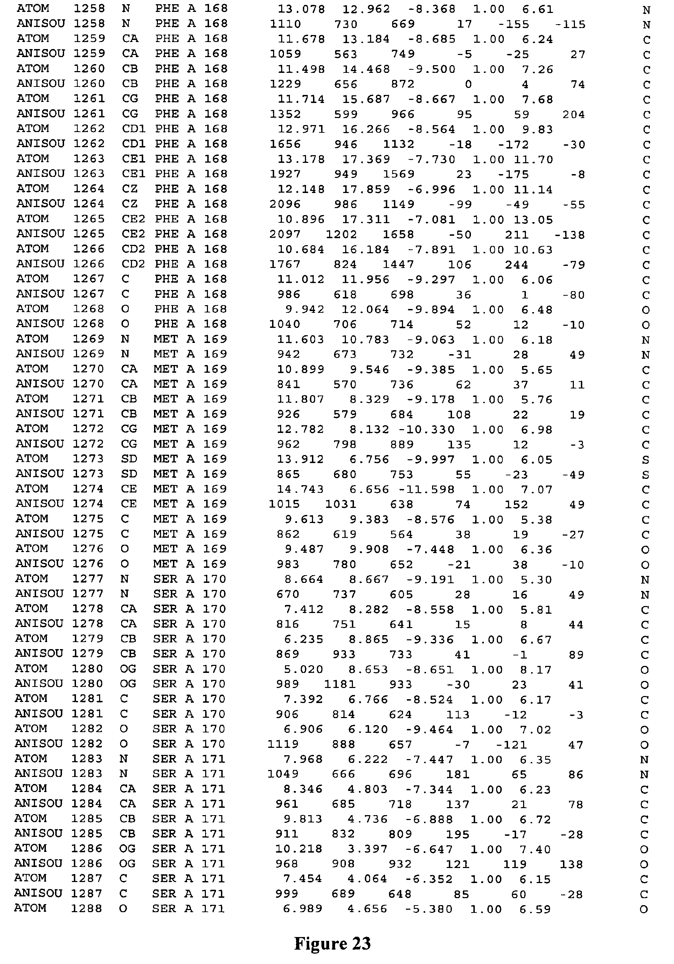

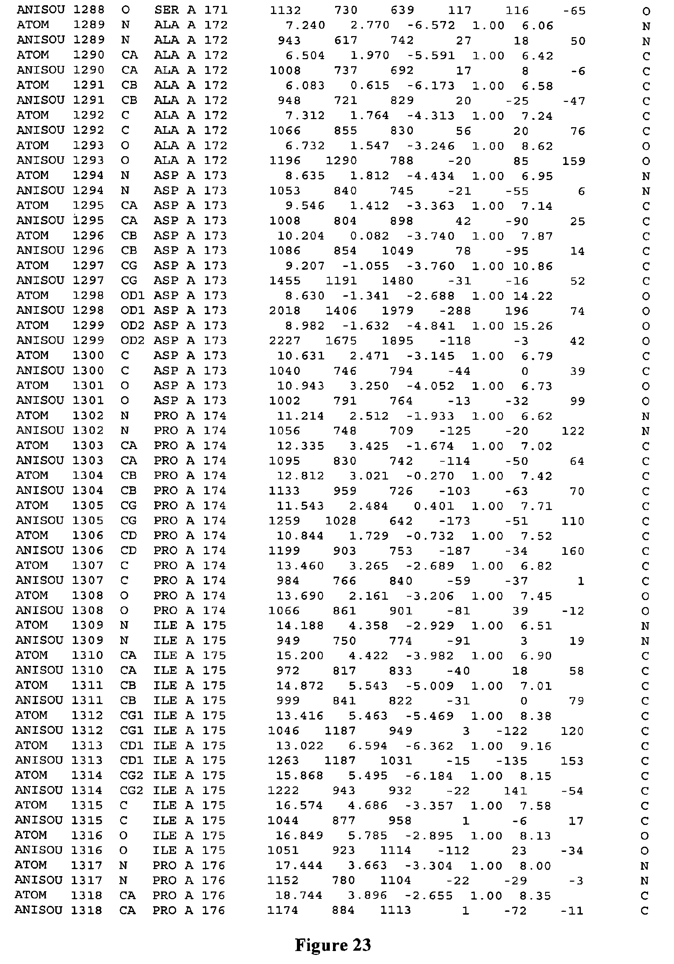

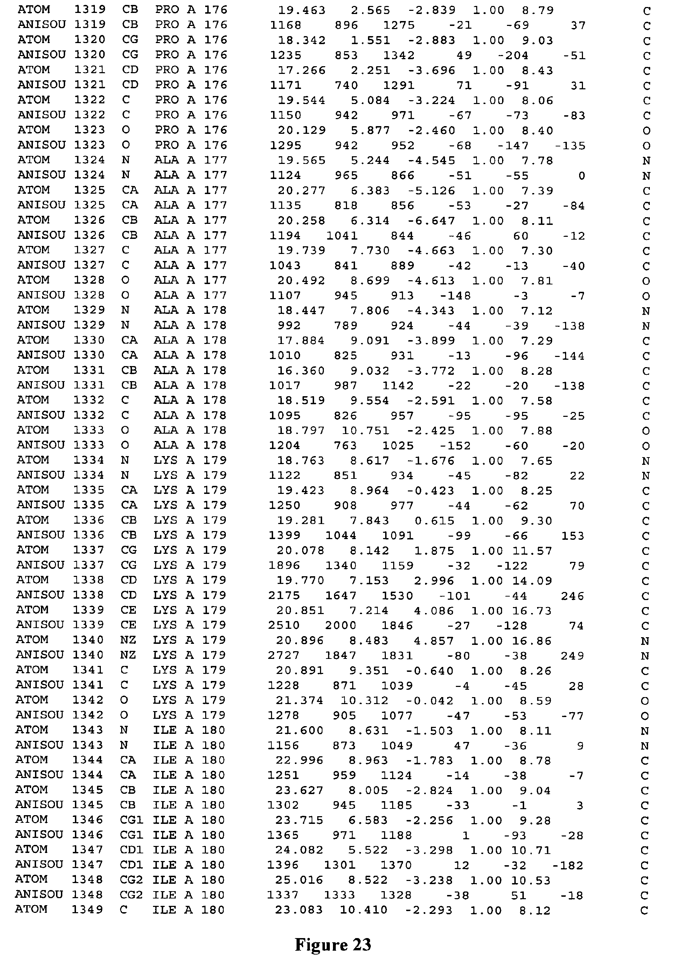

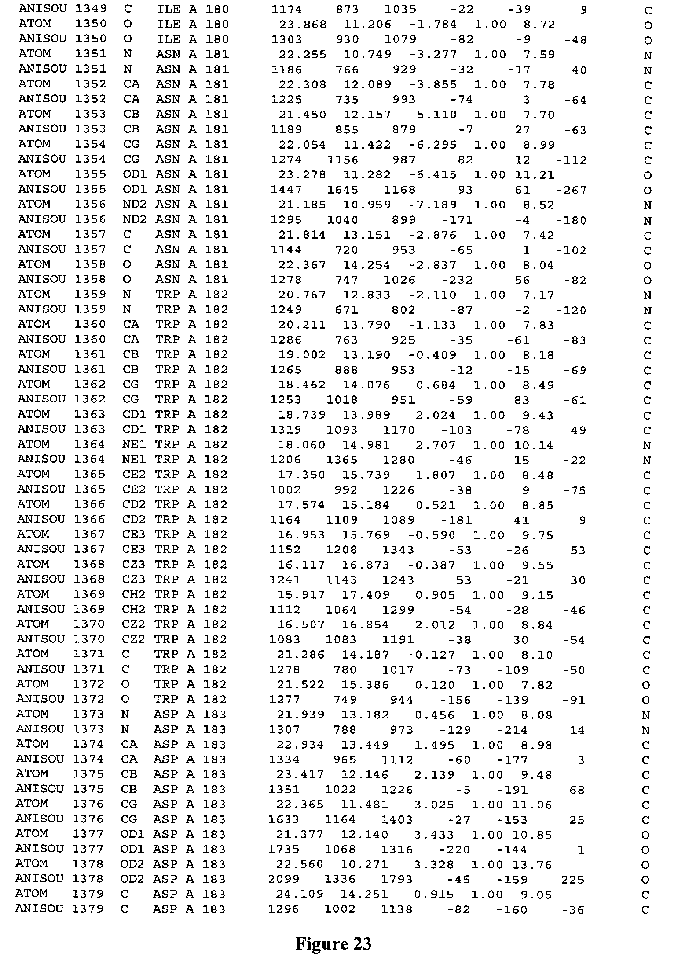

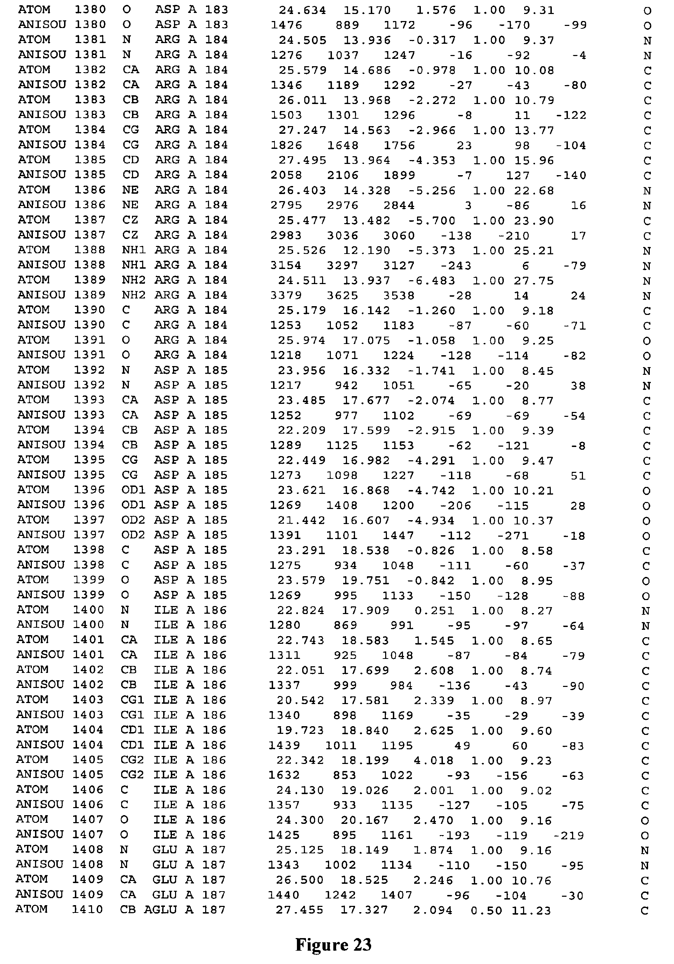

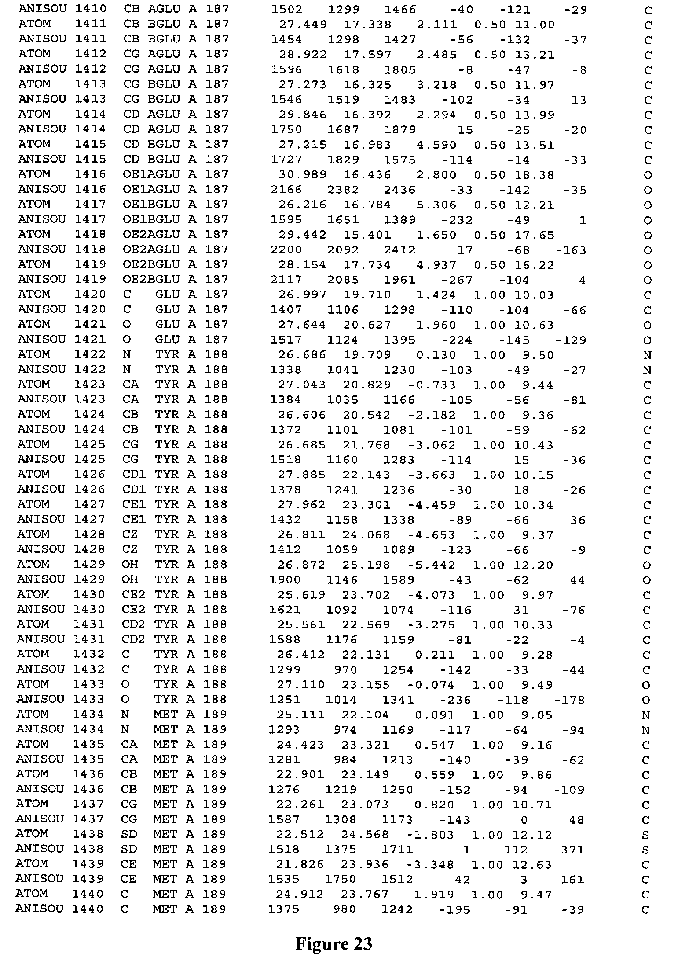

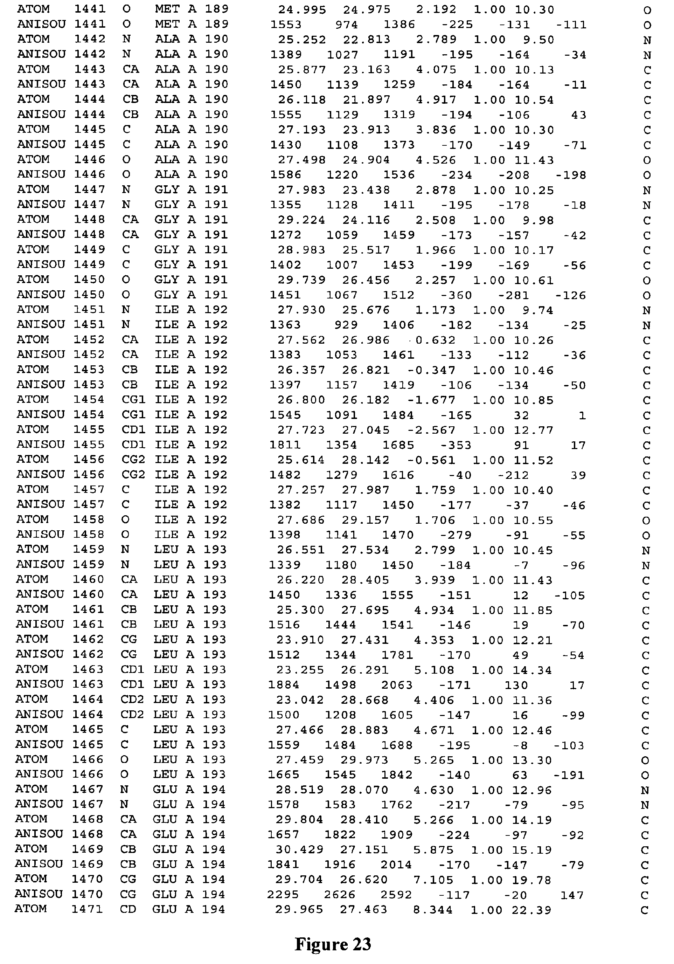

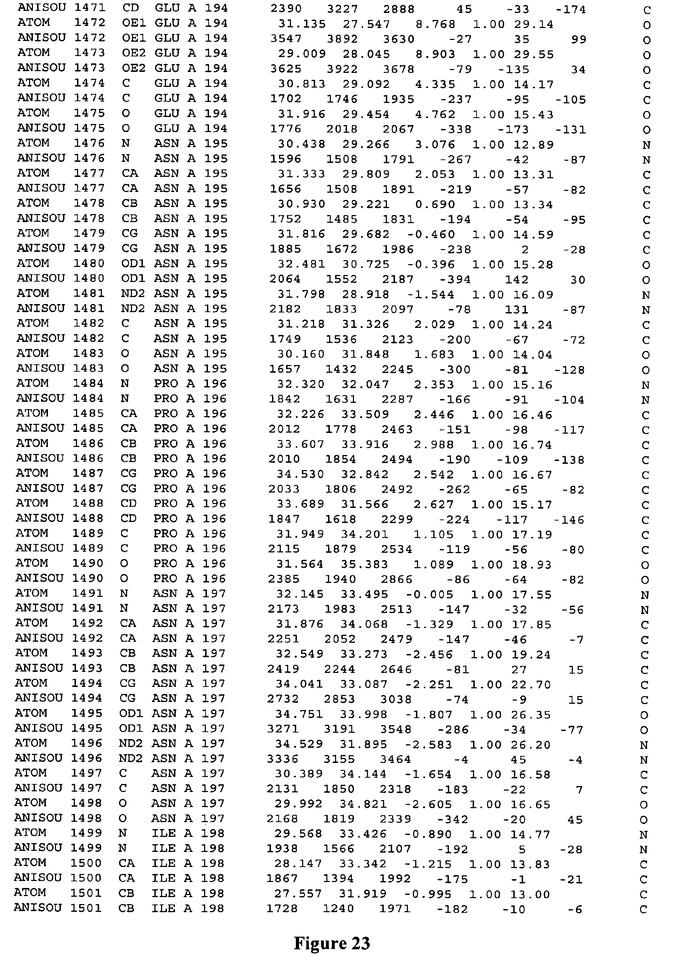

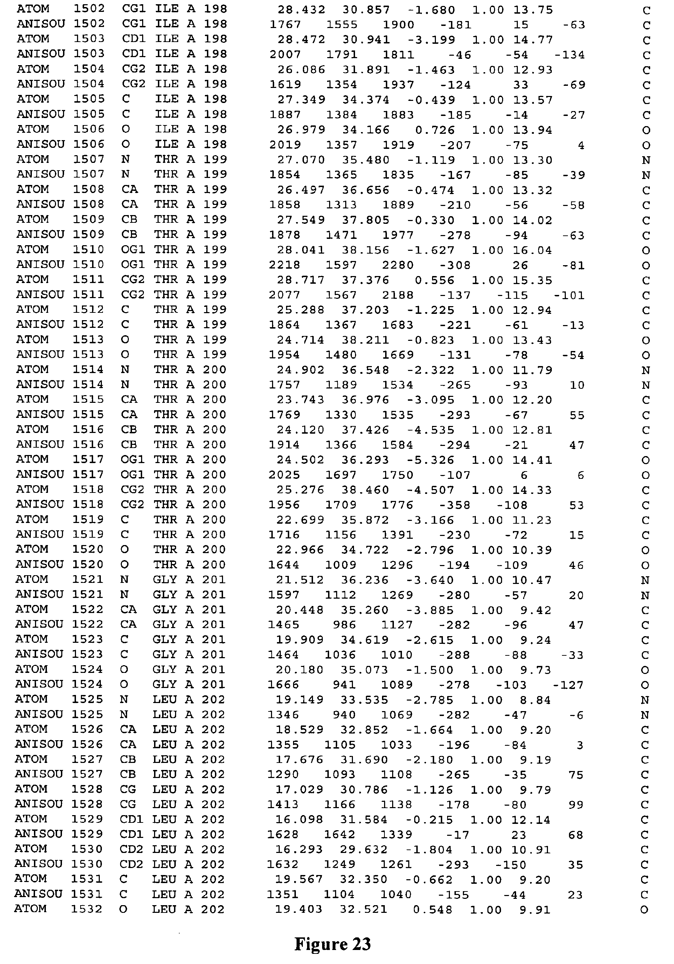

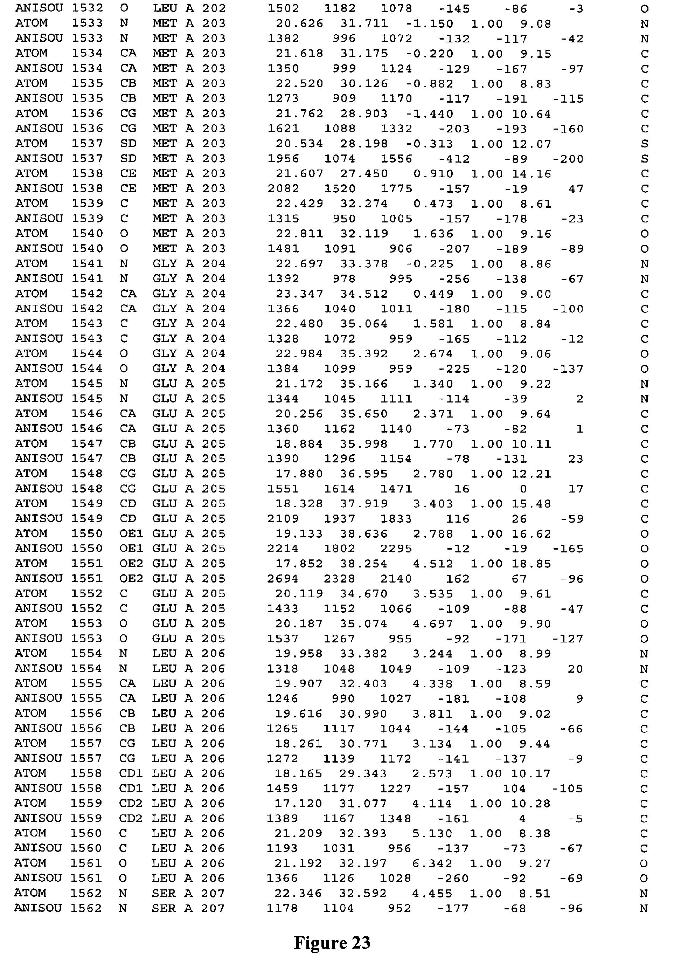

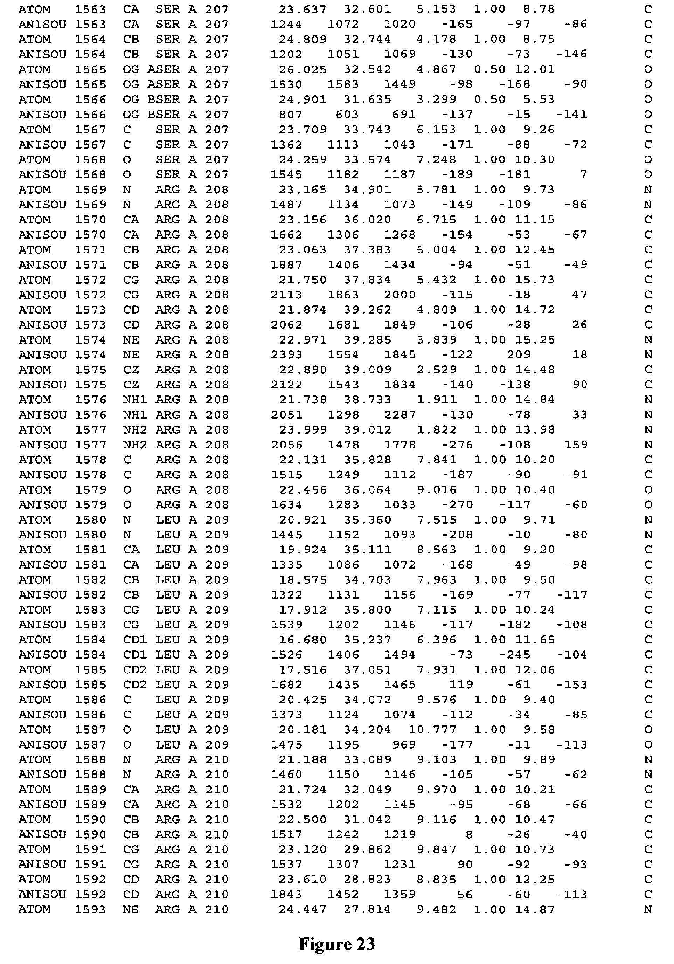

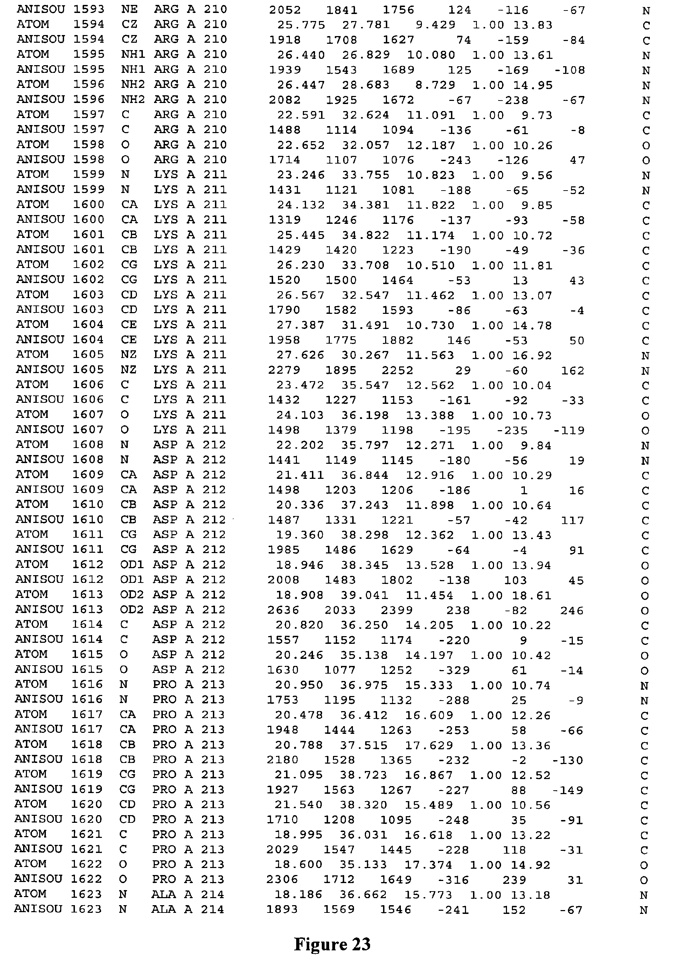

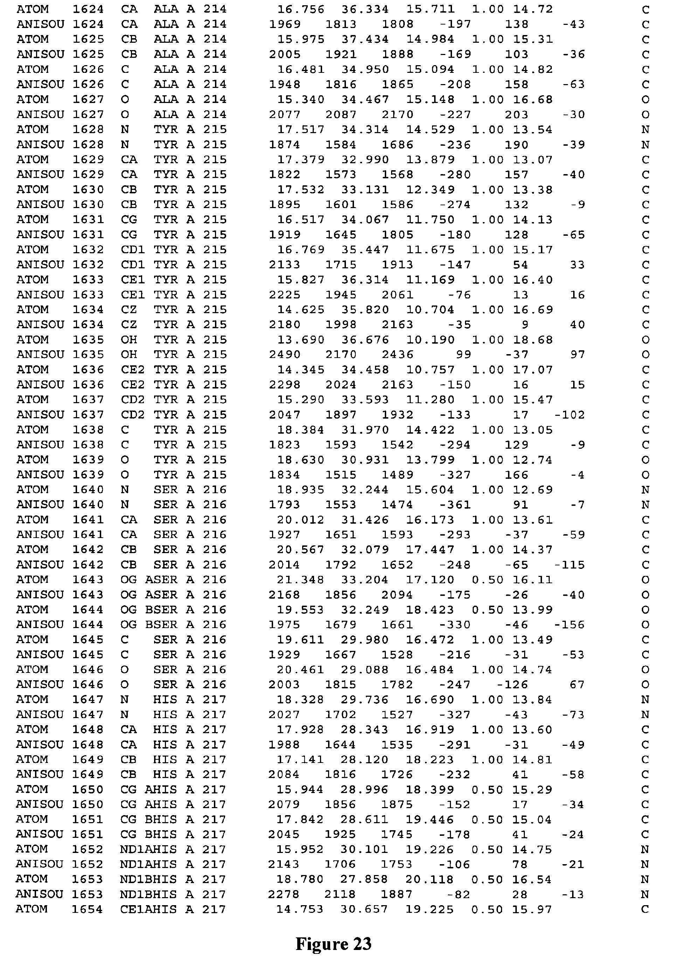

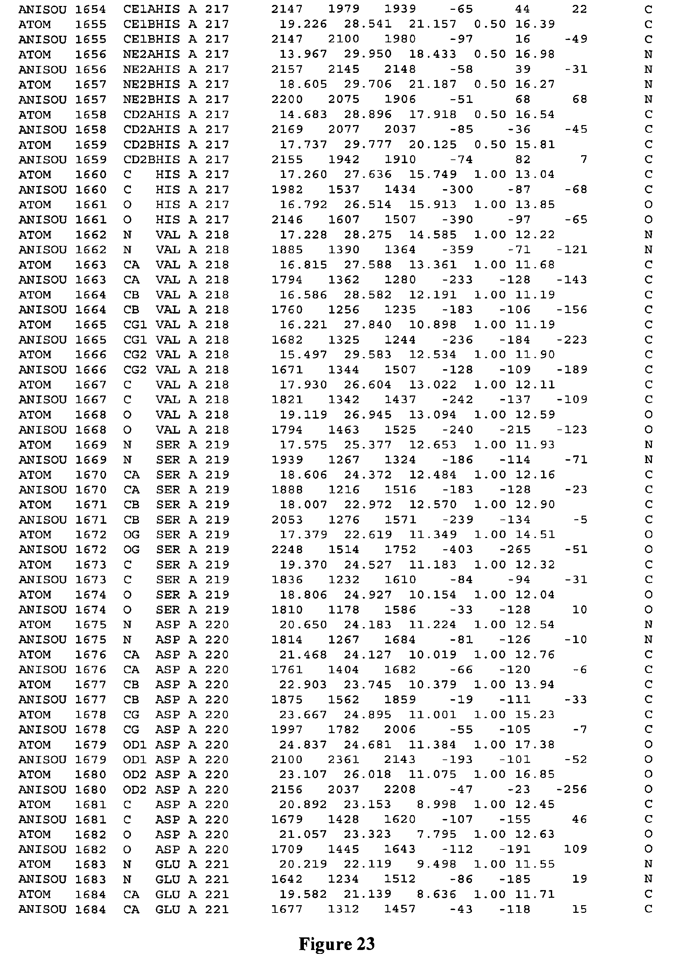

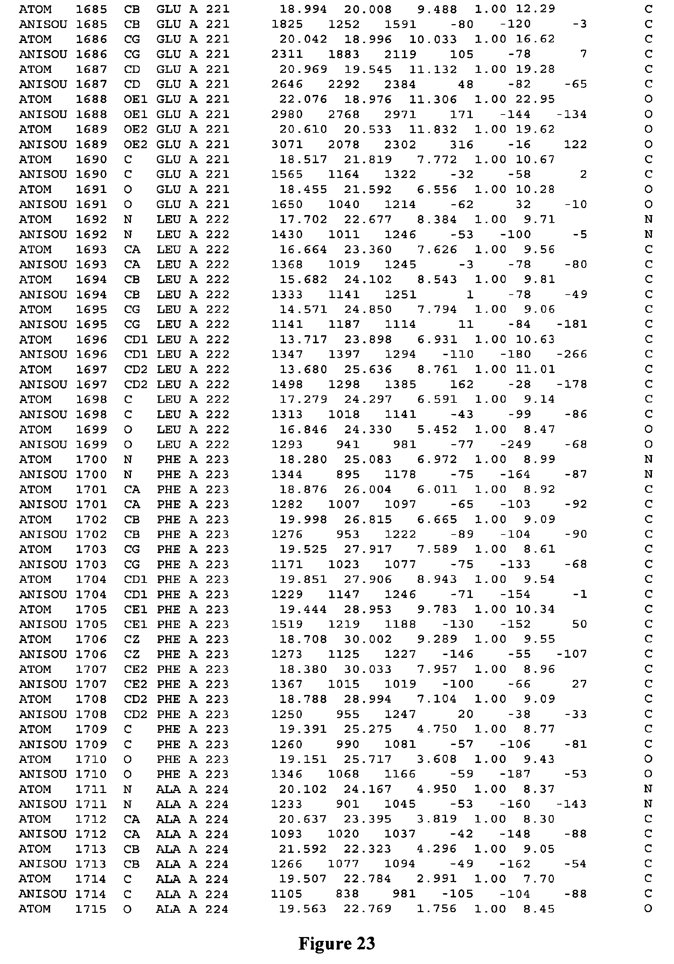

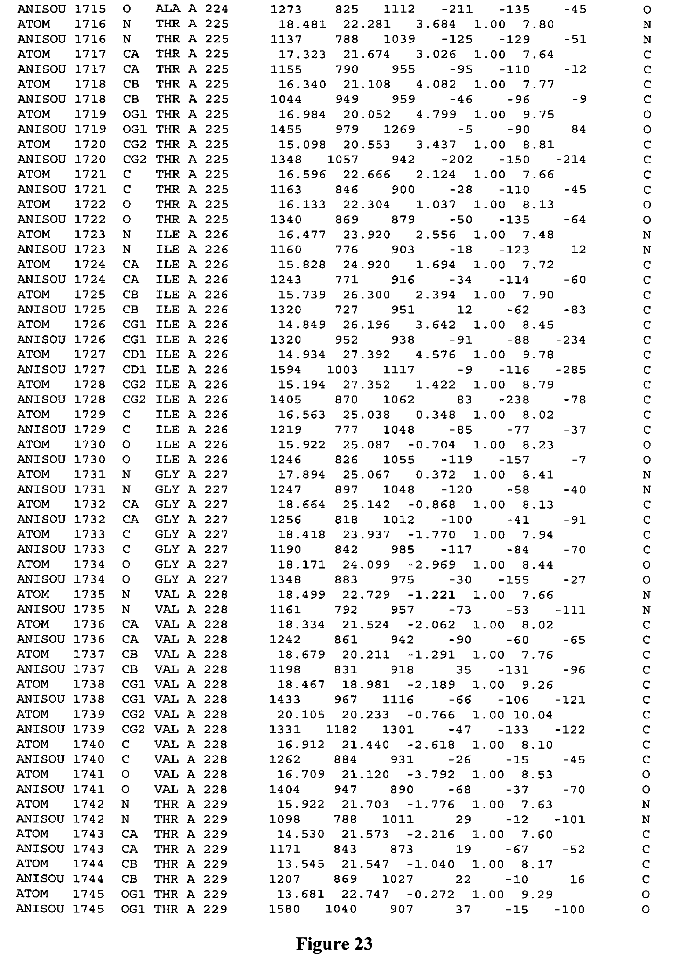

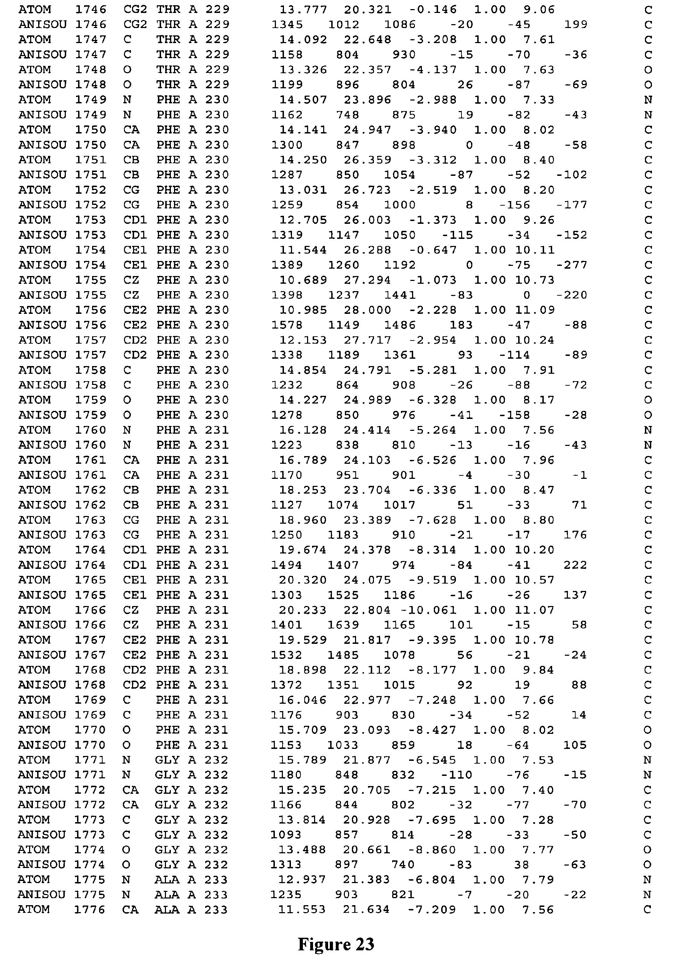

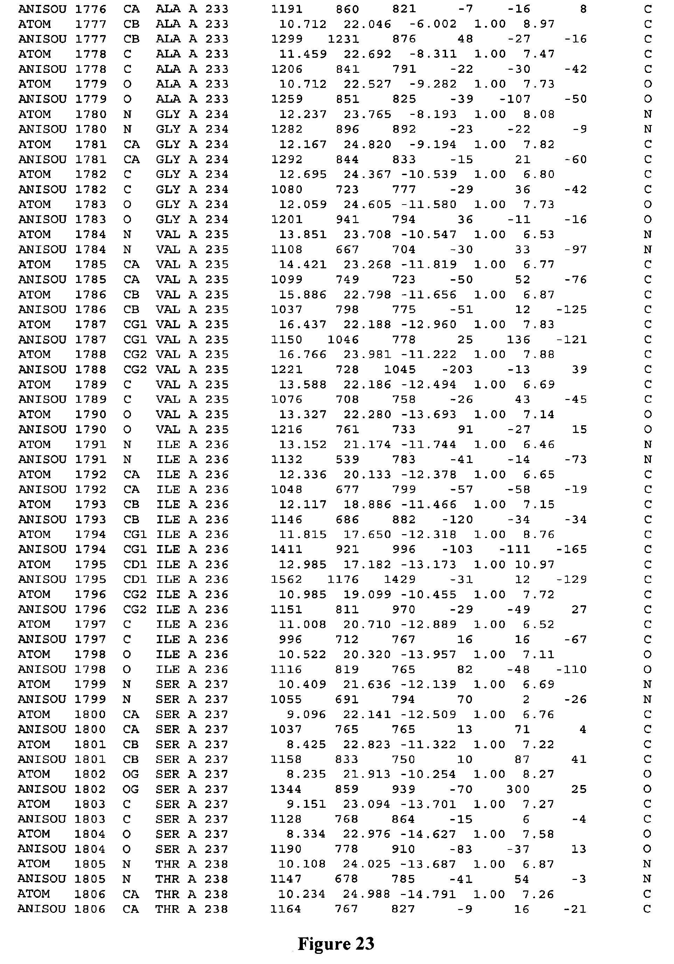

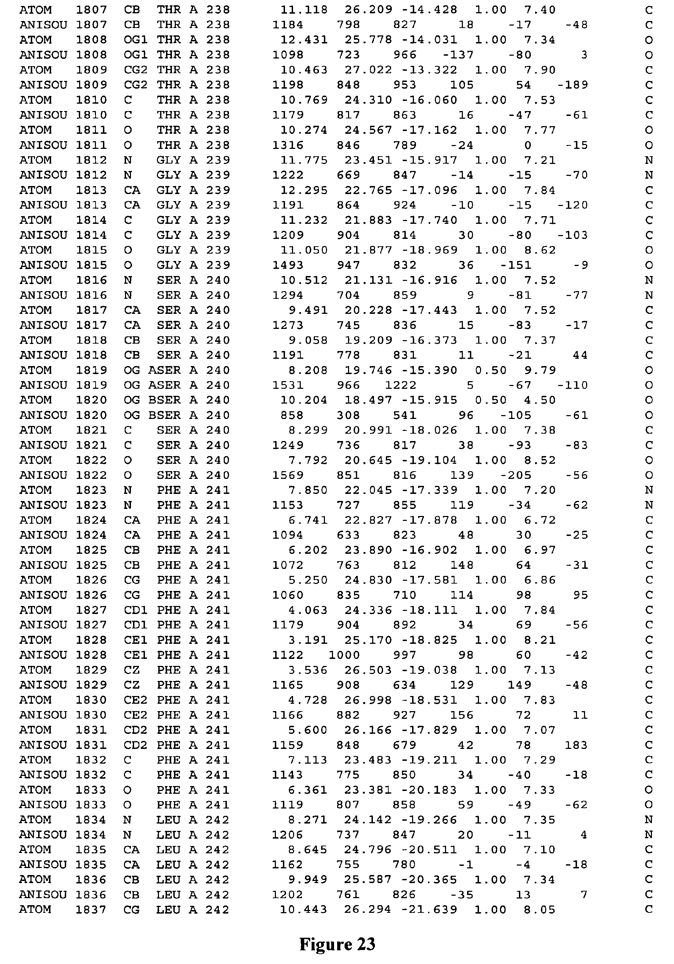

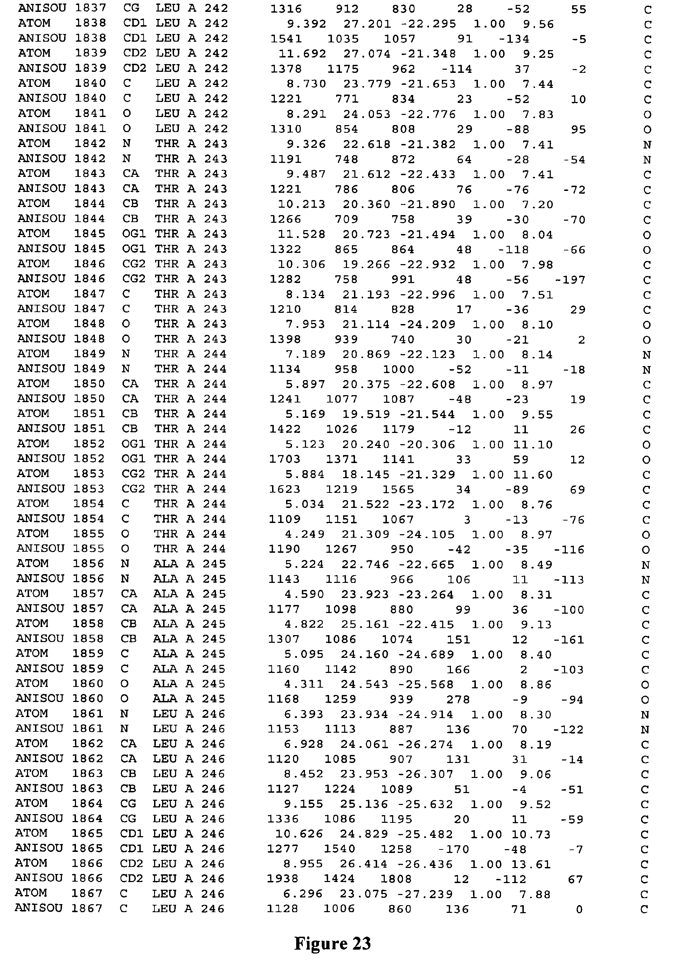

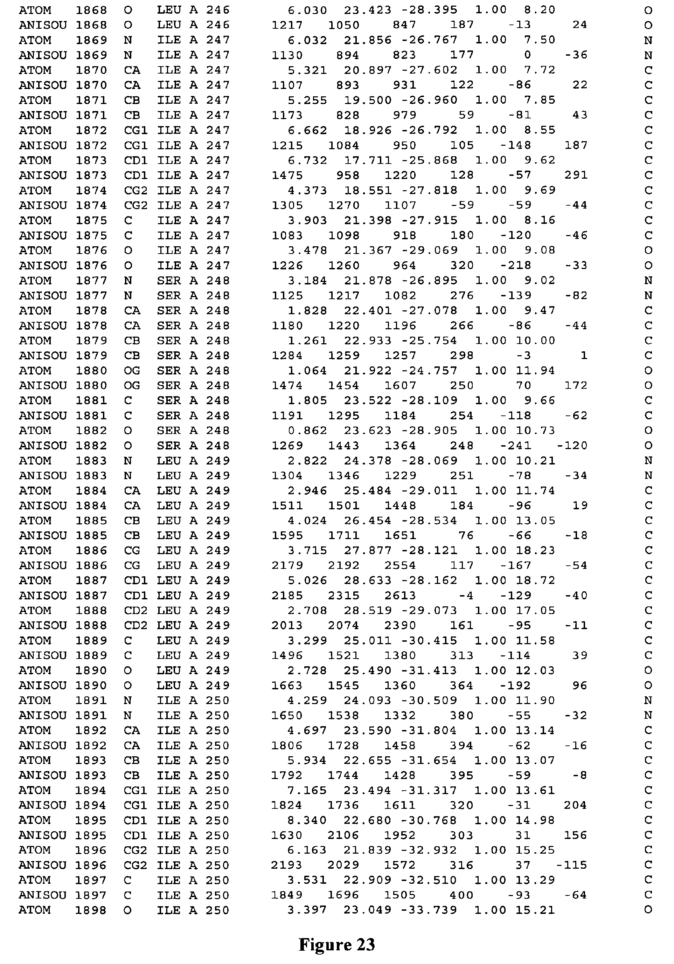

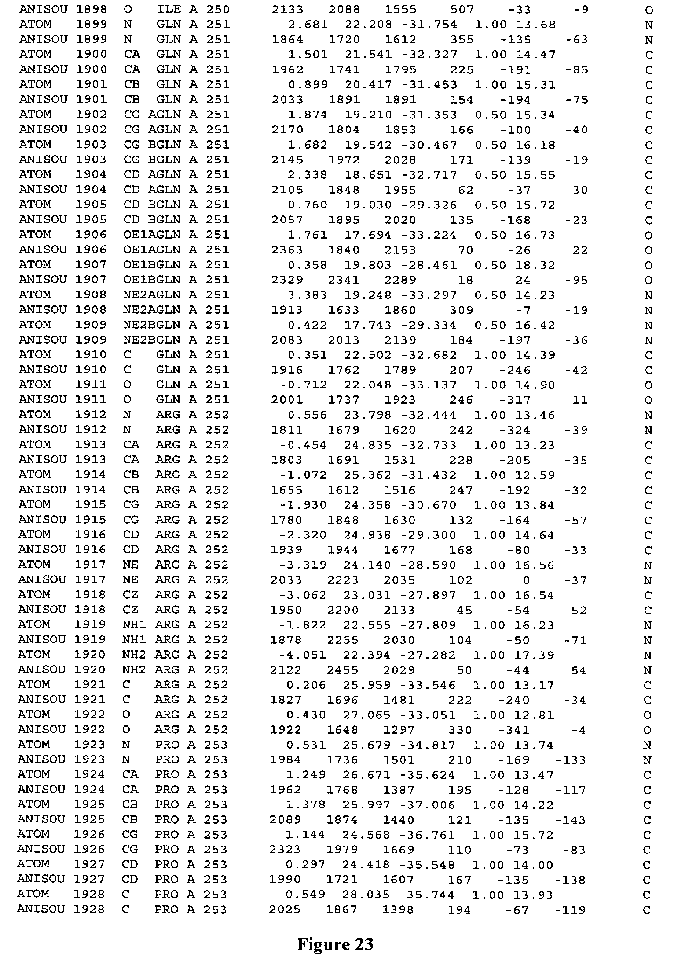

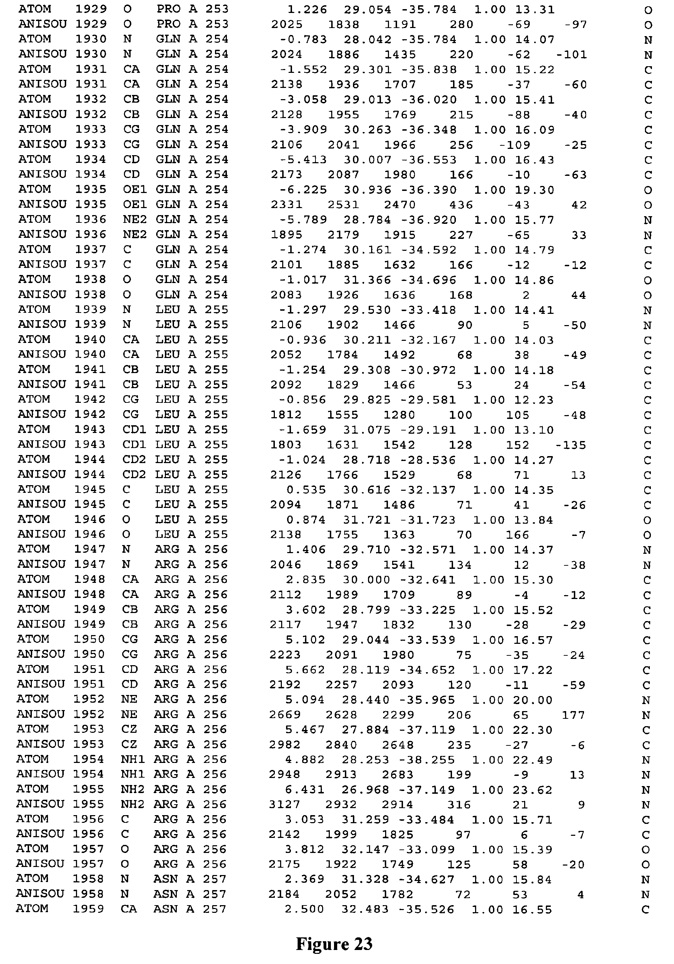

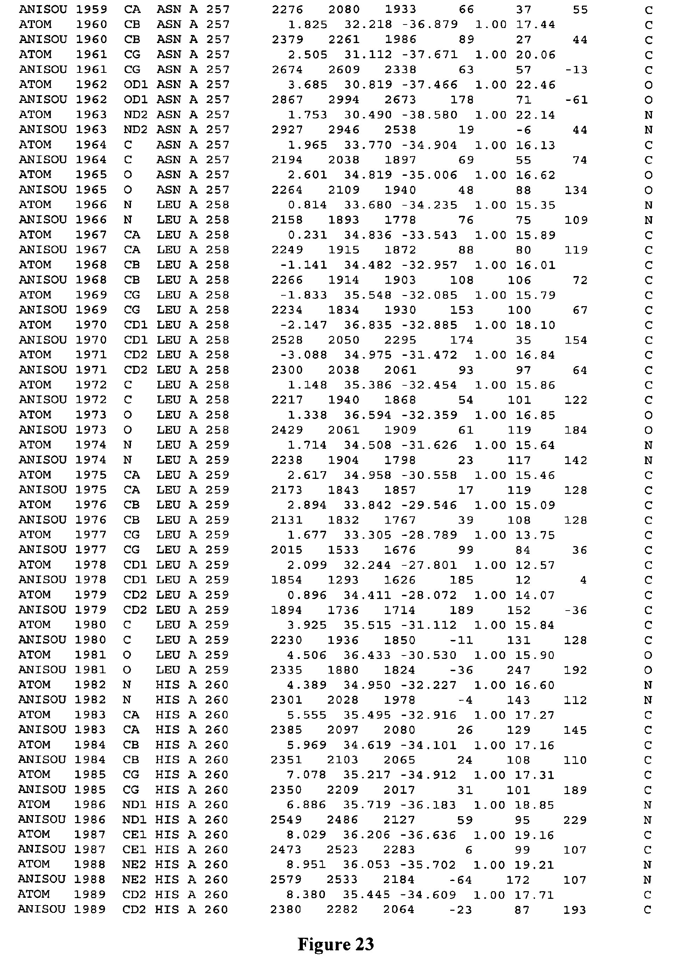

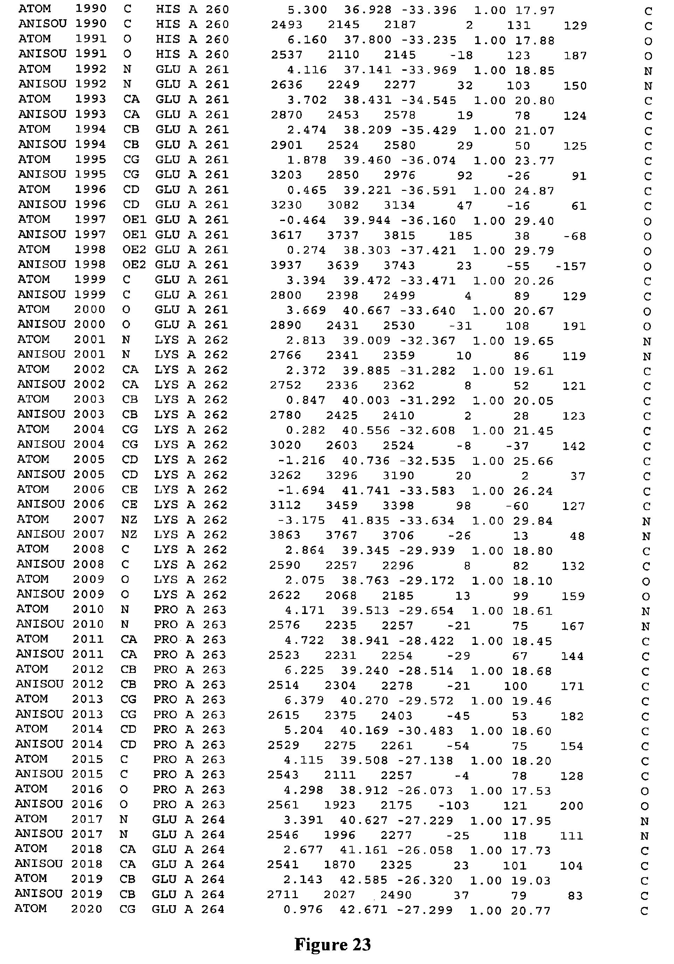

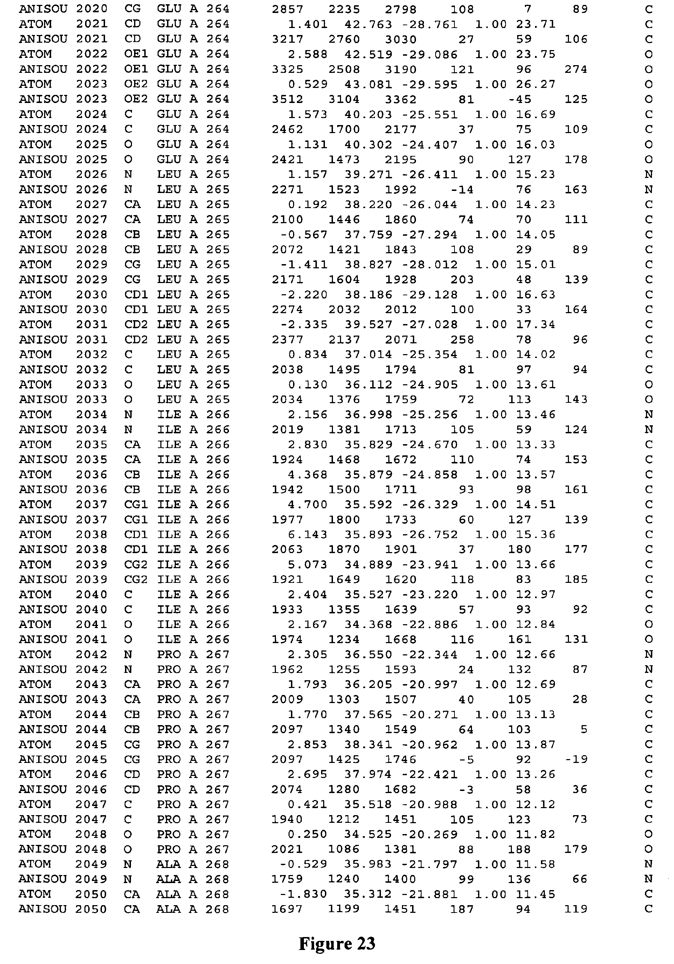

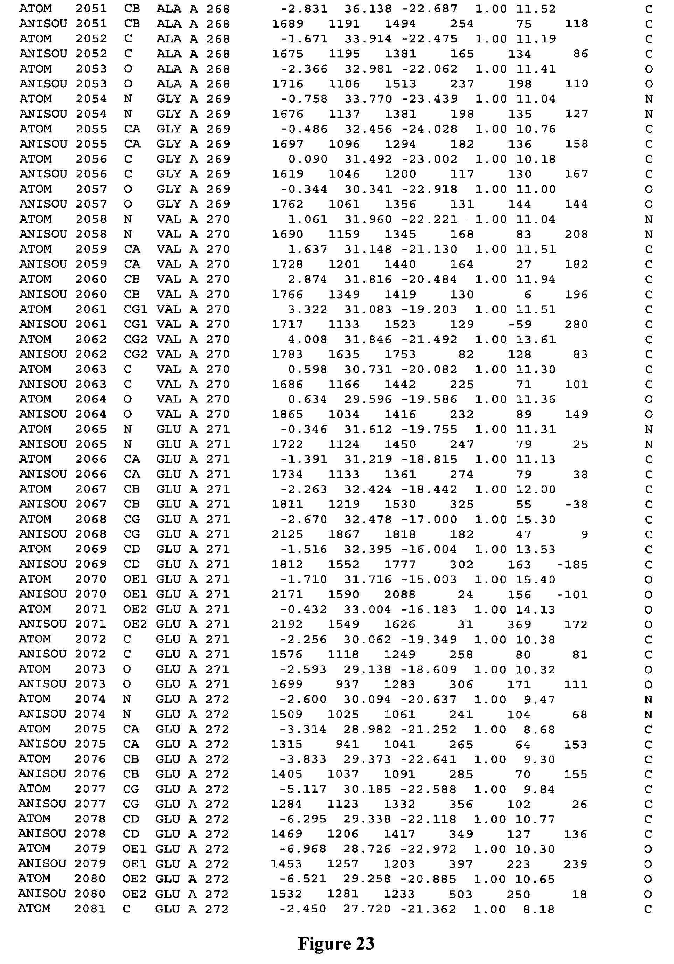

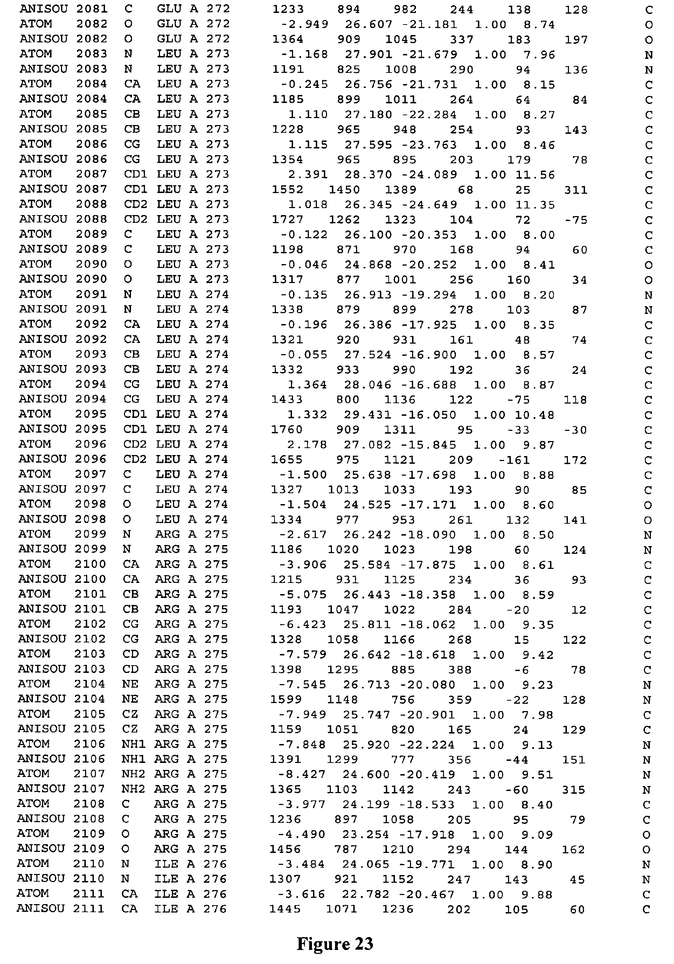

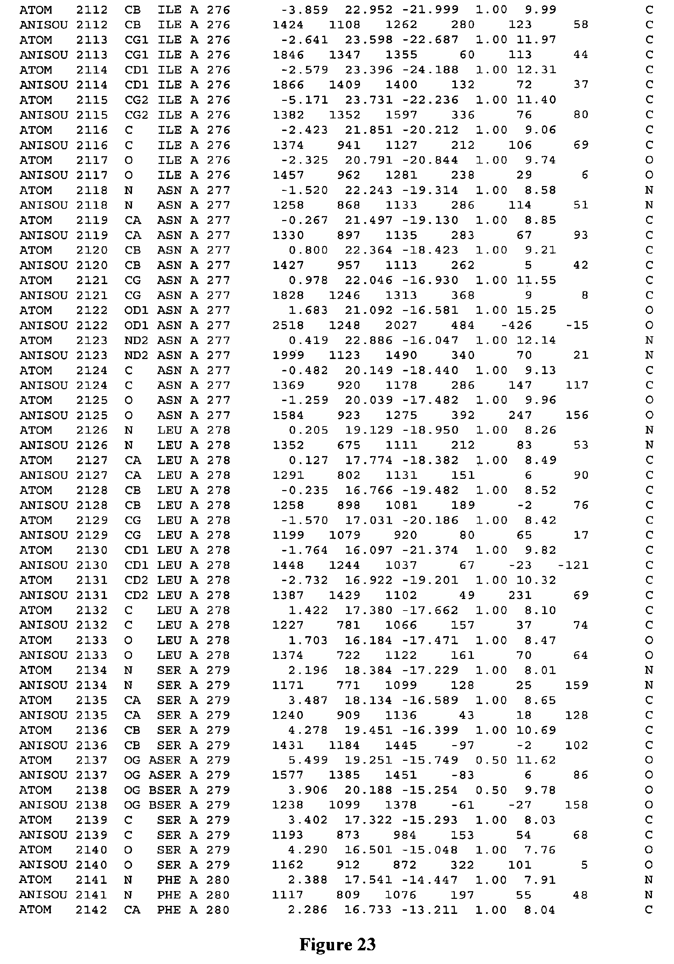

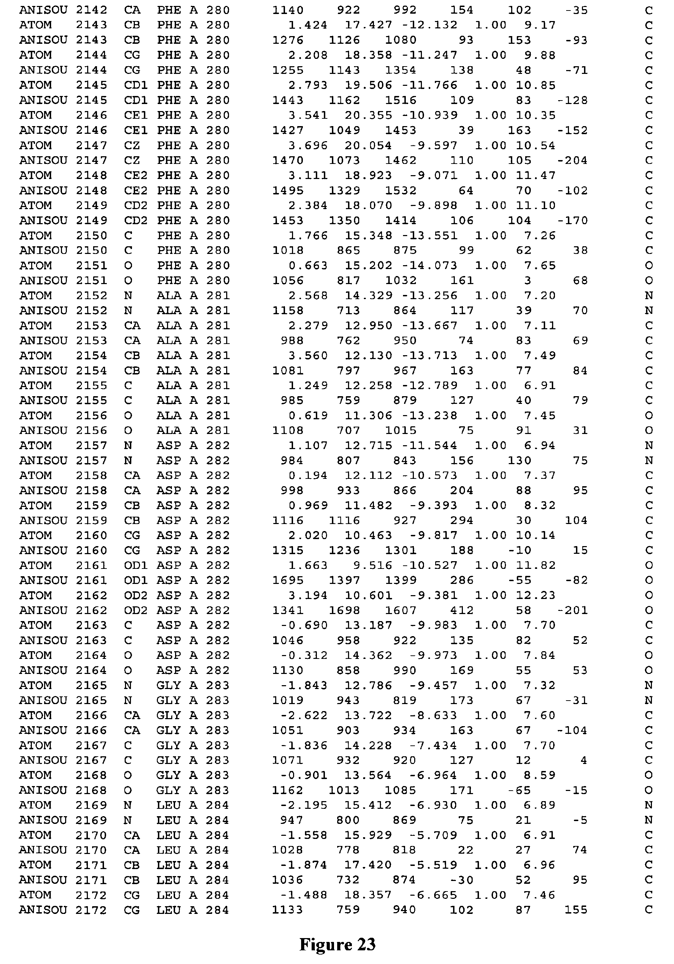

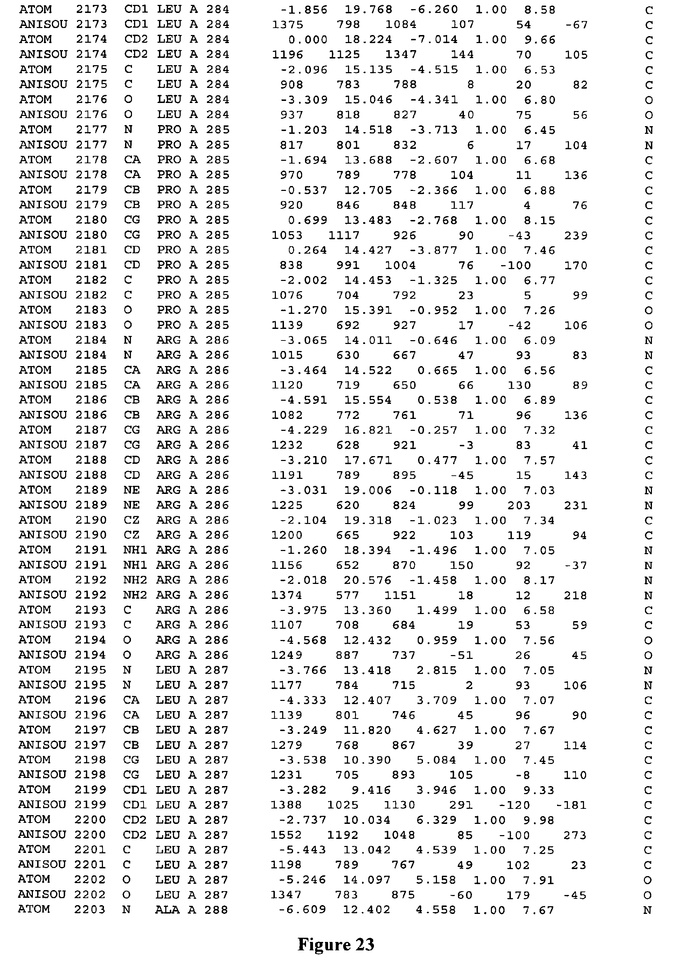

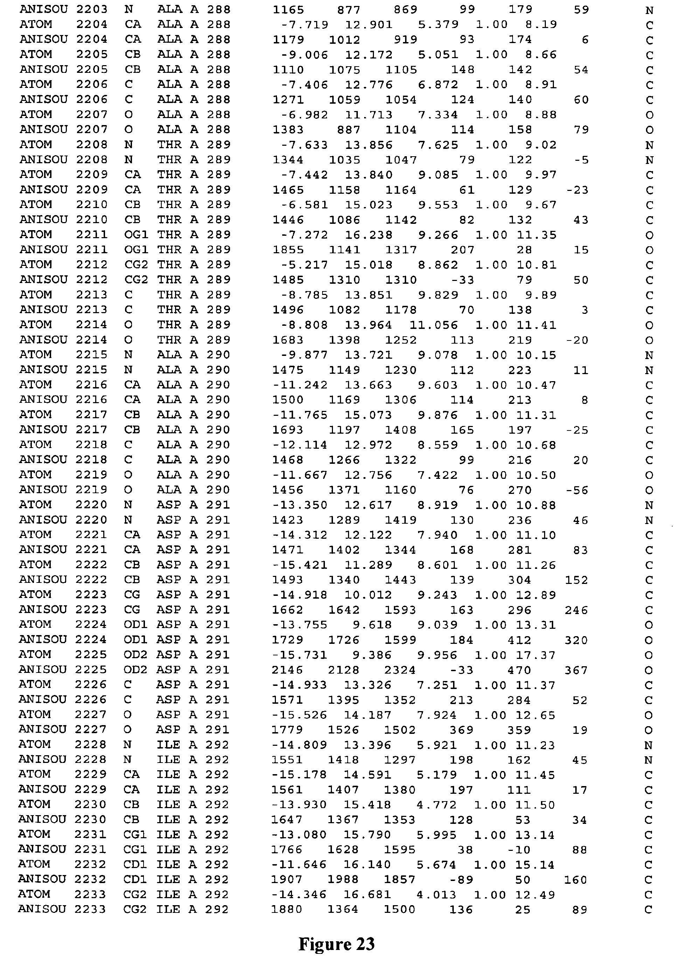

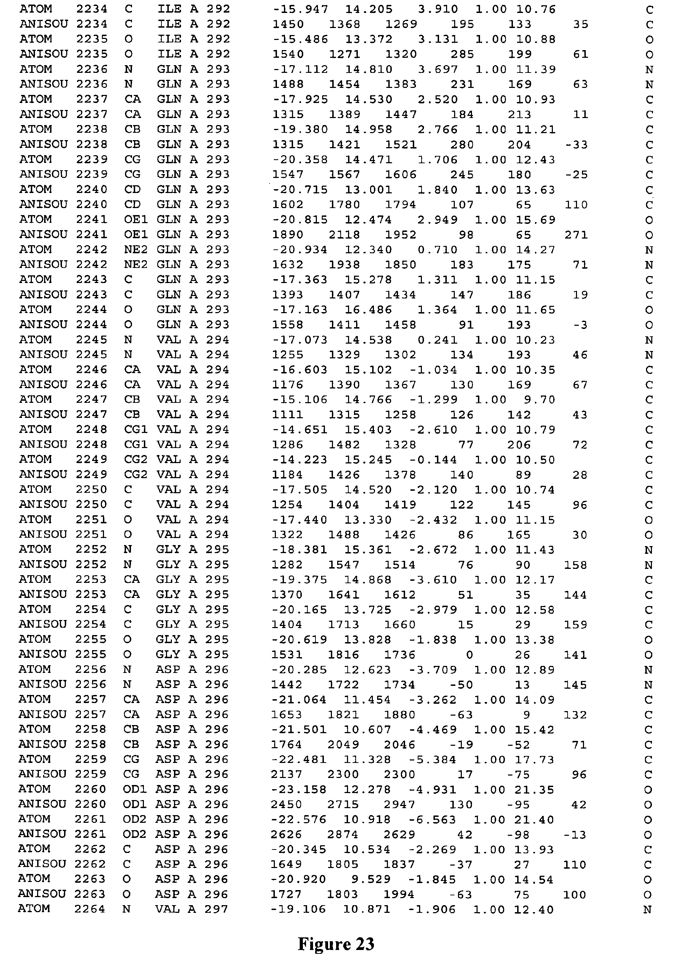

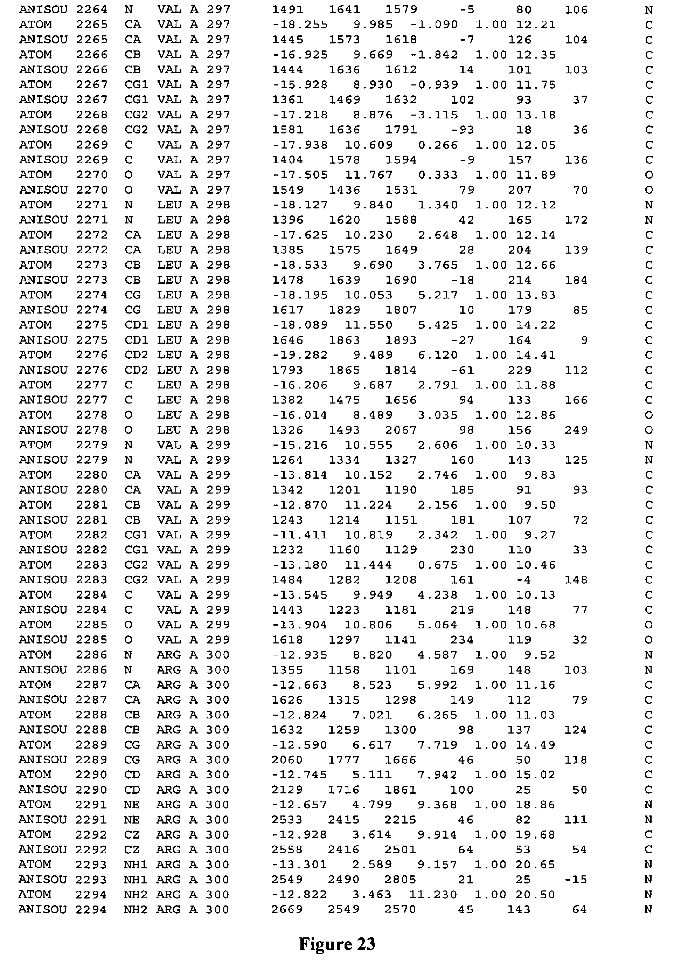

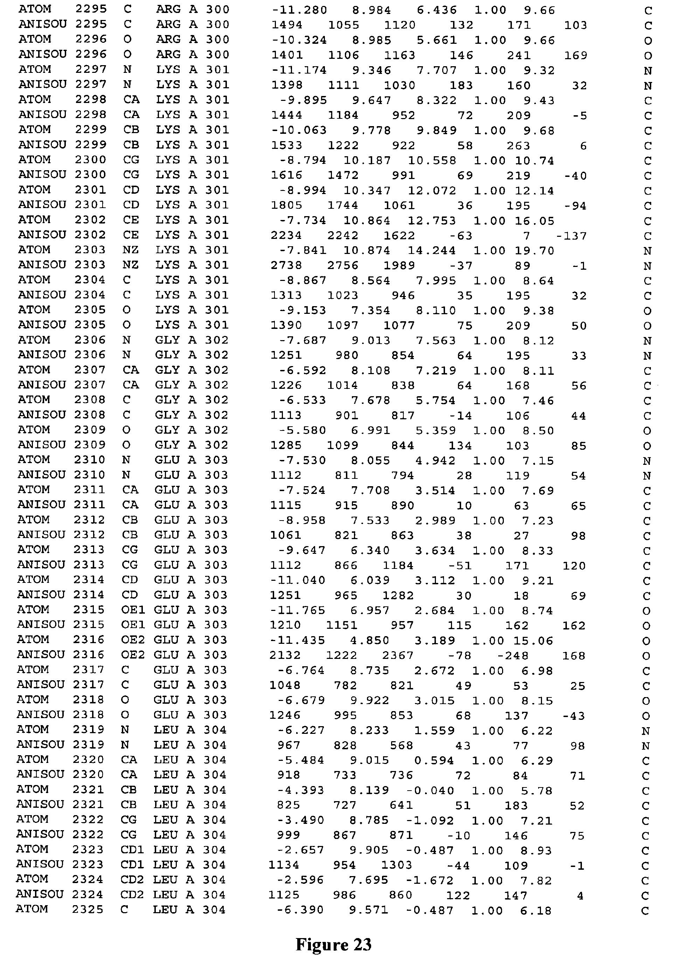

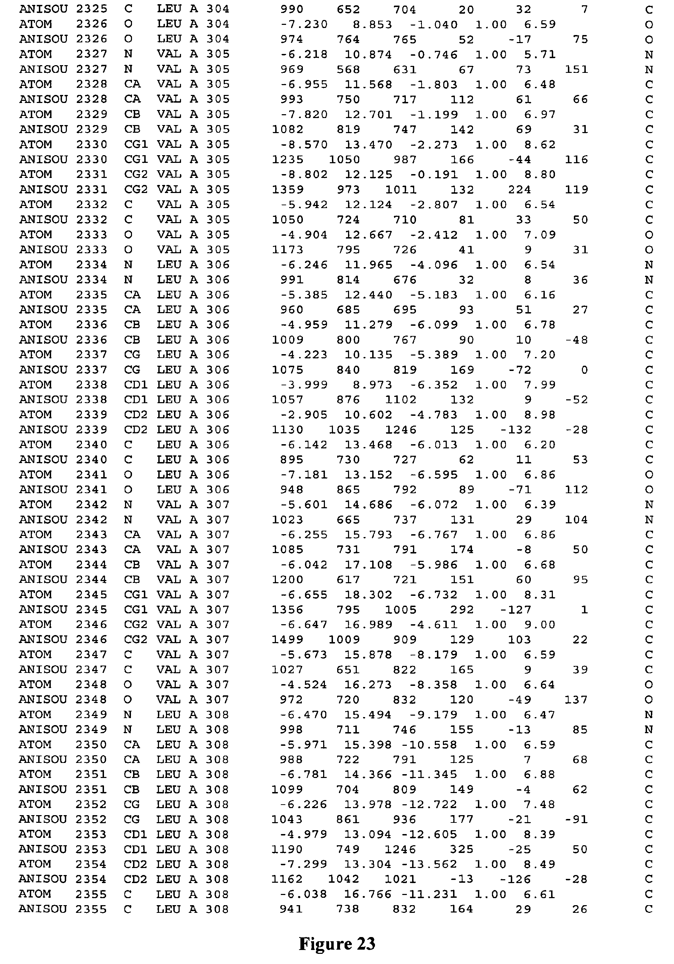

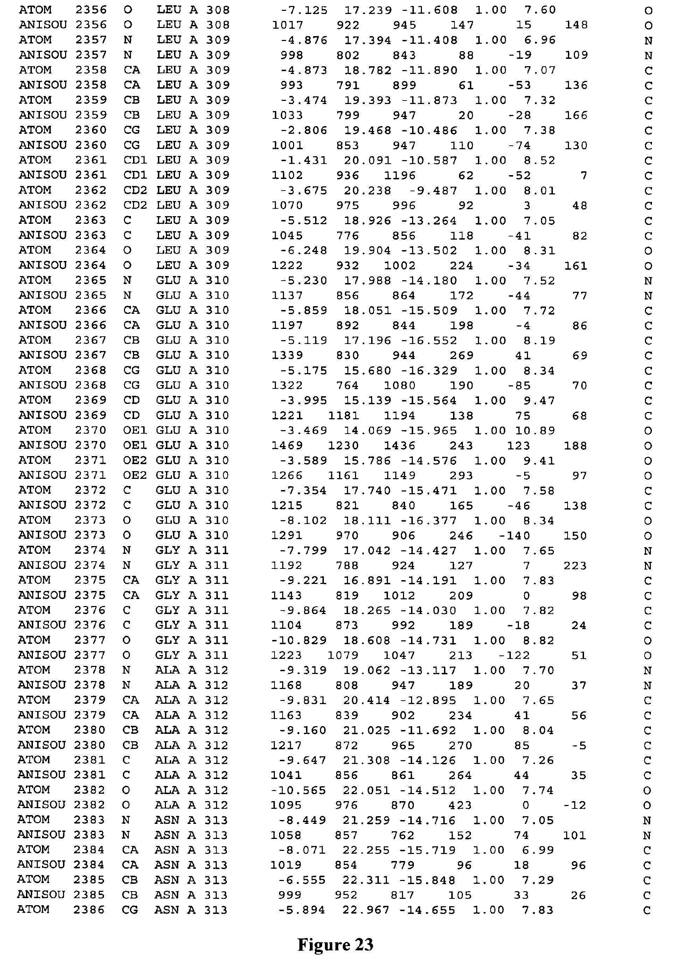

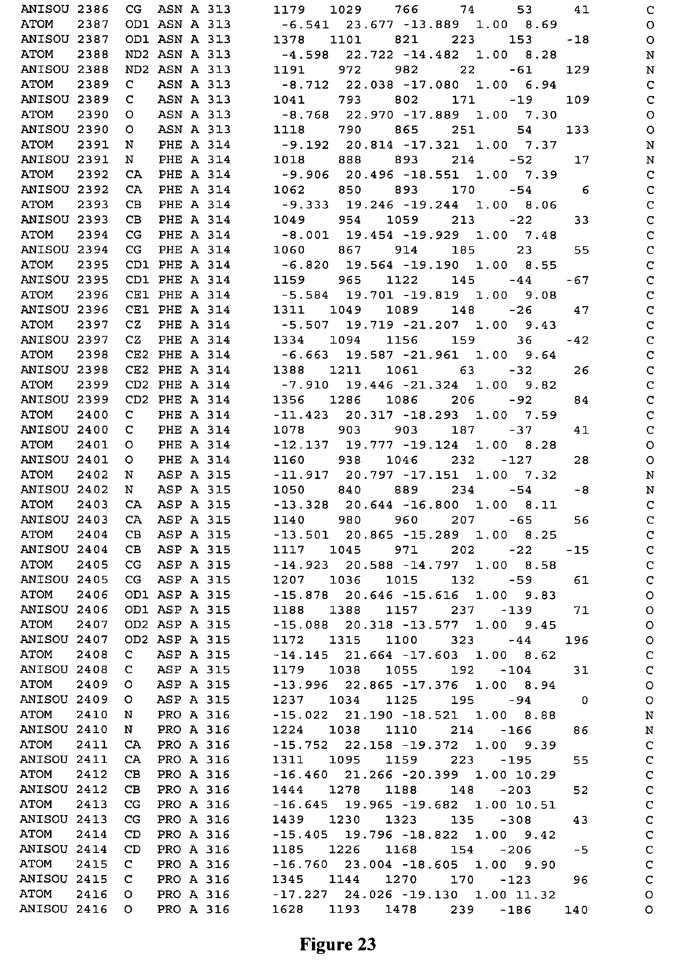

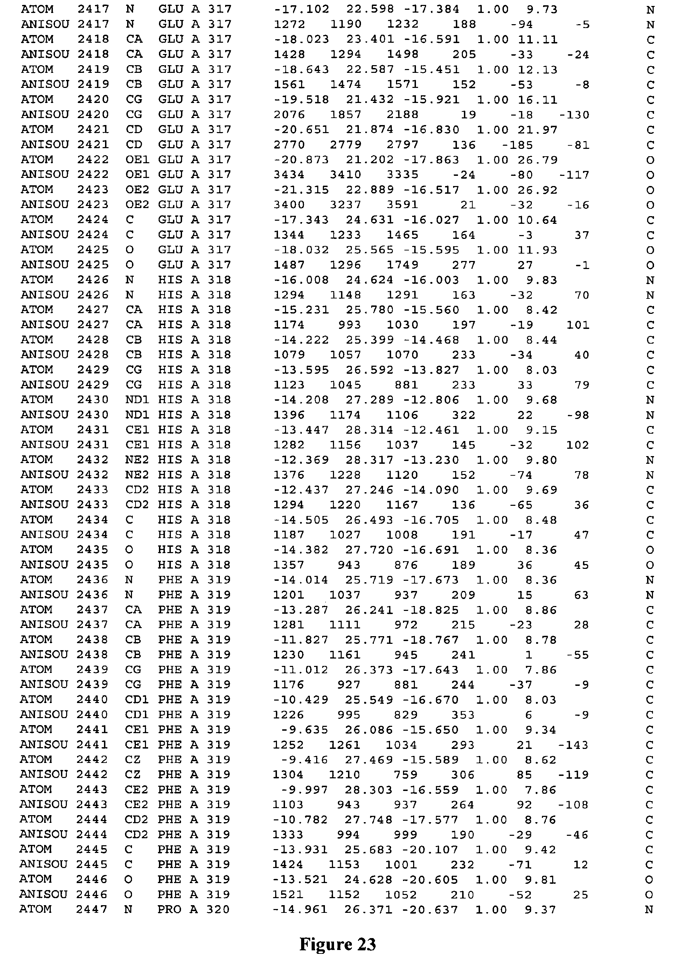

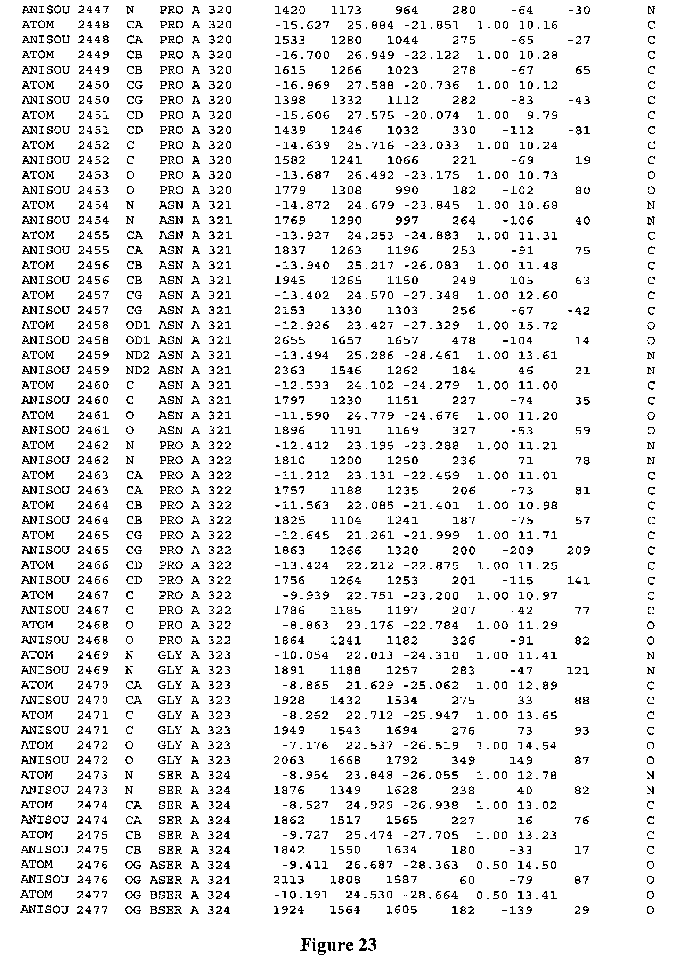

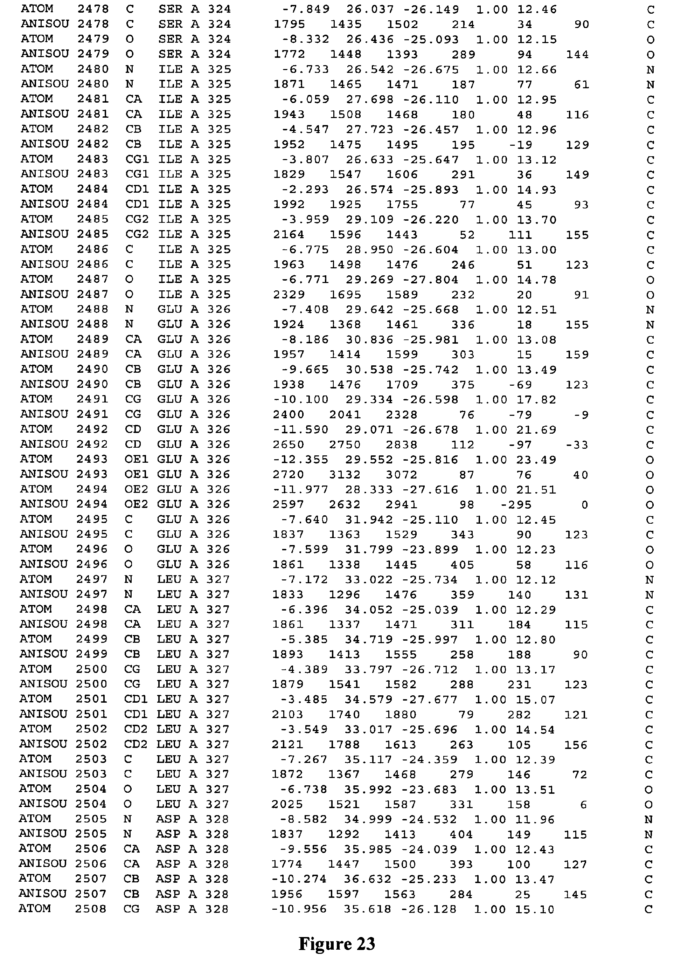

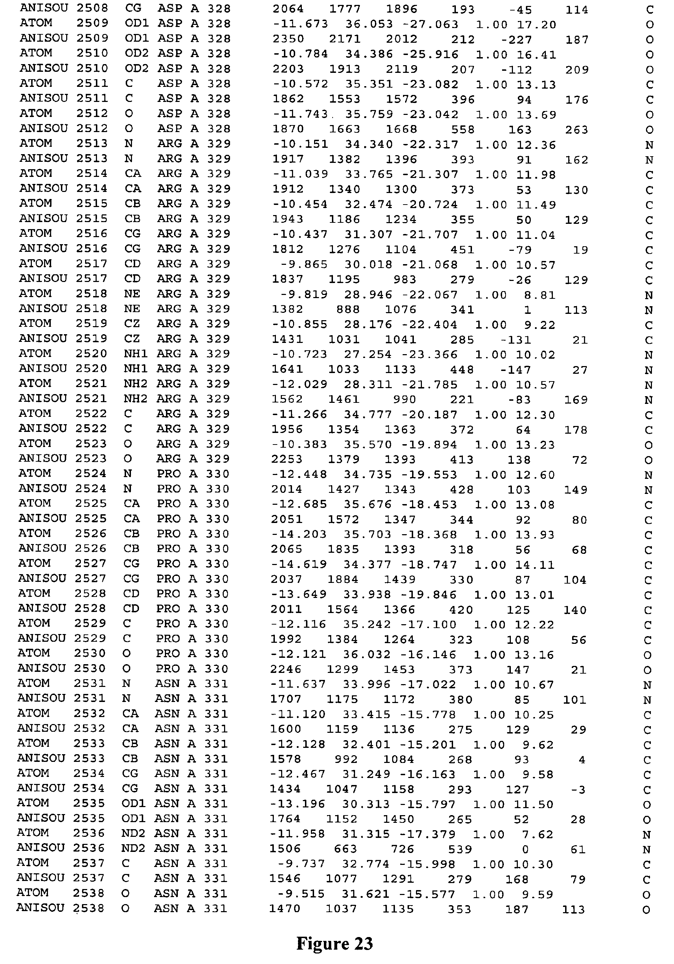

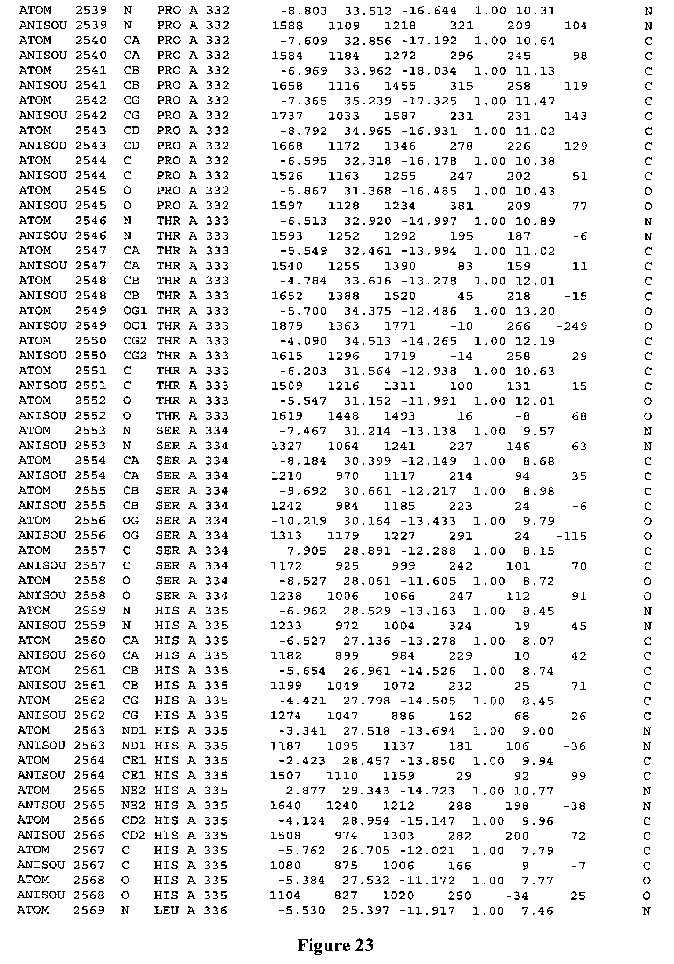

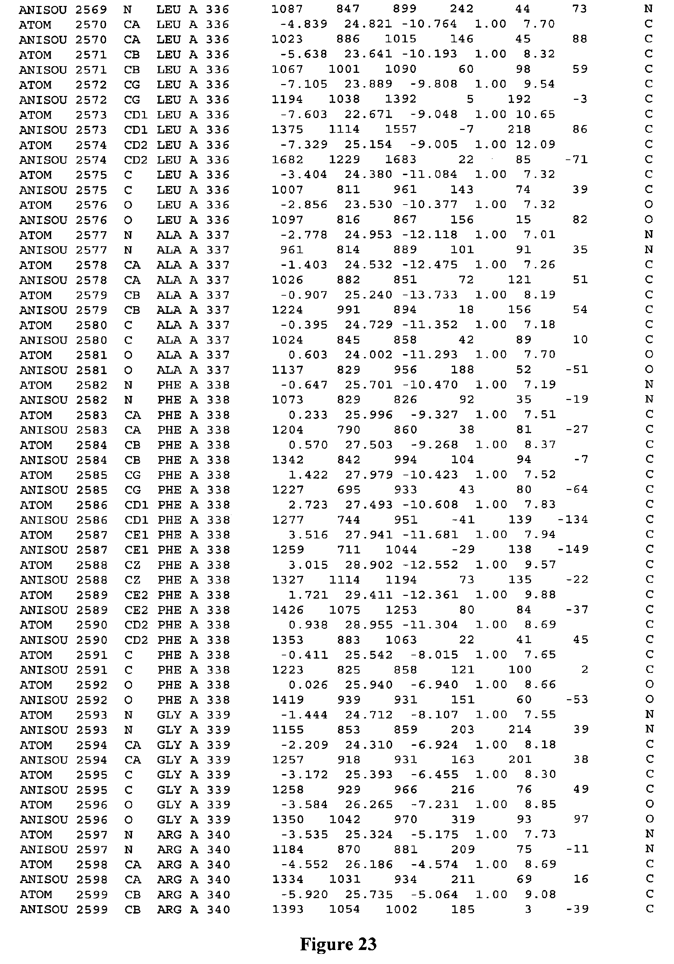

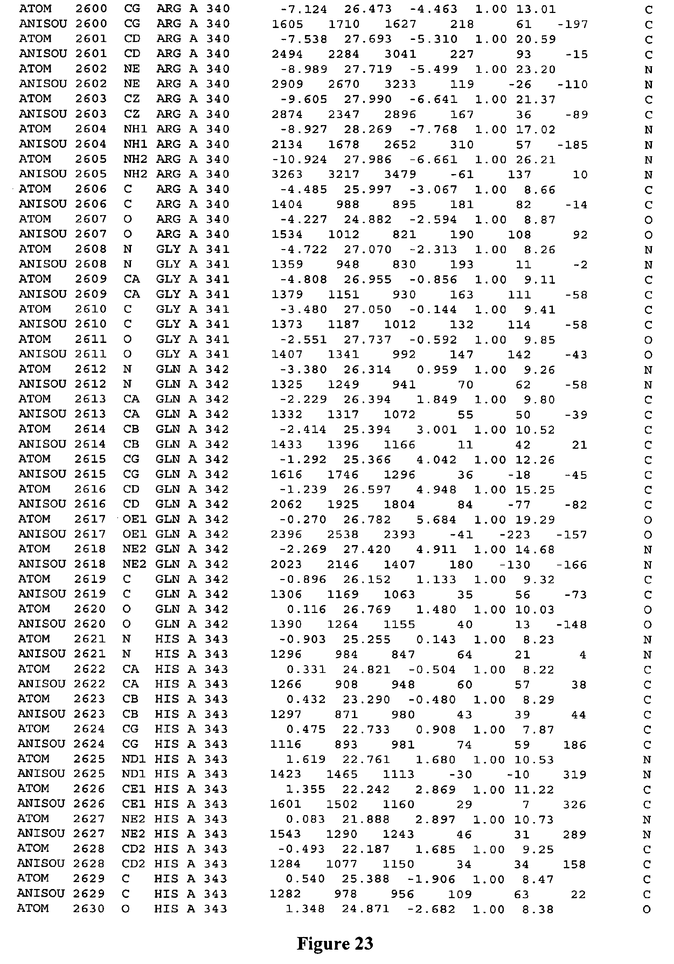

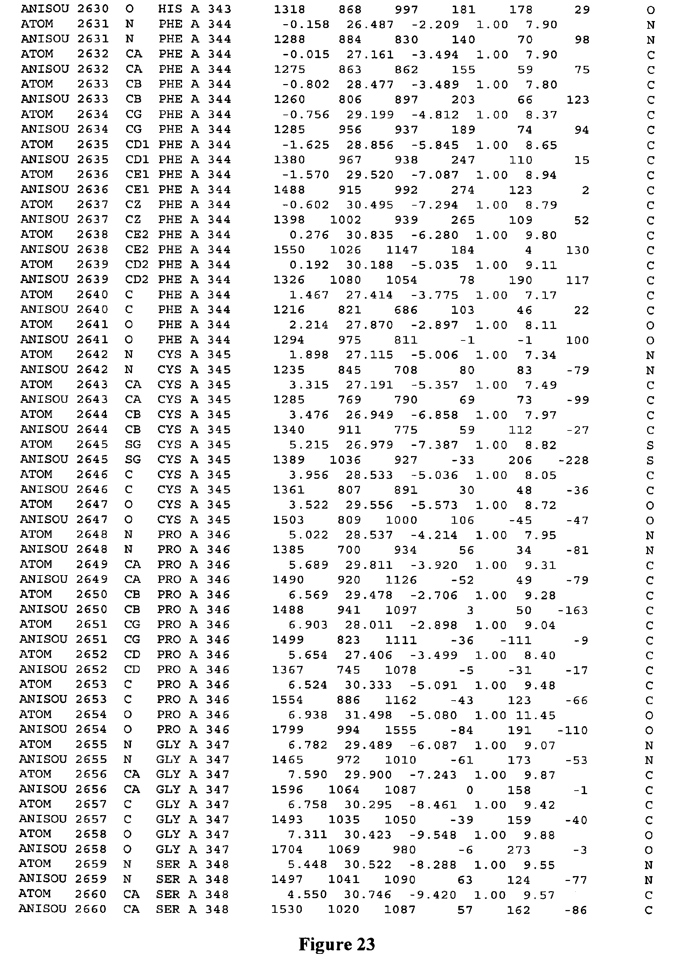

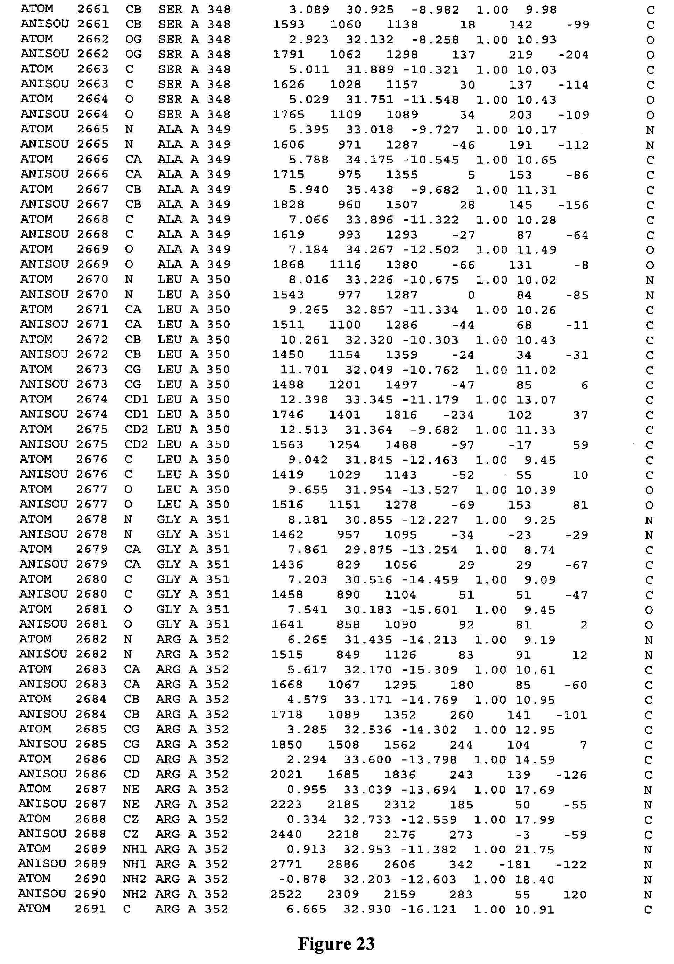

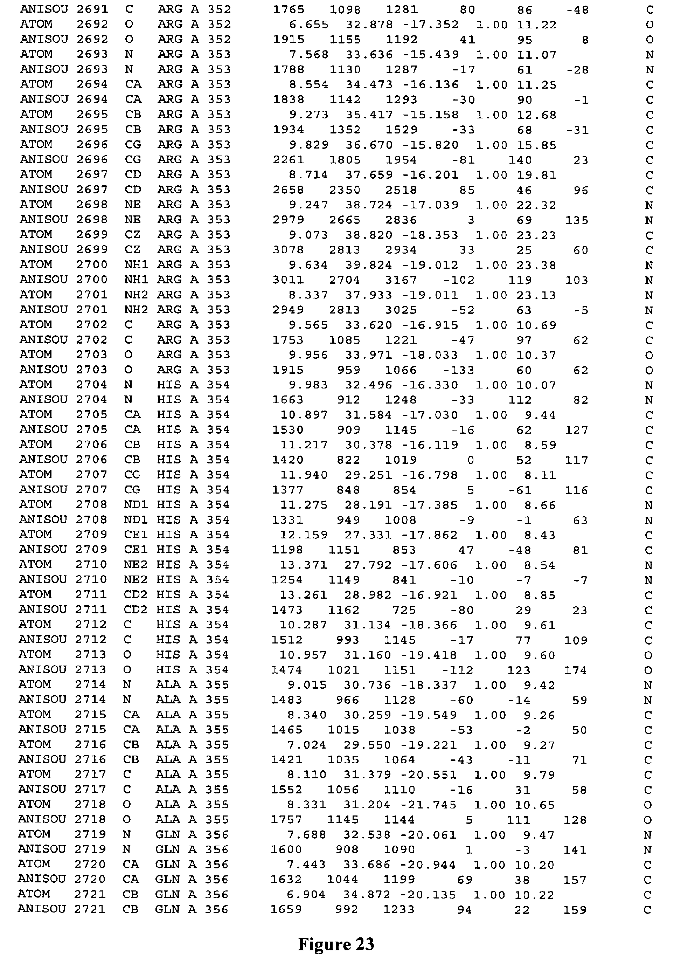

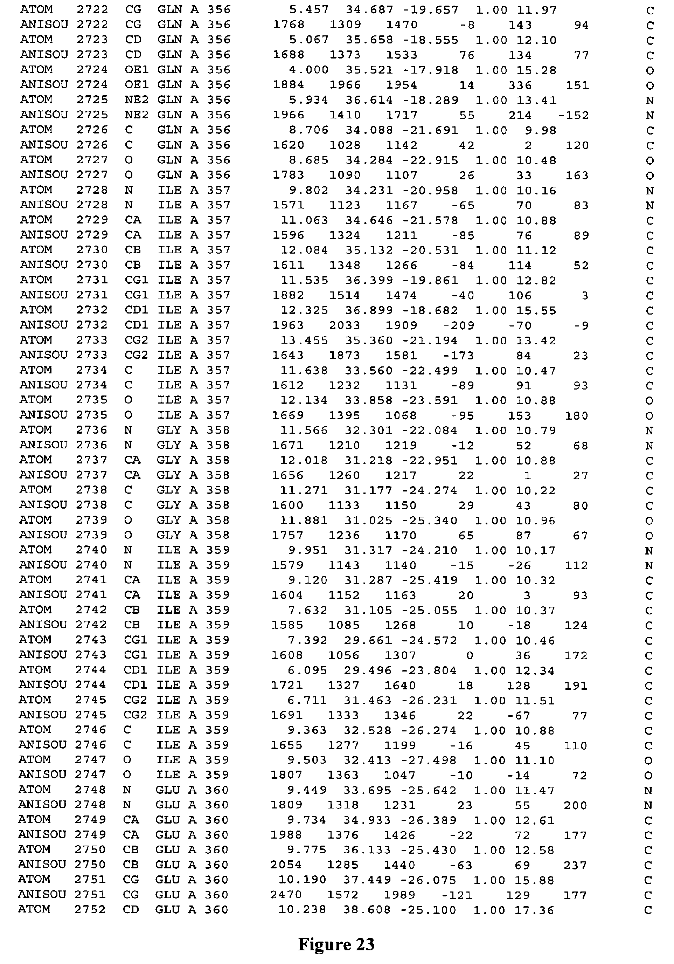

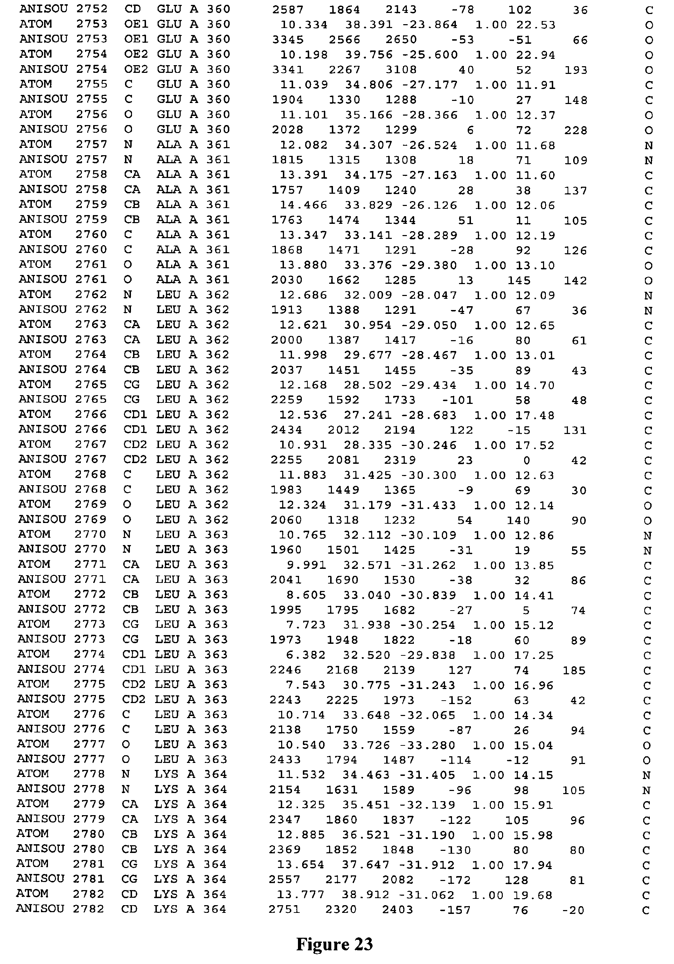

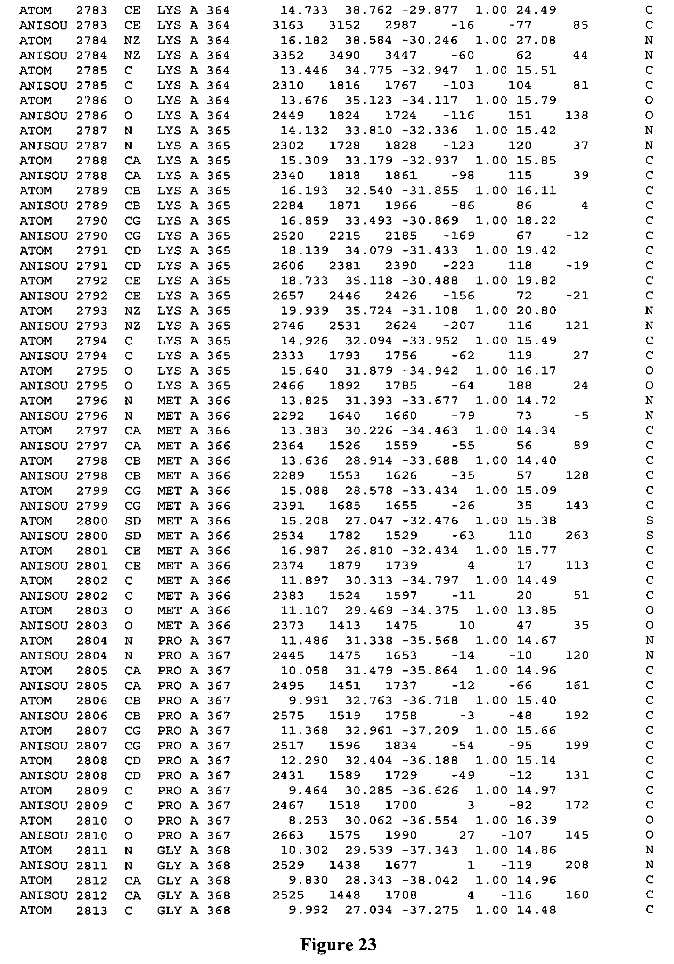

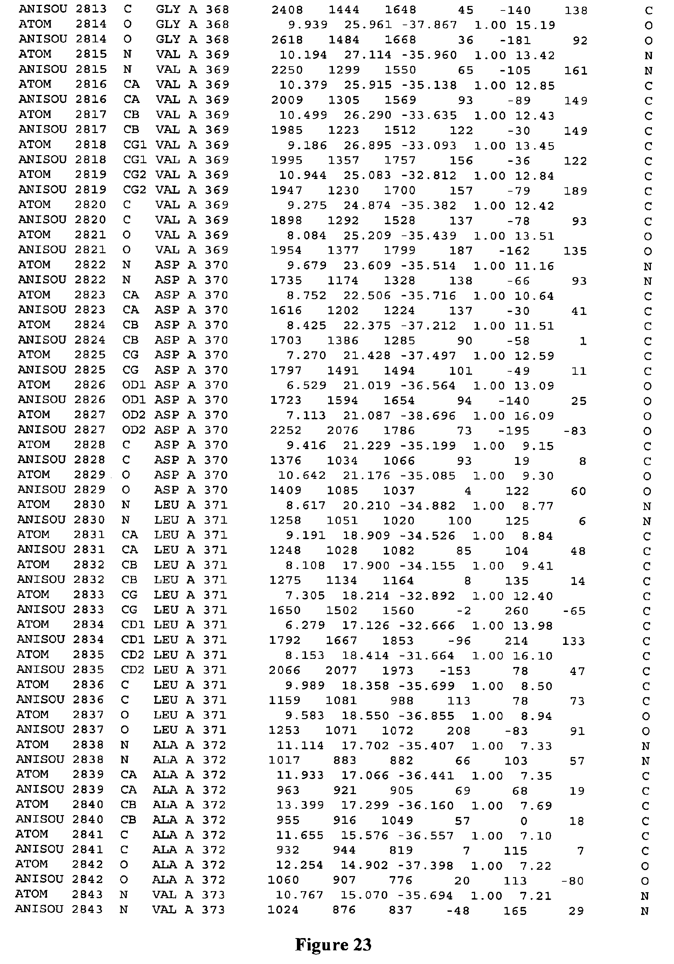

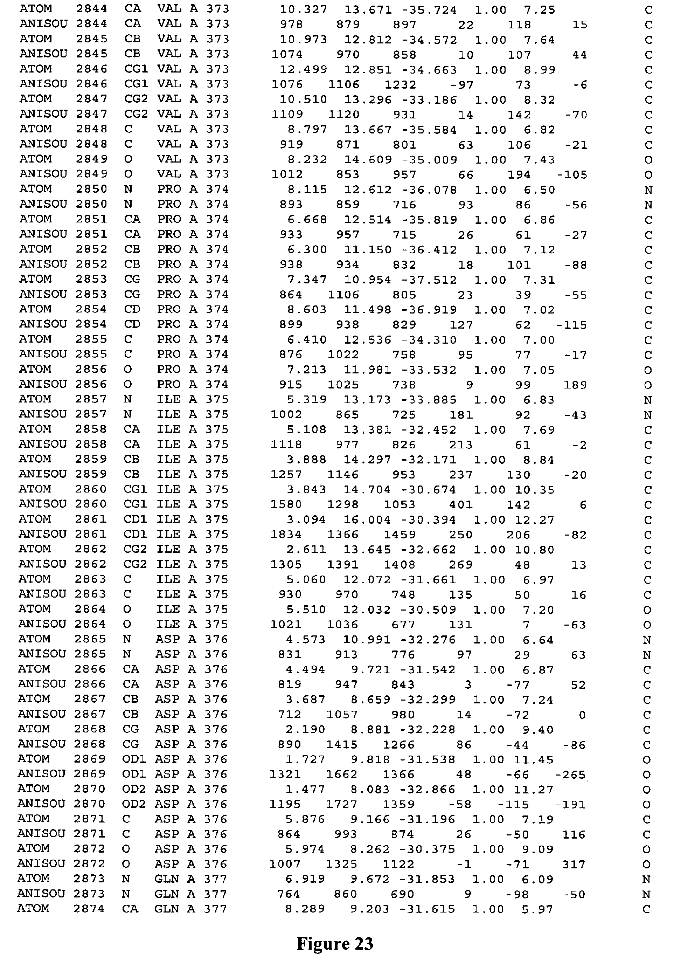

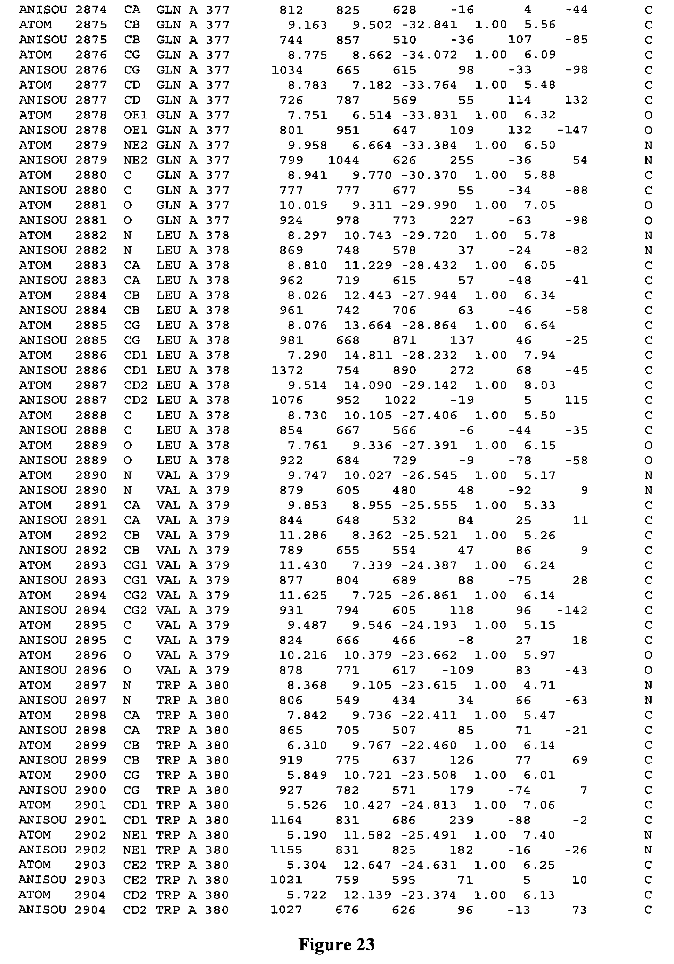

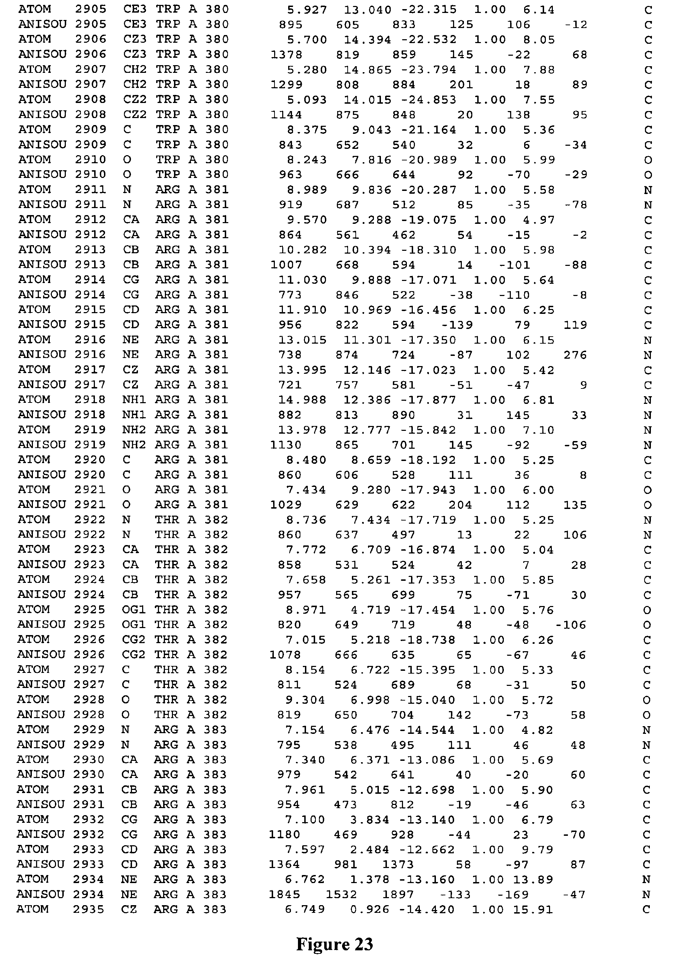

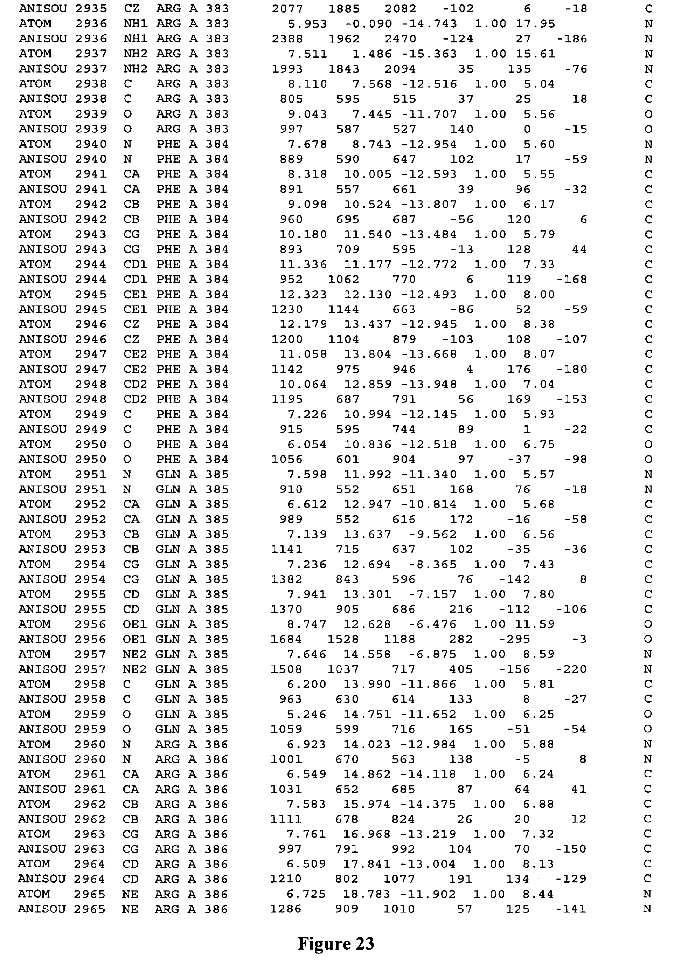

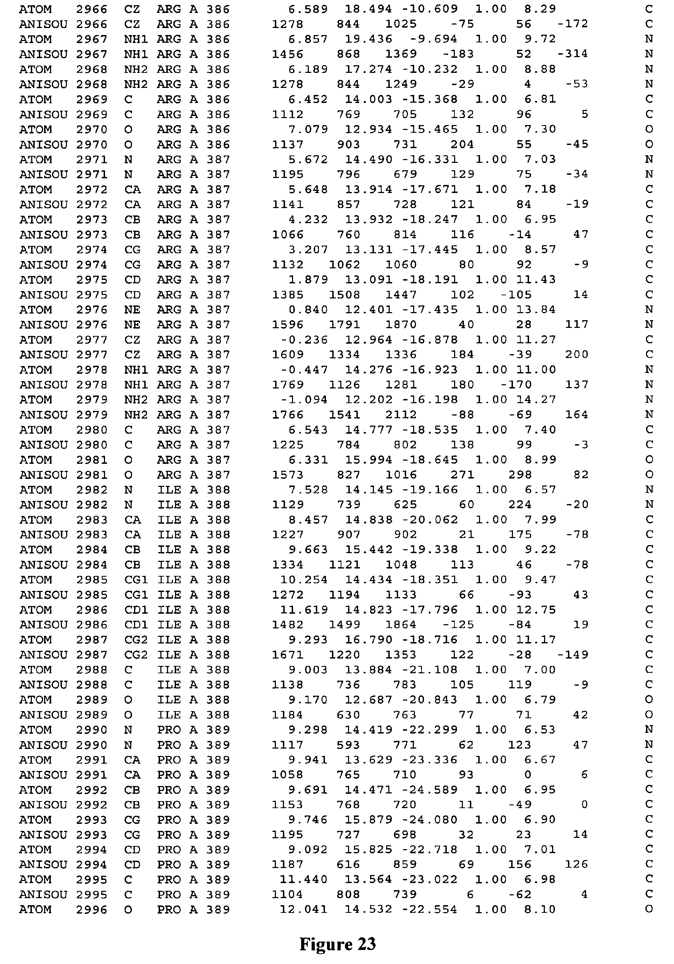

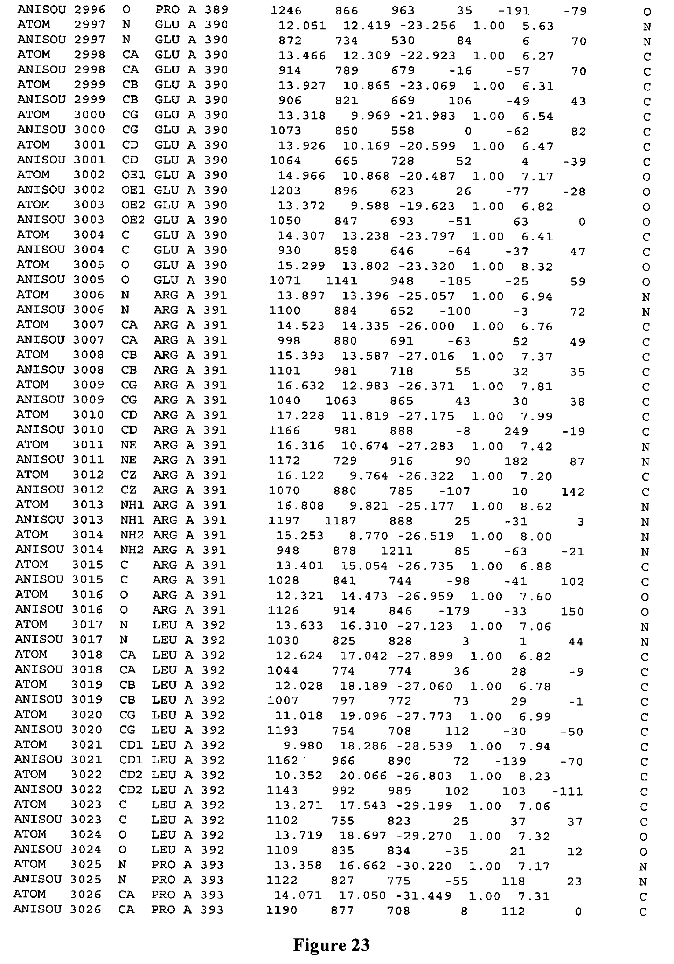

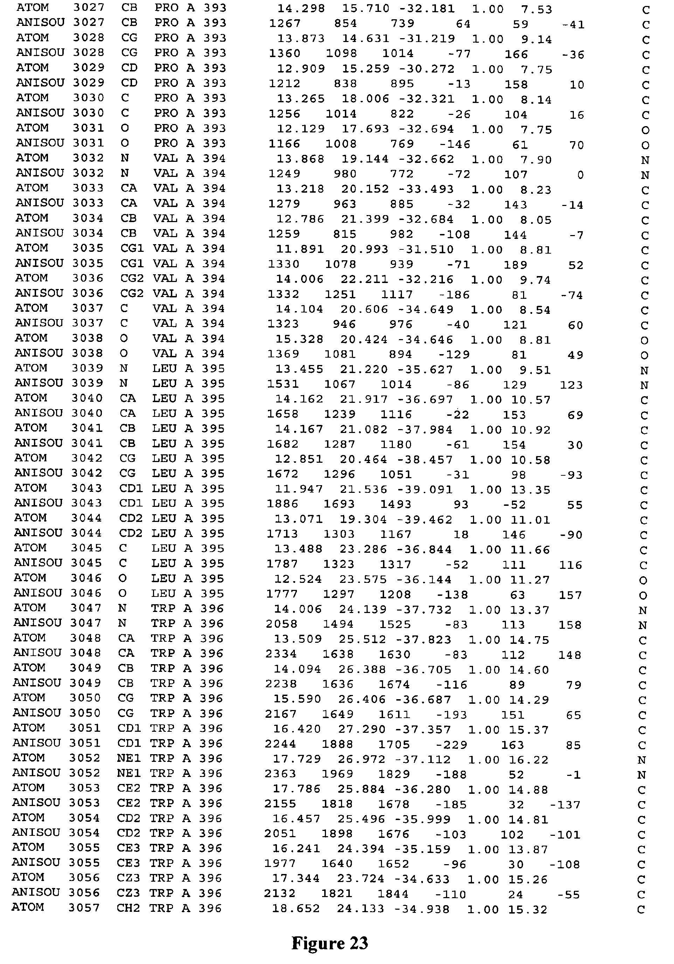

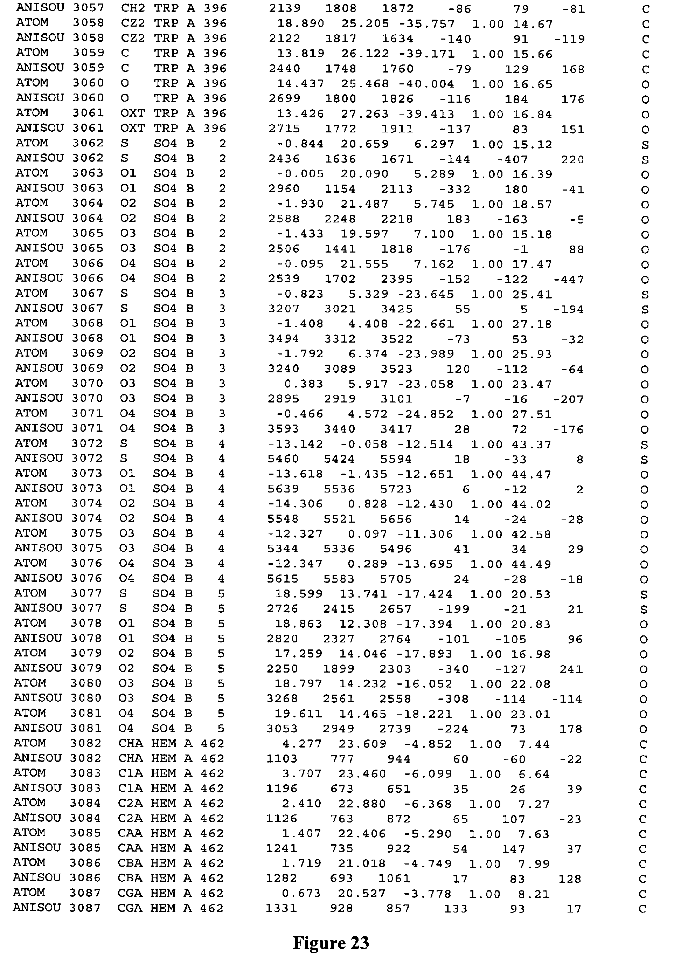

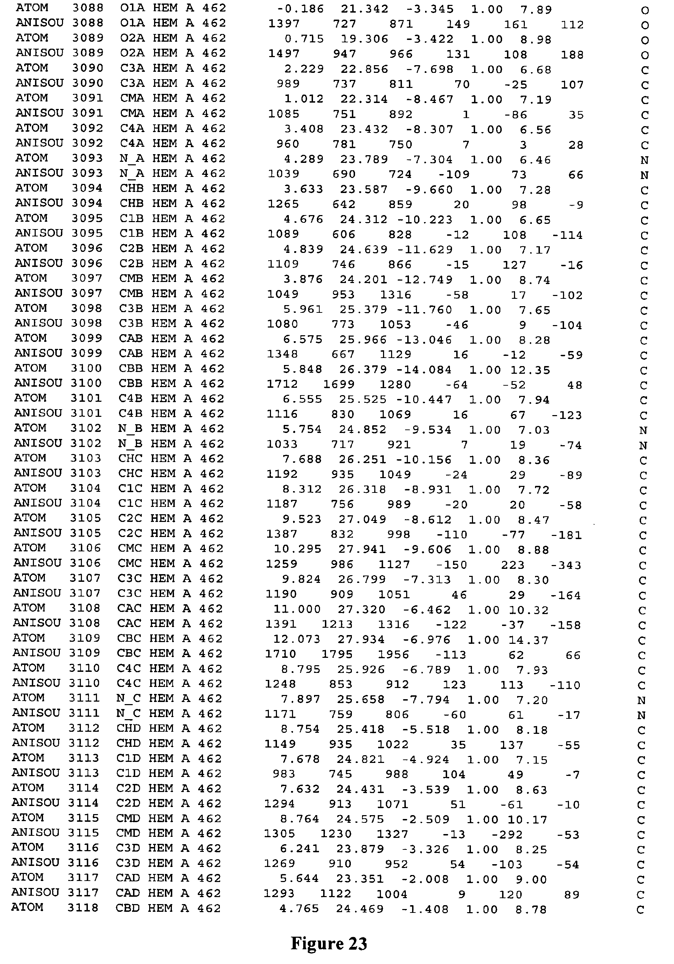

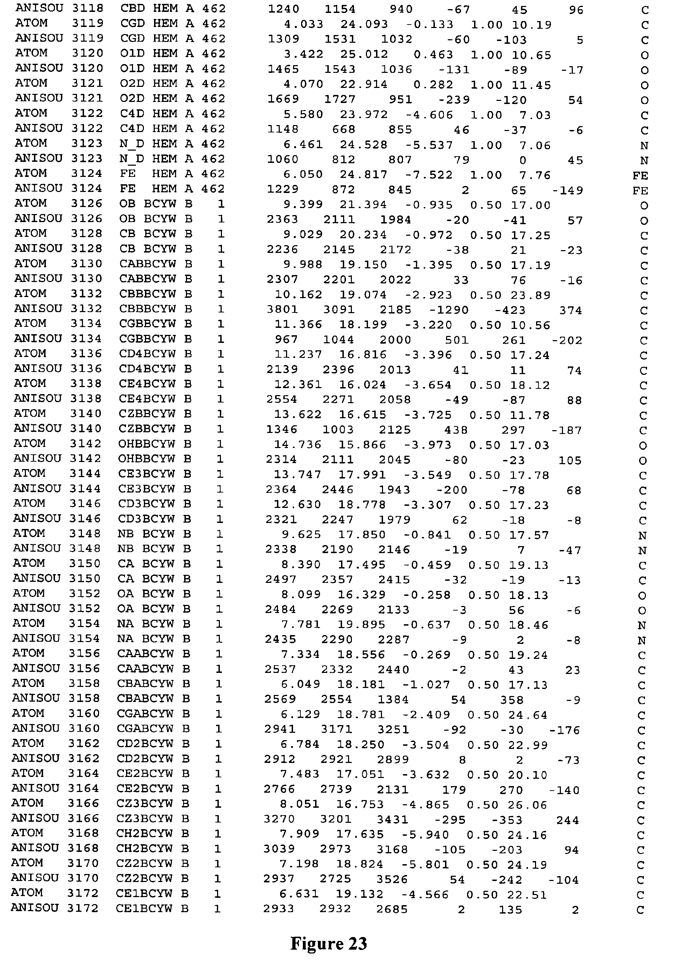

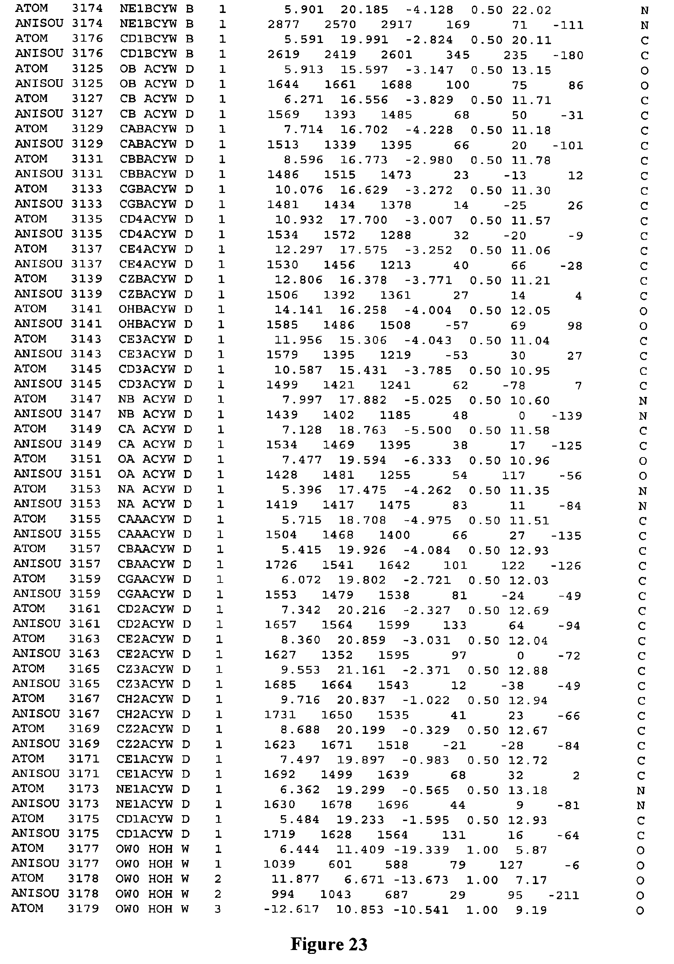

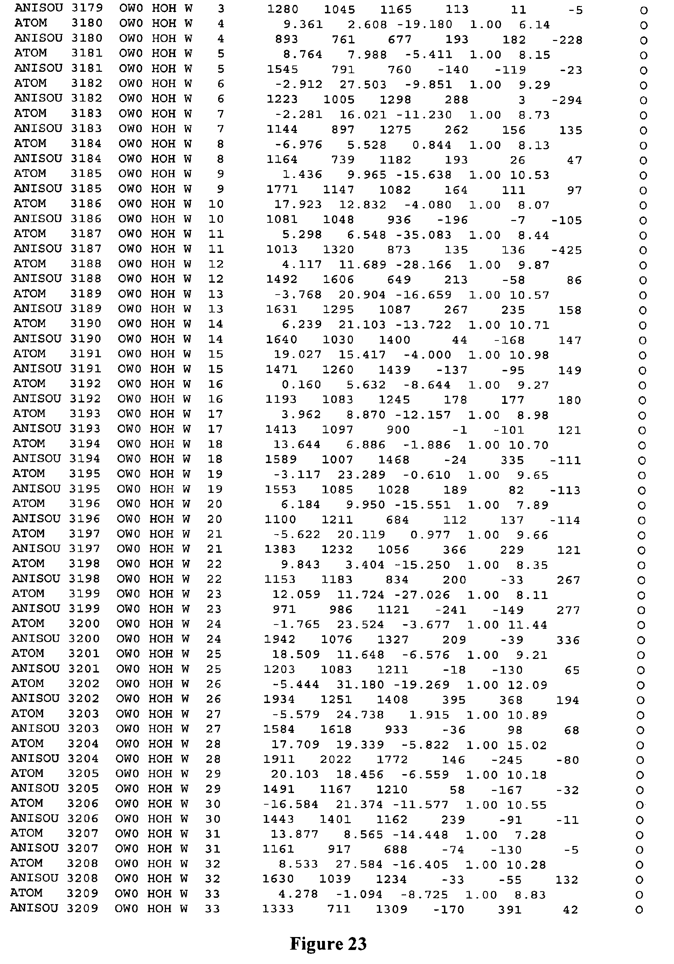

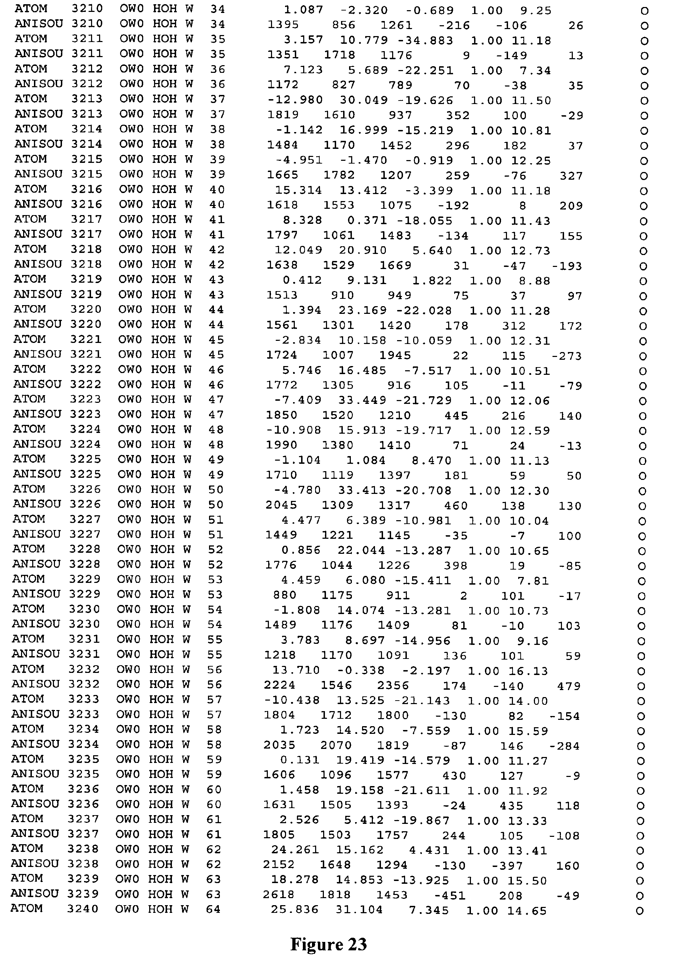

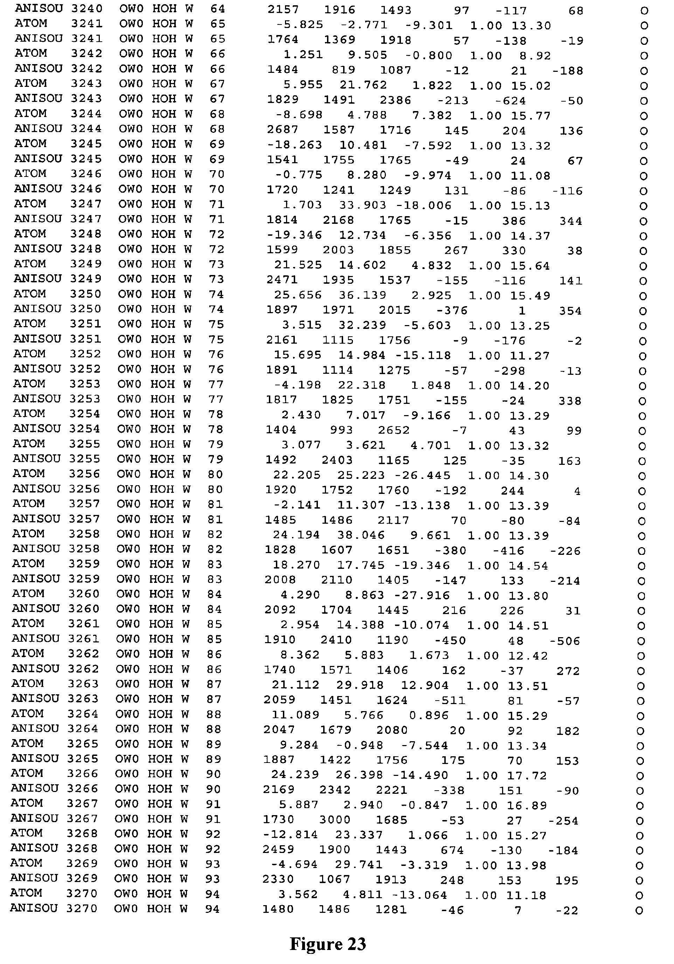

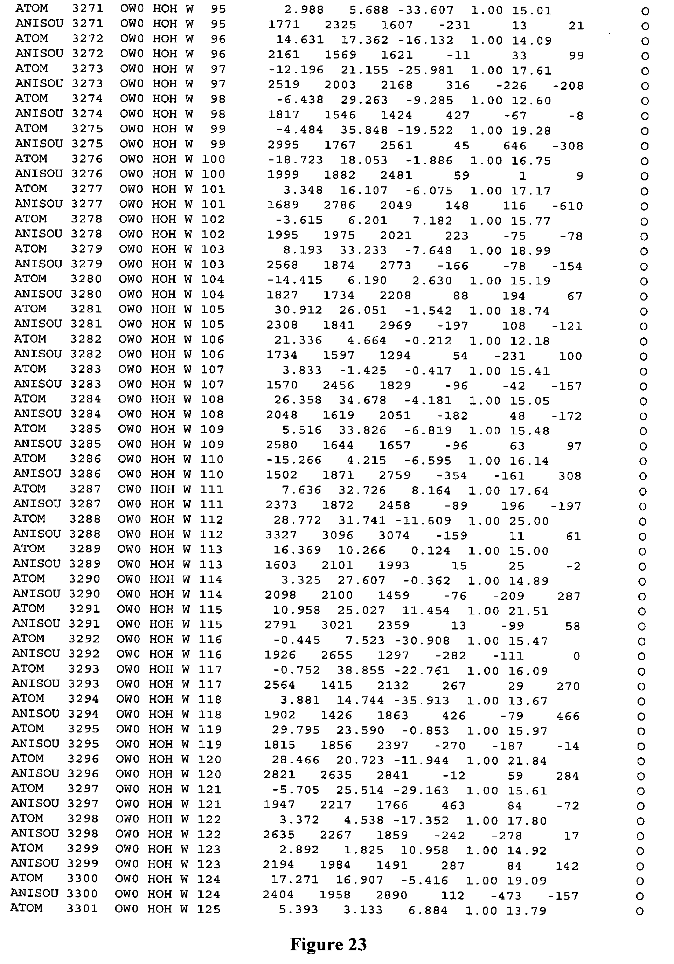

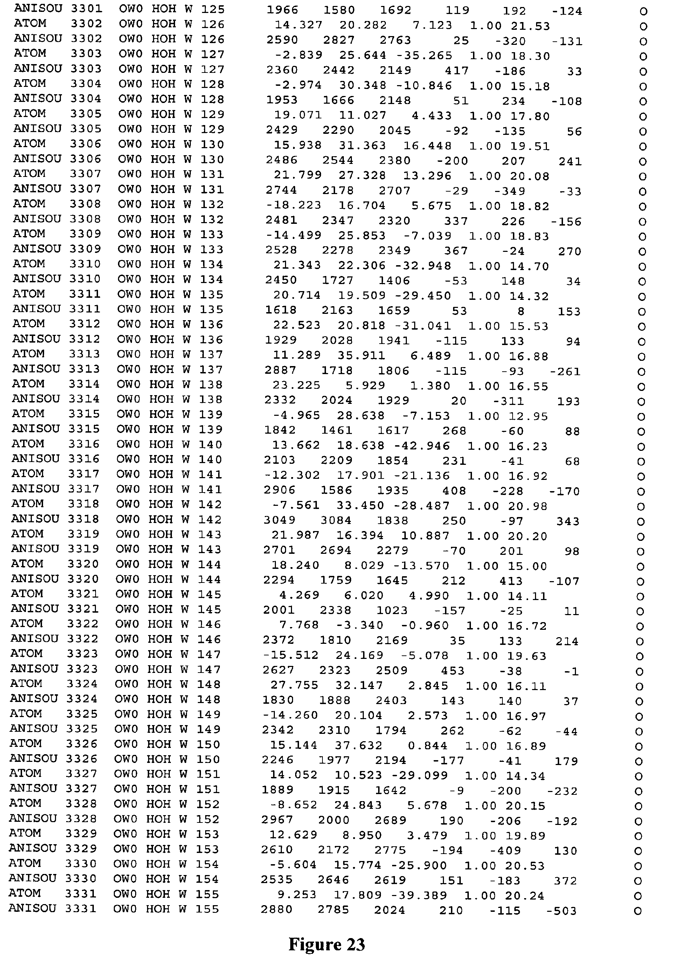

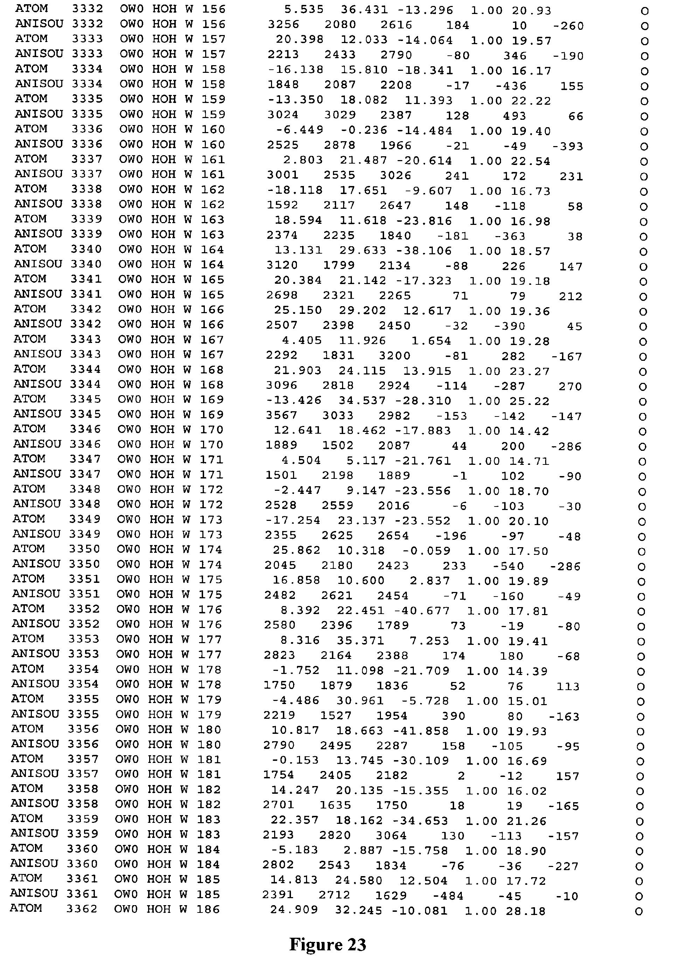

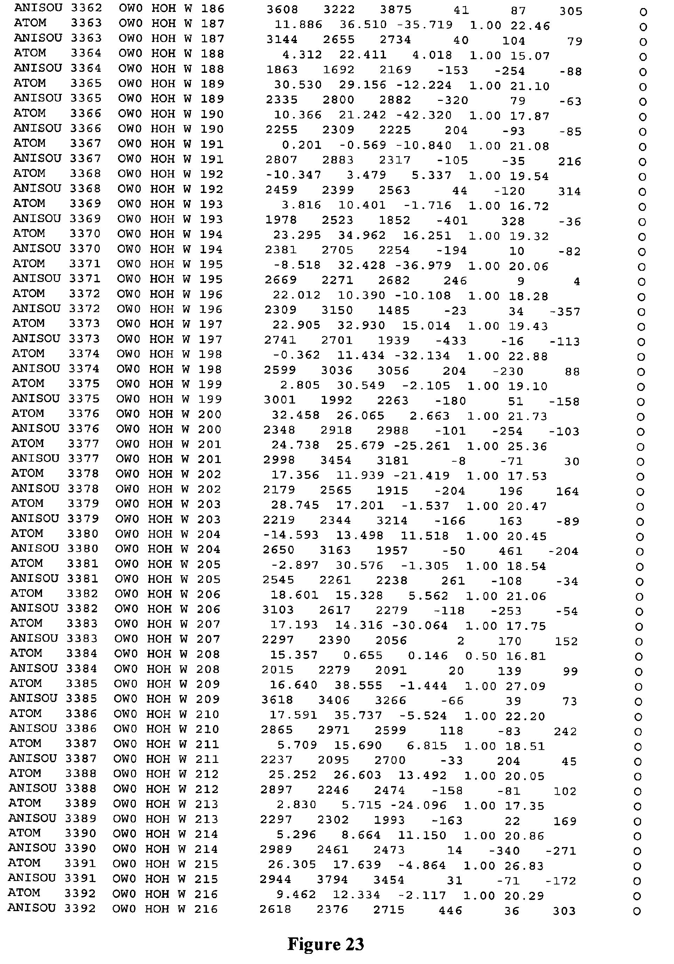

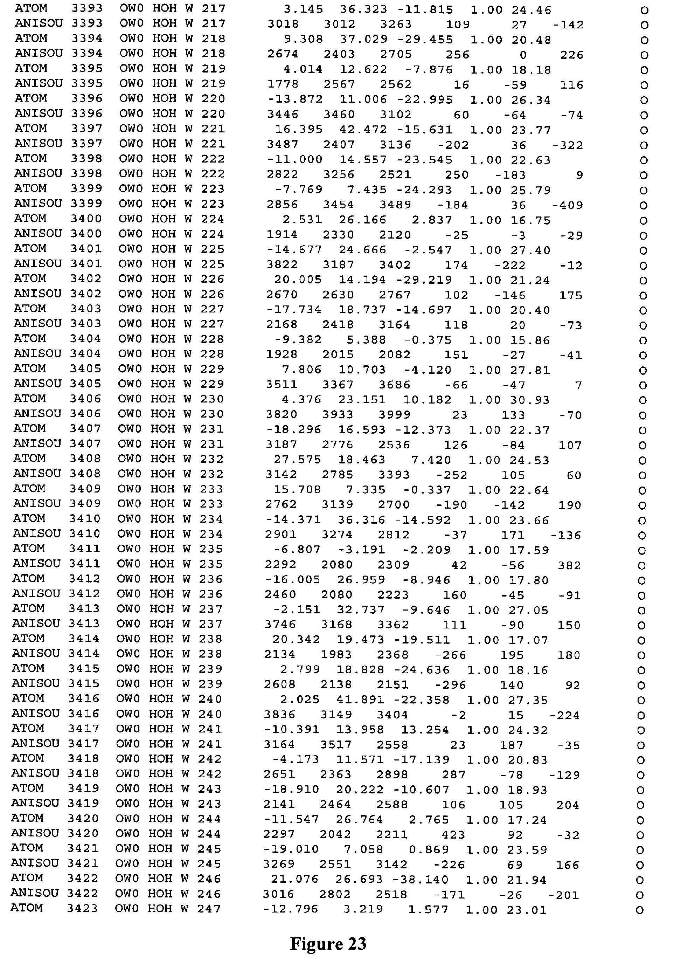

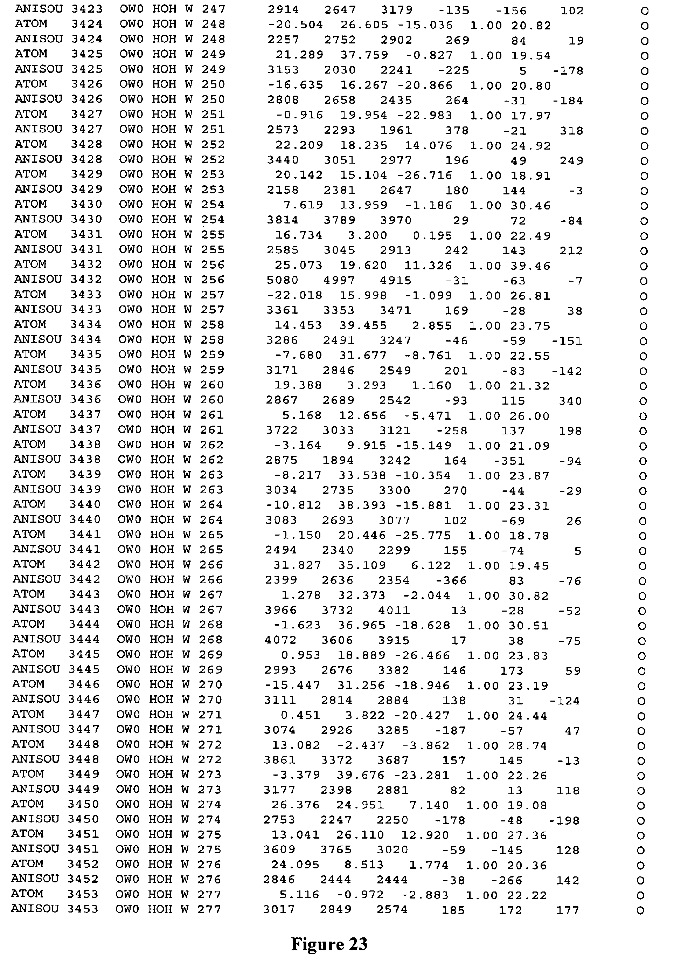

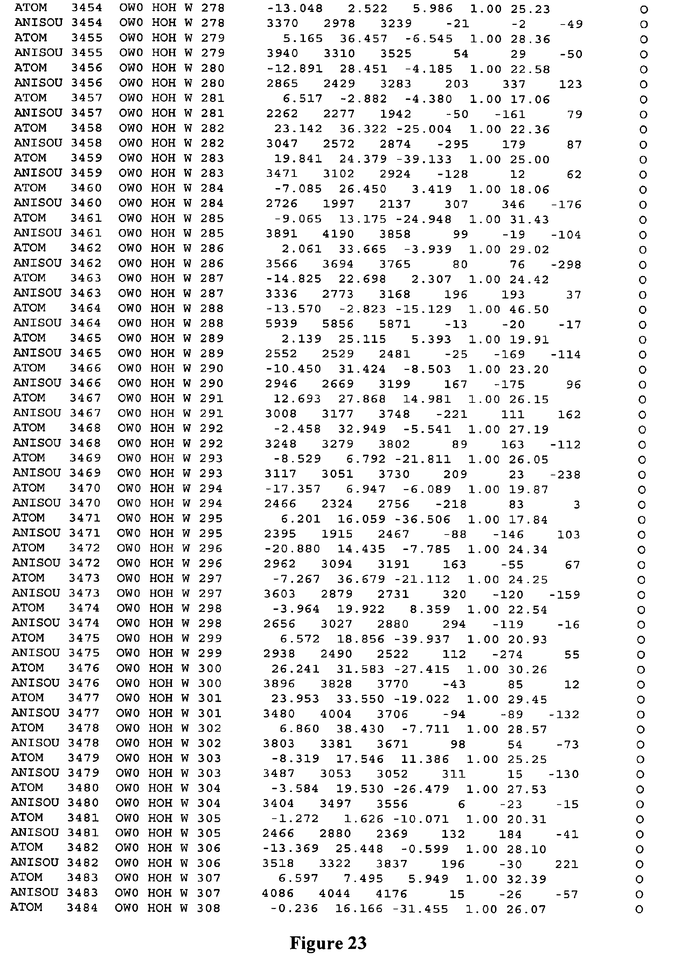

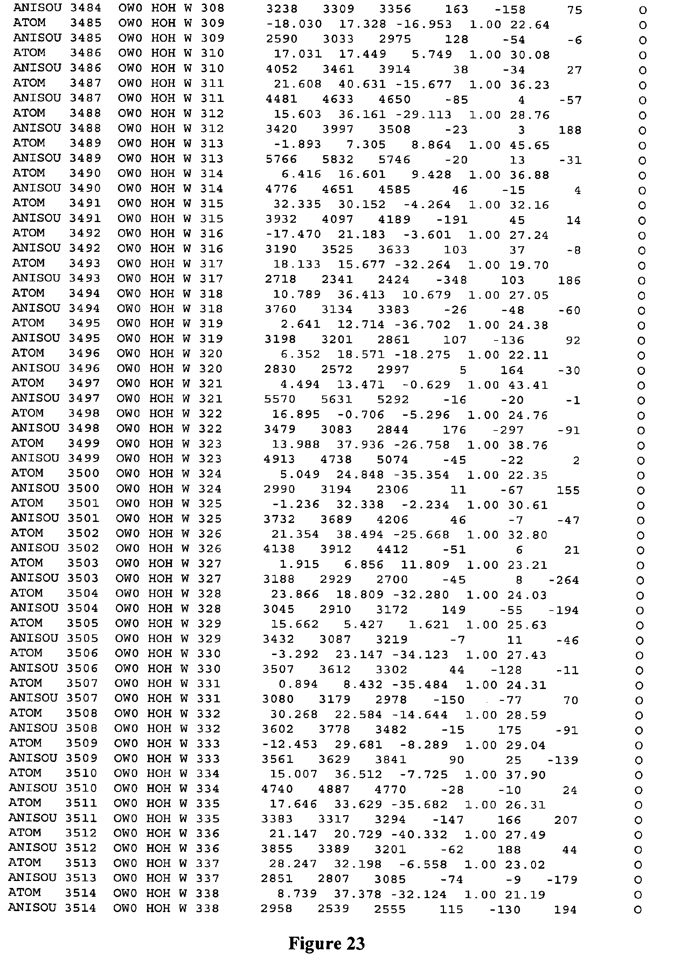

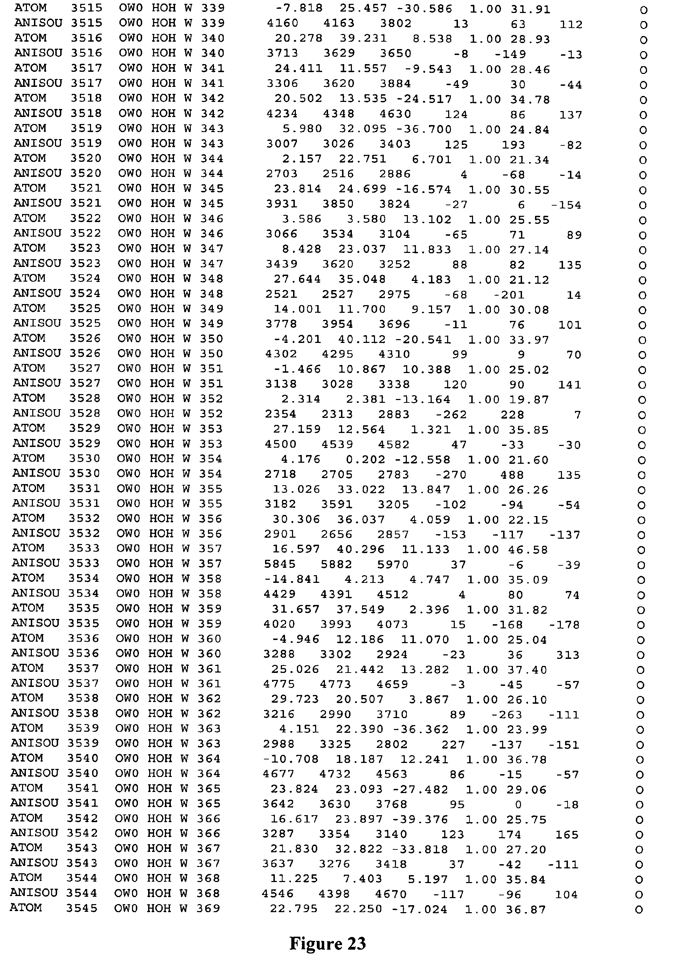

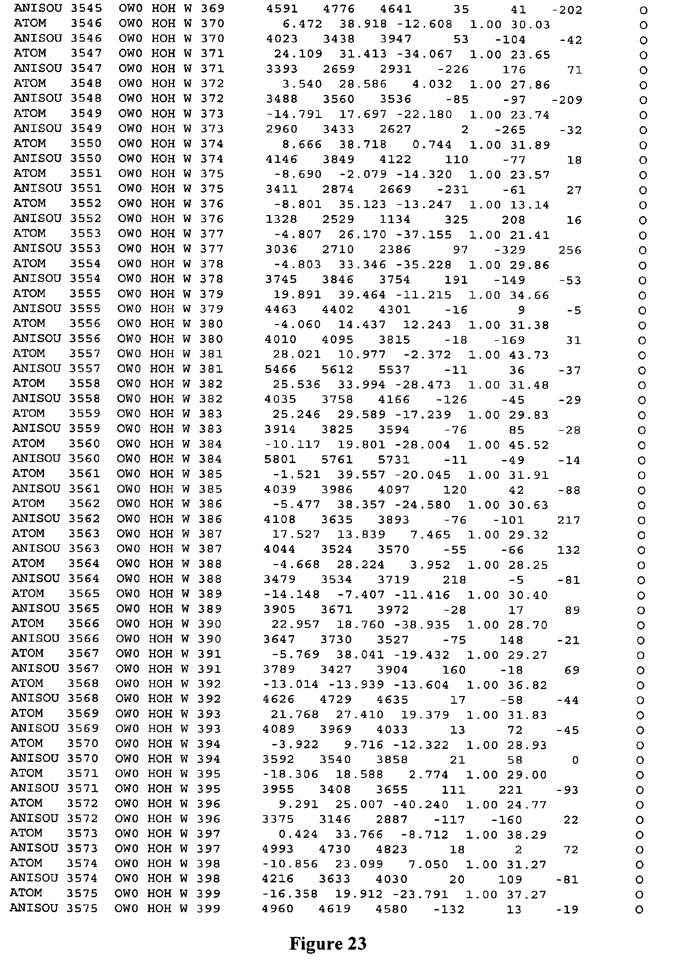

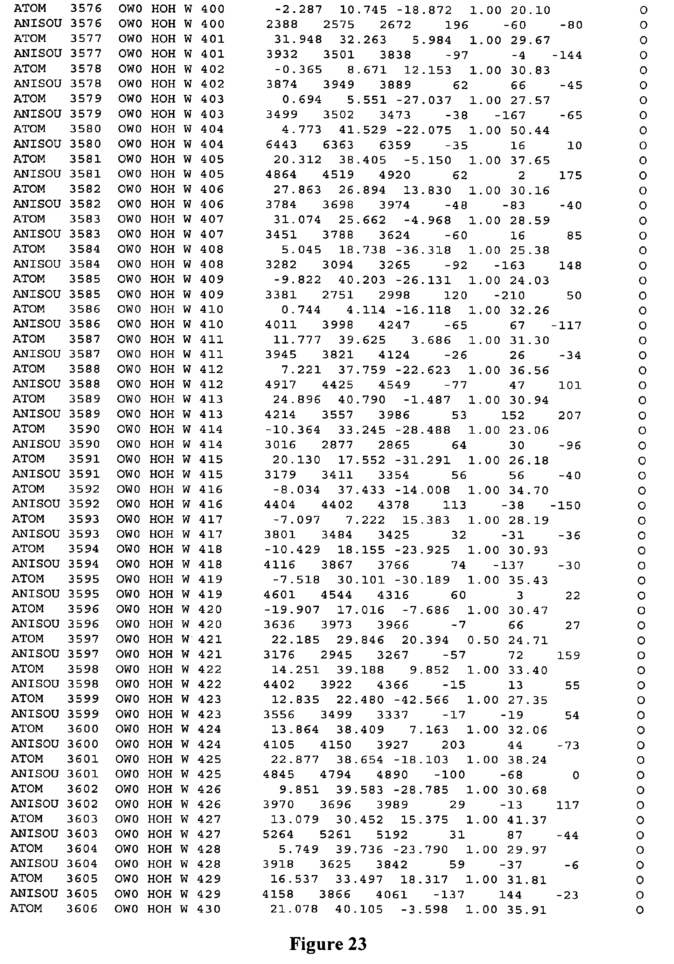

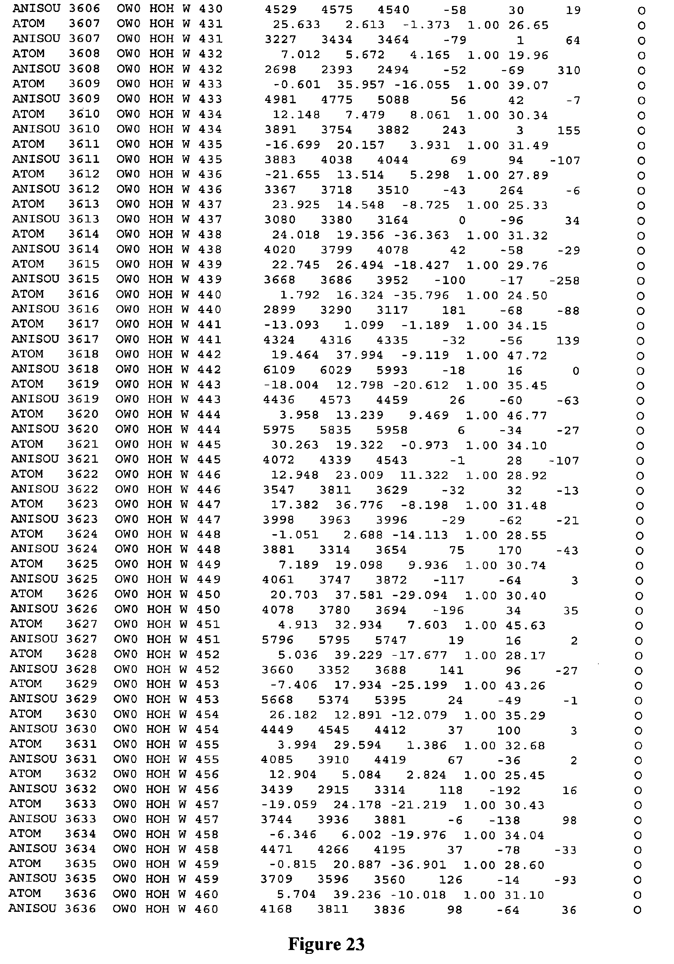

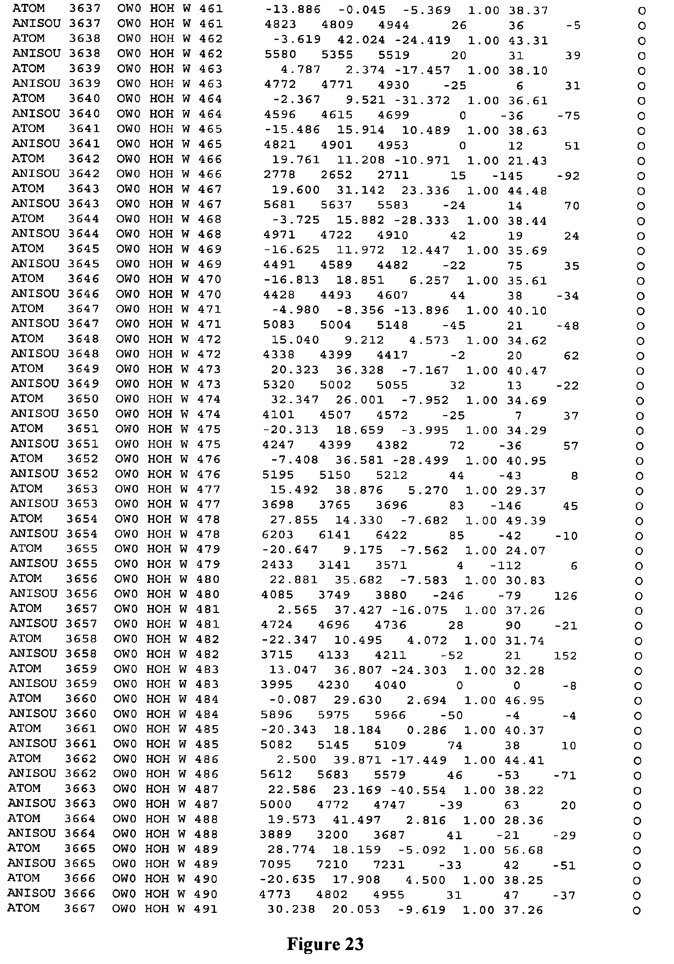

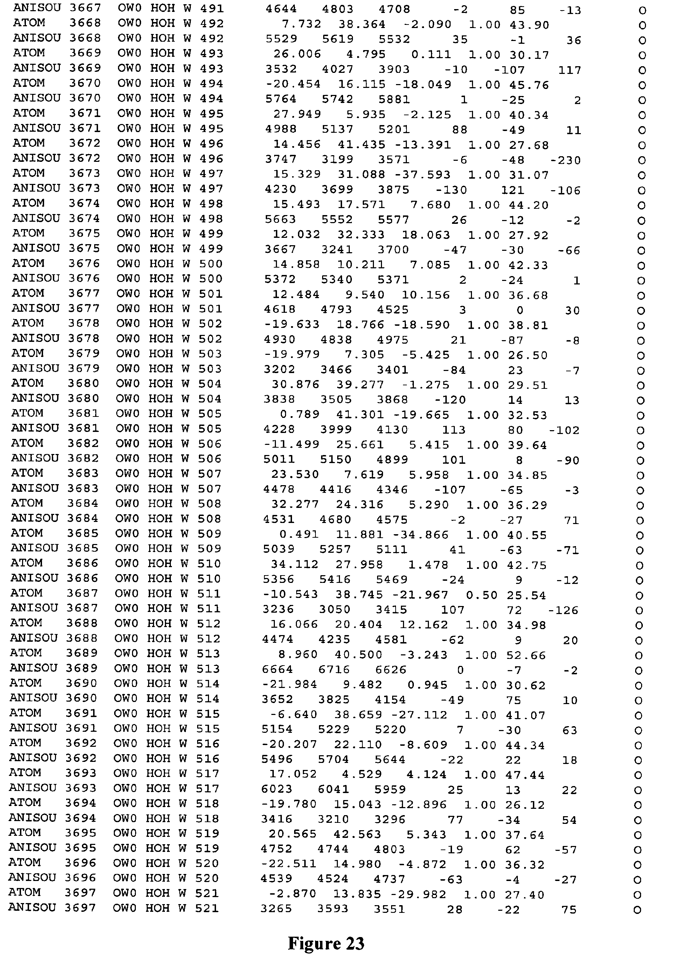

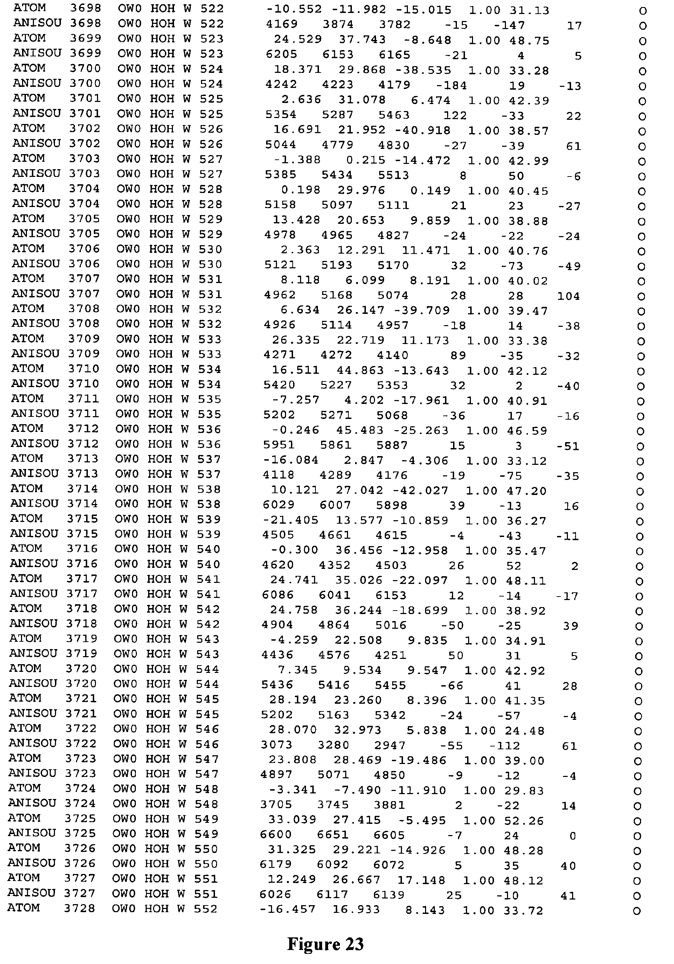

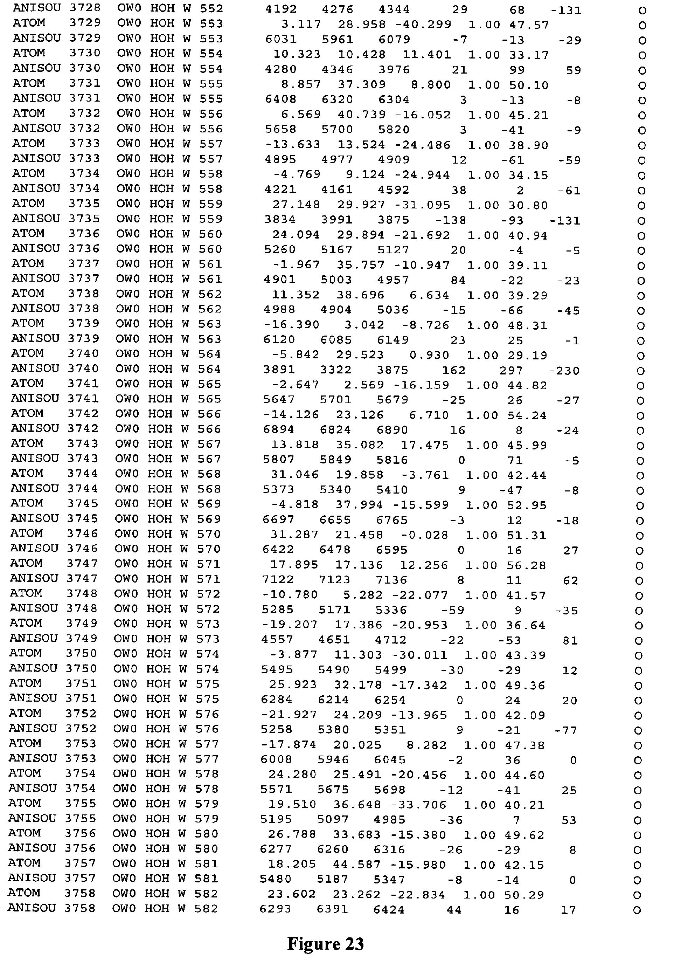

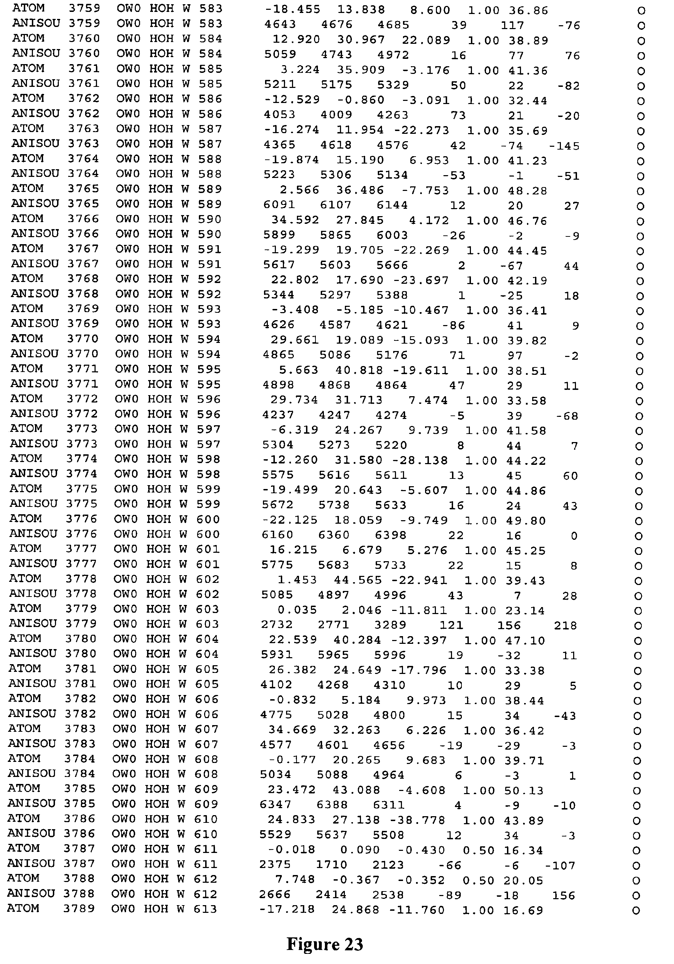

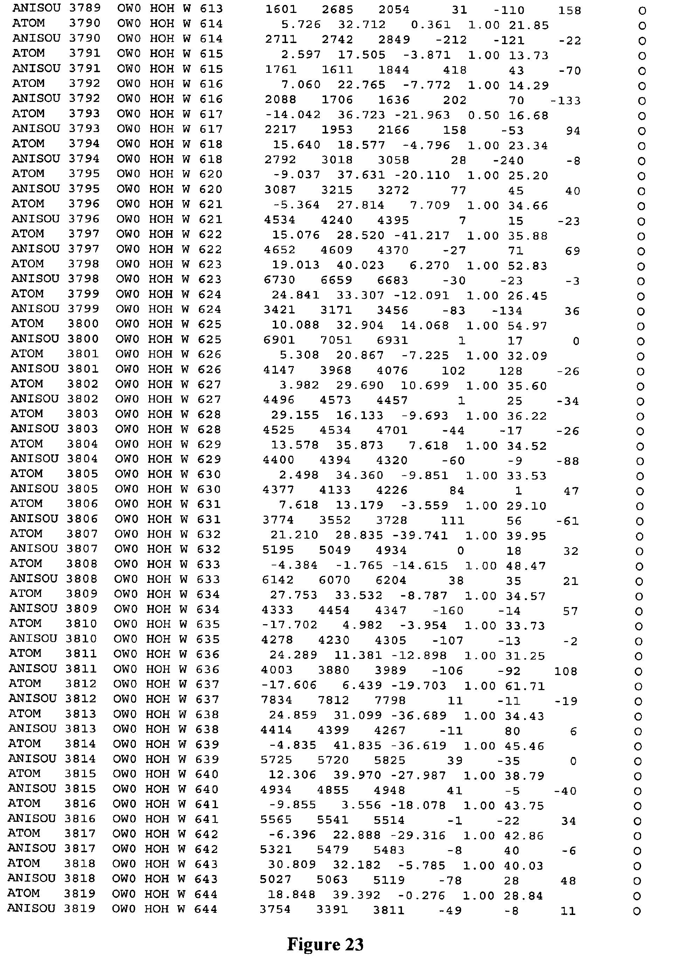

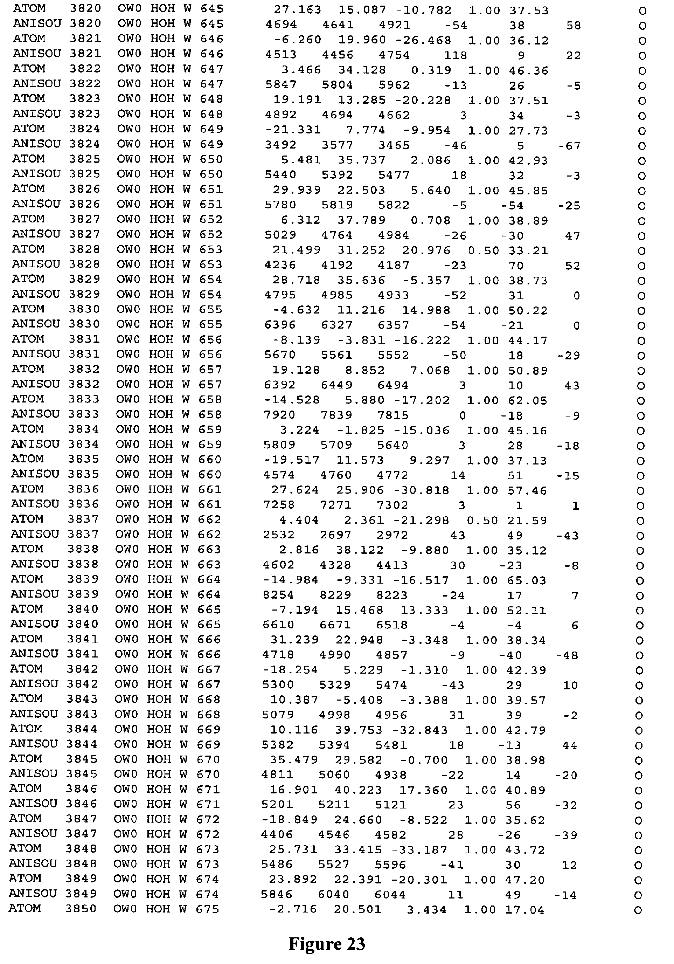

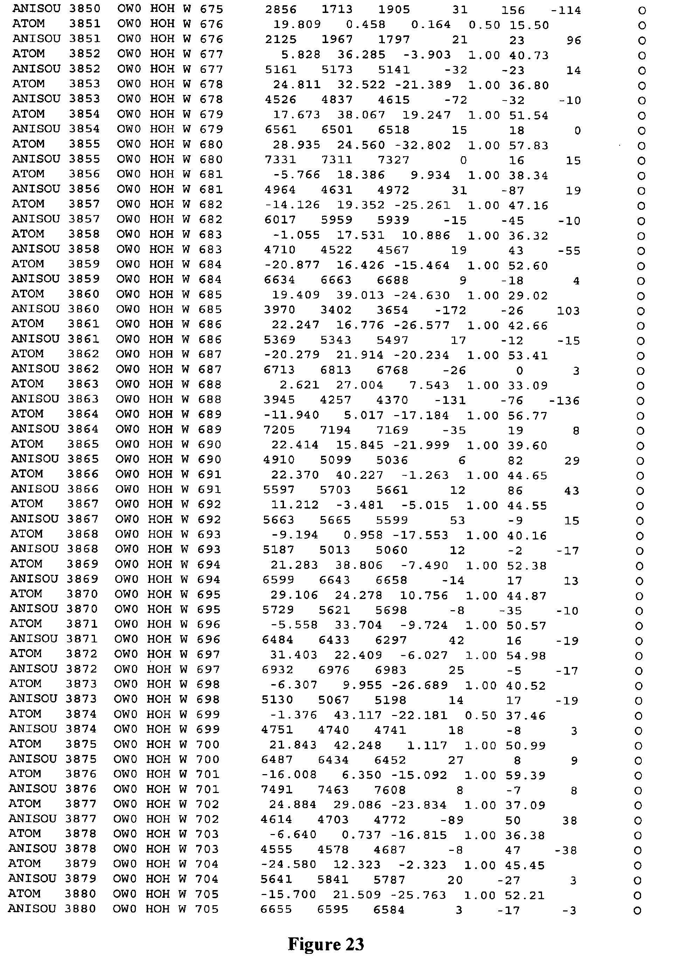

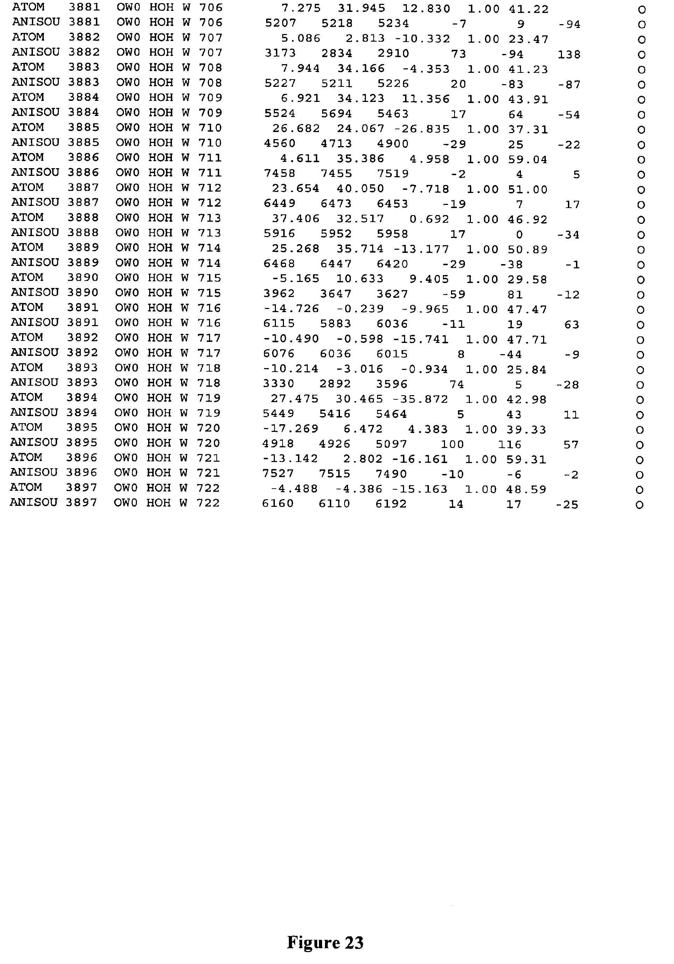

According to a seventh aspect of the present invention there is provided data array comprising the atomic coordinate as set forth in Table I and Figures 19, 20, 21, 22 and 23, of CYP121 in combination with a target selected from the group consisting of SEQ ID NO: 4, SEQ ID NO: 5, SEQ ID NO: 6, SEQ ID NO: 7, SEQ ID NO: 8; SEQ ID NO: 9;
which when acted upon by a protein modelling algorithm yields a representation of the 3-D structure of CYP121 in combination with its target.

For a better understanding of the invention and to show how the same may be carried into effect, there will now be shown by way of example only, specific embodiments, methods and processes according to the present invention with reference to the accompanying drawings in which:
Figure 1 shows the spectral changes induced on titration of CYP121 (2 µM) with cyclo(L-Tyr-L-Tyr) (SEQ ID NO:4) (0-463 µM) are shown.
Figure 2 shows RP-HPLC chromatograms recorded after incubation of CYP121, an electron transport chain, NADPH and cyclo(L-Tyr-L-Tyr) (SEQ ID NO:4) at 30°C for various times (0 min, 1 min, 3 min, 10 min, 30 min and 60 min).
Figure 3 shows RP-HPLC chromatograms and the effect of reaction conditions on cyclo(1,-Tyr-L-Tyr) (SEQ ID NO:4) transformation by CYP121.
Figure 4 shows X-ray structure of cyclo(L-Tyr-L-Tyr)-bound CYP121.
Figure 5 shows the structure of cyclo(L-DOPA-L-Tyr) (SEQ ID NO:8).
Figure 6 shows the spectral changes recorded upon addition of increasing amount of cyclo(L-DOPA-L-Tyr) (SEQ ID NO:8)
Figure 7 shows RP-HPLC chromatograms of the incubation of cyclo(L-DOPA-L-Tyr) (SEQ ID NO:8) with CYP121, an electron transport chain, NADPH at 30°C for various times (0 min, 1 min, 3 min, 10 min, 30 min and 60 min)..
Figure 8 shows RP-HPLC chromatograms of the inhibition of CYP121 activity by cyclo(L-DOPA-L-Tyr) (SEQ ID NO:8).
Figure 9 shows the X-ray structure of cyclo(L-DOPA-L-Tyr)-bound CYP121.
Figure 10 shows the spectral changes induced on titration of CYP121 (2 µM) with cyclo(L-Tyr-L-Trp) (SEQ ID NO:5).
Figure 11 shows RP-HPLC chromatograms of cyclo(L-Tyr-L-Trp) (SEQ ID NO:5) incubation with CYP121, an electron transport chain, NADPH at 30°C for various times (0 min, 1 min, 3 min, 10 min, 30 min and 60 min)..
Figure 12 shows the inhibition of CYP121 activity by cyclo(L-Tyr-L-Trp) (SEQ ID NO:5).
Figure 13 shows the X-ray structure of cyclo(L-Tyr-L-Trp)-bound CYP121 with cyclo(L-Tyr-L-Trp) bound in conformation 1.
Figure 14 shows the X-ray structure of cyclo(L-Tyr-L-Trp)-bound CYP121 with cyclo(L-Tyr-L-Trp) (SEQ ID NO:5) bound in conformation 2.
Figure 15 shows the X-ray structure of cyclo(L-Tyr-L-Phe)-bound CYP121.
Figure 16 shows the X-ray structure of cyclo(L-Tyr-L-Ala)-bound CYP121.
Figure 17 shows the crystal structure of cyclo(L-Tyr-L-Tyr)-bound (panel A) and native (panel B) CYP121 active sites.
Figure 18 shows a schematic representation of the oxygen binding site of substrate-bound (panel A) and ligand-free (panel B) CYP121.
Figure 19 lists the atomic coordinate data of cyclo(L-Tyr-L-Tyr)-bound CYP121.
Figure 20 lists the atomic coordinate data of cyclo(L-DOPA-L-Tyr)-bound CYP121.
Figure 21 lists the atomic coordinate data of cyclo(L-Tyr-L-Phe)-bound CYP121.
Figure 22 lists the atomic coordinate data of Cyclo(L-Tyr-L-Ala)-bound CYP121.
Figure 23 lists the atomic coordinate data of cyclo(L-Tyr-L-Trp)-bound CYP121.
There will now be described by way of example a specific mode contemplated by the Inventors. In the following description numerous specific details are set forth in order to provide a thorough understanding. It will be apparent however, to one skilled in the art, that the present invention may be practiced without limitation to these specific details. In other instances, well known methods and structures have not been described so as not to unnecessarily obscure the description.

### EXAMPLE 1 - EXPERIMENTAL PROCEDURES

### 1.1 Construction of the CYP121 expression vector

The CYP121-encoding gene was amplified from the chromosomal DNA of *Mycobacterium bovis* BCG Pasteur that carries the *mb2299* gene (gi:31793455) 100% identical to the CYP121-encoding gene Rv2276 of *Mycobacterium tuberculosis* H37Rv (gi:15609413) (SEQ ID NO: 1).

PCR reaction was performed with oligonucleotides TTGTTACCCCTGATAGGAGCCCAGCCATATGACCGCGACCGTTCTGCTC (SEQ ID NO: 2) and ATGGCTCGGGTGAGTTTAGGGATCCTACCAGAGCAC CGGAAGGCGTTCG (SEQ ID NO:3) by using the Expand High Fidelity PCR System (Roche). The 1.2 kb PCR product was purified on agarose gel, digested with *Nco*I and *Bam*HI and cloned between the *Nco*I and *Bam*HI sites of pETlla (Novagen), resulting in plasmid pET11a-CYP121 (SEQ ID NO: 10). The nucleotide sequence of the cloned gene was verified using the ABI Prism 310 Genetis Analyser (Applied Biosystems).

### 1.2 Production and purification of CYP121

Recombinant CYP121 was produced in *Escherichia coli* HMS174(DE3) (Novagen) and purified as previously described with some modifications (17).

Bacteria were grown at 30°C with vigorous shaking in terrific broth (18) until A₆₀₀ = 0.3 and 100 µg/ml 5-aminolevulinic acid (Sigma-Aldrich) was added to the culture. Growth was continued until A₆₀₀ = 0.5 and temperature was decreased to 20°C. After 15 min at 20°C, 100 µM IPTG was added to the culture that was incubated for a further 20h at 20°C with agitation. At the end of the culture, 1 mM PMSF (Fluka) was added before centrifugation (7000 x g, 30 min. 4°C).

Bacteria were washed in ice-cold 50 mM Tris-HCl pH 7.2 EDTA 1mM (buffer A) and re-centrifuged. At this time, the cell pellet was frozen in liquid nitrogen and stored at -80°C until use. Bacteria were thawed on ice and resuspended in a small volume of buffer A plus 1mM PMSF and 1mM benzamidine hydrochloride (Sigma-Aldrich). Bacteria were lysed in an Eaton pressure cell (Rassant). After thawing, the lysate was sonicated on ice using a VibraCell 72442 sonicator in order to shear DNA (1s pulse/1s rest during 2 min) and centrifuged at 20000 x g for 30 min at 4°C. CYP121 was purified from supernatant as described by McLean *et al*. (17).

Protein purity was checked throughout the purification by SDS-PAGE and by determining the A₄₁₆/A₂₈₀ ratio. Finally, enzyme was concentrated by ultrafiltration (Vivaspin 10, Vivasciences) and stored at -80°C in buffer A plus 50% glycerol. Protein concentration was determined using the extinction coefficient ε₄₁₆ = 95 mM⁻¹ cm⁻¹ as previously described (17).

### 1.3 Amino-terminal sequencing

Purified CYP121 solution was desalted and concentrated directly onto an inert PVDF membrane by the use of ProSorb cartridges from Applied Biosystems.

The sample was then submitted to N-terminal sequencing according to Edman chemistry on a Procise Model 492HT automatic sequencer from Applied Biosystems - Perkin Elmer (Foster City, CA, USA).

### 1.4 Mass spectrometry

Electrospray mass spectrometry was carried out using an Esquire HCT ion trap mass spectrometer (Bruker Daltonik GmbH, Germany). Desalted CYP121 solution in ACN/MilliQ water containing 0.1% formic acid was prepared through successive ultrafiltration steps (Vivaspin 10, Vivasciences). Cyclodipeptide solutions were diluted in ACN/MilliQ water containing 0.1% formic acid. Samples were directly infused at a flow rate of 3 ml/min and ionization was carried out in positive mode with a nebulizing gas set at 9 psi, a drying gas set at 5 µl/min and a drying temperature set at 300°C for optimal spray and desolvatation. For MSMS fragmentation an isolation width of 1.0 mass unit was set for isolating the parent ion. A fragmentation energy ramp was used for automatically varying the fragmentation amplitude in order to optimize the MSMS fragmentation process. Full scan MS and MS/MS spectra were acquired using EsquireControl software and all data were processed using DataAnalysis software.

### 1.5 Cyclodipeptide solutions.

Cyclo(1.-Tyr-L-Tyr) (SEQ ID NO: 4) and cyclo(L-Tyr-1.-Trp) (SEQ ID NO: 5) were obtained from NeoMPS and Bachem AG, respectively. Cyclo(L-Tyr-L-Phe) (SEQ ID NO: 6), cyclo(L-Tyr-L-Ala) (SEQ ID NO: 7) and cyclo(L-3 or 5,4-dihydroxyphenylalanine-L-Tyr) (cited therein as cyclo(L-DOPA-L-Tyr) (SEQ ID NO: 8)) were chemically synthesized according to a procedure previously described (19). Purification to homogeneity (>95%) and physico-chemical characterization was achieved by reverse phase HPLC and mass spectrometry, respectively.

Cyclodipeptide solutions (10-120 mM) were made in DMSO (Sigma-Aldrich). Concentrations of cyclodipeptide solutions were determined by amino acids analyses using standard conditions: after dilution in MilliQ water to bring DMSO concentration < 2%, samples were transferred into glass tubes, vacuum dried and sealed before a 18-hours hydrolysis at 110°C in the presence of vapour-phase constant-boiling 6N HCl (Pierce). Amino acids hydrolysates obtained were solubilized in MilliQ water and analyzed by ion-exchanged HPLC coupled on-line to a post-column ninhydrin derivatization on an AminoTac JLC-500/V amino acids analyzer (JEOL, Japan). Quantification was processed according to a calibration table obtained with standard amino acids mix from Pierce.

### 1.6 CYP121 absorbance spectroscopy

The absorbance experiments with CYP121 were carried out using a double-beam Uvikon 943 spectrophotometer and 1-cm path length quartz cells. For CYP121 titration by cyclodipeptide, absorbance spectra were recorded after each consecutive addition of 0.5-1 µl aliquot of cyclodipeptide solution to 1 ml of 2 µM CYP121 solution in Tris-HCl 50 mM pH 7.6 10% glycerol. After each addition, the same aliquot of DMSO was added to the reference cuvette. The total volume of added cyclodipeptide solution did not exceed 10 µl.

For each cyclodipeptide concentration in the cuvette, difference spectra were generated by the subtraction of cyclodipeptide-free spectrum from each cyclodipeptide-bound spectrum. Spectrally estimated dissociation constants (Ks) were determined by plotting the difference between A₃₈₈ and A₄₁₆ (ΔA) calculated from each difference spectrum against the cyclodipeptide concentration. The data were fitted to the following equation ΔA = (ΔAₘₐₓ x [L])/(Ks + [L]) using Kaleidagraph (Synergy Software), where L represents the ligand. Given values of Ks are the means of four independently made experiments.

### 1.7 Enzyme assay

CYP121 activity on cyclodipeptides was detected by incubating at 30°C purified CYP121 (5 µM) and cyclodipeptides (∼ 0.1 mM) supplemented with 1 µM spinach ferredoxin (Sigma-Aldrich), 0.1 unit spinach ferredoxin-NADP⁺ reductase (Sigma-Aldrich), 1 mM NADPH (Sigma-Aldrich) in 60 mM K₂HPO₄/KH₂PO₄ at pH 7.2. Reaction was started by adding NADPH (100 mM stock solution) after 5 min incubation at 30°C. At various times, 80 µl were withdrawn and reaction was stopped by adding 5 µl 20% trifluoroacetic acid. Samples (50 µl) were analyzed by reverse phase HPLC (C 18 column 4.6 X 250 mm, Vydac) using a linear gradient from 0 to 50% CH₃CN in 0.1% trifluoroacetic acid. In some experiments, one of the compounds was omitted. The effect of clotrimazole (50 mM stock solution in DMSO) on CYP121 activity was tested by including 0.5 mM clotrimazole in sample before starting the reaction.

### 1.8 Crystallisation and diffraction data collection

Purified CYP121 (9 mg/ml; ∼ 200 µM) was crystallized by the seating drop method using condition similar to the previously described (20). Co-crystals of CYP121 in complex with cyclodipeptide were obtained in similar conditions with addition of cyclodipeptide in the protein solution at various concentrations depending upon its solubility. Final cyclodipeptide concentration was 2 mM, 2mM, 0.2 mM, 0.8 mM, and 1.5 mM for cyclo(L-Tyr-L-Tyr) (SEQ ID NO:4), cyclo(L-DOPA-L-Tyr) (SEQ ID NO:8), cyclo(L-Tyr-L-Phe) (SEQ ID NO:6), cyclo(L-Tyr-L-Ala) (SEQ ID NO:7), and cyclo(L-Tyr-L-Trp) (SEQ ID NO:5) respectively.

Final DMSO concentration was less than 2%. The crystals were frozen in liquid nitrogen, and all data set were collected at the European Synchrotron Radiation Facility (Grenoble) on beamline ID23-2 at resolution comprised between 1.4 Å and 1.9 Å. The data were processed with MOSFLM and scaled with SCALA from CCP4 package (21). Despite the availability of CYP121 native structure, the structure of our native CYP121 was solved by molecular replacement with the program MOLREP from CCP4 (22) using the available structure of CYP121 as a search model (Protein Data Bank entry 1N40) and the native data set. The structure was refined with REFMAC from CCP4 (23) and inspected with the program TURBO-FRODO (24).

In the case of the complexes, each cyclodipeptide was built at the end of the refinement process based on the Fo-Fc electron density map. The quality of the electron density was compatible with a complete building of the molecule except in the case of cyclo(L-Tyr-L-Trp) (SEQ ID NO:5) that adopts an alternate conformation. X-ray analysis statistics are summarized in Table I.

The complete coordinate data for each of these models is provided in figures 19, 20, 21, 22 and 23 for each of cyclo(L-Tyr-L-Tyr) (SEQ ID NO:4), cyclo(L-DOPA-L-Tyr) (SEQ ID NO:8), cyclo(L-Tyr-L-Phe) (SEQ ID NO:6), cyclo(L-Tyr-L-Ala) (SEQ ID NO:7), and cyclo(L-Tyr-L-Trp) (SEQ ID NO:5) in combination with CYP121 respectively.

### EXAMPLE 2 - INHIBITION OF CYP121 ACTIVITY BY CYCLO(L-TYR-L-TYR) ANALOGS

### 2.1 Cyclo(L-Tyr-L-Tyr) exhibit type I binding to CYP121

In order to address whether cyclo(L-Tyr-L-Tyr) (SEQ ID NO:4) could be the substrate of CYP121, the inventors determined the effects of cyclo(L-Tyr-L-Tyr) (SEQ ID NO:4) on the spectral properties of purified CYP121 by performing spectral binding titrations. Substrate binding to P450 causes shifts in spin equilibrium of heme iron that can be monitored by UV/Vis spectroscopy (27).

The inventors expressed and purified CYP121 essentially as previously described (17). Amino terminal sequencing of purified CYP121 indicated the loss of the initial methionine that is in agreement with the molecular mass determined by electrospray ionization mass spectrometry (43127.5 ± 0.9 Da). Absolute spectra of the oxidized CYP121 and the ferrous-carbon monoxy form were found identical to those previously described (not shown).

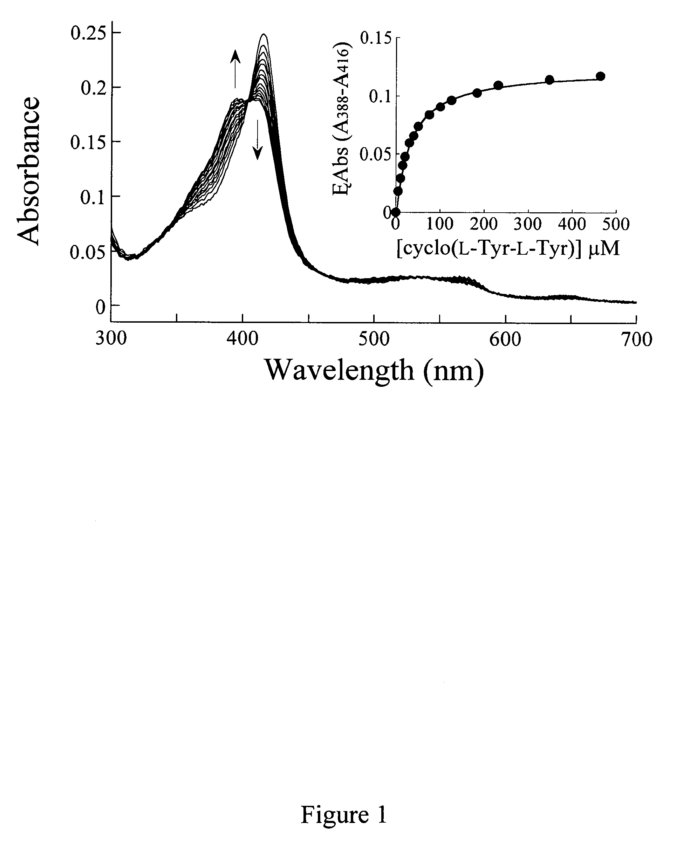

Addition of increasing concentrations of cyclo(L-Tyr-L-Tyr) (SEQ ID NO:4) to a 2 µM CYP121 solution induces a shift of the major Soret band centred at 416 nm toward 388 nm with an isobestic point at 406 nm and the appearance of the small 645 nm charge-transfer absorption band (Fig. 1). These spectral variations are typical of substrate binding (type I binding) and reflect the shift of the heme iron from the low spin form to the high spin form (27). The binding constant (Ks) was then determined by plotting the overall absorbance variations (ΔA₃₈₈-ΔA₄₁₆) versus the cyclodipeptide concentration and was found to be 27.6 ±3.4 µM.

In figure 1 the spectral changes induced on titration of CYP121 (2 µM) with cyclo(L-Tyr-L-Tyr) (0-463 µM) are shown. Arrows indicate directions of change of spectra observed upon cyclodipeptide additions. Difference spectrum was generated for each cyclodipeptide concentration in the cuvette by subtracting the initial spectrum to the spectrum obtained for each cyclodipeptide concentration. From these difference spectra, the overall absorbance variation ΔAbs(A₃₈₈-A₄₁₆) was calculated for each cyclodipeptide concentration by subtracting the absorbance variation at 416 nm to the absorbance variation at 388 nm. The overall absorbance variations plotted against cyclo(L-Tyr-L-Tyr) (SEQ ID NO:4) concentration are represented in the insets. They were fitted to a rectangular hyperbola that was used to calculate the binding constant.

### 2.2 Cyclo(L-Tyr-L-Tyr) is transformed by CYP121

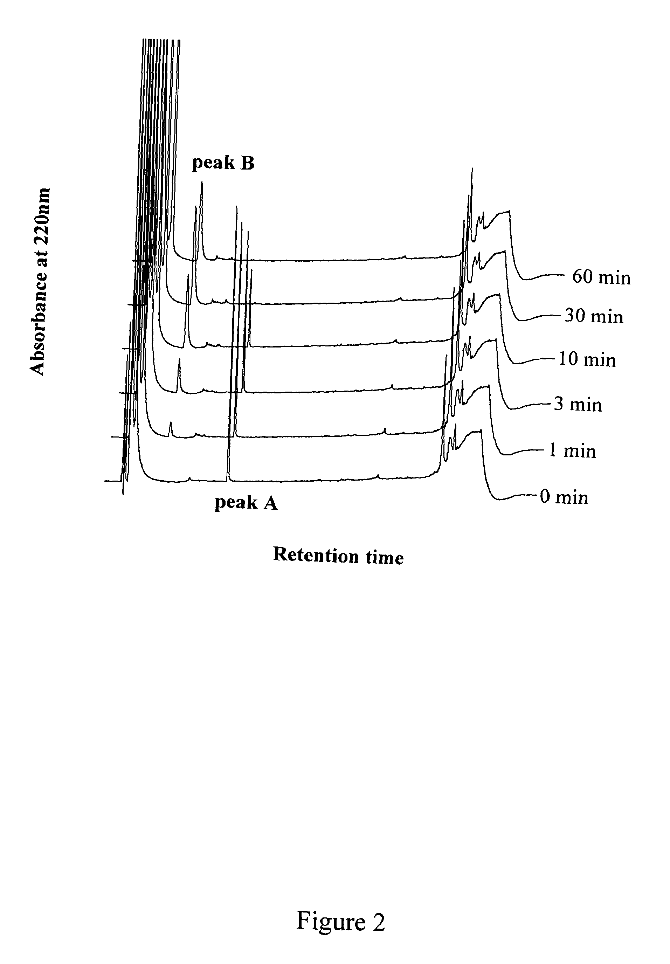

Transformation of cyclo(L-Tyr-L-Tyr) (SEQ ID NO:4) by CYP121 was assessed *in vitro* by using purified CYP121 supplemented with an exogenous electron transport chain and NADPH. Incubation of 0.11 mM cyclo(L-Tyr-L-Tyr) (SEQ ID NO:4) with CYP121 and an exogenous electron transport chain resulted in the complete consumption of cyclo(L-Tyr-L-Tyr) (SEQ ID NO:4) in 30 min (peak A in Fig. 2 as identified by MS/MS analysis; retention time ∼ 22 min) with the concomitant appearance of a more hydrophilic product with a retention time ∼ 12 min (peak B in Fig. 2).

In figure 2 RP-HPLC chromatograms were recorded after incubation of CYP121, an electron transport chain, NADPH and cyclo(L-Tyr-L-Tyr) (SEQ ID NO:4) at 30°C for various times (0 min, 1 min, 3 min, 10 min, 30 min and 60 min). Reaction was stopped by acidification as described above, before analysis. Peak A (retention time - 22 min) corresponds to cyclo(L-Tyr-L-Tyr) (SEQ ID NO:4) as determined by MS/MS analysis. Peak B corresponds to a product that appears upon incubation and displays a retention time ∼ 12 min.

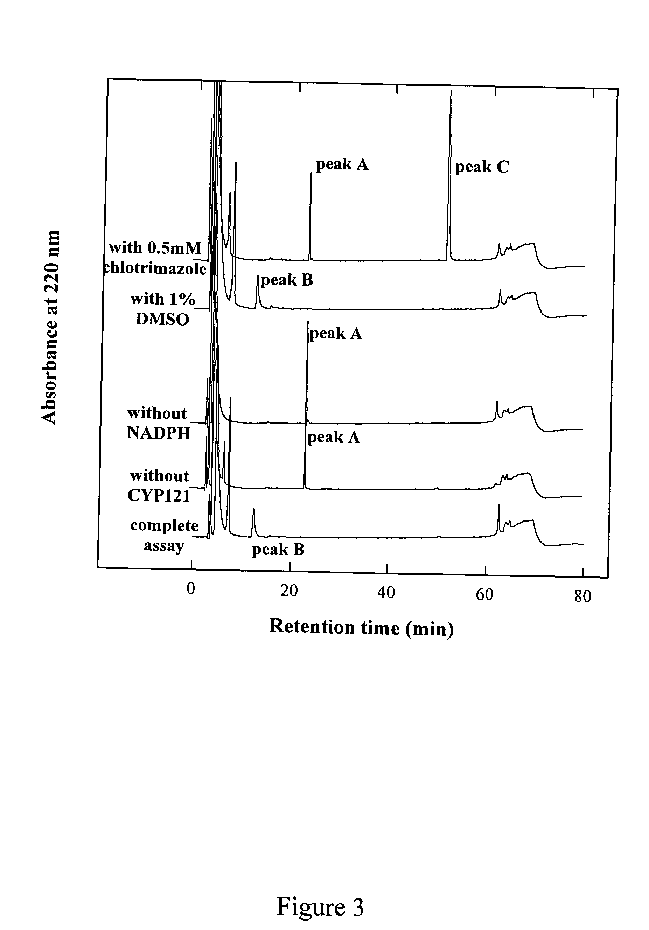

The omission of CYP121 or NADPH in the sample tube resulted in the absence of consumption of cyclo(L-Tyr-L-Tyr) (SEQ ID NO:4) after 60 min incubation and no product appeared with a retention time ∼ 12 min (Fig. 3).

In figure 3 transformation of cyclo(L-Tyr-L-Tyr) (SEQ ID NO:4) was assessed as described in Fig. 2 (complete assay) and either some constituents of the reaction mixture were omitted (without CYP121; without NADPH) or chemicals were added (with 1% DMSO; with 0.5 mM clotrimazole). Samples were incubated 60 min at 30°C and reaction was stopped by acidification before RP-HPLC analysis. Peak A and peak B correspond to the same products than for peaks A and B described in Fig. 2, respectively. Their identity was confirmed by mass analysis.

Incubation in the presence of 0.5 mM clotrimazole, a P450 inhibitor displaying high affinity for CYP121, resulted in the absence of detection of cyclo(L-Tyr-L-Tyr) (SEQ ID NO:4) transformation after 60 min, while the addition of DMSO corresponding to the same volume of added clotrimazole had no effect (Fig. 3). These results clearly indicate that CYP121 metabolizes cyclo(L-Tyr-L-Tyr) (SEQ ID NO:4)when supplemented with an exogenous electron transport chain, thus showing that cyclo(L-Tyr-L-Tyr) (SEQ ID NO:4) is a substrate for CYP121.

There have been very few previous reports of metabolic pathways involving cyclodipeptides transformation by P450 enzymes. The P450 cytochrome CypX of *Bacillus subtilis* is suspected to be involved in the double-oxygenation of the DKP ring of cyclo(Leu-Leu) in the pulcherrimin biosynthetic pathway, resulting in the pucherriminic acid (35).

However, several observations suggest that CYP121 does not catalyse the same reaction as CypX. First, the two cytochromes share only ∼ 22% identity and 40% similarity over the entire sequence. Second, the positioning of cyclo(L-Tyr-L-Tyr) (SEQ ID NO:4) in CYP121 active site is not consistent with DKP ring oxygenation but rather with an attack on one tyrosyl moiety. Third, preliminary data on the characterization of the product resulting from CYP121 activity on cyclo(L-Tyr-L-Tyr) (SEQ ID NO:4) indicate a mass difference of 2 Da that is not consistent with a double-oxygenation.

### 2.3 X-ray structure of cyclo(L-Tyr-L-Tyr)-bound CYP121

In order to obtain information at a molecular level, the inventors solved the crystal structure of native CYP121 and in complex with cyclo(L-Tyr-L-Tyr) (SEQ ID NO:4). The crystals of native CYP121 and cyclo(L-Tyr-L-Tyr)-bound CYP121 belong to the same crystal form (space group = P6₅22) as previously described for unbound CYP121 (13). The structure of native CYP121 and CYP121/cyclo(L-Tyr-L-Tyr) were solved and refined at 1.4 Å resolution (Table I) demonstrating that the quality of the structure is compatible with fine structural analysis.

**Table I : Data collection and refinement statistics.**

| | **Cyclodipeptide bound to CYP121** | | | | | |
|---|---|---|---|---|---|---|
| | none | c(L-Tyr-L-Tyr) (SEQ ID NO:4) | c(L-DOPA-L-Tyr) (SEQ ID NO:8) | c(L-Tyr-L-Trp) (SEQ ID NO:5) | c(L-Tyr-L-Phe) (SEQ ID NO:6) | c(L-Tyr-L-Ala) (SEQ ID NO:7) |
| **Data collection** | | | | | | |
| Resolution limits (outer shell) | 1.4 (1.48-1.4) | 1.4 (1.48-1.4) | 1.4 (1.48-1.4) | 1.4 (1.48-1.4) | 1.8 (1.9-1.8) | 1.9 (2.0-1.9) |
| Rmerge | 0.078 (0.347) | 0.069 (0.620) | 0.099 (0.285) | 0.092 (0.346) | 0.152 (0.541) | 0.146 (0.435) |
| Total number of observations | 1130964 (111928) | 716613 (54443) | 389209 (33474) | 1099644 (100350) | 296243 (47663) | 206187 (32849) |
| Total number of unique | 93031 (13293) | 88013 (9517) | 86350 (10768) | 93373 (13226) | 44879 (6445) | 37824 (5442) |
| Mean (I) / sd(I) | 21.9 (5.3) | 19.6 (2.8) | 11.5 (2.8) | 18.1 (4.4) | 9.6 (3.4) | 10.4 (3.9) |
| Completeness | 100 (99.9) | 94.6 (71.8) | 92.5 (80.7) | 99.8 (99.0) | 99.8 (100.0) | 98.4 (99.6) |
| Multiplicity | 12.2 (8.4) | 8.1 (5.7) | 4.5 (3.1) | 11.8 (7.6) | 6.6 (7.4) | 5.5 (6.0) |

| **Molecular Replacement:** | | | | | | |
|---|---|---|---|---|---|---|
| Rotation: Rf / sigma (second solution) | 9.8 (2.8) | | | | | |
| Translation: Rf Correlation | 0.356 0.681 | | | | | |

| **Refinement:** | | | | | | |
|---|---|---|---|---|---|---|
| R-factor | 0.1588 | 0.1510 | 0.1723 | 0.1535 | 0.1776 | 0.1624 |
| R-free | 0.1902 | 0.1806 | 0.1982 | 0.1794 | 0.2285 | 0.2043 |
| Figure of merit | 0.9097 | 0.9171 | 0.8972 | 0.9224 | 0.8910 | 0.8952 |
| Total number of atoms | 3897 | 3816 | 3823 | 3872 | 3735 | 3751 |
| Number of water molecules | 722 | 683 | 674 | 722 | 588 | 610 |
| Rmsd bond | 0.011 | 0.012 | 0.011 | 0.010 | 0.010 | 0.010 |
| Rmsd angle | 1.418 | 1.436 | 1.389 | 1.327 | 1.196 | 1.273 |

For structural comparison, the inventors used a structure of ligand-free CYP121 determined in their laboratory, highly similar to the available CYP121 structure (entry 1N40; rms deviation on 388 Cα of 0.09 Å). Slight differences between the two ligand-free structures are observed at few amino acids side chains and at the dioxygen that is absent in the inventors structure (13). The overall structure of cyclo(L-Tyr-L-Tyr)-bound CYP121 is highly conserved compared with native CYP121 with rms deviation on 388 Cα of 0.16 Å.

No significant structural variations are observed in particular for secondary structures known to be sensitive to substrate binding. The putative substrate access channels (28) remain in a native conformation with no modification of temperature factors and the I helix shows no alteration of its hydrogen bonding patterns.

The cyclo(L-Tyr-L-Tyr) (SEQ ID NO:4) occupies the binding pocket with a full occupancy according to the B-factor of the ligand (10 Å²) that remains in the same order than the neighbouring residues after refinement. The DKP ring adopts a boat conformation with one tyrosyl moiety pointing between helices F and G (the distal tyrosine) and the other tyrosyl part lying above the heme (the proximal tyrosine) (Fig. 4).

Figure 4 shows the X-ray structure of cyclo(L-Tyr-L-Tyr) (SEQ ID NO:4) bound CYP121. In panel A, the overall CYP121 holoprotein is represented as ribbons and atoms of the heme are shown as spheres. Cyclo(L-Tyr-L-Tyr) (SEQ ID NO:4) is depicted as sticks. The names of the helices that are in contact with cyclo(L-Tyr-L-Tyr) (SEQ ID NO:4) are indicted. A detailed view of the heme binding pocket is shown in panel B. Residues surrounding this pocket are indicated.

From figure 4, the DKP ring establishes contacts with residues of the B' helix and one propionate chain of the heme. One nitrogen of the DKP cycle is bridged to the second carboxylate group of the heme and to the main chain azote of Met86 through the same water molecule (WAT68). One carbonyl of the DKP cycle interacts with the NH1 of Asn85, and the second carbonyl with a water molecule that belongs to a water molecules network.

The distal tyrosine shows very low temperature factor (8 Å² while the whole complex shows a temperature factor of 15 Å²). It points between helices F and G with stacking interaction with Phe168 and Trp182. The hydroxyl interacts first with the main chain oxygen of Thr77 through a water-bridged hydrogen bond (*via* WAT40) and second with a water molecule that also belongs to a water molecules network.

The proximal tyrosine ring is located close to the heme with the hydroxyl and the CE carbon atom positioned at 6.25 Å and 6.07 Å from the iron, respectively. This proximal ring approaches the iron from the side parallel to the heme, a position suitable for further attack by the active iron-oxygen species. This ring tightly interacts with the heme and is also implicated in a large network of stacking interaction that involves Trp182, the distal tyrosyl residue, Phe168, Phe280 and the proximal tyrosyl moiety.

Furthermore, the hydroxyl interacts directly with the NH1 from the guanidinium of Arg386, with a water molecule (WAT46) that is stabilized by interactions with the NE2 from glutamine 385 and the NH2 from the guanidinium of Arg386, and with the iron of the heme through two closely H-bonded water molecules (WAT2 and WAT1). In addition, we observe a π-cation delocalization between the ring of the proximal tyrosyl moiety and a cation that could be an NH4⁺, and that bridges the ring with one carboxylate group of the heme. Therefore, altogether this network is very favourable to the stabilization of the cyclo(L-Tyr-L-Tyr) (SEQ ID NO:4) through its two tyrosyl residues.

Although a common scheme of the P450 catalytic cycle is now generally accepted, there are numerous differences in the molecular mechanisms that lead to the scission of dioxygen and formation of the reactive species (36). In this scheme, substrate binding prepares the heme pocket for oxygen binding and is often accompanied by structural changes and the positioning of catalytic water molecules. CYP121 is particularly interesting because of the specificity of the oxygen binding site previously observed in the ligand-free crystal structure that suggests a novel mode of oxygen activation (13).

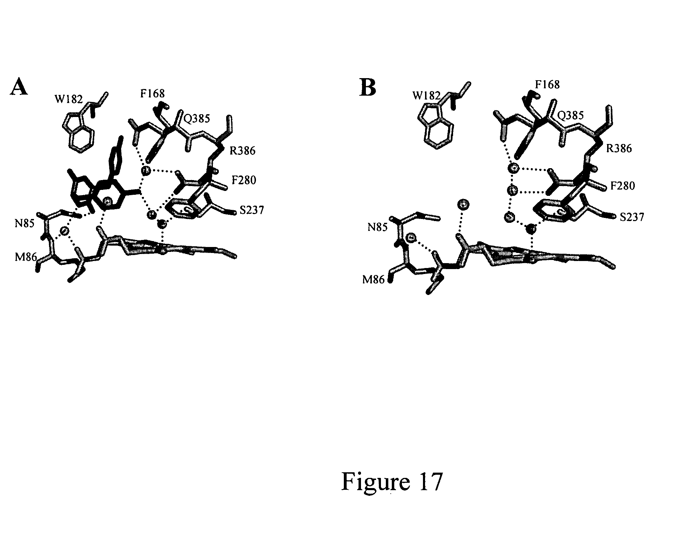

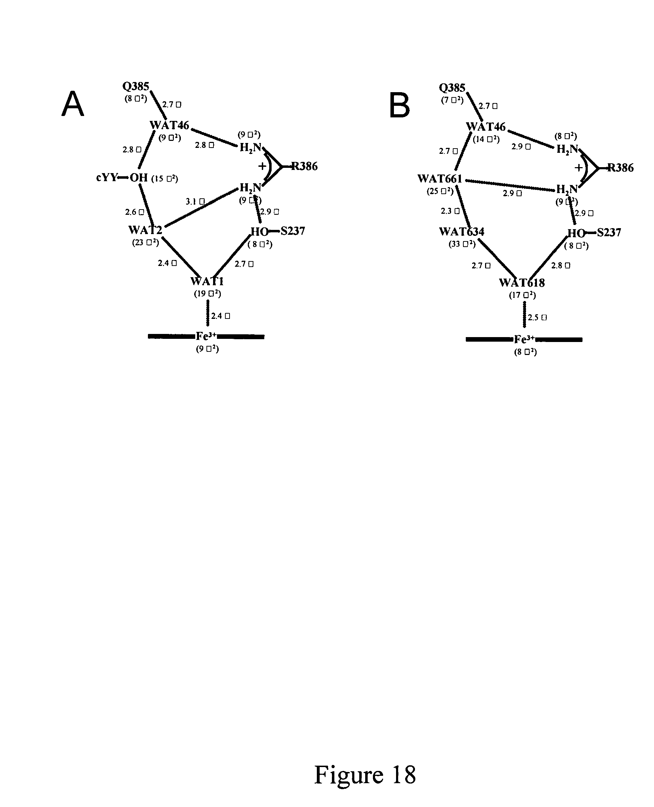

The absence of significant structural changes upon ligand binding to CYP121 confirms the unusual rigidity of CYP121 highlighted in previous studies (13, 14). Moreover, binding of the substrate cyclo(L-Tyr-L-Tyr) (SEQ ID NO:4) to CYP121 proceeds through an original mode of heme ligation involving the two propionate groups and a H-bonded water network between one substrate hydrolyl and the heme iron (Fig. 17a and Fig. 18a).

Figure 17 shows the crystal structure of cyclo(L-Tyr-L-Tyr)-bound and native CYP121 active sites. Detailed views in stick representation of the active sites of CYP121 in complex with cyclo(L-Tyr-L-Tyr) (panel A) and without ligand (panel B) are presented. Cyclodipeptide is shown in black, the heme and CYP121 residues are in grey. Water molecules are represented as grey spheres. Probable H-bonds between CYP121, cyclodipeptide and non-bonded molecules are indicated by dotted lines.

Figure 18 shows a schematic representation of the oxygen binding site of substrate-bound (panel A) and ligand-free (panel B) CYP121. H-bonds between the iron (Fe³⁺), water molecules (WAT), CYP121 residues and the cyclo(L-Tyr-L-Tyr) (cYY-OH) are shown by dotted lines. B factor values are indicated in brackets.

Consequently, the heme iron water sixth ligand is not displaced, as indicated by B factor value of the corresponding molecule in the structure. As in the native enzyme, the heme iron water sixth ligand remains H-bonded to the hydroxyl of Ser237 and this peculiar feature of CYP121 could account for the difficulties to expulse the iron sixth ligand as also observed in fluconazole-bound CYP121 (14).

The presence of the Arg386 side chain above the oxygen binding site is another unusual feature of CYP121 and it was supposed to be linked to proton transfer (13). Upon substrate binding Arg386 guanidinium becomes linked to heme iron through two water molecules that is consistent with a role in proton transfer (Fig. 17 and Fig. 18). The presence of one hydroxyl of the substrate closed to the oxygen binding site is another feature of CYP 121 that could be implicated in proton transfer.

Previous studies also highlighted the role of a substrate hydroxyl in proton transfer to the iron-bound dioxygen for P450 EryF and CYP158A2 (37,38). In these cases, the presence of substrate hydroxyl close to the dioxygen binding site is involved either in the positioning of a catalytic water or in the direct ligation of the distal oxygen of iron-bound dioxygen. In CYP121/cyclo(L-Tyr-L-Tyr) complex the substrate hydroxyl is sufficiently close to the iron (6.25 Å) so that it is not possible to exclude a role in proton transfer. It is assumed that binding of dioxygen to CYP121 will modify the water network around the iron and it is difficult to predict the resulting organization. However, it is noteworthy to observe that the orientation of the two water molecules bridging the iron and the substrate hydroxyl and the angle formed by these two water molecules and the iron (117°) are very close to the orientation of dioxygen and the angle formed by atoms of dioxygen and the iron (120°) in the previously published CYP121/dioxygen complex (14): these water molecules could mimic the orientation of the dioxygen molecule in ferrous CYP121.

If so, the distal oxygen would be in interaction with the guanidinium of Arg386 and the hydroxyl of the substrate that could both donate hydrogen for dioxygen scission. In this hypothesis, the presence of this positively charged guanidinium at 3.5-4.5 Å from the hydroxyl of one tyrosyl moiety of the substrate raises the intriguing possibility of another role for Arg386: assuming that one proton originates from the hydroxyl of the substrate to participate to dioxygen scission as hypothesized for P450EryF (34), the phenolate anion that could form would first be stabilized by the guanidinium and second induce increased reactivity of position ortho to the hydroxyle. Ser237 that occupies the position of a highly conserved catalytic threonine could hence be involved in direct ligation of the proximal oxygen through its hydroxyl, a situation that has not been previously observed with the hydroxyl of the conserved threonine in other P450s. Such interaction could help with the scission of dioxygen. The resolution of the ferrous dioxygen complex structure will shed light on the role of the hydroxyl of the substrate in O₂ activation and help to precise the function of Arg386 and Ser237 in this mechanism.

The presence of this water sixth ligand raises the question of the molecular mechanisms inducing the low-to-high spin transition observed by spectrophotometry during CYP121 titration by cyclo(L-Tyr-L-Tyr) (SEQ ID NO:4). Substrate binding usually provokes the expulsion of the water sixth ligand resulting in a modification of the heme iron coordination and the appearance of high spin ferric signal necessary to the initiation of the catalytic cycle (4).

Even in the presence of a large excess of substrate, the spin transition is not complete and CYP121 is composed of a mixed population of high and low spin ferric heme iron indicating the difficulty to get a full high spin species (see Fig. 1). Furthermore, it was observed that the spin equilibrium of CYP121 is temperature-dependent with a reduction of the absorption peak at 416 nm and the concomitant enhancement of the absorption peak at 388 nm while increasing temperature (data not shown). Furthermore, the structural data indicate neither a significant variation of Fe-sixth ligand distance nor an increased mobility of the iron sixth ligand, modifications that were previously suspected to induce high spin formation (36).

Previous studies on P450cam suggested that a modification of the OH⁻/H₂O equilibrium of the iron sixth ligand could result in a diminution of the ligand field strength and then the increase of high spin (36). In CYP121/cyclo(L-Tyr-L-Tyr) complex, such modification of the equilibrium could be favoured by the H-bonds network due to substrate binding and specially by guanidinium of Arg386 that is in interaction with the iron sixth ligand through one water molecule.

### 2.4 Inhibition of CYP121 activity by cyclo(L-Tyr-L-Tyr) substrate analog

One of the most convenient ways to inhibit P450 activity is the use of substrate analogs (29). To be efficient, these analogs should bind tightly to P450s and not be metabolized. The X-ray structure of cyclo(L-Tyr-L-Tyr)-bound CYP121 clearly indicates that cyclo(L-Tyr-L-Tyr) (SEQ ID NO:4) is anchored to CYP121 *via* hydrophobic interactions implicating the two tyrosyl moiety but also via ionic interactions involving essentially the two hydroxyl groups.

In the design of a first analog to be tested for CYP121 activity inhibition, the inventors choose to conserve the overall cyclo(L-Tyr-L-Tyr) scaffold and to add only a small chemical group. They hence decided to synthesize the cyclo(L-3 or 5,4-dihydroxyphenylalanine-L-Tyr) (SEQ ID NO:8) referred to as cyclo(L-DOPA-L-Tyr) (Fig. 5 shows its structure) and to test it as a substrate analog candidate. Cyclo(L-DOPA-L-Tyr) (SEQ ID NO:8) was synthesized as described in the experimental procedure and its identity was verified by mass spectrometry analysis.

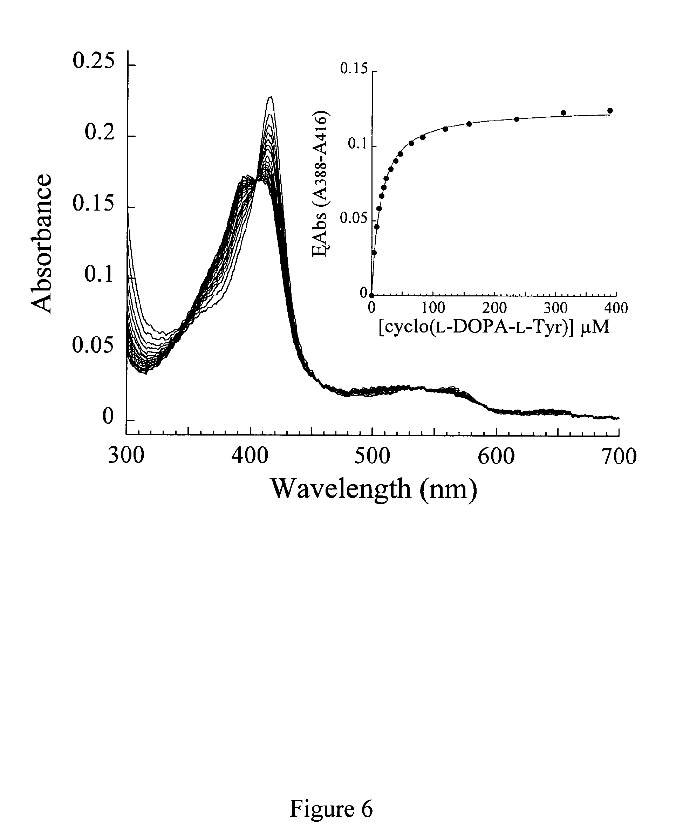

Binding of cyclo(L-DOPA-L-Tyr) (SEQ ID NO:8) to CYP121 was found to induce low-to-high spin transition as previously observed for cyclo(L-Tyr-L-Tyr) (SEQ ID NO:4) with binding constant (14.7 ± 1.5 µM) in the same order than Ks of cyclo(L-Tyr-L-Tyr) (SEQ ID NO:4) for CYP121 (Fig. 6).

Figure 6 show the spectral changes recorded upon addition of increasing amount of cyclo(L-DOPA-L-Tyr) (0 to 389 µM) are shown. The overall absorbance variations were obtained as described in figure 1 and plotted against cyclo(L-DOPA-L-Tyr) (SEQ ID NO:8) concentration. The data were fitted to a rectangular hyperbola to determine Ks.

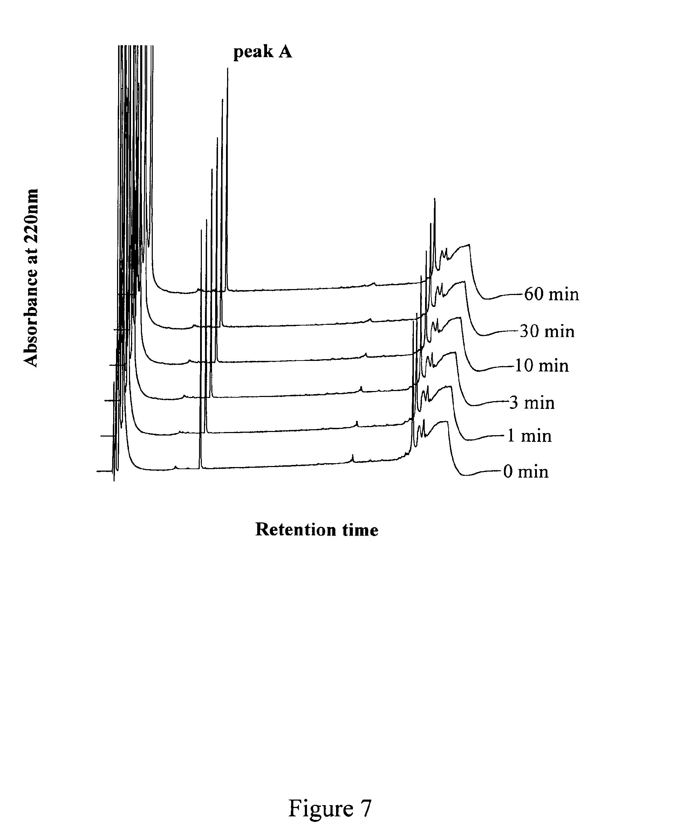

Unlike cyclo(L-Tyr-L-Tyr) (SEQ ID NO:4), no transformation was detected when cyclo(L-DOPA-L-Tyr) (SEQ ID NO:8) was incubated with CYP121 and an electron transport chain plus NADPH at 30°C for 60 min (Fig. 7, cyclo(L-DOPA-L-Tyr) (SEQ ID NO:8) compare peak A at 0 and 60 min).

Figure 7 shows the transformation of cyclo(L-DOPA-L-Tyr) (SEQ ID NO:8) by CYP121. 0.12 mM cyclo(L-DOPA-L-Tyr) (SEQ ID NO:8) was incubated with CYP121, an electron transport chain and NADPH at 30°C for various times (0 min, 1 min, 3 min, 10 min, 30 min and 60 min). Reaction was stopped by acidification before RP-HPLC analysis. Peak A (retention time ∼ 20 min) corresponds to cyclo(L-DOPA-L-Tyr) (SEQ ID NO:8) as determined by MS/MS analysis.

Clearly, the presence of one additional hydroxyl ortho to the hydroxyl group of one tyrosyl moiety of cyclo(L-Tyr-L-Tyr) (SEQ ID NO:4) completely impedes metabolization by CYP121, without significantly changing the binding constant to CYP121.

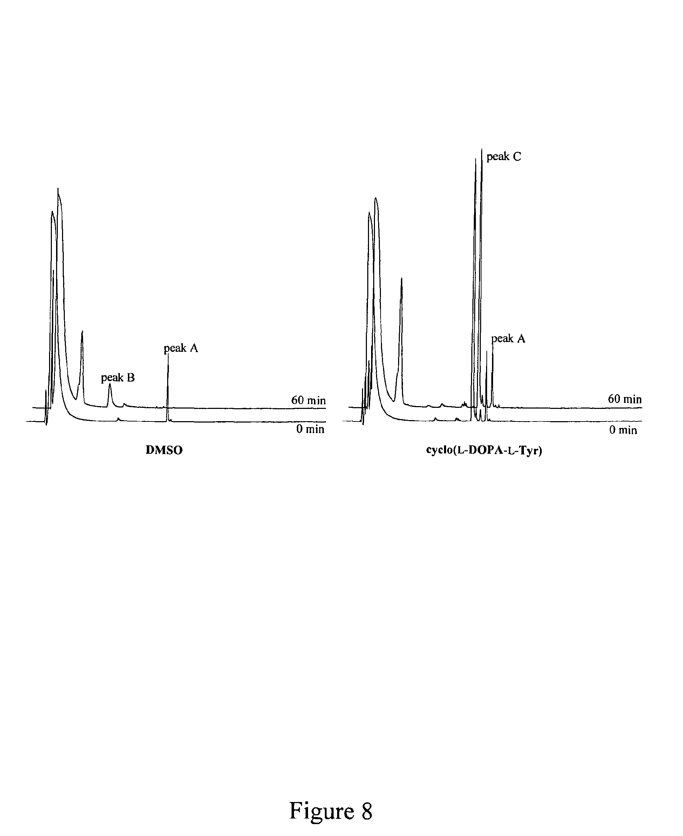

This substrate analog was then tested for its capacity to inhibit CYP121 activity. It was observed that the presence of 1.2 mM cyclo(L-DOPA-L-Tyr) (SEQ ID NO:8) completely inhibits cyclo(L-Tyr-L-Tyr) (SEQ ID NO:4) transformation (Fig. 8): after 60 min incubation at 30°C, no transformation of cyclo(L-Tyr-L-Tyr) (peak A in Fig. 8) was detected in the presence of cyclo(L-DOPA-L-Tyr) (peak C in Fig. 8) while cyclo(L-Tyr-L-Tyr) (SEQ ID NO:4) was completely converted into a product with a retention time ∼ 12 min (peak B in Fig. 8) in less than 30 min at 30°C when cyclo(L-DOPA-L-Tyr) (SEQ ID NO:8) was omitted. Altogether, these data clearly show that cyclo(L-DOPA-L-Tyr) (SEQ ID NO:8) is a good substrate analogue candidate to inhibit CYP121 activity *in vitro.*

Figure 8 shows the inhibition of CYP121 activity by cyclo(L-DOPA-L-Tyr) (SEQ ID NO:8). CYP121 was incubated with cyclo(L-Tyr-L-Tyr) (SEQ ID NO:4) as previously described, except that various compounds were added before starting the reaction: 5% DMSO or 1.2 mM cyclo(L-DOPA-L-Tyr) (SEQ ID NO:4). Samples were either withdrawn just before starting the reaction (0 min) or incubated 60 min at 30°C. Reaction was stopped by acidification and analysed by RP-HPLC. Peak A corresponds to cyclo(L-Tyr-L-Tyr) (SEQ ID NO:4), peak B to a compound with a retention time ∼ 12 min already observed in Fig. 2, peak C to cyclo(L-DOPA-L-Tyr). Identity of these peaks was established by mass analysis. Chromatograms are shown for elution times ranging from 0 to 50 min.

### 2.5 X-ray structure of cyclo(L-DOPA-L-Tyr)-bound CYP121

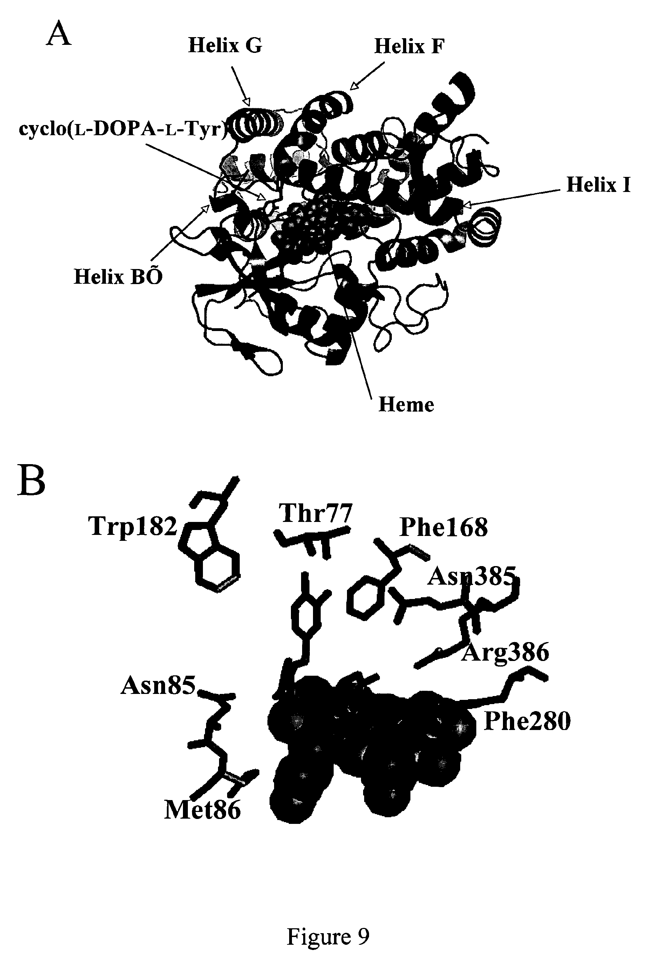

In order to find information at a molecular level, the inventors solved the crystal structure of cyclo(L-DOPA-L-Tyr)-bound CYP121. The crystals of cyclo(L-DOPA-L-Tyr)-bound CYP121 belong to the same crystal form as previously described for unbound CYP121 (space group = P6₅22). The structure of cyclo(L-DOPA-L-Tyr)-bound CYP121 was solved and refined at 1.4 Å resolution (Table I) demonstrating that the quality of the structure is compatible with fine structural analysis. In this complex, the atoms of cyclo(L-DOPA-L-Tyr) (SEQ ID NO:8) are superimposable to those of cyclo(L-Tyr-L-Tyr) (SEQ ID NO:4) in cyclo(L-Tyr-L-Tyr)-bound CYP121 except for the additional hydroxyl found in cyclo(L-DOPA-L-Tyr) (Fig. 9).

Figure 9 shows the X-ray structure of cyclo(L-DOPA-L-Tyr)-bound CYP121. In panel A, the overall CYP121 holoprotein is represented as ribbons and atoms of the heme are shown as spheres. Cyclo(L-DOPA-L-Tyr) (SEQ ID NO:8) is depicted as sticks. The names of the helices that are in contact with cyclo(L-DOPA-L-Tyr) (SEQ ID NO:8) are indicted. A detailed view of the heme binding pocket is shown in panel B. Residues surrounding this pocket are indicated.

All the contacts made between cyclo(L-Tyr-L-Tyr) (SEQ ID NO:4) and CYP121 in the crystal structure of cyclo(L-Tyr-L-Tyr)-bound CYP121 are found between cylo(L-DOPA-L-Tyr) (SEQ ID NO:4) and CYP121. The additional hydroxyl points toward a cavity located between the F/G loop and B/B' loop and establishes polar contacts with the carbonyl of Ala167, the hydroxyl of Thr77 and a water molecule.

### 2.6 Inhibitor design using the cyclo(L-Tyr-L-Tyr) scaffold

As demonstrated with the cyclo(L-DOPA-L-Tyr) (SEQ ID NO:8), it is possible to use the cyclo(L-Tyr-L-Tyr) (SEQ ID NO:4) molecule as a scaffold to find new molecules with inhibitory activity toward CYP121. The inventors propose that these molecules could be obtained i) by screening for CYP121 binding a chemical library obtained with the cyclo(L-Tyr-L-Tyr) (SEQ ID NO:4) cyclodipeptide as scaffold or ii) by any modelling method starting from X-ray structures of CYP121 bound to cyclo(L-Tyr-L-Tyr) (SEQ ID NO:4) or cyclo(L-DOPA-L-Tyr) (SEQ ID NO:8) or any cyclo(L-Tyr-L-Tyr)-derived molecule, and followed by chemical synthesis. By using these modelling methods, chemical groups could be designed to fit each of the three water-filled cavities surrounding the position occupied either by the ligand in the cyclo(L-Tyr-L-Tyr)-bound CYP121 and cyclo(L-DOPA-L-Tyr)-bound CYP121 structures (the first cavity located between helices F and G is bordered by residues S163 L164 I166 A167 F168 I175 A178 K179 N181 W182 D185; the second cavity located between the F/G loop and B/B' loop is bordered by residues R72 L73 N74 L76 V78 A167 F168 M169 S 170 S 171 W182; the third cavity located just above the heme is bordered by residues M62 R72 L73 N74 L76 T77 F168 A233 I236 S237 F280 D282 G283 L284 P285 Q385 R386 and the heme).

By using these methods, chemical groups could also be designed to link covalently the cyclo(L-Tyr-L-Tyr) scaffold to CYP121, leading to the conception of suicide or mechanism-based inhibitors (30). These modelling methods could also be used to design molecules with chemical groups in tight interaction with the heme iron through conjugation of electron pair (like azole molecules).

### EXAMPLE 3 - INHIBITION OF CYP121 ACTIVITY BY CYCLO(L-TYR-L-TRP) ANALOGS

### 3.1 Cyclo(L-Tyr-L-Trp) binding to CYP121 and transformation by CYP121

The inventors went onto study whether cyclodipeptides other than the CYP121 substrate cyclo(L-Tyr-L-Tyr) (SEQ ID NO:4) could bind to CYP121 and be transformed.

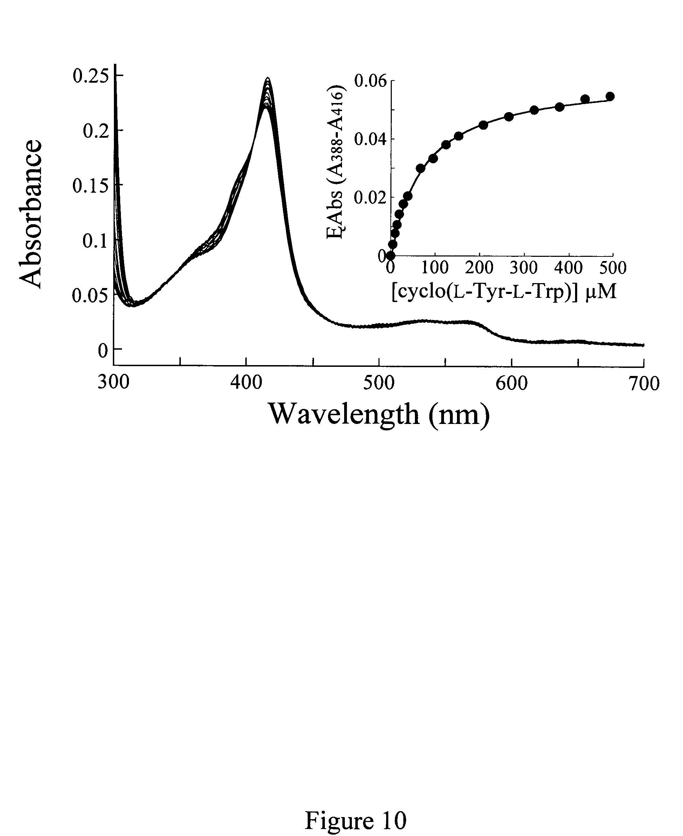

Such cyclodipeptides could provide a scaffold for the conception of substrate analogs other than those derived from the substrate cyclo(L-Tyr-L-Tyr) (SEQ ID NO:4). As example, the inventors focused their attention on the cyclo(L-Tyr-L-Trp) (SEQ ID NO:5). Binding was quantified by measuring the spectral variations obtained upon addition of increasing concentration of cyclodipeptide to a 2 µM CYP121 solution. Upon addition of cyclo(L-Tyr-L-Trp) (SEQ ID NO:5) we observe the shift of the major Soret band to 388 nm but in very much less extent than with cyclo(L-Tyr-L-Tyr) (SEQ ID NO:4) showing different capacities of cyclodipeptides to induce high spin formation (Fig. 10). The binding constant (Ks) was then determined by plotting the overall absorbance variations (ΔA₃₈₈-ΔA₄₁₆) versus the cyclodipeptide concentration and was found to be 63.4 ± 8.8 µM.

Figure 10 shows the spectral changes induced on titration of CYP121 (2 µM) with cyclo(L-Tyr-L-Trp) (0-492 µM) are shown. Arrows indicate directions of change of spectra observed upon cyclodipeptide additions. Difference spectrum was generated for each cyclodipeptide concentration in the cuvette by subtracting the initial spectrum to the spectrum obtained for each cyclodipeptide concentration. From these difference spectra, the overall absorbance variation ΔAbs(A₃₈₈-A₄₁₆) was calculated for each cyclodipeptide concentration by subtracting the absorbance variation at 416 nm to the absorbance variation at 388 nm. The overall absorbance variations plotted against cyclo(L-Tyr-L-Trp) (SEQ ID NO:5) concentration are represented in the insets. They were fitted to a rectangular hyperbola that was used to calculate binding constant.

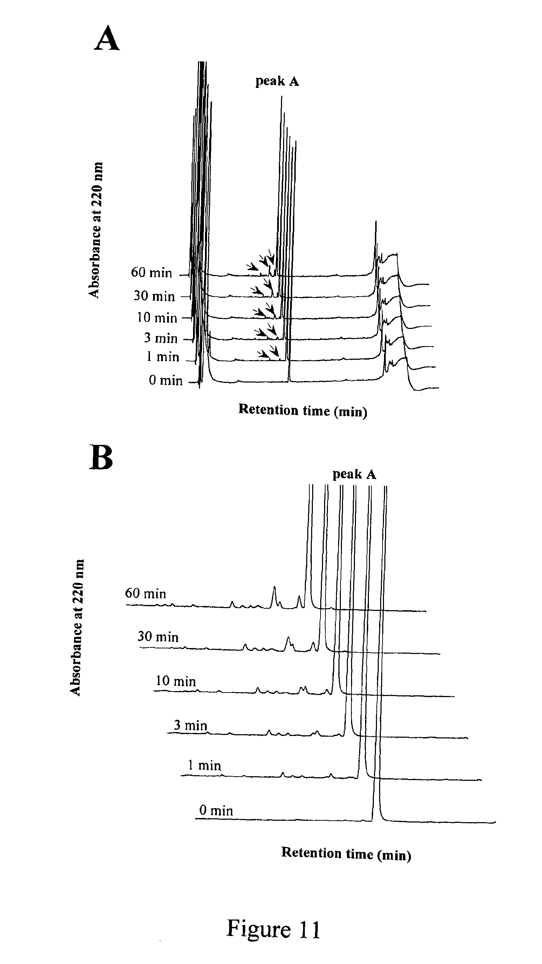

The transformation of cyclo(L-Tyr-L-Trp) (SEQ ID NO:5) by CYP121 was assessed as described previously for cyclo(L-Tyr-L-Tyr) (SEQ ID NO:4) except that cyclo(L-Tyr-L-Tyr) (SEQ ID NO:4) was replaced by cyclo(L-Tyr-L-Trp) (SEQ ID NO:5). Cyclo(L-Tyr-L-Trp) (SEQ ID NO:5) was incubated at 30°C with CYP121, an electron transport chain and NADPH. At various times, sample was withdrawn, acidified with TFA and analysed by RP-HPLC (Fig. 11). After 1-hour incubation, a large proportion of initial cyclo(L-Tyr-L-Trp) (SEQ ID NO:5) is always present in sample (peak A in Fig. 11). However, some additional peaks can be detected on the chromatograms corresponding to samples incubated 1 min to 60 min when compared to the chromatogram corresponding to sample without incubation. These data show that cyclo(L-Tyr-L-Trp) (SEQ ID NO:5) can be transformed by CYP121, albeit less efficiently than cyclo(L-Tyr-L-Tyr) (SEQ ID NO:4).

Figure 11 shows cyclo(L-Tyr-L-Trp) (SEQ ID NO:5) transformation by CYP121. 0.12 mM cyclo(L-Tyr-L-Trp) (SEQ ID NO:5) were incubated with CYP121, an electron transport chain and NADPH at 30°C for various times (0 min, 1 min, 3 min, 10 min, 30 min and 60 min). Reaction was stopped by acidification before RP-HPLC analysis. Peak A (retention time ∼ 32 min) corresponds to cyclo(L-Tyr-L-Trp) (SEQ ID NO:5) as determined by MS/MS analysis. Arrows indicate peaks that appear in samples upon incubation. Panel A represents a full view of the chromatograms and panel B a detailed view of the chromatograms with retention time ranging from 20 min to 40 min.

### 3.2 Inhibition of CYP121 activity by cyclo(L-Tyr-L-Trp)

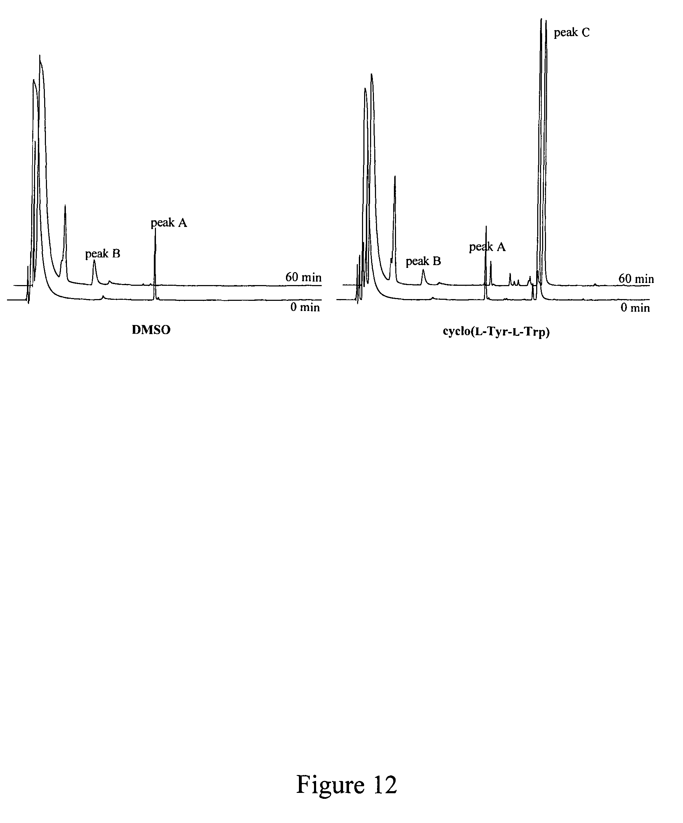

The inventors tested the effect of cyclo(L-Tyr-L-Trp) (SEQ ID NO:5) on CYP121 activity. Effectively, cyclo(L-Tyr-L-Trp) (SEQ ID NO:5) binds to CYP121 with affinity in the same order than cyclo(L-Tyr-L-Tyr) (SEQ ID NO:5)but is not significantly metabolized by CYP121. A CYP121 activity assay was realized as described previously with 0.11 mM cyclo(L-Tyr-L-Tyr) (SEQ ID NO:4), and 1.2 mM cyclo(L-Tyr-L-Trp) (SEQ ID NO:5) was added or not. In an additional sample without cyclo(L-Tyr-L-Trp) (SEQ ID NO:5), a volume of DMSO (corresponding to the volume of cyclo(L-Tyr-L-Trp) (SEQ ID NO:5) added previously) was added. After 60 min incubation at 30°C, reaction was stopped by acidification and samples were analysed by RP-HPLC (Fig. 12).

Figure 12 shows the inhibition of CYP121 activity by cyclo(L-Tyr-L-Trp) (SEQ ID NO:5). CYP121 was incubated with cyclo(L-Tyr-L-Tyr) (SEQ ID NO:4) as previously described, except that various compounds were added before starting the reaction: 5% DMSO or 1.2 mM cyclo(L-Tyr-L-Trp) (SEQ ID NO:5). Samples were either withdrawn just before starting the reaction (0 min) or incubated 60 min at 30°C. Reaction was stopped by acidification and analysed by RP-HPLC. Peak A corresponds to cyclo(L-Tyr-L-Tyr) (SEQ ID NO:4), peak B to a compound with a retention time ∼ 12 min already observed in Fig. 2, peak C to cyclo(L-Tyr-L-Trp) (SEQ ID NO:5). Chromatograms are shown for elution times ranging from 0 to 50 min.

While DMSO had no effect on the transformation of cyclo(L-Tyr-L-Tyr) (SEQ ID NO:4) by CYP121, the addition of cyclo(L-Tyr-L-Trp) (SEQ ID NO:5) resulted in a less efficient production of the compound with a retention time ∼ 12 min and an incomplete transformation of cyclo(L-Tyr-L-Tyr) (SEQ ID NO:4): in the presence of cyclo(L-Tyr-L-Trp) (SEQ ID NO:5), ∼30 % of initial cyclo(L-Tyr-L-Tyr) (SEQ ID NO:4) was detected after 60 min incubation while it was completely absent after 30 min incubation in the absence of cyclo(L-Tyr-L-Trp) (SEQ ID NO:5). Clearly, the addition of cyclo(L-Tyr-L-Trp) (SEQ ID NO:5) diminishes the efficiency of cyclo(L-Tyr-L-Tyr) (SEQ ID NO:4) transformation in the CYP121-catalysed reaction, indicating that cyclodipeptides could inhibit the reaction catalysed by CYP 121.

### 3.3X-ray structure of cyclo(L-Tyr-L-Trp)-bound CYP121

The crystals of cyclo(L-Tyr-L-Trp)-bound CYP 121 belong to the same crystal form (space group = P6₅22) as previously described for unbound CYP 121 (13). The structure of CYP121/cyclo(L-Tyr-L-Trp) was solved and refined at 1.4 Å resolution (Table I) demonstrating that the quality of the structure is compatible with fine structural analysis.

For structural comparison, the inventors used the structure of ligand-free CYP121 determined in our laboratory, highly similar to the available CYP121 structure (entry 1N40; rms deviation on 388 Cα of 0.09 Å). Two conformations of cyclo(L-Tyr-L-Trp) (SEQ ID NO:5) have been modelled based on the electron density, and their relative occupancies have been adjusted to 0.3 (conformation 1) and 0.7 (conformation 2) respectively in order to have B-factors of the same order for each conformation.

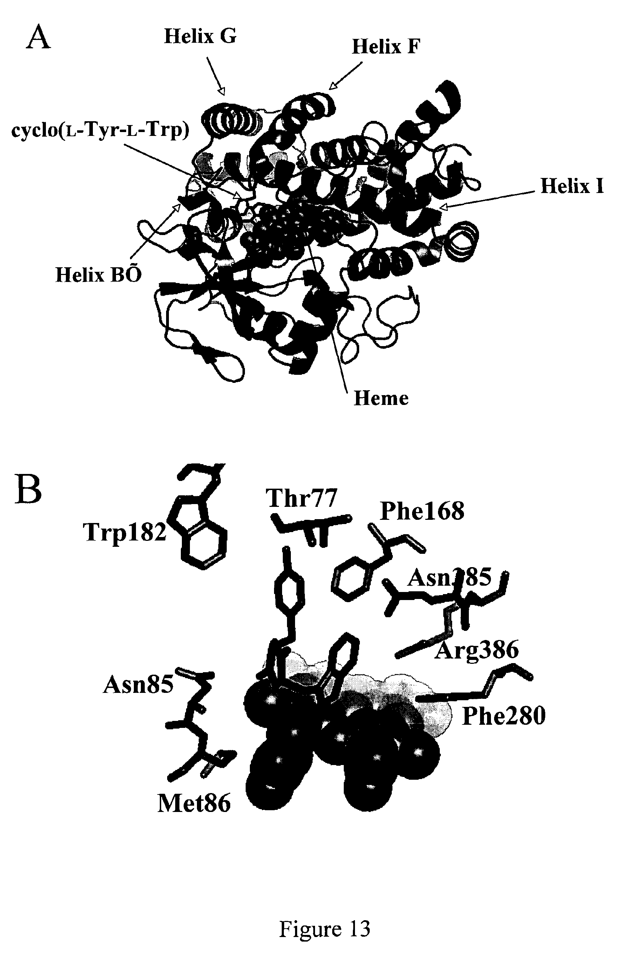

Conformation 1 corresponds to the canonical conformation observed for this family of compound with the tyrosyl moiety at the distal position and the tryptophanyl part at the proximal position (Fig. 13).

In figure 13 the X-ray structure of cyclo(L-Tyr-L-Trp)-bound CYP121 with cyclo(L-Tyr-L-Trp) bound in conformation 1. In panel A, the overall CYP121 holoprotein is represented as ribbons and atoms of the heme are shown as spheres. Cyclo(L-Tyr-L-Trp) (SEQ ID NO:5) is depicted as sticks. The names of the helices that are in contact with cyclo(L-Tyr-L-Trp) (SEQ ID NO:5) are indicted. A detailed view of the heme binding pocket is shown in panel B. Residues surrounding this pocket are indicated.

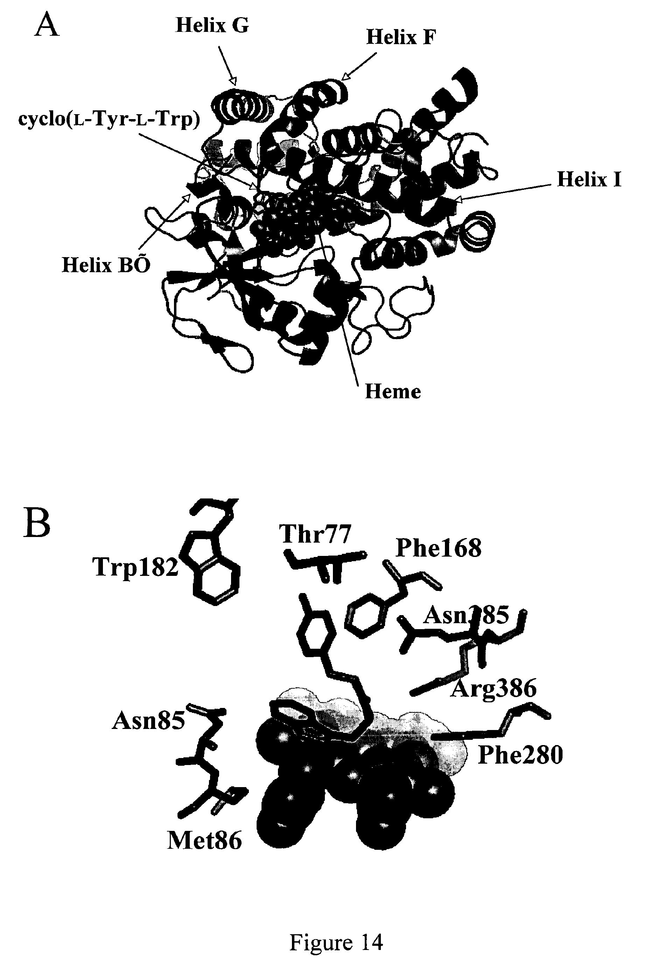

In this conformation, the NE1 of the indole ring interacts with the iron through two water molecules (WAT626 and WAT616) and with the guanidinium of Arg386 through WAT626. The CH2 of the phenyl ring of the indole approaches close to the CE2 of Phe168 (3.21 Å) and is probably responsible of the slight movement of Phe168 (0.6 Å). The interactions network observed at the DKP ring and of the hydroxyl of the distal tyrosyl is conserved as compared with other complexes. However, in the inventors crystallization conditions an alternative conformation could be modelled (Fig. 14), with a quite different orientation of the cyclo(L-Tyr-L-Trp).

Figure 14 shows the X-ray structure of cyclo(L-Tyr-L-Trp)-bound CYP121 with cyclo(L-Tyr-L-Trp) bound in conformation 2. In panel A, the overall CYP121 holoprotein is represented as ribbons and atoms of the heme are shown as spheres. Cyclo(L-Tyr-L-Trp) (SEQ ID NO:5) is depicted as sticks. The names of the helices that are in contact with cyclo(L-Tyr-L-Trp) (SEQ ID NO:5) are indicated. A detailed view of the heme binding pocket is shown in panel B. Residues surrounding this pocket are indicated.

The tyrosyl moiety remains at a distal position, is involved in stacking with Phe168 and Trp182 and the hydroxyl maintains its interactions with water molecules. The main change occurs from the orientation of the Cα-Cβ-Cχ angle of the distal tyrosine which is opposite in both conformations. As a consequence, the DKP ring of conformation 2 occupies roughly the position of the tryptophane indole ring of conformation 1, and the tryptophane indole ring of conformation 2 is located close to the position of the DKP ring of conformation 1.

In conformation 2, one carbonyl of the DKP ring occupies a position very close (∼ 1 Å) to that of the proximal substrate hydroxyle in the CYP121/cyclo(L-Tyr-L-Tyr) (SEQ ID NO:4)complex. Consequently, the water-mediated H-bonding pattern observed between this carbonyl and the iron is identical to that observed between the substrate proximal hydroxyl and the iron in the CYP121/cyclo(L-Tyr-L-Tyr) (SEQ ID NO:4) complex. As in the CYP121/cyclo(L-Tyr-L-Tyr) (SEQ ID NO:4)complex, the H-bonds are very tight (2.67 Å, 2.64 Å and 2.30 Å for the three consecutive H-bonds from the carbonyl to the iron, respectively).

Furthermore, this carbonyl interacts also with the guanidinium of Arg386 as does the substrate proximal hydroxyl of the CYP121/cyclo(L-Tyr-L-Tyr) (SEQ ID NO:4) complex. Altogether, these data show that conformation 2 produces a heme ligation mode similar to that observed with cyclo(L-Tyr-L-Tyr) (SEQ ID NO:4). The interaction of the ligand in both conformations with CYP121 involves leads to similar interaction network due to compensation effects of the ligands atoms. However, the second orientation involves 11 ionic interactions as compared to 7 ionic interactions in conformation 1, in agreement with the higher occupancy observed in conformation 2.

### 3.4 Inhibitors design by using the cyclo(L-Tyr-L-Trp) scaffold

The inventors data show that the cyclo(L-Tyr-L-Trp) (SEQ ID NO:5) belongs to the CYP121 substrate family like the cyclo(L-Tyr-L-Tyr) (SEQ ID NO:4). In the section 2.1, the inventors have shown that it is possible to modify a substrate molecule transformed by CYP121 in order to obtain a molecule that is no more transformed by CYP121 and inhibits CYP121 activity. The inventors propose that the CYP121 substrate cyclo(L-Tyr-L-Trp) (SEQ ID NO:5) could also be modified into a CYP121 inhibitor. The inventors propose that these molecules could be obtained i) by screening for CYP121 binding a chemical library obtained with the cyclo(L-Tyr-L-Trp) cyclodipeptide as scaffold or ii) by any modelling method starting from X-ray structure of CYP121 bound to cyclo(L-Tyr-L-Trp) and followed by chemical synthesis.

### EXAMPLE 4 - Inhibition of CYP121 activity by cyclo(L-Tyr-L-X) analogs

The inventors spectroscopic and crystallographic data presented herein clearly show that two cyclodipeptides can bind to CYP121. The inventors therefore decided to investigate if other cyclodipeptides could be CYP121 ligands and hence be used as scaffold for the design of CYP121 inhibitors. To answer this question, the inventors solved the crystal structure of CYP121 bound to two other cyclodipeptides, cyclo(L-Tyr-L-Phe) (SEQ ID NO:6) and cyclo(L-Tyr-L-Ala) (SEQ ID NO:7).

### 4.1 X-ray structure of cyclo(L-Tyr-L-Phe)-bound CYP121

The crystals of cyclo(L-Tyr-L-Phe)-bound CYP121 belong to the same crystal form (space group = P6₅22) as previously described for unbound CYP121 (13). The structure of CYP121/cyclo(L-Tyr-L-Phe) was solved and refined at 1.8 Å resolution (Table I) demonstrating that the quality of the structure is compatible with fine structural analysis. For structural comparison, the inventors used the structure of ligand-free CYP121 determined in their laboratory, highly similar to the available CYP121 structure (entry 1N40; rms deviation on 388 Cα of 0.09 Å).

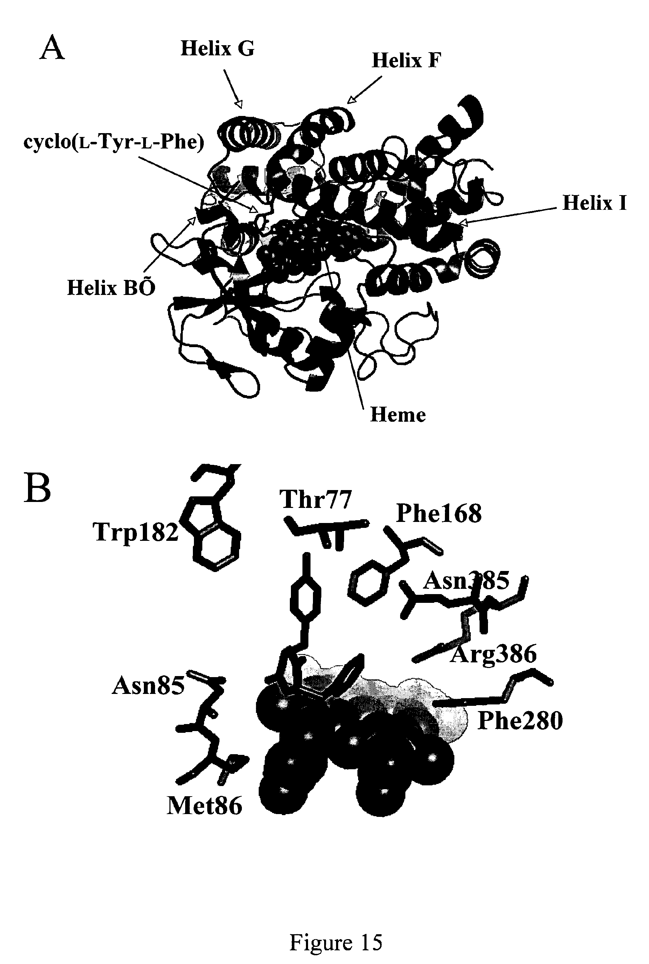

In this complex, the cyclodipeptide occupies the same position than cyclo(L-Tyr-L-Tyr) (SEQ ID NO:6) in cyclo(L-Tyr-L-Tyr)-bound CYP121 with the tyrosyl moiety at the distal position. However, the temperature factors of cyclo(L-Tyr-L-Phe) (SEQ ID NO:6) are approximately twice those obtained for side chain atoms of residues lining the active site cavity (32 Å² and 17-21 Å² respectively). The only difference occurs at the phenylalanyl moiety located closed to the heme (Fig. 15). The phenylalanyl ring is stabilized by a stacking interaction with Phe168 and Phe280 and interacts with the heme only through van der waals interactions.

Figure 15 shows the X-ray structure of cyclo(L-Tyr-L-Phe)-bound CYP121. In panel A, the overall CYP121 holoprotein is represented as ribbons and atoms of the heme are shown as spheres. Cyclo(L-Tyr-L-Phe) (SEQ ID NO:6) is depicted as sticks. The names of the helices that are in contact with cyclo(L-Tyr-L-Phe) (SEQ ID NO:6) are indicted. A detailed view of the heme binding pocket is shown in panel B. Residues surrounding this pocket are indicated.

The CE1 carbon atom of the phenylalanyl moiety is located at 5.3 Å from the iron, i.e. 0.8 Å closer to the iron than cyclo(L-Tyr-L-Tyr) (SEQ ID NO:4). The distal tyrosyl and the DKP ring are stabilized in the same way than in cyclo(L-Tyr-L-Tyr) (SEQ ID NO:4), while the proximal phenylalanyl residue loses the interaction with Arg386 guanidinium and a water molecule, the π-cation delocalization, and the interaction with the iron through two closely H-bonded water molecules.

### 4.2 X-ray structure of cyclo(L-Tyr-L-Ala)-bound CYP121

The crystals of cyclo(L-Tyr-L-Ala)-bound CYP121 belong to the same crystal form (space group = P6₅22) as previously described for unbound CYP121 (13). The structure of CYP121/cyclo(L-Tyr-L-Ala) was solved and refined at 1.9 Å resolution (Table I) demonstrating that the quality of the structure is compatible with fine structural analysis. For structural comparison, we used the structure of ligand-free CYP121 determined in our laboratory, highly similar to the available CYP121 structure (entry 1N40; rms deviation on 388 Cα of 0.09 Å).

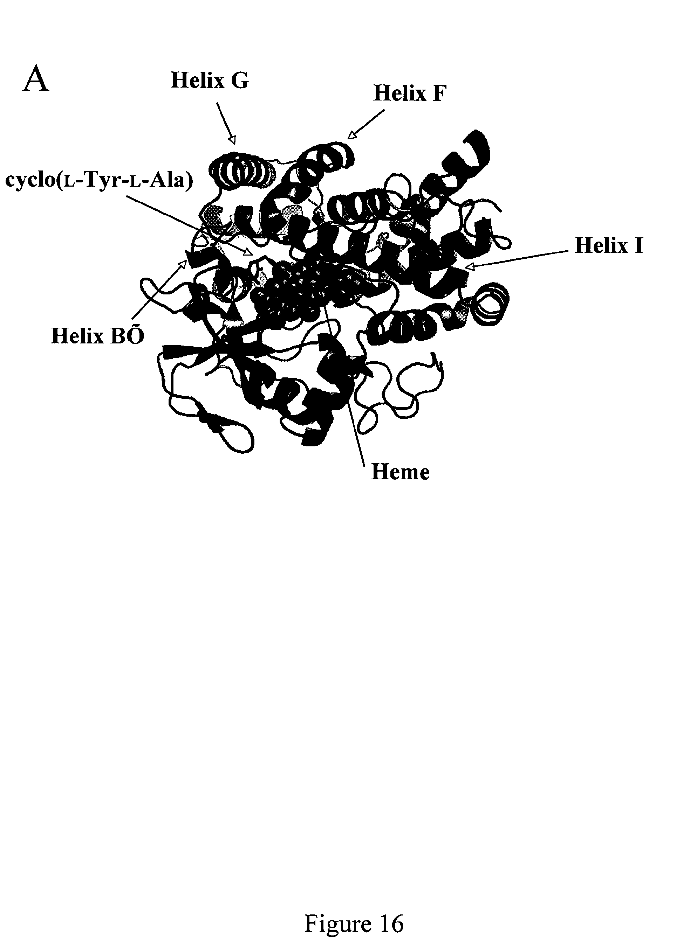

The DKP ring of cyclo(L-Tyr-L-Ala) (SEQ ID NO:7) occupies a position similar to this observed with the previous compounds with a tilt of about 20° centred at the CB of the tyrosyl moiety: one carbonyl of the ring interacts directly with Asn85 and one nitrogen is H-bonded to one propionate of the heme. The CB of the tyrosyl moiety is located close to the position of the CB of the distal tyrosyl in CYP121/cyclo(L-Tyr-L-Tyr) complex (∼ 0.7Å) but the ring of the tyrosyl part rotates by more than 120° around the CA-CB bond and points toward Phe280 (Fig. 16).

Figure 16 shows the X-ray structure of cyclo(L-Tyr-L-Ala)-bound CYP121. In panel A, the overall CYP121 holoprotein is represented as ribbons and atoms of the heme are shown as spheres. Cyclo(L-Tyr-L-Ala) (SEQ ID NO:7) is depicted as sticks. The names of the helices that are in contact with cyclo(L-Tyr-L-Ala) (SEQ ID NO:7) are indicted. A detailed view of the heme binding pocket is shown in panel B. Residues surrounding this pocket are indicated.

Although its hydroxyl is located close (∼ 0.8 Å) to the position of the substrate proximal hydroxyl in CYP121/cyclo(L-Tyr-L-Tyr) complex, the orientation of the hydroxyle is compatible with the interaction with Arg386, but not with the water-mediated interaction with the heme iron. We observe residual density located at the distal position of tyrosyl moiety that suggests an alternate conformation of the ligand molecule. However, this alternate conformation could not be modelled because of a too low occupancy, and we modelled three water molecules instead (WAT570, 575 and 579). Overall, cyclo(L-Tyr-L-Ala) (SEQ ID NO:7) is involved in less interaction than the previous two cyclodipeptides and the presence of residual density at the tyrosyl distal position reflects the mobility of the compound in the pocket.

### 4.3 Inhibitor design by using cyclo(L-Tyr-L-X) as a lead molecule, X being any amino acid

The inventors experimental data show that the cyclodipeptides cyclo(L-Tyr-L-Phe) (SEQ ID NO:6) and cyclo(L-Tyr-L-Ala) (SEQ ID NO:7) bind to CYP121. They propose that cyclo(L-Tyr-L-Phe) (SEQ ID NO:6), cyclo(L-Tyr-L-Ala) (SEQ ID NO:7) and also other cyclodipeptides of general formula cyclo(L-Tyr-L-X) (SEQ ID NO: 9) could be used as lead molecules for the design of CYP121 inhibitors with increased inhibitory activity toward CYP121. They propose that these new inhibitors could be obtained i) by screening for CYP121 binding a chemical library obtained with cyclo(L-Tyr-L-X) (SEQ ID NO:9) as scaffold or ii) by any modelling method starting from X-ray structures of CYP121 bound to cyclo(L-Tyr-L-X) (SEQ ID NO:9) or to any other cyclodipeptides, and followed by chemical synthesis. By using these modelling methods, chemical groups could be designed to fit each of the water-filled cavities surrounding the position occupied either by the ligand in cyclodipeptide-bound CYP121 structure. By using these methods, chemical groups could also be designed to interact tightly with the heme iron through conjugation of electron pair (like azole molecules). By using these methods, chemical groups could also be designed to link covalently the cyclodipeptide scaffold to CYP121, leading to the conception of suicide or mechanism-based inhibitors (30). Examples of such chemical groups efficient for P450 inactivation have been described elsewhere (32).

### EXAMPLE 5 - Inhibition of CYP121 activity by diketopiperazines

### 5.1 Inhibitor design by using the DKP scaffold as a lead molecule

Cyclodipeptides belong to the diketopiperazines (DKPs) family of molecules, shown in formula (i).

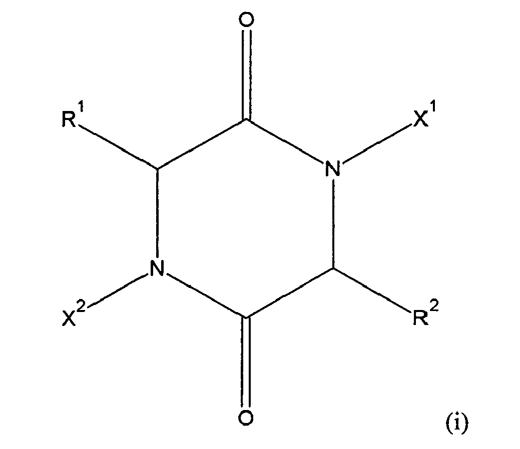

In the last years, DKPs have focused attention because of the large set of biological activities supported by the DKP ring (33).

Diketopiperazines are a class of cyclic organic compounds that result from peptide bonds between two amino acids to form a lactam. They are combinations of ketones and the compound piperazine. Piperazine is an organic compound that consists of a six-membered ring containing two opposing nitrogen atoms.

The physical and chemical properties of the DKP ring make it a suitable scaffold for medicinal chemistry, leading to the use of the DKP scaffold in combinatorial chemistry for the synthesis of libraries and the search for molecules with potential therapeutic properties (34).

The crystal structures of CYP121 in complex with five different cyclodipeptides reveals the molecular determinants of DKPs binding to CYP121 and opens the way for the design of specific inhibitors.

In five of the six complexes observed (two complexes are observed for cyclo(L-Tyr-L-Trp)-bound CYP121), the DKP ring occupies the same position packed against the 3₁₀ helix that precedes helix B' and establishes the same polar contacts with ND2 atom of Asn85 and one propionate of the heme, either directly or through a water molecule.

In the CYP121/cyclo(L-Tyr-L-Trp) complex, this conformation is present with a partial occupancy (0.3), probably because the alternative conformation is better stabilized through additional interactions. The second anchoring point of cyclodipeptides to CYP121 is the stacking interaction involving Phe168, Trp182 and a tyrosyl moiety of the cyclodipeptide. In cyclo(L-Tyr-L-Tyr)-bound and cyclo(L-Tyr-L-Phe)-bound CYP121, the positions of the tyrosyl ring stacked between Phe168 and Trp182 are nearly identical and superimposable with the position of the DOPA moiety of cyclo(L-DOPA-L-Tyr) (SEQ ID NO:8) in the cyclo(L-DOPA-L-Tyr)-bound CYP121 except for the additional hydroxyl.

In the CYP121/cyclo(L-Tyr-L-Trp) complex, the two conformations show this stacking interaction, with the hydroxyl localized nearly at the same position. In the five complexes, the hydroxyls of the tyrosyl moieties are oriented toward a water-filled cavity located between helices F and G while the CE4/CD4 atoms face another water-filled cavity bordered by the F/G loop and the B/B' loop.

According to the inventors data, they can define a set of interactions that could help to design lead candidate CYP121 inhibitors: we propose that molecules establishing contacts with i) the heme, ii) residues of the 3₁₀ helix-B' helix region (P79 P80 E81 V82 V83 N84 N85 M86 G87 N88 I89 A90 D91 A92), iii) with Phe168 and/or Trp182and /or Phe280 and iv) with Asn385 and/or Arg386 could constitute lead molecules for the design of CYP121 inhibitors. Assuming that the DKP ring interacts with the 3₁₀ helix-B' helix region and the heme as observed in the different cyclodipeptide-bound CYP121 complexes presented in this work, the DKP ring may constitute a lead molecule for the design of CYP121 inhibitors. Starting from this ring, library could be constituted and screened for efficient binding to CYP121. Inhibitors could also be designed by using the crystal structures presented in this work and modelling the positioning of chemical groups on the DKP ring. As previously mentioned in this work, chemical groups could be added i) to fit the water-filled cavities of CYP121 substrate binding site, ii) to interact tightly with the heme iron through conjugation of electron pair (like azole molecules), iii) to link covalently the DKP molecule to CYP121, leading to the conception of suicide or mechanism-based inhibitors (30). Examples of such chemical groups efficient for P450 inactivation have been described elsewhere (32). Mechanism-based enzyme inhibition, by definition, requires metabolism of a substrate to a reactive intermediate that can bind to the enzyme irreversibly, resulting in the loss of enzyme activity. This process of inactivation has been exploited in the design of P450 form-selective inactivators by the incorporation of dihalomethyl or acetylenic moieties at the preferred site of oxidation. Furthermore, the inactivation process can occur by heme alkylation and destruction and/or apoprotein modification.

### REFERENCES

1. Corbett, E. L., Watt, C. J., Walker, N., Maher, D., Williams, B. G., Raviglione, M. C., and Dye, C. (2003) Arch Intern Med 163(9), 1009-1021
2. Espinal, M. A. (2003) Tuberculosis (Edinb) 83(1-3), 44-51
3. O'Brien, R. J., and Nunn, P. P. (2001) Am J Respir Crit Care Med 163(5), 1055-1058
4. Duncan, K., and Barry, C. E., 3rd. (2004) Curr Opin Microbiol 7(5), 460-465
5. McLean, K. J., Dunford, A. J., Neeli, R., Driscoll, M. D., and Munro, A. W. (2007) Arch Biochem Biophys
6. Guengerich, F. P. (2001) Chem Res Toxicol 14(6), 611-650
7. Cole, S. T., Brosch, R., Parkhill, J., Garnier, T., Churcher, C., Harris, D., Gordon, S. V., Eiglmeier, K., Gas, S., Barry, C. E., 3rd, Tekaia, F., Badcock, K., Basham, D., Brown, D., Chillingworth, T., Connor, R., Davies, R., Devlin, K., Feltwell, T., Gentles, S., Hamlin, N., Holroyd, S., Homsby, T., Jagels, K., Krogh, A., McLean, J., Moule, S., Murphy, L., Oliver, K., Osborne, J., Quail, M. A., Rajandream, M. A., Rogers, J., Rutter, S., Seeger, K., Skelton, J., Squares, R., Squares, S., Sulston, J. E., Taylor, K., Whitehead, S., and Barrell, B. G. (1998) Nature 393(6685), 537-544
8. Brennan, P. J. (2003) Tuberculosis (Edinb) 83(1-3), 91-97
9. Burguiere, A., Hitchen, P. G., Dover, L. G., Dell, A., and Besra, G. S. (2005) Microbiology 151 (Pt 6), 2087-2095
10. Ahmad, Z., Sharma, S., and Khuller, G. K. (2006) FEMS Microbiol Lett 261(2), 181-186
11. Ahmad, Z., Sharma, S., and Khuller, G. K. (2006) FEMS Microbiol Lett 258(2), 200-203
12. McLean, K. J., Marshall, K. R., Richmond, A., Hunter, I. S., Fowler, K., Kieser, T., Gurcha, S. S., Besra, G. S., and Munro, A. W. (2002) Microbiology 148(Pt 10), 2937-2949
13. Leys, D., Mowat, C. G., McLean, K. J., Richmond, A., Chapman, S. K., Walkinshaw, M. D., and Munro, A. W. (2003) J Biol Chem 278(7), 5141-5147
14. Seward, H. E., Roujeinikova, A., McLean, K. J., Munro, A. W., and Leys, D. (2006) J Biol Chem 281(51), 39437-39443
15. Sun, G., Thai, S. F., Lambert, G. R., Wolf, D. C., Tully, D. B., Goetz, A. K., George, M. H., Grindstaff, R. D., Dix, D. J., and Nesnow, S. (2006) Toxicol Lett 164(1), 44-53
16. Brodie, A., and Long, B. (2001) Clin Cancer Res 7(12 Suppl), 4343s-4349s; discussion 4411s-4412s
17. McLean, K. J., Cheesman, M. R., Rivers, S. L., Richmond, A., Leys, D., Chapman, S. K., Reid, G. A., Price, N. C., Kelly, S. M., Clarkson, J., Smith, W. E., and Munro, A. W. (2002) J Inorg Biochem 91(4), 527-541
18. Sambrook, J., and Russell, D. W. (2001) Molecular cloning: a laboratory manual, Cold Spring Harbor Laboratory Press, Cold Spring Harbor, New York
19. Jeedigunta, S., Krenisky, J. M., and Kerr, R. G. (2000) Tetrahedron 56, 3303-3307
20. Mowat, C. G., Leys, D., McLean, K. J., Rivers, S. L., Richmond, A., Munro, A. W., Ortiz Lombardia, M., Alzari, P. M., Reid, G. A., Chapman, S. K., and Walkinshaw, M. D. (2002) Acta Crystallogr D Biol Crystallogr 58(Pt 4), 704-705
21. COLLABORATIVE COMPUTATIONAL PROJECT, N. (1994) Acta Crystallogr D Biol Crystallogr D50, 760-763
22. Vagin, A., and Teplyakov, A. (1997) J Appl Cryst 30, 1022-1025
23. Murshudov, G. N., Vagin, A. A., and Dodson, E. J. (1997) Acta Crystallogr D Biol Crystallogr 53(Pt 3), 240-255
24. Roussel, A., and Cambillau, C. (1989)
25. Roback, P., Beard, J., Baumann, D., Gille, C., Henry, K., Krohn, S., Wiste, H., Voskuil, M. I., Rainville, C., and Rutherford, R. (2007) Nucleic Acids Res 35(15), 5085-5095
26. Lautru, S., Gondry, M., Genet, R., and Pernodet, J. L. (2002) Chem Biol 9(12), 1355-1364
27. Schenkman, J. B., and Jansson, I. (2006) Methods Mol Biol 320, 11-18
28. Cojocaru, V., Winn, P. J., and Wade, R. C. (2007) Biochim Biophys Acta 1770(3), 390-401
29. Schuster, I., and Bernhardt, R. (2007) Drug Met Rev 39, 481-499
30. Silverman, R. B. (1995) Methods Enzymol 249, 240-283
31. Denisov, I. G., Makris, T. M., Sligar, S. G., and Schlichting, I. (2005) Chem Rev 105(6), 2253-2277
32. Kent, U. M., Juschyshyn, M. I., and Hollenberg, P. F. (2001) Curr Drug Metab 2(3), 215-243
33. Prasad, C. (1995) Peptides 16(1), 151-164
34. Besada, P., Mamedova, L., Thomas, C. J., Costanzi, S., and Jacobson, K. A. (2005) Org Biomol Chem 3(10), 2016-2025
35. Tang, M. R., Sternberg, D., Behr, R. K., Sloma, A., and Berka, R. M. (2006) Industrial Biotechnology 2, 66-74
36. Makris, T. M., Denisov, I. G., Schlichting, I., and Sligar, S. G. (2005) Activation of Molecular Oxygen by Cytochrome P450. In: Ortiz de Montellano, P. R. (ed). Cytochrome P450: Structure, Mechanism and Biochemistry, 3e, Kluwer Academic/Plenum, New York
37. Nagano, S., Cupp-Vickery, J. R., and Poulos, T. L. (2005) J Biol Chem 280(23), 22102-22107
38. Zhao, B., Guengerich, F. P., Voehler, M., and Waterman, M. R. (2005) J Biol Chem 280(51), 42188-42197
39. Raag, R., and Poulos, T. L. (1991) Biochemistry 30(10), 2674-2684
40. Davydov, R., Macdonald, I. D. G., Makris, T. M., Sligar, S. G., and Hoffman, B. M. (1999) J Am Chem Soc 121, 10654-10655

## Claims

1. A compound of the formula (i): 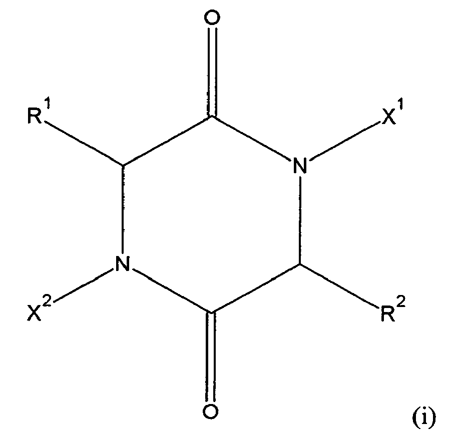 wherein one of R¹ and R² is a tyrosyl side chain or a substituted tyrosyl side chain, then the other is any proteinogenic or non-proteinogenic amino acid side chain except for a tyrosyl side chain when said one of R¹ and R² is a tyrosyl side chain; and
wherein X¹ and X² which may be identical or different are any suitable chemical groups and in particular may be a hydrogen atom, a hydroxyl group, amino group, a (C₁-C₆) alkyl group, an aromatic group optionally substituted and suitable combinations of these groups and in particular O-alkyl moiety, O-aromatic moiety, N-alkyl moiety, N-aromatic moiety, functionalized alkyl group, functionalized aromatic group;
for use in a method of inhibiting CYP121 activity in at least one condition wherein said CYP121 is active.

2. The compound of claim 1, wherein said at least one condition is caused by infection by a Mycobacterium.

3. The compound of claim 1 or 2, wherein said compound is not catalysed by CYP121.

4. The compound of claims 1, 2 or 3 wherein said compound establishes contacts with one or more portions of CYP121 selected from the group:
i) the heme group,
ii) at least one of the residues of the 3₁₀ helix-B' helix region which comprises residues P79 P80 E81 V82 V83 N84 N85 M86 G87 N88 I89 A90 D91 A92,
iii) residues Gln385 and/or Arg386
iv) residues Phe168 and/or Trp182 and/or Phe280.

5. The compound of anyone of claims 1 to 4, wherein interaction of said compound with CYP121 causes a shift in spin equilibrium of the heme iron of CYP121.

6. The compound of any one of claims 1 to 5 wherein said compound is selected from the list consisting of: SEQ ID NO: 5, SEQ ID NO: 6, SEQ ID NO: 7, SEQ ID NO: 8; SEQ ID NO: 9.

7. The compound of any one of claims 1, 2, 4, 5 or 6, wherein said compound following catalysis by CYP121 generates a reactive intermediate that can bind to CYP 121 irreversibly.

8. The compound of claim 7, wherein said compound comprises at least one dihalomethyl and/or acetylenic moiety.

9. A medicament, **characterized in that** it comprises a compound of the formula (i): 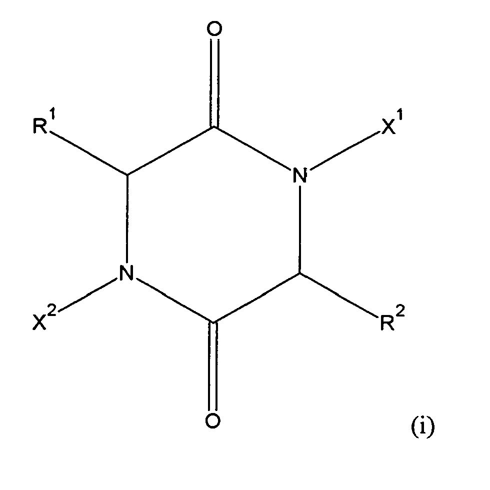 wherein one of R¹ and R² is a tyrosyl side chain or a substituted tyrosyl side chain, then the other is any proteinogenic or non-proteinogenic amino acid side chain except for a tyrosyl side chain when said one of R¹ and R² is a tyrosyl side chain; and
wherein X¹ and X² which may be identical or different are any suitable chemical groups and in particular may be a hydrogen atom, a hydroxyl group, amino group, a (C₁-C₆) alkyl group, an aromatic group optionally substituted and suitable combinations of these groups and in particular O-alkyl moiety, O-aromatic moiety, N-alkyl moiety, N-aromatic moiety, functionalized alkyl group, functionalized aromatic group;
for use in a method of treating a *Mycobacterium* infection.

10. A pharmaceutical composition, **characterized in that** it comprises:
a compound of the formula (i): 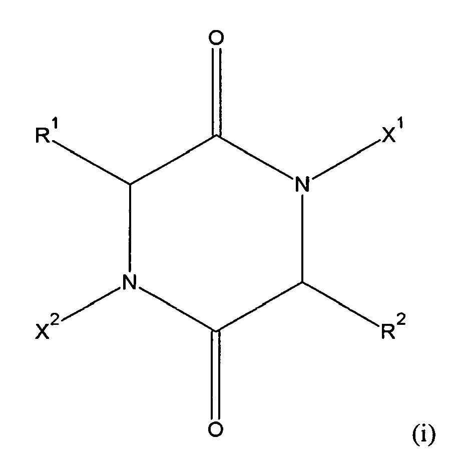
wherein R¹ is a tyrosyl side chain or a substituted tyrosyl side chain and R² is any proteinogenic or non-proteinogenic amino acid side chain except for a tyrosyl side chain when R¹ is a tyrosyl side chain; and
wherein X¹ and X² are any suitable chemical groups and in particular may be a hydrogen atom, a hydroxyl group, amino group, a (C₁-C₆) alkyl group, an aromatic group optionally substituted and suitable combinations of these groups and in particular O-alkyl moiety, O-aromatic moiety, N-alkyl moiety, N-aromatic moiety, functionalized alkyl group, functionalized aromatic group;
at least one pharmaceutically acceptable vehicle;
for use in a method of treating a *Mycobacterium* infection.

11. X-ray three dimensional structure of CYP121 in combination with a target selected from the list consisting of SEQ ID NO: 4, SEQ ID NO: 5, SEQ ID NO: 6, SEQ ID NO: 7, SEQ ID NO: 8; SEQ ID NO: 9;
generated with the atomic coordinates listed in Table I and Figures 19, 20, 21, 22 and 23.

12. A method to design a diketopiperazine containing compound which inhibits the activity of CYP121, comprising the steps:
a) generating a 3-dimensional model of said compound in combination with CYP121 by using the interaction network between CYP121 and at least one cyclodipeptide selected from the list consisting of: SEQ ID NO:4, SEQ ID NO: 5, SEQ ID NO: 6, SEQ ID NO: 7, SEQ ID NO: 8; SEQ ID NO: 9:
b) designing said compound by modifying chemical groups thereon and testing the interaction of said compound with CYP121 using the model generated in step a) by using bioinformatics tools to determine interactions of said compound with one of the features of CYP121 selected from the group:
i) the heme group,
ii) at least one of the residues bordering the active site first cavity located between helices F and G and comprising residues S163 L 164 1166 A 167 F 168 I175 A178 K179 N181 W182 D185,
iii) at least one of the residues bordering the active site second cavity located between the F/G loop and B/B' loop and comprising residues R72 L73 N74 L76 V78 A167 F168 M169 S170 S171 W182;
iv) at least one of the residues bordering the active site third cavity located just above the heme and comprising residues M62 R72 L73 N74 L76 T77 F168 A233 I236 S237 F280 D282 G283 L284 P285 Q385 R386.

13. The method of claim 12, wherein said compound is designed by adding at least one chemical group to the diketopiperazine cycle.

14. The method of claim 12 or 13, wherein said compound is designed by substituting at least one chemical group of the diketopiperazine cycle.

15. The method of claim 12, 13 or 14, wherein said compound is designed by modifying the size of the diketopiperazine cycle.

16. A method to screen at least one compound for the ability to inhibit CYP121 activity, comprising the steps:
a) synthesizing said at least one compound,
b) incubating CYP121, an exogenous electron transport chain, NADPH, a substrate of CYP121 and said at least one compound together in predetermined reaction conditions,
c) collecting the products of step b),
d) analysing the products of step c),
e) comparing the products of step c) to a negative control experiment in which said at least one compound is omitted from step b),
f) determining the inhibitory effects of said at least one compound upon CYP121 based upon the results of step e).

17. The method according to claim 16, wherein said substrate is cyclo(L-Tyr-L-Tyr) (SEQ ID NO: 4).

18. The method of claim 16 or 17, wherein said at least one compound is designed according any one of claims 12 to 15.

19. The method of any one of claims 16, 17 or 18, wherein method is used to screen a library of compounds.

20. The method of any one of claims 16 to 19, wherein the analysis of step d) is performed by reverse phase HPLC.

21. The method of any one of claims 16 to 20, comprising a further step:
e') of comparing the products of step c) to a positive control experiment in which an azole is present in step b).

22. The method of claim 21, wherein said azole is selected from the group comprising: clotrimazole, ketoconazole, miconazole, fluconazole, econazole.

23. A data array comprising the atomic coordinate as set forth in Table I and Figures 19, 20, 21, 22, 23, of CYP121 in combination with a target selected from the group consisting of SEQ ID NO: 4, SEQ ID NO: 5, SEQ ID NO: 6, SEQ ID NO: 7, SEQ ID NO: 8; SEQ ID NO: 9;
which when acted upon by a protein modelling algorithm yields a representation of the 3-D structure of CYP121 in combination with its target.


SEQUENCE LISTING

<110>  Commissariat à l'énergie atomique

<120>  Novel inhibitors of Mycobacterium tuberculosis complex cytochrome P450
CYP121

<130>  F263-244

<160>  10

<170>  PatentIn version 3.3

<210>  1
<211>  1191
<212>  DNA
<213>  Mycobacterium tuberculosis

<400>  1
atgaccgcga ccgttctgct cgaggtcccg ttctctgcac gtggggatcg gattcctgac      60

gccgtcgcag aattacgaac ccgcgagcct atccgcaagg tacggaccat taccggcgcc     120

gaagcctggc tcgtctcctc gtatgcactg tgcacacagg tgctcgagga tcggcgtttt     180

tccatgaagg aaaccgccgc tgccggcgcc ccccgcctga acgcgctgac tgttccaccc     240

gaagtggtca acaacatggg aaacatcgcc gacgcgggac tgcgcaaggc ggtgatgaaa     300

gcgatcacac ccaaggcacc cgggttggag caattcctac gagacaccgc gaactcgctg     360

ctggacaacc tgattaccga gggcgcacca gccgatctgc gcaatgactt cgccgacccg     420

ctggccactg ccctgcactg caaggttctg ggcatcccgc aagaagacgg cccgaagctg     480

ttccgtagct tgagtatcgc tttcatgagt tcggccgacc cgatccccgc cgcgaagatc     540

aactgggatc gcgacatcga atacatggcc ggaattctgg aaaacccaaa catcacgacc     600

ggcctcatgg gtgagctcag ccgcctccgg aaagatcccg cctactcgca cgtctccgac     660

gaactattcg cgaccatcgg cgtcactttc ttcggtgccg gcgtcatctc aaccggcagc     720

ttcctcacca ccgcgctgat atcgctgata caacgcccgc aacttcggaa cttgttgcac     780

gagaagccgg aactgatccc ggccggtgta gaggaactgc tgcggatcaa tctctccttc     840

gccgacgggt taccgcgcct ggccaccgcc gacatccagg tcggcgacgt gctggtccgc     900

aagggggagc tggtgctggt gctgctcgag ggcgccaact tcgatcccga gcacttccct     960

aacccgggca gcatcgaact cgaccggccc aaccccacct cgcacctcgc gttcggccgc    1020

ggccaacact tctgtcctgg atcagctctc ggtcgccgcc acgcacagat cggcatcgaa    1080

gcgctgttga aaaagatgcc cggcgtcgac ctggctgtgc ccatcgacca attggtctgg    1140

cgcacccgat tccaaagacg catccccgaa cgccttccgg tgctctggta g             1191


<210>  2
<211>  49
<212>  DNA
<213>  Artificial

<220>
<223>  Sequence of cloning primer 1

<400>  2
ttgttacccc tgataggagc ccagccatat gaccgcgacc gttctgctc                49


<210>  3
<211>  49
<212>  DNA
<213>  Artificial

<220>
<223>  Sequence of Cloning Primer 2

<400>  3
atggctcggg tgagtttagg gatcctacca gagcaccgga aggcgttcg                49


<210>  4
<211>  2
<212>  PRT
<213>  Artificial

<220>
<223>  cyclo(L-Tyr-L-Tyr)

<400>  4

Tyr Tyr
1


<210>  5
<211>  2
<212>  PRT
<213>  Artificial

<220>
<223>  cyclo(L-Tyr-L-Trp)

<400>  5

Tyr Trp
1


<210>  6
<211>  2
<212>  PRT
<213>  Artificial

<220>
<223>  Cyclo(L-Tyr-L-Phe)

<400>  6

Tyr Phe
1


<210>  7
<211>  2
<212>  PRT
<213>  Artificial

<220>
<223>  Cyclo(L-Tyr-L-Ala)

<400>  7

Tyr Ala
1

<210> 8
<211> 2
<212> PRT
<213> Artificial

<220>
<223> Cyclo(L-dihydroxyphenylalanine-L-Tyr)

<400> 8

Tyr Tyr
1

<210> 9
<211> 2
<212> PRT
<213> Artificial

<220>
<223> Cyclo(L-Tyr-L-X)

<220>
<221> misc_feature
<222> (2)..(2)
<223> Xaa can be any naturally occurring amino acid

<400> 9

Tyr Xaa
1

<210> 10
<211> 6831
<212> DNA
<213> Artificial

<220>
<223> pET11a-CYP121 sequence

<400> 10
ttctcatgtt tgacagctta tcatcgataa gctttaatgc ggtagtttat cacagttaaa 60

ttgctaacgc agtcaggcac cgtgtatgaa atctaacaat gcgctcatcg tcatcctcgg 120

caccgtcacc ctggatgctg taggcatagg cttggttatg ccggtactgc cgggcctctt 180

gcgggatatc cggatatagt tcctcctttc agcaaaaaac ccctcaagac ccgtttagag 240

gccccaaggg gttatgctag ttattgctca gcggtggcag cagccaactc agcttccttt 300

cgggctttgt tagcagccgg atcctaccag agcaccggaa ggcgttcggg gatgcgtctt 360

tggaatcggg tgcgccagac caattggtcg atgggcacag ccaggtcgac gccgggcatc 420

tttttcaaca gcgcttcgat gccgatctgt gcgtggcggc gaccgagagc tgatccagga 480

cagaagtgtt ggccgcggcc gaacgcgagg tgcgaggtgg ggttgggccg gtcgagttcg 540

atgctgcccg ggttagggaa gtgctcggga tcgaagttgg cgccctcgag cagcaccagc 600

accagctccc ccttgcggac cagcacgtcg ccgacctgga tgtcggcggt ggccaggcgc 660

ggtaacccgt cggcgaagga gagattgatc cgcagcagtt cctctacacc ggccgggatc 720
agttccggct tctcgtgcaa caagttccga agttgcgggc gttgtatcag cgatatcagc 780
gcggtggtga ggaagctgcc ggttgagatg acgccggcac cgaagaaagt gacgccgatg 840
gtcgcgaata gttcgtcgga gacgtgcgag taggcgggat ctttccggag gcggctgagc 900
tcacccatga ggccggtcgt gatgtttggg ttttccagaa ttccggccat gtattcgatg 960
tcgcgatccc agttgatctt cgcggcgggg atcgggtcgg ccgaactcat gaaagcgata 1020
ctcaagctac ggaacagctt cgggccgtct tcttgcggga tgcccagaac cttgcagtgc 1080
agggcagtgg ccagcgggtc ggcgaagtca ttgcgcagat cggctggtgc gccctcggta 1140
atcaggttgt ccagcagcga gttcgcggtg tctcgtagga attgctccaa cccgggtgcc 1200
ttgggtgtga tcgctttcat caccgccttg cgcagtcccg cgtcggcgat gtttcccatg 1260
ttgttgacca cttcgggtgg aacagtcagc gcgttcaggc ggggggcgcc ggcagcggcg 1320
gtttccttca tggaaaaacg ccgatcctcg agcacctgtg tgcacagtgc atacgaggag 1380
acgagccagg cttcggcgcc ggtaatggtc cgtaccttgc ggataggctc gcgggttcgt 1440
aattctgcga cggcgtcagg aatccgatcc ccacgtgcag agaacgggac ctcgagcaga 1500
acggtcgcgg tcatatgtat atctccttct taaagttaaa caaaattatt tctagagggg 1560
aattgttatc cgctcacaat tcccctatag tgagtcgtat taatttcgcg ggatcgagat 1620
ctcgatcctc tacgccggac gcatcgtggc cggcatcacc ggcgccacag gtgcggttgc 1680
tggcgcctat atcgccgaca tcaccgatgg ggaagatcgg gctcgccact tcgggctcat 1740
gagcgcttgt ttcggcgtgg gtatggtggc aggccccgtg gccgggggac tgttgggcgc 1800
catctccttg catgcaccat tccttgcggc ggcggtgctc aacggcctca acctactact 1860
gggctgcttc ctaatgcagg agtcgcataa gggagagcgt cgagatcccg gacaccatcg 1920
aatggcgcaa aacctttcgc ggtatggcat gatagcgccc ggaagagagt caattcaggg 1980
tggtgaatgt gaaaccagta acgttatacg atgtcgcaga gtatgccggt gtctcttatc 2040
agaccgtttc ccgcgtggtg aaccaggcca gccacgtttc tgcgaaaacg cgggaaaaag 2100
tggaagcggc gatggcggag ctgaattaca ttcccaaccg cgtggcacaa caactggcgg 2160
gcaaacagtc gttgctgatt ggcgttgcca cctccagtct ggccctgcac gcgccgtcgc 2220
aaattgtcgc ggcgattaaa tctcgcgccg atcaactggg tgccagcgtg gtggtgtcga 2280
tggtagaacg aagcggcgtc gaagcctgta aagcggcggt gcacaatctt ctcgcgcaac 2340
gcgtcagtgg gctgatcatt aactatccgc tggatgacca ggatgccatt gctgtggaag 2400
ctgcctgcac taatgttccg gcgttatttc ttgatgtctc tgaccagaca cccatcaaca 2460
gtattatttt ctcccatgaa gacggtacgc gactgggcgt ggagcatctg gtcgcattgg 2520
gtcaccagca aatcgcgctg ttagcgggcc cattaagttc tgtctcggcg cgtctgcgtc 2580
tggctggctg gcataaatat ctcactcgca atcaaattca gccgatagcg gaacgggaag 2640
gcgactggag tgccatgtcc ggttttcaac aaaccatgca aatgctgaat gagggcatcg 2700

ttcccactgc gatgctggtt gccaacgatc agatggcgct gggcgcaatg cgcgccatta 2760
ccgagtccgg gctgcgcgtt ggtgcggata tctcggtagt gggatacgac gataccgaag 2820
acagctcatg ttatatcccg ccgttaacca ccatcaaaca ggattttcgc ctgctggggc 2880
aaaccagcgt ggaccgcttg ctgcaactct ctcagggcca ggcggtgaag ggcaatcagc 2940
tgttgcccgt ctcactggtg aaaagaaaaa ccaccctggc gcccaatacg caaaccgcct 3000
ctccccgcgc gttggccgat tcattaatgc agctggcacg acaggtttcc cgactggaaa 3060
gcgggcagtg agcgcaacgc aattaatgta agttagctca ctcattaggc accgggatct 3120
cgaccgatgc ccttgagagc cttcaaccca gtcagctcct tccggtgggc gcggggcatg 3180
actatcgtcg ccgcacttat gactgtcttc tttatcatgc aactcgtagg acaggtgccg 3240
gcagcgctct gggtcatttt cggcgaggac cgctttcgct ggagcgcgac gatgatcggc 3300
ctgtcgcttg cggtattcgg aatcttgcac gccctcgctc aagccttcgt cactggtccc 3360
gccaccaaac gtttcggcga gaagcaggcc attatcgccg gcatggcggc cgacgcgctg 3420
ggctacgtct tgctggcgtt cgcgacgcga ggctggatgg ccttccccat tatgattctt 3480
ctcgcttccg gcggcatcgg gatgcccgcg ttgcaggcca tgctgtccag gcaggtagat 3540
gacgaccatc agggacagct tcaaggatcg ctcgcggctc ttaccagcct aacttcgatc 3600
actggaccgc tgatcgtcac ggcgatttat gccgcctcgg cgagcacatg gaacgggttg 3660
gcatggattg taggcgccgc cctatacctt gtctgcctcc ccgcgttgcg tcgcggtgca 3720
tggagccggg ccacctcgac ctgaatggaa gccggcggca cctcgctaac ggattcacca 3780
ctccaagaat tggagccaat caattcttgc ggagaactgt gaatgcgcaa accaaccctt 3840
ggcagaacat atccatcgcg tccgccatct ccagcagccg cacgcggcgc atctcgggca 3900
gcgttgggtc ctggccacgg gtgcgcatga tcgtgctcct gtcgttgagg acccggctag 3960
gctggcgggg ttgccttact ggttagcaga atgaatcacc gatacgcgag cgaacgtgaa 4020
gcgactgctg ctgcaaaacg tctgcgacct gagcaacaac atgaatggtc ttcggtttcc 4080
gtgtttcgta aagtctggaa acgcggaagt cagcgccctg caccattatg ttccggatct 4140
gcatcgcagg atgctgctgg ctaccctgtg gaacacctac atctgtatta acgaagcgct 4200
ggcattgacc ctgagtgatt tttctctggt cccgccgcat ccataccgcc agttgtttac 4260
cctcacaacg ttccagtaac cgggcatgtt catcatcagt aacccgtatc gtgagcatcc 4320
tctctcgttt catcggtatc attaccccca tgaacagaaa tcccccttac acggaggcat 4380
cagtgaccaa acaggaaaaa accgccctta acatggcccg ctttatcaga agccagacat 4440
taacgcttct ggagaaactc aacgagctgg acgcggatga acaggcagac atctgtgaat 4500
cgcttcacga ccacgctgat gagctttacc gcagctgcct cgcgcgtttc ggtgatgacg 4560
gtgaaaacct ctgacacatg cagctcccgg agacggtcac agcttgtctg taagcggatg 4620
ccgggagcag acaagcccgt cagggcgcgt cagcgggtgt tggcgggtgt cggggcgcag 4680
ccatgaccca gtcacgtagc gatagcggag tgtatactgg cttaactatg cggcatcaga 4740

gcagattgta ctgagagtgc accatatatg cggtgtgaaa taccgcacag atgcgtaagg 4800
agaaaatacc gcatcaggcg ctcttccgct tcctcgctca ctgactcgct gcgctcggtc 4860
gttcggctgc ggcgagcggt atcagctcac tcaaaggcgg taatacggtt atccacagaa 4920
tcaggggata acgcaggaaa gaacatgtga gcaaaaggcc agcaaaaggc caggaaccgt 4980
aaaaaggccg cgttgctggc gtttttccat aggctccgcc cccctgacga gcatcacaaa 5040
aatcgacgct caagtcagag gtggcgaaac ccgacaggac tataaagata ccaggcgttt 5100
ccccctggaa gctccctcgt gcgctctcct gttccgaccc tgccgcttac cggatacctg 5160
tccgcctttc tcccttcggg aagcgtggcg ctttctcata gctcacgctg taggtatctc 5220
agttcggtgt aggtcgttcg ctccaagctg ggctgtgtgc acgaaccccc cgttcagccc 5280
gaccgctgcg ccttatccgg taactatcgt cttgagtcca acccggtaag acacgactta 5340
tcgccactgg cagcagccac tggtaacagg attagcagag cgaggtatgt aggcggtgct 5400
acagagttct tgaagtggtg gcctaactac ggctacacta gaaggacagt atttggtatc 5460
tgcgctctgc tgaagccagt taccttcgga aaaagagttg gtagctcttg atccggcaaa 5520
caaaccaccg ctggtagcgg tggttttttt gtttgcaagc agcagattac gcgcagaaaa 5580
aaaggatctc aagaagatcc tttgatcttt tctacggggt ctgacgctca gtggaacgaa 5640
aactcacgtt aagggatttt ggtcatgaga ttatcaaaaa ggatcttcac ctagatcctt 5700
ttaaattaaa aatgaagttt taaatcaatc taaagtatat atgagtaaac ttggtctgac 5760
agttaccaat gcttaatcag tgaggcacct atctcagcga tctgtctatt tcgttcatcc 5820
atagttgcct gactccccgt cgtgtagata actacgatac gggagggctt accatctggc 5880
cccagtgctg caatgatacc gcgagaccca cgctcaccgg ctccagattt atcagcaata 5940
aaccagccag ccggaagggc cgagcgcaga agtggtcctg caactttatc cgcctccatc 6000
cagtctatta attgttgccg ggaagctaga gtaagtagtt cgccagttaa tagtttgcgc 6060
aacgttgttg ccattgctgc aggcatcgtg gtgtcacgct cgtcgtttgg tatggcttca 6120
ttcagctccg gttcccaacg atcaaggcga gttacatgat cccccatgtt gtgcaaaaaa 6180
gcggttagct ccttcggtcc tccgatcgtt gtcagaagta agttggccgc agtgttatca 6240
ctcatggtta tggcagcact gcataattct cttactgtca tgccatccgt aagatgcttt 6300
tctgtgactg gtgagtactc aaccaagtca ttctgagaat agtgtatgcg gcgaccgagt 6360
tgctcttgcc cggcgtcaac acgggataat accgcgccac atagcagaac tttaaaagtg 6420
ctcatcattg gaaaacgttc ttcggggcga aaactctcaa ggatcttacc gctgttgaga 6480
tccagttcga tgtaacccac tcgtgcaccc aactgatctt cagcatcttt tactttcacc 6540
agcgtttctg ggtgagcaaa aacaggaagg caaaatgccg caaaaaaggg aataagggcg 6600
acacggaaat gttgaatact catactcttc ctttttcaat attattgaag catttatcag 6660
ggttattgtc tcatgagcgg atacatattt gaatgtattt agaaaaataa acaaataggg 6720
gttccgcgca catttccccg aaaagtgcca cctgacgtct aagaaaccat tattatcatg 6780

acattaacct ataaaaatag gcgtatcacg aggccctttc gtcttcaaga a 6831